# EUROPEAN PATENT APPLICATION

(11) **EP 4 733 303 A1**
(43) Date of publication of application: **29.04.2026**
(21) Application number: 24825274.4
(22) Date of filing: 19.06.2024
(51) Int. Cl.: C07D 471/04, A61K 31/519, A61P 25/16, A61P 25/18, A61P 25/28, A61P 25/14

(54) **MUSCARINIC M4 RECEPTOR AGONIST AND USE THEREOF**

(30) Priority: 20.06.2023 CN 202310733937; 19.07.2023 CN 202310888297; 24.08.2023 CN 202311073971; 27.09.2023 CN 202311261376; 02.11.2023 CN 202311448138; 06.12.2023 CN 202311663277; 04.02.2024 CN 202410158431; 27.03.2024 CN 202410358125; 30.04.2024 CN 202410535692; 22.05.2024 CN 202410640941
(71) Applicant: Haisco Pharmaceutical Group Co., Ltd., Shannan, Tibet 856000 (CN)
(72) Inventor: LI, Yao, Chengdu, Sichuan 611130 (CN); ZHANG, Guobiao, Chengdu, Sichuan 611130 (CN); CHEN, Lei, Chengdu, Sichuan 611130 (CN); ZHANG, Haoliang, Chengdu, Sichuan 611130 (CN); WANG, Haodong, Chengdu, Sichuan 611130 (CN); ZHENG, Dengyu, Chengdu, Sichuan 611130 (CN); WANG, Yaoling, Chengdu, Sichuan 611130 (CN); LIU, Man, Chengdu, Sichuan 611130 (CN); WANG, Zheng, Chengdu, Sichuan 611130 (CN); TANG, Yingde, Chengdu, Sichuan 611130 (CN); GUI, Naicheng, Chengdu, Sichuan 611130 (CN); TANG, Pingming, Chengdu, Sichuan 611130 (CN); ZHANG, Chen, Chengdu, Sichuan 611130 (CN); YAN, Pangke, Chengdu, Sichuan 611130 (CN)
(74) Representative: AWA Denmark A/S
(86) International application number: PCT/CN2024/100207
(87) International publication number: WO 2024/260386

(57) **Abstract**

A CHRM4 receptor agonist and the use thereof. Disclosed in the present invention are a compound as represented by formula (I), or a stereoisomer, a deuterated substance, a solvate, an eutectic or a pharmaceutically acceptable salt thereof, and a pharmaceutical composition thereof, and the use thereof in the preparation of a drug for treating/preventing CHRM4-mediated diseases, wherein each group in formula (I) is as defined in the description.

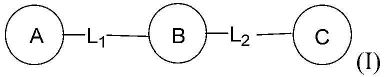

## Description

### Technical Field

The present invention belongs to the field of pharmaceuticals, and particularly relates to a small molecule compound having CHRM4 receptor agonist activity, or a stereoisomer, a deuterated substance, a solvate, a co-crystal or a pharmaceutically acceptable salt thereof, and the use thereof in the preparation of a drug for treating related diseases.

### Background Art

mAChRs are widely distributed in the human body, and based on their locations and receptor subtypes, they mediate a variety of physiological functions in the cardiovascular, renal, gastrointestinal, pulmonary, central, and peripheral nervous systems. mAChRs are generally expressed in various regions of the brain, and their functions are associated with a wide range of brain circuits that regulate neuronal excitability, synaptic plasticity, and acetylcholine release. All five mAChRs are expressed in both neurons and glial cells of the brain, but their receptor abundance varies across regions. The muscarinic acetylcholine receptor M4 (also known as muscarinic 4 or CHRM4) is a protein encoded by the CHRM4 gene in humans. The M4 receptor is predominantly expressed in the brain. The key brain regions where M4 receptor expression occurs are the striatum, cortex, and hippocampus, with the highest expression (approximately 46%) in the striatum, where M4 is the major muscarinic subtype. M4 is occasionally expressed in the periphery (e.g., testis, skin, and colon).

### Summary of the Invention

The present invention provides a small molecule compound having CHRM4 activity, or a stereoisomer, a deuterated substance, a solvate, a co-crystal or a pharmaceutically acceptable salt thereof, wherein the compound has a structure of formula (I), (I-1), (I-1a), (I-2), (1-3), (1-4), (1-5), (1-6), (1-7), (I-8), (I-1b), or (II), 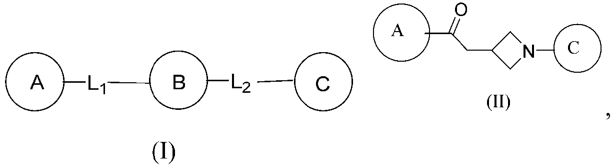 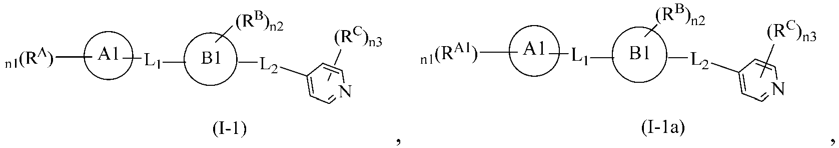 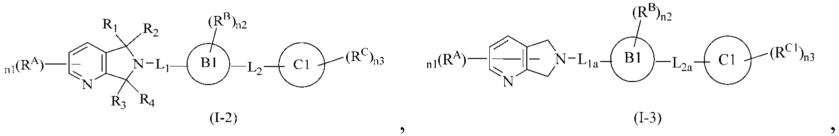 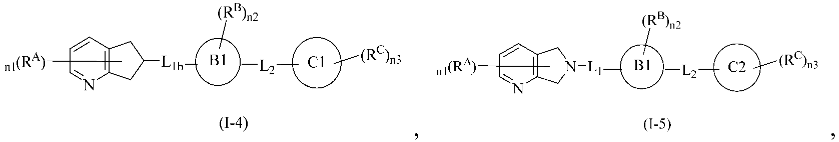 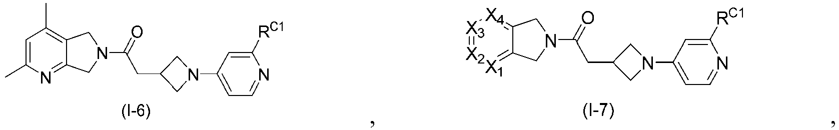 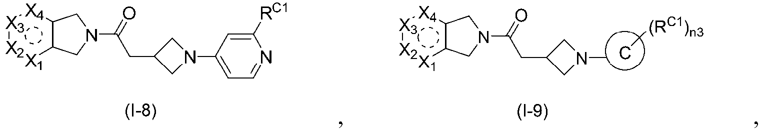 or 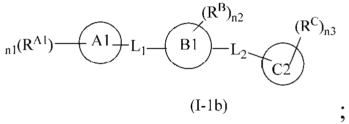 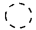 indicates that the ring formed by X₁-X₄ contains one or more double bonds;
A is selected from 3- to 20-membered heterocycloalkyl or 5- to 20-membered heteroaryl; the heterocycloalkyl and heteroaryl contain 1-5 heteroatoms selected from N, O or S; and the heterocyclyl and heteroaryl are optionally substituted with 0-5 R^{A}; in some embodiments, A is selected from 5- to 6-membered monocyclic heteroaryl, 6- to 10-membered bicyclic heterocycloalkyl, 9- to 10-membered bicyclic heteroaryl, 10- to 14-membered tricyclic heterocycloalkyl, or 10- to 14-membered tricyclic heteroaryl, and the heterocycloalkyl and heteroaryl are optionally substituted with 1-5 R^{A}; in some embodiments, A is selected from 5- to 6-membered monocyclic heteroaryl, 6- to 10-membered bicyclic heteroaryl, 6- to 10-membered bicyclic heterocycloalkyl, 10- to 14-membered tricyclic heteroaryl, or 10- to 14-membered tricyclic heterocycloalkyl, and the heterocycloalkyl and heteroaryl are optionally substituted with 1-5 R^{A}; in some embodiments, A is selected from 5- to 6-membered monocyclic heteroaryl, 6- to 10-membered bicyclic heterocycloalkyl, or 10- to 14-membered tricyclic heterocycloalkyl, and the heterocycloalkyl and heteroaryl are optionally substituted with 1-5 R^{A}; in some embodiments, A is selected from 5-membered heteroaryl, 6-membered heteroaryl, 5- to 6-membered heteroaryl fused 5- to 6-membered heteroaryl, 5- to 6-membered heteroaryl fused 5- to 7-membered heterocycloalkyl, 5- to 6-membered heteroaryl fused 5- to 6-membered carbocyclyl, 10- to 14-membered tricyclic heteroaryl, or 10- to 14-membered tricyclic heterocycloalkyl; and the heteroaryl, heterocycloalkyl, and carbocyclyl are optionally substituted with 1-5 R^{A}; in some embodiments, A is selected from one of the groups formed by the following structures optionally substituted with 1-5 R^{A}: 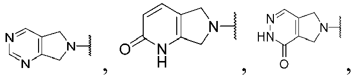 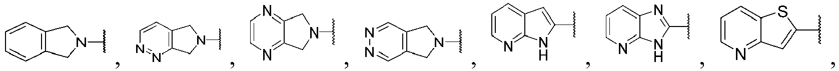 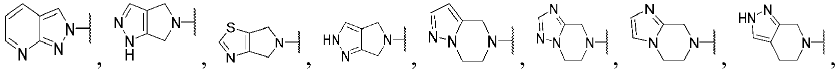 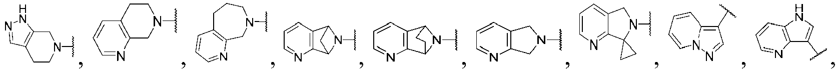 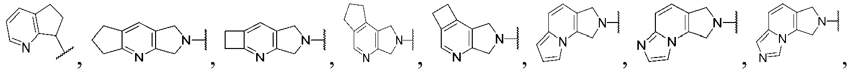 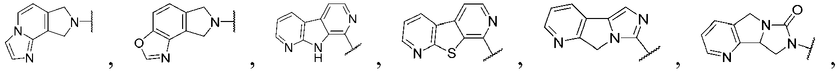 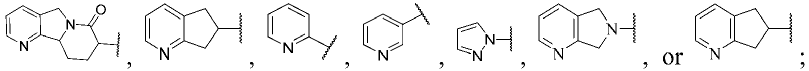 in some embodiments, ring A is selected from the following structures optionally further substituted with 1, 2 or 3 R^{A}: 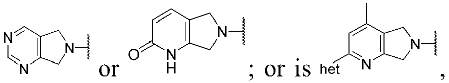 wherein het is 5-membered heteroaryl, and the heteroaryl is optionally substituted with 1 and 2 groups selected from halogen, C₁₋₂ alkyl, halogenated C₁₋₂ alkyl, or deuterated C₁₋₂ alkyl; in some embodiments, ring A is selected from 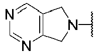 or 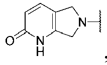 , and the ring A is optionally further substituted with 1, 2 or 3 R^{A}; in some embodiments, ring A is selected from 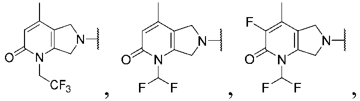 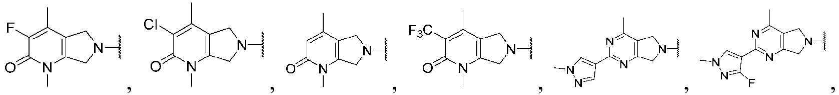 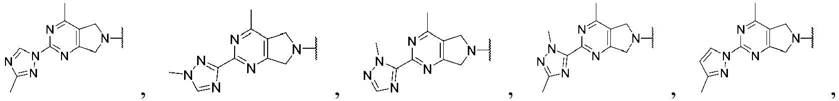 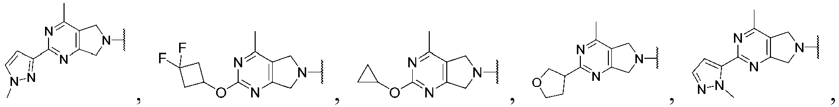 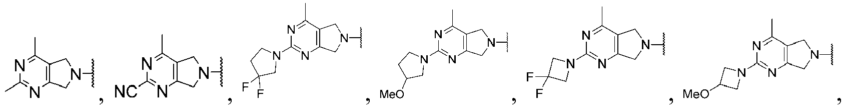 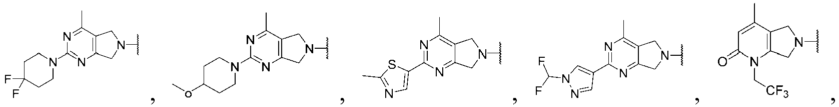 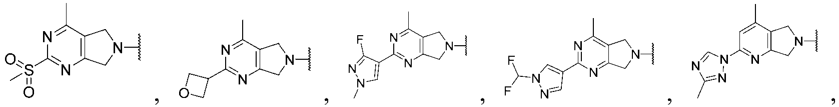 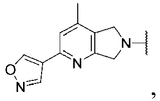 or 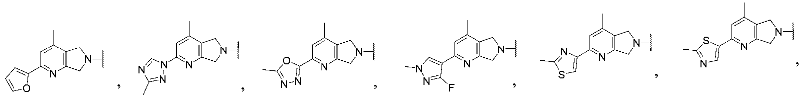 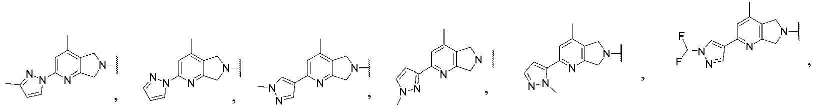 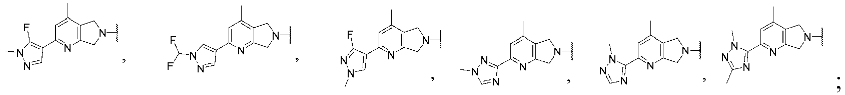 in some embodiments, ring A is selected from 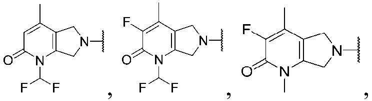 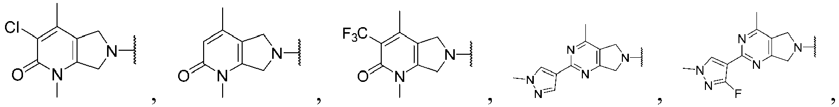 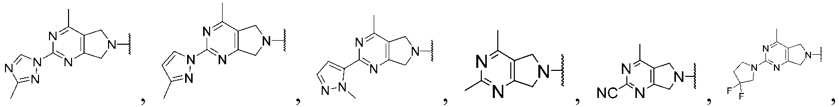 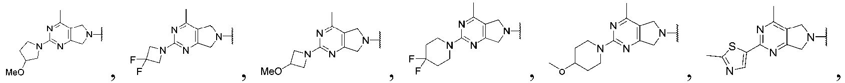 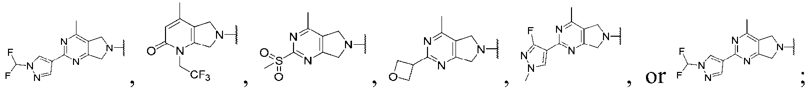 in some embodiments, ring A is 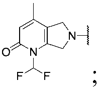
A1 is selected from the following groups: 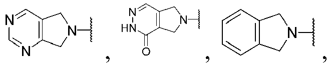 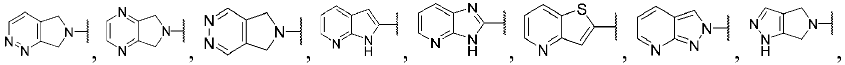 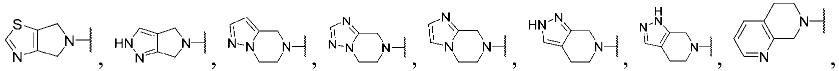 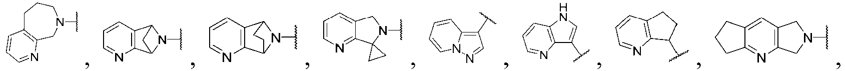 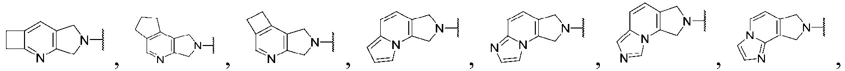 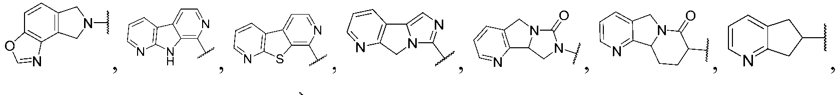 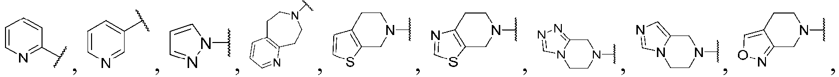 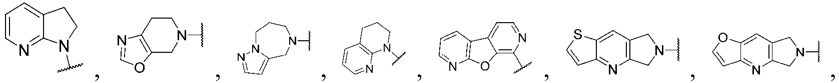 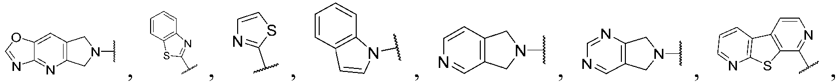 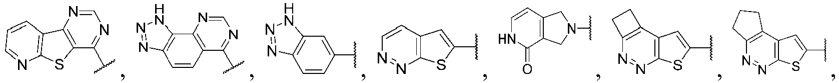 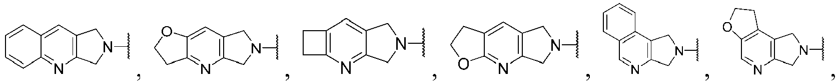 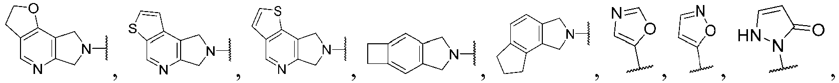 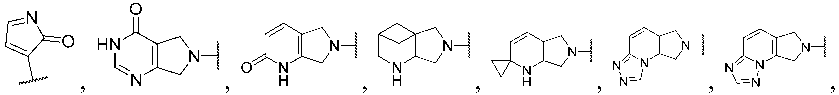 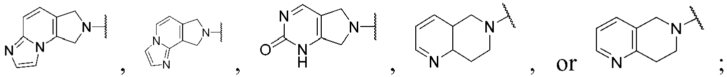 in some embodiments, A1 is selected from the following groups: 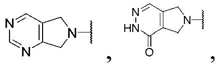 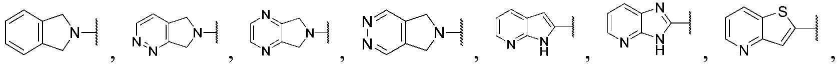 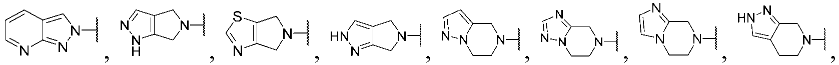 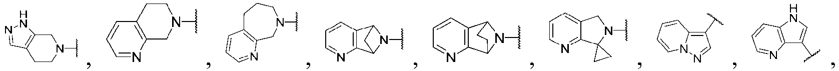 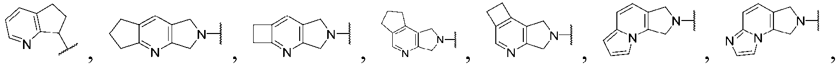 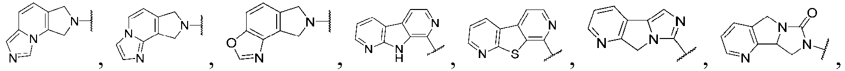 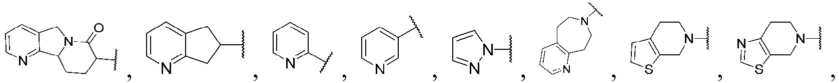 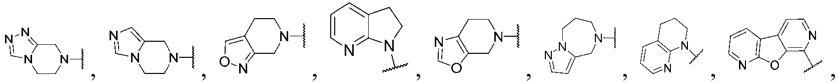 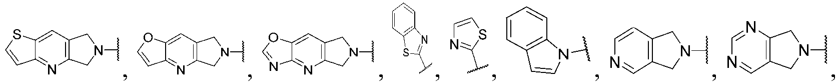 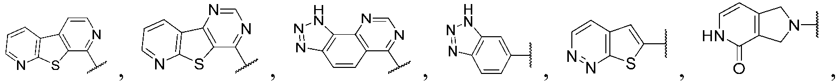 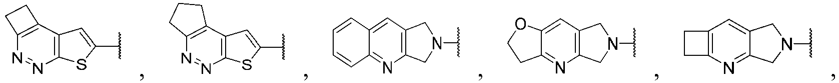 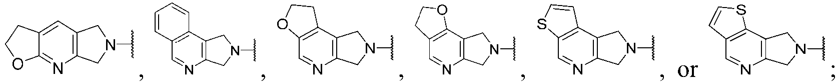 or A1 is selected from the following groups: 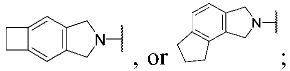 or A1 is selected from the following groups: 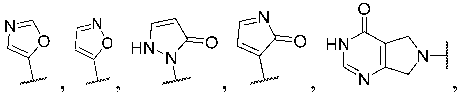 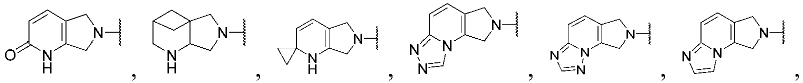 or 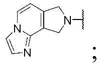 in some embodiments, A1 is selected from the following groups: 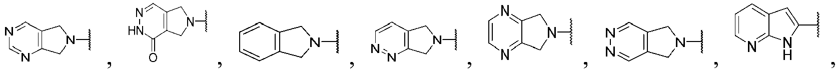 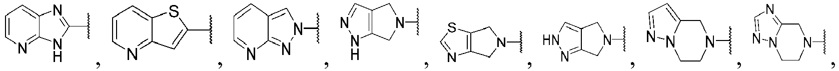 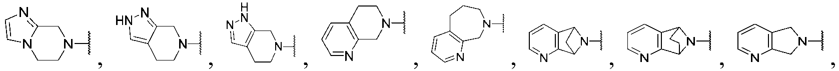 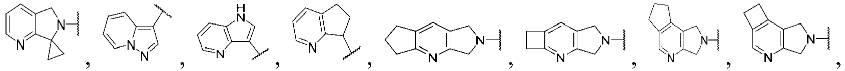 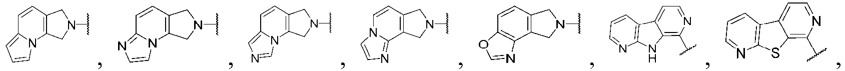 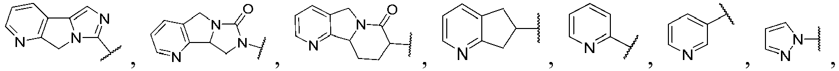 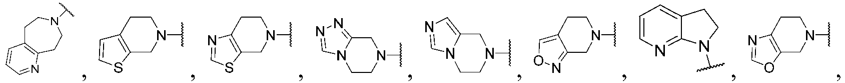 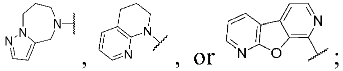 or A1 is selected from the following groups: 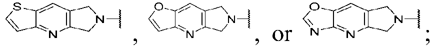 or A1 is selected from the following groups: 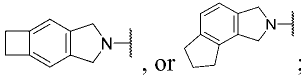 ; or A1 is selected from the following groups: 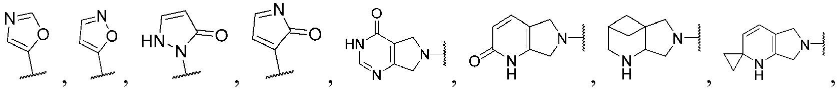 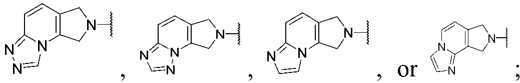 or A1 is selected from the following groups: 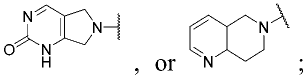 in some embodiments, A1 is selected from the following groups: 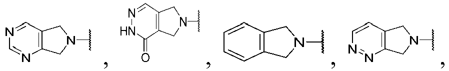 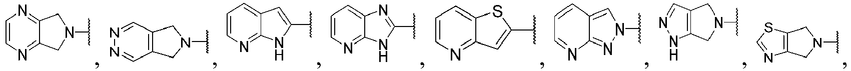 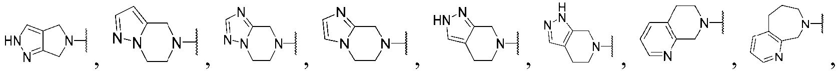 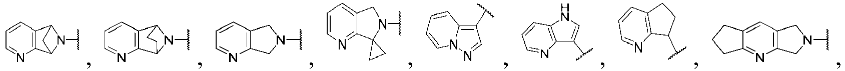 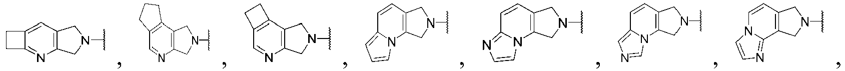 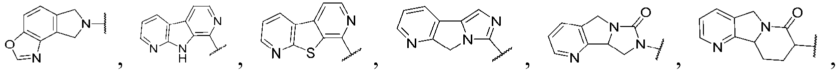 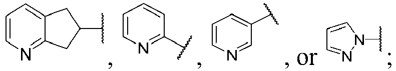
B is selected from a bond, C₃₋₁₀ cycloalkyl, C₆₋₁₂ aryl, 3- to 12-membered heterocycloalkyl or 5- to 12-membered heteroaryl; the heterocycloalkyl and heteroaryl contain 1-5 heteroatoms selected from N, O or S; and the cycloalkyl, aryl, heterocycloalkyl and heteroaryl are optionally substituted with 0-5 R^{B}; in some embodiments, B is selected from a bond, 4- to 7-membered monocyclic heterocycloalkyl, C₃₋₈ cycloalkyl, 5- to 6-membered heteroaryl, 5- to 6-membered heteroaryl fused 5- to 6-membered heterocycloalkyl, 5- to 12-membered spiro heterocycloalkyl, 3- to 6-membered cycloalkyl fused 5- to 6-membered heterocycloalkyl, or benzo 5- to 6-membered heterocycloalkyl, and the heterocycloalkyl, heteroaryl, cycloalkyl and aryl are optionally substituted with 1-5 R^{B}; in some embodiments, B is selected from a bond, 4- to 7-membered monocyclic heterocycloalkyl, 3- to 8-membered cycloalkyl, 5- to 6-membered heteroaryl, 5- to 6-membered heteroaryl fused 5- to 6-membered heterocycloalkyl, 5- to 12-membered spiro heterocycloalkyl, 5- to 6-membered cycloalkyl fused 5-to 6-membered heterocycloalkyl, or benzo 5- to 6-membered heterocycloalkyl, and the heterocycloalkyl, heteroaryl, cycloalkyl and aryl are optionally substituted with 1-5 R^{B};
in some embodiments, B is selected from the following groups optionally substituted with 1-5 R^{B}: a bond, 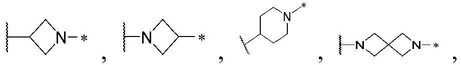 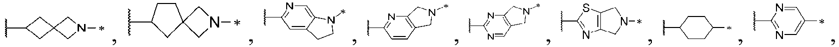 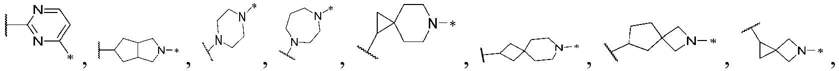 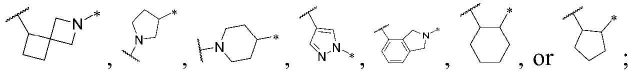 B1 is selected from the following groups: a bond, 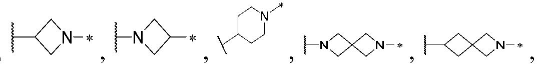 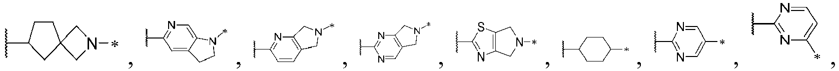 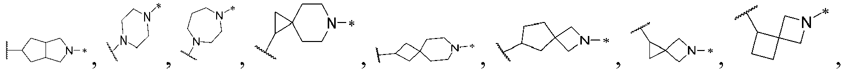 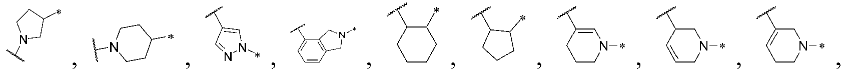 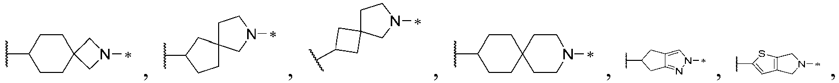 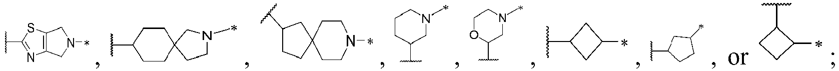 or B1 is the following group: 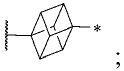 or B1 is selected from the following groups: 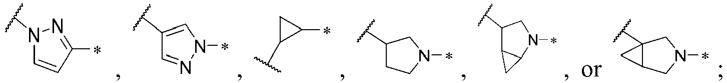 in some embodiments, B1 is selected from the following groups: a bond, 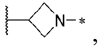 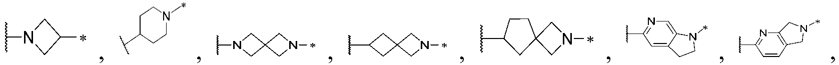 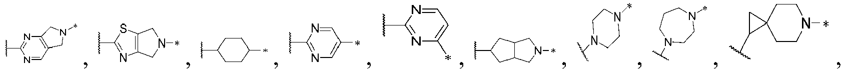 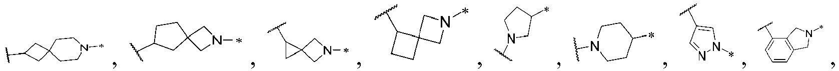 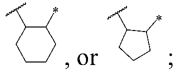
wherein the " 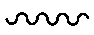 " end is connected to L₁ or L₁ₐ or L_{1b}, and the "*" end is connected to L₂ or L₂ₐ;
C is selected from C₃₋₁₂ cycloalkyl, C₆₋₁₀ aryl, 3- to 14-membered heterocycloalkyl or 5- to 14-membered heteroaryl; the heterocycloalkyl and heteroaryl contain 1-5 heteroatoms selected from N, O or S; and the cycloalkyl, heterocycloalkyl, aryl and heteroaryl are optionally substituted with 0-5 R^{C}; in some embodiments, C is selected from 5- to 6-membered monocyclic heteroaryl, phenyl, phenyl fused 4- to 6-membered carbocyclyl, phenyl fused 4- to 6-membered heterocycloalkyl, 5- to 6-membered heterocycloalkyl fused 5- to 6-membered heteroaryl, 5- to 6-membered heteroaryl fused 5- to 6-membered carbocyclyl, 8- to 12-membered tricyclic cycloalkyl, or 8- to 14-membered tricyclic heterocyclyl; the heteroaryl, heterocycloalkyl and heterocyclyl contain 1-4 heteroatoms selected from N, O or S; and the phenyl, heterocycloalkyl and carbocyclyl are optionally substituted with 1-5 R^{C}; in some embodiments, C is selected from 5- to 6-membered monocyclic heteroaryl, phenyl, phenyl fused 4- to 6-membered carbocyclyl, 5- to 6-membered heterocycloalkyl fused 5- to 6-membered heteroaryl, 5- to 6-membered heteroaryl fused 5- to 6-membered carbocyclyl, 8- to 12-membered tricyclic cycloalkyl, or 8- to 14-membered tricyclic heterocyclyl; the heteroaryl, heterocycloalkyl and heterocyclyl contain 1-4 heteroatoms selected from N, O or S; and the phenyl, heterocycloalkyl and carbocyclyl are optionally substituted with 1-5 R^{C}; in some embodiments, C is selected from C₃₋₁₂ cycloalkyl, C₆₋₁₀ aryl, 3- to 12-membered heterocycloalkyl or 5-to 12-membered heteroaryl; the heterocycloalkyl and heteroaryl contain 1-5 heteroatoms selected from N, O or S; and the cycloalkyl, heterocycloalkyl, aryl and heteroaryl are optionally substituted with 0-5 R^{C}; in some embodiments, C is selected from 5- to 6-membered monocyclic heteroaryl, phenyl fused 4- to 6-membered carbocyclyl, 5- to 6-membered heterocycloalkyl fused 5- to 6-membered heteroaryl, 5- to 6-membered heteroaryl fused 5- to 6-membered carbocyclyl, or 8- to 12-membered tricyclic cycloalkyl; the heterocycloalkyl contains 1-3 heteroatoms selected from N, O or S; and the phenyl, heterocycloalkyl and carbocyclyl are optionally substituted with 1-5 R^{C}; in some embodiments, C is selected from the following groups optionally substituted with 1-4 R^{C}: 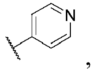 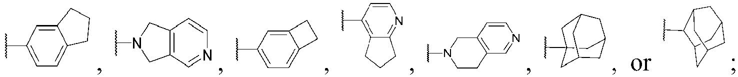 in some embodiments, ring C is selected from 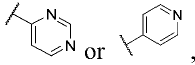 and the ring C is optionally further substituted with 1, 2 or 3 R^{C}; in some embodiments, C is the following group optionally substituted with 1-4 R^{C}: 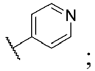
C1 is the following group: 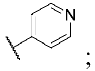
C2 is selected from the following groups: 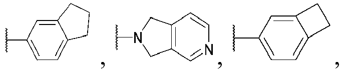 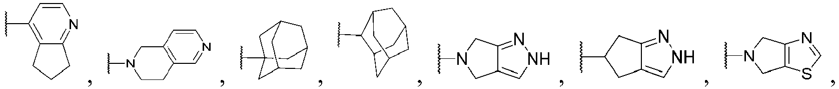 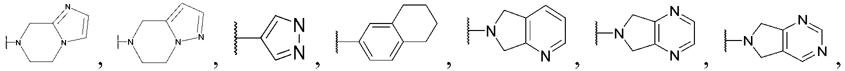 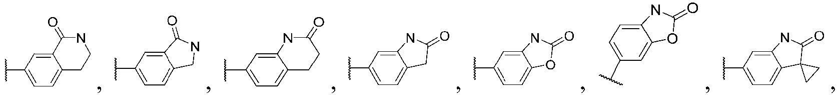 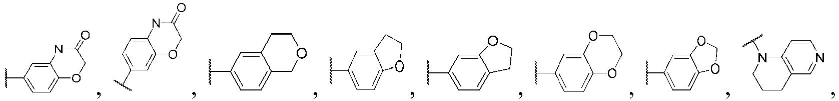 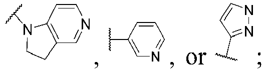 or C2 is selected from the following groups: 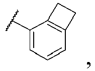 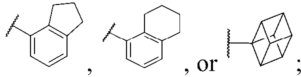 or C2 is selected from the following groups: 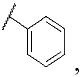 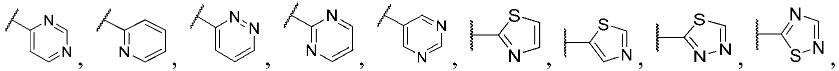 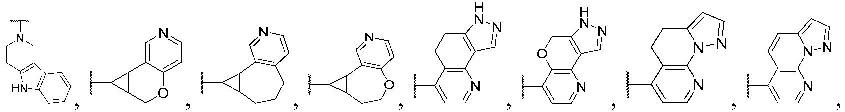 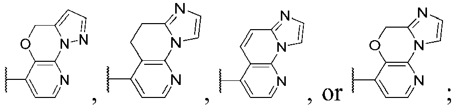 or C2 is selected from the following groups: 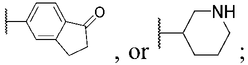 or C2 is the following group: 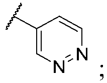 or C2 is the following group: 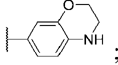 ; in some embodiments, C2 is selected from the following groups: 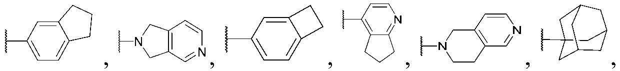 or 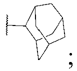
each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, - NRₐCO-C₃₋₆ cycloalkyl, 4- to 12-membered heterocycloalkyl, -O-C₃₋₆ cycloalkyl, - C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4-to 12-membered heterocycloalkyl), -C₁₋₄ alkyl-OC(=O)-NRₐₐ-C₃₋₆ cycloalkyl, - NRₐₐR_{bb}, -SO₂-C₁₋₄ alkyl, -C₃₋₆ cycloalkyl, -NRₐₐ-C₃₋₆ cycloalkyl, 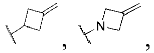 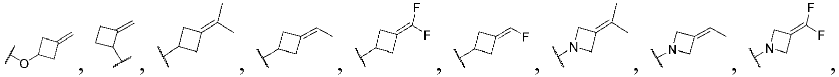 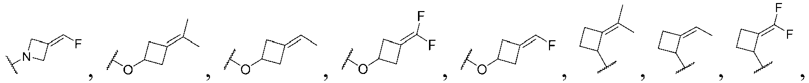 or 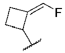 , and the alkyl, alkenyl, alkynyl, heteroaryl, cycloalkyl and heterocycloalkyl are optionally further substituted with 1-5 groups selected from halogen, OH, NH₂, CN, C₁₋₄ alkoxy, -O-halogenated C₁₋₄ alkyl, C₁₋₄ alkyl, -C₁₋₄ alkylhydroxy, -C(=O) C₁₋₄ alkyl, or halogenated C₁₋₄ alkyl; in some embodiments, each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, - C₃₋₆ cycloalkyl, -NRₐₐ-C₃₋₆ cycloalkyl, 5- to 6-membered heteroaryl, 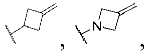 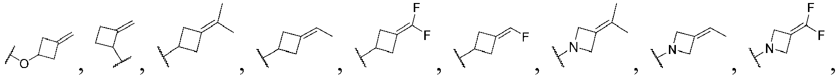 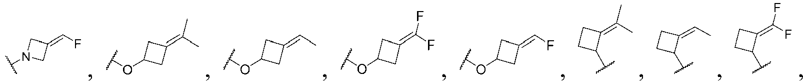 or 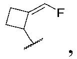 and the alkyl, alkenyl, alkynyl, heteroaryl, cycloalkyl and heterocycloalkyl are optionally further substituted with 1-5 groups selected from halogen, OH, NH₂, CN, C₁₋₄ alkoxy, -O-halogenated C₁₋₄ alkyl, C₁₋₄ alkyl, -C₁₋₄ alkylhydroxy, -C(=O) C₁₋₄ alkyl, or halogenated C₁₋₄ alkyl; in some embodiments, each R^{A} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₄ alkyl-(5- to 6-membered heteroaryl), - O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, -SO₂-C₁₋₄ alkyl, -C₃₋₆ cycloalkyl, - NRₐₐ-C₃₋₆ cycloalkyl, 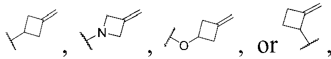 , and the alkyl, heteroaryl, cycloalkyl and heterocycloalkyl are optionally further substituted with 1-5 groups selected from halogen, OH, NH₂, CN, C₁₋₄ alkyl, C₁₋₂ alkoxy, -C₁₋₂ alkylhydroxy, - C(=O) C₁₋₂ alkyl, or halogenated C₁₋₂ alkyl; in some embodiments, each R^{A} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₄ alkyl-(5- to 6-membered heteroaryl), - O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 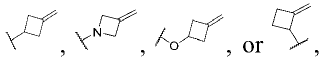 and the alkyl, heteroaryl, cycloalkyl and heterocycloalkyl are optionally further substituted with 1-5 groups selected from halogen, OH, NH₂, CN, C₁₋₄ alkyl, C₁₋₂ alkoxy, -C₁₋₂ alkylhydroxy, -C(=O) C₁₋₂ alkyl, or halogenated C₁₋₂ alkyl; in some embodiments, each R^{A} is independently selected from halogen, =O, C₁₋₂ alkyl, -CF₃, 5- to 6-membered heteroaryl, -NRaCO-C₃₋₆ cycloalkyl, 4- to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), - C₁₋₂ alkyl-O-(4- to 12-membered heterocycloalkyl), -C₁₋₂ alkyl-O-C₃₋₆ cycloalkyl, - C₁₋₂ alkyl-OC(=O)-(4- to 12-membered heterocycloalkyl), -C₁₋₂ alkyl-OC(=O)-NRₐₐ-C₃₋₆ cycloalkyl, -NRₐₐR_{bb}, -SO₂-C₁₋₂ alkyl, -C₃₋₆ cycloalkyl, -NRₐₐ-C₃₋₆ cycloalkyl, 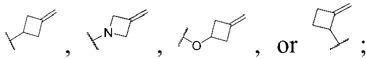 the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, C₁₋₂ alkyl, -C₁₋₂ alkylhydroxy, halogenated C₁₋₂ alkyl, -C(=O)C₁₋₂ alkyl, or C₁₋₂ alkoxy; in some embodiments, each R^{A} is independently selected from halogen, =O, C₁₋₂ alkyl, 5-to 6-membered heteroaryl, -NRaCO-C₃₋₆ cycloalkyl, 4- to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), - C₁₋₂ alkyl-O-(4- to 12-membered heterocycloalkyl), -C₁₋₂ alkyl-O-C₃₋₆ cycloalkyl, - C₁₋₂ alkyl-OC(=O)-(4- to 12-membered heterocycloalkyl), -C₁₋₂ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 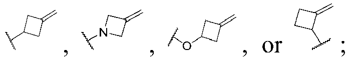 the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, C₁₋₂ alkyl, -C₁₋₂ alkylhydroxy, halogenated C₁₋₂ alkyl, -C(=O) C₁₋₂ alkyl, or C₁₋₂ alkoxy; in some embodiments, each R^{A} is independently selected from F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), - C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), - C₁₋₂ alkyl-OC(=O)-NH-C₃₋₆ cycloalkyl, -NHRₐₐ, cyclopropyl, cyclobutyl, -NH-cyclopropyl, -NH-cyclobutyl, 5- to 6-membered heteroaryl, 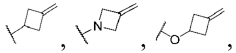 or 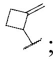 the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, ethyl, -CHF₂, -CH₂F, -CF₃, -C(=O) CH₃, -O-CH₃, or -O-CH₂CH₃; in some embodiments, each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 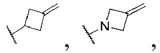 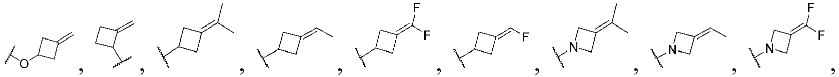 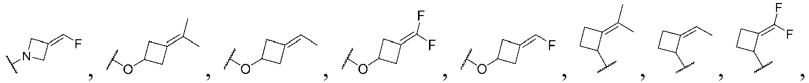 or 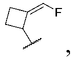 and the alkyl, alkenyl, alkynyl, heteroaryl, cycloalkyl and heterocycloalkyl are optionally further substituted with 1-5 groups selected from halogen, OH, NH₂, CN, C₁₋₄ alkoxy, -O-halogenated C₁₋₄ alkyl, C₁₋₄ alkyl, or -C₁₋₄ alkylhydroxy; in some embodiments, each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 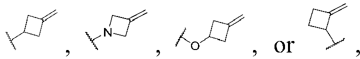 and the alkyl, alkenyl, alkynyl, heteroaryl, cycloalkyl and heterocycloalkyl are optionally further substituted with 1-5 groups selected from halogen, OH, NH₂, CN, C₁₋₄ alkoxy, -O-halogenated C₁₋₄ alkyl, C₁₋₄ alkyl, or -C₁₋₄ alkylhydroxy; in some embodiments, each R^{A} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₄ alkyl-(5- to 6-membered heteroaryl), - O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 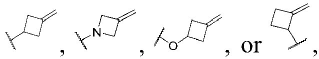 and the alkyl, heteroaryl, cycloalkyl and heterocycloalkyl are optionally further substituted with 1-5 groups selected from halogen, OH, NH₂, CN, C₁₋₄ alkyl, C₁₋₂ alkoxy, or - C₁₋₂ alkylhydroxy; in some embodiments, each R^{A} is independently selected from halogen, =O, C₁₋₂ alkyl, -CF₃, 5- to 6-membered heteroaryl, -NRaCO-C₃₋₆ cycloalkyl, 4- to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), -C₁₋₂ alkyl-O-(4- to 12-membered heterocycloalkyl), -C₁₋₂ alkyl-O-C₃₋₆ cycloalkyl, -C₁₋₂ alkyl-OC(=O)-(4- to 12-membered heterocycloalkyl), -C₁₋₂ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 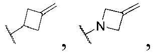 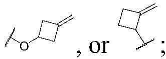 the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, C₁₋₂ alkyl, -C₁₋₂ alkylhydroxy, halogenated C₁₋₂ alkyl, - C(=O) C₁₋₂ alkyl, or C₁₋₂ alkoxy; in some embodiments, each R^{A} is independently selected from halogen, =O, C₁₋₂ alkyl, 5- to 6-membered heteroaryl, -NRaCO-C₃₋₆ cycloalkyl, 4- to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), -C₁₋₂ alkyl-O-(4- to 12-membered heterocycloalkyl), -C₁₋₂ alkyl-O-C₃₋₆ cycloalkyl, -C₁₋₂ alkyl-OC(=O)-(4- to 12-membered heterocycloalkyl), -C₁₋₂ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 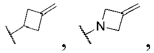 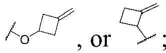 ; the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, C₁₋₂ alkyl, or -C₁₋₂ alkylhydroxy; in some embodiments, each R^{A} is independently selected from F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), - C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), - C₁₋₂ alkyl-OC(=O)-NH-C₃₋₆ cycloalkyl, -NHRₐₐ, -SO₂CH₃, cyclopropyl, cyclobutyl, -NH-cyclopropyl, -NH-cyclobutyl, oxolanyl, 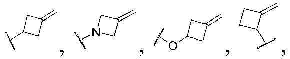 -CF₃, or 7- to 10-membered bicyclic heterocycloalkyl; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, ethyl, - CHF₂, -CH₂F, -CF₃, -C(=O) CH₃, -O-CH₃, or -O-CH₂CH₃; in some embodiments, each R^{A} is independently selected from F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), - C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), - C₁₋₂ alkyl-OC(=O)-NH-C₃₋₆ cycloalkyl, -NHRₐₐ, cyclopropyl, cyclobutyl, -NH-cyclopropyl, -NH-cyclobutyl, 5- to 6-membered heteroaryl, 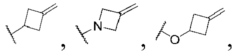 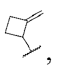 -CF₃, or 7- to 10-membered bicyclic heterocycloalkyl; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, ethyl, -CHF₂, -CH₂F, -CF₃, -C(=O) CH₃, -O-CH₃, or -O-CH₂CH₃; in some embodiments, each R^{A} is independently selected from F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), - C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), - C₁₋₂ alkyl-OC(=O)-NH-C₃₋₆ cycloalkyl, -NHRₐₐ, cyclopropyl, cyclobutyl, -NH-cyclopropyl, -NH-cyclobutyl, 5- to 6-membered heteroaryl, 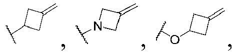 or 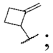 the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, or ethyl; in some embodiments, each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, -NRₐCO-C₃₋₆ cycloalkyl, 4- to 12-membered heterocycloalkyl, -O-C₃₋₆ cycloalkyl, -C₁₋₄ alkyl-(5-to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, or - NRₐₐR_{bb}, and the alkyl, alkenyl, alkynyl, heteroaryl, cycloalkyl and heterocycloalkyl are optionally further substituted with 1-5 groups selected from halogen, OH, NH₂, CN, C₁₋₄ alkoxy, -O-halogenated C₁₋₄ alkyl, or C₁₋₄ alkyl; in some embodiments, each R^{A} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₄ alkyl-(5-to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, or - NRₐₐR_{bb}, and the alkyl, heteroaryl, cycloalkyl and heterocycloalkyl are optionally further substituted with 1-5 groups selected from halogen, OH, NH₂, CN, C₁₋₄ alkyl, or C₁₋₂ alkoxy; in some embodiments, each R^{A} is independently selected from halogen, =O, C₁₋₂ alkyl, 5- to 6-membered heteroaryl, -NRaCO-C₃₋₆ cycloalkyl, 4-to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), -C₁₋₂ alkyl-O-(4- to 12-membered heterocycloalkyl), -C₁₋₂ alkyl-O-C₃₋₆ cycloalkyl, -C₁₋₂ alkyl-OC(=O)-(4- to 12-membered heterocycloalkyl), -C₁₋₂ alkyl-OC(=O)-NRₐₐ-C₃₋₆ cycloalkyl, or -NRₐₐR_{bb}; the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, or C₁₋₂ alkyl; in some embodiments, each R^{A} is independently selected from F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), - C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), - C₁₋₂ alkyl-OC(=O)-NH-C₃₋₆ cycloalkyl, or -NHRₐₐ; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, or ethyl; in some embodiments, each R^{A} is independently selected from halogen, =O, CN, OH, COOH, C₁₋₄ alkyl, C₁₋₄ alkoxy, C₂₋₆ alkenyl, C₂₋₆ alkynyl, 5-to 12-membered heteroaryl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), or -NRₐₐR_{bb}, and the alkyl, alkenyl, alkynyl, heteroaryl, cycloalkyl and heterocycloalkyl are optionally further substituted with 1-4 groups selected from halogen, OH, NH₂, CN, C₁₋₄ alkoxy, -O-halogenated C₁₋₄ alkyl, or C₁₋₄ alkyl; in some embodiments, each R^{A} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₄ alkyl-(5-to 6-membered heteroaryl), or -NRₐₐR_{bb}, and the alkyl, heteroaryl, cycloalkyl and heterocycloalkyl are optionally further substituted with 1-5 groups selected from halogen, OH, NH₂, CN, C₁₋₄ alkyl, or C₁₋₂ alkoxy; in some embodiments, each R^{A} is independently selected from halogen, =O, C₁₋₂ alkyl, 5- to 6-membered heteroaryl, -NRaCO-C₃₋₆ cycloalkyl, 4- to 12-membered heterocycloalkyl, -C₁₋₂ alkyl-(5- to 6-membered heteroaryl), or -NRₐₐR_{bb}; the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, or C₁₋₂ alkyl; in some embodiments, each R^{A} is independently selected from F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), or -NHRₐₐ; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, or ethyl; in some embodiments, each R^{A} is independently selected from halogen, =O, CN, OH, COOH, C₁₋₄ alkyl, C₂₋₆ alkenyl, C₂₋₆ alkynyl, 5- to 12-membered heteroaryl, -NRaCO-C₃₋₆ cycloalkyl, 4- to 12-membered heterocycloalkyl, or -O-C₃₋₆ cycloalkyl, and the alkyl, alkenyl, alkynyl, heteroaryl, cycloalkyl and heterocycloalkyl are optionally further substituted with 1-4 groups selected from halogen, OH, NH₂, CN, -O-halogenated C₁₋₄ alkyl, or C₁₋₄ alkyl; in some embodiments, each R^{A} is independently selected from =O, CN, methyl, ethyl, -NHCO-C₃₋₆ cycloalkyl, 4- to 6-membered monocyclic heterocycloalkyl, 5-membered heteroaryl, or 6-membered heteroaryl; the methyl and ethyl are optionally further substituted with 1-4 groups selected from NH₂, CN, methyl, or ethyl; and the heteroaryl, cycloalkyl and heterocycloalkyl are optionally further substituted with 1-4 groups selected from F, Cl, OH, NH₂, CN, methyl, or ethyl; in some embodiments, each R^{A} is independently selected from =O, C₁₋₂ alkyl, 5- to 6-membered heteroaryl, -NRaCO-C₃₋₆ cycloalkyl, or 4- to 12-membered heterocycloalkyl; the alkyl is optionally further substituted with 1-4 groups selected from NH₂, CN, or C₁₋₂ alkyl; and the heteroaryl, cycloalkyl and heterocycloalkyl are optionally further substituted with 1-4 groups selected from halogen, OH, NH₂, CN, or C₁₋₂ alkyl; in some embodiments, each R^{A} is independently selected from =O, methyl, ethyl, -NHCO-C₃₋₆ cycloalkyl, 4- to 6-membered monocyclic heterocycloalkyl, 5-membered heteroaryl, or 6-membered heteroaryl; the methyl and ethyl are optionally further substituted with 1-4 groups selected from NH₂, CN, methyl, or ethyl; and the heteroaryl, cycloalkyl and heterocycloalkyl are optionally further substituted with 1-4 groups selected from F, Cl, OH, NH₂, CN, methyl, or ethyl; in some embodiments, each R^{A} is independently selected from halogen, =O, CN, OH, COOH, C₁₋₄ alkyl, C₂₋₆ alkenyl, C₂₋₆ alkynyl, or 5- to 12-membered heteroaryl, and the alkyl, alkenyl, alkynyl and heteroaryl are optionally further substituted with 1-4 groups selected from halogen, OH, NH₂, CN, or -O-halogenated C₁₋₄ alkyl; in some embodiments, each R^{A} is independently selected from halogen, =O, CN, OH, COOH, C₁₋₄ alkyl, or 5- to 6-membered heteroaryl, and the alkyl and heteroaryl are optionally further substituted with 1-5 groups selected from halogen, OH, NH₂, CN, or C₁₋₄ alkyl; in some embodiments, each R^{A} is independently selected from halogen, =O, C₁₋₂ alkyl, or 5- to 6-membered heteroaryl, and the alkyl is optionally further substituted with 1-4 groups selected from halogen, OH, NH₂, CN, or C₁₋₂ alkyl; in some embodiments, each R^{A} is independently selected from F, Cl, =O, methyl, ethyl, 5-membered heteroaryl, or 6-membered heteroaryl, and the methyl, ethyl, 5-membered heteroaryl and 6-membered heteroaryl are optionally further substituted with 1-4 groups selected from F, Cl, methyl, or ethyl; in some embodiments, each R^{A} is independently selected from halogen, CN, C₁₋₄ alkyl, C₁₋₄ alkoxy, halogenated C₁₋₄ alkyl, -O-C₃₋₆ cycloalkyl, 4- to 6-membered heterocycloalkyl, 5-to 6-membered heteroaryl, or -SO₂-C₁₋₂ alkyl, and the heteroaryl, cycloalkyl and heterocycloalkyl are optionally further substituted with 1-2 groups selected from halogen, OH, NH₂, CN, C₁₋₂ alkyl, C₁₋₂ alkoxy, -C(=O)C₁₋₂ alkyl, or halogenated C₁₋₂ alkyl;
alternatively, two R^{A}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
in some embodiments, two R^{A}, together with the atoms to which they are connected, form C₃₋₆ cycloalkyl or 4- to 10-membered heterocycloalkyl, and the cycloalkyl and heterocycloalkyl are optionally further substituted with 1-4 groups selected from halogen, OH, NH₂, CN, or C₁₋₄ alkyl; in some embodiments, two R^{A}, together with the atoms to which they are connected, form C₃₋₆ carbocyclyl or 4- to 6-membered heterocyclyl, and the carbocyclyl and heterocyclyl are optionally further substituted with 1-4 groups selected from halogen, OH, NH₂, CN, or C₁₋₂ alkyl;
each R^{A1} is independently selected from halogen, =O, CN, OH, COOH, C₁₋₄ alkyl, C₁₋₄ alkoxy, 5- to 6-membered heteroaryl, -SO₂-C₁₋₄ alkyl, -NRaCO-C₃₋₆ cycloalkyl, 4- to 12-membered heterocycloalkyl, -O-C₃₋₆ cycloalkyl, -C₁₋₂ alkyl-(5-to 6-membered heteroaryl), or -NRₐₐR_{bb}, and the alkyl, heteroaryl, cycloalkyl and heterocycloalkyl are optionally further substituted with 1-5 groups selected from halogen, OH, NH₂, CN, C₁₋₄ alkyl, halogenated C₁₋₄ alkyl, or C₁₋₄ alkoxy; in some embodiments, each R^{A1} is independently selected from F, Cl, =O, CN, OH, COOH, -SO₂CH₃, methyl, ethyl, -O-C₃₋₆ cycloalkyl, -NHRₐₐ, 5-membered heteroaryl, or 4-to 6-membered heterocycloalkyl, and the methyl, ethyl, cycloalkyl, 5-membered heteroaryl and 4- to 6-membered heterocycloalkyl are optionally further substituted with 1-5 groups selected from F, Cl, OH, NH₂, CN, methyl, ethyl, -CHF₂, -CH₂F, - CF₃, or methoxy; in some embodiments, each R^{A1} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₂ alkyl-(5- to 6-membered heteroaryl), or -NRₐₐR_{bb}, and the alkyl, heteroaryl, cycloalkyl and heterocycloalkyl are optionally further substituted with 1-5 groups selected from halogen, OH, NH₂, CN, C₁₋₄ alkyl, or C₁₋₄ alkoxy; in some embodiments, each R^{A1} is independently selected from F, Cl, =O, CN, OH, COOH, methyl, ethyl, -O-C₃₋₆ cycloalkyl, -NHRₐₐ, 5-membered heteroaryl, or 4- to 6-membered heterocycloalkyl, and the methyl, ethyl, cycloalkyl, 5-membered heteroaryl and 4- to 6-membered heterocycloalkyl are optionally further substituted with 1-5 groups selected from F, Cl, OH, NH₂, CN, methyl, ethyl, or methoxy; in some embodiments, each R^{A1} is independently selected from F, Cl, =O, CN, OH, COOH, methyl, ethyl, -O-C₃₋₆ cycloalkyl, or -NHRₐₐ, and the methyl, ethyl and cycloalkyl are optionally further substituted with 1-5 groups selected from F, Cl, OH, NH₂, CN, methyl, ethyl, or methoxy;
each R^{B} is independently selected from halogen, CN, =O, OH, -SF₅, C₁₋₄ alkyl, C₁₋₄ alkoxy, C₂₋₆ alkenyl, or C₂₋₆ alkynyl, and the alkyl, alkoxy, alkenyl and alkynyl are optionally further substituted with a group selected from halogen or C₁₋₂ alkyl; in some embodiments, each R^{B} is independently selected from halogen, =O, or C₁₋₂ alkyl, and the alkyl is optionally further substituted with halogen; in some embodiments, each R^{B} is independently selected from F, Cl, =O, methyl, or ethyl, and the methyl and ethyl are optionally further substituted with a group selected from F or Cl;
each R^{C} is independently selected from D, halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8-to 10-membered bicyclic heteroaryl, 4- to 12-membered heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)2, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocyclyl), - C(=O)NRₐ-(4- to 6-membered heterocyclyl), 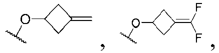 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl, heterocycloalkyl, cycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from D, halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRₐS(=O)₂C₁₋₄ alkyl, - NRₐP(=O)(C₁₋₄ alkyl)₂, -NRₐS(=O)₂NRₐₐR_{bb}, -NRₐC(=O)-C₃₋₆ cycloalkyl, - C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), -NRₐC(=O)-(4- to 6-membered heterocyclyl), -C(=O)NRₐ-(4- to 6-membered heterocyclyl), 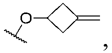 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl, heterocycloalkyl, cycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or - S(=O)₂C₁₋₄ alkyl; in some embodiments, each R^{C} is independently selected from D, halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4- to 12-membered heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)2, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), - NRₐC(=O)-(5- to 6-membered heteroaryl), -C(=O)NRₐ-(4- to 6-membered heteroaryl), -C(=O)NRₐ-(4- to 6-membered heterocycloalkyl), 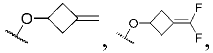 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from D, halogen, C₁₋₂ alkyl, C₁₋₂ alkoxy, halogenated C₁₋₂ alkyl, halogenated C₁₋₂ alkoxy, 5- to 6-membered heteroaryl, CN, =O, -NH-S(=O)₂-C₁₋₂ alkyl, -O-(5- to 6-membered heteroaryl), -O-C₃₋₆ cycloalkyl, -O-(4- to 12-membered heterocycloalkyl), 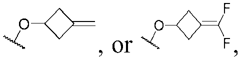 and the alkyl, heteroaryl and cycloalkyl are optionally further substituted with 1-4 groups selected from halogen, C₁₋₂ alkyl, or halogenated C₁₋₂ alkyl; in some embodiments, each R^{C} is independently selected from D, halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, - NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), - NRₐC(=O)-O-Rₐ, -(C₁₋₄ alkyl)ₙ-O-(C₁₋₄ alkyl)ₙ-(4- to 6-membered heterocyclyl), - NRₐC(=O)-(4- to 6-membered heterocycloalkyl), -NRₐC(=O)-(5- to 6-membered heteroaryl), -C(=O)NRₐ-(4- to 6-membered heteroaryl), -C(=O)NRₐ-(4- to 6-membered heterocycloalkyl), 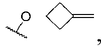 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or - S(=O)₂C₁₋₄ alkyl; in some embodiments, each R^{C} is independently selected from halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, - SF₅, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRₐS(=O)₂C₁₋₄ alkyl, -NRₐP(=O)(C₁₋₄ alkyl)2, -NRₐS(=O)₂NRₐₐR_{bb}, -NRₐC(=O)-C₃₋₆ cycloalkyl, - C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), -NRₐC(=O)-(4- to 6-membered heterocyclyl), -C(=O)NRₐ-(4- to 6-membered heterocyclyl), 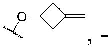 C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl, heterocycloalkyl, cycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or - S(=O)₂C₁₋₄ alkyl; in some embodiments, each R^{C} is independently selected from halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, - SF₅, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRₐS(=O)₂C₁₋₄ alkyl, -NRₐP(=O)(C₁₋₄ alkyl)2, -NRₐS(=O)₂NRₐₐR_{bb}, -NRₐC(=O)-C₃₋₆ cycloalkyl, - C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), -NRₐC(=O)-(4- to 6-membered heterocyclyl), -C(=O)NRₐ-(4- to 6-membered heterocyclyl), or 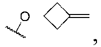 and the alkyl, cycloalkyl, heteroaryl, heterocycloalkyl, cycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl;
in some embodiments, each R^{C} is independently selected from halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRₐS(=O)₂C₁₋₄ alkyl, - NRₐP(=O)(C₁₋₄ alkyl)₂, -NRₐS(=O)₂NRₐₐR_{bb}, -NRₐC(=O)-C₃₋₆ cycloalkyl, - C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), or -NRₐC(=O)-(4- to 6-membered heterocyclyl), and the alkyl, cycloalkyl, heteroaryl, heterocycloalkyl and cycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; each n is independently selected from 0 or 1; in some embodiments, each R^{C} is independently selected from halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRₐS(=O)₂C₁₋₄ alkyl, - NRₐP(=O)(C₁₋₄ alkyl)2, -NRₐS(=O)₂NRₐₐR_{bb}, -NRₐC(=O)-C₃₋₆ cycloalkyl, - C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), -NRₐC(=O)-(4- to 6-membered heterocycloalkyl), -NRₐC(=O)-(5- to 6-membered heteroaryl), - C(=O)NRₐ-(4- to 6-membered heteroaryl), -C(=O)NRₐ-(4- to 6-membered heterocycloalkyl), or 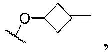 and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4-to 12-membered heterocycloalkyl, -N=S(=O)RₐₐR_{bb}, -P(=O)RₐₐR_{bb}, -NRₐS(=O)₂C₁₋₄ alkyl, -NRₐP(=O)(C₁₋₄ alkyl)2, -NRₐS(=O)₂NRₐₐR_{bb}, -NRₐC(=O)-C₃₋₆ cycloalkyl, - C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), -NRₐC(=O)-(4- to 6-membered heterocycloalkyl), or -NRₐC(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from D, halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, - NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-C₅₋₁₁ spirocycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocyclyl), - C(=O)NRₐ-(4- to 6-membered heterocyclyl), 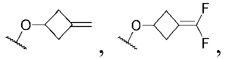 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl, heterocycloalkyl, spirocycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from D, halogen, C₁₋₂ alkyl, C₁₋₂ alkoxy, halogenated C₁₋₂ alkyl, halogenated C₁₋₃ alkoxy, C₃₋₄ cycloalkyl, 5- to 6-membered heteroaryl, CN, =O, -NH-S(=O)₂-C₁₋₂ alkyl, -O-(5- to 6-membered heteroaryl), -O-C₃₋₆ cycloalkyl, -O-(4- to 12-membered heterocycloalkyl), 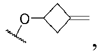 or 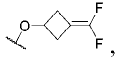 and the alkyl, heteroaryl and cycloalkyl are optionally further substituted with 1-4 groups selected from halogen, C₁₋₂ alkyl, or halogenated C₁₋₂ alkyl; in some embodiments, each R^{C} is independently selected from D, F, Cl, methyl, ethyl, -CHF₂, -CH₂F, -CF₃, -CH₂CF₃, -CH₂CHF₂, -CH₂CH₂F, -OCHF₂, - OCH₂F, -OCF₃, -OCH₂CF₃, -OCH(CH₃)CF₃, cyclopropyl, cyclobutyl, 5-membered heteroaryl, 6-membered heteroaryl, -CN, =O, -NH-S(=O)₂-C₁₋₂ alkyl, -O-(5-membered heteroaryl), -O-C₃₋₆ cycloalkyl, -O-(4- to 6-membered monocyclic heterocycloalkyl), 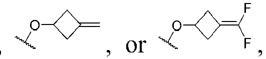 and the methyl, ethyl, cyclopropyl, cyclobutyl, heteroaryl, cycloalkyl and heterocycloalkyl are optionally further substituted with 1-4 groups selected from F, Cl, methyl, ethyl, -CHF₂, -CH₂F, or - CF₃; in some embodiments, each R^{C} is independently selected from halogen, halogenated C₁₋₃ alkyl, halogenated C₁₋₄ alkoxy, C₃₋₅ cycloalkyl, 5- to 6-membered heteroaryl, -O-C₃₋₆ cycloalkyl, -O-(4- to 12-membered heterocycloalkyl), -O-(5- to 6-membered heteroaryl), -O-C₅₋₈ spirocycloalkyl, 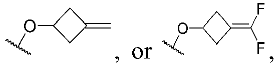 and the heteroaryl, cycloalkyl and spirocycloalkyl are optionally further substituted with 1-3 groups selected from halogen, C₁₋₂ alkyl, halogenated C₁₋₂ alkyl, or deuterated C₁₋₂ alkyl; in some embodiments, each R^{C} is independently selected from 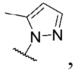 F, Cl, CF₃, CHF₂, CH₂F, -OCHF₂, -OCH₂F, -OCF₃, -OCH₂CF₃, - OCH(CH₃)CF₃, cyclopropyl, cyclobutyl, 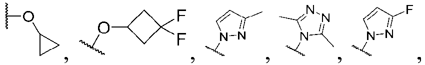 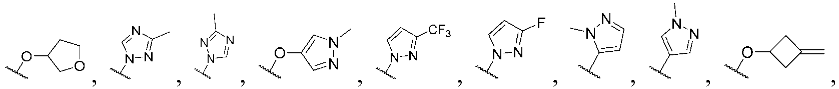 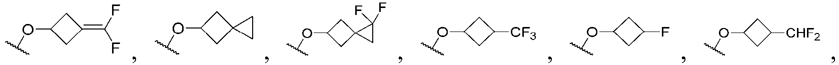 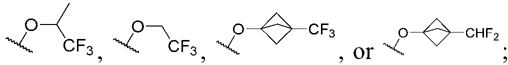 in some embodiments, each R^{C} is independently selected from halogen, C₁₋₂ alkyl, halogenated C₁₋₂ alkyl, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, -N=S(=O)RₐₐR_{bb}, -P(=O)RₐₐR_{bb}, - NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), - NRₐC(=O)-O-Rₐ, -(C₁₋₄ alkyl)ₙ-O-(C₁₋₄ alkyl)ₙ-(4- to 6-membered heterocyclyl), - NRₐC(=O)-(4- to 6-membered heterocycloalkyl), or -NRₐC(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8- to 10-membered bicyclic heteroaryl, 4- to 12-membered heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), or -NRₐS(=O)₂-C₃₋₆ cycloalkyl, and the alkyl, cycloalkyl, heteroaryl, heterocycloalkyl and cycloalkyl are optionally further substituted with 1-4 groups selected from halogen, OH, NH₂, CN, C₁₋₄ alkyl, halogenated C₁₋₄ alkyl, =O, -C(=O) C₁₋₄ alkyl, -NH-C(=O) C₁₋₄ alkyl, or - S(=O)₂C₁₋₄ alkyl; in some embodiments, each R^{C} is independently selected from halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4- to 12-membered heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), or -NRₐS(=O)₂-C₃₋₆ cycloalkyl, and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from D, halogen, C₁₋₂ alkyl, C₁₋₂ alkoxy, halogenated C₁₋₂ alkyl, halogenated C₁₋₂ alkoxy, 5- to 6-membered heteroaryl, CN, =O, -NH-S(=O)₂-C₁₋₂ alkyl, -O-(5- to 6-membered heteroaryl), -O-C₃₋₆ cycloalkyl, -O-(4- to 12-membered heterocycloalkyl), or 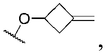 and the alkyl, heteroaryl and cycloalkyl are optionally further substituted with 1-4 groups selected from halogen, C₁₋₂ alkyl, or halogenated C₁₋₂ alkyl; in some embodiments, each R^{C} is independently selected from halogen, C₁₋₂ alkyl, C₁₋₂ alkoxy, halogenated C₁₋₂ alkyl, halogenated C₁₋₂ alkoxy, 5- to 6-membered heteroaryl, CN, =O, or -NH-S(=O)₂-C₁₋₂ alkyl, and the alkyl and heteroaryl are optionally further substituted with 1-4 groups selected from halogen or C₁₋₂ alkyl; in some embodiments, each R^{C} is independently selected from D, F, Cl, methyl, ethyl, - CHF₂, -CH₂F, -CF₃, -CH₂CF₃, -CH₂CHF₂, -CH₂CH₂F, -OCHF₂, -OCH₂F, -OCF₃, 5-membered heteroaryl, 6-membered heteroaryl, -CN, =O, -NH-S(=O)₂-C₁₋₂ alkyl, -O-(5-membered heteroaryl), -O-C₃₋₆ cycloalkyl, -O-(4- to 6-membered monocyclic heterocycloalkyl), 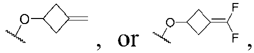 and the methyl, ethyl, heteroaryl and cycloalkyl are optionally further substituted with 1-4 groups selected from F, Cl, methyl, ethyl, -CHF₂, -CH₂F, or -CF₃; in some embodiments, each R^{C} is independently selected from D, F, Cl, methyl, ethyl, -CHF₂, -CH₂F, - CF₃, -CH₂CF₃, -CH₂CHF₂, -CH₂CH₂F, -OCHF₂, -OCH₂F, -OCF₃, 5-membered heteroaryl, 6-membered heteroaryl, -CN, =O, -NH-S(=O)₂-C₁₋₂ alkyl, -O-(5-membered heteroaryl), -O-C₃₋₆ cycloalkyl, -O-(4- to 6-membered monocyclic heterocycloalkyl), or 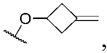 and the methyl, ethyl, heteroaryl and cycloalkyl are optionally further substituted with 1-4 groups selected from F, Cl, methyl, ethyl, or -CF₃; in some embodiments, each R^{C} is independently selected from F, Cl, methyl, ethyl, -CHF₂, -CH₂F, -CF₃, -CH₂CF₃, -CH₂CHF₂, -CH₂CH₂F, -OCHF₂, - OCH₂F, -OCF₃, 5-membered heteroaryl, 6-membered heteroaryl, CN, =O, or -NH-S(=O)₂-C₁₋₂ alkyl, and the methyl, ethyl and heteroaryl are optionally further substituted with 1-4 groups selected from F, Cl, methyl, or ethyl; in some embodiments, each R^{C} is independently selected from halogen, CN, =O, OH, C₁₋₄ alkyl, halogenated C₁₋₄ alkyl, C₃₋₅ cycloalkyl, C₂₋₄ alkenyl, C₂₋₄ alkynyl, -SCF₃, - SF₅, 5- to 6-membered heteroaryl, 8- to 10-membered heteroaryl, 4- to 12-membered heterocycloalkyl, -N=S(=O)RaaRbb, -P(=O)RaaRbb, -NRaS(=O)₂C₁₋₄ alkyl, -NRaP(=O)(C₁₋₄ alkyl)₂, -NRaS(=O)₂NRaaRbb, -NRaC(=O)-C₃₋₆ cycloalkyl, or -C(=O)NRa-C₃₋₆ cycloalkyl, and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, =O, -C(=O) C₁₋₄ alkyl, or -NH-C(=O) C₁₋₄ alkyl; in some embodiments, each R^{C} is independently selected from halogen, CN, =O, C₁₋₂ alkyl, halogenated C₁₋₂ alkyl, 5- to 6-membered heteroaryl, 8- to 10-membered heteroaryl, 4- to 12-membered heterocycloalkyl, - N=S(=O)RaaRbb, -P(=O)RaaRbb, -NRaS(=O)₂C₁₋₄ alkyl, -NRaP(=O)(C₁₋₄ alkyl)₂, -NRaS(=O)₂NRaaRbb, -NRaC(=O)-C₃₋₆ cycloalkyl, or -C(=O)NRa-C₃₋₆ cycloalkyl, and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, =O, -C(=O) C₁₋₄ alkyl, or -NH-C(=O) C₁₋₄ alkyl; in some embodiments, each R^{C} is independently selected from halogen, C₁₋₂ alkyl, halogenated C₁₋₂ alkyl, 5- to 6-membered heteroaryl, CN, or =O, and the alkyl and heteroaryl are optionally further substituted with 1-4 groups selected from halogen or C₁₋₂ alkyl; in some embodiments, each R^{C} is independently selected from F, Cl, methyl, ethyl, -CHF₂, -CH₂F, -CF₃, -CH₂CF₃, -CH₂CHF₂, -CH₂CH₂F, 5-membered heteroaryl, 6-membered heteroaryl, 8-membered heteroaryl, 9-membered heteroaryl, 10-membered heteroaryl, CN, or =O, and the methyl, ethyl and heteroaryl are optionally further substituted with 1-4 groups selected from F, Cl, methyl, or ethyl;
each R^{C1} is independently selected from halogen, C₁₋₂ alkyl, halogenated C₁₋₂ alkyl, halogenated C₁₋₃ alkoxy, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), - NRₐC(=O)-(5- to 6-membered heteroaryl), -C(=O)NRₐ-(5- to 6-membered heteroaryl), 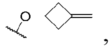 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O)C₁₋₄ alkyl, or -S(=O)₂C₁₋₄ alkyl; in some embodiments, each R^{C1} is independently selected from halogen, C₁₋₂ alkyl, halogenated C₁₋₂ alkyl, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)2, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), - NRₐC(=O)-(5- to 6-membered heteroaryl), -C(=O)NRₐ-(5- to 6-membered heteroaryl), 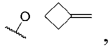 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C1} is independently selected from halogen, C₁₋₂ alkyl, halogenated C₁₋₂ alkyl, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), - NRₐC(=O)-(5- to 6-membered heteroaryl), -C(=O)NRₐ-(5- to 6-membered heteroaryl), or 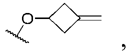 and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C1} is independently selected from halogen, C₁₋₂ alkyl, halogenated C₁₋₂ alkyl, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), or - NRₐC(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O)C₁₋₄ alkyl, -NH-C(=O) C₁₋₄ alkyl, or -S(=O)₂C₁₋₄ alkyl; in some embodiments, each R^{C1} is independently selected from F, Cl, methyl, ethyl, -CHF₂, -CH₂F, -CF₃, -CH₂CF₃, -CH₂CHF₂, -CH₂CH₂F, -OCHF₂, -OCH₂F, -OCF₃, - OCH₂CF₃, -OCH(CH₃)CF₃, cyclopropyl, cyclobutyl, cyclopentyl, cubane, 5-membered heteroaryl, 6-membered heteroaryl, 8-membered fused heteroaryl, 9-membered fused heteroaryl, 10-membered fused heteroaryl, 7- to 8-membered spiro heterocycloalkyl, 8- to 9-membered fused heterocycloalkyl, 7-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 6-membered heterocycloalkyl), -O-(5- to 6-membered heteroaryl), -NH-C₃₋₆ cycloalkyl, -NH-(4- to 6-membered heterocycloalkyl), -NH-(5- to 6-membered heteroaryl), -NH-S(=O)₂-C₃₋₆ cycloalkyl, -C₁₋₂ alkyl-(5-membered heteroaryl), -C₁₋₂ alkyl-(6-membered heteroaryl), -C₁₋₂ alkyl-phenyl, - C₁₋₂ alkyl-(4- to 6-membered heterocycloalkyl), -NRₐC(=O)-O-Rₐ, -(C₁₋₂ alkyl)ₙ-O-(C₁₋₂ alkyl)ₙ-(4- to 6-membered heterocyclyl), -NRₐC(=O)-(4- to 6-membered heterocyclyl), -C(=O)NRₐ-(4- to 6-membered heterocyclyl), 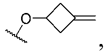 -C(=O)-C₃₋₆ cycloalkyl, -C(=O)-(4- to 12-membered heterocycloalkyl), or -C(=O)-(5- to 6-membered heteroaryl), and the cyclopropyl, cyclobutyl, cyclopentyl, alkyl, cycloalkyl, heterocyclyl, heteroaryl, heterocycloalkyl and cubane are optionally further substituted with 1-4 groups selected from F, Cl, OH, NH₂, CN, methyl, ethyl, deuterated methyl, -CHF₂, -CH₂F, -CF₃, -CH₂CF₃, -CH₂CHF₂, -CH₂CH₂F, =O, -C(=O) C₁₋₄ alkyl, -NH-C(=O) C₁₋₄ alkyl, or -S(=O)₂C₁₋₂ alkyl; in some embodiments, each R^{C1} is independently selected from F, Cl, methyl, ethyl, -CHF₂, -CH₂F, -CF₃, -CH₂CF₃, -CH₂CHF₂, -CH₂CH₂F, C₄₋₅ cycloalkyl, cubane, 5-membered heteroaryl, 6-membered heteroaryl, 8-membered fused heteroaryl, 9-membered fused heteroaryl, 10-membered fused heteroaryl, 7- to 8-membered spiro heterocycloalkyl, 8- to 9-membered fused heterocycloalkyl, 7-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 6-membered heterocycloalkyl), -O-(5- to 6-membered heteroaryl), -NH-C₃₋₆ cycloalkyl, -NH-(4- to 6-membered heterocycloalkyl), -NH-(5- to 6-membered heteroaryl), -NH-S(=O)₂-C₃₋₆ cycloalkyl, -C₁₋₂ alkyl-(5-membered heteroaryl), -C₁₋₂ alkyl-(6-membered heteroaryl), -C₁₋₂ alkyl-phenyl, - C₁₋₂ alkyl-(4- to 6-membered heterocycloalkyl), -NRₐC(=O)-O-Rₐ, -(C₁₋₂ alkyl)ₙ-O-(C₁₋₂ alkyl)ₙ-(4- to 6-membered heterocyclyl), or -NRₐC(=O)-(4- to 6-membered heterocyclyl), and the alkyl, cycloalkyl, heteroaryl, heterocycloalkyl and cubane are optionally further substituted with 1-4 groups selected from F, Cl, OH, NH₂, CN, methyl, ethyl, deuterated methyl, -CHF₂, -CH₂F, -CF₃, -CH₂CF₃, -CH₂CHF₂, - CH₂CH₂F, =O, -C(=O) C₁₋₄ alkyl, -NH-C(=O) C₁₋₄ alkyl, or -S(=O)₂C₁₋₂ alkyl; in some embodiments, each R^{C1} is independently selected from halogenated C₁₋₂ alkyl, 5- to 6-membered heteroaryl, -O-C₃₋₆ cycloalkyl, -O-4- to 6-membered heterocycloalkyl, or -O-5- to 6-membered heteroaryl, and the heteroaryl, cycloalkyl and heterocycloalkyl are optionally further substituted with 1-4 groups selected from halogen, OH, NH₂, CN, C₁₋₄ alkyl, halogenated C₁₋₄ alkyl, =O, or - C(=O) C₁₋₄ alkyl; in some embodiments, each R^{C1} is independently selected from 5-membered heteroaryl, 6-membered heteroaryl, or -O-C₃₋₆ cycloalkyl, and the heteroaryl and cycloalkyl are optionally further substituted with 1-4 groups selected from F, Cl, OH, NH₂, CN, methyl, ethyl, deuterated methyl, -CHF₂, - CH₂F, -CF₃, -CH₂CF₃, -CH₂CHF₂, -CH₂CH₂F, =O, -C(=O) C₁₋₄ alkyl, -NH-C(=O) C₁₋₄ alkyl, or -S(=O)₂C₁₋₂ alkyl; in some embodiments, each R^{C1} is independently selected from halogenated C₁₋₂ alkyl, pyrrolyl, imidazolyl, pyrazolyl, thiazolyl, triazolyl, -O-cyclopropyl, -O-cyclobutyl, -O-cyclopentyl, -O-bicyclo[1.1.1]pentane, -O-spiro[2.3]hexane, or -O-oxolanyl, and the pyrrolyl, imidazolyl, pyrazolyl, thiazolyl, cyclopropyl, cyclobutyl, cyclopentyl, bicyclo[1.1.1]pentane, spiro[2.3]hexane and oxolanyl are optionally further substituted with 1-4 groups selected from F, Cl, OH, NH₂, CN, methyl, ethyl, -CHF₂, -CH₂F, -CF₃, -CH₂CF₃, - CH₂CHF₂, -CH₂CH₂F, or =O; in some embodiments, each R^{C1} is independently selected from pyrrolyl, imidazolyl, pyrazolyl, thiazolyl, -O-cyclopropyl, -O-cyclobutyl, or -O-cyclopentyl, and the pyrrolyl, imidazolyl, pyrazolyl, thiazolyl, cyclopropyl, cyclobutyl and cyclopentyl are optionally further substituted with 1-4 groups selected from F, Cl, OH, NH₂, CN, methyl, ethyl, deuterated methyl, -CHF₂, -CH₂F, -CF₃, -CH₂CF₃, -CH₂CHF₂, -CH₂CH₂F, or =O;
Rₐ is selected from H, halogen, 3- to 6-membered cycloalkyl, C₁₋₄ alkyl, or halogenated 3- to 6-membered cycloalkyl;
in some embodiments, Rₐ is selected from H, halogen, 3- to 6-membered cycloalkyl, C₁₋₂ alkyl, or halogenated 3- to 6-membered cycloalkyl; in some embodiments, Rₐ is selected from H, F, Cl, methyl, ethyl, cyclopropyl, cyclobutyl, cyclopentyl, or difluorocyclobutyl; in some embodiments, Rₐ is selected from H, F, Cl, methyl, ethyl, cyclopropyl, cyclobutyl, or cyclopentyl;
Rₐₐ and R_{bb} are each independently selected from H, halogen, =O, C₁₋₄ alkyl, C₁₋₄ alkylhydroxy, halogenated C₁₋₄ alkyl, C₁₋₄ alkoxy or halogenated C₁₋₄ alkoxy; in some embodiments, Raa and Rbb are each independently selected from H, halogen, =O, C₁₋₄ alkyl, halogenated C₁₋₄ alkyl, C₁₋₄ alkoxy or halogenated C₁₋₄ alkoxy; in some embodiments, Rₐₐ and R_{bb} are each independently selected from H, F, Cl, =O, methyl, ethyl, -CH₂-CH₂-OH, -CH₂-OH, -CHF₂, -CH₂F, -CF₃, -CH₂CF₃, -CH₂CHF₂, -CH₂CH₂F, -OCH₃, -OCH₂CH₃, -OCHF₂, -OCH₂F, -OCF₃, -OCH₂CF₃, -OCH₂CHF₂, or -OCH₂CH₂F; in some embodiments, Rₐₐ and R_{bb} are each independently selected from H, F, Cl, =O, methyl, ethyl, -CHF₂, -CH₂F, -CF₃, - CH₂CF₃, -CH₂CHF₂, -CH₂CH₂F, -OCH₃, -OCH₂CH₃, -OCHF₂, -OCH₂F, -OCF₃, - OCH₂CF₃, -OCH₂CHF₂, or -OCH₂CH₂F;
alternatively, Rₐₐ and R_{bb}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
in some embodiments, Rₐₐ and R_{bb}, together with the atoms to which they are connected, form 4- to 6-membered heterocyclyl, and the heterocyclyl is optionally further substituted with 1-4 groups selected from F, Cl, OH, NH₂, CN, methyl, or ethyl; in some embodiments, each R^{C} is independently selected from halogen, CN, =O, OH, C₁₋₄ alkyl, halogenated C₁₋₄ alkyl, C₃₋₅ cycloalkyl, C₂₋₄ alkenyl, C₂₋₄ alkynyl, -SCF₃, -SF₅, or 5- to 6-membered heteroaryl, and the alkyl, cycloalkyl and heteroaryl are optionally further substituted with 1-4 groups selected from halogen, OH, NH₂, CN, or C₁₋₄ alkyl; in some embodiments, each R^{C} is independently selected from halogen, C₁₋₂ alkyl, halogenated C₁₋₂ alkyl, or 5- to 6-membered heteroaryl, and the alkyl and heteroaryl are optionally further substituted with 1-5 groups selected from halogen or C₁₋₂ alkyl; in some embodiments, each R^{C} is independently selected from halogen, halogenated C₁₋₂ alkyl, or 5- to 6-membered heteroaryl, and the alkyl and heteroaryl are optionally further substituted with 1-4 groups selected from halogen or C₁₋₂ alkyl; in some embodiments, each R^{C} is independently selected from F, Cl, -CF₃, -CHF₂, -CH₂CH₂F, -CH₂CF₃, -CH₂CHF₂, -CH₂CH₂F, 5-membered heteroaryl, or 6-membered heteroaryl, and the heteroaryl is optionally further substituted with 1-4 groups selected from F, Cl, methyl, or ethyl;
alternatively, two R^{C}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, R^{B} and R^{C}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl; in some embodiments, alternatively, R^{B} and R^{C}, together with the atoms to which they are connected, form 5- to 6-membered heterocyclyl, and the heterocyclyl is optionally further substituted with 1-4 groups selected from halogen or C₁₋₂ alkyl;
L₁ and L₂ are each independently W₁-R^{L}-W₂, wherein L₁ is connected to A on the left side, and L₂ is connected to B on the left side; L₁ and L₂ are not both bonds;
R^{L} is selected from a bond, C₁₋₄ alkylene, C₂₋₄ alkenylene, C₂₋₄ alkynylene, C₃₋₆ cycloalkyl, 5- to 12-membered heteroaryl, or 4- to 10-membered heterocycloalkyl containing 1-3 heteroatoms selected from N, O or S, and the alkylene, alkenylene, alkynylene, cycloalkyl, heteroaryl and heterocycloalkyl are optionally further substituted with 1-4 R^{L1}; each R^{L1} is independently selected from halogen, =O, C₁₋₄ alkyl, C₂₋₄ alkenyl, C₁₋₄ alkoxy, 3- to 6-membered cycloalkyl or 5- to 6-membered heteroaryl, and the alkyl, alkoxy, cycloalkyl and heteroaryl are optionally further substituted with 1-4 substituents selected from halogen, CN, OH, C₁₋₄ alkoxy and NH₂; in some embodiments, R^{L} is selected from a bond, C₁₋₄ alkylene, or C₂₋₄ alkenylene, and the alkylene and alkenylene are optionally further substituted with 1-4 R^{L1}; in some embodiments, R^{L} is selected from a bond, C₁₋₄ alkylene, C₂₋₄ alkenylene, C₂₋₄ alkynylene, C₃₋₆ cycloalkyl, 5- to 12-membered heteroaryl, or 4- to 10-membered heterocycloalkyl containing 1-3 heteroatoms selected from N, O or S, and the alkylene, alkenylene, alkynylene, cycloalkyl, heteroaryl and heterocycloalkyl are optionally further substituted with 1-4 R^{L1}; each R^{L1} is independently selected from halogen, =O, C₁₋₄ alkyl, C₂₋₄ alkenyl, C₁₋₄ alkoxy, or 3- to 6-membered cycloalkyl, and the alkyl, alkoxy and cycloalkyl are optionally further substituted with 1-4 substituents selected from halogen, CN, OH and NH₂; in some embodiments, each R^{L1} is independently selected from halogen, =O, C₁₋₂ alkyl, C₂₋₄ alkenyl, C₁₋₂ alkoxy, or 3- to 6-membered cycloalkyl, and the alkyl, alkoxy and cycloalkyl are optionally further substituted with 1-4 substituents selected from halogen, CN, OH and NH₂; in some embodiments, each R^{L1} is independently selected from F, Cl, Br, =O, -CH₂-, -CH₂CH₂-, C₂₋₄ alkenyl, -O-CH₂-, -O-CH₂CH₂-, or cyclopropyl, and the CH₂, alkenyl and cyclopropyl are optionally further substituted with 1-4 substituents selected from halogen, CN, OH and NH₂; in some embodiments, each R^{L1} is independently selected from F, Cl, Br, =O, methyl, ethyl, -CF₃, -CHF₂, -CH₂F, vinyl, propenyl, methoxy, ethoxy, -OCF₃, -OCHF₂, -OCH₂F, cyclopropyl, or cyclobutyl;
W₁ and W₂ are each independently selected from a bond, C₁₋₄ alkylene, -O-, - S-, -S(=O)-, -S(=O)₂-, -NR^{W1}-, -CONR^{W1}-, -NR^{W1}CO-, -C(=O)O-, -OC(=O)-, - C(=S)-, -C(=O)- or -(C₁₋₄ alkylene)-NR^{W1}CO-, and the alkylene is optionally further substituted with 1-4 groups selected from halogen, =O, C₁₋₄ alkyl, CN, OH and NH₂; in some embodiments, W₁ and W₂ are each independently selected from a bond, -O-, -S-, -NR^{W1}-, -CONR^{W1}-, -NR^{W1}CO-, -C(=O)O-, or -OC(=O)-; in some embodiments, W₁ and W₂ are each independently selected from -O-, -S-, -NR^{W1}-, - CONR^{W1}-, -NR^{W1}CO-, -C(=O)O-, or -OC(=O)-; in some embodiments, W₁ and W₂ are each independently selected from -O-, -S-, -NH-, -N(CH₃)-, -CON(CH₃)-, - N(CH₃)CO-, -C(=O)O-, or -OC(=O)-;
R^{W1} is selected from H, C₁₋₄ alkyl, or halogen;
in some embodiments, R^{W1} is selected from H, C₁₋₂ alkyl, or halogen; in some embodiments, R^{W1} is selected from H, -CH₃, -CH₂CH₃, F, Cl, or Br;
L₁ is selected from a bond, C₁₋₂ alkylene, -C(=O)-C₁₋₂ alkylene-, -N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-C₂₋₄ alkenylene, - C(=O)-NH-, -C(=O)-N(CH₃)-, -C(=O)-O-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, 3- to 6-membered cycloalkyl, 5- to 6-membered heteroaryl, or C₁₋₂ alkyl-C₁₋₂ alkoxy; in some embodiments, L₁ is selected from a bond, C₁₋₂ alkylene, -C(=O)-C₁₋₂ alkylene-, -N(C₁₋₂ alkylene)-C(=O)-C₁₋₂ alkylene-, -NH-C(=O)-C₁₋₂ alkylene-, - S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-C₂₋₄ alkenylene, -C(=O)-NH-, -C(=O)-O-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, 3- to 6-membered cycloalkyl, 5- to 6-membered heteroaryl, or C₁₋₂ alkyl-C₁₋₂ alkoxy; in some embodiments, L₁ is selected from a bond, C₁₋₂ alkylene, -C(=O)-C₁₋₂ alkylene-, - N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-NH-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl; in some embodiments, L₁ is selected from a bond, C₁₋₂ alkylene, -C(=O)-C₁₋₂ alkylene-, -N(C₁₋₂ alkylene)-C(=O)-C₁₋₂ alkylene-, -NH-C(=O)-C₁₋₂ alkylene-, - S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-NH-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, 3- to 6-membered cycloalkyl, 5- to 6-membered heteroaryl, or C₁₋₂ alkyl-C₁₋₂ alkoxy; in some embodiments, L₁ is selected from a bond, vinylene, -CH₂-, -CH₂-CH₂-, 4- to 6-membered monocyclic heterocycloalkyl, (4- to 6-membered monocyclic heterocycloalkyl)-C(=O)-CH₂-, - (4- to 6-membered monocyclic heterocycloalkyl)-CH₂-, -CH₂-(4- to 6-membered monocyclic heterocycloalkyl)-, -CH(CH₃)NHC(=O)-CH₂-, -CH₂NHC(=O)-CH₂-, - S(=O)₂-CH₂-, -C(=S)-CH₂-, -C(=O)-C₃₋₆ cycloalkyl, -C(=O)-CH(CH₃)-, -C(=O)-CH(C₃₋₆ cycloalkyl)-, -C(=O)-CF₂-, -C(=O)-C(CH₃)₂-, -CH₂-C(=O)-, -C(=O)-C(=O)-, C₃₋₆ cycloalkyl, -(5- to 6-membered heteroaryl)-CH₂-, 5- to 6-membered heteroaryl, -C(=O)-, -C(=O)-CH₂-, -N(CH₃)-CH₂-, -CF₂-CH₂-, -C(=O)-C₂₋₄ alkenylene, or -C(=O)-O-, and the -CH₂-, alkenylene, cycloalkyl, heterocycloalkyl and heteroaryl are optionally further substituted with 1-3 R^{L1}; each R^{L1} is independently selected from F, Cl, Br, =O, methyl, ethyl, -CF₃, -CHF₂, -CH₂F, vinyl, propenyl, methoxy, ethoxy, -OCF₃, -OCHF₂, -OCH₂F, cyclopropyl, cyclobutyl, methyl-methoxy, or 5-membered heteroaryl; in some embodiments, L₁ is selected from a bond, vinylene, -CH₂-, -CH₂-CH₂-, 4- to 6-membered monocyclic heterocycloalkyl, (4- to 6-membered monocyclic heterocycloalkyl)-C(=O)-CH₂-, -(4- to 6-membered monocyclic heterocycloalkyl)-CH₂-, -CH₂-(4- to 6-membered monocyclic heterocycloalkyl)-, -CH(CH₃)NHC(=O)-CH₂-, - CH₂NHC(=O)-CH₂-, -S(=O)₂-CH₂-, -C(=S)-CH₂-, -C(=O)-C₃₋₆ cycloalkyl, - C(=O)-CH(CH₃)-, -C(=O)-CH(C₃₋₆ cycloalkyl)-, -C(=O)-CF₂-, -C(=O)-C(CH₃)₂-, - CH₂-C(=O)-, -C(=O)-C(=O)-, C₃₋₆ cycloalkyl, -(5- to 6-membered heteroaryl)-CH₂-, 5- to 6-membered heteroaryl, -C(=O)-, -C(=O)-CH₂-, -N(CH₃)-CH₂-, or - CF₂-CH₂-, and the -CH₂-, cycloalkyl, heterocycloalkyl and heteroaryl are optionally further substituted with 1-3 R^{L1}; each R^{L1} is independently selected from F, Cl, Br, =O, methyl, ethyl, -CF₃, -CHF₂, -CH₂F, vinyl, propenyl, methoxy, ethoxy, -OCF₃, -OCHF₂, -OCH₂F, cyclopropyl, cyclobutyl, methyl-methoxy, or 5-membered heteroaryl; in some embodiments, L₁ is selected from a bond, vinylene, -CH₂-, 4- to 6-membered monocyclic heterocycloalkyl, 4- to 6-membered monocyclic heterocycloalkyl-C(=O)-CH₂-, -4- to 6-membered monocyclic heterocycloalkyl-CH₂-, -CH₂-4- to 6-membered monocyclic heterocycloalkyl-, - CH(CH₃)NHC(=O)-CH₂-, -CH₂NHC(=O)-CH₂-, -S(=O)₂-CH₂-, -C(=S)-CH₂-, - C(=O)-C₃₋₆ cycloalkyl, -C(=O)-CH(CH₃)-, -C(=O)-CH(C₃₋₆ cycloalkyl)-, -C(=O)-CF₂-, -C(=O)-C(CH₃)₂-, -CH₂-C(=O)-, -C(=O)-C(=O)-, C₃₋₆ cycloalkyl, -5- to 6-membered heteroaryl-CH₂-, -5- to 6-membered heteroaryl-, -C(=O)-, -C(=O)-CH₂-, or N(CH₃)-CH₂-, and the -CH₂-, cycloalkyl, heterocycloalkyl and heteroaryl are optionally further substituted with 1-3 R^{L1}; each R^{L1} is independently selected from F, Cl, Br, =O, methyl, ethyl, -CF₃, -CHF₂, -CH₂F, vinyl, propenyl, methoxy, ethoxy, -OCF₃, -OCHF₂, -OCH₂F, cyclopropyl, or cyclobutyl;
in some embodiments, L₁ is 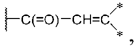 wherein the " * " end is connected to ring B;
L₂ is selected from a bond, C₁₋₂ alkylene, C₂₋₄ alkenylene, C₂₋₄ alkynylene, - C(=O)-NH-, -NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl; in some embodiments, L₂ is selected from a bond, vinylene, ethynylene, -CH₂-, -CH₂-CH₂-, -O-, -NH-, -C(=O)-, or -N(CH₃)-; in some embodiments, L₂ is selected from a bond, vinylene, ethynylene, -O-, -NH-, -C(=O)-, or -N(CH₃)-; in some embodiments, provided that: when L₂ is selected from -O- or -NH-, L₁ is not - C(=O)-, or B1 is not a bond;
R₁, R₂, R₃ and R₄ are each independently selected from H or halogen; provided that R₁, R₂, R₃ and R₄ are not all H;
alternatively, any two substituents of R₁, R₂, R₃ and R₄ together form C₃₋₆ cycloalkyl; in some embodiments, R₁, R₂, R₃ and R₄ are each independently selected from H, F, Cl, or Br; in some embodiments, R₁ and R₂ are F, and R₃ and R₄ are H; in some embodiments, R₃ and R₄ are F, and R₁ and R₂ are H; in some embodiments, alternatively, R₃ and R₄ together form =O;
L₁ₐ is selected from: a bond, C₁₋₂ alkylene, -C(=O)-CH(CH₃)-, -C(=O)-C(RₐR_{b})-, -N(C₁₋₂ alkylene)-C(=O)-C₁₋₂ alkylene-, -NH-C(=O)-C₁₋₂ alkylene-, - S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl; in some embodiments, L₁ₐ is selected from: a bond, C₁₋₂ alkylene, -C(=O)-CH(CH₃)-, -C(=O)-C(RₐR_{b})-, -N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-C₂₋₄ alkenylene, -C(=O)-NH-, -C(=O)-O-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl; in some embodiments, L₁ₐ is selected from: a bond, C₁₋₂ alkylene, -C(=O)-CH(CH₃)-, -C(=O)-C(RₐR_{b})-, - N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, 5- to 6-membered heteroaryl-C₁₋₂ alkylene-, 4- to 6-membered monocyclic heterocycloalkyl, C₃₋₆ cycloalkyl, 5-to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
in some embodiments, when L₁ₐ is selected from: -C(=O)-CH₂- or -C(=O)-CH₂CH₂-, and L₂ₐ is a bond, R^{C1} is 5- to 6-membered heteroaryl, and the heteroaryl is optionally further substituted with 1-4 groups selected from halogen or C₁₋₂ alkyl;
Rₐ and R_{b} are each independently selected from H, halogen, 3- to 6-membered cycloalkyl, or C₁₋₂ alkyl; provided that Rₐ and R_{b} are not both H;
L₂ₐ is selected from a bond, C₂₋₄ alkenylene, C₂₋₄ alkynylene, -C(=O)-NH-, - NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl; provided that when L₁ₐ is -C(=O)-, and L₂ₐ is selected from -NH- or -O-, B1 is not a bond; in some embodiments, L₂ₐ is selected from a bond, C₂₋₄ alkenylene, C₂₋₄ alkynylene, -C(=O)-NH-, -NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
in some embodiments, when L₁ₐ is selected from: -C(=O)-CH₂- or -C(=O)-CH₂CH₂-, and L₂ₐ is a bond, at least one R^{C1} is selected from =O, 5- to 6-membered heteroaryl, 8- to 10-membered bicyclic heteroaryl, 4- to 12-membered heterocycloalkyl, -N=S(=O)RaaRbb, -P(=O)RaaRbb, -NRaS(=O)₂C₁₋₄ alkyl, - NRaP(=O)(C₁₋₄ alkyl)₂, -NRaS(=O)₂NRₐₐR_{bb}, -NRaC(=O)-C₃₋₆ cycloalkyl, - C(=O)NRa-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, or cubane, and the alkyl, cycloalkyl, heteroaryl, heterocycloalkyl and cubane are optionally further substituted with 1-4 groups selected from halogen, OH, NH₂, CN, C₁₋₄ alkyl, halogenated C₁₋₄ alkyl, =O, - C(=O) C₁₋₄ alkyl, -NH-C(=O) C₁₋₄ alkyl, or -S(=O)₂C₁₋₄ alkyl;
in some embodiments, when L₁ₐ is selected from: -C(=O)-CH₂- or -C(=O)-CH₂CH₂-, and L₂ₐ is a bond, at least one R^{C1} is selected from 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRaS(=O)₂C₁₋₄ alkyl, - NRₐP(=O)(C₁₋₄ alkyl)2, -NRₐS(=O)₂NRₐₐR_{bb}, -NRaC(=O)-C₃₋₆ cycloalkyl, or - C(=O)NRa-C₃₋₆ cycloalkyl, and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, =O, -C(=O)C₁₋₄ alkyl, or -NH-C(=O)C₁₋₄ alkyl;
in some embodiments, provided that when L₁ₐ is -C(=O)-, and L₂ₐ is selected from -NH- or -O-, B1 is not a bond;
in some embodiments, when L₁ₐ is selected from: -C(=O)-CH₂- or -C(=O)-CH₂CH₂-, and R^{C1} is -CF₃, L₂ₐ is not a bond;
in some embodiments, when L₁ₐ is: -C(=O)-CH₂-, R^{C1} is selected from -CHF₂ or -CF₃, and L₂ₐ is a bond, at least one R^{A} is not C₁₋₂ alkyl, and n3 is selected from 1, 2, 3, 4 or 5;
in some embodiments, L_{1b} is selected from: a bond, 4- to 6-membered monocyclic heterocycloalkyl, or -C(=O)-NH-, and the heterocycloalkyl is optionally further substituted with 1-4 groups selected from halogen, =O, or C₁₋₂ alkyl;
alternatively, 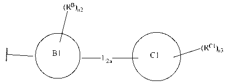 is 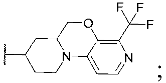
each n is independently selected from 0 or 1;
n1, n2 and n3 are each independently selected from 0, 1, 2, 3, 4 or 5;
in some embodiments, n1 is selected from 0, 1, 2 or 3; in some embodiments, n1 is selected from 1, 2 or 3;
in some embodiments, n2 is selected from 0 or 1;
in some embodiments, n3 is selected from 0, 1, 2, 3 or 4; in some embodiments, n3 is selected from 0, 1 or 2;
X₁ is selected from CR^{X1} or N; or X₁ is NR^{X1};
X₂ is selected from CR^{X2} or N;
X₃ is selected from CR^{X3} or N;
X₄ is selected from CR^{X4} or N;
R^{X1}, R^{X2}, R^{X3} and R^{X4} are each independently selected from H, halogen, =O, CN, OH, COOH, C₁₋₄ alkyl, C₁₋₄ alkoxy, C₂₋₆ alkenyl, C₂₋₆ alkynyl, 5- to 12-membered heteroaryl, -SO₂-C₁₋₄ alkyl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, or -NRₐₐR_{bb}, and the alkyl, alkenyl, alkynyl, heteroaryl, cycloalkyl and heterocycloalkyl are optionally further substituted with 1-4 groups selected from halogen, OH, NH₂, CN, C₁₋₄ alkoxy, -O-halogenated C₁₋₄ alkyl, or C₁₋₄ alkyl; in some embodiments, R^{X1}, R^{X2}, R^{X3} and R^{X4} are each independently selected from H, halogen, =O, CN, OH, COOH, C₁₋₄ alkyl, C₁₋₄ alkoxy, C₂₋₆ alkenyl, C₂₋₆ alkynyl, 5- to 12-membered heteroaryl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, or -NRₐₐR_{bb}, and the alkyl, alkenyl, alkynyl, heteroaryl, cycloalkyl and heterocycloalkyl are optionally further substituted with 1-4 groups selected from halogen, OH, NH₂, CN, C₁₋₄ alkoxy, -O-halogenated C₁₋₄ alkyl, or C₁₋₄ alkyl; in some embodiments, R^{X1}, R^{X2}, R^{X3} and R^{X4} are each independently selected from H, halogen, =O, CN, OH, COOH, C₁₋₂ alkyl, C₁₋₂ alkoxy, C₂₋₄ alkenyl, C₂₋₄ alkynyl, 5-to 6-membered heteroaryl, -SO₂-C₁₋₂ alkyl, -NRₐCO-C₃₋₆ cycloalkyl, 4- to 7-membered heterocycloalkyl, -O-C₃₋₆ cycloalkyl, -C₁₋₄ alkyl-(5- to 6-membered heteroaryl), -O-(4- to 7-membered heterocycloalkyl), -C₁₋₄ alkyl-(4- to 7-membered heterocycloalkyl), -C₁₋₄ alkyl-O-(4- to 7-membered heterocycloalkyl), - C₁₋₄ alkyl-O-C₃₋₆ cycloalkyl, -C₁₋₄ alkyl-OC(=O)-(4- to 7-membered heterocycloalkyl), -C₁₋₄ alkyl-OC(=O)-NRₐₐ-C₃₋₆ cycloalkyl, or -NRₐₐR_{bb}, and the alkyl, alkenyl, alkynyl, heteroaryl, cycloalkyl and heterocycloalkyl are optionally further substituted with 1-4 groups selected from halogen, OH, NH₂, CN, C₁₋₂ alkoxy, O-halogenated C₁₋₂ alkyl, or C₁₋₂ alkyl; in some embodiments, R^{X1}, R^{X2}, R^{X3} and R^{X4} are each independently selected from H, halogen, =O, CN, OH, COOH, C₁₋₂ alkyl, C₁₋₂ alkoxy, C₂₋₄ alkenyl, C₂₋₄ alkynyl, 5- to 6-membered heteroaryl, -NRₐCO-C₃₋₆ cycloalkyl, 4- to 7-membered heterocycloalkyl, -O-C₃₋₆ cycloalkyl, -C₁₋₄ alkyl-(5- to 6-membered heteroaryl), -O-(4- to 7-membered heterocycloalkyl), -C₁₋₄ alkyl-(4- to 7-membered heterocycloalkyl), -C₁₋₄ alkyl-O-(4- to 7-membered heterocycloalkyl), -C₁₋₄ alkyl-O-C₃₋₆ cycloalkyl, -C₁₋₄ alkyl-OC(=O)-(4- to 7-membered heterocycloalkyl), -C₁₋₄ alkyl-OC(=O)-NRₐₐ-C₃₋₆ cycloalkyl, or -NRₐₐR_{bb}, and the alkyl, alkenyl, alkynyl, heteroaryl, cycloalkyl and heterocycloalkyl are optionally further substituted with 1-4 groups selected from halogen, OH, NH₂, CN, C₁₋₂ alkoxy, -F, O-halogenated C₁₋₂ alkyl, or C₁₋₂ alkyl; in some embodiments, R^{X1}, R^{X2}, R^{X3} and R^{X4} are each independently selected from H, F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), -C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), -C₁₋₂ alkyl-OC(=O)-NH-C₃₋₆ cycloalkyl, or -NHRₐₐ; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, or ethyl; in some embodiments, R^{X1}, R^{X2}, R^{X3} and R^{X4} are each independently selected from H, F, Cl, =O, CN, -SO₂CH₃, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), -C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), -C₁₋₂ alkyl-OC(=O)-NH-C₃₋₆ cycloalkyl, or -NHRₐₐ, and the methyl, ethyl, heteroaryl, cycloalkyl and heterocycloalkyl are optionally further substituted with 1-4 groups selected from F, Cl, OH, NH₂, CN, methyl, ethyl, or methoxy; in some embodiments, R^{X1}, R^{X2}, R^{X3} and R^{X4} are each independently selected from H, F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), -C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), -C₁₋₂ alkyl-OC(=O)-NH-C₃₋₆ cycloalkyl, or -NHRₐₐ, and the methyl, ethyl, heteroaryl, cycloalkyl and heterocycloalkyl are optionally further substituted with 1-4 groups selected from F, Cl, OH, NH₂, CN, methyl, ethyl, or methoxy; alternatively, any two of R^{X1}, R^{X2}, R^{X3} and R^{X4}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
in some embodiments, 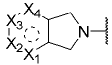 is selected from 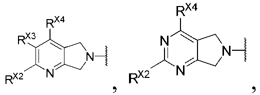 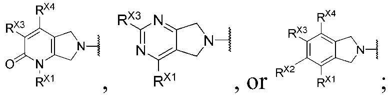
in some embodiments, 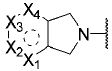 is selected from 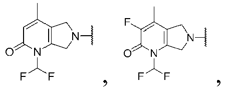 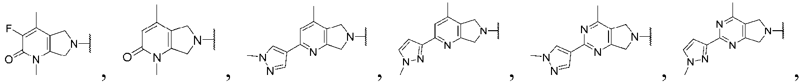 or

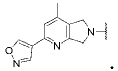

A specific technical solution 1 of the present invention provides a compound as represented by formula (I), or a stereoisomer, a deuterated substance, a solvate, a co-crystal or a pharmaceutically acceptable salt thereof, wherein 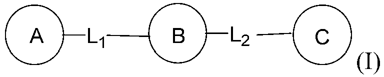
A is selected from 3- to 20-membered heterocycloalkyl or 5- to 20-membered heteroaryl; the heterocycloalkyl and heteroaryl contain 1-5 heteroatoms selected from N, O or S; and the heterocycloalkyl and heteroaryl are optionally substituted with 0-5 R^{A};
B is selected from a bond, C₃₋₁₀ cycloalkyl, C₆₋₁₂ aryl, 3- to 12-membered heterocycloalkyl or 5- to 12-membered heteroaryl; the heterocycloalkyl and heteroaryl contain 1-5 heteroatoms selected from N, O or S; and the cycloalkyl, aryl, heterocyclyl and heteroaryl are optionally substituted with 0-5 R^{B};
C is selected from C₃₋₁₂ cycloalkyl, C₆₋₁₀ aryl, 3- to 14-membered heterocycloalkyl or 5- to 14-membered heteroaryl; the heterocycloalkyl and heteroaryl contain 1-5 heteroatoms selected from N, O or S; and the cycloalkyl, heterocycloalkyl, aryl and heteroaryl are optionally substituted with 0-5 R^{C}; in some embodiments, C is selected from C₃₋₁₂ cycloalkyl, C₆₋₁₀ aryl, 3- to 12-membered heterocycloalkyl or 5- to 12-membered heteroaryl; the heterocycloalkyl and heteroaryl contain 1-5 heteroatoms selected from N, O or S; and the cycloalkyl, heterocycloalkyl, aryl and heteroaryl are optionally substituted with 0-5 R^{C};
L₁ and L₂ are each independently W₁-R^{L}-W₂, wherein L₁ is connected to A on the left side, and L₂ is connected to B on the left side;
R^{L} is selected from a bond, C₁₋₄ alkylene, C₂₋₄ alkenylene, C₂₋₄ alkynylene, C₃₋₆ cycloalkyl, 5- to 12-membered heteroaryl, or 4- to 10-membered heterocycloalkyl containing 1-3 heteroatoms selected from N, O or S, and the alkylene, alkenylene, alkynylene, cycloalkyl, heteroaryl and heterocycloalkyl are optionally further substituted with 1-4 R^{L1}; each R^{L1} is independently selected from halogen, =O, C₁₋₄ alkyl, C₂₋₄ alkenyl, C₁₋₄ alkoxy, 3- to 6-membered cycloalkyl or 5- to 6-membered heteroaryl, and the alkyl, alkoxy, cycloalkyl and heteroaryl are optionally further substituted with 1-4 substituents selected from halogen, CN, OH, C₁₋₄ alkoxy and NH₂;
W₁ and W₂ are each independently selected from a bond, C₁₋₄ alkylene, -O-, - S-, -S(=O)-, -S(=O)₂-, -NR^{W1}-, -CONR^{W1}-, -NR^{W1}CO-, -C(=O)O-, -OC(=O)-, - C(=S)-, -C(=O)- or -(C₁₋₄ alkylene)-NR^{W1}CO-, and the alkylene is optionally further substituted with 1-4 groups selected from halogen, =O, C₁₋₄ alkyl, CN, OH and NH₂;
R^{W1} is selected from H, C₁₋₄ alkyl, or halogen;
alternatively, L₁ is 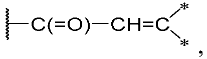 wherein the " * " end is connected to ring B;
each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, - NRₐCO-C₃₋₆ cycloalkyl, 4- to 12-membered heterocycloalkyl, -O-C₃₋₆ cycloalkyl, - C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4-to 12-membered heterocycloalkyl), -C₁₋₄ alkyl-OC(=O)-NRₐₐ-C₃₋₆ cycloalkyl, - NRₐₐR_{bb}, -SO₂-C₁₋₄ alkyl, -C₃₋₆ cycloalkyl, -NRₐₐ-C₃₋₆ cycloalkyl, 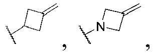 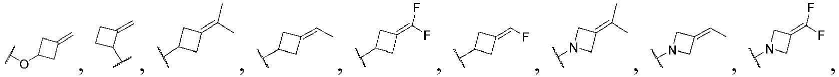 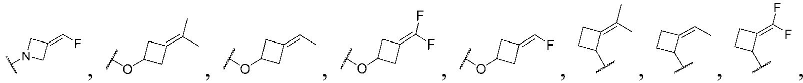 or 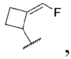 and the alkyl, alkenyl, alkynyl, heteroaryl, cycloalkyl and heterocycloalkyl are optionally further substituted with 1-5 groups selected from halogen, OH, NH₂, CN, C₁₋₄ alkoxy, -O-halogenated C₁₋₄ alkyl, C₁₋₄ alkyl, -C₁₋₄ alkylhydroxy, -C(=O) C₁₋₄ alkyl, or halogenated C₁₋₄ alkyl;
in some embodiments, each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), - O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, -C3-6 cycloalkyl, -NRₐₐ-C₃₋₆ cycloalkyl, 5- to 6-membered heteroaryl, 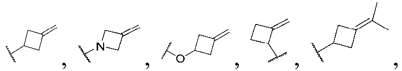 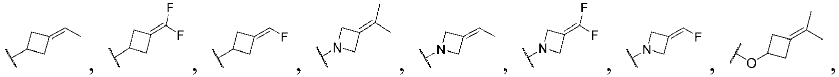 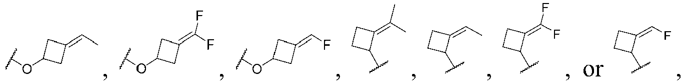 and the alkyl, alkenyl, alkynyl, heteroaryl, cycloalkyl and heterocycloalkyl are optionally further substituted with 1-5 groups selected from halogen, OH, NH₂, CN, C₁₋₄ alkoxy, -O-halogenated C₁₋₄ alkyl, C₁₋₄ alkyl, -C₁₋₄ alkylhydroxy, -C(=O) C₁₋₄ alkyl, or halogenated C₁₋₄ alkyl;
in some embodiments, each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), - O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 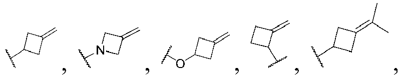 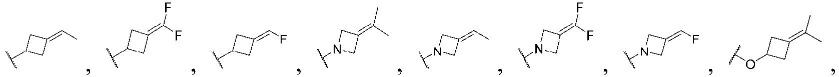 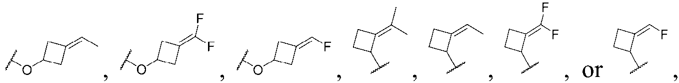 and the alkyl, alkenyl, alkynyl, heteroaryl, cycloalkyl and heterocycloalkyl are optionally further substituted with 1-5 groups selected from halogen, OH, NH₂, CN, C₁₋₄ alkoxy, -O-halogenated C₁₋₄ alkyl, C₁₋₄ alkyl, or -C₁₋₄ alkylhydroxy;
in some embodiments, each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), - O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, or -NRₐₐR_{bb}, and the alkyl, alkenyl, alkynyl, heteroaryl, cycloalkyl and heterocycloalkyl are optionally further substituted with 1-5 groups selected from halogen, OH, NH₂, CN, C₁₋₄ alkoxy, -O-halogenated C₁₋₄ alkyl, or C₁₋₄ alkyl;
in some embodiments, each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), or -NRₐₐR_{bb}, and the alkyl, alkenyl, alkynyl, heteroaryl, cycloalkyl and heterocycloalkyl are optionally further substituted with 1-4 groups selected from halogen, OH, NH₂, CN, C₁₋₄ alkoxy, -O-halogenated C₁₋₄ alkyl, or C₁₋₄ alkyl; in some embodiments, each R^{A} is independently selected from halogen, =O, CN, OH, COOH, C₁₋₄ alkyl, C₂₋₆ alkenyl, C₂₋₆ alkynyl, 5- to 12-membered heteroaryl, - NRₐCO-C₃₋₆ cycloalkyl, 4- to 12-membered heterocycloalkyl, or -O-C₃₋₆ cycloalkyl, and the alkyl, alkenyl, alkynyl, heteroaryl, cycloalkyl and heterocycloalkyl are optionally further substituted with 1-4 groups selected from halogen, OH, NH₂, CN, -O-halogenated C₁₋₄ alkyl, or C₁₋₄ alkyl;
alternatively, two R^{A}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
each R^{B} is independently selected from halogen, CN, =O, OH, -SF₅, C₁₋₄ alkyl, C₁₋₄ alkoxy, C₂₋₆ alkenyl, or C₂₋₆ alkynyl, and the alkyl, alkoxy, alkenyl and alkynyl are optionally further substituted with a group selected from halogen or C₁₋₂ alkyl;
each R^{C} is independently selected from D, halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8-to 10-membered bicyclic heteroaryl, 4- to 12-membered heterocycloalkyl, - N=S(=O)RₐₐR_{bb}, -P(=O)RₐₐR_{bb}, -NRaS(=O)₂C₁₋₄ alkyl, -NRₐP(=O)(C₁₋₄ alkyl)₂, - NRₐS(=O)₂NRₐₐR_{bb}, -NRaC(=O)-C₃₋₆ cycloalkyl, -C(=O)NRₐ-C₃₋₆ cycloalkyl, -O-C₃₋₆ cycloalkyl, -O-C₅₋₁₁ spirocycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocyclyl), -C(=O)NRₐ-(4- to 6-membered heterocyclyl), 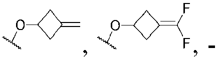 C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl, heterocycloalkyl, spirocycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl;
in some embodiments, each R^{C} is independently selected from D, halogen, C₁₋₂ alkyl, C₁₋₂ alkoxy, halogenated C₁₋₂ alkyl, halogenated C₁₋₃ alkoxy, C₃₋₄ cycloalkyl, 5- to 6-membered heteroaryl, CN, =O, -NH-S(=O)₂-C₁₋₂ alkyl, -O-(5-to 6-membered heteroaryl), -O-C₃₋₆ cycloalkyl, -O-(4- to 12-membered heterocycloalkyl), 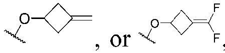 and the alkyl, heteroaryl and cycloalkyl are optionally further substituted with 1-4 groups selected from halogen, C₁₋₂ alkyl, or halogenated C₁₋₂ alkyl;
in some embodiments, each R^{C} is independently selected from D, halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5-to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRaS(=O)₂C₁₋₄ alkyl, -NRₐP(=O)(C₁₋₄ alkyl)₂, -NRaS(=O)₂NRₐₐR_{bb}, -NRaC(=O)-C₃₋₆ cycloalkyl, - C(=O)NRa-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), -NRₐC(=O)-(4- to 6-membered heterocyclyl), -C(=O)NRₐ-(4- to 6-membered heterocyclyl), 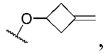 C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl, heterocycloalkyl, cycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or - S(=O)₂C₁₋₄ alkyl;
in some embodiments, each R^{C} is independently selected from halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRaS(=O)₂C₁₋₄ alkyl, - NRₐP(=O)(C₁₋₄ alkyl)2, -NRₐS(=O)₂NRₐₐR_{bb}, -NRaC(=O)-C₃₋₆ cycloalkyl, - C(=O)NRa-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), -NRₐC(=O)-(4- to 6-membered heterocyclyl), -C(=O)NRₐ-(4- to 6-membered heterocyclyl), 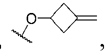 C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl, heterocycloalkyl, cycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or - S(=O)₂C₁₋₄ alkyl;
in some embodiments, each R^{C} is independently selected from halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRaS(=O)₂C₁₋₄ alkyl, - NRₐP(=O)(C₁₋₄ alkyl)₂, -NRaS(=O)₂NRₐₐR_{bb}, -NRaC(=O)-C₃₋₆ cycloalkyl, - C(=O)NRa-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), -NRₐC(=O)-(4- to 6-membered heterocyclyl), -C(=O)NRₐ-(4- to 6-membered heterocyclyl), or 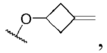 and the alkyl, cycloalkyl, heteroaryl, heterocycloalkyl, cycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRₐS(=O)₂C₁₋₄ alkyl, - NRₐP(=O)(C₁₋₄ alkyl)2, -NRₐS(=O)₂NRₐₐR_{bb}, -NRaC(=O)-C₃₋₆ cycloalkyl, - C(=O)NRa-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), or -NRₐC(=O)-(4- to 6-membered heterocyclyl), and the alkyl, cycloalkyl, heteroaryl, heterocycloalkyl and cycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRaS(=O)₂C₁₋₄ alkyl, - NRₐP(=O)(C₁₋₄ alkyl)₂, -NRaS(=O)₂NRₐₐR_{bb}, -NRaC(=O)-C₃₋₆ cycloalkyl, - C(=O)NRa-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), or - NRₐS(=O)₂-C₃₋₆ cycloalkyl, and the alkyl, cycloalkyl, heteroaryl, heterocycloalkyl and cycloalkyl are optionally further substituted with 1-4 groups selected from halogen, OH, NH₂, CN, C₁₋₄ alkyl, halogenated C₁₋₄ alkyl, =O, -C(=O) C₁₋₄ alkyl, - NH-C(=O) C₁₋₄ alkyl, or -S(=O)₂C₁₋₄ alkyl;
each n is independently selected from 0 or 1;
Rₐ is selected from H, halogen, 3- to 6-membered cycloalkyl, C₁₋₄ alkyl, or halogenated 3- to 6-membered cycloalkyl; in some embodiments, Rₐ is selected from H, halogen, 3- to 6-membered cycloalkyl, or C₁₋₄ alkyl;
Rₐₐ and R_{bb} are each independently selected from H, halogen, =O, C₁₋₄ alkyl, C₁₋₄ alkylhydroxy, halogenated C₁₋₄ alkyl, C₁₋₄ alkoxy or halogenated C₁₋₄ alkoxy;
or Rₐₐ and R_{bb} are each independently selected from H, halogen, =O, C₁₋₄ alkyl, halogenated C₁₋₄ alkyl, C₁₋₄ alkoxy or halogenated C₁₋₄ alkoxy;
alternatively, Rₐₐ and R_{bb}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, two R^{C}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, R^{B} and R^{C}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl.

Further, technical solution 1 provides a compound as represented by formula (I), or a stereoisomer, a deuterated substance, a solvate, a co-crystal or a pharmaceutically acceptable salt thereof, wherein 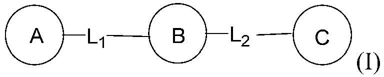
A is selected from 3- to 20-membered heterocycloalkyl or 5- to 20-membered heteroaryl; the heterocycloalkyl and heteroaryl contain 1-5 heteroatoms selected from N, O or S; and the heterocycloalkyl and heteroaryl are optionally substituted with 0-5 R^{A};
B is selected from a bond, C₃₋₁₀ cycloalkyl, C₆₋₁₂ aryl, 3- to 12-membered heterocycloalkyl or 5- to 12-membered heteroaryl; the heterocycloalkyl and heteroaryl contain 1-5 heteroatoms selected from N, O or S; and the cycloalkyl, aryl, heterocyclyl and heteroaryl are optionally substituted with 0-5 R^{B};
C is selected from C₃₋₁₂ cycloalkyl, C₆₋₁₀ aryl, 3- to 12-membered heterocycloalkyl or 5- to 12-membered heteroaryl; the heterocycloalkyl and heteroaryl contain 1-5 heteroatoms selected from N, O or S; and the cycloalkyl, heterocycloalkyl, aryl and heteroaryl are optionally substituted with 0-5 R^{C};
L₁ and L₂ are each independently W₁-R^{L}-W₂, wherein L₁ is connected to A on the left side, and L₂ is connected to B on the left side;
R^{L} is selected from a bond, C₁₋₄ alkylene, C₂₋₄ alkenylene, C₂₋₄ alkynylene, C₃₋₆ cycloalkyl, 5- to 12-membered heteroaryl, or 4- to 10-membered heterocycloalkyl containing 1-3 heteroatoms selected from N, O or S, and the alkylene, alkenylene, alkynylene, cycloalkyl, heteroaryl and heterocycloalkyl are optionally further substituted with 1-4 R^{L1}; each R^{L1} is independently selected from halogen, =O, C₁₋₄ alkyl, C₂₋₄ alkenyl, C₁₋₄ alkoxy, 3- to 6-membered cycloalkyl or 5- to 6-membered heteroaryl, and the alkyl, alkoxy, cycloalkyl and heteroaryl are optionally further substituted with 1-4 substituents selected from halogen, CN, OH, C₁₋₄ alkoxy and NH₂;
W₁ and W₂ are each independently selected from a bond, C₁₋₄ alkylene, -O-, - S-, -S(=O)-, -S(=O)₂-, -NR^{W1}-, -CONR^{W1}-, -NR^{W1}CO-, -C(=O)O-, -OC(=O)-, - C(=S)-, -C(=O)- or -(C₁₋₄ alkylene)-NR^{W1}CO-, and the alkylene is optionally further substituted with 1-4 groups selected from halogen, =O, C₁₋₄ alkyl, CN, OH and NH₂;
R^{W1} is selected from H, C₁₋₄ alkyl, or halogen;
alternatively, L₁ is 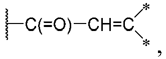 wherein the " * " end is connected to ring B;
each R^{A} is independently selected from halogen, =O, CN, OH, COOH, C₁₋₄ alkyl, C₂₋₆ alkenyl, C₂₋₆ alkynyl, 5- to 12-membered heteroaryl, -NRₐCO-C₃₋₆ cycloalkyl, 4- to 12-membered heterocycloalkyl, or -O-C₃₋₆ cycloalkyl, and the alkyl, alkenyl, alkynyl, heteroaryl, cycloalkyl and heterocycloalkyl are optionally further substituted with 1-4 groups selected from halogen, OH, NH₂, CN, -O-halogenated C₁₋₄ alkyl, or C₁₋₄ alkyl;
alternatively, two R^{A}, together with the atoms to which they are connected, form C₃₋₆ cycloalkyl or 4- to 10-membered heterocycloalkyl, and the cycloalkyl and heterocycloalkyl are optionally further substituted with 1-4 groups selected from halogen, OH, NH₂, CN, or C₁₋₄ alkyl;
each R^{B} is independently selected from halogen, CN, =O, OH, -SF₅, C₁₋₄ alkyl, C₁₋₄ alkoxy, C₂₋₆ alkenyl, or C₂₋₆ alkynyl, and the alkyl, alkoxy, alkenyl and alkynyl are optionally further substituted with a group selected from halogen or C₁₋₂ alkyl;
each R^{C} is independently selected from halogen, CN, =O, OH, C₁₋₄ alkyl, halogenated C₁₋₄ alkyl, C₃₋₅ cycloalkyl, C₂₋₄ alkenyl, C₂₋₄ alkynyl, -SCF₃, -SF₅, 5-to 6-membered heteroaryl, 8- to 10-membered bicyclic heteroaryl, 4- to 12-membered heterocycloalkyl, -N=S(=O)RaaRbb, -P(=O)RaaRbb, -NRaS(=O)₂C₁₋₄ alkyl, -NRaP(=O)(C₁₋₄ alkyl)₂, -NRaS(=O)₂NRₐₐR_{bb}, -NRaC(=O)-C₃₋₆ cycloalkyl, or -C(=O)NRa-C₃₋₆ cycloalkyl, and the alkyl, cycloalkyl, heteroaryl, heterocycloalkyl and cycloalkyl are optionally further substituted with 1-4 groups selected from halogen, OH, NH₂, CN, C₁₋₄ alkyl, halogenated C₁₋₄ alkyl, =O, - C(=O) C₁₋₄ alkyl, or -NH-C(=O) C₁₋₄ alkyl;
Rₐ is selected from H, halogen, 3- to 6-membered cycloalkyl, or C₁₋₄ alkyl; Raa and Rbb are each independently selected from H, halogen, =O, C₁₋₄ alkyl, halogenated C₁₋₄ alkyl, C₁₋₄ alkoxy or halogenated C₁₋₄ alkoxy;
alternatively, Raa and Rbb, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, two R^{C}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, R^{B} and R^{C}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl.

Further, technical solution 1 provides a compound as represented by formula (I), or a stereoisomer, a deuterated substance, a solvate, a co-crystal or a pharmaceutically acceptable salt thereof, wherein 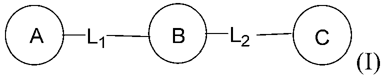
A is selected from 3- to 20-membered heterocyclyl or 5- to 20-membered heteroaryl; the heterocyclyl contains 1-5 heteroatoms selected from N, O or S; and the heterocyclyl and heteroaryl are optionally substituted with 0-5 R^{A};
B is selected from a bond, C₃₋₁₀ cycloalkyl, C₆₋₁₂ aryl, 3- to 12-membered heterocyclyl or 5- to 12-membered heteroaryl; the heterocyclyl and heteroaryl contain 1-5 heteroatoms selected from N, O or S; and the cycloalkyl, aryl, heterocyclyl and heteroaryl are optionally substituted with 0-5 R^{B};
C is selected from C₃₋₁₂ cycloalkyl, C₆₋₁₀ aryl, 3- to 12-membered heterocyclyl or 5- to 12-membered heteroaryl; the heterocyclyl and heteroaryl contain 1-5 heteroatoms selected from N, O or S; and the cycloalkyl, heterocyclyl, aryl and heteroaryl are optionally substituted with 0-5 R^{C};
L₁ and L₂ are each independently W₁-R^{L}-W₂, wherein L₁ is connected to A on the left side, and L₂ is connected to B on the left side;
R^{L} is selected from a bond, C₁₋₄ alkylene, C₂₋₄ alkenylene, C₂₋₄ alkynylene, C₃₋₆ cycloalkyl, 5- to 12-membered heteroaryl, or 4- to 10-membered heterocycloalkyl containing 1-3 heteroatoms selected from N, O or S, and the alkylene, alkenylene, alkynylene, cycloalkyl, heteroaryl and heterocycloalkyl are optionally further substituted with 1-4 R^{L1}; each R^{L1} is independently selected from halogen, =O, C₁₋₄ alkyl, C₂₋₄ alkenyl, C₁₋₄ alkoxy, or 3- to 6-membered cycloalkyl, and the alkyl, alkoxy and cycloalkyl are optionally further substituted with 1-4 substituents selected from halogen, CN, OH and NH₂;
W₁ and W₂ are each independently selected from a bond, C₁₋₄ alkylene, -O-, - S-, -S(=O)-, -S(=O)₂-, -NR^{W1}-, -CONR^{W1}-, -NR^{W1}CO-, -C(=O)O-, -OC(=O)-, - C(=S)-, -C(=O)- or -(C₁₋₄ alkylene)-NR^{W1}CO-, and the alkylene is optionally further substituted with 1-4 groups selected from halogen, =O, C₁₋₄ alkyl, CN, OH and NH₂;
R^{W1} is selected from H, C₁₋₄ alkyl, or halogen;
each R^{A} is independently selected from halogen, =O, CN, OH, COOH, C₁₋₄ alkyl, C₂₋₆ alkenyl, C₂₋₆ alkynyl, or 5- to 12-membered heteroaryl, and the alkyl, alkenyl, alkynyl and heteroaryl are optionally further substituted with 1-4 groups selected from halogen, OH, NH₂, CN, or -O-halogenated C₁₋₄ alkyl;
alternatively, two R^{A}, together with the atoms to which they are connected, form C₃₋₆ cycloalkyl or 4- to 10-membered heterocycloalkyl, and the cycloalkyl and heterocycloalkyl are optionally further substituted with 1-4 groups selected from halogen, OH, NH₂, CN, or C₁₋₄ alkyl;
each R^{B} is independently selected from halogen, CN, =O, OH, -SF₅, C₁₋₄ alkyl, C₁₋₄ alkoxy, C₂₋₆ alkenyl, or C₂₋₆ alkynyl, and the alkyl, alkoxy, alkenyl and alkynyl are optionally further substituted with a group selected from halogen or C₁₋₂ alkyl;
each R^{C} is independently selected from halogen, CN, =O, OH, C₁₋₄ alkyl, halogenated C₁₋₄ alkyl, C₃₋₅ cycloalkyl, C₂₋₄ alkenyl, C₂₋₄ alkynyl, -SCF₃, -SF₅, or 5- to 6-membered heteroaryl, and the alkyl, cycloalkyl and heteroaryl are optionally further substituted with 1-4 groups selected from halogen, OH, NH₂, CN, or C₁₋₄ alkyl;
alternatively, two R^{C}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, R^{B} and R^{C}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl.
A specific technical solution 2 of the present invention provides a compound as represented by formula (I), or a stereoisomer, a deuterated substance, a solvate, a co-crystal or a pharmaceutically acceptable salt thereof, wherein
A is selected from 5- to 6-membered monocyclic heteroaryl, 6- to 10-membered bicyclic heterocycloalkyl, 9- to 10-membered bicyclic heteroaryl, 10- to 14-membered tricyclic heterocycloalkyl, or 10- to 14-membered tricyclic heteroaryl, and the heterocycloalkyl and heteroaryl are optionally substituted with 1-5 R^{A}; in some embodiments, A is selected from 5- to 6-membered monocyclic heteroaryl, 6- to 10-membered bicyclic heterocycloalkyl, or 10- to 14-membered tricyclic heterocycloalkyl, and the heterocycloalkyl and heteroaryl are optionally substituted with 1-5 R^{A};
each R^{A} is independently selected from halogen, =O, CN, OH, COOH, C₁₋₄ alkyl, C₁₋₄ alkoxy, 5- to 6-membered heteroaryl, -NRₐCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, -SO₂-C₁₋₄ alkyl, -C₃₋₆ cycloalkyl, -NRₐₐ-C₃₋₆ cycloalkyl, 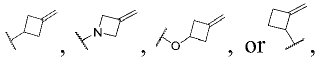 and the alkyl, heteroaryl, cycloalkyl and heterocycloalkyl are optionally further substituted with 1-5 groups selected from halogen, OH, NH₂, CN, C₁₋₄ alkyl, C₁₋₂ alkoxy, -C₁₋₂ alkylhydroxy, -C(=O) C₁₋₂ alkyl, or halogenated C₁₋₂ alkyl;
in some embodiments, each R^{A} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), - C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, - C₃₋₆ cycloalkyl, -NRₐₐ-C₃₋₆ cycloalkyl, 5- to 6-membered heteroaryl, 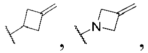 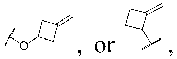 and the alkyl, heteroaryl, cycloalkyl and heterocycloalkyl are optionally further substituted with 1-5 groups selected from halogen, OH, NH₂, CN, C₁₋₄ alkyl, C₁₋₂ alkoxy, or -C₁₋₂ alkylhydroxy;
in some embodiments, each R^{A} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), - C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, or - NRₐₐR_{bb}, and the alkyl, heteroaryl, cycloalkyl and heterocycloalkyl are optionally further substituted with 1-5 groups selected from halogen, OH, NH₂, CN, C₁₋₄ alkyl, or C₁₋₂ alkoxy;
in some embodiments, each R^{A} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₄ alkyl-(5- to 6-membered heteroaryl), or -NRₐₐR_{bb}, and the alkyl, heteroaryl, cycloalkyl and heterocycloalkyl are optionally further substituted with 1-5 groups selected from halogen, OH, NH₂, CN, C₁₋₄ alkyl, or C₁₋₂ alkoxy;
in some embodiments, each R^{A} is independently selected from halogen, =O, CN, OH, COOH, C₁₋₄ alkyl, 5- to 6-membered heteroaryl, -NRaCO-C₃₋₆ cycloalkyl, 4- to 12-membered heterocycloalkyl, or -O-C₃₋₆ cycloalkyl, and the alkyl, heteroaryl, cycloalkyl and heterocycloalkyl are optionally further substituted with 1-5 groups selected from halogen, OH, NH₂, CN, or C₁₋₄ alkyl;
alternatively, two R^{A}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
B is selected from a bond, 4- to 7-membered monocyclic heterocycloalkyl, C₃₋₈ cycloalkyl, 5- to 6-membered heteroaryl, 5- to 6-membered heteroaryl fused 5- to 6-membered heterocycloalkyl, 5- to 12-membered spiro heterocycloalkyl, 3- to 6-membered cycloalkyl fused 5- to 6-membered heterocycloalkyl, or benzo 5- to 6-membered heterocycloalkyl, and the heterocycloalkyl, heteroaryl, cycloalkyl and aryl are optionally substituted with 1-5 R^{B}; in some embodiments, B is selected from a bond, 4- to 7-membered monocyclic heterocycloalkyl, C₃₋₈ cycloalkyl, 5- to 6-membered heteroaryl, 5- to 6-membered heteroaryl fused 5- to 6-membered heterocycloalkyl, 5- to 12-membered spiro heterocycloalkyl, 5- to 6-membered cycloalkyl fused 5- to 6-membered heterocycloalkyl, or benzo 5- to 6-membered heterocycloalkyl, and the heterocycloalkyl, heteroaryl, cycloalkyl and aryl are optionally substituted with 1-5 R^{B};
each R^{B} is independently selected from halogen, =O, or C₁₋₂ alkyl, and the alkyl is optionally further substituted with halogen;
C is selected from 5- to 6-membered monocyclic heteroaryl, phenyl, phenyl fused 4- to 6-membered carbocyclyl, phenyl fused 4- to 6-membered heterocycloalkyl, 5- to 6-membered heterocycloalkyl fused 5- to 6-membered heteroaryl, 5- to 6-membered heteroaryl fused 5- to 6-membered carbocyclyl, 8- to 12-membered tricyclic cycloalkyl, or 8- to 14-membered tricyclic heterocyclyl; the heteroaryl, heterocycloalkyl and heterocyclyl contain 1-4 heteroatoms selected from N, O or S; and the phenyl, heterocycloalkyl and carbocyclyl are optionally substituted with 1-5 R^{C}; in some embodiments, C is selected from 5- to 6-membered monocyclic heteroaryl, phenyl, phenyl fused 4- to 6-membered carbocyclyl, 5- to 6-membered heterocycloalkyl fused 5- to 6-membered heteroaryl, 5- to 6-membered heteroaryl fused 5- to 6-membered carbocyclyl, 8- to 12-membered tricyclic cycloalkyl, or 8- to 14-membered tricyclic heterocyclyl; the heteroaryl, heterocycloalkyl and heterocyclyl contain 1-4 heteroatoms selected from N, O or S; and the phenyl, heterocycloalkyl and carbocyclyl are optionally substituted with 1-5 R^{C}; in some embodiments, C is selected from 5- to 6-membered monocyclic heteroaryl, phenyl fused 4- to 6-membered carbocyclyl, 5-to 6-membered heterocycloalkyl fused 5- to 6-membered heteroaryl, 5- to 6-membered heteroaryl fused 5- to 6-membered carbocyclyl, or 8- to 12-membered tricyclic cycloalkyl; the heterocycloalkyl contains 1-3 heteroatoms selected from N, O or S; and the phenyl, heterocycloalkyl and carbocyclyl are optionally substituted with 1-5 R^{C};
each R^{C} is independently selected from D, halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4-to 12-membered heterocycloalkyl, -N=S(=O)RₐₐR_{bb}, -P(=O)RₐₐR_{bb}, -NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, - C(=O)NRₐ-C₃₋₆ cycloalkyl, -O-C₃₋₆ cycloalkyl, -O-(4- to 12-membered heterocycloalkyl), -O-C₅₋₁₁ spirocycloalkyl, -O-(5- to 6-membered heteroaryl), - NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), - NRₐC(=O)-(5- to 6-membered heteroaryl), -C(=O)NRₐ-(4- to 6-membered heteroaryl), -C(=O)NRₐ-(4- to 6-membered heterocycloalkyl), 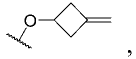 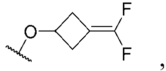 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl, heterocycloalkyl, spirocycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, - NH-C(=O) C₁₋₄ alkyl, or -S(=O)₂C₁₋₄ alkyl;
in some embodiments, each R^{C} is independently selected from D, halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4- to 12-membered heterocycloalkyl, -N=S(=O)RₐₐR_{bb}, - P(=O)RₐₐR_{bb}, -NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4-to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), - NRₐC(=O)-O-Rₐ, -(C₁₋₄ alkyl)ₙ-O-(C₁₋₄ alkyl)ₙ-(4- to 6-membered heterocyclyl), - NRₐC(=O)-(4- to 6-membered heterocycloalkyl), -NRₐC(=O)-(5- to 6-membered heteroaryl), -C(=O)NRₐ-(4- to 6-membered heteroaryl), -C(=O)NRₐ-(4- to 6-membered heterocycloalkyl), 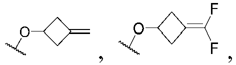 -C(=O)-C₃₋₆ cycloalkyl, - C(=O)-(4- to 12-membered heterocycloalkyl), or -C(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, - NH-C(=O) C₁₋₄ alkyl, or -S(=O)₂C₁₋₄ alkyl;
in some embodiments, each R^{C} is independently selected from halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4- to 12-membered heterocycloalkyl, -N=S(=O)RₐₐR_{bb}, - P(=O)RₐₐR_{bb}, -NRaS(=O)₂C₁₋₄ alkyl, -NRaP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4-to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), - NRₐC(=O)-O-Rₐ, -(C₁₋₄ alkyl)ₙ-O-(C₁₋₄ alkyl)ₙ-(4- to 6-membered heterocyclyl), - NRₐC(=O)-(4- to 6-membered heterocycloalkyl), -NRₐC(=O)-(5- to 6-membered heteroaryl), -C(=O)NRₐ-(4- to 6-membered heteroaryl), -C(=O)NRₐ-(4- to 6-membered heterocycloalkyl), 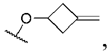 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or - S(=O)₂C₁₋₄ alkyl;
in some embodiments, each R^{C} is independently selected from halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4- to 12-membered heterocycloalkyl, -N=S(=O)RₐₐR_{bb}, - P(=O)RₐₐR_{bb}, -NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4-to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), - NRₐC(=O)-O-Rₐ, -(C₁₋₄ alkyl)ₙ-O-(C₁₋₄ alkyl)ₙ-(4- to 6-membered heterocyclyl), - NRₐC(=O)-(4- to 6-membered heterocycloalkyl), -NRₐC(=O)-(5- to 6-membered heteroaryl), -C(=O)NRₐ-(4- to 6-membered heteroaryl), -C(=O)NRₐ-(4- to 6-membered heterocycloalkyl), or 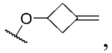 and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4-to 12-membered heterocycloalkyl, -N=S(=O)RₐₐR_{bb}, -P(=O)RₐₐR_{bb}, -NRₐS(=O)₂C₁₋₄ alkyl, -NRₐP(=O)(C₁₋₄ alkyl)2, -NRₐS(=O)₂NRₐₐR_{bb}, -NRₐC(=O)-C₃₋₆ cycloalkyl, - C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), - NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, - (C₁₋₄ alkyl)ₙ-O-(C₁₋₄ alkyl)ₙ-(4- to 6-membered heterocyclyl), -NRₐC(=O)-(4- to 6-membered heterocycloalkyl), or -NRₐC(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C} is independently selected from halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4- to 12-membered heterocycloalkyl, -N=S(=O)RₐₐR_{bb}, -P(=O)RₐₐR_{bb}, -NRₐS(=O)₂C₁₋₄ alkyl, - NRaP(=O)(C₁₋₄ alkyl)2, -NRaS(=O)₂NRₐₐR_{bb}, -NRₐC(=O)-C₃₋₆ cycloalkyl, - C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), or - NRₐS(=O)₂-C₃₋₆ cycloalkyl, and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, =O, -C(=O) C₁₋₄ alkyl, - NH-C(=O) C₁₋₄ alkyl, or -S(=O)₂C₁₋₄ alkyl;
Rₐ is selected from H, halogen, 3- to 6-membered cycloalkyl, C₁₋₂ alkyl, or halogenated 3- to 6-membered cycloalkyl; in some embodiments, Rₐ is selected from H, halogen, 3- to 6-membered cycloalkyl, or C₁₋₂ alkyl;
Rₐₐ and R_{bb} are each independently selected from H, halogen, =O, C₁₋₄ alkyl, C₁₋₄ alkylhydroxy, halogenated C₁₋₄ alkyl, C₁₋₄ alkoxy or halogenated C₁₋₄ alkoxy;
in some embodiments, Rₐₐ and R_{bb} are each independently selected from H, halogen, =O, C₁₋₄ alkyl, halogenated C₁₋₄ alkyl, C₁₋₄ alkoxy or halogenated C₁₋₄ alkoxy;
alternatively, Rₐₐ and R_{bb}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, R^{B} and R^{C}, together with the atoms to which they are connected, form 5- to 6-membered heterocyclyl, and the heterocyclyl is optionally further substituted with 1-4 groups selected from halogen or C₁₋₂ alkyl;
L₁ is selected from a bond, C₁₋₂ alkylene, -C(=O)-C₁₋₂ alkylene-, -N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-C₂₋₄ alkenylene, - C(=O)-NH-, -C(=O)-N(CH₃)-, -C(=O)-O-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, 3- to 6-membered cycloalkyl, 5- to 6-membered heteroaryl, or C₁₋₂ alkyl-C₁₋₂ alkoxy; in some embodiments, L₁ is selected from a bond, C₁₋₂ alkylene, -C(=O)-C₁₋₂ alkylene-, -N(C₁₋₂ alkylene)-C(=O)-C₁₋₂ alkylene-, -NH-C(=O)-C₁₋₂ alkylene-, - S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-C₂₋₄ alkenylene, -C(=O)-NH-, -C(=O)-O-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, 3- to 6-membered cycloalkyl, 5- to 6-membered heteroaryl, or C₁₋₂ alkyl-C₁₋₂ alkoxy; in some embodiments, L₁ is selected from a bond, C₁₋₂ alkylene, -C(=O)-C₁₋₂ alkylene-, - N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-NH-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, 3- to 6-membered cycloalkyl, 5- to 6-membered heteroaryl, or C₁₋₂ alkyl-C₁₋₂ alkoxy;
alternatively, L₁ is 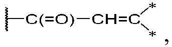 wherein the " * " end is connected to ring B;
L₂ is selected from a bond, C₁₋₂ alkylene, C₂₋₄ alkenylene, C₂₋₄ alkynylene, - C(=O)-NH-, -NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
provided that: when L₂ is selected from -O- or -NH-, L₁ is not -C(=O)-, or B1 is not a bond.

Further, technical solution 2 provides a compound as represented by formula (I), or a stereoisomer, a deuterated substance, a solvate, a co-crystal or a pharmaceutically acceptable salt thereof, wherein
A is selected from 5- to 6-membered monocyclic heteroaryl, 6- to 10-membered bicyclic heterocycloalkyl, or 10- to 14-membered tricyclic heterocycloalkyl, and the heterocycloalkyl and heteroaryl are optionally substituted with 1-5 R^{A};
each R^{A} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 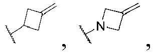 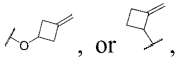 and the alkyl, heteroaryl, cycloalkyl and heterocycloalkyl are optionally further substituted with 1-5 groups selected from halogen, OH, NH₂, CN, C₁₋₄ alkyl, C₁₋₂ alkoxy, -C₁₋₂ alkylhydroxy, -C(=O) C₁₋₂ alkyl, or halogenated C₁₋₂ alkyl; or
each R^{A} is independently selected from halogen, =O, CN, OH, COOH, C₁₋₄ alkyl, 5- to 6-membered heteroaryl, -NRaCO-C₃₋₆ cycloalkyl, 4- to 12-membered heterocycloalkyl, or -O-C₃₋₆ cycloalkyl, and the alkyl, heteroaryl, cycloalkyl and heterocycloalkyl are optionally further substituted with 1-5 groups selected from halogen, OH, NH₂, CN, or C₁₋₄ alkyl;
B is selected from a bond, 4- to 7-membered monocyclic heterocycloalkyl, 3-to 8-membered cycloalkyl, 5- to 6-membered heteroaryl, 5- to 6-membered heteroaryl fused 5- to 6-membered heterocycloalkyl, 5- to 12-membered spiro heterocycloalkyl, 5- to 6-membered cycloalkyl fused 5- to 6-membered heterocycloalkyl, or benzo 5- to 6-membered heterocycloalkyl, and the heterocycloalkyl, heteroaryl, cycloalkyl and aryl are optionally substituted with 1-5 R^{B};
each R^{B} is independently selected from halogen, =O, or C₁₋₂ alkyl, and the alkyl is optionally further substituted with halogen;
C is selected from 5- to 6-membered monocyclic heteroaryl, phenyl fused 4-to 6-membered carbocyclyl, 5- to 6-membered heterocycloalkyl fused 5- to 6-membered heteroaryl, 5- to 6-membered heteroaryl fused 5- to 6-membered carbocyclyl, or 8- to 12-membered tricyclic cycloalkyl; the heterocycloalkyl contains 1-3 heteroatoms selected from N, O or S; and the phenyl, heterocycloalkyl and carbocyclyl are optionally substituted with 1-5 R^{C};
each R^{C} is independently selected from halogen, CN, =O, C₁₋₂ alkyl, halogenated C₁₋₂ alkyl, 5- to 6-membered heteroaryl, 8- to 10-membered bicyclic heteroaryl, 4- to 12-membered heterocycloalkyl, -N=S(=O)RaaRbb, - P(=O)RaaRbb, -NRₐS(=O)₂C₁₋₄ alkyl, -NRaP(=O)(C₁₋₄ alkyl)₂, - NRaS(=O)₂NRaaRbb, -NRₐC(=O)-C₃₋₆ cycloalkyl, or -C(=O)NRₐ-C₃₋₆ cycloalkyl, and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, =O, -C(=O) C₁₋₄ alkyl, or -NH-C(=O) C₁₋₄ alkyl;
Rₐ is selected from H, halogen, 3- to 6-membered cycloalkyl, or C₁₋₂ alkyl;
Raa and Rbb are each independently selected from H, halogen, =O, C₁₋₄ alkyl, halogenated C₁₋₄ alkyl, C₁₋₄ alkoxy or halogenated C₁₋₄ alkoxy;
alternatively, Raa and Rbb, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, R^{B} and R^{C}, together with the atoms to which they are connected, form 5- to 6-membered heterocyclyl, and the heterocyclyl is optionally further substituted with 1-4 groups selected from halogen or C₁₋₂ alkyl;
L₁ is selected from a bond, C₁₋₂ alkylene, -C(=O)-C₁₋₂ alkylene-, -N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-NH-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, 3- to 6-membered cycloalkyl, 5- to 6-membered heteroaryl, or C₁₋₂ alkyl-C₁₋₂ alkoxy;
R^{W1} is selected from H, C₁₋₄ alkyl, or halogen;
alternatively, L₁ is 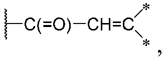 wherein the " * " end is connected to ring B; L₂ is selected from a bond, C₁₋₂ alkylene, C₂₋₄ alkenylene, C₂₋₄ alkynylene, - C(=O)-NH-, -NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
provided that: when L₂ is selected from -O- or -NH-, L₁ is not -C(=O)-, or B1 is not a bond.

Further, technical solution 2 provides a compound as represented by formula (I), or a stereoisomer, a deuterated substance, a solvate, a co-crystal or a pharmaceutically acceptable salt thereof, wherein
A is selected from 5- to 6-membered monocyclic heteroaryl, 6- to 10-membered bicyclic heterocyclyl, or 10- to 14-membered tricyclic heterocyclyl, and the heterocyclyl and heteroaryl are optionally substituted with 1-5 R^{A};
each R^{A} is independently selected from halogen, =O, CN, OH, COOH, C₁₋₄ alkyl, or 5- to 6-membered heteroaryl, and the alkyl and heteroaryl are optionally further substituted with 1-5 groups selected from halogen, OH, NH₂, CN, or C₁₋₄ alkyl;
B is selected from a bond, 4- to 7-membered monocyclic heterocycloalkyl, 3-to 8-membered cycloalkyl, 5- to 6-membered heteroaryl, 5- to 6-membered heteroaryl fused 5- to 6-membered heterocyclyl, 5- to 12-membered spiro heterocycloalkyl, 5- to 6-membered cycloalkyl fused 5- to 6-membered heterocycloalkyl, or benzo 5- to 6-membered heterocyclyl, and the heterocyclyl, heteroaryl, cycloalkyl and aryl are optionally substituted with 1-5 R^{B};
each R^{B} is independently selected from halogen, =O, or C₁₋₂ alkyl, and the alkyl is optionally further substituted with halogen;
C is selected from 5- to 6-membered monocyclic heteroaryl, phenyl fused 4-to 6-membered carbocyclyl, 5- to 6-membered heterocyclyl fused 5- to 6-membered heteroaryl, 5- to 6-membered heteroaryl fused 5- to 6-membered carbocyclyl, or 8- to 12-membered tricyclic cycloalkyl; the heterocyclyl contains 1-3 heteroatoms selected from N, O or S; and the phenyl, heterocyclyl and carbocyclyl are optionally substituted with 1-5 R^{C};
each R^{C} is independently selected from halogen, C₁₋₂ alkyl, halogenated C₁₋₂ alkyl, or 5- to 6-membered heteroaryl, and the alkyl and heteroaryl are optionally further substituted with 1-5 groups selected from halogen or C₁₋₂ alkyl;
alternatively, R^{B} and R^{C}, together with the atoms to which they are connected, form 5- to 6-membered heterocyclyl, and the heterocyclyl is optionally further substituted with 1-4 groups selected from halogen or C₁₋₂ alkyl;
L₁ is selected from a bond, C₁₋₂ alkylene, -C(=O)-C₁₋₂ alkylene-, -N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-NH-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
L₂ is selected from a bond, C₁₋₂ alkylene, C₂₋₄ alkenylene, C₂₋₄ alkynylene, - C(=O)-NH-, -NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
provided that: when L₂ is selected from -O- or -NH-, L₁ is not -C(=O)-, or B1 is not a bond.
A specific technical solution 3 of the present invention is the compound, or the stereoisomer, the deuterated substance, the solvate, the co-crystal or the pharmaceutically acceptable salt thereof described in solution 1 or solution 2, wherein
A is selected from 5- to 6-membered monocyclic heteroaryl, 6- to 10-membered bicyclic heterocyclyl, or 10- to 14-membered tricyclic heterocyclyl, and the heterocyclyl and heteroaryl are optionally substituted with 1-5 R^{A};
each R^{A} is independently selected from halogen, =O, CN, OH, COOH, C₁₋₄ alkyl, or 5- to 6-membered heteroaryl, and the alkyl and heteroaryl are optionally further substituted with 1-5 groups selected from halogen, OH, NH₂, CN, or C₁₋₄ alkyl.
A specific technical solution 4 of the present invention is the compound, or the stereoisomer, the deuterated substance, the solvate, the co-crystal or the pharmaceutically acceptable salt thereof described in solution 1 or solution 2, wherein
B is selected from a bond, 4- to 7-membered monocyclic heterocycloalkyl, 3-to 8-membered cycloalkyl, 5- to 6-membered heteroaryl, 5- to 6-membered heteroaryl fused 5- to 6-membered heterocyclyl, 5- to 12-membered spiro heterocycloalkyl, 5- to 6-membered cycloalkyl fused 5- to 6-membered heterocycloalkyl, or benzo 5- to 6-membered heterocyclyl, and the heterocyclyl, heteroaryl, cycloalkyl and aryl are optionally substituted with 1-5 R^{B};
each R^{B} is independently selected from halogen, =O, or C₁₋₂ alkyl, and the alkyl is optionally further substituted with halogen.
A specific technical solution 5 of the present invention is the compound, or the stereoisomer, the deuterated substance, the solvate, the co-crystal or the pharmaceutically acceptable salt thereof described in solution 1 or solution 2, wherein
C is selected from 5- to 6-membered monocyclic heteroaryl, phenyl fused 4-to 6-membered carbocyclyl, 5- to 6-membered heterocyclyl fused 5- to 6-membered heteroaryl, 5- to 6-membered heteroaryl fused 5- to 6-membered carbocyclyl, or 8- to 12-membered tricyclic cycloalkyl; the heterocyclyl contains 1-3 heteroatoms selected from N, O or S; and the phenyl, heterocyclyl and carbocyclyl are optionally substituted with 1-5 R^{C};
each R^{C} is independently selected from halogen, C₁₋₂ alkyl, halogenated C₁₋₂ alkyl, or 5- to 6-membered heteroaryl, and the alkyl and heteroaryl are optionally further substituted with 1-5 groups selected from halogen or C₁₋₂ alkyl.
A specific technical solution 6 of the present invention is the compound, or the stereoisomer, the deuterated substance, the solvate, the co-crystal or the pharmaceutically acceptable salt thereof described in solution 1 or solution 2, wherein
alternatively, R^{B} and R^{C}, together with the atoms to which they are connected, form 5- to 6-membered heterocyclyl, and the heterocyclyl is optionally further substituted with 1-4 groups selected from halogen or C₁₋₂ alkyl.
A specific technical solution 7 of the present invention is the compound, or the stereoisomer, the deuterated substance, the solvate, the co-crystal or the pharmaceutically acceptable salt thereof described in solution 1 or solution 2, wherein
L₁ is selected from a bond, C₁₋₂ alkylene, -C(=O)-C₁₋₂ alkylene-, -N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-NH-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
L₂ is selected from a bond, C₁₋₂ alkylene, C₂₋₄ alkenylene, C₂₋₄ alkynylene, - C(=O)-NH-, -NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
provided that: when L₂ is selected from -O- or -NH-, L₁ is not -C(=O)-, or B1 is not a bond.

A specific technical solution 8 of the present invention is the compound as represented by formula (I), or the stereoisomer, the deuterated substance, the solvate, the co-crystal or the pharmaceutically acceptable salt thereof described in any one of technical solutions 1 to 7, having a structure of formula (I-1a), (I-2), (I-3), (I-4), (I-5) or (I-1b): 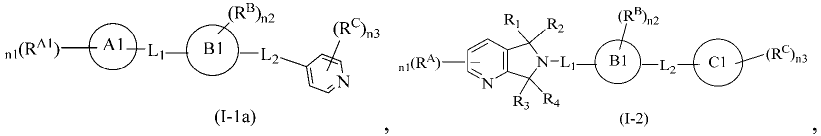 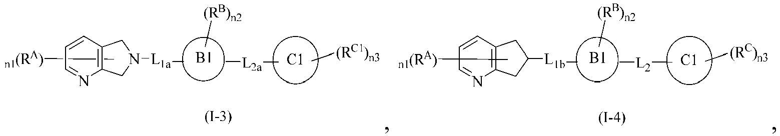 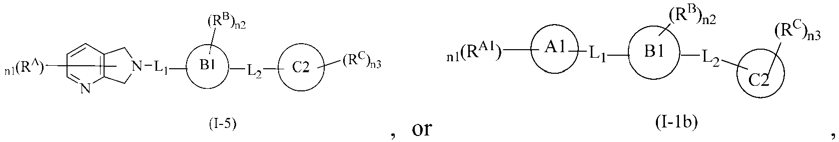 wherein
A1 is selected from the following groups: 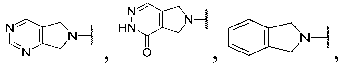 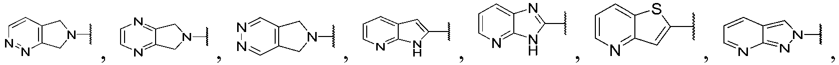 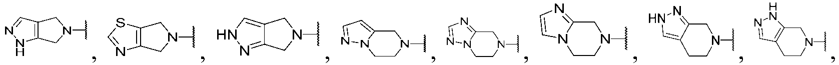 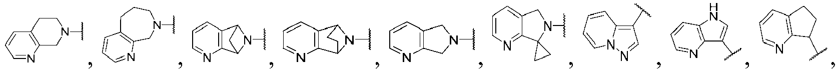 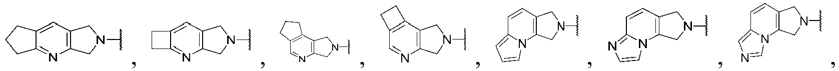 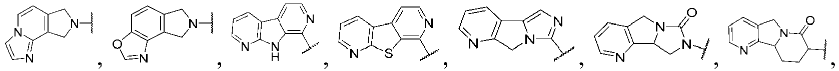 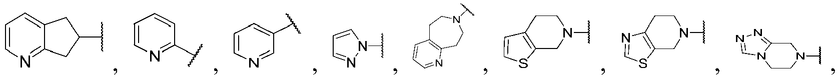 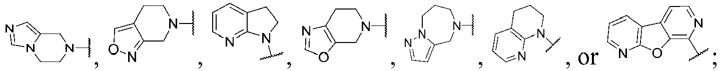
or A1 is selected from the following groups: 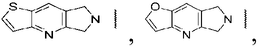 or 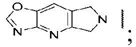
or A1 is selected from the following groups: 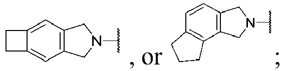
or A1 is selected from the following groups: 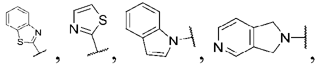 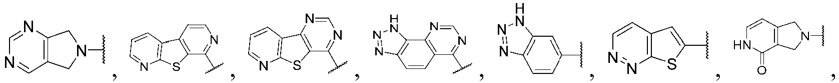 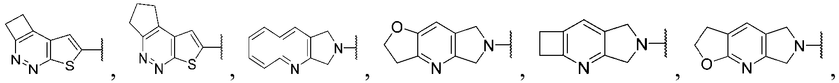 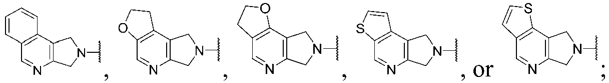
or A1 is selected from the following groups: 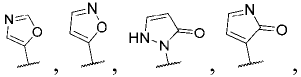 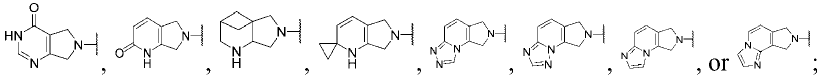
or A1 is selected from the following groups: 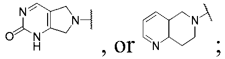
or A1 is the following group: 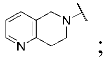
B1 is selected from the following groups: a bond, 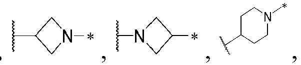 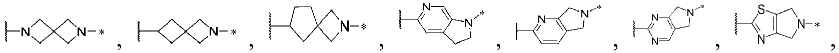 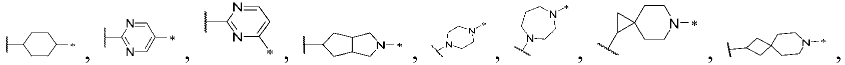 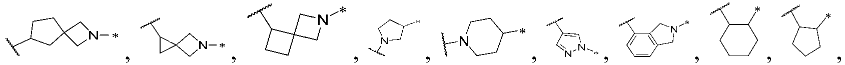 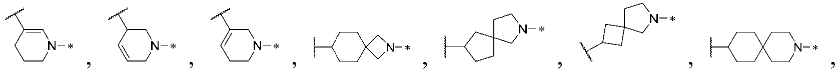 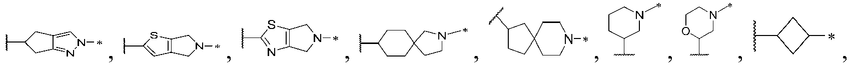 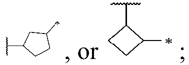
or B1 is the following group: 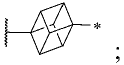
or B1 is selected from the following groups: 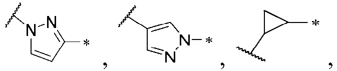 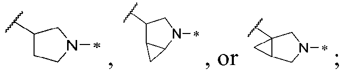
wherein the " 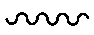 " end is connected to L₁ or L₁ₐ or L_{1b}, and the "*" end is connected to L₂ or L₂ₐ;
C1 is the following group: 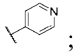
C2 is selected from the following groups: 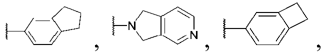 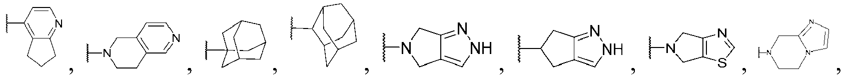 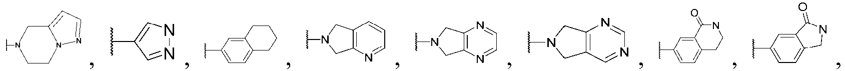 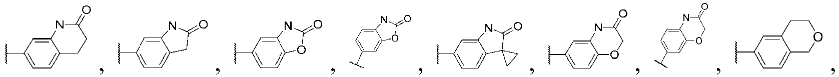 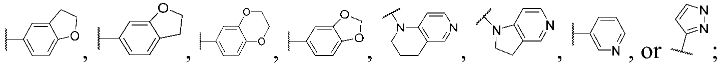
or C2 is selected from the following groups: 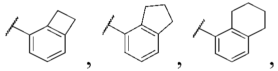 or 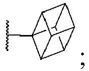
or C2 is selected from the following groups: 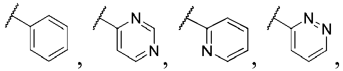 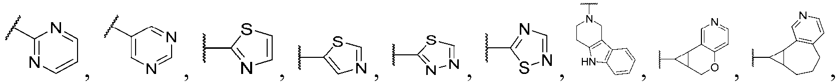 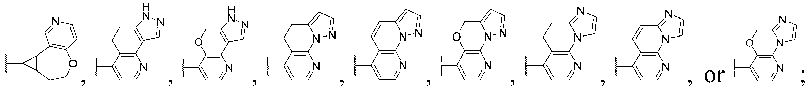 or C2 is selected from the following groups: 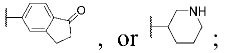 or C2 is the following group: 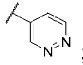 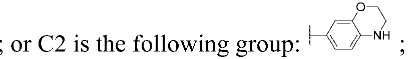
provided that: when C2 is 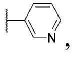 L₂ is not a bond; or when C2 is 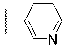 and L₂ is a bond, n3 is not 0 and R^{C} is not F;
R₁, R₂, R₃ and R₄ are each independently selected from H or halogen; provided that R₁, R₂, R₃ and R₄ are not all H;
alternatively, R₃ and R₄ together form =O;
formula (I-3) satisfies one or more of the following conditions:
   (1). L₁ₐ is selected from: a bond, C₁₋₂ alkylene, -C(=O)-CH(CH₃)-, -C(=O)-C(RₐR_{b})-, -N(C₁₋₂ alkylene)-C(=O)-C₁₋₂ alkylene-, -NH-C(=O)-C₁₋₂ alkylene-, - S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-C₂₋₄ alkenylene, -C(=O)-NH-, -C(=O)-N(CH₃)-, -C(=O)-O-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl; in some embodiments, L₁ₐ is selected from: a bond, C₁₋₂ alkylene, -C(=O)-CH(CH₃)-, -C(=O)-C(RₐR_{b})-, - N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-C₂₋₄ alkenylene, - C(=O)-NH-, -C(=O)-O-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl; in some embodiments, L₁ₐ is selected from: a bond, C₁₋₂ alkylene, - C(=O)-CH(CH₃)-, -C(=O)-C(RₐR_{b})-, -N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, 5- to 6-membered heteroaryl-C₁₋₂ alkylene-, 4- to 6-membered monocyclic heterocycloalkyl, C₃₋₆ cycloalkyl, 5- to 6-membered heteroaryl, - C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
      L₁ₐ is connected to ring B1 on the right side;
      Rₐ and R_{b} are each independently selected from H, halogen, 3- to 6-membered cycloalkyl, or C₁₋₂ alkyl; provided that Rₐ and R_{b} are not both H;
      L₂ₐ is selected from a bond, C₂₋₄ alkenylene, C₂₋₄ alkynylene, -C(=O)-NH-, - NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl; provided that when L₁ₐ is -C(=O)-, and L₂ₐ is selected from -NH- or -O-, B1 is not a bond;
   (2). when L₁ₐ is selected from: -C(=O)-CH₂- or -C(=O)-CH₂CH₂-, and L₂ₐ is a bond, at least one R^{C1} is selected from =O, 5- to 6-membered heteroaryl, 8- to 10-membered bicyclic heteroaryl, 4- to 12-membered heterocycloalkyl, - N=S(=O)RaaRbb, -P(=O)RaaRbb, -NRₐS(=O)₂C₁₋₄ alkyl, -NRaP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, - O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, cubane, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocyclyl), -C(=O)NRₐ-(4- to 6-membered heteroaryl), or 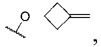 and the alkyl, cycloalkyl, heteroaryl, heterocycloalkyl and cubane are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, when L₁ₐ is selected from: - C(=O)-CH₂- or -C(=O)-CH₂CH₂-, and L₂ₐ is a bond, at least one R^{C1} is selected from =O, 5- to 6-membered heteroaryl, 8- to 10-membered bicyclic heteroaryl, 4-to 12-membered heterocycloalkyl, -N=S(=O)RaaRbb, -P(=O)RaaRbb, - NRaS(=O)₂C₁₋₄ alkyl, -NRaP(=O)(C₁₋₄ alkyl)₂, -NRₐS(=O)₂NRₐₐR_{bb}, - NRaC(=O)-C₃₋₆ cycloalkyl, -C(=O)NRₐ-C₃₋₆ cycloalkyl, -O-C₃₋₆ cycloalkyl, -O-(4-to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, cubane, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), or -NRₐC(=O)-(4- to 6-membered heterocyclyl), and the alkyl, cycloalkyl, heteroaryl, heterocycloalkyl and cubane are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, when L₁ₐ is selected from: - C(=O)-CH₂- or -C(=O)-CH₂CH₂-, and L₂ₐ is a bond, at least one R^{C1} is selected from =O, 5- to 6-membered heteroaryl, 8- to 10-membered bicyclic heteroaryl, 4-to 12-membered heterocycloalkyl, -N=S(=O)RaaRbb, -P(=O)RaaRbb, - NRaS(=O)₂C₁₋₄ alkyl, -NRaP(=O)(C₁₋₄ alkyl)₂, -NRₐS(=O)₂NRₐₐR_{bb}, - NRaC(=O)-C₃₋₆ cycloalkyl, -C(=O)NRₐ-C₃₋₆ cycloalkyl, -O-C₃₋₆ cycloalkyl, -O-(4-to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, or cubane, and the alkyl, cycloalkyl, heteroaryl, heterocycloalkyl and cubane are optionally further substituted with 1-4 groups selected from halogen, OH, NH₂, CN, C₁₋₄ alkyl, halogenated C₁₋₄ alkyl, =O, -C(=O) C₁₋₄ alkyl, -NH-C(=O) C₁₋₄ alkyl, or -S(=O)₂C₁₋₄ alkyl; preferably, when L₁ₐ is selected from: -C(=O)-CH₂- or -C(=O)-CH₂CH₂-, and L₂ₐ is a bond, at least one R^{C1} is selected from 5- to 6-membered heteroaryl, 8- to 10-membered bicyclic heteroaryl, 4- to 12-membered heterocycloalkyl, -N=S(=O)RaaRbb, - P(=O)RaaRbb, -NRₐS(=O)₂C₁₋₄ alkyl, -NRaP(=O)(C₁₋₄ alkyl)₂, - NRaS(=O)₂NRaaRbb, -NRₐC(=O)-C₃₋₆ cycloalkyl, or -C(=O)NRₐ-C₃₋₆ cycloalkyl, and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, =O, -C(=O)C₁₋₄ alkyl, or -NH-C(=O) C₁₋₄ alkyl;
   (3). L₁ₐ is selected from: -C(=O)-CH₂- or -C(=O)-CH₂CH₂-, and L₂ₐ is selected from C₁₋₂ alkylene, C₂₋₄ alkenylene, C₂₋₄ alkynylene, -C(=O)-NH-, -NH-, - O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
   (4). when L₁ₐ is: -C(=O)-CH₂-, and L₂ₐ is a bond, at least one R^{A} is not C₁₋₂ alkyl, and the compound is not 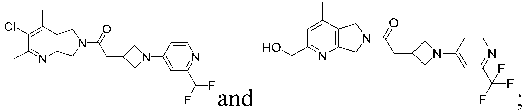
L_{1b} is selected from: a bond, 4- to 6-membered monocyclic heterocycloalkyl, or -C(=O)-NH-, and the heterocycloalkyl is optionally further substituted with 1-4 groups selected from halogen, =O, or C₁₋₂ alkyl;
alternatively, 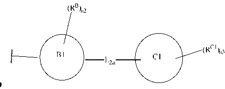 is 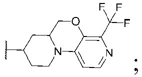
each R^{A} is independently selected from halogen, =O, C₁₋₂ alkyl, -CF₃, 5- to 6-membered heteroaryl, -NRaCO-C₃₋₆ cycloalkyl, 4- to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), - C₁₋₂ alkyl-O-(4- to 12-membered heterocycloalkyl), -C₁₋₂ alkyl-O-C₃₋₆ cycloalkyl, - C₁₋₂ alkyl-OC(=O)-(4- to 12-membered heterocycloalkyl), -C₁₋₂ alkyl-OC(=O)-NRₐₐ-C₃₋₆ cycloalkyl, -NRₐₐR_{bb}, -SO₂-C₁₋₂ alkyl, -C₃₋₆ cycloalkyl, -NRₐₐ-C₃₋₆ cycloalkyl, 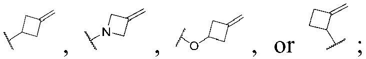 the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, C₁₋₂ alkyl, -C₁₋₂ alkylhydroxy, halogenated C₁₋₂ alkyl, -C(=O)C₁₋₂ alkyl, or C₁₋₂ alkoxy;
in some embodiments, each R^{A} is independently selected from halogen, =O, C₁₋₂ alkyl, -CF₃, 5- to 6-membered heteroaryl, -NRaCO-C₃₋₆ cycloalkyl, 4- to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), -C₁₋₂ alkyl-O-(4- to 12-membered heterocycloalkyl), -C₁₋₂ alkyl-O-C₃₋₆ cycloalkyl, -C₁₋₂ alkyl-OC(=O)-(4- to 12-membered heterocycloalkyl), -C₁₋₂ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 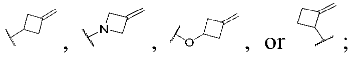 the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, C₁₋₂ alkyl, -C₁₋₂ alkylhydroxy, halogenated C₁₋₂ alkyl, -C(=O) C₁₋₂ alkyl, or C₁₋₂ alkoxy;
in some embodiments, each R^{A} is independently selected from halogen, =O, C₁₋₂ alkyl, 5- to 6-membered heteroaryl, -NRaCO-C₃₋₆ cycloalkyl, 4- to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), -C₁₋₂ alkyl-O-(4- to 12-membered heterocycloalkyl), -C₁₋₂ alkyl-O-C₃₋₆ cycloalkyl, -C₁₋₂ alkyl-OC(=O)-(4- to 12-membered heterocycloalkyl), -C₁₋₂ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 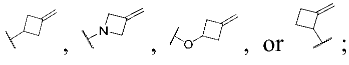 the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, C₁₋₂ alkyl, -C₁₋₂ alkylhydroxy, halogenated C₁₋₂ alkyl, -C(=O) C₁₋₂ alkyl, or C₁₋₂ alkoxy;
in some embodiments, each R^{A} is independently selected from halogen, =O, C₁₋₂ alkyl, 5- to 6-membered heteroaryl, -NRaCO-C₃₋₆ cycloalkyl, 4- to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), -C₁₋₂ alkyl-O-(4- to 12-membered heterocycloalkyl), -C₁₋₂ alkyl-O-C₃₋₆ cycloalkyl, -C₁₋₂ alkyl-OC(=O)-(4- to 12-membered heterocycloalkyl), -C₁₋₂ alkyl-OC(=O)-NRₐₐ-C₃₋₆ cycloalkyl, -NRₐₐR_{bb}, -C₃₋₆ cycloalkyl, -NRₐₐ-C₃₋₆ cycloalkyl, 5- to 6-membered heteroaryl, 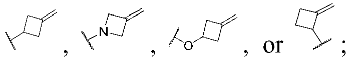 the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, C₁₋₂ alkyl, or - C₁₋₂ alkylhydroxy;
in some embodiments, each R^{A} is independently selected from halogen, =O, C₁₋₂ alkyl, 5- to 6-membered heteroaryl, -NRaCO-C₃₋₆ cycloalkyl, 4- to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), -C₁₋₂ alkyl-O-(4- to 12-membered heterocycloalkyl), -C₁₋₂ alkyl-O-C₃₋₆ cycloalkyl, -C₁₋₂ alkyl-OC(=O)-(4- to 12-membered heterocycloalkyl), -C₁₋₂ alkyl-OC(=O)-NRₐₐ-C₃₋₆ cycloalkyl, or -NRₐₐR_{bb}; the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, or C₁₋₂ alkyl; in some embodiments, each R^{A} is independently selected from halogen, =O, C₁₋₂ alkyl, 5- to 6-membered heteroaryl, -NRaCO-C₃₋₆ cycloalkyl, 4- to 12-membered heterocycloalkyl, -C₁₋₂ alkyl-(5- to 6-membered heteroaryl), or -NRₐₐR_{bb}; the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, or C₁₋₂ alkyl; in some embodiments, each R^{A} is independently selected from =O, C₁₋₂ alkyl, 5- to 6-membered heteroaryl, -NRaCO-C₃₋₆ cycloalkyl, or 4- to 12-membered heterocycloalkyl; the alkyl is optionally further substituted with 1-4 groups selected from NH₂, CN, or C₁₋₂ alkyl; and the heteroaryl, cycloalkyl and heterocycloalkyl are optionally further substituted with 1-4 groups selected from halogen, OH, NH₂, CN, or C₁₋₂ alkyl;
each R^{A1} is independently selected from halogen, =O, CN, OH, COOH, C₁₋₄ alkyl, C₁₋₄ alkoxy, 5- to 6-membered heteroaryl, -SO₂-C₁₋₄ alkyl, -NRaCO-C₃₋₆ cycloalkyl, 4- to 12-membered heterocycloalkyl, -O-C₃₋₆ cycloalkyl, -C₁₋₂ alkyl-(5-to 6-membered heteroaryl), or -NRₐₐR_{bb}, and the alkyl, heteroaryl, cycloalkyl and heterocycloalkyl are optionally further substituted with 1-5 groups selected from halogen, OH, NH₂, CN, C₁₋₄ alkyl, halogenated C₁₋₄ alkyl, or C₁₋₄ alkoxy; in some embodiments, each R^{A1} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₂ alkyl-(5-to 6-membered heteroaryl), or -NRₐₐR_{bb}, and the alkyl, heteroaryl, cycloalkyl and heterocycloalkyl are optionally further substituted with 1-5 groups selected from halogen, OH, NH₂, CN, C₁₋₄ alkyl, or C₁₋₄ alkoxy; in some embodiments, each R^{A1} is independently selected from halogen, =O, CN, OH, COOH, C₁₋₄ alkyl, 5- to 6-membered heteroaryl, -NRaCO-C₃₋₆ cycloalkyl, 4- to 12-membered heterocycloalkyl, or -O-C₃₋₆ cycloalkyl, and the alkyl, heteroaryl, cycloalkyl and heterocycloalkyl are optionally further substituted with 1-5 groups selected from halogen, OH, NH₂, CN, or C₁₋₄ alkyl;
alternatively, two R^{A}, together with the atoms to which they are connected, form C₃₋₆ carbocyclyl or 4- to 6-membered heterocyclyl, and the carbocyclyl and heterocyclyl are optionally further substituted with 1-4 groups selected from halogen, OH, NH₂, CN, or C₁₋₂ alkyl;
each R^{B} is independently selected from halogen, =O, or C₁₋₂ alkyl, and the alkyl is optionally further substituted with halogen;
each R^{C} is independently selected from D, halogen, C₁₋₂ alkyl, C₁₋₂ alkoxy, halogenated C₁₋₂ alkyl, halogenated C₁₋₃ alkoxy, C₃₋₄ cycloalkyl, 5- to 6-membered heteroaryl, CN, =O, -NH-S(=O)₂-C₁₋₂ alkyl, -O-(5- to 6-membered heteroaryl), -O-C₃₋₆ cycloalkyl, -O-(4- to 12-membered heterocycloalkyl), 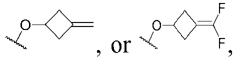 and the alkyl, heteroaryl and cycloalkyl are optionally further substituted with 1-4 groups selected from halogen, C₁₋₂ alkyl, or halogenated C₁₋₂ alkyl;
in some embodiments, each R^{C} is independently selected from D, halogen, C₁₋₂ alkyl, C₁₋₂ alkoxy, halogenated C₁₋₂ alkyl, halogenated C₁₋₂ alkoxy, 5- to 6-membered heteroaryl, CN, =O, -NH-S(=O)₂-C₁₋₂ alkyl, -O-(5- to 6-membered heteroaryl), -O-C₃₋₆ cycloalkyl, -O-(4- to 12-membered heterocycloalkyl), 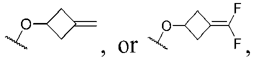 and the alkyl, heteroaryl and cycloalkyl are optionally further substituted with 1-4 groups selected from halogen, C₁₋₂ alkyl, or halogenated C₁₋₂ alkyl;
in some embodiments, each R^{C} is independently selected from D, halogen, C₁₋₂ alkyl, C₁₋₂ alkoxy, halogenated C₁₋₂ alkyl, halogenated C₁₋₂ alkoxy, 5- to 6-membered heteroaryl, CN, =O, -NH-S(=O)₂-C₁₋₂ alkyl, -O-(5- to 6-membered heteroaryl), -O-C₃₋₆ cycloalkyl, -O-(4- to 12-membered heterocycloalkyl), or 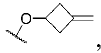 and the alkyl, heteroaryl and cycloalkyl are optionally further substituted with 1-4 groups selected from halogen, C₁₋₂ alkyl, or halogenated C₁₋₂ alkyl;
in some embodiments, each R^{C} is independently selected from halogen, C₁₋₂ alkyl, C₁₋₂ alkoxy, halogenated C₁₋₂ alkyl, halogenated C₁₋₂ alkoxy, 5- to 6-membered heteroaryl, CN, =O, or -NH-S(=O)₂-C₁₋₂ alkyl, and the alkyl and heteroaryl are optionally further substituted with 1-4 groups selected from halogen or C₁₋₂ alkyl;
preferably, each R^{C} is independently selected from halogen, C₁₋₂ alkyl, halogenated C₁₋₂ alkyl, 5- to 6-membered heteroaryl, CN, or =O, and the alkyl and heteroaryl are optionally further substituted with 1-4 groups selected from halogen or C₁₋₂ alkyl;
each R^{C1} is independently selected from halogen, C₁₋₂ alkyl, halogenated C₁₋₂ alkyl, halogenated C₁₋₃ alkoxy, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), - NRₐC(=O)-(5- to 6-membered heteroaryl), -C(=O)NRₐ-(5- to 6-membered heteroaryl), 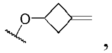 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O)C₁₋₄ alkyl, or -S(=O)₂C₁₋₄ alkyl;
in some embodiments, each R^{C1} is independently selected from halogen, C₁₋₂ alkyl, halogenated C₁₋₂ alkyl, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), - NRₐC(=O)-(5- to 6-membered heteroaryl), -C(=O)NRₐ-(5- to 6-membered heteroaryl), 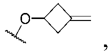 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl;
in some embodiments, each R^{C1} is independently selected from halogen, C₁₋₂ alkyl, halogenated C₁₋₂ alkyl, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), - NRₐC(=O)-(5- to 6-membered heteroaryl), -C(=O)NRₐ-(5- to 6-membered heteroaryl), or 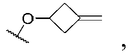 and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C1} is independently selected from halogen, C₁₋₂ alkyl, halogenated C₁₋₂ alkyl, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)2, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), or - NRₐC(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl; in some embodiments, each R^{C1} is independently selected from halogen, C₁₋₂ alkyl, halogenated C₁₋₂ alkyl, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), or -NRₐS(=O)₂-C₃₋₆ cycloalkyl, and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, =O, -C(=O) C₁₋₄ alkyl, -NH-C(=O) C₁₋₄ alkyl, or -S(=O)₂C₁₋₄ alkyl;
preferably, each R^{C1} is independently selected from halogen, C₁₋₂ alkyl, halogenated C₁₋₂ alkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, -N=S(=O)RₐₐR_{bb}, -P(=O)RₐₐR_{bb}, -NRₐS(=O)₂C₁₋₄ alkyl, -NRₐP(=O)(C₁₋₄ alkyl)2, -NRₐS(=O)₂NRₐₐR_{bb}, -NRₐC(=O)-C₃₋₆ cycloalkyl, or -C(=O)NRₐ-C₃₋₆ cycloalkyl, and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, =O, -C(=O)C₁₋₄ alkyl, or -NH-C(=O)C₁₋₄ alkyl;
Rₐₐ and R_{bb} are each independently selected from H, halogen, =O, C₁₋₄ alkyl, C₁₋₄ alkylhydroxy, halogenated C₁₋₄ alkyl, C₁₋₄ alkoxy or halogenated C₁₋₄ alkoxy;
in some embodiments, Rₐₐ and R_{bb} are each independently selected from H, halogen, =O, C₁₋₄ alkyl, halogenated C₁₋₄ alkyl, C₁₋₄ alkoxy or halogenated C₁₋₄ alkoxy;
alternatively, Rₐₐ and R_{bb}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
n1, n2 and n3 are each independently selected from 0, 1, 2, 3, 4 or 5.

Further, the compound, or the stereoisomer, the deuterated substance, the solvate, the co-crystal or the pharmaceutically acceptable salt thereof described in technical solution 8 has a structure of formula (1-1), (I-2), (I-3), (I-4) or (I-5): 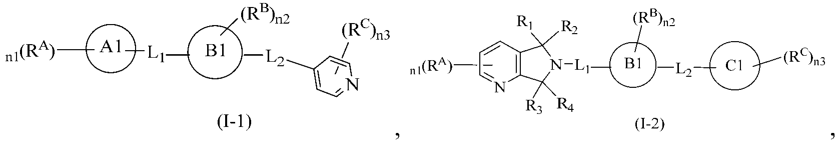 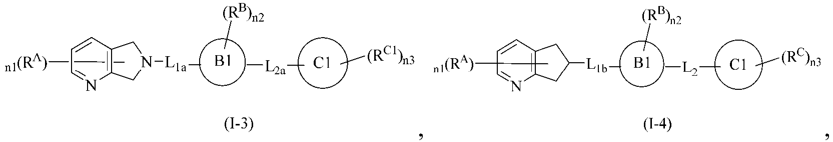 or 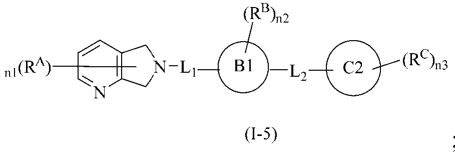 wherein
A1 is selected from the following groups: 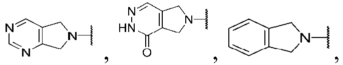 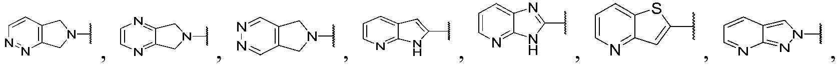 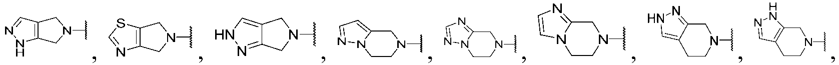 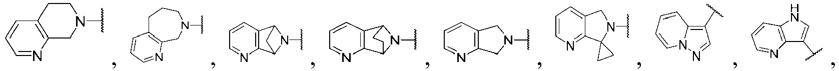 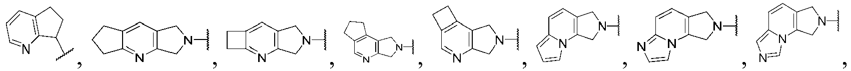 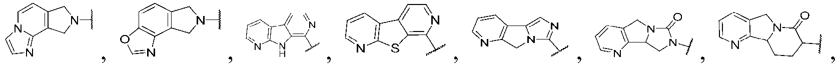 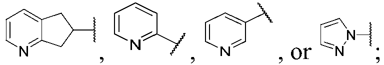
B1 is selected from the following groups: a bond, 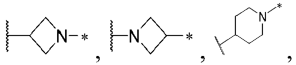 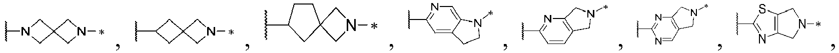 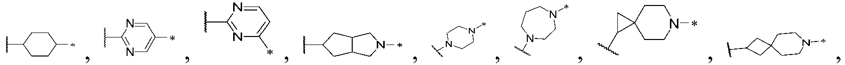 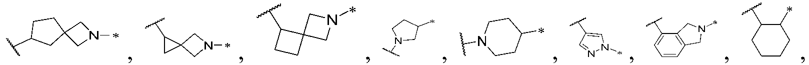 or 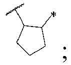
wherein the 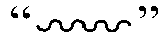 end is connected to L₁, and the "*" end is connected to L₂;
C1 is the following group: 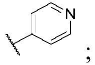
C2 is selected from the following groups: 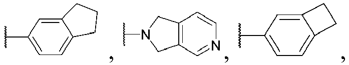 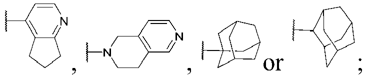
R₁, R₂, R₃ and R₄ are each independently selected from H or halogen; provided that R₁, R₂, R₃ and R₄ are not all H;
alternatively, R₃ and R₄ together form =O;
   (1). L₁ₐ is selected from: a bond, C₁₋₂ alkylene, -C(=O)-CH(CH₃)-, -C(=O)-C(RₐR_{b})-, -N(C₁₋₂ alkylene)-C(=O)-C₁₋₂ alkylene-, -NH-C(=O)-C₁₋₂ alkylene-, - S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
      Rₐ and R_{b} are each independently selected from H, halogen, 3- to 6-membered cycloalkyl, or C₁₋₂ alkyl; provided that Rₐ and R_{b} are not both H;
      L₂ₐ is selected from a bond, C₂₋₄ alkenylene, C₂₋₄ alkynylene, -C(=O)-NH-, - NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl; provided that when L₁ₐ is -C(=O)-, and L₂ₐ is selected from -NH- or -O-, B1 is not a bond;
   (2). when L₁ₐ is selected from: -C(=O)-CH₂- or -C(=O)-CH₂CH₂-, and L₂ₐ is a bond, R^{C1} is 5- to 6-membered heteroaryl, and the heteroaryl is optionally further substituted with 1-4 groups selected from halogen or C₁₋₂ alkyl;
   (3). when L₁ₐ is selected from: -C(=O)-CH₂- or -C(=O)-CH₂CH₂-, and R^{C1} is - CF₃, L₂ₐ is not a bond;
L_{1b} is selected from: a bond, 4- to 6-membered monocyclic heterocycloalkyl, or -C(=O)-NH-, and the heterocycloalkyl is optionally further substituted with 1-4 groups selected from halogen, =O, or C₁₋₂ alkyl;
alternatively, 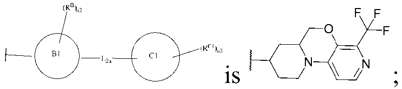
each R^{A} is independently selected from halogen, =O, C₁₋₂ alkyl, or 5- to 6-membered heteroaryl, and the alkyl is optionally further substituted with 1-4 groups selected from halogen, OH, NH₂, CN, or C₁₋₂ alkyl;
each R^{B} is independently selected from halogen, =O, or C₁₋₂ alkyl, and the alkyl is optionally further substituted with halogen;
each R^{C} is independently selected from halogen, halogenated C₁₋₂ alkyl, or 5-to 6-membered heteroaryl, and the alkyl and heteroaryl are optionally further substituted with 1-4 groups selected from halogen or C₁₋₂ alkyl;
n1, n2 and n3 are each independently selected from 0, 1, 2, 3, 4 or 5.

A specific technical solution 9 of the present invention is the compound as represented by formula (I-1a), (I-2), (I-3), (I-4) or (I-5), or the stereoisomer, the deuterated substance, the solvate, the co-crystal or the pharmaceutically acceptable salt thereof described in solution 7 or solution 8, wherein
each R^{A} is independently selected from F, Cl, =O, CN, methyl, ethyl, - NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), - C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), - C₁₋₂ alkyl-OC(=O)-NH-C₃₋₆ cycloalkyl, -NHRₐₐ, cyclopropyl, cyclobutyl, -NH-cyclopropyl, -NH-cyclobutyl, 5- to 6-membered heteroaryl, 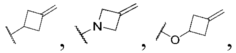 or 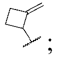 the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, ethyl, -CHF₂, -CH₂F, -CF₃, -C(=O) CH₃, -O-CH₃, or -O-CH₂CH₃;
in some embodiments, each R^{A} is independently selected from F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), -C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), -C₁₋₂ alkyl-OC(=O)-NH-C₃₋₆ cycloalkyl, or -NHRₐₐ; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, or ethyl; in some embodiments, each R^{A} is independently selected from F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), or -NHRₐₐ; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, or ethyl; in some embodiments, each R^{A} is independently selected from =O, CN, methyl, ethyl, -NHCO-C₃₋₆ cycloalkyl, 4- to 6-membered monocyclic heterocycloalkyl, 5-membered heteroaryl, or 6-membered heteroaryl; the methyl and ethyl are optionally further substituted with 1-4 groups selected from NH₂, CN, methyl, or ethyl; and the heteroaryl, cycloalkyl and heterocycloalkyl are optionally further substituted with 1-4 groups selected from F, Cl, OH, NH₂, CN, methyl, or ethyl;
alternatively, two R^{A}, together with the atoms to which they are connected, form 5- to 6-membered heterocyclyl, and the heterocyclyl is optionally further substituted with 1-4 groups selected from F, Cl, OH, NH₂, CN, methyl, or ethyl;
each R^{A1} is independently selected from F, Cl, =O, CN, OH, COOH, methyl, ethyl, -O-C₃₋₆ cycloalkyl, or -NHRₐₐ, and the methyl, ethyl and cycloalkyl are optionally further substituted with 1-5 groups selected from F, Cl, OH, NH₂, CN, methyl, ethyl, or methoxy; in some embodiments, each R^{A1} is independently selected from F, Cl, =O, CN, OH, COOH, methyl, ethyl, or -O-C₃₋₆ cycloalkyl, and the methyl, ethyl and cycloalkyl are optionally further substituted with 1-5 groups selected from F, Cl, OH, NH₂, CN, methyl, or ethyl;
each R^{B} is independently selected from F, Cl, =O, methyl, or ethyl, and the methyl and ethyl are optionally further substituted with a group selected from F or Cl;
each R^{C} is independently selected from F, Cl, methyl, ethyl, -CHF₂, -CH₂F, - CF₃, -CH₂CF₃, -CH₂CHF₂, -CH₂CH₂F, -OCHF₂, -OCH₂F, -OCF₃, -OCH₂CF₃, - OCH(CH₃)CF₃, cyclopropyl, cyclobutyl, 5-membered heteroaryl, 6-membered heteroaryl, -CN, =O, or -NH-S(=O)₂-C₁₋₂ alkyl, and the methyl, ethyl, cyclopropyl, cyclobutyl, heteroaryl and heterocycloalkyl are optionally further substituted with 1-4 groups selected from F, Cl, methyl, or ethyl;
each R^{C1} is independently selected from F, Cl, methyl, ethyl, -CHF₂, -CH₂F, - CF₃, -CH₂CF₃, -CH₂CHF₂, -CH₂CH₂F, -OCHF₂, -OCH₂F, -OCF₃, -OCH₂CF₃, - OCH(CH₃)CF₃, cyclopropyl, cyclobutyl, cyclopentyl, cubane, 5-membered heteroaryl, 6-membered heteroaryl, 8-membered fused heteroaryl, 9-membered fused heteroaryl, 10-membered fused heteroaryl, 7- to 8-membered spiro heterocycloalkyl, 8- to 9-membered fused heterocycloalkyl, 7-membered bridged heterocycloalkyl, 4- to 6-membered monocyclic heterocycloalkyl, -N=S(=O)RₐₐR_{bb}, -P(=O)RₐₐR_{bb}, -NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4-to 6-membered heterocycloalkyl), -O-(5- to 6-membered heteroaryl), -NH-C₃₋₆ cycloalkyl, -NH-(4- to 6-membered heterocycloalkyl), -NH-(5- to 6-membered heteroaryl), -NH-S(=O)₂-C₃₋₆ cycloalkyl, -C₁₋₂ alkyl-(5-membered heteroaryl), -C₁₋₂ alkyl-(6-membered heteroaryl), -C₁₋₂ alkyl-phenyl, -C₁₋₂ alkyl-(4- to 6-membered heterocycloalkyl), -NRₐC(=O)-O-Rₐ, -(C₁₋₂ alkyl)ₙ-O-(C₁₋₂ alkyl)ₙ-(4- to 6-membered heterocyclyl), or -NRₐC(=O)-(4- to 6-membered heterocyclyl), and the alkyl, cycloalkyl, heteroaryl, heterocycloalkyl and cubane are optionally further substituted with 1-4 groups selected from F, Cl, OH, NH₂, CN, methyl, ethyl, deuterated methyl, -CHF₂, -CH₂F, -CF₃, -CH₂CF₃, -CH₂CHF₂, -CH₂CH₂F, =O, - C(=O) C₁₋₄ alkyl, -NH-C(=O) C₁₋₄ alkyl, or -S(=O)₂C₁₋₂ alkyl; in some embodiments, each R^{C1} is independently selected from F, Cl, methyl, ethyl, -CHF₂, -CH₂F, -CF₃, -CH₂CF₃, -CH₂CHF₂, -CH₂CH₂F, C₄₋₅ cycloalkyl, cubane, 5-membered heteroaryl, 6-membered heteroaryl, 8-membered fused heteroaryl, 9-membered fused heteroaryl, 10-membered fused heteroaryl, 7- to 8-membered spiro heterocycloalkyl, 8- to 9-membered fused heterocycloalkyl, 7-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)2, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 6-membered heterocycloalkyl), -O-(5- to 6-membered heteroaryl), -NH-C₃₋₆ cycloalkyl, -NH-(4- to 6-membered heterocycloalkyl), -NH-(5- to 6-membered heteroaryl), or -NH-S(=O)₂-C₃₋₆ cycloalkyl, and the cyclopropyl, cyclobutyl, cyclopentyl, alkyl, cycloalkyl, heteroaryl, heterocycloalkyl and cubane are optionally further substituted with 1-4 groups selected from F, Cl, OH, NH₂, CN, methyl, ethyl, -CHF₂, -CH₂F, -CF₃, -CH₂CF₃, - CH₂CHF₂, -CH₂CH₂F, =O, -C(=O) C₁₋₄ alkyl, -NH-C(=O) C₁₋₄ alkyl, or -S(=O)₂C₁₋₂ alkyl;
Rₐ is selected from H, F, Cl, methyl, ethyl, cyclopropyl, cyclobutyl, cyclopentyl, or difluorocyclobutyl; in some embodiments, Rₐ is selected from H, F, Cl, methyl, ethyl, cyclopropyl, cyclobutyl, or cyclopentyl;
Rₐₐ and R_{bb} are each independently selected from H, F, Cl, =O, methyl, ethyl, -CHF₂, -CH₂F, -CF₃, -CH₂CF₃, -CH₂CHF₂, -CH₂CH₂F, -OCH₃, -OCH₂CH₃, - OCHF₂, -OCH₂F, -OCF₃, -OCH₂CF₃, -OCH₂CHF₂, or -OCH₂CH₂F;
alternatively, Rₐₐ and R_{bb}, together with the atoms to which they are connected, form 4- to 6-membered heterocyclyl, and the heterocyclyl is optionally further substituted with 1-4 groups selected from F, Cl, OH, NH₂, CN, methyl, or ethyl;
n1 is selected from 1, 2 or 3; in some embodiments, n1 is selected from 1, 2 or 3;
n2 is selected from 0 or 1;
n3 is selected from 0, 1 or 2;
L₁ is selected from a bond, vinylene, -CH₂-, -CH₂-CH₂-, 4- to 6-membered monocyclic heterocycloalkyl, (4- to 6-membered monocyclic heterocycloalkyl)-C(=O)-CH₂-, -(4- to 6-membered monocyclic heterocycloalkyl)-CH₂-, -CH₂-(4- to 6-membered monocyclic heterocycloalkyl)-, -CH(CH₃)NHC(=O)-CH₂-, - CH₂NHC(=O)-CH₂-, -S(=O)₂-CH₂-, -C(=S)-CH₂-, -C(=O)-C₃₋₆ cycloalkyl, - C(=O)-CH(CH₃)-, -C(=O)-CH(C₃₋₆ cycloalkyl)-, -C(=O)-CF₂-, -C(=O)-C(CH₃)₂-, - CH₂-C(=O)-, -C(=O)-C(=O)-, C₃₋₆ cycloalkyl, -(5- to 6-membered heteroaryl)-CH₂-, 5- to 6-membered heteroaryl, -C(=O)-, -C(=O)-CH₂-, -N(CH₃)-CH₂-, -CF₂-CH₂-, -C(=O)-C₂₋₄ alkenylene, or -C(=O)-O-, and the -CH₂-, alkenylene, cycloalkyl, heterocycloalkyl and heteroaryl are optionally further substituted with 1-3 R^{L1}; each R^{L1} is independently selected from F, Cl, Br, =O, methyl, ethyl, -CF₃, -CHF₂, -CH₂F, vinyl, propenyl, methoxy, ethoxy, -OCF₃, -OCHF₂, -OCH₂F, cyclopropyl, cyclobutyl, methyl-methoxy, or 5-membered heteroaryl; in some embodiments, L₁ is selected from a bond, vinylene, -CH₂-, -CH₂-CH₂-, 4- to 6-membered monocyclic heterocycloalkyl, (4- to 6-membered monocyclic heterocycloalkyl)-C(=O)-CH₂-, -(4- to 6-membered monocyclic heterocycloalkyl)-CH₂-, -CH₂-(4- to 6-membered monocyclic heterocycloalkyl)-, - CH(CH₃)NHC(=O)-CH₂-, -CH₂NHC(=O)-CH₂-, -S(=O)₂-CH₂-, -C(=S)-CH₂-, - C(=O)-C₃₋₆ cycloalkyl, -C(=O)-CH(CH₃)-, -C(=O)-CH(C₃₋₆ cycloalkyl)-, -C(=O)-CF₂-, -C(=O)-C(CH₃)₂-, -CH₂-C(=O)-, -C(=O)-C(=O)-, C₃₋₆ cycloalkyl, -(5- to 6-membered heteroaryl)-CH₂-, 5- to 6-membered heteroaryl, -C(=O)-, -C(=O)-CH₂-, -N(CH₃)-CH₂-, or -CF₂-CH₂-, and the -CH₂-, cycloalkyl, heterocycloalkyl and heteroaryl are optionally further substituted with 1-3 R^{L1}; each R^{L1} is independently selected from F, Cl, Br, =O, methyl, ethyl, -CF₃, -CHF₂, -CH₂F, vinyl, propenyl, methoxy, ethoxy, -OCF₃, -OCHF₂, -OCH₂F, cyclopropyl, cyclobutyl, methyl-methoxy, or 5-membered heteroaryl;
alternatively, L₁ is 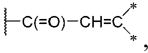 wherein the "* " end is connected to ring B;
L₂ is selected from a bond, vinylene, ethynylene, -CH₂-, -CH₂-CH₂-, -O-, - NH-, -C(=O)-, or -N(CH₃)-.

Further, in the compound as represented by formula (I), or the stereoisomer, the deuterated substance, the solvate, the co-crystal or the pharmaceutically acceptable salt thereof described in technical solution 9,
each R^{A} is independently selected from =O, methyl, ethyl, -NHCO-C₃₋₆ cycloalkyl, 4- to 6-membered monocyclic heterocycloalkyl, 5-membered heteroaryl, or 6-membered heteroaryl; the methyl and ethyl are optionally further substituted with 1-4 groups selected from NH₂, CN, methyl, or ethyl; and the heteroaryl, cycloalkyl and heterocycloalkyl are optionally further substituted with 1-4 groups selected from F, Cl, OH, NH₂, CN, methyl, or ethyl;
each R^{A1} is independently selected from F, Cl, =O, CN, OH, COOH, methyl, ethyl, or -O-C₃₋₆ cycloalkyl, and the methyl, ethyl and cycloalkyl are optionally further substituted with 1-5 groups selected from F, Cl, OH, NH₂, CN, methyl, or ethyl;
each R^{B} is independently selected from F, Cl, =O, methyl, or ethyl, and the methyl and ethyl are optionally further substituted with a group selected from F or Cl;
each R^{C} is independently selected from F, Cl, methyl, ethyl, -CHF₂, -CH₂F, - CF₃, -CH₂CF₃, -CH₂CHF₂, -CH₂CH₂F, 5-membered heteroaryl, 6-membered heteroaryl, CN, or =O, and the methyl, ethyl and heteroaryl are optionally further substituted with 1-4 groups selected from F, Cl, methyl, or ethyl;
each R^{C1} is independently selected from F, Cl, methyl, ethyl, -CHF₂, -CH₂F, - CF₃, -CH₂CF₃, -CH₂CHF₂, -CH₂CH₂F, 5-membered heteroaryl, 6-membered heteroaryl, 8-membered fused heteroaryl, 9-membered fused heteroaryl, 10-membered fused heteroaryl, 7- to 8-membered spiro heterocycloalkyl, 8- to 9-membered fused heterocycloalkyl, 7-membered bridged heterocycloalkyl, 4- to 6-membered monocyclic heterocycloalkyl, -N=S(=O)RₐₐR_{bb}, -P(=O)RₐₐR_{bb}, - NRₐS(=O)₂C₁₋₄ alkyl, -NRₐP(=O)(C₁₋₄ alkyl)2, -NRₐS(=O)₂NRₐₐR_{bb}, -NRₐC(=O)-C₃₋₆ cycloalkyl, or -C(=O)NRₐ-C₃₋₆ cycloalkyl, and the alkyl, cycloalkyl, heteroaryl and heterocycloalkyl are optionally further substituted with 1-4 groups selected from F, Cl, OH, NH₂, CN, methyl, ethyl, -CHF₂, -CH₂F, -CF₃, -CH₂CF₃, - CH₂CHF₂, -CH₂CH₂F, =O, -C(=O) C₁₋₄ alkyl, or -NH-C(=O) C₁₋₄ alkyl;
Rₐ is selected from H, F, Cl, methyl, ethyl, cyclopropyl, cyclobutyl, or cyclopentyl;
Rₐₐ and R_{bb} are each independently selected from H, F, Cl, =O, methyl, ethyl, -CHF₂, -CH₂F, -CF₃, -CH₂CF₃, -CH₂CHF₂, -CH₂CH₂F, -OCH₃, -OCH₂CH₃, - OCHF₂, -OCH₂F, -OCF₃, -OCH₂CF₃, -OCH₂CHF₂, or -OCH₂CH₂F;
alternatively, Rₐₐ and R_{bb}, together with the atoms to which they are connected, form 4- to 6-membered heterocyclyl, and the heterocyclyl is optionally further substituted with 1-4 groups selected from F, Cl, OH, NH₂, CN, methyl, or ethyl; n1 is selected from 1, 2 or 3;
n2 is selected from 0 or 1;
n3 is selected from 0, 1 or 2;
L₁ is selected from a bond, vinylene, -CH₂-, -CH₂-CH₂-, 4- to 6-membered monocyclic heterocycloalkyl, (4- to 6-membered monocyclic heterocycloalkyl)-C(=O)-CH₂-, -(4- to 6-membered monocyclic heterocycloalkyl)-CH₂-, -CH₂-(4- to 6-membered monocyclic heterocycloalkyl)-, -CH(CH₃)NHC(=O)-CH₂-, - CH₂NHC(=O)-CH₂-, -S(=O)₂-CH₂-, -C(=S)-CH₂-, -C(=O)-C₃₋₆ cycloalkyl, - C(=O)-CH(CH₃)-, -C(=O)-CH(C₃₋₆ cycloalkyl)-, -C(=O)-CF₂-, -C(=O)-C(CH₃)₂-, - CH₂-C(=O)-, -C(=O)-C(=O)-, C₃₋₆ cycloalkyl, -(5- to 6-membered heteroaryl)-CH₂-, 5- to 6-membered heteroaryl, -C(=O)-, -C(=O)-CH₂-, -N(CH₃)-CH₂-, or - CF₂-CH₂-, and the -CH₂-, cycloalkyl, heterocycloalkyl and heteroaryl are optionally further substituted with 1-3 R^{L1}; each R^{L1} is independently selected from F, Cl, Br, =O, methyl, ethyl, -CF₃, -CHF₂, -CH₂F, vinyl, propenyl, methoxy, ethoxy, -OCF₃, -OCHF₂, -OCH₂F, cyclopropyl, cyclobutyl, methyl-methoxy, or 5-membered heteroaryl;
alternatively, L₁ is 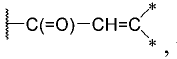 wherein the "* " end is connected to ring B;
L₂ is selected from a bond, vinylene, ethynylene, -CH₂-, -CH₂-CH₂-, -O-, - NH-, -C(=O)-, or -N(CH₃)-.

Further, in the compound, or the stereoisomer, the deuterated substance, the solvate, the co-crystal or the pharmaceutically acceptable salt thereof described in technical solution 9,
each R^{A} is independently selected from F, Cl, =O, CN, methyl, ethyl, - NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), - C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), - C₁₋₂ alkyl-OC(=O)-NH-C₃₋₆ cycloalkyl, -NHRₐₐ, cyclopropyl, cyclobutyl, -NH-cyclopropyl, -NH-cyclobutyl, 5- to 6-membered heteroaryl, 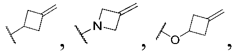 or 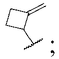 the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, or ethyl;
in some embodiments, each R^{A} is independently selected from F, Cl, =O, methyl, ethyl, 5-membered heteroaryl, or 6-membered heteroaryl, and the methyl, ethyl, 5-membered heteroaryl and 6-membered heteroaryl are optionally further substituted with 1-4 groups selected from F, Cl, methyl, or ethyl;
each R^{B} is independently selected from F, Cl, =O, methyl, or ethyl, and the methyl and ethyl are optionally further substituted with a group selected from F or Cl;
each R^{C} is independently selected from F, Cl, -CF₃, -CHF₂, -CH₂CH₂F, - CH₂CF₃, -CH₂CHF₂, -CH₂CH₂F, 5-membered heteroaryl, or 6-membered heteroaryl, and the heteroaryl is optionally further substituted with 1-4 groups selected from F, Cl, methyl, or ethyl;
n1 is selected from 1, 2 or 3;
n2 is selected from 0 or 1;
n3 is selected from 0, 1 or 2;
L₁ is selected from a bond, vinylene, -CH₂-, 4- to 6-membered monocyclic heterocycloalkyl, 4- to 6-membered monocyclic heterocycloalkyl-C(=O)-CH₂-, -4-to 6-membered monocyclic heterocycloalkyl-CH₂-, -CH₂-4- to 6-membered monocyclic heterocycloalkyl-, -CH(CH₃)NHC(=O)-CH₂-, -CH₂NHC(=O)-CH₂-, - S(=O)₂-CH₂-, -C(=S)-CH₂-, -C(=O)-C₃₋₆ cycloalkyl, -C(=O)-CH(CH₃)-, -C(=O)-CH(C₃₋₆ cycloalkyl)-, -C(=O)-CF₂-, -C(=O)-C(CH₃)₂-, -CH₂-C(=O)-, -C(=O)-C(=O)-, C₃₋₆ cycloalkyl, -5- to 6-membered heteroaryl-CH₂-, -5- to 6-membered heteroaryl-, -C(=O)-, -C(=O)-CH₂-, or N(CH₃)-CH₂-, and the -CH₂-, cycloalkyl, heterocycloalkyl and heteroaryl are optionally further substituted with 1-3 R^{L1}; each R^{L1} is independently selected from F, Cl, Br, =O, methyl, ethyl, -CF₃, -CHF₂, -CH₂F, vinyl, propenyl, methoxy, ethoxy, -OCF₃, -OCHF₂, -OCH₂F, cyclopropyl, or cyclobutyl;
L₂ is selected from a bond, vinylene, ethynylene, -O-, -NH-, -C(=O)-, or - N(CH₃)-.

Further, the compound, or the stereoisomer, the deuterated substance, the solvate, the co-crystal or the pharmaceutically acceptable salt thereof described in technical solution 10 has a structure of formula (1-6), (I-7) or (I-8): 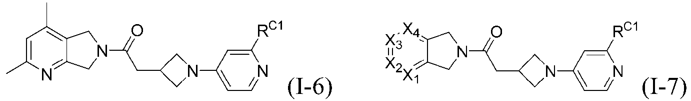 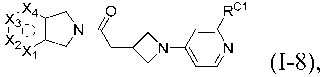 wherein
each R^{C1} is independently selected from halogenated C₁₋₂ alkyl, 5- to 6-membered heteroaryl, -O-C₃₋₆ cycloalkyl, -O-4- to 6-membered heterocycloalkyl, or -O-5- to 6-membered heteroaryl, and the heteroaryl, cycloalkyl and heterocycloalkyl are optionally further substituted with 1-4 groups selected from halogen, OH, NH₂, CN, C₁₋₄ alkyl, halogenated C₁₋₄ alkyl, =O, or -C(=O) C₁₋₄ alkyl; preferably, each R^{C1} is independently selected from 5-membered heteroaryl, 6-membered heteroaryl, or -O-C₃₋₆ cycloalkyl, and the heteroaryl and cycloalkyl are optionally further substituted with 1-4 groups selected from halogen, OH, NH₂, CN, C₁₋₄ alkyl, halogenated C₁₋₄ alkyl, =O, or -C(=O) C₁₋₄ alkyl; preferably, each R^{C1} is independently selected from 5-membered heteroaryl, -O-cyclopropyl, -O-cyclobutyl, or -O-cyclopentyl, and the pyrrolyl, imidazolyl, pyrazolyl, thiazolyl, cyclopropyl, cyclobutyl and cyclopentyl are optionally further substituted with 1-4 groups selected from halogen, OH, NH₂, CN, C₁₋₂ alkyl, halogenated C₁₋₂ alkyl, =O, or -C(=O) C₁₋₂ alkyl; more preferably, each R^{C1} is independently selected from halogenated C₁₋₂ alkyl, pyrrolyl, imidazolyl, pyrazolyl, thiazolyl, triazolyl, -O-cyclopropyl, -O-cyclobutyl, -O-cyclopentyl, -O-bicyclo[1.1.1]pentane, -O-spiro[2.3]hexane, or -O-oxolanyl, and the pyrrolyl, imidazolyl, pyrazolyl, thiazolyl, cyclopropyl, cyclobutyl, cyclopentyl, bicyclo[1.1.1]pentane, spiro[2.3]hexane and oxolanyl are optionally further substituted with 1-4 groups selected from F, Cl, OH, NH₂, CN, methyl, ethyl, -CHF₂, -CH₂F, -CF₃, -CH₂CF₃, -CH₂CHF₂, -CH₂CH₂F, or =O; more preferably, each R^{C1} is independently selected from pyrrolyl, imidazolyl, pyrazolyl, thiazolyl, -O-cyclopropyl, -O-cyclobutyl, or -O-cyclopentyl, and the pyrrolyl, imidazolyl, pyrazolyl, thiazolyl, cyclopropyl, cyclobutyl and cyclopentyl are optionally further substituted with 1-4 groups selected from F, Cl, OH, NH₂, CN, methyl, ethyl, -CHF₂, -CH₂F, -CF₃, -CH₂CF₃, -CH₂CHF₂, -CH₂CH₂F, or =O; more preferably, each R^{C1} is independently selected from -CF₃, 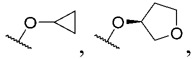 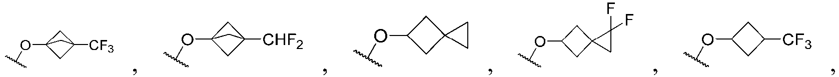 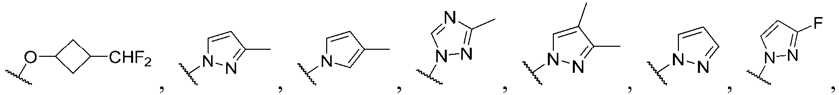 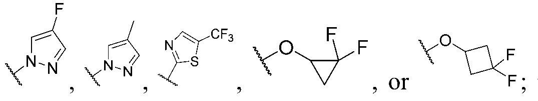 more preferably, each R^{C1} is independently selected from 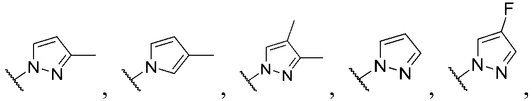 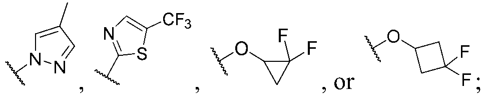
X₁ is selected from CR^{X1} or N; or X₁ is NR^{X1};
X₂ is selected from CR^{X2} or N;
X₃ is selected from CR^{X3} or N;
X₄ is selected from CR^{X4} or N;
R^{X1}, R^{X2}, R^{X3} and R^{X4} are each independently selected from H, halogen, =O, CN, OH, COOH, C₁₋₄ alkyl, C₁₋₄ alkoxy, C₂₋₆ alkenyl, C₂₋₆ alkynyl, 5- to 12-membered heteroaryl, -SO₂-C₁₋₄ alkyl, -NRaCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, or -NRₐₐR_{bb}, and the alkyl, alkenyl, alkynyl, heteroaryl, cycloalkyl and heterocycloalkyl are optionally further substituted with 1-4 groups selected from halogen, OH, NH₂, CN, C₁₋₄ alkoxy, -O-halogenated C₁₋₄ alkyl, or C₁₋₄ alkyl; preferably, R^{X1}, R^{X2}, R^{X3} and R^{X4} are each independently selected from H, halogen, =O, CN, OH, COOH, C₁₋₄ alkyl, C₁₋₄ alkoxy, C₂₋₆ alkenyl, C₂₋₆ alkynyl, 5- to 12-membered heteroaryl, -NRaCO-C₃₋₆ cycloalkyl, 4- to 12-membered heterocycloalkyl, -O-C₃₋₆ cycloalkyl, -C₁₋₄ alkyl-(5-to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, or - NRₐₐR_{bb}, and the alkyl, alkenyl, alkynyl, heteroaryl, cycloalkyl and heterocycloalkyl are optionally further substituted with 1-4 groups selected from halogen, OH, NH₂, CN, C₁₋₄ alkoxy, -O-halogenated C₁₋₄ alkyl, or C₁₋₄ alkyl; preferably, R^{X1}, R^{X2}, R^{X3} and R^{X4} are each independently selected from H, halogen, =O, CN, OH, COOH, C₁₋₂ alkyl, C₁₋₂ alkoxy, C₂₋₄ alkenyl, C₂₋₄ alkynyl, 5- to 6-membered heteroaryl, -SO₂-C₁₋₂ alkyl, -NRaCO-C₃₋₆ cycloalkyl, 4- to 7-membered heterocycloalkyl, -O-C₃₋₆ cycloalkyl, -C₁₋₄ alkyl-(5- to 6-membered heteroaryl), - O-(4- to 7-membered heterocycloalkyl), -C₁₋₄ alkyl-(4- to 7-membered heterocycloalkyl), -C₁₋₄ alkyl-O-(4- to 7-membered heterocycloalkyl), -C₁₋₄ alkyl-O-C₃₋₆ cycloalkyl, -C₁₋₄ alkyl-OC(=O)-(4- to 7-membered heterocycloalkyl), -C₁₋₄ alkyl-OC(=O)-NRₐₐ-C₃₋₆ cycloalkyl, or -NRₐₐR_{bb}, and the alkyl, alkenyl, alkynyl, heteroaryl, cycloalkyl and heterocycloalkyl are optionally further substituted with 1-4 groups selected from halogen, OH, NH₂, CN, C₁₋₂ alkoxy, O-halogenated C₁₋₂ alkyl, or C₁₋₂ alkyl; preferably, R^{X1}, R^{X2}, R^{X3} and R^{X4} are each independently selected from H, halogen, =O, CN, OH, COOH, C₁₋₂ alkyl, C₁₋₂ alkoxy, C₂₋₄ alkenyl, C₂₋₄ alkynyl, 5- to 6-membered heteroaryl, -NRaCO-C₃₋₆ cycloalkyl, 4- to 7-membered heterocycloalkyl, -O-C₃₋₆ cycloalkyl, -C₁₋₄ alkyl-(5- to 6-membered heteroaryl), -O-(4- to 7-membered heterocycloalkyl), -C₁₋₄ alkyl-(4- to 7-membered heterocycloalkyl), -C₁₋₄ alkyl-O-(4- to 7-membered heterocycloalkyl), - C₁₋₄ alkyl-O-C₃₋₆ cycloalkyl, -C₁₋₄ alkyl-OC(=O)-(4- to 7-membered heterocycloalkyl), -C₁₋₄ alkyl-OC(=O)-NRₐₐ-C₃₋₆ cycloalkyl, or -NRₐₐR_{bb}, and the alkyl, alkenyl, alkynyl, heteroaryl, cycloalkyl and heterocycloalkyl are optionally further substituted with 1-4 groups selected from halogen, OH, NH₂, CN, C₁₋₂ alkoxy, -F, O-halogenated C₁₋₂ alkyl, or C₁₋₂ alkyl; more preferably, R^{X1}, R^{X2}, R^{X3} and R^{X4} are each independently selected from H, F, Cl, =O, CN, methyl, ethyl, - NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), - C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), - C₁₋₂ alkyl-OC(=O)-NH-C₃₋₆ cycloalkyl, or -NHRₐₐ; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, or ethyl;
in some embodiments, R^{X1}, R^{X2}, R^{X3} and R^{X4} are each independently selected from H, F, Cl, =O, CN, -SO₂CH₃, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), -C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), -C₁₋₂ alkyl-OC(=O)-NH-C₃₋₆ cycloalkyl, or -NHRₐₐ, and the methyl, ethyl, heteroaryl, cycloalkyl and heterocycloalkyl are optionally further substituted with 1-4 groups selected from F, Cl, OH, NH₂, CN, methyl, ethyl, or methoxy;
in some embodiments, R^{X1}, R^{X2}, R^{X3} and R^{X4} are each independently selected from H, F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), -C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), -C₁₋₂ alkyl-OC(=O)-NH-C₃₋₆ cycloalkyl, or -NHRₐₐ, and the methyl, ethyl, heteroaryl, cycloalkyl and heterocycloalkyl are optionally further substituted with 1-4 groups selected from F, Cl, OH, NH₂, CN, methyl, ethyl, or methoxy;
alternatively, any two of R^{X1}, R^{X2}, R^{X3} and R^{X4}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
in some embodiments, 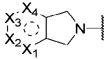 is selected from 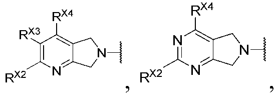 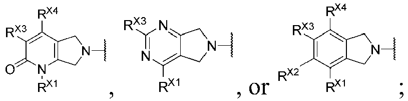
in some embodiments, 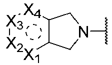 is selected from 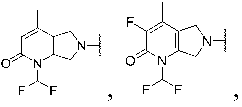 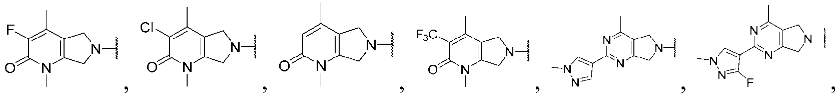 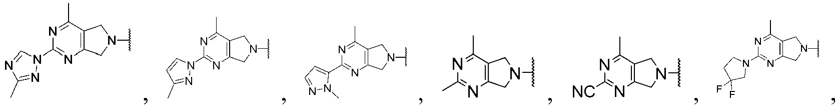 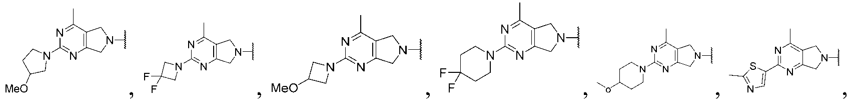 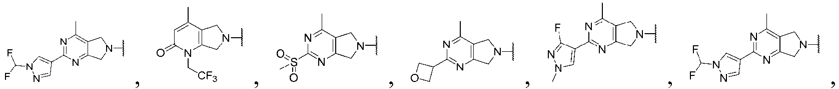 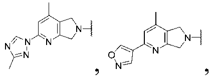 or 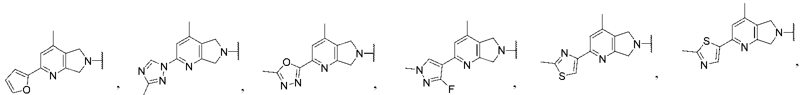 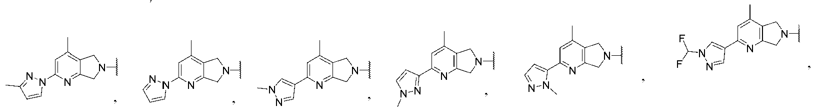 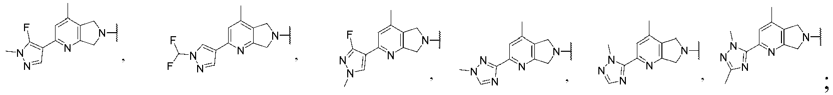
Rₐₐ and R_{bb} are each independently selected from H, halogen, =O, C₁₋₄ alkyl, C₁₋₄ alkylhydroxy, halogenated C₁₋₄ alkyl, C₁₋₄ alkoxy or halogenated C₁₋₄ alkoxy.

Further, in the compound, or the stereoisomer, the deuterated substance, the solvate, the co-crystal or the pharmaceutically acceptable salt thereof described in technical solution 11,
each R^{A} is independently selected from F, Cl, =O, CN, methyl, ethyl, - NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), - C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), - C₁₋₂ alkyl-OC(=O)-NH-C₃₋₆ cycloalkyl, -NHRₐₐ, -SO₂CH₃, cyclopropyl, cyclobutyl, -NH-cyclopropyl, -NH-cyclobutyl, oxolanyl, 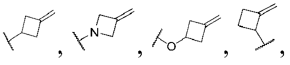 -CF₃, or 7- to 10-membered bicyclic heterocycloalkyl; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, ethyl, - CHF₂, -CH₂F, -CF₃, -C(=O) CH₃, -O-CH₃, or -O-CH₂CH₃;
in some embodiments, each R^{A} is independently selected from F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), -C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), -C₁₋₂ alkyl-OC(=O)-NH-C₃₋₆ cycloalkyl, -NHRₐₐ, cyclopropyl, cyclobutyl, -NH-cyclopropyl, -NH-cyclobutyl, 5- to 6-membered heteroaryl, 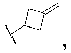 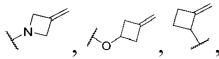 -CF₃, or 7- to 10-membered bicyclic heterocycloalkyl; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, ethyl, -CHF₂, -CH₂F, -CF₃, -C(=O) CH₃, -O-CH₃, or - O-CH₂CH₃;
in some embodiments, each R^{A} is independently selected from F, Cl, =O, CN, methyl, ethyl, -NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), -C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), -C₁₋₂ alkyl-OC(=O)-NH-C₃₋₆ cycloalkyl, -NHRₐₐ, cyclopropyl, cyclobutyl, -NH-cyclopropyl, -NH-cyclobutyl, 5- to 6-membered heteroaryl, 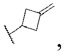 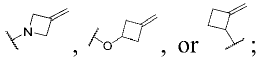 the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, or ethyl;
alternatively, two R^{A}, together with the atoms to which they are connected, form 5- to 6-membered heterocyclyl, and the heterocyclyl is optionally further substituted with 1-4 groups selected from F, Cl, OH, NH₂, CN, methyl, or ethyl;
each R^{A1} is independently selected from F, Cl, =O, CN, OH, COOH, - SO₂CH₃, methyl, ethyl, -O-C₃₋₆ cycloalkyl, -NHRₐₐ, 5-membered heteroaryl, or 4-to 6-membered heterocycloalkyl, and the methyl, ethyl, cycloalkyl and 4- to 6-membered heterocycloalkyl are optionally further substituted with 1-5 groups selected from F, Cl, OH, NH₂, CN, methyl, ethyl, -CHF₂, -CH₂F, -CF₃, or methoxy; in some embodiments, each R^{A1} is independently selected from F, Cl, =O, CN, OH, COOH, methyl, ethyl, -O-C₃₋₆ cycloalkyl, -NHRₐₐ, 5-membered heteroaryl, or 4- to 6-membered heterocycloalkyl, and the methyl, ethyl, cycloalkyl, 5-membered heteroaryl and 4- to 6-membered heterocycloalkyl are optionally further substituted with 1-5 groups selected from F, Cl, OH, NH₂, CN, methyl, ethyl, or methoxy;
in some embodiments, each R^{A1} is independently selected from F, Cl, =O, CN, OH, COOH, methyl, ethyl, -O-C₃₋₆ cycloalkyl, or -NHRₐₐ, and the methyl, ethyl and cycloalkyl are optionally further substituted with 1-5 groups selected from F, Cl, OH, NH₂, CN, methyl, ethyl, or methoxy;
each R^{B} is independently selected from F, Cl, =O, methyl, or ethyl, and the methyl and ethyl are optionally further substituted with a group selected from F or Cl;
each R^{C} is independently selected from D, F, Cl, methyl, ethyl, -CHF₂, -CH₂F, -CF₃, -CH₂CF₃, -CH₂CHF₂, -CH₂CH₂F, -OCHF₂, -OCH₂F, -OCF₃, 5-membered heteroaryl, 6-membered heteroaryl, -CN, =O, -NH-S(=O)₂-C₁₋₂ alkyl, -O-(5-membered heteroaryl), -O-C₃₋₆ cycloalkyl, -O-(4- to 6-membered monocyclic heterocycloalkyl), 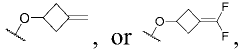 and the methyl, ethyl, heteroaryl and cycloalkyl are optionally further substituted with 1-4 groups selected from F, Cl, methyl, ethyl, -CHF₂, -CH₂F, or -CF₃; in some embodiments, each R^{C} is independently selected from D, F, Cl, methyl, ethyl, -CHF₂, -CH₂F, -CF₃, -CH₂CF₃, -CH₂CHF₂, -CH₂CH₂F, -OCHF₂, -OCH₂F, -OCF₃, 5-membered heteroaryl, 6-membered heteroaryl, -CN, =O, -NH-S(=O)₂-C₁₋₂ alkyl, -O-(5-membered heteroaryl), -O-C₃₋₆ cycloalkyl, -O-(4- to 6-membered monocyclic heterocycloalkyl), or 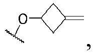 and the methyl, ethyl, heteroaryl and cycloalkyl are optionally further substituted with 1-4 groups selected from F, Cl, methyl, ethyl, or -CF₃;
in some embodiments, each R^{C} is independently selected from F, Cl, methyl, ethyl, -CHF₂, -CH₂F, -CF₃, -CH₂CF₃, -CH₂CHF₂, -CH₂CH₂F, -OCHF₂, -OCH₂F, - OCF₃, 5-membered heteroaryl, 6-membered heteroaryl, -CN, =O, or -NH-S(=O)₂-C₁₋₂ alkyl, and the methyl, ethyl and heteroaryl are optionally further substituted with 1-4 groups selected from F, Cl, methyl, or ethyl;
each R^{C1} is independently selected from F, Cl, methyl, ethyl, -CHF₂, -CH₂F, - CF₃, -CH₂CF₃, -CH₂CHF₂, -CH₂CH₂F, C₄₋₅ cycloalkyl, cubane, 5-membered heteroaryl, 6-membered heteroaryl, 8-membered fused heteroaryl, 9-membered fused heteroaryl, 10-membered fused heteroaryl, 7- to 8-membered spiro heterocycloalkyl, 8- to 9-membered fused heterocycloalkyl, 7-membered bridged heterocycloalkyl, 4- to 6-membered monocyclic heterocycloalkyl, -N=S(=O)RₐₐR_{bb}, -P(=O)RₐₐR_{bb}, -NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4-to 6-membered heterocycloalkyl), -O-(5- to 6-membered heteroaryl), -NH-C₃₋₆ cycloalkyl, -NH-(4- to 6-membered heterocycloalkyl), -NH-(5- to 6-membered heteroaryl), -NH-S(=O)₂-C₃₋₆ cycloalkyl, -C₁₋₂ alkyl-(5-membered heteroaryl), -C₁₋₂ alkyl-(6-membered heteroaryl), -C₁₋₂ alkyl-phenyl, -C₁₋₂ alkyl-(4- to 6-membered heterocycloalkyl), -NRₐC(=O)-O-Rₐ, -(C₁₋₂ alkyl)ₙ-O-(C₁₋₂ alkyl)ₙ-(4- to 6-membered heterocyclyl), -NRₐC(=O)-(4- to 6-membered heterocyclyl), - C(=O)NRₐ-(4- to 6-membered heterocyclyl), 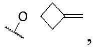 -C(=O)-C₃₋₆ cycloalkyl, - C(=O)-(4- to 12-membered heterocycloalkyl), or -C(=O)-(5- to 6-membered heteroaryl), and the alkyl, cycloalkyl, heterocyclyl, heteroaryl, heterocycloalkyl and cubane are optionally further substituted with 1-4 groups selected from F, Cl, OH, NH₂, CN, methyl, ethyl, deuterated methyl, -CHF₂, -CH₂F, -CF₃, -CH₂CF₃, - CH₂CHF₂, -CH₂CH₂F, =O, -C(=O) C₁₋₄ alkyl, -NH-C(=O) C₁₋₄ alkyl, or -S(=O)₂C₁₋₂ alkyl;
Rₐ is selected from H, F, Cl, methyl, ethyl, cyclopropyl, cyclobutyl, cyclopentyl, or difluorocyclobutyl;
Rₐₐ and R_{bb} are each independently selected from H, F, Cl, =O, methyl, ethyl, -CH₂-CH₂-OH, -CH₂-OH, -CHF₂, -CH₂F, -CF₃, -CH₂CF₃, -CH₂CHF₂, -CH₂CH₂F, -OCH₃, -OCH₂CH₃, -OCHF₂, -OCH₂F, -OCF₃, -OCH₂CF₃, -OCH₂CHF₂, or - OCH₂CH₂F;
alternatively, Rₐₐ and R_{bb}, together with the atoms to which they are connected, form 4- to 6-membered heterocyclyl, and the heterocyclyl is optionally further substituted with 1-4 groups selected from F, Cl, OH, NH₂, CN, methyl, or ethyl; n1 is selected from 0, 1, 2 or 3;
n2 is selected from 0 or 1;
n3 is selected from 0, 1, 2, 3 or 4;
in some embodiments, n3 is selected from 0, 1 or 2;
L₁ is selected from a bond, vinylene, -CH₂-, -CH₂-CH₂-, 4- to 6-membered monocyclic heterocycloalkyl, (4- to 6-membered monocyclic heterocycloalkyl)-C(=O)-CH₂-, -(4- to 6-membered monocyclic heterocycloalkyl)-CH₂-, -CH₂-(4- to 6-membered monocyclic heterocycloalkyl)-, -CH(CH₃)NHC(=O)-CH₂-, - CH₂NHC(=O)-CH₂-, -S(=O)₂-CH₂-, -C(=S)-CH₂-, -C(=O)-C₃₋₆ cycloalkyl, - C(=O)-CH(CH₃)-, -C(=O)-CH(C₃₋₆ cycloalkyl)-, -C(=O)-CF₂-, -C(=O)-C(CH₃)₂-, - CH₂-C(=O)-, -C(=O)-C(=O)-, C₃₋₆ cycloalkyl, -(5- to 6-membered heteroaryl)-CH₂-, 5- to 6-membered heteroaryl, -C(=O)-, -C(=O)-CH₂-, -N(CH₃)-CH₂-, -CF₂-CH₂-, -C(=O)-C₂₋₄ alkenylene, -C(=O)-O-, -C(=O)-NH-, or -C(=O)-N(CH₃)-, and the -CH₂-, alkenylene, cycloalkyl, heterocycloalkyl and heteroaryl are optionally further substituted with 1-3 R^{L1}; each R^{L1} is independently selected from F, Cl, Br, =O, methyl, ethyl, -CF₃, -CHF₂, -CH₂F, vinyl, propenyl, methoxy, ethoxy, -OCF₃, -OCHF₂, -OCH₂F, cyclopropyl, cyclobutyl, methyl-methoxy, or 5-membered heteroaryl;
alternatively, Li is 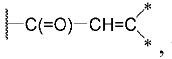 wherein the " * " end is connected to ring B;
L₂ is selected from a bond, vinylene, ethynylene, -CH₂-, -CH₂-CH₂-, -O-, - NH-, -C(=O)-, or -N(CH₃)-.

Further, the compound, or the stereoisomer, the deuterated substance, the solvate, the co-crystal or the pharmaceutically acceptable salt thereof described in technical solution 12 has a structure of formula (II), wherein 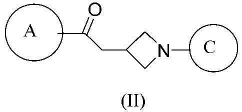
ring A is selected from the following structures optionally further substituted with 1, 2 or 3 R^{A}: 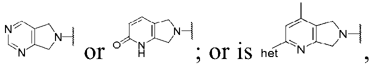 wherein het is 5-membered heteroaryl, and the heteroaryl is optionally substituted with 1 and 2 groups selected from halogen, C₁₋₂ alkyl, halogenated C₁₋₂ alkyl, or deuterated C₁₋₂ alkyl;
each R^{A} is independently selected from halogen, CN, C₁₋₄ alkyl, C₁₋₄ alkoxy, halogenated C₁₋₄ alkyl, -O-C₃₋₆ cycloalkyl, 4- to 6-membered heterocycloalkyl, 5-to 6-membered heteroaryl, or -SO₂-C₁₋₂ alkyl, and the heteroaryl, cycloalkyl and heterocycloalkyl are optionally further substituted with 1-2 groups selected from halogen, OH, NH₂, CN, C₁₋₂ alkyl, C₁₋₂ alkoxy, -C(=O)C₁₋₂ alkyl, or halogenated C₁₋₂ alkyl;
ring C is selected from 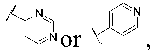 and the ring C is optionally further substituted with 1, 2 or 3 R^{C};
each R^{C} is independently selected from halogen, halogenated C₁₋₃ alkyl, halogenated C₁₋₄ alkoxy, C₃₋₅ cycloalkyl, 5- to 6-membered heteroaryl, -O-C₃₋₆ cycloalkyl, -O-(4- to 12-membered heterocycloalkyl), -O-(5- to 6-membered heteroaryl), -O-C₅₋₈ spirocycloalkyl, 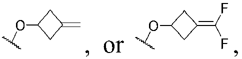 and the heteroaryl, cycloalkyl and spirocycloalkyl are optionally further substituted with 1-3 groups selected from halogen, C₁₋₂ alkyl, halogenated C₁₋₂ alkyl, or deuterated C₁₋₂ alkyl.

Further, in the compound, or the stereoisomer, the deuterated substance, the solvate, the co-crystal or the pharmaceutically acceptable salt thereof described in technical solution 13, ring A is selected from 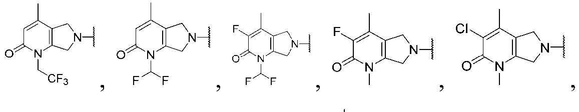 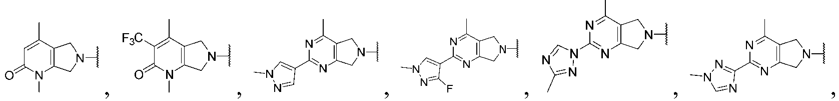 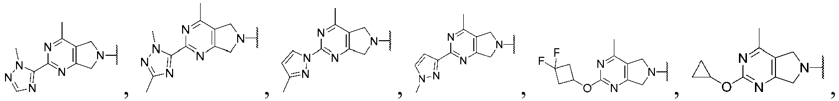 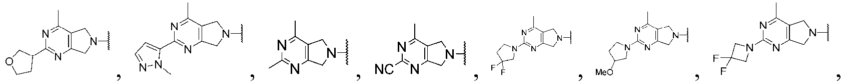 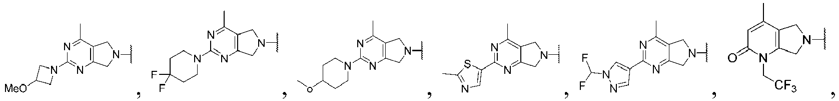 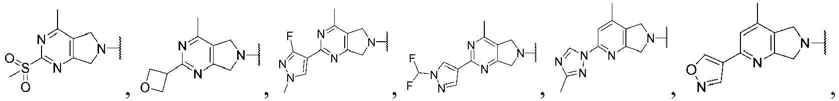 or 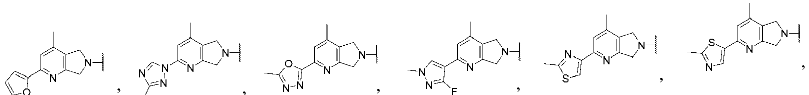 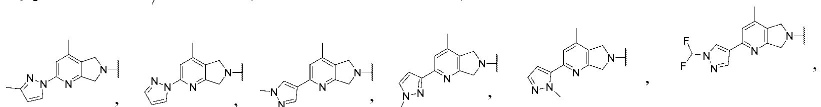 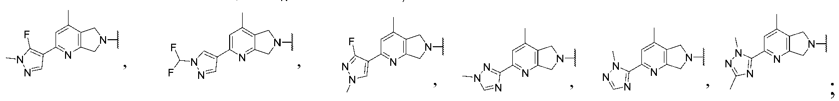 each R^{C} is independently selected from 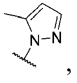 F, Cl, CF₃, CHF₂, CH₂F, - OCHF₂, -OCH₂F, -OCF₃, -OCH₂CF₃, -OCH(CH₃)CF₃, cyclopropyl, cyclobutyl,

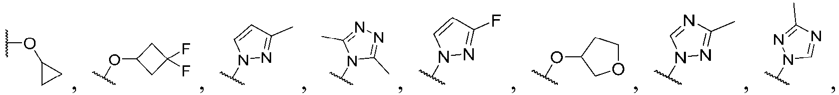

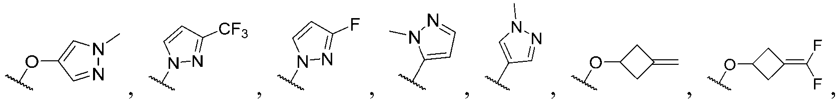

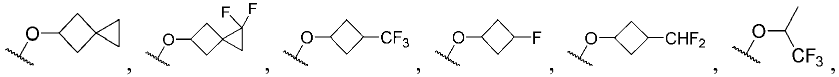

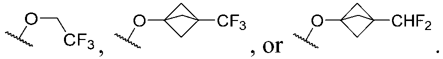

A specific technical solution of the present invention is the compound, or the stereoisomer, the deuterated substance, the solvate, the co-crystal or the pharmaceutically acceptable salt thereof described in the present invention, wherein the compound is of a structure selected from one of the structures in Table A below:

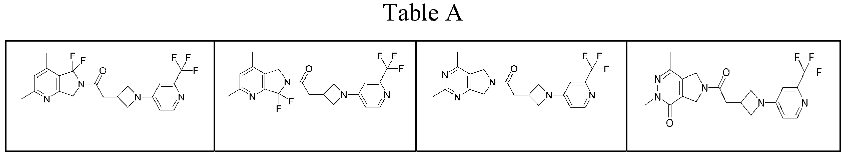

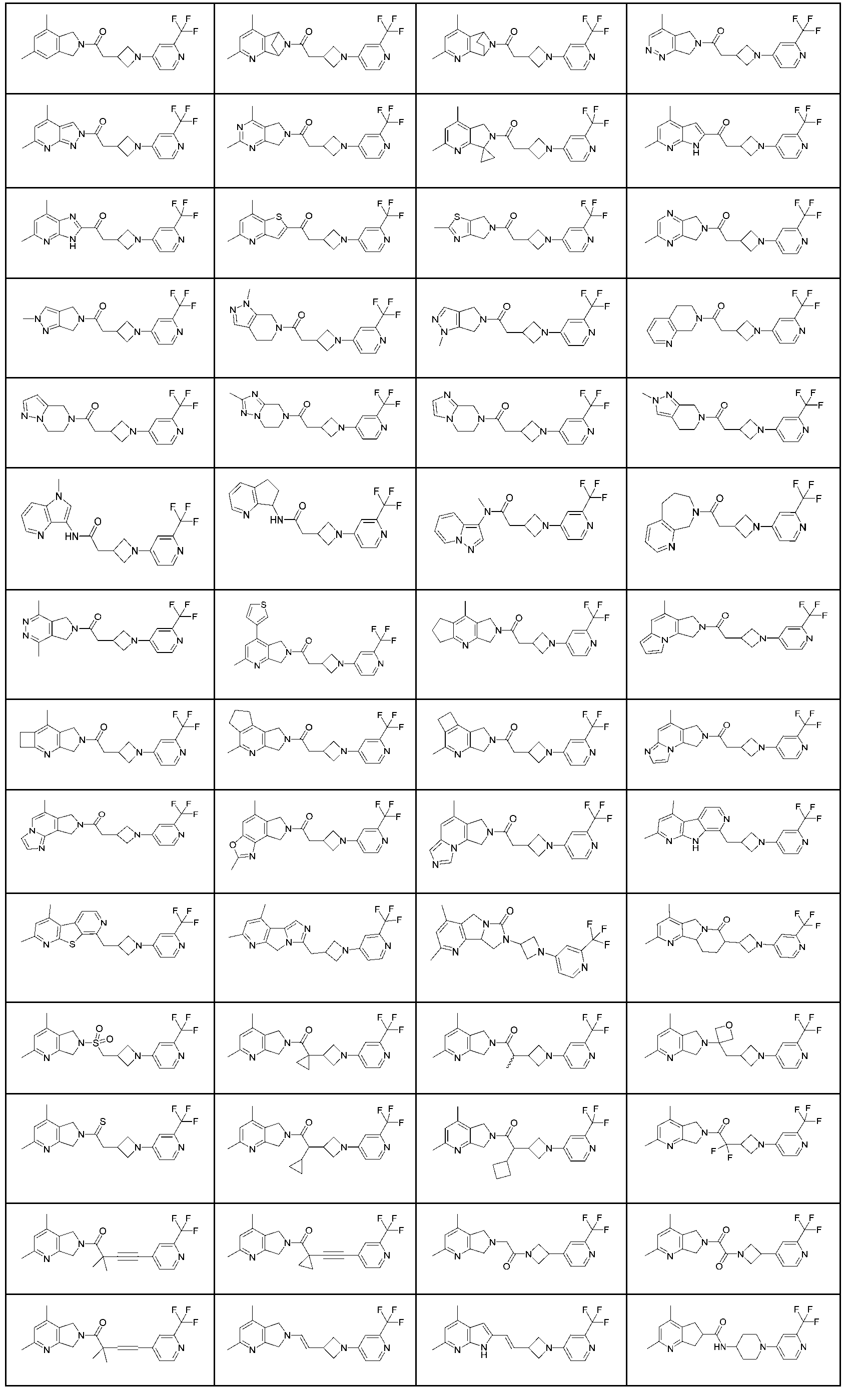

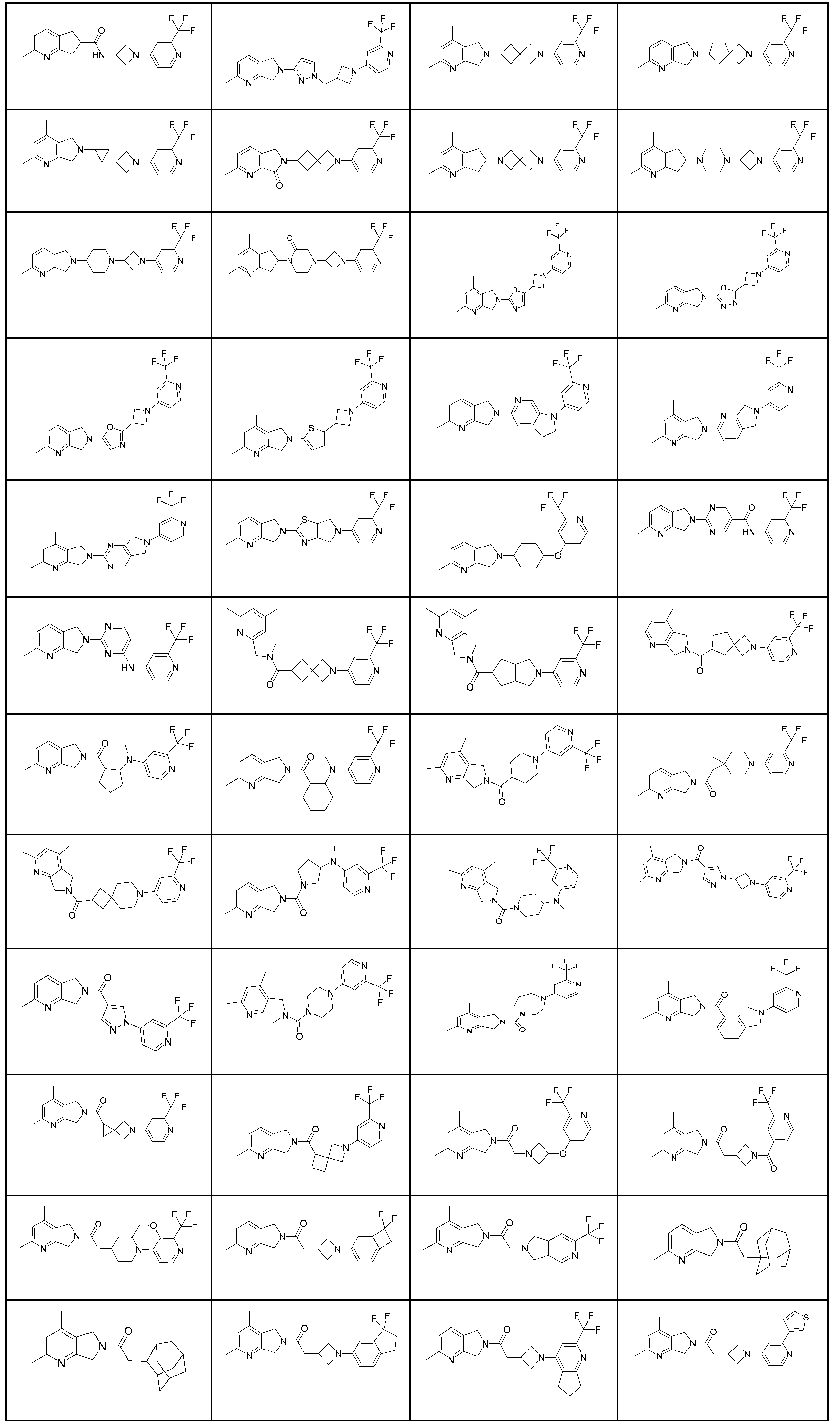

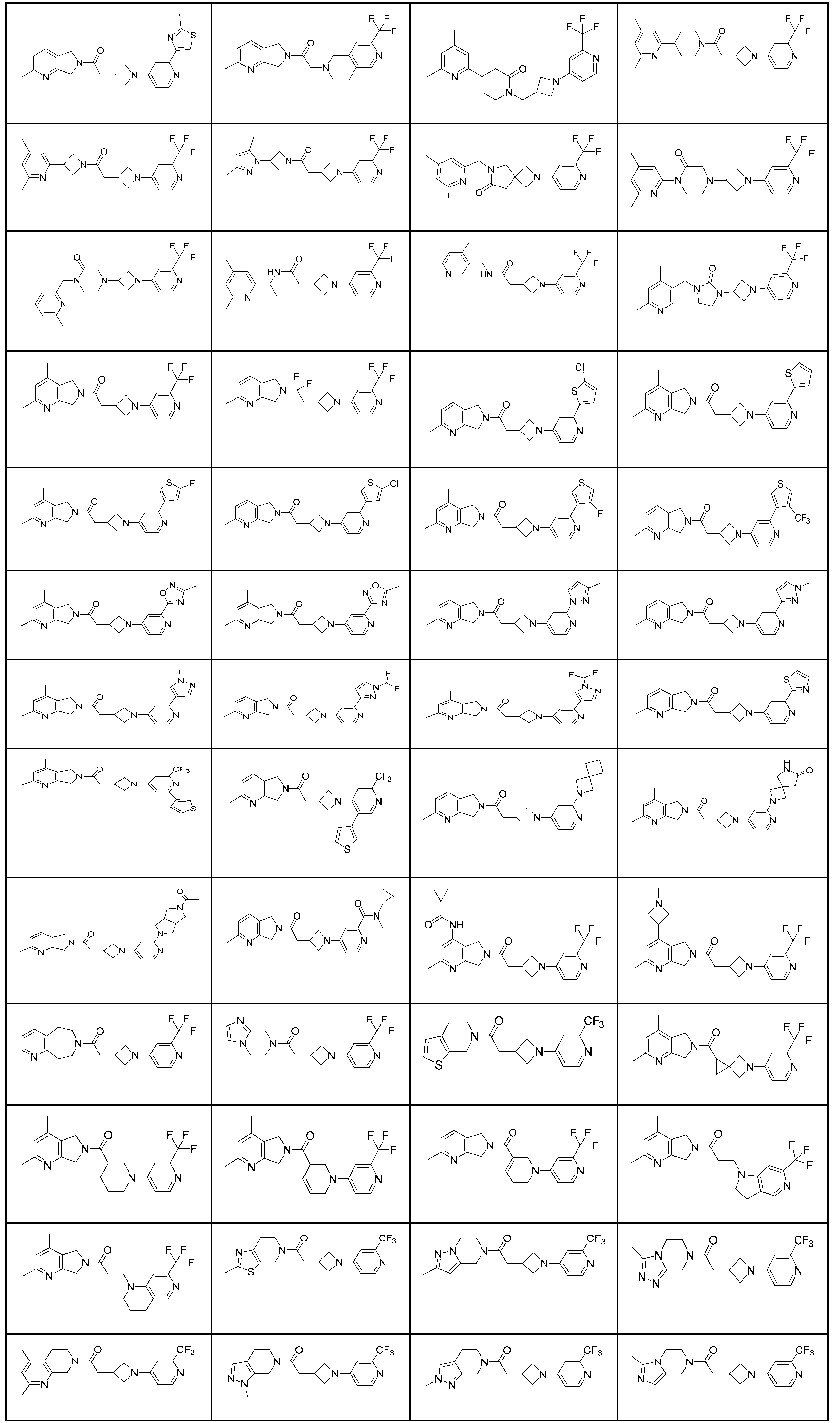

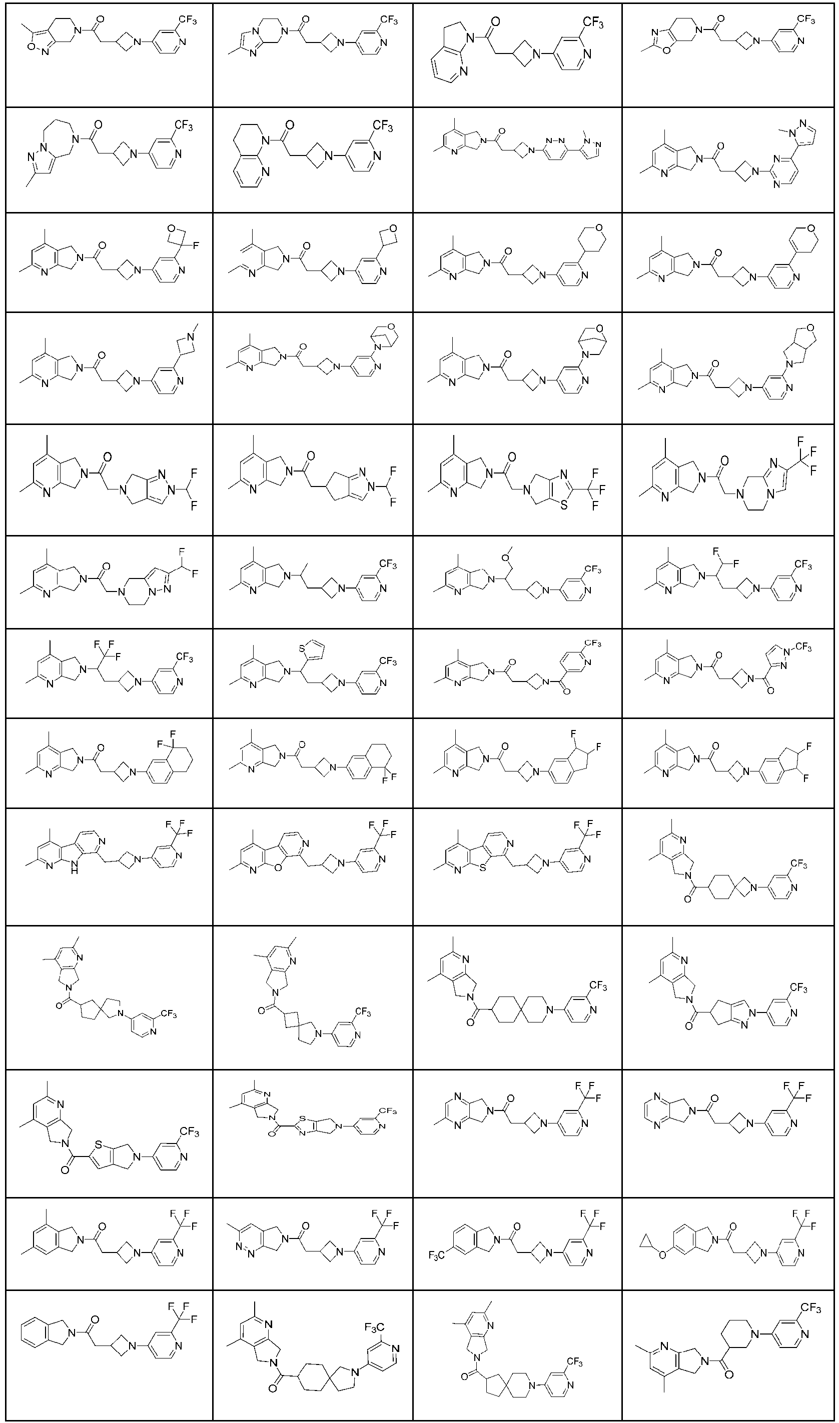

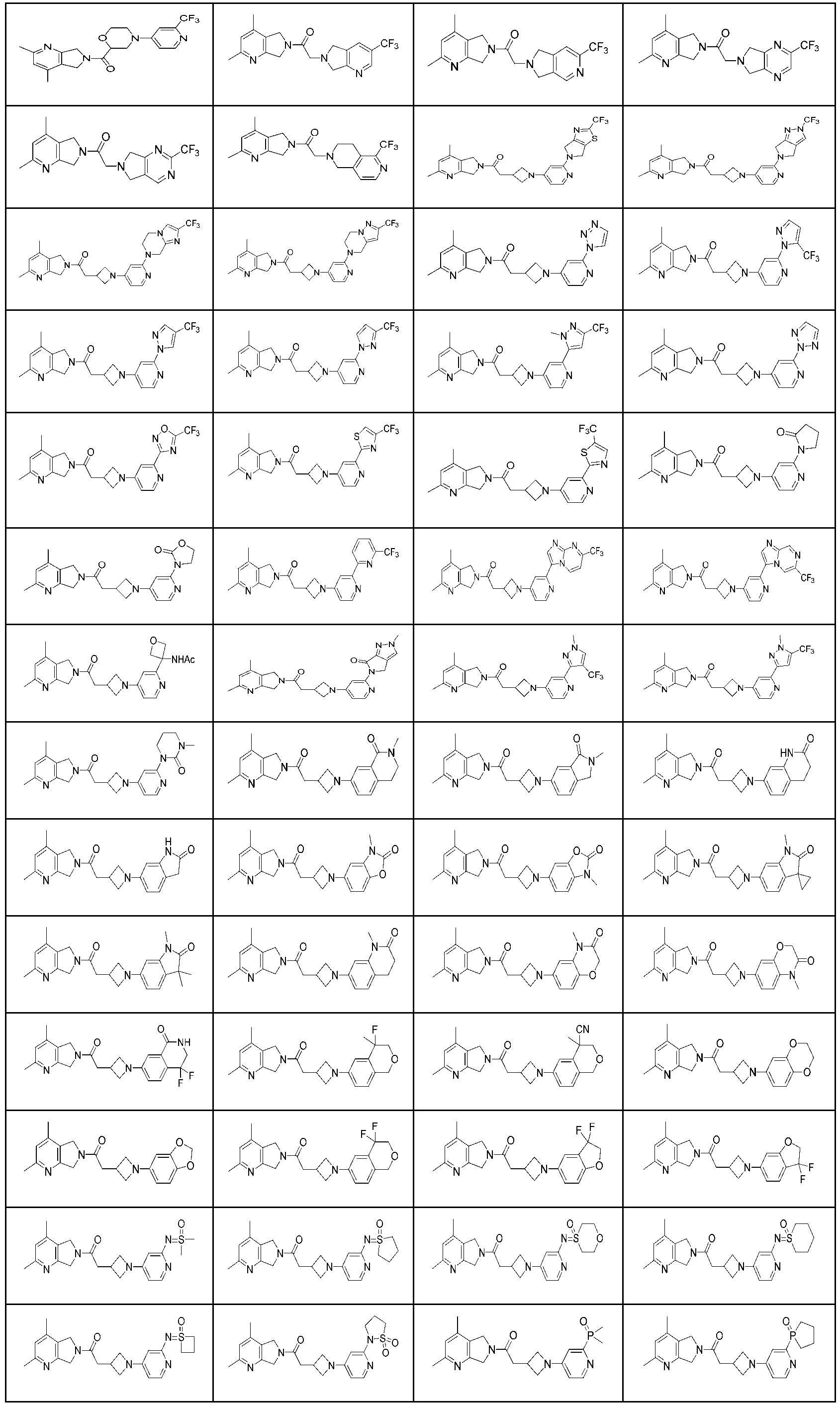

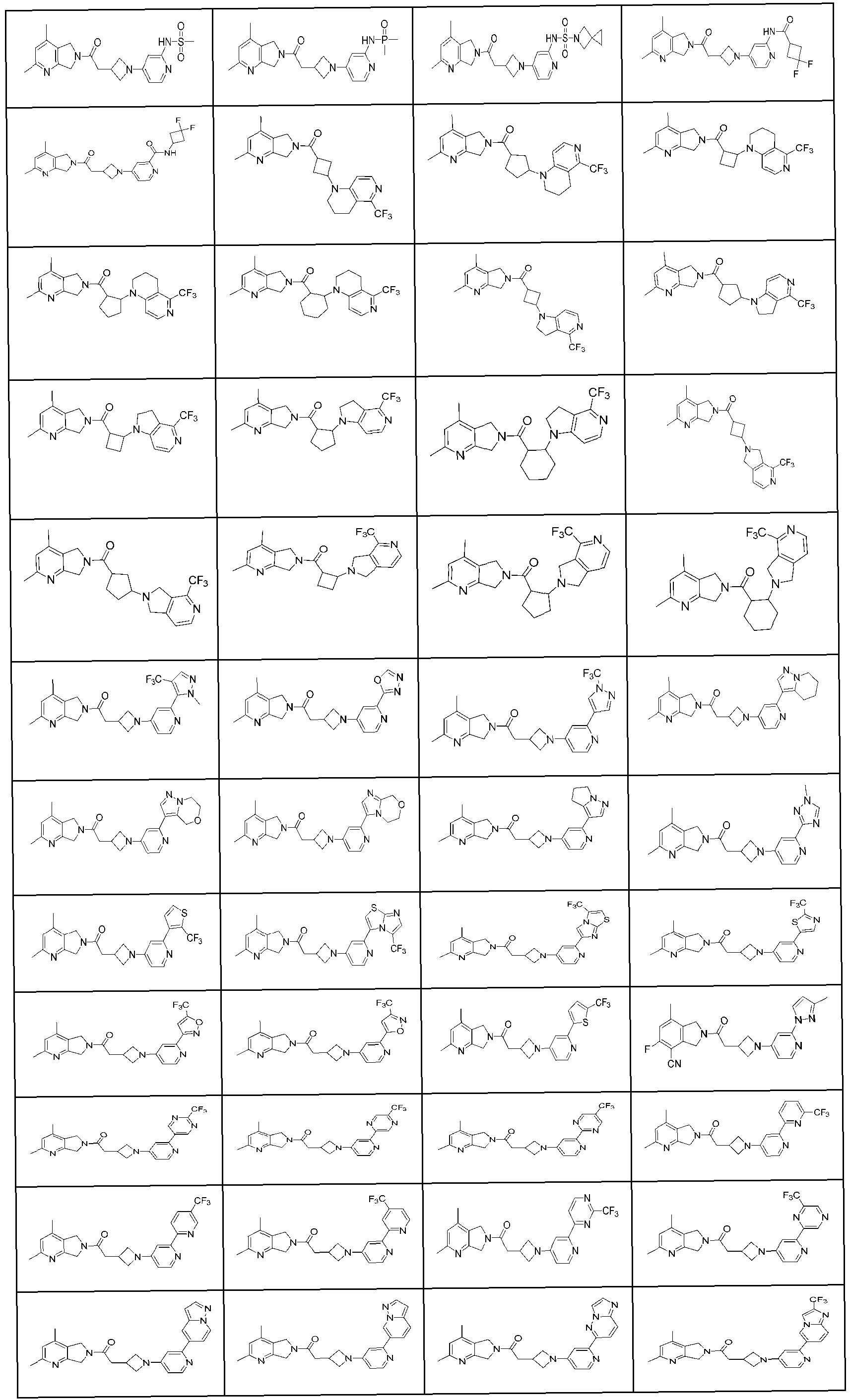

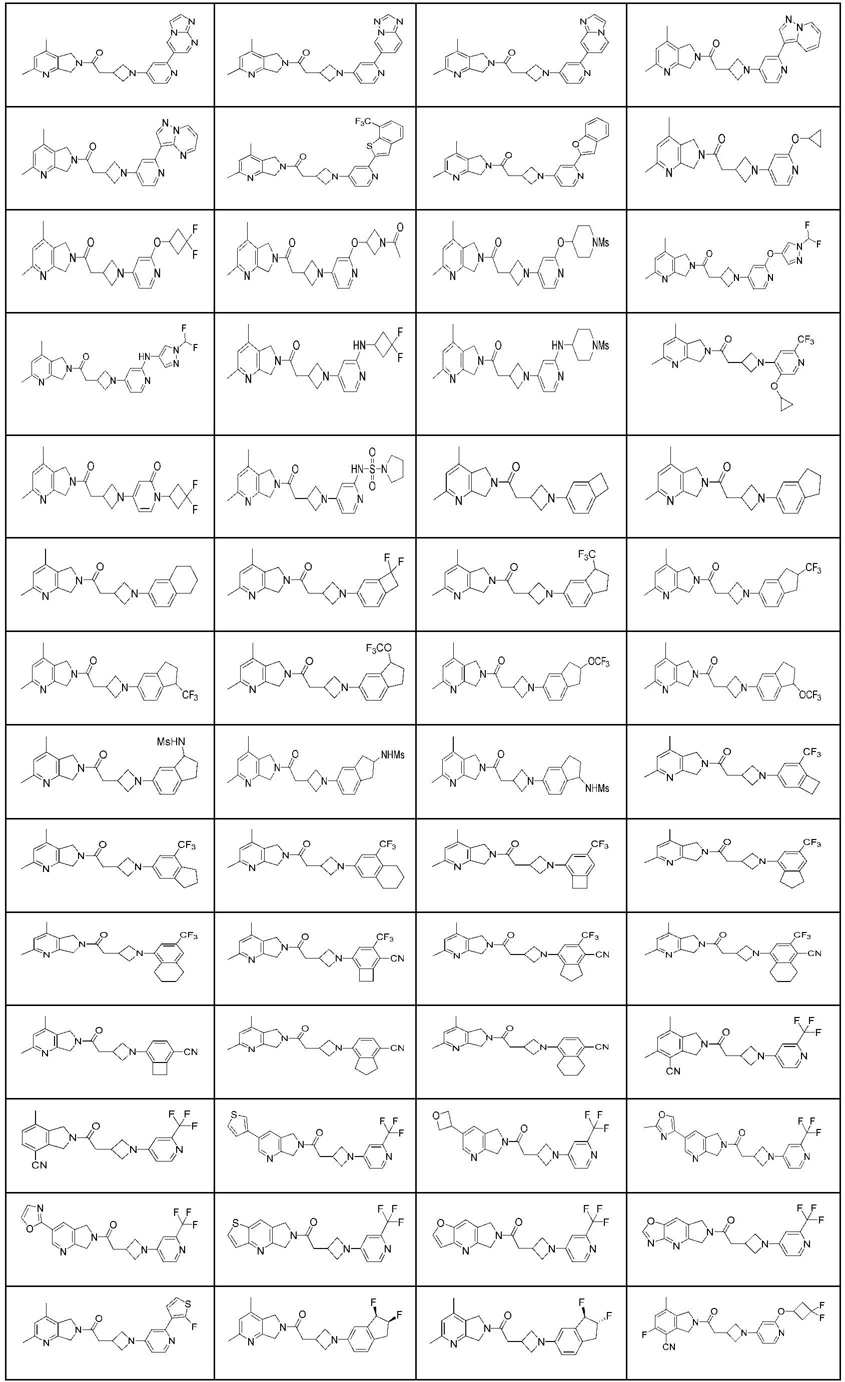

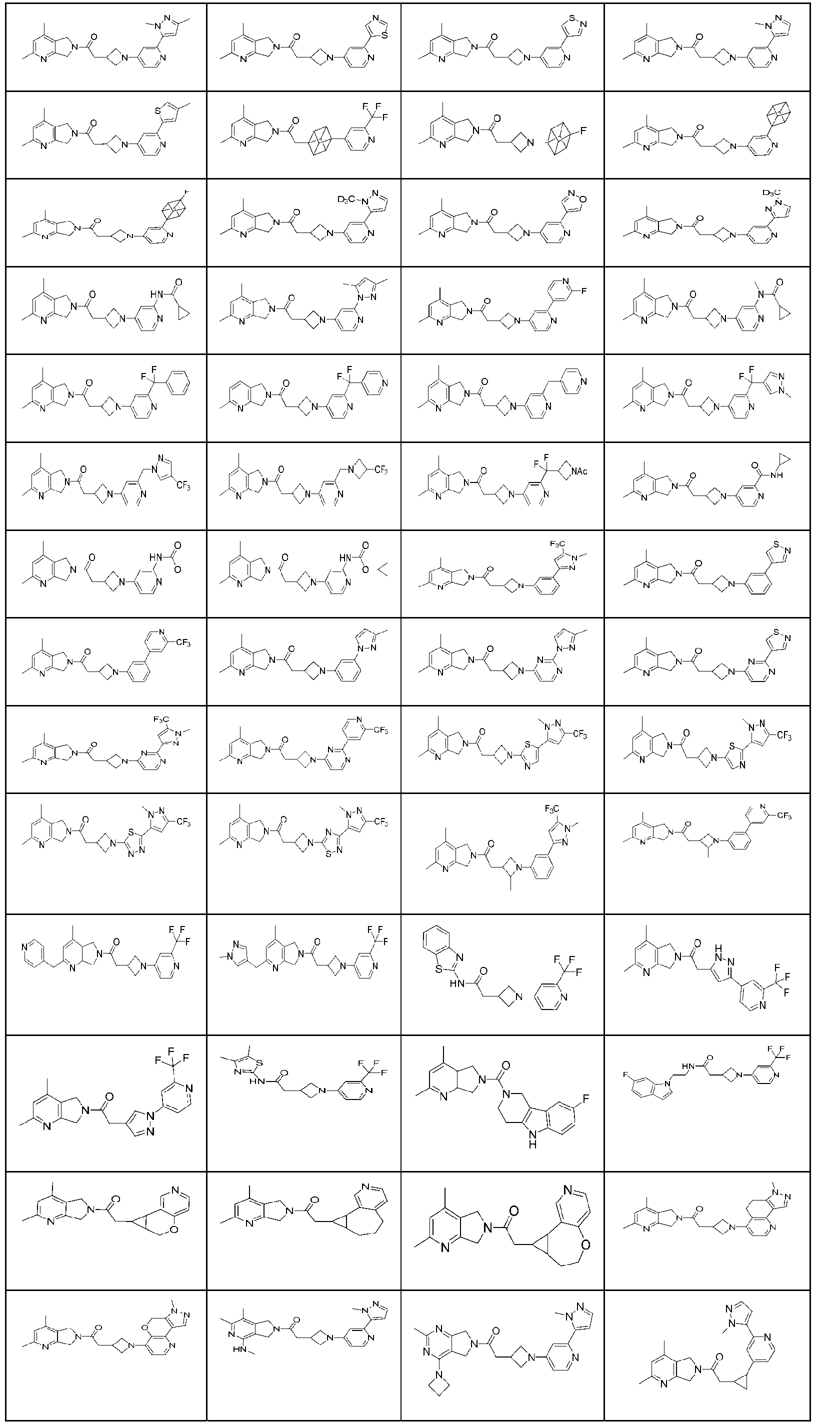

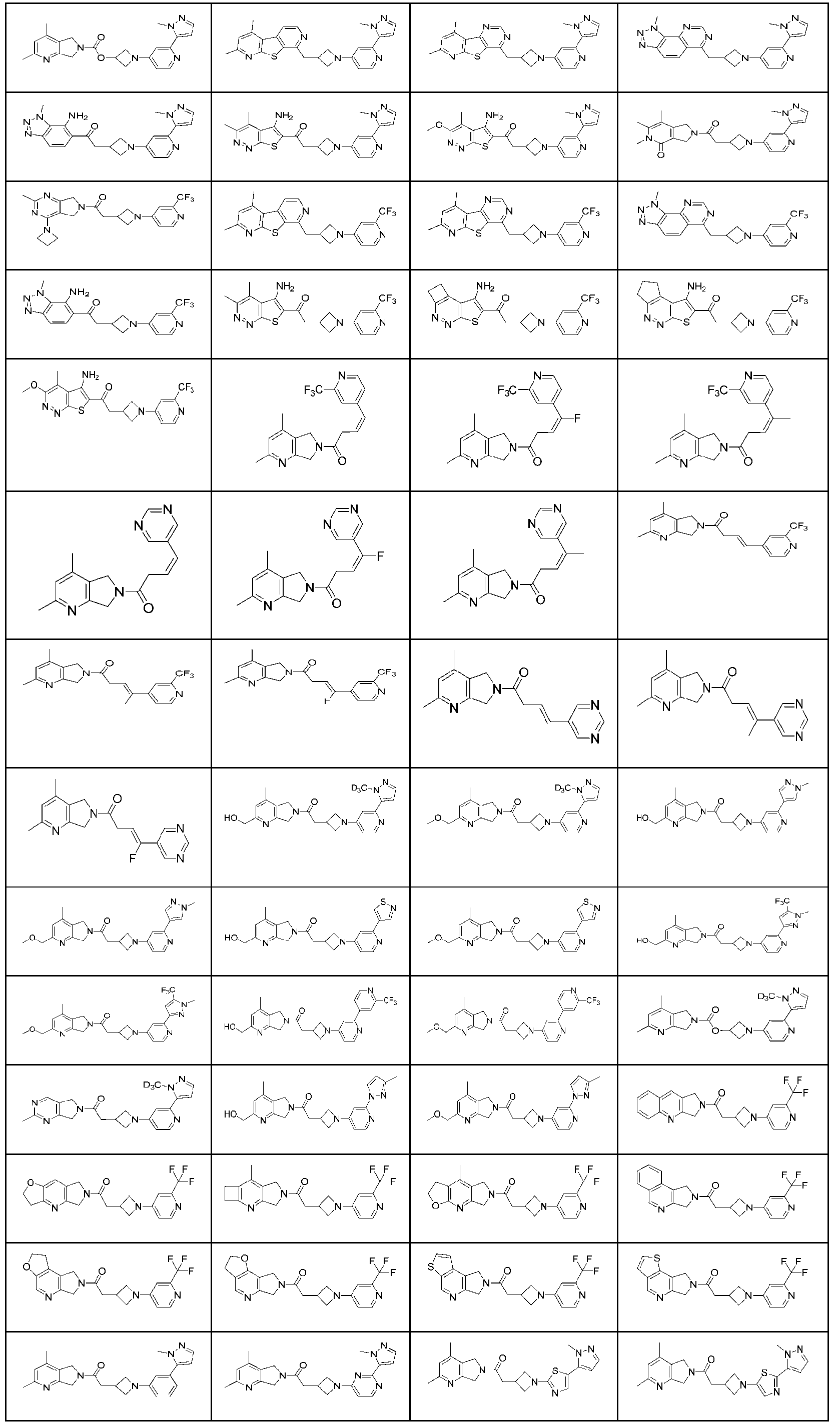

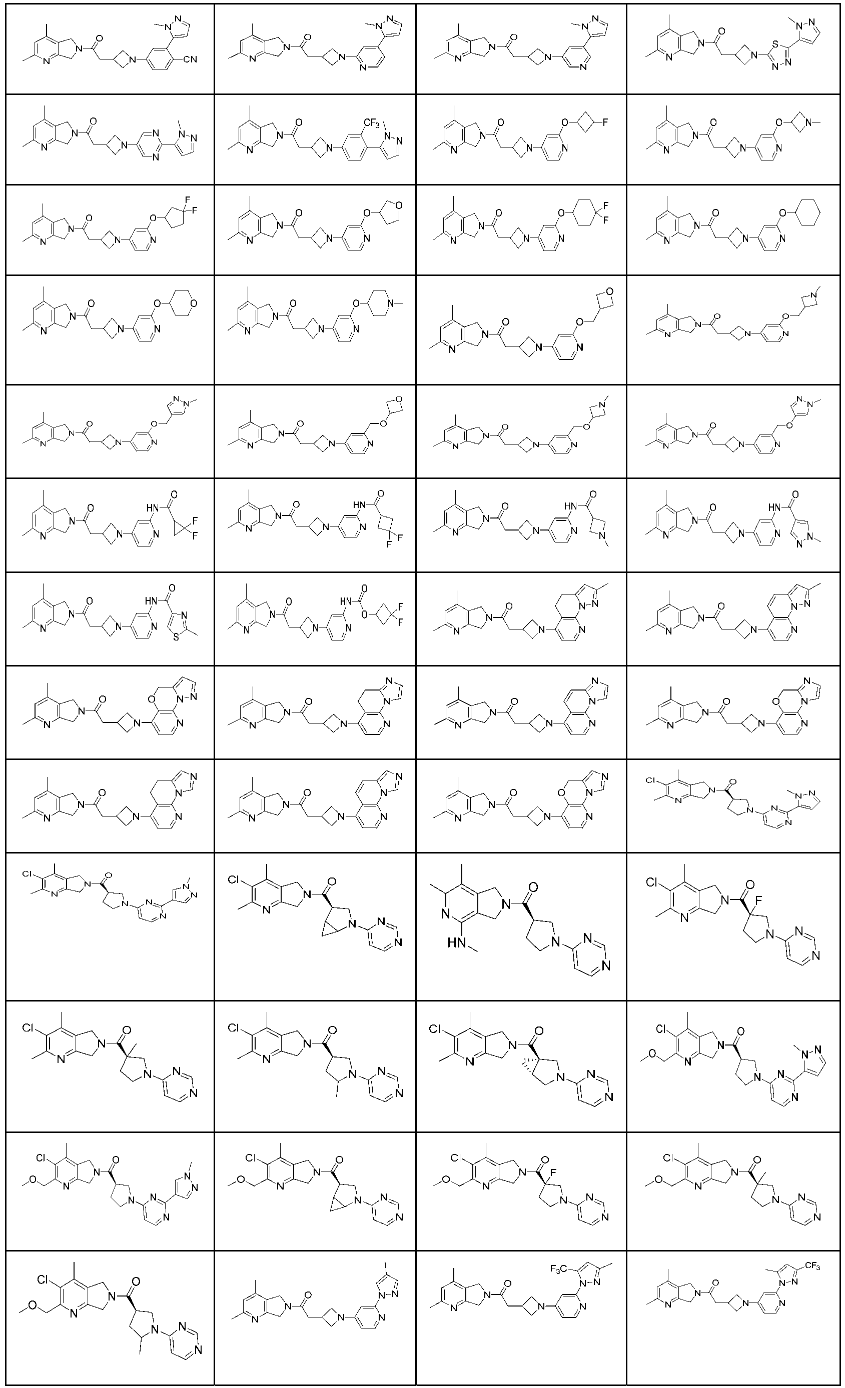

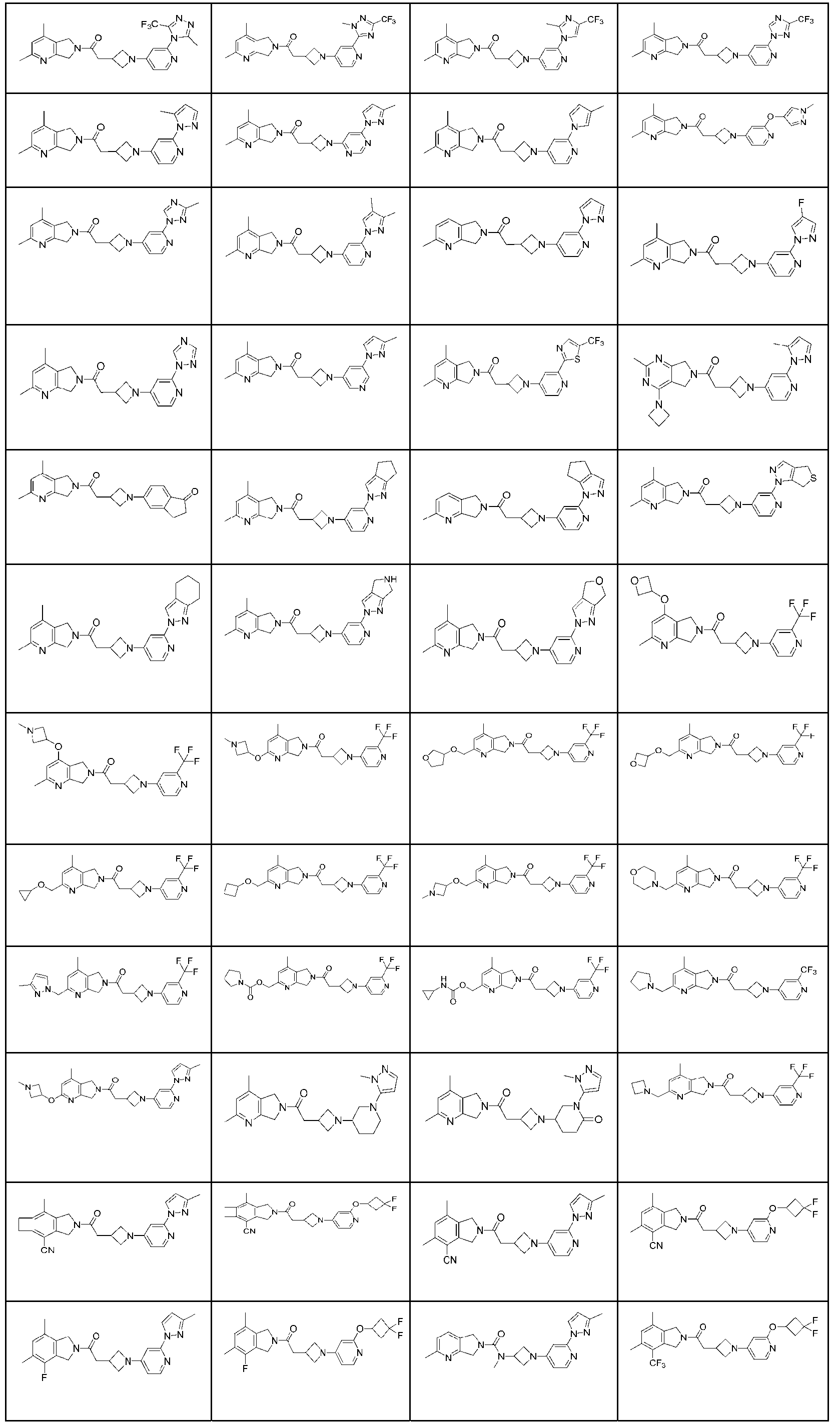

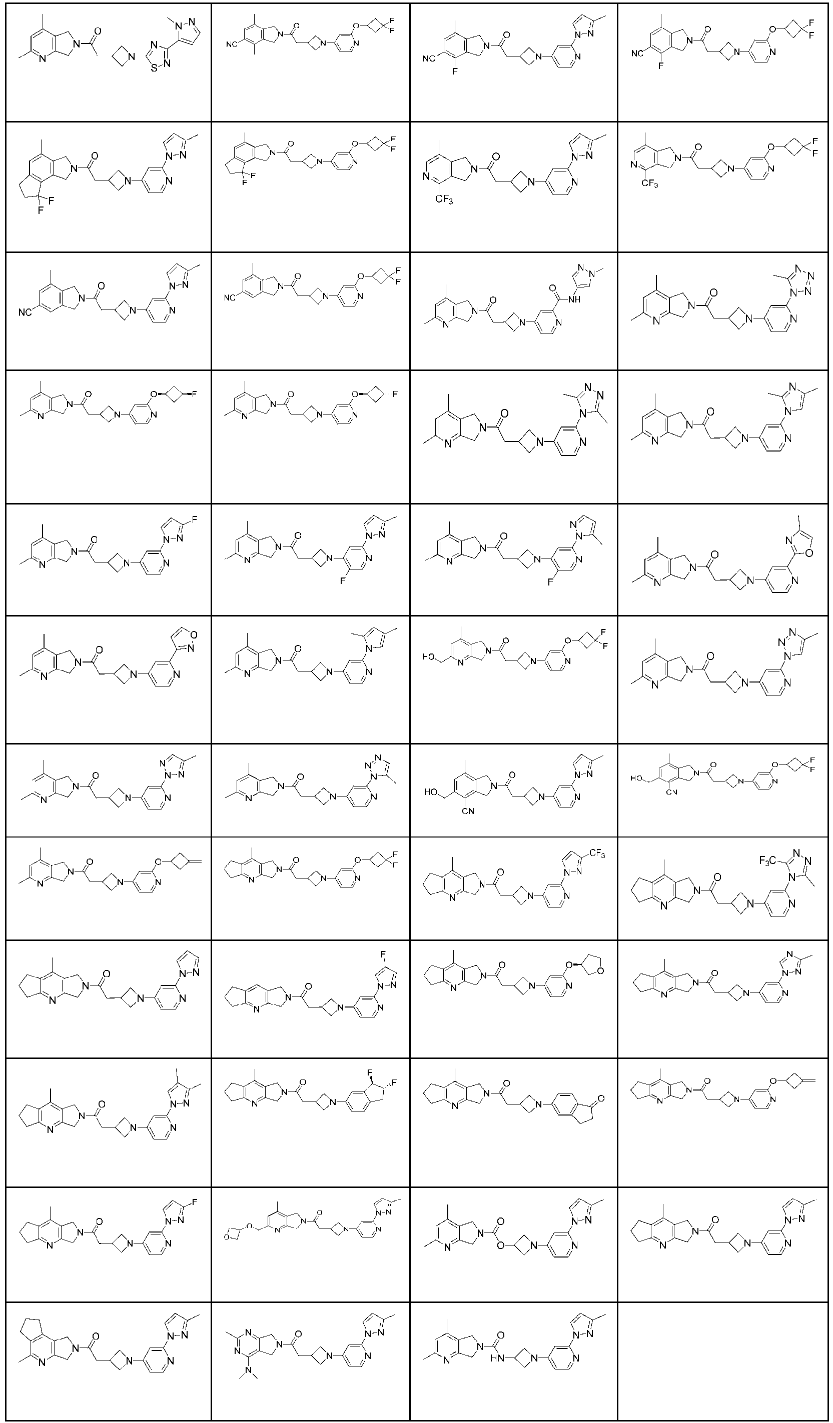

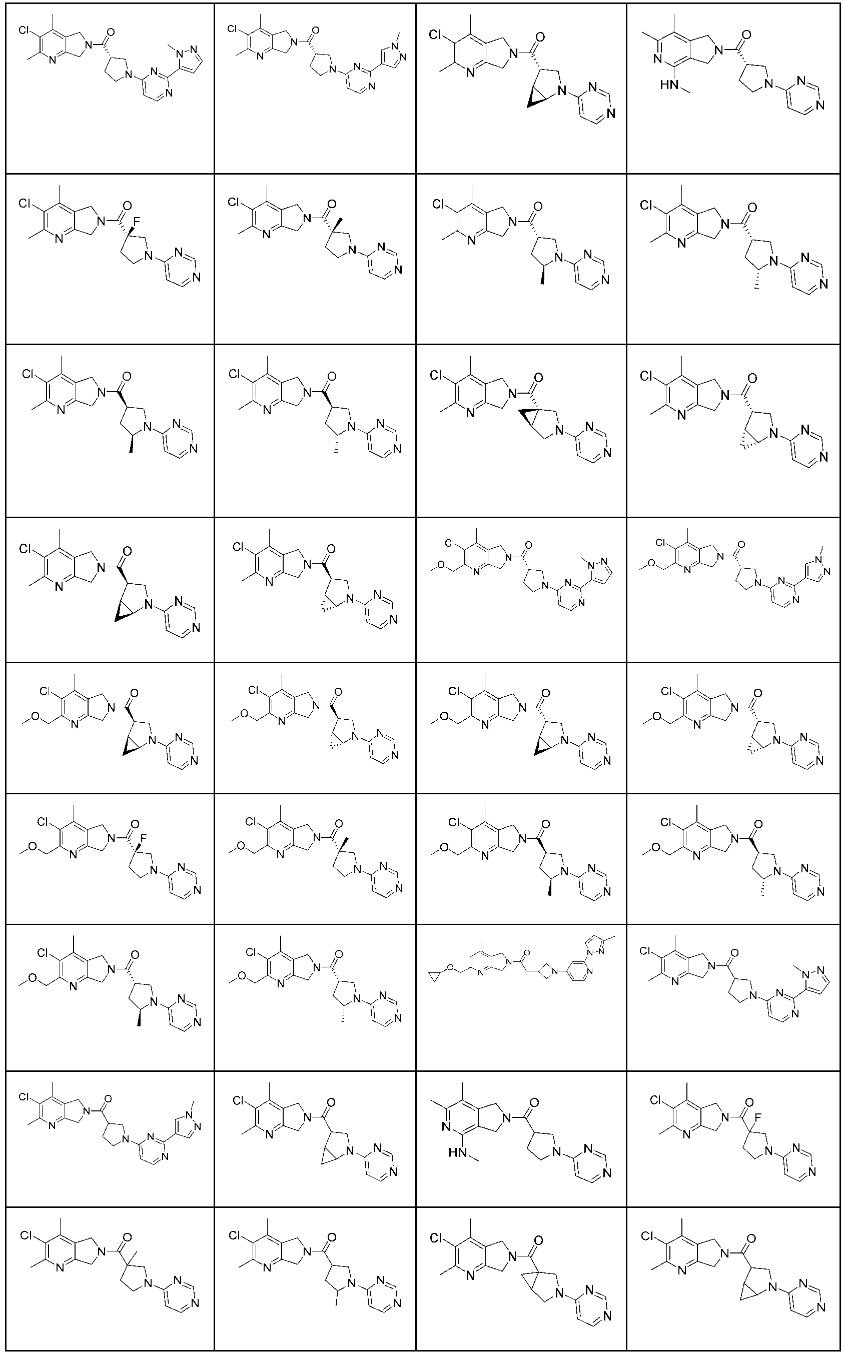

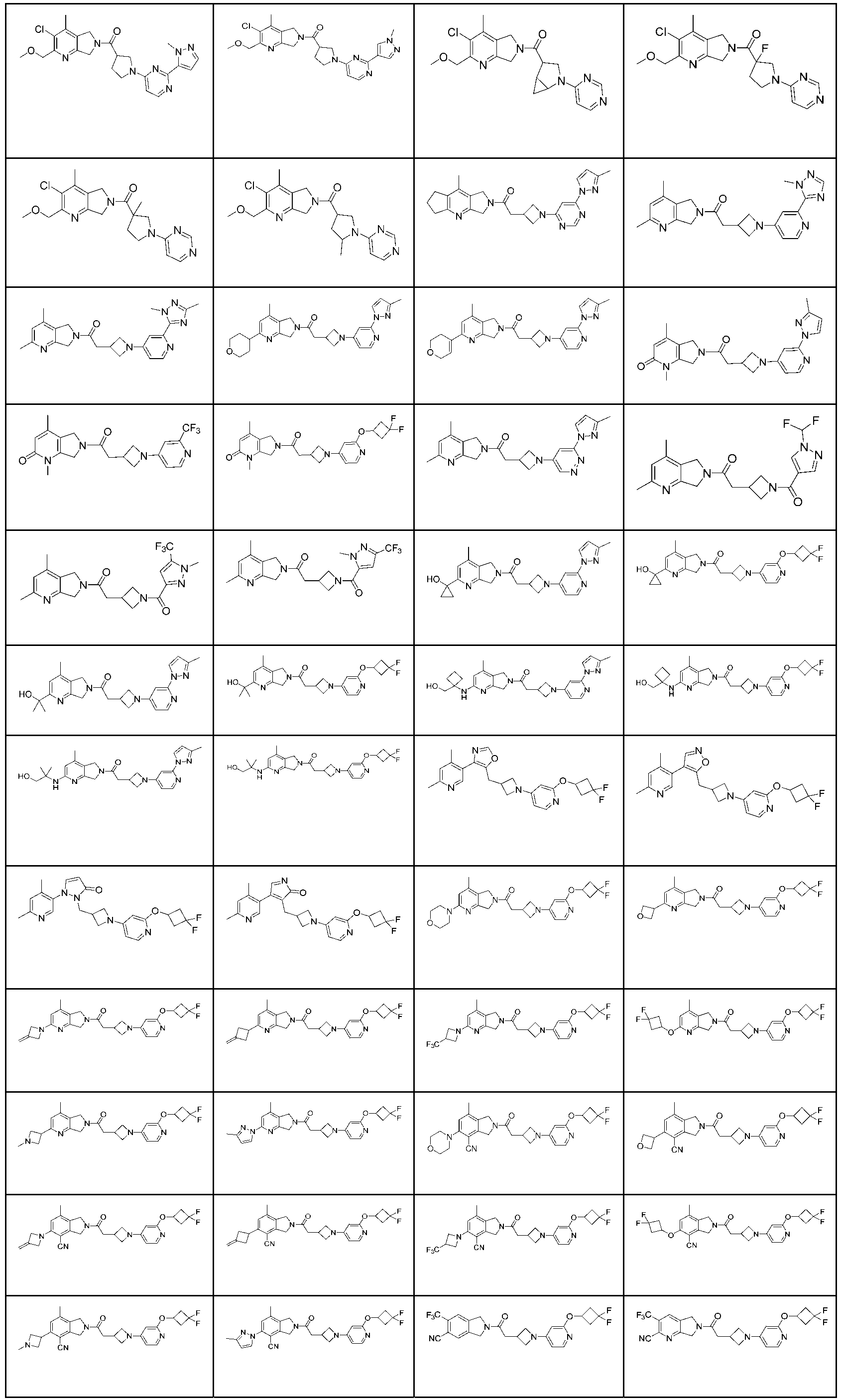

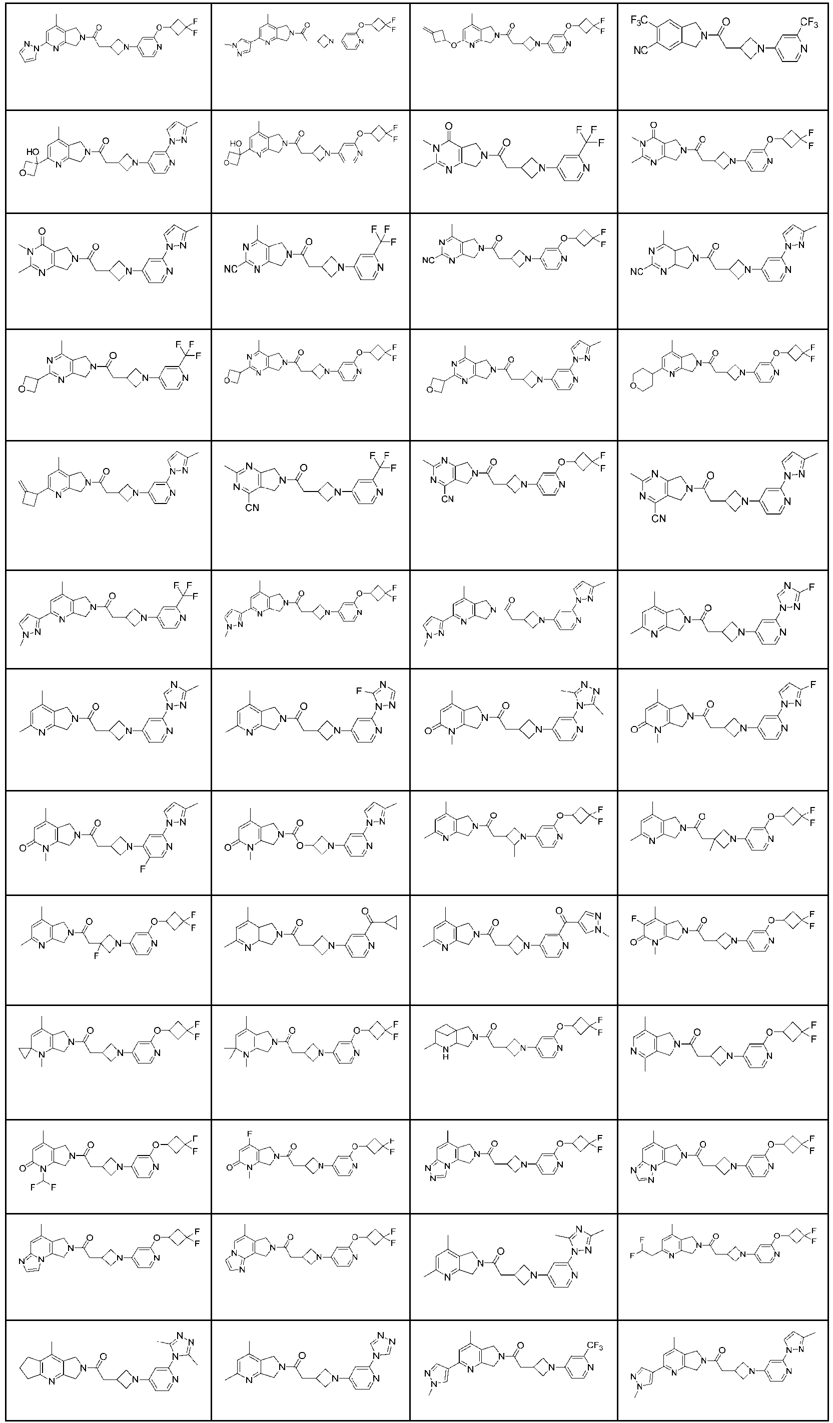

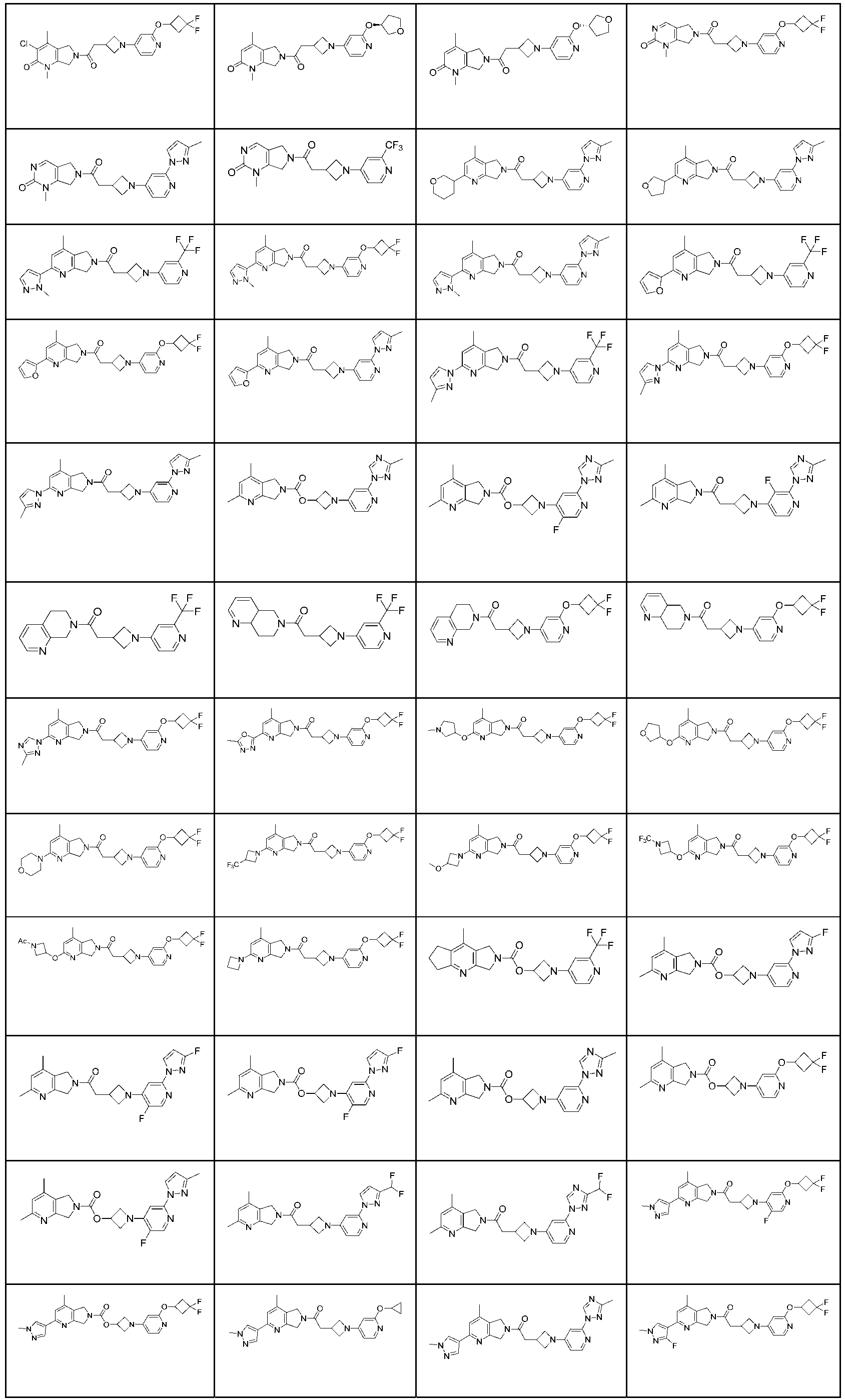

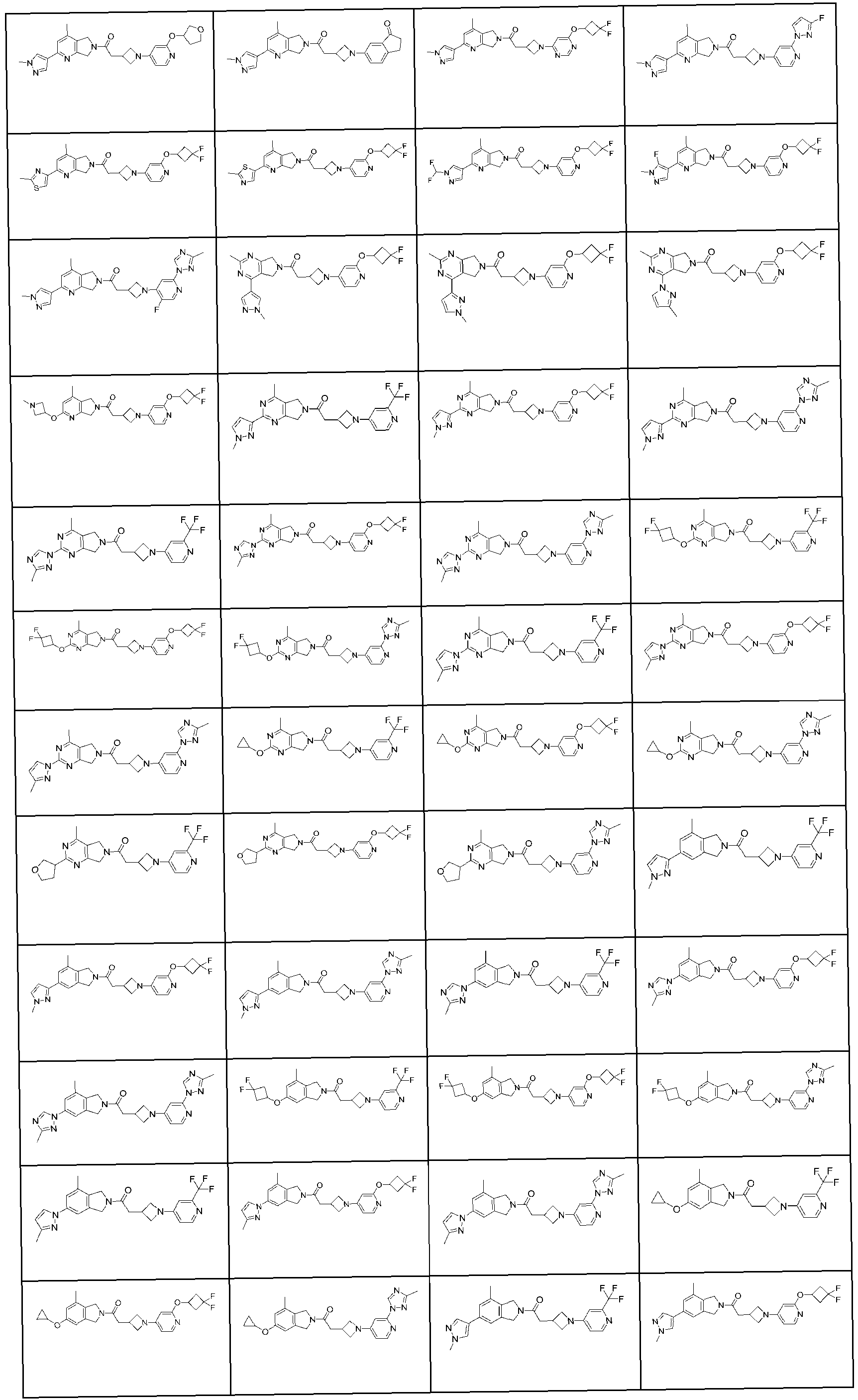

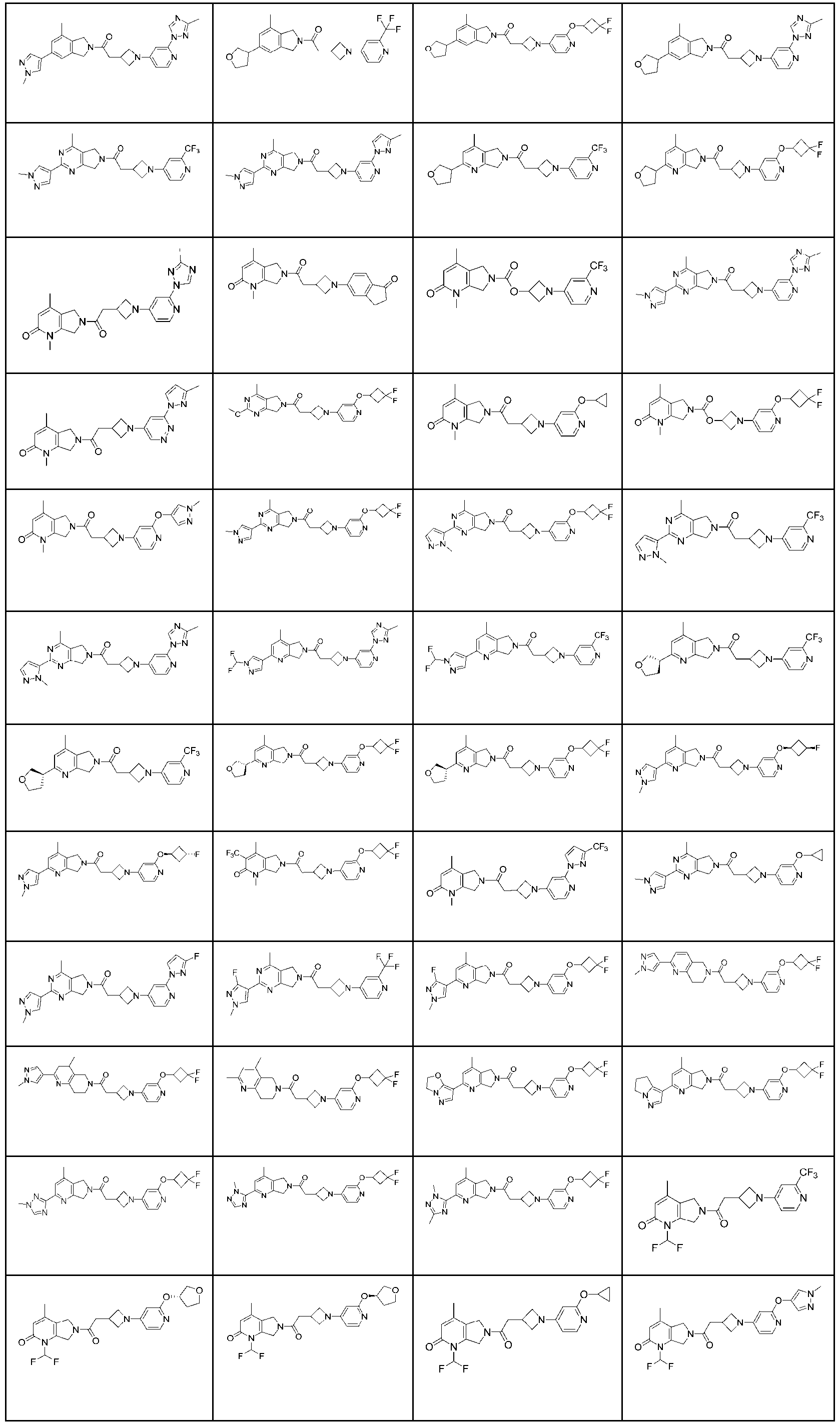

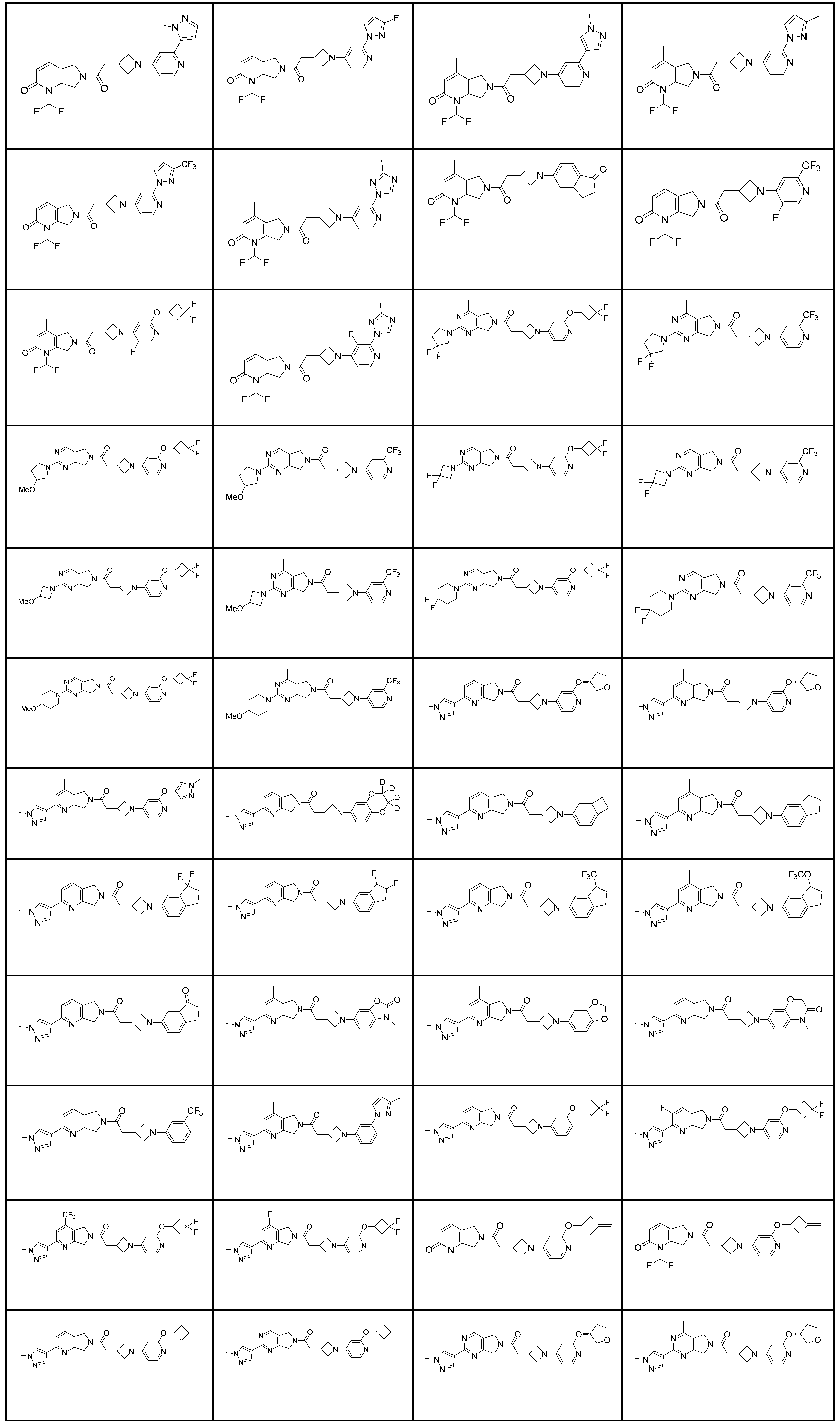

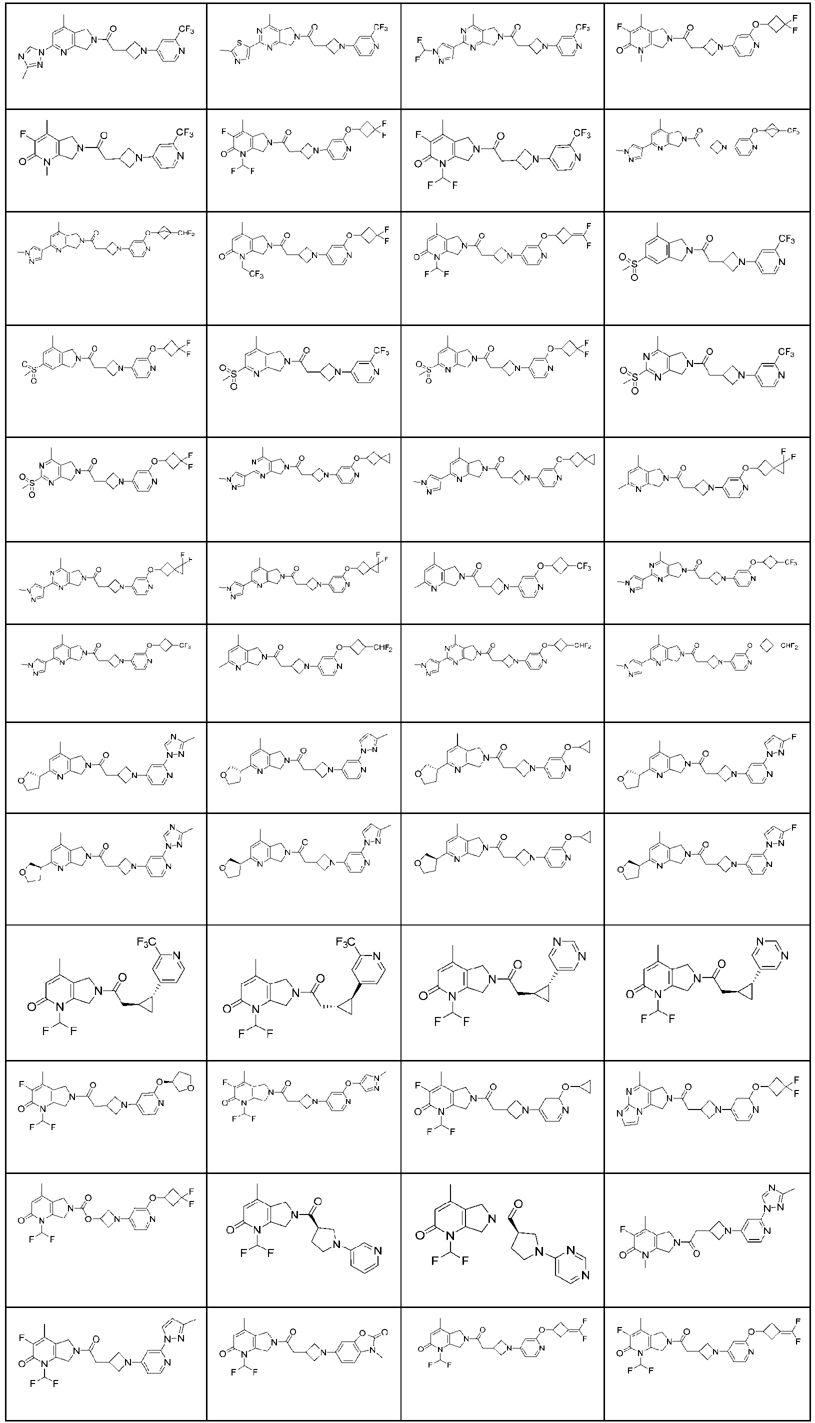

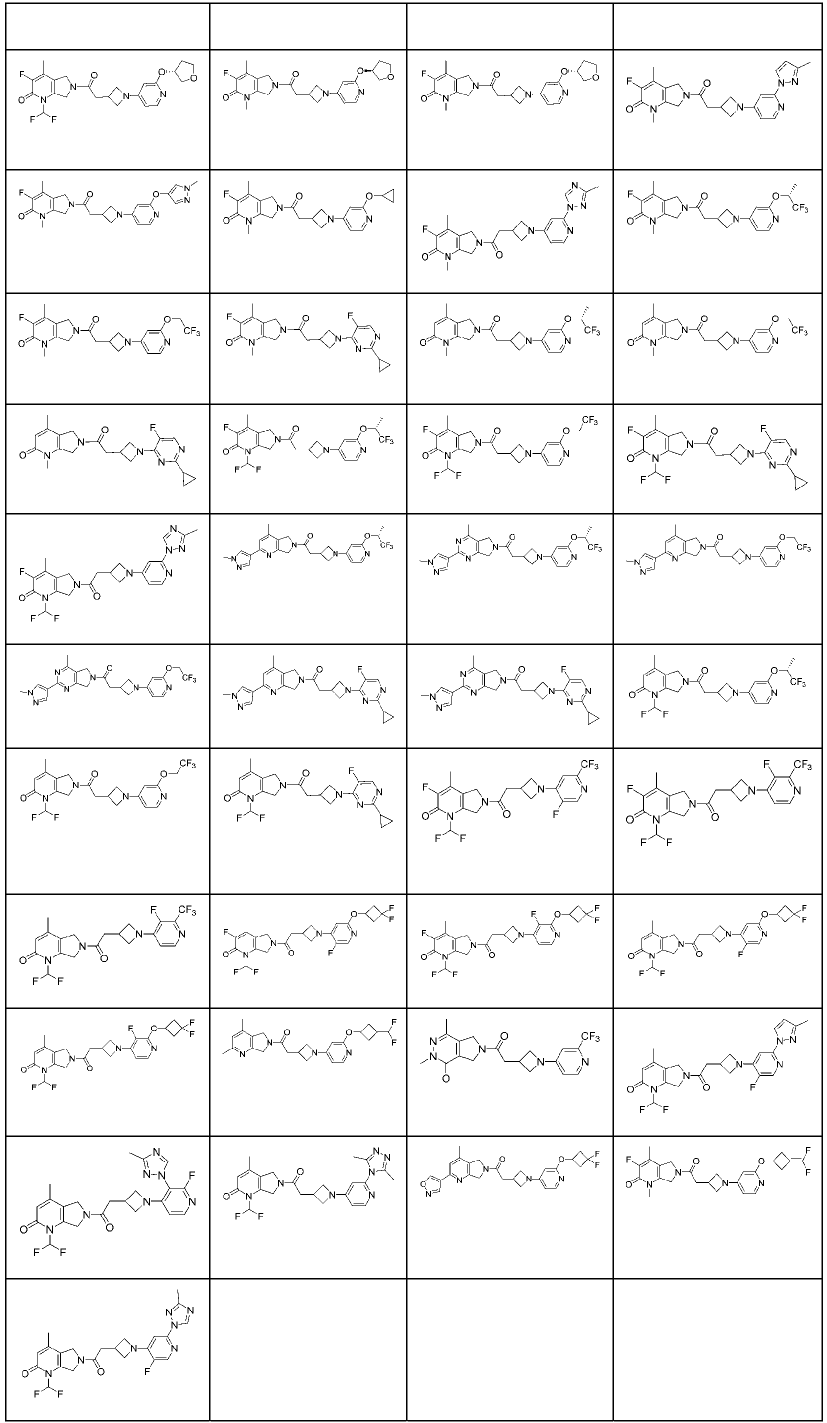

The present invention further provides a pharmaceutical composition or pharmaceutical preparation comprising the compound, or the stereoisomer, the deuterated substance, the solvate, the co-crystal or the pharmaceutically acceptable salt thereof described in any one of the preceding solutions, and a pharmaceutically acceptable carrier and/or excipient.

Further, the composition or pharmaceutical preparation of the present invention contains 1-1500 mg of the compound, or the stereoisomer, the deuterated substance, the solvate, the co-crystal or the pharmaceutically acceptable salt thereof described in any one of the preceding solutions, and a carrier and/or excipient.

The present invention further provides the use of the compound, or the stereoisomer, the deuterated substance, the solvate, the co-crystal or the pharmaceutically acceptable salt thereof described in any one of the preceding solutions, or the composition described in any one of the preceding solutions in the preparation of a drug for treating/preventing a CHRM4-mediated disease; further, the CHRM4-mediated disease is selected from Alzheimer's disease, schizophrenia or psychosis, pain, addiction, sleep disorder, cognitive impairment, Parkinson's disease, Parkinson's disease-levodopa-induced dyskinesia, Huntington's disease, dyskinesia, xerostomia, pulmonary arterial hypertension, chronic obstructive pulmonary disease, asthma, urinary incontinence, glaucoma, trisomy 21 syndrome, cerebral amyloid angiopathy, dementia, hereditary cerebral haemorrhage with amyloidosis-Dutch type, prion disease, amyotrophic lateral sclerosis, progressive supranuclear palsy, head trauma, stroke, pancreatitis, inclusion body myositis, other peripheral amyloidosis, diabetes, autism and atherosclerosis; preferably Alzheimer's disease, schizophrenia, pain, addiction and sleep disorder.

The present invention further provides a method for treating a disease in a mammal or human, comprising administering to a subject a therapeutically effective amount of the compound, or the stereoisomer, the deuterated substance, the solvate or the pharmaceutically acceptable salt thereof described in any one of the preceding solutions, wherein the therapeutically effective amount is preferably 1-1500 mg; the disease is selected from Alzheimer's disease, schizophrenia or psychosis, pain, addiction, sleep disorder, cognitive impairment, Parkinson's disease, Parkinson's disease-levodopa-induced dyskinesia, Huntington's disease, dyskinesia, xerostomia, pulmonary arterial hypertension, chronic obstructive pulmonary disease, asthma, urinary incontinence, glaucoma, trisomy 21 syndrome, cerebral amyloid angiopathy, dementia, hereditary cerebral haemorrhage with amyloidosis-Dutch type, prion disease, amyotrophic lateral sclerosis, progressive supranuclear palsy, head trauma, stroke, pancreatitis, inclusion body myositis, other peripheral amyloidosis, diabetes, autism and atherosclerosis; and the disease is preferably Alzheimer's disease, schizophrenia, pain, addiction and sleep disorder, preferably Alzheimer's disease, schizophrenia, pain, addiction and sleep disorder. In some embodiments, the mammal described in the present invention does not include human.

The "effective amount" or "therapeutically effective amount" described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a kit, which may comprise a composition in a single-dose or multi-dose form, wherein the kit comprises the compound, or the stereoisomer, the deuterated substance or the pharmaceutically acceptable salt thereof according to the present invention, and the amount of the compound, or the stereoisomer, the deuterated substance or the pharmaceutically acceptable salt thereof according to the present invention is the same as that in the above-mentioned pharmaceutical composition.

In the present invention, the amount of the compound, or the stereoisomer or the pharmaceutically acceptable salt thereof according to the present invention is calculated in the form of a free base in each case.

The term "preparation strength" refers to the weight of the active drug contained in each vial, tablet or other unit preparation.

### Synthetic route

Patent documents such as WO 2018002760 A1 have introduced a method for preparing a CHRM4 receptor agonist, and those skilled in the art would have been able to prepare the compounds of the present invention in conjunction with this document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalogue are optionally prepared by custom chemical synthesis plants, wherein many standard chemical supply plants provide custom synthesis services.

### Terms

Unless otherwise specified in the present invention, the terms of the present invention have the following meanings.

Unless otherwise specified in the present invention, the terms of the present invention have the following meanings.

The carbon, hydrogen, oxygen, sulphur, nitrogen and halogen involved in the groups and compounds of the present invention all include isotopes thereof, and are optionally further replaced by one or more of the corresponding isotopes thereof, wherein the isotopes of carbon include ¹²C, ¹³C and ¹⁴C; the isotopes of hydrogen include protium (H), deuterium (D, also known as heavy hydrogen) and tritium (T, also known as superheavy hydrogen); the isotopes of oxygen include ¹⁶O, ¹⁷O and ¹⁸O; the isotopes of sulphur include ³²S, ³³S, ³⁴S and ³⁶S; the isotopes of nitrogen include ¹⁴N and ¹⁵N; the isotope of fluorine includes ¹⁹F; the isotopes of chlorine include ³⁵Cl and ³⁷Cl; and the isotopes of bromine include ⁷⁹Br and ⁸¹Br.

The term "halogen" herein refers to F, Cl, Br, I, or isotopes thereof.

The term "halo" or "substituted with halogen" means that a hydrogen atom is substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen.

The term "deuterated" or "deuterated substance" refers to the case where a hydrogen atom on alkyl, cycloalkyl, alkylene, aryl, heteroaryl, mercapto, heterocycloalkyl, alkenyl, alkynyl and other groups is substituted with at least one isotope deuterium, wherein the upper limit of the number of deuterium substituents is equal to the sum of the number of hydrogens that can be substituted in the group to be substituted. Without particular limitation, the number of deuterium substituents is any integer between 1 and the upper limit, preferably 1-20 deuterium atoms, more preferably 1-10 deuterium atoms, more preferably 1-6 deuterium atoms, and further preferably 1-3 deuterium atoms.

The term "alkyl" refers to a monovalent linear or branched saturated aliphatic hydrocarbon group. Unless otherwise specially specified, the alkyl refers to an alkyl group comprising 1 to 20 carbon atoms, preferably an alkyl group comprising 1 to 8 carbon atoms, more preferably an alkyl group comprising 1 to 6 carbon atoms, further preferably an alkyl group comprising 1 to 4 carbon atoms, and further preferably an alkyl group comprising 1-2 carbon atoms. Non-limiting examples include methyl, ethyl, n-propyl, isopropyl, n-butyl, sec-butyl, neobutyl, tert-butyl, n-pentyl, isoamyl, neopentyl, n-hexyl, and various branched isomers thereof.

The term "alkylene" refers to a divalent linear or branched saturated alkyl, and examples of alkylene include, but are not limited to, methylene, ethylene, propylene, butylene, etc.

The term "cycloalkyl" refers to a monovalent non-aromatic, partially unsaturated or fully saturated, substituted or unsubstituted carbocyclic hydrocarbon group, which generally has 3 to 12 carbon atoms, preferably 3-10 carbon atoms, more preferably 3-6 carbon atoms, and further preferably 3-4 carbon atoms, unless otherwise specially specified. Non-limiting examples include cyclopropyl, cyclobutyl, cyclopentyl, cyclohexyl, 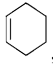 , cycloheptyl, etc.

The term "cycloalkylene" refers to a divalent group of "cycloalkyl", and non-limiting examples include cyclopropylene, cyclobutylene, etc.

The term "heterocyclic ring" or "heterocyclyl" refers to a substituted or unsubstituted, saturated or unsaturated, aromatic or non-aromatic ring comprising 1 to 3 heteroatoms selected from N, O or S unless otherwise specifically defined, including monocyclic heterocyclic rings, bicyclic bridged heterocyclic rings, bicyclic fused heterocyclic rings, bicyclic spiro heterocyclic rings, tricyclic fused heterocyclic rings, etc., which are 3- to 14-membered heterocyclic rings, more preferably 4- to 12-membered heterocyclic rings, more preferably 4- to 10-membered heterocyclic rings, and further preferably 4- to 7-membered heterocyclic rings unless otherwise specifically defined. The definition thereof comprises heterocycloalkyl and heteroaryl. The N and S in the heterocyclyl ring may be oxidized into various oxidation states. Heterocyclyl can be connected to a heteroatom or a carbon atom, and non-limiting examples include oxiranyl, aziridinyl, oxetanyl, azetidinyl, 1,3-dioxolanyl, 1,4-dioxolanyl, 1,3-dioxanyl, azepanyl, pyridinyl, furyl, thienyl, pyranyl, N-alkylpyrrolyl, pyrimidinyl, pyrazinyl, pyrazolyl, pyridazinyl, imidazolyl, piperidinyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzimidazolyl, benzopyridinyl, pyrrolopyridinyl, benzodihydrofuryl, azabicyclo[3.2.1]octyl, azabicyclo[5.2.0]nonanyl, oxatricyclo[5.3.1.1]dodecyl, azaadamantyl, oxaspiro[3.3]heptyl, 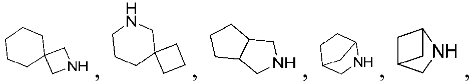 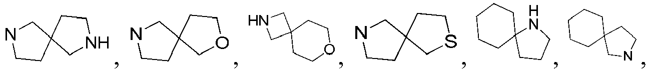 etc.

The term "heterocyclylene" is a divalent group corresponding to "heterocyclyl", and non-limiting examples include imidazolylene, piperidinylene, aziridinylene, etc.

The term "carbocyclic ring" or "carbocyclyl" refers to a substituted or unsubstituted, saturated or unsaturated, aromatic or non-aromatic carbocyclic group, including monocyclic carbocyclic rings, bicyclic bridged rings, bicyclic fused rings, bicyclic spiro rings, etc., which have 3 to 12 carbon atoms, preferably 3-10 carbon atoms, and further preferably 3-6 carbon atoms unless otherwise specially specified. The definition thereof comprises cycloalkyl and aryl. In non-limiting examples, monocyclic carbocyclic rings include cyclopropyl, cyclobutyl, cyclopentyl, cyclohexyl, cycloheptyl, phenyl, 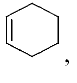 etc.; bicyclic bridged rings include 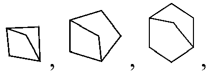 etc.; bicyclic fused rings include 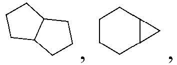 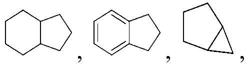 etc.; and bicyclic spiro rings include 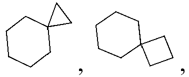 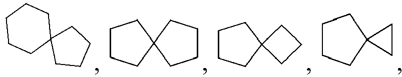 etc.

The term "aryl" refers to an aromatic carbocyclic ring. Non-limiting examples include phenyl, naphthyl, etc.

The term "alkynyl" refers to a monovalent linear or branched unsaturated hydrocarbon group containing one or more carbon-carbon triple bonds. Unless otherwise specifically specified, the alkynyl contains 2-6 carbon atoms, preferably 2-4 carbon atoms, and non-limiting examples include ethynyl, propynyl, propargyl, etc.

The term "alkenyl" refers to a monovalent linear or branched unsaturated hydrocarbon group containing one or more carbon-carbon double bonds. Unless otherwise specifically specified, the alkynyl contains 2-6 carbon atoms, preferably 2-4 carbon atoms, and non-limiting examples include vinyl, propenyl, allyl, 2-butenyl, 1-butenyl, etc.

The term "alkoxy" or "alkyloxy" refers to -O-alkyl. Without particular limitation, the alkoxy or alkyloxy is -O-C₁₋₈ alkyl, preferably -O-C₁₋₆ alkyl, more preferably -O-C₁₋₄ alkyl, and further preferably -O-C₁₋₂ alkyl. Non-limiting examples include methoxy, ethoxy, n-propoxy, isopropoxy, n-butoxy, secbutoxy, tert-butoxy, n-pentoxy, n-hexyloxy, cyclopropoxy, cyclobutoxy, etc.

The term "haloalkoxy" refers to -O-haloalkyl. Without particular limitation, the haloalkoxy is -O-halogenated C₁₋₈ alkyl, preferably -O-halogenated C₁₋₆ alkyl, more preferably -O-halogenated C₁₋₄ alkyl, and further preferably -O-halogenated C₁₋₂ alkyl. Non-limiting examples include monofluoromethoxy, difluoromethoxy, trifluoromethoxy, difluoroethyloxy, etc.

The term "C₁₋₄ alkylacyl" refers to C₁₋₄ alkyl-C(O)-. Non-limiting examples include formyl, acetyl, and propionyl.

The term "C₁₋₄ alkylsulphonyl" refers to C₁₋₄ alkyl-S(O)₂-. Non-limiting examples include methylsulphonyl, ethylsulphonyl, and propylsulphonyl.

The term "heteroaromatic ring" or "heteroaryl" refers to an aromatic heterocyclic ring. Non-limiting examples include pyrazolyl, pyrimidinyl, thiazolyl, pyridinyl, furyl, pyrone, pyridone, etc.

The term "heterocycloalkyl" refers to a non-aromatic, partially unsaturated or fully saturated heterocyclic ring, which generally has 4 to 12 ring members, preferably 4 to 10 ring members, more preferably 4 to 7 ring members, and further preferably 5 or 6 ring members. In addition to carbon atoms, heterocycloalkyl also comprises 1-3 heteroatoms selected from N, S and O as ring members. Non-limiting examples include azetidinyl, morpholinyl, piperazinyl, piperidinyl, tetrahydropyranyl, oxetanyl, etc.

The term "optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

When the specific structures or segments enumerated in the present invention are further substituted, the hydrogen atoms shown in the specific structures may also be optionally substituted. For example, in general formulas such as formula (I) and (II), ring A is selected from 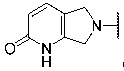 optionally further substituted with 1, 2 or 3 R^{A}, which includes that the hydrogen atoms on nitrogen in the 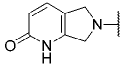 structure may still be substituted with R^{A}.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or co-crystals thereof as described herein and other components including physiologically/pharmaceutically acceptable carriers and/or excipients.

The term "carrier" refers to a system that does not cause significant irritation to the organism and does not eliminate the biological activity and characteristics of the administered compound and can change the way the drug enters the human body and the distribution of the drug in the body, control the release rate of the drug and deliver the drug to targeted organs. Non-limiting examples of the carrier include microcapsule, microsphere, nanoparticle, liposome, etc.

The term "excipient" refers to a substance that is not a therapeutic agent per se, but used as a diluent, adjuvant, binder and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrant, an absorbent, a preservative, a surfactant, a colourant, a flavouring agent, and a sweetening agent. Examples of pharmaceutically acceptable excipients include, but are not limited to: (1) sugars, such as lactose, glucose, and sucrose; (2) starches, such as corn starch and potato starch; (3) cellulose and derivatives thereof, such as sodium carboxymethyl cellulose, ethyl cellulose, cellulose acetate, hydroxypropyl methylcellulose, hydroxypropyl cellulose, microcrystalline cellulose, and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil, and soybean oil; (10) diols, such as propylene glycol; (11) polyols, such as glycerol, sorbitol, mannitol, and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffer solutions; (21) polyesters, polycarbonates and/or polyanhydrides; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The compounds of the present invention may exist in specific geometric or stereoisomeric forms. All such compounds of the present invention include cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present invention . Additional asymmetric carbon atoms may be present in the substituents of the compounds of the present invention. All such isomers, as well as mixtures thereof, are included within the scope of the present invention. In certain embodiments, preferred compounds are those isomeric compounds that exhibit superior biological activity. Purified or partially purified isomers and stereoisomers, or racemic mixtures or diastereomeric mixtures of the compounds of the present invention are also included within the scope of the present invention. Purification and isolation of such materials can be achieved by standard techniques known in the art.

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

### Detailed Description of Embodiments

The content of the present invention is described in detail by means of the following examples. In examples in which no specific conditions are indicated, experimental methods are carried out under conventional conditions. The listed examples are intended to better illustrate the content of the present invention but should not be construed as limiting the content of the present invention. Non-essential improvements and adjustments made to the embodiments by a person of ordinary skill in the art according to the above Summary of the Invention still fall within the scope of protection of the present invention.

### Test method

The structures of the compounds are determined by nuclear magnetic resonance (NMR) or (and) mass spectrometry (MS). The NMR shift (δ) is given in the unit of 10-6 (ppm). NMR is determined with Bruker Avance III 400 and Bruker Avance 300; the solvent for determination is deuterated dimethyl sulphoxide (DMSO-d6), deuterated chloroform (CDCl3) and deuterated methanol (CD3OD); and the internal standard is tetramethylsilane (TMS);
MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 µM).

Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

### Example 1:

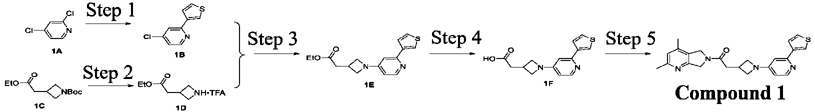

Step 1: Compound **1A** (1.4 g, 9.44 mmol), 3-thiopheneboronic acid (1.81 g, 14.16 mmol), and tetrakis(triphenylphosphine)palladium (0.55 g, 0.47 mmol) were successively dissolved in a mixed solution of toluene/ethanol (24 mL/6 mL), and bubbled with nitrogen for 5 minutes. 2 M potassium carbonate solution (10 mL) was added, and the resulting mixture was bubbled with nitrogen for another 5 minutes. Subsequently, under nitrogen atmosphere, the mixture was warmed to 90°C and reacted overnight. The reaction solution was cooled to room temperature, and then extracted with ethyl acetate (30 mL×2). The combined organic phases were washed with saturated aqueous sodium chloride solution, dried over anhydrous sodium sulphate, filtered, and concentrated to obtain a crude product. The crude product was purified using Biotage Isolera One (20 g silica gel column, eluents: 0-30% ethyl acetate/petroleum ether) to obtain compound **1B** (1.50 g, 81.21%).

LC-MS (ESI): m/z =196.1 [M+H]⁺.

Step 2: Compound **1C** (1.0 g, 4.11 mmol) was dissolved in dichloromethane (10 mL), and then trifluoroacetic acid (4.0 mL) was added dropwise. After the dropwise addition was completed, the mixture was reacted at room temperature overnight. The reaction solution was concentrated to obtain the crude compound **1D,** which was directly used in the next step.

LC-MS (ESI): m/z =144.1 [M+H]⁺.

Step 3: Compound **1B** (0.5 g, 2.56 mmol) was dissolved in dimethyl sulphoxide (10 mL), and then the crude compound **1D** obtained in step 2, DIPEA (0.99 g, 7.66 mmol), and caesium fluoride (0.39 g, 2.56 mmol) were successively added. After the addition, the mixture was warmed to 100°C and reacted overnight. After the reaction was completed, the mixture was cooled to room temperature, water (30 mL) was added and the resulting mixture was extracted with ethyl acetate (30 mL×2). The combined organic phases were washed with saturated aqueous sodium chloride solution, dried over anhydrous sodium sulphate, filtered, and concentrated to obtain a crude product. The crude product was purified using Biotage Isolera One (20 g silica gel column, eluents: 0-50% ethyl acetate/petroleum ether) to obtain compound **1E** (0.31 g, 40.05%).

LC-MS (ESI): m/z =303.1 [M+H]⁺.

Step 4: Compound **1E** (0.3 g, 0.99 mmol) was dissolved in tetrahydrofuran (3 mL), and then lithium hydroxide monohydrate (0.083g, 1.98 mmol) and water (3 mL) were successively added. The mixture was stirred at room temperature overnight. After the reaction was completed, the reaction mixture was adjusted to pH 5-6 by dropwise adding 5% potassium bisulphate solution, and concentrated to remove the solvent. Tetrahydrofuran (5 mL) was added and the resulting mixture was stirred evenly, and then concentrated to dryness. Subsequently, methanol (10 mL) was added to the residue, and the mixture was stirred and filtered. The filter cake was washed with methanol (5 mL×2), and the filtrates were combined and concentrated to dryness to obtain compound **1F** (0.19 g, 69.96%).

LC-MS (ESI): m/z=275.1 [M+H]⁺.

Step 5: Compound **1F** (150 mg, 0.55 mmol), 2,4-dimethyl-6,7-dihydro-5H-pyrrolo[3,4-B]pyridine dihydrochloride **(intermediate 1)** (0.15 g, 0.83 mmol), and DIPEA (0.36 g, 2.78 mmol) were successively added to pyridine (10 mL), and the mixture was cooled to 0°C. Then T3P (1.04 g, 50% in EA, 1.63 mmol) was added dropwise. After the dropwise addition was completed, the mixture was naturally warmed to room temperature and reacted overnight. The reaction mixture was concentrated, and then ethyl acetate (30 mL) was added to the residue. The resulting mixture was stirred and diluted. 10% aqueous potassium carbonate solution (30 mL) was added and the mixture was stirred for 10 minutes and left to stand for layer separation. The separated organic phase was washed with saturated aqueous sodium chloride solution, dried over anhydrous sodium sulphate, and concentrated to obtain a product crude. The crude product was purified using Biotage Isolera One (20 g silica gel column, eluents: 0-50% ethyl acetate/petroleum ether) to obtain **compound 1** (85 mg, 38.20%).

LC-MS (ESI): m/z=405.4[M+H]⁺.

¹H NMR (400 MHz, CDCl₃) δ 8.25 (d, 1H), 7.87 (s, 1H), 7.58 (d, 1H), 7.36 (dd, 1H), 6.91(d, 1H), 6.56(s, 1H), 6.20-6.18 (m, 1H), 4.79-4.73 (m, 4H), 4.30-4.25 (m, 2H), 3.77-3.72 (m, 2H), 3.33-3.28 (m, 1H), 2.82 (t, 2H), 2.52 (d, 3H),2.27 (d, 3H).

### Example 2:

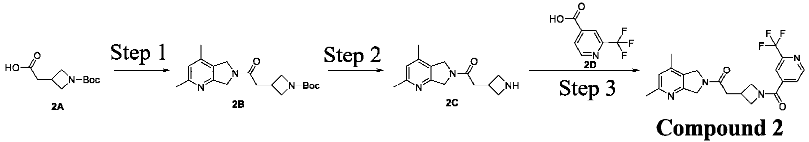

Step 1: **2A** (0.35 g, 1.62 mmol), **intermediate 1** (0.2 g, 1.35 mmol), N-methylimidazole (0.23 g, 2.76 mmol), and TEA (0.0.41 g, 4.05 mmol) were successively dissolved in DCM (10 mL), and the mixture was stirred at room temperature for half an hour. Then TCFH (0.45 g, 1.61 mmol) was added and the mixture was reacted at room temperature for another 2 h. The reaction solution was poured into water, and the resulting mixture was extracted with ethyl acetate three times. The combined organic phases were washed with saturated aqueous sodium chloride solution, dried over anhydrous sodium sulphate, filtered, and concentrated under reduced pressure to obtain a crude product. The crude product was purified using a medium-pressure preparation instrument Biotage Isolera One (12 g silica gel column, eluents: 0-5% MeOH/DCM) to obtain the target compound **2B** (0.25 g, yield: 53.61%).

LC-MS (ESI): m/z =346.2 [M+H]⁺.

Step 2: **2B** (0.25 g, 0.69 mmol) was dissolved in DCM (10 mL), then TFA (3 mL) was added, and the resulting mixture was reacted at room temperature for 2 h. After the reaction was completed, the reaction mixture was concentrated, and the residue was dissolved in ethyl acetate. Then saturated aqueous sodium bicarbonate solution was added dropwise, and the resulting mixture was adjusted to a basic pH and extracted. The aqueous phase was extracted with ethyl acetate twice, and the combined organic phases were washed with saturated aqueous sodium chloride solution, dried over anhydrous sodium sulphate, filtered, and concentrated to obtain **2C** (0.13 g, yield: 76.80%).

LC-MS (ESI): m/z =246.2 [M+H]⁺.

Step 3: **2C** (0.13 g, 0.53 mmol), **2D** (0.15g, 0.8 mmol), and N-methylimidazole (0.13 g, 1.59 mmol) were successively dissolved in DCM (10 mL). The mixture was stirred at room temperature for half an hour, TCFH (0.18 g, 0.64 mmol) was added, and the resulting mixture was reacted at room temperature for another 2 h. After the reaction was completed, the reaction solution was poured into water, and the resulting mixture was extracted with ethyl acetate three times. The combined organic phases were washed with saturated aqueous sodium chloride solution, dried over anhydrous sodium sulphate, filtered, and concentrated under reduced pressure to obtain a crude product. The crude product was purified using a medium-pressure preparation instrument Biotage Isolera One (12 g silica gel column, eluents: 0-5% MeOH/DCM) to obtain the target **compound 2** (0.08 g, yield: 36.08%).

LC-MS (ESI): m/z =419.1 [M+H]⁺.

¹H NMR (400 MHz, Chloroform*-d*) δ 8.85-8.79 (m, 1H), 7.93-7.86 m, 1H), 7.72-7.65 (m, 1H), 6.94-6.88 (m, 1H), 4.78-4.68 (m, 4H), 4.67-4.55 (m, 1H), 4.51-4.38 (m, 1H), 4.14-4.06 (m, 1H), 4.03-3.94 (m, 1H), 3.33-3.17 (m, 1H), 2.86-2.67 (m, 2H), 2.55-5.50 (m, 3H), 2.28-2.23 (m, 3H).

### Example 3:

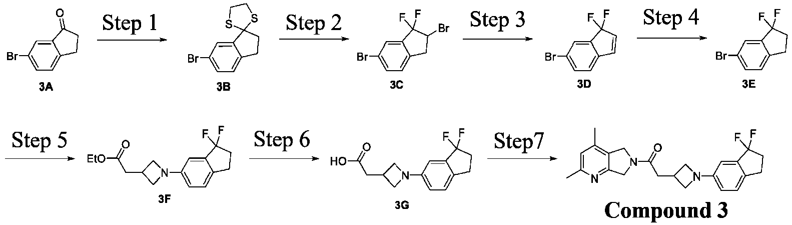

Step 1: Compound **3A** (10.0 g, 47.85 mmol) was dissolved in toluene (100 mL), p-toluenesulphonic acid (1.6 g, 9.57 mmol) and ethanedithiol (5.4 g, 57.42 mmol) were added, and the resulting mixture was reacted at 130°C for 12 hours. After the reaction was completed, the reaction mixture was concentrated. The residue was subjected to silica gel column chromatography (petroleum ether: ethyl acetate (v/v)=5:1) to obtain compound **3B** (10.0 g, 73.1%).

LCMS (ESI): m/z =287.1 [M+H]⁺.

Step 2: Dibromohydantoin (32.0 g, 111.88 mmol) was dissolved in dichloromethane (300 mL), and pyridine hydrofluoride (22.2 g, 223.76 mmol) was added at -78°C. A solution of compound **3B** (8.0 g, 27.97 mmol) in dichloromethane (80 mL) was added dropwise. After the dropwise addition was completed, the mixture was naturally warmed to room temperature and reacted for 2 hours. Water (300 mL) was added and the mixture was extracted. The aqueous phase was further extracted with dichloromethane (300 mL). The combined organic phases were washed with saturated aqueous sodium chloride solution (300 mL), dried over anhydrous sodium sulphate, filtered, and then concentrated. The residue was subjected to silica gel column chromatography (petroleum ether: ethyl acetate (v/v)=100:0) to obtain compound **3C** (4.8 g, 55.4%).

¹H NMR (400 MHz, CDCl₃) δ 7.83-7.69 (m, 1H), 7.67-7.56 (m, 1H), 7.20-7.11 (m, 1H), 4.66- 4.46 (m, 1H), 3.60-3.41 (m, 1H), 3.30-3.11 (m, 1H).

Step 3: Compound **3C** (4.7 g, 15.16 mmol) was dissolved in dichloromethane (50 mL), 1,8-diazabicyclo[5.4.0]undec-7-ene (4.7 g, 15.16 mmol) was added, and the resulting mixture was reacted at room temperature for 12 hours. After the reaction was completed, the reaction mixture was concentrated. The residue was subjected to silica gel column chromatography (petroleum ether: ethyl acetate (v/v)=100:0) to obtain compound **3D** (2.7 g, 77.4%).

¹H NMR (400 MHz, CDCl₃) δ7.61-7.55 (m, 1H), 7.54-7.40 (m, 1H), 7.03-6.95 (m, 1H), 6.77- 6.72 (m, 1H), 6.21-6.09 (m, 1H).

Step 4: Compound **3D** (1.0 g, 4.35 mmol) and 2-nitrobenzenesulphonyl chloride (1.2 g, 5.22 mmol) were dissolved in acetonitrile (20 mL), and potassium phosphate (184 mg, 0.87 mmol) and hydrazine hydrate (873 mg, 1.74 mmol) were added at 0°C. Subsequently, the resulting mixture was reacted at room temperature for 12 hours. After the reaction was completed, the reaction mixture was concentrated. The residue was subjected to silica gel column chromatography (petroleum ether: ethyl acetate (v/v)= 100:0) to obtain compound **3E** (0.9 g, 89.9%).

¹H NMR (400 MHz, CDCl₃) δ7.72-7.63 (m, 1H), 7.55-7.50 (m, 1H), 7.19-7.12 (m, 1H), 3.02- 2.92 (m, 2H), 2.67-2.49 (m, 2H).

Step 5: Compound **3E** (500 mg, 2.26 mmol) was dissolved in toluene (15 mL), and ethyl 2-(azetidin-3-yl)acetate trifluoroacetate (580 mg, 2.26 mmol), caesium carbonate (2.2 g, 6.78 mmol), tris(dibenzylideneacetone)dipalladium (210 mg, 0.23 mmol), and 4,5-bisdiphenylphosphino-9,9-dimethylxanthene (260 mg, 0.45 mmol) were successively added. Subsequently, under nitrogen atmosphere, the mixture was warmed to 100°C and reacted for 12 hours. After the reaction was completed, the reaction mixture was filtered, and the filtrate was concentrated. The resulting residue was separated by silica gel column chromatography (petroleum ether: ethyl acetate (v/v)= 5:1) to obtain compound **3F** (0.3 g, 45.0%).

LCMS (ESI): m/z =296.1 [M+H]⁺.

Step 6: Compound **3F** (300 mg, 1.02 mmol) was dissolved in methanol (3 mL), a solution of lithium hydroxide (300 mg) in water (3 mL) was added, and the resulting mixture was reacted at room temperature for 1 hour. The mixture was adjusted to pH=5-6 by dropwise adding hydrochloric acid (6 N), and extracted with dichloromethane (30 mL×2). The combined organic phases were dried over anhydrous sodium sulphate, filtered, and concentrated to obtain compound **3G** (150 mg, 55.1%).

LCMS (ESI): m/z =268.1 [M+H]⁺.

Step 7: Compound **3G** (150 mg, 0.56 mmol), benzotriazole-N,N,N',N'-tetramethyluronium hexafluorophosphate (213 mg, 0.56 mmol), 2,4-dimethyl-6,7-dihydro-5H-pyrrolo[3,4-B]pyridine dihydrochloride (123 mg, 0.56 mmol) and N,N-diisopropylethylamine (361 mg, 2.8 mmol) were successively dissolved in DMF (5 mL), and the resulting mixture was reacted at room temperature for 1 hour. Water (10 mL) was added, and the resulting mixture was extracted with ethyl acetate (10 mL x 3). The combined organic phases were washed with water (10 mL) and saturated aqueous sodium chloride solution (10 mL), dried over anhydrous sodium sulphate, filtered, and concentrated. The residue was subjected to silica gel column chromatography (dichloromethane: methanol (v/v)= 100:6) to obtain the title **compound 3** (55 mg, 24.8%).

LC-MS (ESI): m/z =398.2 [M+H]⁺.

¹H NMR (400 MHz, CDCl₃) δ 7.15-7.04 (m, 1H), 6.93-6.87 (m, 1H), 6.61-6.48 (m, 2H), 4.83- 4.68 (m, 4H), 4.25-4.09 (m, 2H), 3.65-3.56 (m, 2H), 3.30-3.13 (m, 1H), 2.99-2.87 (m, 2H), 2.87-2.75 (m, 2H), 2.61-2.44 (m, 5H), 2.33-2.19 (m, 3H).

### Example 4:

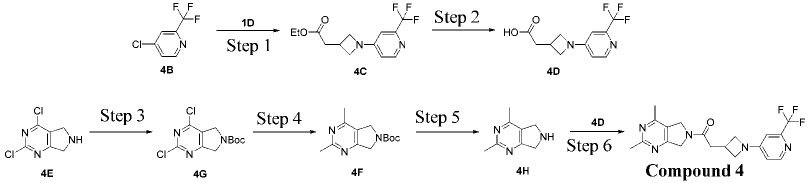

Step 1: Compound **4B** (1.18 g, 8.24 mmoL), potassium carbonate (2.85 g, 20.57 mmoL), and 2-trifluoromethyl-4-chloropyridine (1.50 g, 8.24 mmoL) were successively dissolved in dimethyl sulphoxide (30 mL). The mixture was stirred evenly and then warmed to 100°C and reacted for 2 hours. After the reaction was completed, the mixture was cooled to room temperature. Ethyl acetate (200 mL) and saturated aqueous sodium chloride solution (200 mL) were added and the mixture was subjected to extraction and phase separation. The organic phase was washed with saturated aqueous sodium chloride solution three times, then dried over anhydrous sodium sulphate, filtered, and concentrated. The residue was separated and purified by silica gel column chromatography (ethyl acetate: petroleum ether (v:v)=0-100%) to obtain compound **4C** (0.8 g, 33.68%).

LC-MS (ESI): m/z=289.2 [M+H]⁺.

Step 2: Compound **4C** (0.8 g, 2.78 mmoL) and lithium hydroxide (0.1 g, 4.18 mmoL) were successively dissolved in a mixed solution of methanol (20 mL) and water (0.5 mL), and the mixture was warmed to 50°C and reacted for 2 hours. After the reaction was completed, the reaction mixture was concentrated under reduced pressure to obtain crude compound **4D** (0.8 g).

LC-MS (ESI): m/z=261.1 [M+H]⁺.

Step 3: 2,4-Dichloro-6,7-dihydro-5H-pyrrolo[3,4-D]pyrimidine hydrochloride (5.00 g, 22.08 mmoL) and triethylamine (3.35 g, 33.12 mmoL) were successively dissolved in dichloromethane (100 mL), di-tert-butyl dicarbonate (4.82 g, 22.08 mmoL) was added, and the mixture was reacted at room temperature overnight. After the reaction was completed, the organic phase was washed with water three times, dried over anhydrous sodium sulphate, filtered, and concentrated under reduced pressure. The residue was separated and purified by silica gel column chromatography (ethyl acetate: petroleum ether (v:v) = 0-100%) to obtain compound **4G** (6 g, 93.65%).

LC-MS (ESI): m/z=290.1 [M+H]⁺.

Step 4: Compound **4G** (3.00 g, 10.33 mmoL) was dissolved in tetrahydrofuran (50 mL). Under nitrogen atmosphere, 2 moL/L methylzinc chloride tetrahydrofuran solution (15 mL) and chloro(2-dicyclohexylphosphino-2',4',6'-triisopropyl-1,1'-biphenyl)[2-(2'-amino-1,1'-biphenyl)]palladium (II) (0.81 g, 1.03 mmoL) were successively added, and then the mixture was warmed to 70°C and reacted overnight. After the reaction was completed, the mixture was cooled to room temperature. The reaction was quenched by adding water (1 mL). The mixture was filtered, and the filtrate was concentrated. The residue was separated and purified by silica gel column chromatography (ethyl acetate: petroleum ether (v:v)=0-100%) to obtain compound **4F** (0.8 g, 31.06%).

LC-MS (ESI): m/z=250.30 [M+H]⁺.

Step 5: Compound **4F** (0.80 g, 3.21 mmoL) was dissolved in 4 moL/L hydrogen chloride 1,4-dioxane (10 mL), and then the mixture was reacted at room temperature for 1 hour. After the reaction was completed, the mixture was concentrated to obtain crude compound **4H** (1.00 g).

LC-MS (ESI): m/z=150.20 [M+H]⁺.

Step 6: Compound **4H** (0.50 g, 3.35 mmoL), compound **4D** (0.87 g, 3.35 mmoL), N,N-diisopropylethylamine (2.16 g, 16.75 mmoL), and 2-(7-azobenzotriazole)-N,N,N',N'-tetramethyluronium hexafluorophosphate (1.53 g, 4.02 mmoL) were successively dissolved in N,N-dimethylformamide (15 mL), and the mixture was reacted at room temperature for 5 hours. After the reaction was completed, the mixture was filtered, and the filtrate was separated and purified using a reverse-phase preparative column (C18 spherical 20-35 nm 100 A 80 g) (acetonitrile: water (v:v) = 10%-80%) to obtain **compound 4** (651 mg, 49.65%).

LC-MS (ESI): m/z = 392.20 [M+H]⁺.

¹H NMR (400 MHz, DMSO*-d6*) δ 8.21 (d, 1H), 6.71 (d, 1H), 6.54-6.52 (m, 1H), 4.90-4.77 (m, 2H), 4.59 (d, 2H), 4.20-4.15 (m, 2H), 3.72-3.68 (m, 2H), 3.19-3.06 (m, 1H), 2.87-2.81 (m, 2H), 2.57 (d, 3H), 2.39 (d, 3H).

### Example 5:

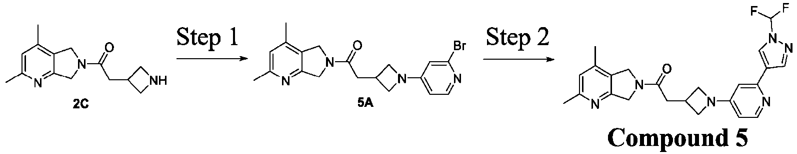

Step 1: **2C** (1.2 g, 1.41 mmol), 2-bromo-4-fluoropyridine (1.03 g, 5.88 mmol), caesium fluoride (894 mg, 5.88 mmol) and triethylamine (1.98 g, 19.61 mmol) were successively dissolved in dimethyl sulphoxide (25 mL), and the mixture was reacted at 100°C overnight. After the reaction was completed, the reaction solution was cooled to room temperature, water (50 mL) was added to the reaction solution, and the resulting mixture was extracted with ethyl acetate (25 mL×3). The organic layers were combined, washed with saturated aqueous sodium chloride solution (30 mL), dried over anhydrous sodium sulphate, and filtered. The filtrate was concentrated and the residue was separated and purified by silica gel column chromatography (dichloromethane: methanol (v/v) = 30 : 1) to obtain **5A** (980 mg, 50%).

LC-MS (ESI): m/z= 401.6 [M+H]⁺.

Step 2: **5A** (330 mg, 0.82 mmol), (1-(difluoromethyl)-1H-pyrazol-4-yl)boronic acid pinacol (241 mg, 0.99 mmol), Pd(dppf)Cl₂ (60 mg, 0.082 mmol) and potassium carbonate (341 mg, 2.47 mmol) were successively dissolved in mixed solvents of 1,4-dioxane (10 mL) and water (1 mL), and under nitrogen atmosphere, the mixture was reacted at 90°C overnight. After the reaction was completed, the reaction solution was cooled to room temperature, water (30 mL) was added to the reaction solution, and the resulting mixture was extracted with ethyl acetate (15 mL×3). The organic layers were combined, washed with saturated aqueous sodium chloride solution (30 mL), dried over anhydrous sodium sulphate, and filtered. The filtrate was concentrated and the residue was separated and purified by silica gel column chromatography (dichloromethane: methanol (v/v) = 20 : 1) to obtain **compound 5** (80 mg, 22%).

¹H NMR (400 MHz, CD₃Cl) δ 8.31 (s, 1H), 8.17-8.15 (m, 1H), 8.00 (s, 1H), 7.29-6.97 (m, 1H), 6.85-6.83 (m, 1H), 6.38-6.37 (m, 1H), 6.14-6.11 (m, 1H), 4.72-4.66 (m, 4H), 4.25-4.19 (m, 2H), 3.73-3.66 (m, 2H), 3.28-3.21 (m, 1H), 2.78-2.73 (m, 2H), 2.47-2.45 (m, 3H), 2.20-2.18 (m, 3H).

LC-MS (ESI): m/z = 439.2 [M+H]⁺.

### Example 6:

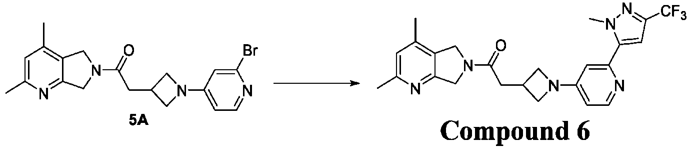

**5A** (150 mg, 0.37 mmol), (1-methyl-3-(trifluoromethyl)-1H-pyrazol-5-yl)boronic acid (115 mg, 0.56 mmol), Pd(dppf)Cl₂ (27 mg, 0.037 mmol) and potassium carbonate (153 mg, 1.11 mmol) were dissolved in mixed solvents of 1,4-dioxane (6 mL) and water (0.5 mL), and under nitrogen atmosphere, the mixture was reacted at 100°C overnight. After the reaction was completed, the reaction solution was cooled to room temperature, water (25 mL) was added to the reaction solution, and the resulting mixture was extracted with ethyl acetate (15 mL×3). The organic layers were combined, washed with saturated aqueous sodium chloride solution (30 mL), dried over anhydrous sodium sulphate, and filtered. The filtrate was concentrated and the residue was separated and purified by silica gel column chromatography (dichloromethane: methanol (v/v) = 25 : 1) to obtain **compound 6** (30 mg, 17%).

¹H NMR (400 MHz, CDCl₃) δ 8.30-8.27 (m, 1H), 6.92-6.90 (m, 1H), 6.71 (s, 1H), 6.46 (s, 1H), 6.28-6.25 (m, 1H), 4.79-4.73 (m, 4H), 4.29-4.24 (m, 2H), 4.19 (s, 3H), 3.79-3.72 (m, 2H), 3.38-3.27 (m, 1H), 2.85-2.80 (m, 2H), 2.54-2.52 (m, 3H), 2.28-2.26 (m, 3H).

LC-MS (ESI): m/z = 471.6 [M+H]⁺.

### Example 7:

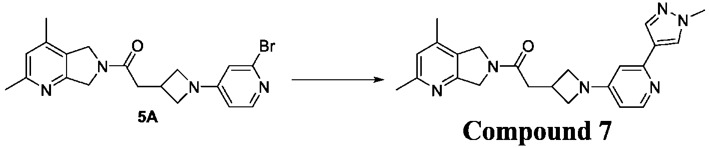

**5A** (150 mg, 0.37 mmol), (1-methyl-1H-pyrazol-4-yl)boronic acid pinacol (117 mg, 0.56 mmol), Pd(dppf)Cl₂ (27 mg, 0.037 mmol) and potassium carbonate (153 mg, 1.11 mmol) were dissolved in mixed solvents of 1,4-dioxane (6 mL) and water (0.5 mL), and under nitrogen atmosphere, the mixture was reacted at 100°C overnight. After the reaction was completed, the reaction solution was cooled to room temperature, water (25 mL) was added to the reaction solution, and the resulting mixture was extracted with ethyl acetate (15 mL×3). The organic layers were combined, washed with saturated aqueous sodium chloride solution (30 mL), dried over anhydrous sodium sulphate, and filtered. The filtrate was concentrated and the residue was separated and purified by silica gel column chromatography (dichloromethane : methanol (v/v) = 20 : 1) to obtain **compound 7** (60 mg, 40%).

¹H NMR (400 MHz, CDCl₃) δ 8.18-8.15 (m, 1H), 7.97-7.95 (m, 1H), 7.87 (s, 1H), 6.92-6.89 (m, 1H), 6.41 (s, 1H), 6.14-6.12 (m, 1H), 4.79-4.73 (m, 4H), 4.28-4.24 (m, 2H), 3.94 (s, 3H), 3.78-3.69 (m, 2H), 3.35-3.24 (m, 1H), 2.84-2.79 (m, 2H), 2.54-2.52 (m, 3H), 2.27-2.25 (m, 3H).

LC-MS (ESI): m/z = 403.3 [M+H]⁺.

### Example 8:

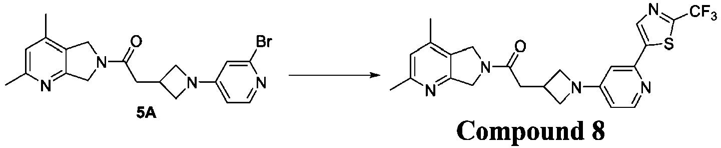

**5A** (150 mg, 0.37 mmol), (2-(trifluoromethyl)thiazol-5-yl)boronic acid pinacol (156 mg, 0.56 mmol), Pd(dppf)Cl₂ (27 mg, 0.037 mmol) and potassium carbonate (153 mg, 1.11 mmol) were dissolved in mixed solvents of 1,4-dioxane (6 mL) and water (0.5 mL), and under nitrogen atmosphere, the mixture was reacted at 100°C overnight. After the reaction was completed, the reaction solution was cooled to room temperature, water (25 mL) was added to the reaction solution, and the resulting mixture was extracted with ethyl acetate (15 mL×3). The organic layers were combined, washed with saturated aqueous sodium chloride solution (30 mL), dried over anhydrous sodium sulphate, and filtered. The filtrate was concentrated and the residue was separated and purified by silica gel column chromatography (dichloromethane : methanol (v/v) = 25 : 1) to obtain **compound 8** (22 mg, 12%).

¹H NMR (400 MHz, CDCl₃) δ 8.24 (s, 1H), 8.22-8.21 (m, 1H), 6.93-6.90 (m, 1H), 6.61 (d, J = 2.1 Hz, 1H), 6.26-6.23 (m, 1H), 4.79-4.73 (m, 4H), 4.33-4.28 (m, 2H), 3.82-3.75 (m, 2H), 3.37-3.29 (m, 1H), 2.85-2.80 (m, 2H), 2.54-2.52 (m, 3H), 2.28-2.26 (m, 3H).

LC-MS (ESI): m/z = 474.0 [M+H]⁺.

### Example 9:

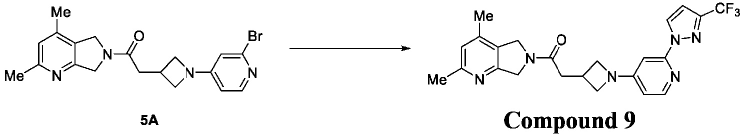

Compound **5A** (150 mg, 0.47 mmol) was dissolved in DMF (5 mL), and then 3-trifluoromethylpyrazole (0.096 g, 0.70 mmol), cuprous iodide (0.031 g, 0.094 mmol) and caesium carbonate (0.18 g, 0.94 mmol) were successively added. After the addition, the mixture was purged with nitrogen, warmed to 120°C and reacted for 6 hours. After the reaction was completed, the reaction solution was cooled to room temperature and added to tap water (20 mL). The mixture was extracted with ethyl acetate (10 mL×3), washed with saturated aqueous sodium chloride solution, dried, and concentrated to obtain a crude product. The crude product was purified using Biotage Isolera One (20 g silica gel column, eluents: 0-60% ethyl acetate/petroleum ether) to obtain compound **9** (28 mg, 13.1%).

LC-MS (ESI): m/z = 457.4 [M+H]⁺.

¹H NMR (400 MHz, CDCl₃) δ 8.58 (d, 1H), 8.03 (d, 1H), 6.93 (m, 2H), 6.66 (s, 1H), 6.62 (d, 1H), 4.79-4.74 (m, 4H), 4.33-4.29 (m, 2H), 3.82-3.77 (m, 2H), 3.35-3.27 (m, 1H), 2.82 (t, 2H), 2.54 (s, 3H),2.27 (d, 3H).

### Example 10:

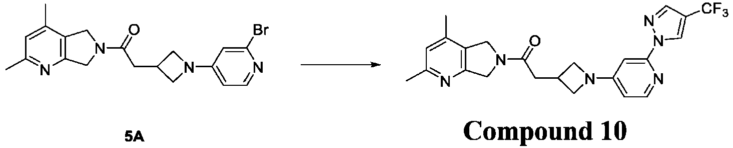

**Compound 5A** (150 mg, 0.37 mmol), 4-(trifluoromethyl)-1H-pyrazole (60 mg, 0.44 mmol), potassium carbonate (102 mg, 0.74 mmol), cuprous iodide (7 mg, 0.037 mmol) and L-proline (8 mg, 0.074 mmol) were dissolved in DMSO (15 mL), and under nitrogen atmosphere, the mixture was reacted at 100°C overnight. After the reaction was completed as monitored by TLC, the reaction was quenched with water (20 mL). The resulting mixture was extracted three times with EA (20 mL), and the organic phases were combined, washed once with saturated aqueous sodium chloride solution, dried over anhydrous sodium sulphate, and concentrated to obtain a crude product, which was separated and purified by silica gel column chromatography (dichloromethane : methanol (v/v) = 10 : 1), to obtain the target **compound 10** (8 mg, yield: 4.74%).

LC-MS (ESI): m/z = 457.5 [M+H]⁺.

¹H NMR (400 MHz, DMSO-d₆) δ 9.04 (s, 1H), 8.20 (s, 1H), 8.07-8.00 (m, 1H), 7.03-6.97 (m, 1H), 6.82 (s, 1H), 6.44-6.35 (m, 1H), 4.85-4.76 (m, 2H), 4.63-4.52 (m, 2H), 4.23-4.16 (m, 2H), 3.78-3.70 (m, 2H), 3.15 (s, 1H), 2.91-2.82 (m, 2H), 2.42 (s, 3H), 2.26-2.19 (m, 3H).

### Example 11:

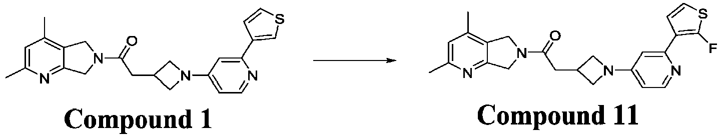

Compound **1** (0.5 g, 1.24 mmol) was dissolved in dry acetonitrile (20 mL), and then 1-chloromethyl-4-fluoro-1,4-diazoniabicyclo[2.2.2]octane bis(tetrafluoroborate) (1.32 g, 3.72 mmol) was added in one portion. After the addition, the mixture was warmed to 70°C and reacted overnight. After the reaction was completed, the reaction solution was concentrated to dryness, ethyl acetate (30 mL) and tap water (50 mL) were added, and the mixture was stirred for phase separation. The organic phase was washed with saturated aqueous sodium chloride solution, dried over anhydrous sodium sulphate, filtered and concentrated to obtain a crude product. The crude product was purified using Biotage Isolera One (20 g silica gel column, eluents: 0-70% ethyl acetate/petroleum ether) to obtain **compound 11** (55 mg, 10.5%).

LC-MS (ESI): m/z= 423.4 [M+H]⁺.

¹H NMR (400 MHz, CDCl₃) δ 8.07 (d, 1H), 7.98 (s, 1H), 7.76 (dd, 1H), 7.36 (t, 1H), 6.91 (d, 1H), 6.23 (dd, 1H), 4.79-4.73 (m, 4H), 4.43 (t, 2H), 3.92 (t, 2H), 3.30-3.27 (m, 1H), 2.85-2.81 (m, 2H), 2.52 (d, 3H), 2.27 (d, 3H).

### Example 12:

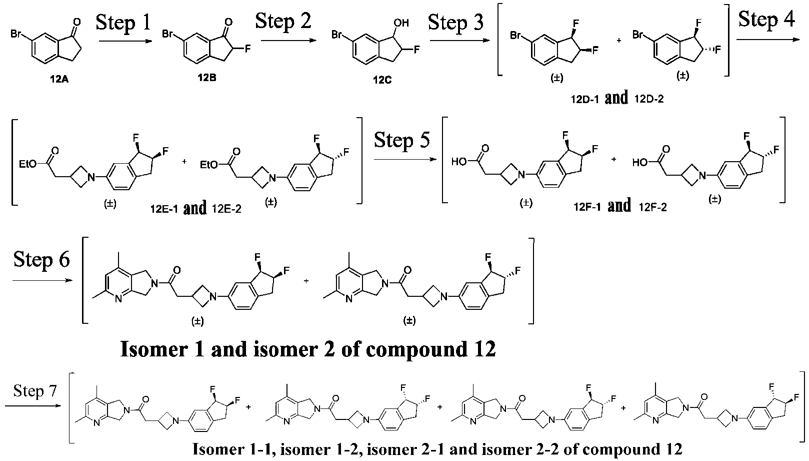

Step 1: **12A** (30 g, 142.14 mmol) and 1-chloromethyl-4-fluoro-1,4diazabicyclo[2.2.2]octane bis(tetrafluoroborate) (Selectfluor) (58.41 g,164.88 mmol) were added to methanol (300 mL), and the mixture was reacted at 60°C for 5 h. After the reaction was completed, the mixture was cooled to room temperature and filtered. The filter cake was washed with methanol, and the filtrate was concentrated under reduced pressure. The residue was separated and purified by silica gel column chromatography (EA/PE = 1/5) to obtain **12B** (11 g, 33.79%).

LC-MS (ESI): m/z = 229.1, 231.1[M+H]⁺.

Step 2: **12B** (6 g, 26.20 mmol) was dissolved in anhydrous ethanol (60 mL). At 0°C, sodium borohydride (1.19 g, 31.44 mmol) was added in portions, and the mixture was slowly warmed to room temperature and reacted for 2 h. The reaction solution was poured into water (300 mL). The mixture was extracted with ethyl acetate (50 mL×3). The combined organic phases were washed with saturated aqueous sodium chloride solution, dried over anhydrous sodium sulphate, and filtered. The filtrate was concentrated under reduced pressure to obtain **12C** (5.7 g, 94.16%).

LC-MS (ESI): m/z = 212.9, 214.9[M+H]⁺.

¹H NMR (400 MHz, CDCl₃) δ 7.45-7.38 (m, 2H), 7.35-7.33 (m, 1H), 5.37-5.20 (m, 1H), 5.09-5.03 (m, 1H), 3.27-3.02 (m, 2H), 2.26 (s, 1H).

Step 3: **12C** (5.5 g, 23.80 mmol) was dissolved in dichloromethane (70 mL), and at -20°C, diethylaminosulphur trifluoride (9.59 g,59.5 mmol) was added dropwise. After the dropwise addition was completed, the mixture was naturally warmed to room temperature and reacted for 3 h. After the reaction was completed, the reaction solution was poured into ice water, and extracted and subjected to phase separation. The organic phase was concentrated under reduced pressure and the residue was separated and purified by silica gel column chromatography (EA/PE = 0-8%), to obtain **12D**-1 (Rf = 0.8 (EA/PE = 1/10), 2.1 g) and **12D**-2 (Rf = 0.6 (EA/PE = 1/10), 2.0 g), respectively.

Compound **12D**-1: ¹H NMR (400 MHz, CDCl₃) δ 7.46-7.42 (m, 2H), 7.35-7.33 (m, 1H), 6.00-5.83 (m, 1H), 5.50-5.30 (m, 1H), 3.50-3.39 (m, 1H), 3.18-3.00 (m, 1H).

Compound **12D**-2: ¹H NMR (400 MHz, CDCl₃) δ 7.46-7.44 (m, 2H), 7.37-7.34 (m, 1H), 5.80-5.63 (m, 1H), 5.35-5.16 (m, 1H), 3.35-3.13 (m, 2H).

Step 4: **12D**-1 (0.9 g, 3.86 mmol), **1D** (1.49 g, 5.79 mmol), Ruphos-Pd-G3 (0.32g, 0.38 mmol) and caesium carbonate (5 g, 15.44 mmol) were successively added to dioxane (20 mL). The system was purged with N2 and reacted at 100°C for 16 h. After the reaction was completed, the mixture was cooled to room temperature and filtered through diatomaceous earth. The filtrate was concentrated under reduced pressure and the residue was separated and purified by silica gel column chromatography (EA/PE = 1/7) to obtain **12E**-1 (0.9 g, 78.95%).

LC-MS (ESI): m/z =296.2[M+H]⁺.

With reference to the above-mentioned operation, **12E**-2 (0.9 g, 78.9%) was obtained using **12D-**2 (0.9 g, 3.86 mmol) as a starting material.

LC-MS (ESI): m/z = 296.2[M+H]⁺.

Step 5: **12E**-1 (0.7 g, 2.37 mmol) and lithium hydroxide monohydrate (0.50 g, 11.92 mmol) were added to a mixed system of methanol (5 mL), tetrahydrofuran (5 mL) and water (5 mL), and the mixture was reacted at room temperature for 3 h. The reaction solution was directly concentrated under reduced pressure to dryness to obtain crude product 12F-1.

LC-MS (ESI): m/z = 268.2[M+H]⁺.

With reference to the above-mentioned operation, crude product **12F**-2 was obtained using **12E**-2 (0.7 g, 2.37 mmol) as a starting material.

LC-MS (ESI): m/z = 268.2[M+H]⁺.

Step 6: **Intermediate 1** (0.52 g, 2.37 mmol), 12F-1 (2.37 mmol), 2-(7-azobenzotriazole)-N,N,N',N'-tetramethyluronium hexafluorophosphate (1.17 g, 3.08 mmol), and N,N-diisopropylethylamine (1.53 g, 11.85 mmol) were successively added to N,N-dimethylformamide (15 mL), and the mixture was reacted at room temperature for 5 h. Water (60 ml) was added to the reaction system. The mixture was extracted with ethyl acetate (20 mL×3), and the combined organic phases were washed with saturated aqueous sodium chloride solution, dried over anhydrous sodium sulphate, and filtered. The filtrate was concentrated under reduced pressure and the residue was purified and separated using a C18 reverse-phase column (composition of mobile phases A and B: mobile phase A: acetonitrile; and mobile phase B: water (containing 0.1% aqueous ammonia), (A/B = 53%/47%)) to obtain **compound 12,** isomer 1 (800 mg, 84.9%).

With reference to the above-mentioned operation, **compound 12,** isomer 2 (800 mg. 84.9%) was obtained using **12F**-2 (2.37 mmol) as a starting material.

**Compound 12,** isomer 1: ¹H NMR (400 MHz, CDCl₃) δ 7.08 (d, 1H), 6.91-6.89 (m, 1H), 6.53 (s, 1H), 6.49-6.43 (m, 1H), 6.03-5.85 (m, 1H), 5.50-5.25 (m, 1H), 4.78-4.73 (m, 4H), 4.18-4.06 (m, 2H), 3.62-3.58 (m, 2H), 3.40-3.30 (m, 1H), 3.28-3.16 (m, 1H), 3.02-2.92 (m, 1H), 2.84-2.74 (m, 2H), 2.55-2.50 (m, 3H), 2.28-2.23 (m, 3H).

LC-MS (ESI): m/z = 398.3[M+H]⁺.

**compound 12,** isomer 2: ¹H NMR (400 MHz, CDCl₃) δ 7.08 (d, 1H), 6.91-6.89 (m, 1H), 6.53 (s, 1H), 6.50-6.46 (m, 1H), 5.78-5.61 (m, 1H), 5.32-5.11 (m, 1H), 4.80-4.70 (m, 4H), 4.16-4.10 (m, 2H), 3.62-3.56 (m, 2H), 3.28-3.06 (m, 3H), 2.83-2.77 (m,2H), 2.55-2.50 (m, 3H), 2.29-2.23 (m, 3H).

LC-MS (ESI): m/z = 398.3[M+H]⁺.

Step 7: **Compound 12,** isomer 1 (500 mg, 1.26 mmol) was subjected to chiral preparation to obtain **compound 12,** isomer 1-1 (205 mg, 41%) and **compound 12,** isomer 1-2 (189 mg, 38%).

Chiral HPLC analytical method: 1. instrument: SHIMADZU LC-30ADsf; 2. chromatographic column: Chiral Whelk column; 3. mobile phase system: A for CO₂; B for 0.05% DEA in ethanol; 4. gradient: B for 40% 5. flow rate: 3 mL/min. **Compound 12,** isomer 1-1 (retention time: 2.293 min) and **compound 12,** isomer 1-2 (retention time: 2.138 min).

Chiral preparative separation conditions: 1. instrument: Waters 150 Prep-SFC; 2. chromatographic column: Chiral AD column; 3. mobile phase: A for CO₂; B for 0.1% NH3•H2O in ethanol and acetonitrile; 4. gradient: B for 55%; 5. flow rate: 100 mL/min;

**compound 12,** isomer 2 (550 mg, 1.38 mmol) was subjected to chiral preparation to obtain **compound 12,** isomer 2-1 (238 mg, 43%) and **compound 12,** isomer 2-2 (227 mg, 41%).

Chiral HPLC analytical method: 1. instrument: SHIMADZU LC-30ADsf; 2. chromatographic column: Chiral AD column; 3. mobile phase system: A for CO₂; B for 0.05% DEA in methanol and acetonitrile; 4. gradient: B for 40% 5. flow rate: 3 mL/min. **Compound 12,** isomer 2-1 (retention time: 2.356 min) and **compound 12,** isomer 2-2 (retention time: 2.564 min).

Chiral preparative separation conditions: 1. instrument: Waters 150 Prep-SFC; 2. chromatographic column: Chiral AD column; 3. mobile phase: A for CO₂; B for 0.1% NH3•H2O in methanol and acetonitrile; 4. gradient: B for 60%; 5. flow rate: 100 mL/min;

**Compound 12,** isomer 1-1: ¹H NMR (400 MHz, DMSO-d6) δ 7.12 (d, 1H), 6.99 (s, 1H), 6.52-6.47 (m, 2H), 6.11-5.93 (m, 1H), 5.56-5.32 (m, 1H), 4.79 (d, 2H), 4.56 (d, 2H), 4.01-3.97 (m, 2H), 3.52-3.48 (m, 2H), 3.38-3.31 (m, 1H), 3.11-2.99 (m, 1H), 2.94-2.80 (m, 3H), 2.42 (s, 3H), 2.22 (d, 3H).

LC-MS (ESI): m/z = 398.3[M+H]⁺.

**Compound 12,** isomer 1-2: ¹H NMR (400 MHz, DMSO-d6) δ 7.12 (d, 1H), 6.99 (s, 1H), 6.52-6.47 (m, 2H), 6.11-5.93 (m, 1H), 5.54-5.35 (m, 1H), 4.79 (d, 2H), 4.56 (d, 2H), 4.01-3.97 (m, 2H), 3.52-3.48 (m, 2H), 3.39-3.31 (m, 1H), 3.10-2.98 (m, 1H), 2.95-2.80 (m, 3H), 2.42 (s, 3H), 2.22 (d, 3H).

LC-MS (ESI): m/z = 398.3[M+H]⁺.

**Compound 12,** isomer 2-1: ¹H NMR (400 MHz, DMSO-d6) δ 7.13 (d, 1H), 6.99 (s, 1H), 6.54 (s, 1H), 6.49-6.45 (m, 1H), 5.89-5.73 (m, 1H), 5.42-5.22 (m, 1H), 4.79 (d, 2H), 4.56 (d, 2H), 4.01-3.97 (m, 2H), 3.52-3.48 (m, 2H), 3.16-2.93 (m, 3H), 2.83-2.80 (m, 2H), 2.42 (s, 3H), 2.22 (d, 3H).

LC-MS (ESI): m/z = 398.3[M+H]⁺.

**Compound 12,** isomer 2-2: ¹H NMR (400 MHz, DMSO-d6) δ 7.13 (d, 1H), 6.99 (s, 1H), 6.54 (s, 1H), 6.49-6.45 (m, 1H), 5.89-5.72 (m, 1H), 5.42-5.22 (m, 1H), 4.79 (d, 2H), 4.56 (d, 2H), 4.01-3.97 (m, 2H), 3.52-3.48 (m, 2H), 3.15-2.95 (m, 3H), 2.83-2.79 (m, 2H), 2.42 (s, 3H), 2.22 (d, 3H).

LC-MS (ESI): m/z = 398.3[M+H]⁺.

### Example 13:

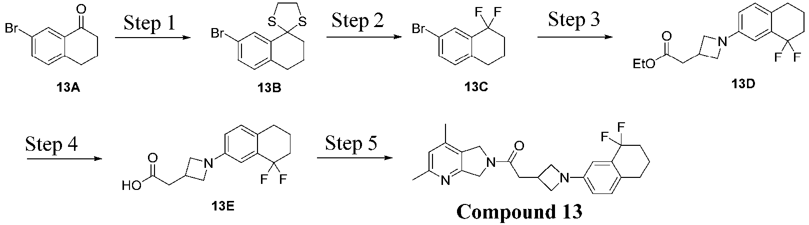

Step 1: At room temperature, compound **13A** (2.0 g, 8.89 mmol) was dissolved in dichloromethane (20 mL), the mixture was cooled to 0°C, and 1,2-ethanedithiol (1.67 g, 17.78 mmol) and boron trifluoride diethyl etherate (0.63 g, 4.44 mmol) were slowly added. The mixture was reacted at room temperature for 15 h. After the reaction was completed, the reaction mixture was adjusted to pH 7 with 1 N sodium hydroxide solution, and extracted with dichloromethane (20 mL×3). The combined organic phases were washed with saturated aqueous sodium chloride solution (20 mL), dried over anhydrous sodium sulphate, and filtered. The filtrate was concentrated and the residue was separated and purified by silica gel column chromatography (petroleum ether : ethyl acetate (v/v) = 2 : 1) to obtain compound **13B** (1.5 g, 56%).

¹H NMR (400 MHz, CDCl₃) δ 8.04 (d, 1H), 7.21 (dd, 1H), 6.85 (d, 1H), 3.62-3.55 (m, 2H), 3.48-3.41 (m, 2H), 2.75-2.71 (m, 2H), 2.37-2.33 (m, 2H), 2.01-1.96 (m, 2H).

Step 2: At room temperature, N-iodosuccinimide (2.24 g, 9.96 mmol) was dissolved in dichloromethane (15 mL), and the mixture was cooled to -70°C. Hydrofluoride-pyridine (1.97 g, 19.92 mmol) and a solution of compound **13B** (1.5 g, 4.98 mmol) in dichloromethane (15 mL) were slowly added. After the dropwise addition was completed, the reaction mixture was reacted at -70°C for 30 min. After the reaction was completed, the reaction mixture was adjusted to pH 7 with saturated aqueous sodium bicarbonate solution, and extracted with dichloromethane (10 mL×2). The combined organic phases were washed with saturated aqueous sodium chloride solution (20 mL), dried over anhydrous sodium sulphate, and filtered. The filtrate was concentrated and the residue was separated and purified by silica gel column chromatography (petroleum ether : ethyl acetate (v/v) = 9 : 1) to obtain compound **13C** (0.8 g, 65%).

LC-MS (ESI): m/z = 247.2 [M+H]⁺.

Step 3: At room temperature, compound **13C** (0.8 g, 3.24 mmol), **1D** (0.55 g, 3.89 mmol), Ruphos-Pd-G3 (0.27 g, 0.32 mmol) and caesium carbonate (3.16 g, 9.72 mmol) were successively dissolved in 1,4-dioxane (10 mL), and under nitrogen atmosphere, the mixture was warmed to 100°C and reacted for 16 h. After the reaction was completed, the reaction mixture was cooled to room temperature, and filtered through diatomaceous earth, and the filtrate was concentrated under reduced pressure. The residue was separated and purified by silica gel column chromatography (petroleum ether : ethyl acetate (v/v) = 7 : 1) to obtain compound **13D** (0.7 g, 70%).

LC-MS (ESI): m/z = 310.1 [M+H]⁺.

Step 4: At room temperature, compound **13D** (0.7 g, 2.26 mmol) and lithium hydroxide monohydrate (0.28 g, 6.78 mmol) were dissolved in a mixed system of methanol (3 mL), tetrahydrofuran (3 mL) and water (3 mL). The mixture was reacted at room temperature for 2 h. After the reaction was completed, the reaction solution was directly concentrated under reduced pressure to obtain crude compound **13E** (0.72 g), which was directly used in the next reaction without purification.

LC-MS (ESI): m/z = 282.2 [M+H]⁺.

Step 5: At room temperature, compound **13E** (0.3 g, 1.07 mmol), **intermediate 1** (0.23 g, 1.07 mmol), and 2-(7-azobenzotriazole)-N,N,N',N'-tetramethyluronium hexafluorophosphate (0.53 g, 1.39 mmol) were successively dissolved in N,N-dimethylformamide (10 mL), and then N,N-diisopropylethylamine (0.41 g, 3.21 mmol) was added. The mixture was reacted at room temperature for 5 h. After the reaction was completed, water (20 mL) was added to the reaction system. The reaction mixture was extracted with ethyl acetate (20 mL×2). The combined organic phases were washed with saturated aqueous sodium chloride solution (20 mL), dried over anhydrous sodium sulphate, and filtered. The filtrate was concentrated under reduced pressure and the residue was purified and separated using a C18 reverse-phase column (composition of mobile phases A and B: mobile phase A: acetonitrile; and mobile phase B: water (containing 0.1% aqueous ammonia), (A/B = 60%/40%)) to obtain **compound 13** (0.2 g. 46%).

¹H NMR (400 MHz, CDCl₃) δ 6.99 (d, 1H), 6.91-6.87 (m, 1H), 6.72 (d, 1H), 6.48 (dd, 1H), 4.79-4.75 (m, 2H), 4.74-4.71 (m, 2H), 4.13 (t, 2H), 3.62-3.57 (m, 2H), 3.27-3.16 (m, 1H), 2.83-2.75 (m, 2H), 2.74-2.69 (m, 2H), 2.54-2.50 (m, 3H), 2.27-2.25 (m, 3H), 2.24-2.16 (m, 2H), 1.98-1.91 (m, 2H).

LC-MS (ESI): m/z = 412.2 [M+H]⁺.

### Example 14:

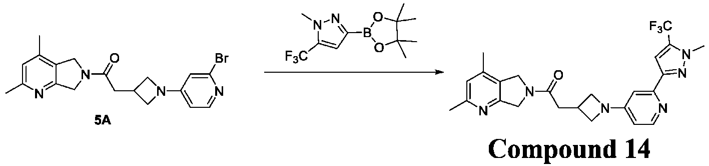

**5A** (80 mg, 0.2 mmol), 1-methyl-3-(4,4,5,5-tetramethyl-1,3,2-dioxaborolan-2-yl)-5-(trifluoromethyl)-1H-pyrazole (72 mg, 0.26 mmol), Pd(dppf)Cl₂ (16 mg, 0.02 mmol) and potassium carbonate (83 mg, 0.60 mmol) were successively added to mixed solvents of 1,4-dioxane (5 mL) and water (1 mL), and under nitrogen atmosphere, the mixture was heated to 90°C and reacted for 12 h. After the reaction was completed, the reaction mixture was cooled to room temperature and filtered, and the filtrate was concentrated. The residue was separated by silica gel column chromatography (dichloromethane : methanol = 10 : 1) to obtain **compound 14** (35 mg, 37%).

LC-MS (ESI): m/z = 471.5 [M+H]⁺.

¹H NMR (400 MHz, DMSO-d6) δ 8.14 (d, 1H), 7.26 (s, 1H), 7.00 (s, 1H), 6.87 (d, 1H), 6.33-6.35 (m, 1H), 4.80 (d, 2H), 4.57(d, 2H), 4.15 (t, 2H), 4.02 (s, 3H), 3.66-3.70 (m, 2H), 3.10-3.15 (m, 1H), 2.85-2.86 (m, 2H), 2.42 (s, 3H), 2.23 (d, 3H).

### Example 15:

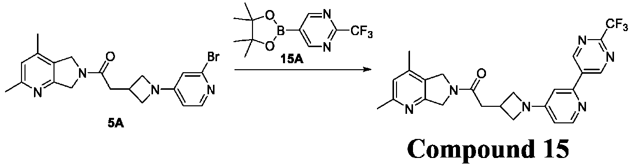

Compound **5A** (100 mg, 0.25 mmol) was dissolved in 1,4-dioxane (8 mL), and **15A** (88 mg, 0.32 mmol), potassium carbonate (86 mg, 0.62 mmol) and water (2 mL) were successively added. Under nitrogen atmosphere, 1,1-bis(diphenylphosphino)ferrocene palladium dichloride (27 mg, 0.04 mmol) was added and then the mixture was heated to 85°C and stirred for 16 h. After the reaction was completed, the reaction mixture was naturally warmed to room temperature, diluted with water (30 mL), and extracted with ethyl acetate (50 mL×3). The combined organic phases were dried over anhydrous sodium sulphate, filtered, and concentrated to obtain a crude product. The crude product was purified by column chromatography (dichloromethane : methanol = 95 : 5) to obtain the target **compound 15** (45 mg, 38.55%).

¹H NMR (400 MHz, CDCl₃) δ 9.39 (s, 2H), 8.36 - 8.34 (m, 1H), 6.92 - 6.91 (m, 1H), 6.66 (s, 1H), 6.64 - 6.32 (m, 1H), 4.79 - 4.74 (m, 4H), 4.33 - 4.29 (m, 2H), 3.83 - 3.78 (m, 2H), 3.36 - 3.32 (m, 1H), 2.86 - 2.82 (m, 2H), 2.54 (s, 3H), 2.74 (s, 3H).

LC-MS (ESI): m/z=469.1 [M+H]⁺.

### Example 16:

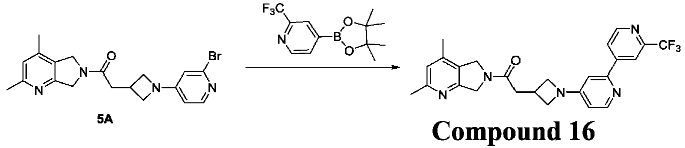

**5A** (80 mg, 0.2 mmol), 4-(4,4,5,5-tetramethyl-1,3,2-dioxaborolan-2-yl)-2-(trifluoromethyl)pyridine (71 mg, 0.26 mmol), Pd(dppf)₂Cl₂ (16 mg, 0.02 mmol) and potassium carbonate (83 mg, 0.60 mmol) were successively added to mixed solvents of 1,4-dioxane (5 mL) and water (1 mL), and under nitrogen atmosphere, the mixture was heated to 90°C and reacted for 12 h. After the reaction was completed, the reaction mixture was cooled to room temperature, filtered, and concentrated. The residue was separated and purified by column chromatography (dichloromethane : methanol = 10 : 1) to obtain **compound 16** (40 mg, 43%).

LC-MS (ESI): m/z = 468.5 [M+H]⁺.

¹H NMR (400 MHz, DMSO-d6) δ 8.84 (d, 1H), 8.50 (s, 1H), 8.37 (d, 1H), 8.28 (d, 1H), 7.20 (d, 1H), 7.00 (s, 1H), 6.44-6.46 (m, 1H), 4.80 (d, 2H), 4.58(d, 2H), 4.21 (t, 2H), 3.74 (t, 2H), 3.10-3.15 (m, 1H), 2.86-2.88 (m, 2H), 2.42 (s, 3H), 2.23 (d, 3H).

### Example 17:

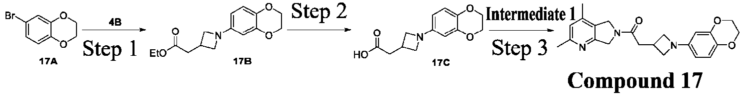

Step 1: At room temperature, compound **17A** (0.5 g, 2.33 mmol), **1D** (0.40 g, 2.79 mmol), Ruphos-Pd-G3 (0.19 g, 0.23 mmol) and caesium carbonate (2.27 g, 6.99 mmol) were successively dissolved in 1,4-dioxane (10 mL), and under nitrogen atmosphere, the mixture was warmed to 100°C and reacted for 16 h. After the reaction was completed, the reaction solution was cooled to room temperature, and filtered through diatomaceous earth. The filtrate was concentrated under reduced pressure. The resulting residue was separated and purified by silica gel column chromatography (petroleum ether : ethyl acetate (v/v) = 3 : 1) to obtain compound **17B** (0.3 g, 46%).

LC-MS (ESI): m/z = 278.2 [M+H]⁺.

Step 2: At room temperature, compound **17B** (0.3 g, 1.08 mmol) and lithium hydroxide monohydrate (0.14 g, 3.24 mmol) were dissolved in mixed solvents of methanol (2 mL), tetrahydrofuran (2 mL) and water (2 mL). The mixture was reacted at room temperature for 2 h. After the reaction was completed, the reaction solution was directly concentrated under reduced pressure to obtain crude compound **17C** (0.35 g), which was directly used in the next reaction without purification.

LC-MS (ESI): m/z = 250.1 [M+H]⁺.

Step 3: At room temperature, compound **17C** (0.27 g, 1.08 mmol), **intermediate 1** (0.24 g, 1.08 mmol), and 2-(7-azobenzotriazole)-N,N,N',N'-tetramethyluronium hexafluorophosphate (0.53 g, 1.40 mmol) were successively dissolved in DMF (10 mL), and then N,N-diisopropylethylamine (0.41 g, 3.24 mmol) was added. The mixture was reacted at room temperature for 5 h. After the reaction was completed, water (20 mL) was added to the reaction system. The reaction mixture was extracted with ethyl acetate (20×2 mL). The combined organic phases were washed with saturated aqueous sodium chloride solution (20 mL), dried over anhydrous sodium sulphate, and filtered. The filtrate was concentrated under reduced pressure and the residue was purified and separated using a C18 reverse-phase column (composition of mobile phases A and B: mobile phase A: acetonitrile; and mobile phase B: water (containing 0.1% aqueous ammonia), (A/B = 65%/35%)) to obtain **compound 17** (0.15 g. 36%).

LC-MS (ESI): m/z = 380.1 [M+H]⁺.

¹H NMR (400 MHz, CDCl₃) δ 6.92-6.87 (m, 1H), 6.76-6.70 (m, 1H), 6.03-5.96 (m, 2H), 4.79-4.70 (m, 4H), 4.25-4.20 (m, 2H), 4.19-4.16 (m, 2H), 4.04 (t, 2H), 3.55- 3.49 (m, 2H), 3.23-3.11 (m, 1H), 2.83-2.75 (m, 2H), 2.54-2.50 (m, 3H), 2.27-2.23 (m, 3H).

### Example 18:

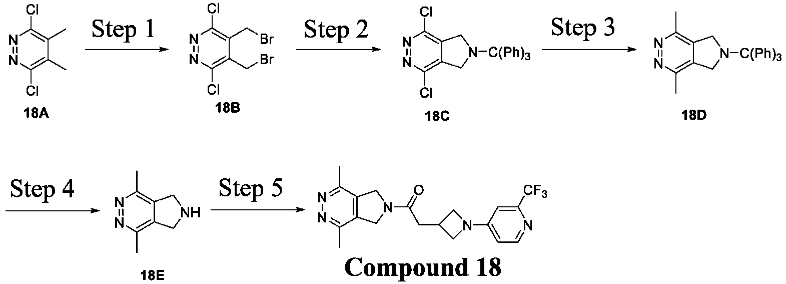

Step 1: Compound **18A** (10.0 g, 56.47 mmol) was dissolved in CCl₄ (100 mL), NBS (30 g, 169.41 mmol) and AIBN (0.46 g, 2.82 mmol) were added, and the mixture was reacted under reflux for 16 hours. After the reaction was completed as monitored by TLC, the reaction product was cooled to room temperature, and concentrated under reduced pressure. The residue was separated and purified by silica gel column chromatography (PE : EA = 10 : 1 to PE : EA = 3 : 1) to obtain compound **18B** (8.5 g, yield: 44.96%).

LC-MS(ESI):m/z = 336.8[M+H]⁺.

Step 2: Triphenylmethanamine (6.58 g, 25.39 mmol) was dissolved in DMF (125 mL), DIPEA (13.6 mL, 76.17 mmol) was added, and then the mixture was warmed to 60°C. A solution of compound **18B** (8.5 g, 25.39 mmol) in DMF (125 mL) was added dropwise. After the dropwise addition was completed, the mixture was reacted at 60°C for 2 hours. After the reaction was completed as monitored by TLC, the reaction product was cooled to room temperature, diluted with water (500 mL), and then extracted with ethyl acetate (300 mL×2). The combined organic phases were dried over anhydrous sodium sulphate, filtered, and concentrated under reduced pressure. The resulting residue was separated and purified by silica gel column chromatography (PE : EA = 50 : 1) to afford compound **18C** (1.0 g, yield: 9.11%).

¹H NMR (400 MHz, CDCl₃) δ 7.55-7.53 (m, 6H), 7.32-7.28 (m, 6H), 7.20-7.17 (m, 3H), 4.28-4.19 (m, 4H).

Step 3: Compound **18C** (0.13 g, 0.30 mmol), trimethylboroxine (113 mg, 0.9 mmol), potassium phosphate (192 mg, 0.9 mmol), Pd₂(dba)₃ (30 mg, 0.03 mmol) and X-Phos (57 mg, 0.12 mmol) were successively dissolved in 1.4-dioxane (10 mL), and under nitrogen atmosphere, the mixture was warmed to 100°C and reacted for 16 hours. After the reaction was completed as monitored by TLC, the reaction product was cooled to room temperature, diluted with water (60 mL), and then extracted with ethyl acetate (50 mL×2). The combined organic phases were dried over anhydrous sodium sulphate and filtered. The filtrate was concentrated under reduced pressure to obtain compound **18D** (300 mg, crude), which was directly used in the next step.

LC-MS(ESI):m/z = 392.2[M+H]⁺.

Step 4: **18D** (300 mg) was dissolved in DCM (10 mL), trifluoroacetic acid (2.5 mL) was added, and then the mixture was reacted at room temperature for 3 hours. After the reaction was completed as monitored by TLC, the reaction product was concentrated under reduced pressure to obtain compound **18E** (190 mg, crude), which was directly used in the next step.

LC-MS(ESI):m/z = 150.2[M+H]⁺.

Step 5: Compound **18E** (190 mg, 0.76 mmol), **4D** (99 mg, 0.38 mmol) and N-methylimidazole (1 mL) were successively dissolved in DMF (10 mL), then chloro-N,N,N',N'-tetramethylformamidinium hexafluorophosphate (210 mg, 0.76 mmol) was added, and the mixture was reacted at room temperature for 2 hours. After the reaction was completed as monitored by TLC, the reaction product was concentrated under reduced pressure. The residue was separated and purified by column chromatography (DCM : MeOH = 20 : 1) to obtain **compound 18** (41 mg, yield: 13.78%).

¹H NMR (400 MHz, CDCl₃) δ 8.21 (d, 1H), 6.52-6.50 (m, 1H), 6.27-6.25 (m, 1H), 4.73 (s, 4H), 4.23-4.19 (m, 2H), 3.68-3.65 (m, 2H), 3.29-3.22 (m, 1H), 2.77 (d, 2H), 2.59 (s, 3H) , 2.58 (s, 3H).

LC-MS(ESI):m/z = 392.2[M+H]⁺.

### Example 19:

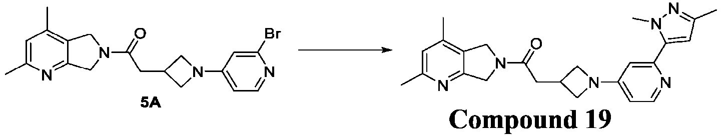

**5A** (150 mg, 0.37 mmol), (1,3-dimethyl-1H-pyrazol-5-yl)boronic acid pinacol (108 mg, 0.49 mmol), Pd(dppf)Cl₂ (27 mg, 0.037 mmol) and potassium carbonate (153 mg, 1.11 mmol) were dissolved in mixed solvents of 1,4-dioxane (6 mL) and water (0.5 mL), and under nitrogen atmosphere, the mixture was reacted at 100°C overnight. After the reaction was completed, the reaction solution was cooled to room temperature, water (25 mL) was added to the reaction solution, and the resulting mixture was extracted with ethyl acetate (15 mL×3). The organic layers were combined, washed with saturated aqueous sodium chloride solution (30 mL), dried over anhydrous sodium sulphate, and filtered. The filtrate was concentrated and the residue was separated and purified by silica gel column chromatography (dichloromethane : methanol (v/v) = 25 : 1) to obtain **compound 19** (70 mg, 45%).

¹H NMR (400 MHz, CDCl₃) δ 8.27-8.25 (m, 1H), 6.92-6.90 (m, 1H), 6.45-6.44 (m, 1H), 6.26 (s, 1H), 6.25-6.21 (m, 1H), 4.79-4.72 (m, 4H), 4.28-4.22 (m, 2H), 4.07 (s, 3H), 3.75-3.69 (m, 2H), 3.35-3.24 (m, 1H), 2.84-2.79 (t, J = 7.9 Hz, 2H), 2.54-2.52 (m, 3H), 2.30-2.25 (m, 6H).

LC-MS (ESI): m/z = 417.5 [M+H]⁺.

### Example 20:

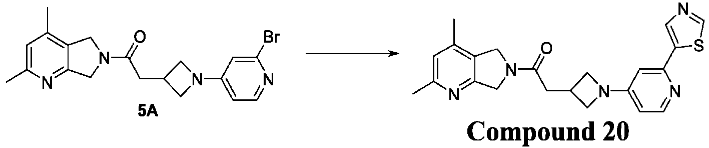

**5A** (150 mg, 0.37 mmol), (thiazol-5-yl)boronic acid pinacol (103 mg, 0.49 mmol), Pd(dppf)Cl₂ (27 mg, 0.037 mmol) and potassium carbonate (153 mg, 1.11 mmol) were dissolved in mixed solvents of 1,4-dioxane (6 mL) and water (0.5 mL), and under nitrogen atmosphere, the mixture was reacted at 100°C overnight. After the reaction was completed, the reaction solution was cooled to room temperature, water (25 mL) was added to the reaction solution, and the resulting mixture was extracted with ethyl acetate (15 mL×3). The organic layers were combined, washed with saturated aqueous sodium chloride solution (30 mL), dried over anhydrous sodium sulphate, and filtered. The filtrate was concentrated and the residue was separated and purified by silica gel column chromatography (dichloromethane : methanol (v/v) = 25 : 1) to obtain **compound 20** (64 mg, 42%).

¹H NMR (400 MHz, CDCl₃) δ 8.72 (s, 1H), 8.21 (s, 1H), 8.14-8.12 (m, 1H), 6.85-6.82 (m, 1H), 6.54 (s, 1H), 6.14-6.12 (m, 1H), 4.72-4.66 (m, 4H), 4.25-4.19 (m, 2H), 3.73-3.65 (m, 2H), 3.28-3.21 (m, 1H), 2.78-2.73 (m, 2H), 2.47-2.45 (m, 3H), 2.20-2.18 (m, 3H).

LC-MS (ESI): m/z = 406.4 [M+H]⁺.

### Example 21:

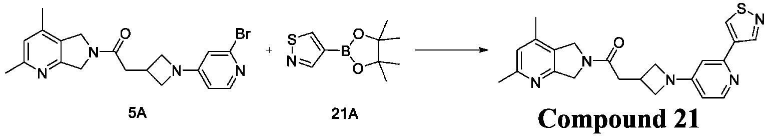

Compound **5A** (0.1 g, 0.20 mmol), compound **21A** (58 mg, 0.28 mmol), Pd(dppf)Cl₂ (37 mg, 0.050 mmol), and potassium carbonate (0.1 g, 0.76 mmol) were successively dissolved in dioxane (10 mL) and water (10 mL), and under nitrogen atmosphere, the mixture was warmed to 100°C, stirred and reacted for 18 hours. After the reaction was completed, the reaction solution was cooled to room temperature, and filtered through diatomaceous earth. Water was added to the filtrate, and the resulting mixture was extracted with ethyl acetate three times. The combined organic phases were washed with saturated aqueous sodium chloride solution, dried over anhydrous sodium sulphate and filtered. The filtrate was concentrated under reduced pressure and the resulting crude product was purified using Biotage Isolera One (12 g silica gel column, eluents: 0-6% MeOH/DCM) to obtain **compound 21** (20 mg, yield: 19.73%).

LC-MS (ESI): m/z = 406.1 [M+H]⁺.

¹H NMR (400 MHz, DMSO-*d*₆) δ 9.65 (s, 1H), 9.19 (s, 1H), 8.17-8.16 (d, 1H), 7.03 (s, 1H), 7.01-7.00 (d, 1H), 6.49-6.48 (d, 1H), 4.83-4.78 (d, 2H), 4.60-4.55 (d, 2H), 4.34-4.29 (t, 2H), 3.86 (s, 2H), 3.21-3.15 (m, 1H), 2.90-2.88 (d, 2H), 2.42 (s, 3H), 2.24-2.23 (d, 3H).

### Example 22:

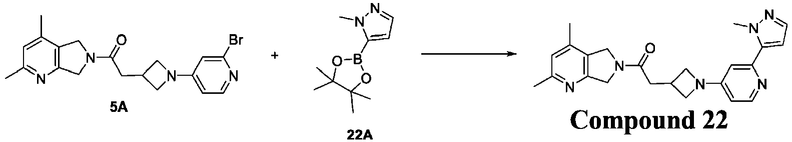

Compound **5A** (0.08 g, 0.20 mmol), compound **22A** (0.054 g, 0.26 mmol), Pd(dppf)Cl₂ (0.029 g, 0.040 mmol), and potassium carbonate (0.041 g, 0.30 mmol) were successively dissolved in dioxane (10 mL) and water (10 mL), and under nitrogen atmosphere, the mixture was warmed to 100°C, stirred and reacted for 18 hours. After the reaction was completed, the reaction solution was cooled to room temperature, and filtered through diatomaceous earth. Water was added to the filtrate, and the mixture was extracted with ethyl acetate three times. The combined organic phases were washed with saturated aqueous sodium chloride solution, dried over anhydrous sodium sulphate and filtered. The filtrate was concentrated under reduced pressure and the resulting crude product was purified using Biotage Isolera One (12 g silica gel column, eluents: 0-6% MeOH/DCM) to obtain **compound 22** (35 mg, yield: 43.48%).

¹H NMR (400 MHz, CDCl₃): δ 8.29 - 8.27 (m, 1H), 7.47 (s, 1H), 6.91 - 6.90 (m, 1H), 6.49-6.47 (m, 2H), 6.25-6.23 (m, 1H), 4.78-4.73 (m, 4H), 4.29-4.24 (m, 2H), 4.15 (s, 3H), 3.77-3.72 (m, 2H), 3.34-3.27 (m, 1H), 2.84-2.80 (m, 2H), 2.53-2.52 (m, 3H), 2.27-2.26 (m, 3H).

LC-MS (ESI): m/z= 403.5 [M+H]⁺.

### Example 23:

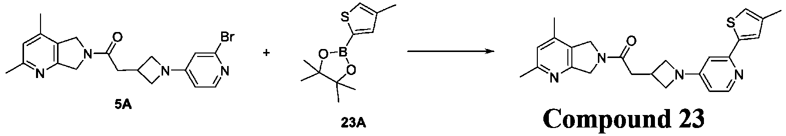

Compound **5A** (0.08 g, 0.20 mmol), compound **23A** (0.067 g, 0.30 mmol), Pd(dppf)Cl₂ (0.029 g, 0.040 mmol), and potassium carbonate (0.041 g, 0.30 mmol) were successively dissolved in dioxane (10 mL) and water (10 mL), and under nitrogen atmosphere, the mixture was warmed to 100°C, stirred and reacted for 18 hours. After the reaction was completed, the reaction solution was cooled to room temperature, and filtered through diatomaceous earth. Water was added to the filtrate, and the resulting mixture was extracted with ethyl acetate three times. The combined organic phases were washed with saturated aqueous sodium chloride solution, dried over anhydrous sodium sulphate and filtered. The filtrate was concentrated under reduced pressure and the resulting crude product was purified using Biotage Isolera One (12 g silica gel column, eluents: 0-6% MeOH/DCM) to obtain **compound 23** (40 mg, yield: 47.78%).

LC-MS (ESI): m/z= 419.1 [M+H]⁺.

¹H NMR (400 MHz, CDCl₃): δ 8.20-8.18 (m, 1H), 7.25 (s, 1H), 6.93-6.90 (m, 2H), 6.55 (s, 1H), 6.17-6.15 (m, 1H), 4.79-4.73 (m, 4H), 4.27 (s, 2H), 3.75 (s, 2H), 3.31-3.30 (m, 1H), 2.84-2.80 (m, 2H), 2.53-2.52 (m, 3H), 2.29-2.26 (m, 6H).

### Example 24:

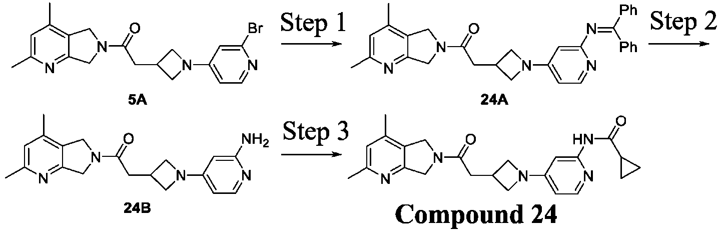

Step 1: Compound **5A** (1.2 g, 3 mmol) was dissolved in dioxane (100 mL), and benzophenone imine (0.65 g, 3.6 mmol), Pd₂(dba)₃ (0.27 g, 0.3 mmol), 4,5-bisdiphenylphosphino-9,9-dimethylxanthene (0.35 g, 0.6 mmol), and caesium carbonate (1.95 g, 6 mmol) were successively added. The resulting mixture was purged with nitrogen three times, then warmed to 80°C, stirred and reacted for 10 hours. After the reaction was completed, the mixture was cooled to room temperature, and concentrated under reduced pressure. The residue was separated by silica gel column chromatography (dichloromethane : methanol (v/v) = 5 : 1) to obtain compound **24A** (0.95 g, yield: 63%).

LC-MS (ESI): m/z = 502.3 [M+H]⁺.

Step 2: Compound **24A** (0.95 g, 1.89 mmol) was dissolved in methanol (30 mL) and tetrahydrofuran (30 mL), 1 M dilute hydrochloric acid (30 mL) was added, and the resulting mixture was reacted at room temperature for 3 hours. After the reaction was completed, the reaction solution was concentrated under reduced pressure to remove the solvent. The remaining aqueous phase was adjusted to pH = 9 with saturated sodium bicarbonate, extracted with dichloromethane (80 mL × 2), dried over anhydrous sodium sulphate, and concentrated under reduced pressure. The residue was separated by silica gel column chromatography (dichloromethane : methanol (v/v) = 3 : 1 + 1% aqueous ammonia) to obtain compound **24B** (0.5 g, yield: 80%).

LC-MS (ESI): m/z = 338.4 [M+H]⁺.

Step 3: Compound **24B** (78 mg, 0.23 mmol) was dissolved in dichloromethane (30 mL), and triethylamine (116 mg, 1.15 mmol) was then added. In an ice bath, cyclopropanecarbonyl chloride (72 mg, 0.69 mmol) was slowly added dropwise, and the mixture was reacted at room temperature for 3 hours. After the reaction was completed, the organic phase was washed with saturated aqueous sodium chloride solution (20 mL × 2), and concentrated under reduced pressure. The residue was dissolved in tetrahydrofuran (30 mL), 10% NaOH (10 mL) was added, and the mixture was stirred at room temperature for 6 hours and subjected to layer separation. The remaining aqueous phase was extracted with dichloromethane (80 mL × 2), and the organic phases were combined, dried over anhydrous sodium sulphate and concentrated under reduced pressure. The residue was separated by silica gel column chromatography (dichloromethane : methanol (v/v) = 5 : 1) to obtain compound **24** (40 mg, yield: 43%).

LC-MS (ESI): m/z = 406.2 [M+H]⁺.

¹H NMR (400 MHz, CDCl₃): δ 8.48 (s, 1H), 7.88-7.82 (m, 1H), 7.21 (s, 1H), 6.92-6.87 (m, 1H), 6.04-5.98 (m, 1H), 4.79-4.74 (m, 2H), 4.73-4.69 (m, 2H), 4.27-4.18 (m, 2H), 3.74-3.65 (m, 2H), 3.32-3.19 (m, 1H), 2.81-2.73 (m, 2H), 2.55-2.49 (m, 3H), 2.26 (s, 3H), 1.59-1.50 (m, 1H), 1.10-1.03 (m, 2H), 0.89-0.82 (m, 2H).

### Example 25:

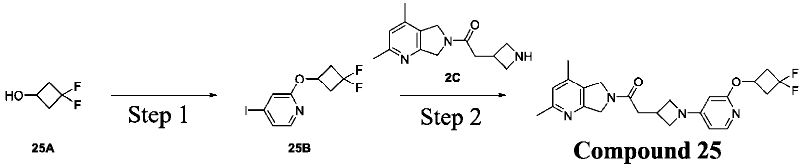

Step 1: At room temperature, compound **25A** (1.0 g, 9.26 mmol) was dissolved in tetrahydrofuran (20 mL), the system was cooled to 0°C, and sodium hydride (0.56 g, 13.89 mmol) was slowly added. At this temperature, the mixture was stirred for 0.5 hours, and then 2-fluoro-4-iodopyridine (2.06 g, 9.26 mmol) was added to the system. Then the mixture was warmed to room temperature, stirred and reacted for 3 hours. Water was added and the resulting mixture was extracted with ethyl acetate three times. The organic phases were combined, washed with saturated aqueous sodium chloride solution, dried over anhydrous sodium sulphate, and concentrated under reduced pressure to obtain a crude product. The crude product was purified using Biotage Isolera One (80 g silica gel column, eluents: 0-15% EA/PE) to obtain compound **25B** (1.0 g, yield: 34.84%).

LC-MS (ESI): m/z= 311.1 [M+H]⁺.

Step 2: In a single-neck flask, compound **2C** (0.20 g, 0.81 mmol), compound **25B** (0.10 g, 0.32 mmol), Ruphos-Pd-G3 (0.026 g, 0.032 mmol), caesium carbonate (0.32 g, 0.96 mmol), and toluene (10 mL) were successively added, and the mixture was purged with nitrogen three times, then warmed to 100°C, stirred and reacted for 18 hours. The mixture was cooled to room temperature, and filtered through diatomaceous earth. The filtrate was poured into water and the resulting mixture was extracted with ethyl acetate three times. The organic phases were combined, washed with saturated aqueous sodium chloride solution, dried over anhydrous sodium sulphate, and concentrated under reduced pressure to obtain a crude product. The crude product was purified using Biotage Isolera One (12 g silica gel column, eluents: 0-6% MeOH/DCM) to obtain compound **25** (15 mg, yield: 10.95%)

LC-MS (ESI): m/z= 429.2 [M+H]⁺.

1H NMR (400 MHz, CDCl₃): δ 7.81-7.80 (m, 1H), 6.91-6.90 (m, 1H), 6.21-6.20 (m, 1H), 6.01-5.99 (m, 1H), 5.63-5.62 (m, 1H), 4.78-4.72 (m, 4H), 4.21-4.17 (m, 2H), 3.26-3.24 (m, 2H), 3.70-3.56 (m, 2H), 3.13-3.03 (m, 2H), 2.81-2.64 (m, 4H), 2.53-2.52 (m, 3H), 2.27-2.26 (m, 3H).

### Example 26:

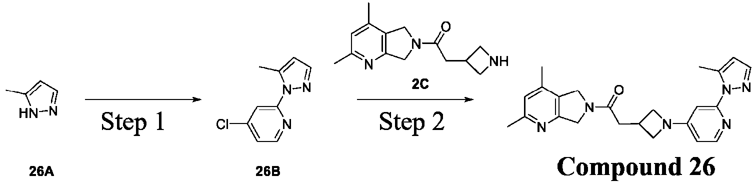

Step 1: In a single-neck flask, compound **26A** (0.24 g, 3.0 mmol), 2-fluoro-4-chloropyridine (0.40 g, 3.0 mmol), potassium carbonate (0.49 g, 3.52 mmol), and dimethyl sulphoxide (10 mL) were successively added, and the mixture was purged with nitrogen three times, then warmed to 45°C, stirred and reacted for 18 hours. The reaction mixture was cooled to room temperature and filtered through diatomaceous earth. The filtrate was poured into water and the resulting mixture was extracted with ethyl acetate three times. The organic phases were combined, washed with saturated aqueous sodium chloride solution, dried over anhydrous sodium sulphate, and concentrated under reduced pressure to obtain a crude product. The crude product was purified using Biotage Isolera One (12 g silica gel column, eluents: 0-15% EA/PE) to obtain compound **26B** (0.3 g, yield: 51.64%).

LC-MS (ESI): m/z= 194.1 [M+H]⁺.

Step 2: In a single-neck flask, compound **2C** (0.11 g, 0.45 mmol), compound **26B** (0.1 g, 0.52 mmol), caesium fluoride (0.1 g, 0.62 mmol), DIPEA (0.081 g, 0.62 mmol), and N,N-dimethylformamide (10 mL) were successively added, and the mixture was purged with nitrogen three times, and then warmed to 100°C, stirred and reacted for 18 hours. The mixture was cooled to room temperature, and filtered through diatomaceous earth. The filtrate was poured into water and the resulting mixture was extracted with ethyl acetate three times. The organic phases were combined, washed with saturated aqueous sodium chloride solution, dried over anhydrous sodium sulphate, and concentrated under reduced pressure to obtain a crude product. The crude product was purified using Biotage Isolera One (12 g silica gel column, eluents: 0-6% MeOH/DCM) to obtain compound **26** (30 mg, yield: 14.33%).

LC-MS (ESI): m/z= 403.3 [M+H]⁺.

¹H NMR (400 MHz, CDCl₃): δ 8.43 (s, 1H), 8.01-7.99 (m, 1H), 6.91-6.90 (m, 1H), 6.86-6.85 (m, 1H), 6.21-6.20 (m, 1H), 6.14-6.12 (m, 1H), 4.78-4.73 (m, 4H), 4.30-4.28 (m, 2H), 3.78-3.76 (m, 2H), 3.31-3.27 (m, 1H), 2.82-2.78 (m, 2H), 2.53-2.52 (m, 3H), 2.36 (s, 3H), 2.27-2.26 (m, 3H).

### Example 27:

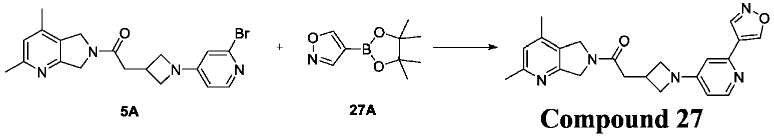

In a single-neck flask, compound **5A** (80 mg, 0.20 mmol), compound 27A (43 mg, 0.22 mmol), Pd(dppf)Cl₂ (30 mg, 0.040 mmol), potassium carbonate (83 mg, 0.6 mmol), dioxane (10 mL), and water (1 mL) were successively added, and the mixture was purged with nitrogen three times, then warmed to 100°C, stirred and reacted for 18 hours. Upon complete depletion of starting materials as monitored by TLC, the reaction was stopped. The reaction mixture was cooled to room temperature and then filtered through diatomaceous earth. The filtrate was poured into water and the resulting mixture was extracted with ethyl acetate three times. The organic phases were combined, washed with saturated aqueous sodium chloride solution, dried over anhydrous sodium sulphate, and concentrated under reduced pressure to obtain a crude product. The crude product was purified using Biotage Isolera One (12 g silica gel column, eluents: 0-6% MeOH/DCM) to obtain compound 27 (15 mg, yield: 19.26%).

LC-MS (ESI): m/z= 390.3 [M+H]⁺.

¹H NMR (400 MHz, DMSO-*d₆*) δ 8.84 (s, 1H), 8.07-8.05 (d, 1H), 7.00 (s, 1H), 6.34 (d, 1H), 6.30-6.29 (d, 1H), 6.29-6.28 (d, 1H), 4.81-4.76 (d, 2H), 4.59-4.54 (d, 2H), 4.12-4.08 (t, 2H), 3.65-3.62 (m, 2H), 3.13-3.09 (m, 1H), 2.88-2.83 (t, 2H), 2.42 (s, 3H), 2.23-2.22 (d, 3H).

### Example 28:

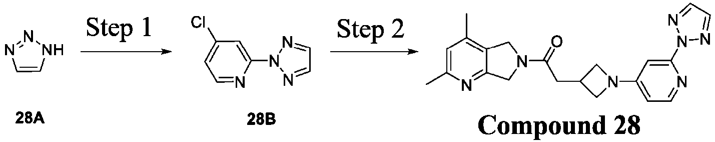

Step 1: At room temperature, **28A** (350 mg, 5.0 mmol) was dissolved in dry DMSO (10 mL), 4-chloro-2-fluoropyridine (660 mg, 5.0 mmol) and potassium carbonate (2.07 g, 15 mmol) were added, and the mixture was reacted at 40°C for 16 hours. Upon complete depletion of starting materials as monitored by TLC, the reaction was stopped. The reaction mixture was cooled to room temperature. To the reaction solution was added EA (30 mL), and the organic phase was washed with water (30 mL×3) and saturated aqueous sodium chloride solution (30 mL×2), dried over anhydrous sodium sulphate and filtered. The filtrate was concentrated and the residue was separated and purified by column chromatography (PE : EA = 10 : 1) to obtain compound **28B** (280 mg, 31.01%).

LC-MS (ESI): m/z= 181.1 [M+H]⁺.

Step 2: At room temperature, **28B** (180 mg, 1.0 mmol) was dissolved in dry DMSO (10 mL), and **2C** (246 mg, 1.0 mmol), potassium carbonate (415 mg, 3.0 mmol), and caesium fluoride (1.0 mmol, 152 mg) were added. The mixture was reacted at 100°C for 16 hours. Upon complete depletion of starting materials as monitored by TLC, the reaction was stopped. The reaction mixture was cooled to room temperature. To the reaction solution was added EA (30 mL), and the organic phase was washed with water (30 mL×3) and saturated aqueous sodium chloride solution (30 mL×2), dried over anhydrous sodium sulphate and filtered. The filtrate was concentrated and the residue was separated and purified by column chromatography (DCM : MeOH = 15 : 1) to obtain compound **28** (12 mg, 3.08%).

LC-MS (ESI): m/z= 390.1 [M+H]⁺.

¹H NMR (400 MHz, CDCl₃) δ 8.19-8.17 (d, 1H), 7.87 (s, 2H), 6.98 (d, 1H), 6.93-6.90 (d, 1H), 6.29-6.27 (m, 1H), 4.79-4.74 (m, 4H), 4.35-4.31 (m, 2H), 3.83-3.78 (m, 2H), 3.36-3.30 (m, 1H), 2.85-2.81 (t, 2H), 2.53 (s, 3H), 2.28-2.26 (d, 3H).

### Example 29:

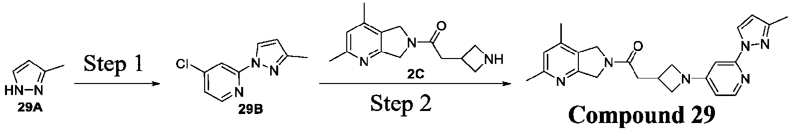

Step 1: **29A** (150 mg, 1.83 mmol), 4-chloro-2-fluoropyridine (240 mg, 1.83 mmol) and potassium carbonate (759 mg, 5.49 mmol) were dissolved in dimethyl sulphoxide (10 mL), and the mixture was reacted at 40°C overnight. After the reaction was completed, the reaction solution was cooled to room temperature, water (30 mL) was added to the reaction solution, and the resulting mixture was extracted with ethyl acetate (20 mL×3). The organic layers were combined, washed with saturated aqueous sodium chloride solution (30 mL), dried over anhydrous sodium sulphate and filtered. The filtrate was concentrated and the residue was separated and purified by silica gel column chromatography (petroleum ether : ethyl acetate (v/v) = 20 : 1) to obtain **29B** (225 mg, 63%).

LC-MS (ESI): m/z = 194.1 [M+H]⁺.

Step 2: **2C** (150 mg, 0.61 mmol), **29B** (118 mg, 0.61 mmol), caesium fluoride (111 mg, 0.73 mmol) and triethylamine (247 mg, 2.44 mmol) were dissolved in dimethyl sulphoxide (6 mL), and under nitrogen atmosphere, the mixture was reacted at 100°C overnight. After the reaction was completed, the reaction solution was cooled to room temperature, water (30 mL) was added to the reaction solution, and the resulting mixture was extracted with ethyl acetate (20 mL×3). The organic layers were combined, washed with saturated aqueous sodium chloride solution (30 mL), dried over anhydrous sodium sulphate, and filtered. The filtrate was concentrated and the residue was separated and purified by silica gel column chromatography (dichloromethane : methanol (v/v) = 25 : 1) to obtain **compound 29** (35 mg, 14%).

¹H NMR (400 MHz, CDCl₃) δ 8.40-8.38 (m, 1H), 8.01-7.98 (m, 1H), 6.91-6.89 (m, 1H), 6.86-6.85 (m, 1H), 6.20-6.19 (m, 1H), 6.14-6.11 (m, 1H), 4.78-4.72 (m, 4H), 4.31-4.26 (m, 2H), 3.79-3.73 (m, 2H), 3.34-3.23 (m, 1H), 2.82-2.78 (m, 2H), 2.53-2.51 (m, 3H), 2.36 (s, 3H), 2.27-2.25 (m, 3H).

LC-MS (ESI): m/z = 403.6 [M+H]⁺.

### Example 30:

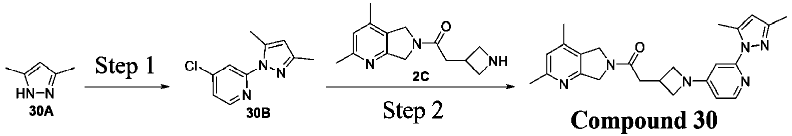

Step 1: **30A** (200 mg, 2.08 mmol), 4-chloro-2-fluoropyridine (274 mg, 2.08 mmol) and potassium carbonate (862 mg, 6.24 mmol) were dissolved in dimethyl sulphoxide (10 mL), and the mixture was reacted at 40°C overnight. After the reaction was completed, the reaction solution was cooled to room temperature, water (30 mL) was added to the reaction solution, and the resulting mixture was extracted with ethyl acetate (20 mL×3). The organic layers were combined, washed with saturated aqueous sodium chloride solution (30 mL), dried over anhydrous sodium sulphate and filtered. The filtrate was concentrated and the residue was separated and purified by silica gel column chromatography (petroleum ether : ethyl acetate (v/v) = 20 : 1) to obtain **30B** (133 mg, 31%).

LC-MS (ESI): m/z = 208.1 [M+H]⁺.

Step 2: **2C** (157 mg, 0.64 mmol), **30B** (133 mg, 0.64 mmol), caesium fluoride (117 mg, 0.77 mmol) and triethylamine (259 mg, 2.56 mmol) were dissolved in dimethyl sulphoxide (6 mL), and under nitrogen atmosphere, the mixture was reacted at 100°C overnight. After the reaction was completed, the reaction solution was cooled to room temperature, water (30 mL) was added to the reaction solution, and the resulting mixture was extracted with ethyl acetate (20 mL×3). The organic layers were combined, washed with saturated aqueous sodium chloride solution (30 mL), dried over anhydrous sodium sulphate, and filtered. The filtrate was concentrated and the residue was separated and purified by silica gel column chromatography (dichloromethane : methanol (v/v) = 25 : 1) to obtain **compound 30** (41 mg, 15%).

¹H NMR (400 MHz, CDCl₃) δ 8.08-8.06 (m, 1H), 6.92-6.90 (m, 1H), 6.69-6.68 (m, 1H), 6.19-6.16 (m, 1H), 5.96 (s, 1H), 4.80-4.71 (m, 4H), 4.31-4.26 (m, 2H), 3.80-3.73 (m, 2H), 3.33-3.25 (m, 1H), 2.82-2.77 (m, 2H), 2.57 (s, 3H), 2.53 (s, 3H), 2.29-2.25 (m, 6H).

LC-MS (ESI): m/z = 417.7 [M+H]⁺.

### Example 31:

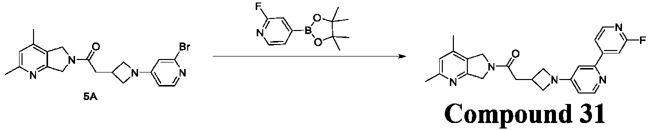

**5A** (80 mg, 0.2 mmol), 4-(4,4,5,5-tetramethyl-1,3,2-dioxaborolan-2-yl)-2-fluoropyridine (58 mg, 0.26 mmol), Pd(dppf)Cl₂ (16 mg, 0.02 mmol) and potassium carbonate (83 mg, 0.60 mmol) were successively added to mixed solvents of 1,4-dioxane (5 mL) and water (1 mL), and under nitrogen atmosphere, the mixture was heated to 90°C and reacted for 12 h. After the reaction was completed, the reaction mixture was cooled to room temperature, filtered, and concentrated. The residue was subjected to column chromatography (dichloromethane : methanol = 10 : 1) to obtain compound **31** (20 mg, 24%).

LC-MS (ESI): m/z = 418.2 [M+H]⁺.

¹H NMR (400 MHz, DMSO-d6) δ 8.31 (d, 1H), 8.26 (d, 1H), 8.00-8.02 (m, 1H), 7.78 (s, 1H), 7.09 (d, 1H), 7.00 (s, 1H), 6.42-6.44 (m, 1H), 4.81 (d, 2H), 4.57 (d, 2H), 4.19 (t, 2H), 3.71-3.74 (m, 2H), 3.11-3.15 (m, 1H), 2.86-2.88 (m, 2H), 2.42 (s, 3H), 2.23 (d, 3H).

### Example 32:

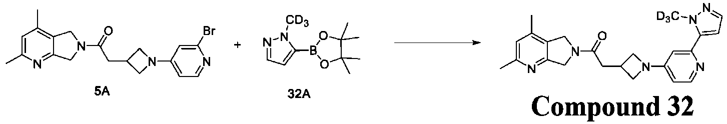

**5A** (80 mg, 0.2 mmol), 1-(methyl-d3)-5-(4,4,5,5-tetramethyl-1,3,2-dioxaborolan-2-yl)-1H-pyrazole (55 mg, 0.26 mmol), Pd(dppf)Cl₂ (16 mg, 0.02 mmol) and potassium carbonate (83 mg, 0.60 mmol) were added to mixed solvents of 1,4-dioxane (5 mL) and water (1 mL). The mixture was heated to 90°C and reacted for 12 h while the system was being purged. After the reaction was completed, the mixture was cooled to room temperature and then filtered. The filtrate was concentrated and the residue was subjected to column chromatography (dichloromethane : methanol = 10 : 1) to obtain compound **32** (20 mg, 25%).

LC-MS (ESI): m/z = 406.2 [M+H]⁺.

¹H NMR (400 MHz, DMSO-*d6*) δ 8.20 (d, 1H), 7.42 (d, 1H), 7.00-7.01 (m, 1H), 6.64-6.66 (m, 2H), 6.33-6.35 (m, 1H), 4.80 (d, 2H), 4.57(d, 2H), 4.14 (t, 2H), 3.66-3.69 (m, 2H), 3.10-3.15 (m, 1H), 2.84-2.86 (m, 2H), 2.42 (s, 3H), 2.23 (d, 3H).

### Example 33:

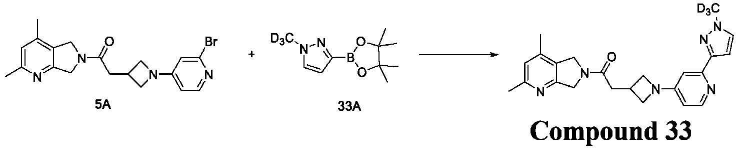

**5A** (80 mg, 0.2 mmol), 1-(methyl-d3)-3-(4,4,5,5-tetramethyl-1,3,2-dioxaborolan-2-yl)-1H-pyrazole (55 mg, 0.26 mmol), Pd(dppf)Cl₂ (16 mg, 0.02 mmol) and potassium carbonate (83 mg, 0.60 mmol) were successively added to mixed solvents of 1,4-dioxane (5 mL) and water (1 mL), and under nitrogen atmosphere, the mixture was heated to 90°C and reacted for 12 h. After the reaction was completed, the mixture was cooled to room temperature and then filtered. The filtrate was concentrated and the residue was separated and purified by column chromatography (dichloromethane : methanol = 10 : 1) to obtain compound **33** (15 mg, 19%).

LC-MS (ESI): m/z = 406.2 [M+H]⁺.

¹H NMR (400 MHz, DMSO-*d6*) δ 8.10 (d, 1H), 7.69 (d, 1H), 7.00 (s, 1H), 6.84 (d, 1H), 6.70 (d, 1H), 6.26-6.28 (m, 1H), 4.80 (d, 2H), 4.57(d, 2H), 4.12 (t, 2H), 3.64-3.67 (m, 2H), 3.10-3.15 (m, 1H), 2.84-2.86 (m, 2H), 2.42 (s, 3H), 2.23 (d, 3H).

### Example 34:

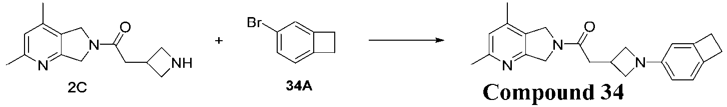

**2C** (200 mg, 0.82 mmol), 4-bromobenzocyclobutene (230 mg, 1.23 mmol), X-antphos (47 mg, 0.08 mmol), Pd₂dba₃ (38 mg, 0.04 mmol) and caesium carbonate (80 mg, 2.46 mmol) were successively added to 1,4-dioxane (5 mL), and under nitrogen atmosphere, the mixture was heated to 95°C and reacted for 12 h. After the reaction was completed, the reaction mixture was cooled to room temperature and filtered, and the filtrate was concentrated. The residue was separated and purified by column chromatography (dichloromethane : methanol = 10 : 1) to obtain compound **34** (20 mg, 7%).

LC-MS (ESI): m/z = 348.5 [M+H]⁺.

¹H NMR (400 MHz, DMSO-*d6*) δ 6.99 (s, 1H), 6.84 (d, 1H), 6.19-6.20 (m, 2H), 4.79 (d, 2H), 4.56(d, 2H), 3.91 (t, 2H), 3.41-3.44 (m, 2H), 2.99-3.03 (m, 5H), 2.78-2.81 (m, 2H), 2.42 (s, 3H), 2.23 (d, 3H).

### Example 35:

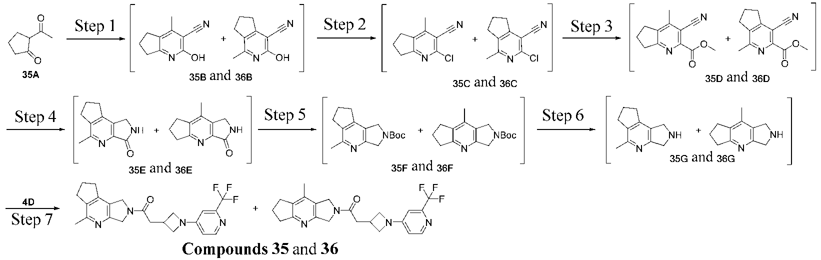

Step 1: Compound **35A** (6.05 g, 48 mmol), 2-cyanoacetamide (4.03 g, 48 mmol), and piperidine (4.5 g, 52.75 mmol) were successively dissolved in 1,4-dioxane (120 mL), and under nitrogen atmosphere, the mixture was reacted at 90°C overnight. After the reaction was completed, the mixture was cooled to room temperature and concentrated to obtain a mixture of compounds **35B** and **36B** (8 g, crude), which was directly used in the next reaction.

LC-MS (ESI): m/z = 175.1 [M+H]⁺.

Step 2: A mixture of compounds **35B** and **36B** (0.8 g, crude) was dissolved in 1,4-dioxane (5 mL), phosphorus oxychloride (5.04 g, 32.87 mmol) was added, and under nitrogen atmosphere, the mixture was reacted at 90°C overnight. After the reaction was completed, the mixture was cooled to room temperature and concentrated. To the residue were added ethyl acetate and sodium bicarbonate aqueous solution. The resulting mixture was extracted and subjected to phase separation, and the organic phase was dried over anhydrous sodium sulphate, filtered and concentrated. The residue was purified by silica gel column chromatography (ethyl acetate : petroleum ether = 0-60%) to obtain a mixture of compounds **35C** and **36C** (0.4 g, crude).

LC-MS (ESI): m/z = 193.2 [M+H]⁺.

Step 3: A mixture of compounds **35C** and **36C** (0.80 g, crude), triethylamine (0.63 g, 6.24 mmol), and 1,1'-bis(diphenylphosphino)ferrocene]palladium dichloride (II) (0.15 g, 0.21 mmol) were successively dissolved in methanol (10 mL) in an autoclave. After the autoclave was sealed, the system was purged by introducing carbon monoxide and pressurized to 2.5 MPa. Then the mixture was reacted at 110°C for 5 hours. After the reaction was completed, the mixture was cooled to room temperature and depressurized, and the reaction solution was concentrated and purified by column chromatography (ethyl acetate : petroleum ether (v : v) = 0-100%) to obtain a mixture of compounds **35D** and **36D** (0.5 g, crude).

LC-MS (ESI): m/z = 217.1 [M+H]⁺.

Step 4: A mixture of compounds **35D** and **36D** (1.7 g, 7.86 mmol) was dissolved in methanol (50 mL), raney nickel was added, and under hydrogen atmosphere, the mixture was reacted at room temperature overnight. After the reaction was completed, the mixture was filtered and the filtrate was concentrated under reduced pressure to obtain a mixture of compounds **35E** and **36E** (1.1 g, crude).

LC-MS (ESI): m/z = 189.1 [M+H]⁺.

Step 5: A mixture of compounds **35E** and **36E** (1.1 g, crude) was dissolved in tetrahydrofuran (20 mL), 10 moL/L borane dimethyl sulphide complex (5 mL) was added, and then the mixture was warmed to 80°C and reacted for 5 hours. The reaction mixture was cooled to room temperature, and concentrated under reduced pressure. To the residue was added 4 mol/L hydrogen chloride methanol solution (30 mL), and the mixture was then warmed to 80°C and reacted for 1 hour. After the reaction was completed, the mixture was cooled to room temperature and concentrated under reduced pressure. Methanol (30 mL) and triethylamine (5 mL) were added. After the mixture was stirred evenly, di-tert-butyl dicarbonate (3.82 g, 17.50 mmoL) was added and then the mixture was reacted at room temperature overnight. After the reaction was completed, the mixture was concentrated under reduced pressure. The resulting residue was separated and purified using a silica gel column (ethyl acetate : petroleum ether = 0-100%) to obtain a mixture of compounds **35F** and **36F** (0.45 g, crude).

LC-MS (ESI): m/z = 275.2 [M+H]⁺.

Step 6: A mixture of compounds **35F** and **36F** (0.45 g, crude) was dissolved in 4 mol/L hydrogen chloride 1,4-dioxane solution (20 mL), and the mixture was reacted at room temperature for 2 hours. After the reaction was completed, the mixture was concentrated to obtain a mixture of compounds **35G** and **36G** (0.5 g, crude).

LC-MS (ESI): m/z = 175.2 [M+H]⁺.

Step 7: A mixture of compounds **35G** and **36G** (0.5 g, crude), compound **4D** (0.42 g, 1.61 mmol), N,N-diisopropylethylamine (1.33 mL), and 2-(7-azobenzotriazole)-N,N,N',N'-tetramethyluronium hexafluorophosphate (0.73 g, 1.93 mmol) were successively dissolved in N,N-dimethylformamide (10 mL), and the mixture was reacted at room temperature for 2 hours. After the reaction was completed, the reaction solution was filtered, and the filtrate was separated and purified using a reverse-phase preparative column (C18 spherical 20-35 nm 100 A 80 g) (acetonitrile: water (0.1% TFA) = 10%-80%) to obtain **compound 35** (69 mg, 20.71%) and **compound 36** (45 mg, 13.51%).

**Compound 35:** ¹H NMR (400 MHz, DMSO-*d6*) δ 8.22 (d, 1H), 6.79-6.78 (m, 1H), 6.58-6.56 (m, 1H), 4.92 (d, 2H), 4.74 (s, 1H), 4.66 (s, 1H), 4.25-4.21 (m, 2H), 3.79-3.74 (m, 2H), 3.17-3.10 (m, 1H), 3.06-2.92 (m, 4H), 2.89-2.87 (m, 2H), 2.54 (d, 3H), 2.20-2.10 (m, 2H).

LC-MS (ESI): m/z = 417.4 [M+H]⁺.

**Compound 36:** ¹H NMR (400 MHz, DMSO-*d6*) δ 8.23 (d, 1H), 6.81-6.80 (m, 1H), 6.59-6.57 (m, 1H), 4.85 (d, 2H), 4.64-4.62 (m, 2H), 4.26-4.22 (m, 2H), 3.80-3.75 (m, 2H), 3.18-3.11 (m, 1H), 3.02-2.96 (m, 2H), 2.91-2.86 (m, 4H), 2.24 (d, 3H), 2.18-2.05 (m, 2H).

LC-MS (ESI): m/z = 417.4 [M+H]⁺.

### Example 37:

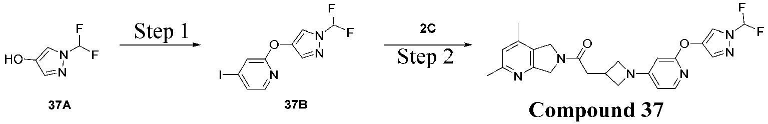

Step 1: With reference to the operation process of step 1 in Example 25, **compound 37B** (0.32 g, yield: 31%) was obtained using compound **37A** (0.4 g, 2.98 mmol) (prepared with reference to CN 113816950 A) as a starting material.

LC-MS (ESI): m/z = 337.2 [M+H]⁺.

Step 2: With reference to the operation process of step 2 in Example 25, **compound 37** (30 mg, yield: 18%) was obtained using compound **37B** (0.12 g, 0.36 mmol) as a starting material.

LC-MS (ESI): m/z =454.2 [M+H]⁺.

¹H NMR (400 MHz, CDCl₃): δ 8.00 -7.99 (m, 1H), 7.86 - 7.85 (m, 1H), 7.66 (s, 1H), 7.11 - 7.10 (m, 1H), 6.96 - 6.90 (m, 1H), 6.09 - 6.07 (m, 1H), 5.81 - 5.80 (m, 1H), 4.81 - 4.73 (m, 4H), 4.25 - 4.20 (m, 2H), 3.73 - 3.69 (m, 2H), 3.31 - 3.26 (m, 1H), 2.83 - 2.79 (m, 2H), 2.53 (s, 3H), 2.27 - 2.26 (m, 3H).

### Example 38:

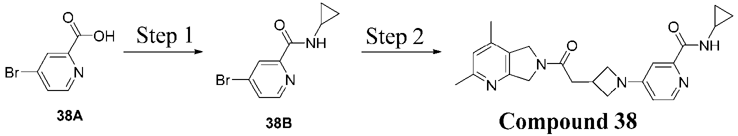

Step 1: Compound **38B** (0.8 g, 3.98 mmol) and cyclopropylamine (0.23 g, 3.98 mmol) were dissolved in DMF (10 mL), 1-methyl-1H-imidazole (0.98 g, 11.94 mmol) was added, and the mixture was stirred evenly. Then TCFH (1.23 g, 4.38 mmol) was added in portions and the resulting mixture was reacted at room temperature for 1 hour. After the reaction was completed, saturated aqueous sodium chloride solution (50 mL) was added, and the mixture was extracted with ethyl acetate (50 mL× 3). The combined organic phases were washed with saturated aqueous sodium chloride solution (60 mL), dried over anhydrous sodium sulphate, and filtered, and the filtrate was concentrated. The residue was separated by silica gel column chromatography (PE : EA (v/v) = 1 : 1) to obtain compound **38B** (0.45 g, yield: 48%).

LC-MS (ESI): m/z =241.2 [M+H]⁺.

Step 2: With reference to the operation process of step 2 in Example 25, **compound 38** (30 mg, yield: 15%) was obtained using compound **38B** (0.12 g, 0.50 mmol) as a starting material.

LC-MS (ESI): m/z =406.2 [M+H]⁺.

¹H NMR (400 MHz, CDCl₃): δ 8.09 -8.08 (m, 2H), 7.19 - 7.18 (m, 1H), 6.91-6.90 (m, 1H), 6.30 - 6.28 (m, 1H), 4.78-4.73 (m, 4H), 4.30 - 4.25 (m, 2H), 3.78-3.73 (m, 2H), 3.33 - 3.26 (m, 1H), 2.83 - 2.79 (m, 2H), 2.53-2.52 (m, 3H), 2.27 - 2.26 (m, 3H), 0.87-0.82 (m, 2H), 0.67-0.63 (m, 2H).

### Example 39:

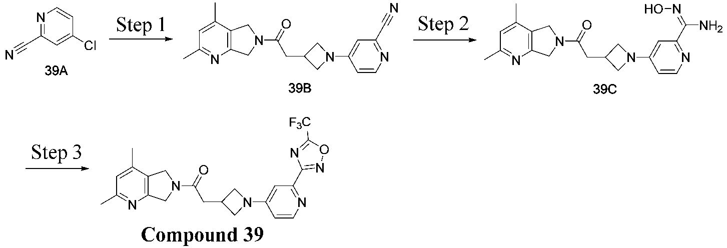

Step 1: With reference to step 1 in Example 10, **39B** (400 mg, yield: 52.10%) was obtained using **2C** (433 mg, 1.77 mmol) and **39A** (300 mg, 2.21 mmol) as starting materials.

LC-MS (ESI): m/z = 348.2 [M+H]⁺.

Step 2: To anhydrous ethanol (15 mL) were added **39B** (400 mg, 1.15 mmol), triethylamine (349 mg, 3.45 mmol) and hydroxylamine hydrochloride (96 mg, 1.38 mmol), and under nitrogen atmosphere, the mixture was stirred at room temperature overnight. After the reaction was completed as monitored by TLC, the reaction solution was concentrated under reduced pressure, and the residue was slurried with ethyl acetate/petroleum ether = 1/2 (5 mL) and filtered to obtain **39C** (400 mg, yield: 91.43%).

LC-MS (ESI): m/z = 381.8 [M+H]⁺.

Step 3: To toluene (10 mL) were added **39C** (400 mg, 0.87 mmol) and trifluoroacetic anhydride (220 mg, 1.04 mmol), and the reaction mixture was reacted under reflux under nitrogen atmosphere overnight. After the reaction was completed as monitored by TLC, the mixture was cooled to room temperature and concentrated under reduced pressure. The residue was separated and purified by silica gel column chromatography (dichloromethane : methanol (v/v) = 20 : 1) to obtain **compound 39** (35 mg, 10.57%).

LC-MS (ESI): m/z = 459.4 [M+H]⁺.

¹H NMR (400 MHz, DMSO-d6) δ 8.30-8.25 (m, 1H), 7.03-6.97 (m, 2H), 6.58- 6.52 (m, 1H), 4.84-4.75 (m, 2H), 4.62-4.52 (m, 2H), 4.25-4.16 (m, 2H), 3.78-3.69 (m, 2H), 3.20-3.09 (m, 1H), 2.88-2.84 (m, 2H), 2.43-2.41 (m, 3H), 2.24-2.21 (m, 3H).

### Example 40:

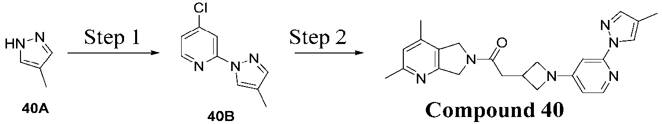

Step 1: **40A** (500 mg, 6.15mmol) was dissolved in DMSO (15 mL), and 4-chloro-2-fluoropyridine (970 mg, 7.38 mmol) and potassium carbonate (1.7 g, 12.31 mmol) were successively added. Subsequently, under nitrogen atmosphere, the mixture was warmed to 100°C, stirred and reacted overnight. After the reaction was completed as monitored by TLC and the reaction mixture was cooled to room temperature, the reaction was quenched with water (30 mL). The resulting mixture was extracted with ethyl acetate (20 mL×3), and the combined organic phases were washed with saturated aqueous sodium chloride solution, dried over anhydrous sodium sulphate, filtered, and concentrated under reduced pressure. The residue was separated and purified by silica gel column chromatography (ethyl acetate : petroleum ether (v/v) = 1 : 1) to obtain **40B** (523 mg, yield: 43.92%).

LC-MS (ESI): m/z = 194.3 [M+H]⁺.

Step 2: **40B** (200 mg, 1.03 mmol), **2C** (240 mg, 0.98 mmol), caesium fluoride (148 mg, 0.98 mmol) and potassium carbonate (270 mg, 1.96 mmol) were successively dissolved in DMSO (15 mL), and under nitrogen atmosphere, the mixture was warmed to 100°C, stirred and reacted overnight. After the reaction was completed as monitored by TLC, the reaction mixture was cooled to room temperature. The reaction was quenched with water (30 mL) and then the reaction mixture was extracted with ethyl acetate (20 mL×3). The combined organic phases were washed with saturated aqueous sodium chloride solution, dried over anhydrous sodium sulphate, filtered, and concentrated under reduced pressure. The residue was separated and purified by silica gel column chromatography (dichloromethane : methanol (v/v) = 15 : 1) to obtain **compound 40** (51 mg, yield: 12.93%).

LC-MS (ESI): m/z = 403.1 [M+H]⁺.

¹H NMR (400 MHz, DMSO-d6) δ 8.31-8.28 (m, 1H), 7.98-7.94 (m, 1H), 7.55 (s, 1H), 7.01-6.98 (m, 1H), 6.75-6.73 (m, 1H), 6.30-6.25 (m, 1H), 4.84-4.76 (m, 2H), 4.60-4.53 (m, 2H), 4.20-4.12 (m, 2H), 3.74-3.66 (m, 2H), 3.18-3.09 (m, 1H), 2.88- 2.83 (m, 2H), 2.44-2.40 (m, 3H), 2.24-2.21 (m, 3H), 2.08 (s, 3H).

### Example 41:

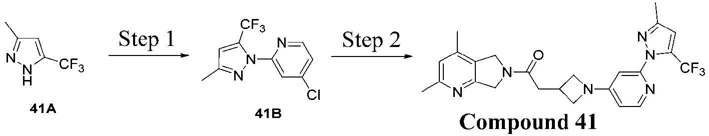

Step 1: With reference to the synthesis method of step 1 in **Example 28,** compound **41B** (180 mg, 69.61%) was synthesized using **41A** (150 mg, 1.0 mmol) as a starting material.

LC-MS (ESI): m/z= 262.0 [M+H]⁺.

Step 2: With reference to the synthesis method of step 2 in **Example 28,** compound **41** (30 mg, 11.12%) was synthesized using **41B** (150 mg, 0.57 mmol) as a starting material.

LC-MS (ESI): m/z= 471.3 [M+H]⁺.

¹H NMR (400 MHz, CDCl₃) δ 8.08-8.06 (d, 1H), 6.92-6.90 (d, 1H), 6.71 (s, 1H), 6.40 (s, 1H), 6.25-6.23 (m, 1H), 4.78-4.73 (m, 4H), 4.31-4.27 (t, 2H), 3.79-3.74 (m, 2H), 3.34-3.27 (m, 1H), 2.82-2.78 (t, 2H), 2.62 (s, 3H), 2.53 (s, 3H), 2.27 (d, 3H).

### Example 42:

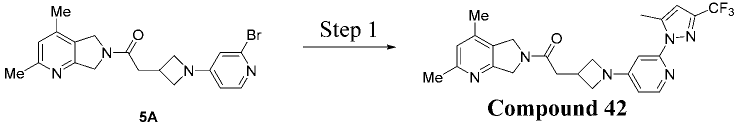

With reference to the synthesis method of step 1 in Example 9, compound **42** (32 mg, yield: 37.21%) was obtained using 3-trifluoromethyl-5-methylpyrazole (56 mg, 0.38 mmol) and **5A** (100 mg, 0.25 mmol) as starting materials.

LC-MS (ESI): m/z=471.1 [M+H]⁺.

¹H NMR (400 MHz, CDCl₃) δ 8.07 (d, 1H), 6.91 (d, 1H), 6.71 (t, 1H), 6.40 (s, 1H), 6.24 (m, 1H), 4.78-4.73 (m, 4H), 4.31-4.27 (m, 2H), 3.79-3.75 (m, 2H), 3.32-3.28 (m, 1H), 2.80 (t, 2H), 2.62 (s, 3H), 2.54 (s, 3H), 2.27 (d, 3H).

### Example 43:

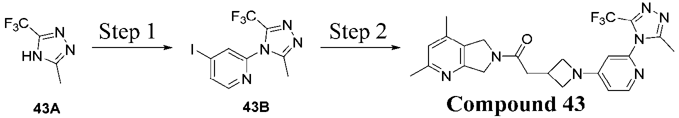

Step 1: With reference to the operation process of step 1 in Example 40, **43B** (488 mg, yield: 41.64%) was obtained using **43A** (500 mg, 3.31 mmol) and 2-fluoro-4-iodopyridine (812 mg, 3.64 mmol) as starting materials.

LC-MS (ESI): m/z = 355.0 [M+H]⁺.

Step 2: **2C** (150 mg, 0.61 mmol), **43B** (432 mg, 1.22 mmol), potassium carbonate (253 mg, 1.83 mmol) and RuPhos Pd G3 catalyst were successively dissolved in toluene (15 mL), and under nitrogen atmosphere, the mixture was warmed to 100°C, stirred and reacted overnight. After the reaction was completed as monitored by TLC, the reaction system was cooled to room temperature. The reaction solution was quenched by adding water (30 mL) and extracted with ethyl acetate (20 mL×3). The combined organic phases were washed with saturated aqueous sodium chloride solution, dried over anhydrous sodium sulphate, filtered, and concentrated under reduced pressure. The residue was separated and purified by silica gel column chromatography (dichloromethane : methanol (v/v) = 15 : 1) to obtain **compound 43** (8 mg, yield: 2.78%).

LC-MS (ESI): m/z = 472.2 [M+H]⁺.

¹H NMR (400 MHz, CDCl₃) δ 8.10-8.07 (m, 1H), 7.00-6.91 (m, 1H), 6.73-6.71 (m, 1H), 6.29-6.25 (m, 1H), 4.88-4.78 (m, 2H), 4.77-4.67 (m, 2H), 4.34-4.27 (m, 2H), 3.82-3.75 (m, 2H), 3.36-3.28 (m, 1H), 2.86 (s, 3H), 2.85-2.78 (m, 2H), 2.60-2.53 (m, 3H), 2.32-2.27 (m, 3H).

### Example 44:

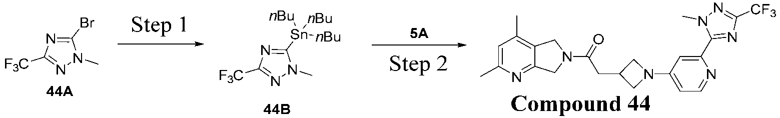

Step 1: **44A** (200 mg, 0.87 mmol) was dissolved in tetrahydrofuran (8 mL). Under nitrogen atmosphere, a solution of 2.5 M n-butyllithium (0.52 mL, 1.30 mmol) in n-hexane was added dropwise at -78°C, and after the dropwise addition was completed, the mixture was stirred for 20 min. Subsequently, a solution of tri-n-butyltin chloride (368 mg, 1.13 mmol) in tetrahydrofuran (2 mL) was added dropwise. After the dropwise addition was completed, the mixture was reacted at - 78°C for 2 h. After the reaction was completed, the mixture was allowed to warm to room temperature. The reaction was quenched with saturated aqueous sodium bicarbonate solution (25 mL) and the reaction mixture was extracted with ethyl acetate (20 mL×3). The combined organic phases were washed with saturated aqueous sodium chloride solution (30 mL), dried over anhydrous sodium sulphate and filtered. The filtrate was concentrated to obtain **44B** (220 mg), which was directly used in the next reaction without further purification.

Step 2: **44B** (220 mg, 0.50 mmol), **5A** (200 mg, 0.50 mmol), Pd₂(dba)₃ (58 mg, 0.05 mmol) and cuprous iodide (19 mg, 0.10 mmol) were successively dissolved in 1,4-dioxane (10 mL), and under nitrogen atmosphere, the mixture was warmed to 100°C and reacted overnight. After the reaction was completed, the reaction solution was cooled to room temperature, water (30 mL) was added to the reaction solution, and the resulting mixture was extracted with ethyl acetate (20 mL×3). The organic phases were combined, washed with saturated aqueous sodium chloride solution (30 mL), dried over anhydrous sodium sulphate, and filtered. The filtrate was concentrated and the resulting residue was separated and purified by silica gel column chromatography (dichloromethane : methanol (v/v) = 25 : 1) to obtain **compound 44** (50 mg, 21%).

¹H NMR (400 MHz, CDCl₃) δ 8.27-8.25 (m, 1H), 7.17-7.16 (m, 1H), 6.92-6.90 (m, 1H), 6.32-6.29 (m, 1H), 4.79-4.73 (m, 4H), 4.39 (s, 3H), 4.32-4.27 (m, 2H), 3.80-3.75 (m, 2H), 3.37-3.26 (m, 1H), 2.84-2.77 (m, 2H), 2.54-2.52 (m, 3H), 2.28-2.26 (m, 3H).

LC-MS (ESI): m/z = 472.1 [M+H]⁺.

### Example 45:

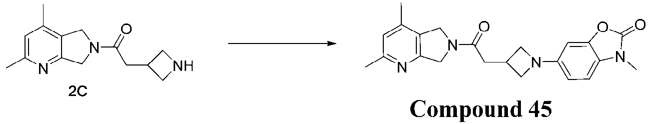

**2C** (200 mg, 0.82 mmol), 6-bromo-3-methylbenzo[d]oxazol-2(3H)-one (280 mg, 1.23 mmol), X-antphos (47 mg, 0.08 mmol), Pd₂(dba)₃ (38 mg, 0.04 mmol) and caesium carbonate (80 mg, 2.46 mmol) were successively dissolved in 1,4-dioxane (5 mL). Under nitrogen atmosphere, the mixture was warmed to 95°C and reacted for 12 h. After the reaction was completed, the mixture was cooled to room temperature and then filtered. The filtrate was concentrated and the residue was subjected to column chromatography (dichloromethane : methanol = 10 : 1) to obtain **compound 45** (9 mg, 3%).

LC-MS (ESI): m/z = 393.2 [M+H]⁺.

¹H NMR (400 MHz, DMSO-*d6*) δ 7.04 (d, 1H), 6.99 (s, 1H), 6.48 (d, 1H), 6.24-6.27 (m, 1H), 4.80 (d, 2H), 4.57(d, 2H), 3.96 (t, 2H), 3.48 (t, 2H), 3.27 (s, 3H), 3.00-3.05 (m, 1H), 2.79-2.82 (m, 2H), 2.42 (s, 3H), 2.23 (d, 3H).

### Example 46:

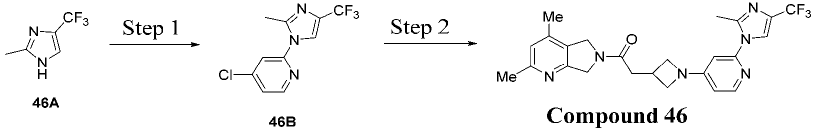

Step 1: With reference to the synthesis method of step 1 in Example 40, compound **46B** (0.31 g, yield: 59.84%) was obtained using compound **46A** (0.3 g, 1.98 mmol) and 4-chloro-2-fluoropyridine (0.3 g, 1.98 mmol) as starting materials.

LC-MS (ESI): m/z=262.1 [M+H]⁺.

Step 2: With reference to the synthesis method of step 2 in Example 40, **compound 46** (31 mg, yield: 5.74%) was obtained using compound **46B** (0.3 g, 1.15 mmol) and compound **2C** (0.54 g, 1.15 mmol) as starting materials.

LC-MS (ESI): m/z=471.6[M+H]⁺.

¹H NMR (400 MHz, CDCl₃) δ 8.13 (d, 1H), 7.53-7.52 (m, 1H), 6.92 (d, 1H), 6.29-6.27 (m, 1H), 6.15 (s, 1H), 4.78-4.73 (m, 4H), 4.30-4.26 (m, 2H), 3.80-3.76 (m, 2H), 3.31-3.27 (m, 1H), 2.85-2.81 (m, 2H), 2.54-2.53 (m, 6H), 2.27 (d, 3H).

### Example 47:

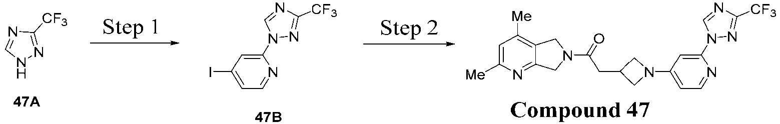

Step 1: With reference to the synthesis method of step 1 in Example 40, compound **47B** (110 mg, yield: 89.86%) was obtained using compound **47A** (50 mg, 0.36 mmol) and 4-iodo-2-fluoropyridine (88 mg, 0.40 mmol) as starting materials.

LC-MS (ESI): m/z = 341.0[M+H]⁺.

Step 2: Compound **2C** (79 mg, 0.32 mmol), compound **47B** (110 mg, 0.32 mmol), RuPhos-Pd-G3 (27 mg, 0.032 mmol) and potassium carbonate (88 mg, 0.64 mmol) were successively dissolved in toluene (5 mL), and under nitrogen atmosphere, the mixture was heated to 100°C and reacted for 16 h. After the reaction was completed, the reaction mixture was cooled to room temperature and filtered, and the filtrate was concentrated. The residue was separated and purified by column chromatography (dichloromethane : methanol = 10 : 1) to obtain **compound 47** (19 mg, 12.9%).

LC-MS (ESI): m/z = 458.5[M+H]⁺.

¹H NMR (400 MHz, CDCl₃) δ 9.17 (s, 1H), 8.05-8.03 (m, 1H), 6.93 (d, 1H), 6.82-6.81 (m, 1H), 6.27-6.25 (m, 1H), 4.84-4.74 (m, 4H), 4.35-4.30 (m, 2H), 3.83-3.78 (m, 2H), 3.35-3.31 (m, 1H), 2.84-2.81 (m, 2H),2.53 (d, 3H), 2.28 (d, 3H).

### Example 48:

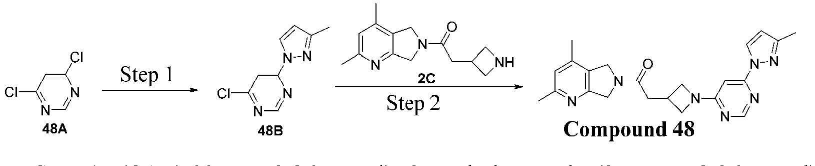

Step 1: **48A** (500 mg, 3.36 mmol), 3-methylpyrazole (275 mg, 3.36 mmol) and caesium carbonate (1.64 g, 5.03 mmol) were dissolved in DMF (20 mL), and the mixture was stirred at room temperature under nitrogen atmosphere overnight. After the reaction was completed, water (40 mL) was added to the reaction solution, and the resulting mixture was extracted with ethyl acetate (20 mL×3). The organic layers were combined, washed with saturated aqueous sodium chloride solution (30 mL), dried over anhydrous sodium sulphate and filtered. The filtrate was concentrated and the residue was separated and purified by silica gel column chromatography (petroleum ether : ethyl acetate (v/v) = 15 : 1) to obtain **48B** (310 mg, 47%).

LC-MS (ESI): m/z = 195.3 [M+H]⁺.

Step 2: With reference to the procedures of step 2 in Example 26, **compound 48** (145 mg, 23%) was synthesized using **48B** (310 mg, 1.59 mmol) as a starting material.

¹H NMR (400 MHz, CDCl₃) δ 8.43-8.37 (m, 2H), 6.92-6.89 (m, 1H), 6.66 (s, 1H), 6.24-6.22 (m, 1H), 4.80-4.71 (m, 4H), 4.44-4.38 (m, 2H), 3.92-3.86 (m, 2H), 3.36-3.25 (m, 1H), 2.84-2.78 (m, 2H), 2.54-2.52 (m, 3H), 2.36 (s, 3H), 2.27-2.25 (m, 3H).

LC-MS (ESI): m/z = 404.5 [M+H]⁺.

### Example 49:

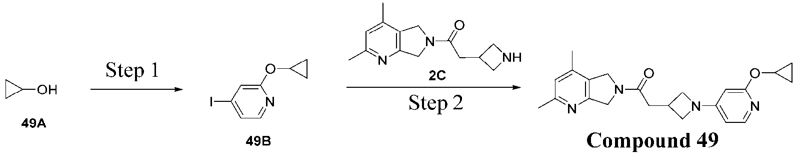

Step 1: With reference to the operation process of step 1 in Example 25, **compound 49B** (0.8 g, yield: 18%) was obtained using compound **49A** (1.0 g, 17.24 mmol) as a starting material.

LC-MS (ESI): m/z= 262.1 [M+H]⁺.

Step 2: With reference to the operation process of step 2 in Example 25, **compound 49** (0.02 g, yield: 14%) was obtained using compound **49B** (0.1 g, 0.38 mmol) as a starting material.

LC-MS (ESI): m/z= 379.2 [M+H]⁺.

¹H NMR (400 MHz, CDCl₃): δ 7.89-7.87 (m, 1H), 6.91-6.90 (m, 1H), 6.21-6.20 (m, 1H), 6.00 -5.98 (m, 1H), 5.71-5.70 (m, 1H), 4.77-4.72 (m, 4H), 4.21-4.16 (m, 2H), 3.69-3.64 (m, 2H), 3.29- 3.22 (m, 1H), 2.81-2.77 (m, 2H), 2.53-2.52 (m, 3H), 2.27-2.26 (m, 3H), 0.77-0.75 (m, 4H).

### Example 50:

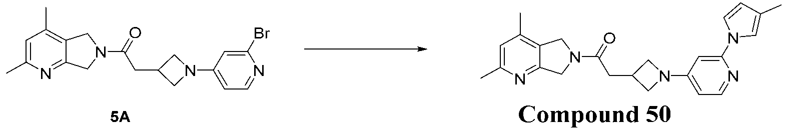

Compound **5A** (0.2 g, 0.50 mmol) was dissolved in DMF (5 mL), and 3-methylpyrrole (0.081 g, 1.0 mmol), cuprous iodide (0.029 g, 0.15 mmol), 1,10-phenanthroline (0.027 g, 0.15 mmol), and caesium carbonate (0.41 g, 1.26 mmol) were successively added. Subsequently, under nitrogen atmosphere, the mixture was warmed to 110°C and reacted for 16 hours. After the reaction was completed, the reaction solution was cooled to room temperature. To the reaction solution was added water (20 mL), and the resulting mixture was extracted with ethyl acetate (20 mL×3). The combined organic phases were washed with saturated aqueous sodium chloride solution, dried over anhydrous sodium sulphate, filtered and concentrated. The resulting crude product was purified using Biotage Isolera One (20 g silica gel column, eluents: 0-15% methanol/dichloromethane) to obtain compound **50** (51 mg, 25.40%).

LC-MS (ESI): m/z = 402.3 [M+H]⁺.

¹H NMR (400 MHz, CDCl₃): δ 8.02 (d, 1H), 7.35 (t, 1H), 7.20-7.18 (m, 1H), 6.91 (d, 1H), 6.14-6.07 (m, 3H), 4.79-4.73 (m, 4H), 4.28-4.23 (m, 2H), 3.76-3.70 (m, 2H), 3.33-3.27 (m, 1H), 2.81(t, 2H), 2.53 (d, 3H), 2.26 (d, 3H), 2.14 (d, 3H).

### Example 51:

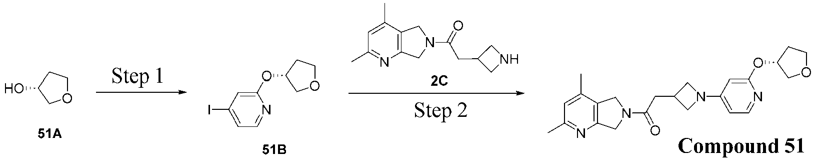

Step 1: With reference to the synthesis method of step 1 in **Example 25,** compound **51B** (0.73 g, 80.12%) was obtained using **51A** (0.7 g, 3.13 mmol) as a starting material.

LC-MS (ESI): m/z= 292.1 [M+H]⁺.

Step 2: With reference to the synthesis method of step 2 in **Example 25,** compound **51** (10 mg, 5.97%) was synthesized using **51B** (100 mg, 0.41 mmol) as a starting material.

LC-MS (ESI): m/z= 409.3 [M+H]⁺.

¹H NMR (400 MHz, CDCl₃): δ 7.81-7.80 (m, 1H), 6.91-6.90 (m, 1H), 5.99-5.97 (m, 1H), 5.65 -5.64 (m, 1H), 5.57-5.54 (m, 1H), 4.78-4.72 (m, 4H), 4.20-4.16 (m, 2H), 4.01-3.85 (m, 4H), 3.67- 3.63 (m, 2H), 3.28-3.24 (m, 1H), 2.81-2.77 (m, 2H), 2.54-2.52 (m, 3H), 2.30-2.26 (m, 3H), 2.24-2.11 (m, 2H).

### Example 52:

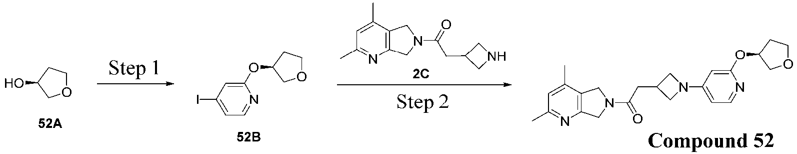

Step 1: With reference to the synthesis method of step 1 in **Example 25,** compound **52B** (0.71 g, 77.93%) was obtained using **52A** (0.7 g, 3.13 mmol) as a starting material.

LC-MS (ESI): m/z= 292.0 [M+H]⁺.

Step 2: With reference to the synthesis method of step 2 in **Example 25,** compound **52** (40 mg, 19.98%) was obtained using **52B** (120 mg, 0.49 mmol) as a starting material.

LC-MS (ESI): m/z = 409.5 [M+H]⁺.

¹H NMR (400 MHz, CDCl₃): δ 7.82-7.80 (m, 1H), 6.91-6.90 (m, 1H), 6.00-5.98 (m, 1H), 5.65 -5.64 (m, 1H), 5.57-5.54 (m, 1H), 4.78-4.73 (m, 4H), 4.21-4.18 (m, 2H), 4.03-3.85 (m, 4H), 3.70- 3.65 (m, 2H), 3.28-3.24 (m, 1H), 2.81-2.77 (m, 2H), 2.54-2.52 (m, 3H), 2.26-2.25 (m, 3H), 2.24-2.13 (m, 2H).

### Example 53:

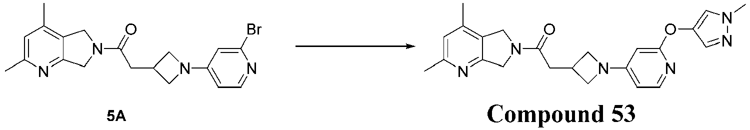

Compound **5A** (120 mg, 0.30 mmol), 1-methyl-1*H*-pyrazol-4-ol (60 mg, 0.44 mmol), and caesium carbonate (0.39 g, 1.2 mmol) were successively dissolved in N,N-dimethylacetamide (5 mL). Subsequently, the mixture was warmed to 140°C and reacted for 6 hours. After the reaction was completed, the reaction solution was cooled to room temperature. Water (20 mL) was added and the resulting mixture was extracted with EA (20 mL×3). The combined organic phases were washed with saturated aqueous sodium chloride solution, dried over anhydrous sodium sulphate, filtered and concentrated. The resulting crude product was purified using Biotage Isolera One (12 g silica gel column, eluents: 0-15% methanol/dichloromethane) to obtain compound **53** (31 mg, yield: 27.88%).

LC-MS (ESI): m/z = 419.5 [M+H]⁺.

¹H NMR (400 MHz, DMSO-*d₆*) δ 7.85 (d, 1H), 7.46 (t, 1H), 7.40 (d, 1H), 6.91 (d, 1H), 6.05-6.03 (m, 1H), 5.76 (d, 1H), 4.77-4.72 (m, 4H), 4.23-4.18 (m, 2H), 3.87 (s, 3H), 3.71-3.67 (m, 2H), 3.30-3.24 (m, 1H), 2.81-2.77 (m, 2H), 2.52 (d, 3H), 2.26 (d, 3H).

### Example 54:

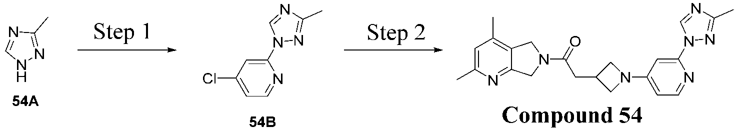

Step 1: With reference to the synthesis method of step 1 in Example 40, compound **54B** (0.51 g, yield: 43.97%) was obtained using compound **54A** (0.5 g, 5.96 mmol) and 2-fluoro-4-chloropyridine (0.78 g, 5.96 mmol) as starting materials.

LC-MS (ESI): m/z = 195.1[M+H]⁺.

Step 2: With reference to the synthesis method of step 2 in Example 40, compound **54** (36 mg, yield: 8.66%) was obtained using compound **54B** (0.2 g, 1.03 mmol) and compound **2C** (0.13 g, 0.13 mmol) as starting materials.

LC-MS (ESI): m/z = 404.2[M+H]⁺.

¹H NMR (400 MHz, CDCl₃) δ 9.01 (s, 1H), 8.02 (d, 1H), 6.93 (d, 1H), 6.75 (t, 1H), 6.20-6.18 (m, 1H), 4.82-4.74 (m, 4H), 4.33-4.28 (m, 2H), 3.80-3.75 (m, 2H), 3.33-3.27 (m, 1H), 2.83-2.79 (m, 2H), 2.55 (d, 3H), 2.48 (s, 3H), 2.27 (d, 3H).

### Example 55:

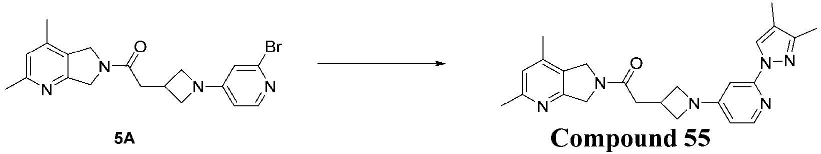

With reference to the synthesis method in Example 50, compound **55** (31 mg, yield: 29.77%) was obtained using compound **5A** (100 mg, 0.25 mmol) and 3,4-dimethyl-1H-pyrazole (96 mg, 1.0 mmol) as starting materials.

LC-MS (ESI): m/z = 417.2[M+H]⁺.

¹H NMR (400 MHz, CDCl₃) δ 8.30 (s, 1H), 7.98 (d, 1H), 6.90 (d, 1H), 6.80 (s, 1H), 6.11 (d, 1H), 4.78-4.72 (m, 4H), 4.33-4.29 (m, 2H), 3.81-3.76 (m, 2H), 3.33-3.27 (m, 1H), 2.80 (t, 2H), 2.53 (d, 3H), 2.27-2.26 (m, 6H), 2.05 (s, 3H).

### Example 56:

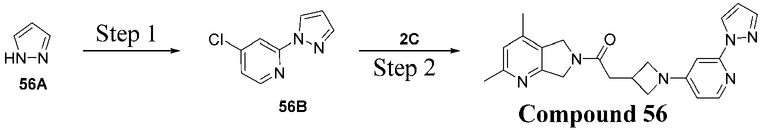

Step 1: With reference to the synthesis method of step 1 in Example 26, **56B** (304 mg, 72%) was obtained using **56A** (160 mg, 2.37 mmol) as a starting material.

LC-MS (ESI): m/z = 180.1 [M+H]⁺.

Step 2: With reference to the synthesis method of step 2 in Example 26, **compound 56** (132 mg, 20%) was obtained using **56B** (304 mg, 1.69 mmol) as a starting material.

¹H NMR (400 MHz, CDCl₃) δ 8.53-8.51 (m, 1H), 8.04-8.01 (m, 1H), 7.69 (s, 1H), 6.92-6.89 (m, 2H), 6.43-6.41 (m, 1H), 6.18-6.15 (m, 1H), 4.79-4.72 (m, 4H), 4.32-4.26 (m, 2H), 3.79-3.73 (m, 2H), 3.35-3.25 (m, 1H), 2.83-2.78 (m, 2H), 2.54-2.51 (m, 3H), 2.27-2.25 (m, 3H).

LC-MS (ESI): m/z = 389.1 [M+H]⁺.

### Example 57:

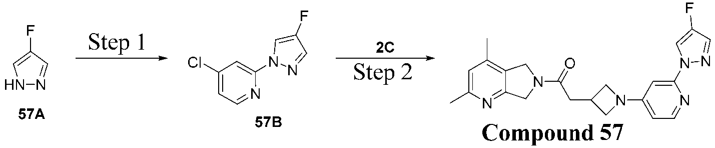

Step 1: With reference to the synthesis method of step 1 in Example 26, **57B** (210 mg, 47%) was obtained using **57A** (197 mg, 2.29 mmol) as a starting material.

LC-MS (ESI): m/z = 198.1 [M+H]⁺.

Step 2: With reference to the synthesis method of step 2 in Example 26, **compound 57** (85 mg, 20%) was obtained using **57B** (210 mg, 1.06 mmol) as a starting material.

¹H NMR (400 MHz, CDCl₃) δ 9.11 (s, 1H), 8.06-7.99 (m, 2H), 6.92-6.89 (m, 1H), 6.82-6.80 (m, 1H), 6.23-6.20 (m, 1H), 4.82-4.69 (m, 4H), 4.32-4.27 (m, 2H), 3.81-3.76 (m, 2H), 3.38-3.26 (m, 1H), 2.85-2.80 (m, 2H), 2.54-2.51 (m, 3H), 2.27-2.25 (m, 3H).

LC-MS (ESI): m/z = 407.1 [M+H]⁺.

### Example 58:

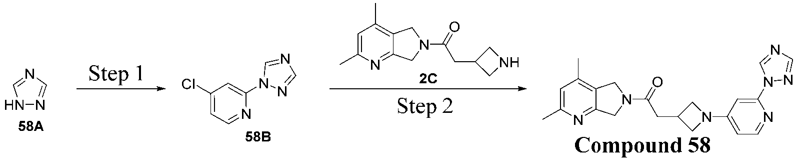

Step 1: With reference to the synthesis method of step 1 in Example 26, **58B** (228 mg, 55%) was obtained using **58A** (160 mg, 2.33 mmol) as a starting material.

LC-MS (ESI): m/z = 181.1 [M+H]⁺.

Step 2: With reference to the synthesis method of step 2 in Example 26, **compound 58** (114 mg, 23%) was obtained using **58B** (228 mg, 1.27 mmol) as a starting material.

¹H NMR (400 MHz, CDCl₃) δ 8.37-8.35 (m, 1H), 8.01-7.98 (m, 1H), 7.54-7.52 (m, 1H), 6.91-6.89 (m, 1H), 6.87-6.85 (m, 1H), 6.18-6.15 (m, 1H), 4.79-4.72 (m, 4H), 4.31-4.25 (m, 2H), 3.79-3.73 (m, 2H), 3.34-3.24 (m, 1H), 2.83-2.78 (m, 2H), 2.54-2.51 (m, 3H), 2.27-2.25 (m, 3H).

LC-MS (ESI): m/z = 390.2 [M+H]⁺.

### Example 59:

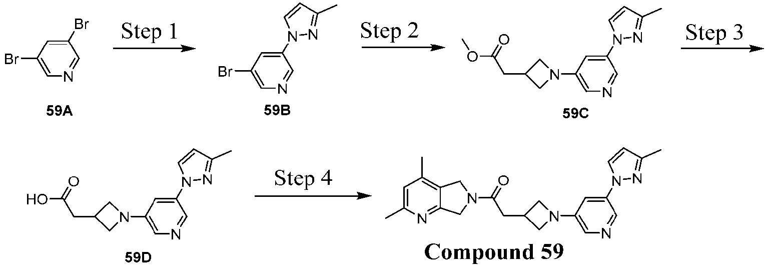

Step 1: **59A** (5 g, 21.13 mmol), 3-methyl-1H-pyrazole (2.26 g, 27.47 mmol), cuprous iodide (0.80 g, 4.23 mmol), N,N'-dimethyl-1,2-ethylenediamine (0.75 g, 8.45 mmol), and potassium carbonate (7.30 g, 52.82 mmol) were successively dissolved in 1,4-dioxane (60 mL), and under nitrogen atmosphere, the mixture was warmed to 105°C and reacted for 12 hours. After the reaction was completed, the mixture was cooled to room temperature and concentrated. The resulting crude product was separated and purified by column chromatography (petroleum ether : ethyl acetate = 5 : 1) to obtain **59B** (1.7 g, 33%).

LC-MS (ESI): m/z = 238.1[M+H]⁺.

Step 2: **59B** (0.6 g, 2.52 mmol), methyl 2-(azetidin-3-yl)acetate (0.81 g, 6.27 mmol), caesium carbonate (1.64 g, 5.04 mmol), and methanesulphonato(2-dicyclohexylphosphino-2',6'-di-isopropoxy-1,1'-biphenyl)(2'-amino-1,1'-biphenyl-2-yl)palladium (II) (0.21 g, 0.25 mmol) were successively dissolved in 1,4-dioxane (15 mL). Subsequently, under nitrogen atmosphere, the mixture was warmed to 95°C and reacted for 12 hours. After the reaction was completed, the mixture was cooled to room temperature and concentrated. The resulting crude product was separated and purified by column chromatography (petroleum ether : ethyl acetate = 1 : 4) to obtain **59C** (0.4 g, 55%).

LC-MS (ESI): m/z = 287.2 [M+H]⁺.

Step 3: **59C** (0.1 g, 0.35 mmol) was dissolved in mixed solvents of methanol (2 mL), tetrahydrofuran (2 mL) and water (2 mL), lithium hydroxide (0.021 g, 0.88 mmol) was added, and subsequently, the mixture was reacted at room temperature for 2 hours. After the reaction was completed, the mixture was concentrated to obtain crude product **59D** (90 mg).

LC-MS (ESI): m/z = 273.2 [M+H]⁺.

Step 4: **59D** (0.090 g, 0.33 mmol), **intermediate 1** (0.059 g, 0.40 mmol), 1-methylimidazole hydrochloride (0.051 g, 0.43 mmol), and [chloro(dimethylamino)methylene]-dimethylazane hexafluorophosphate (0.12 g, 0.43 mmol) were successively dissolved in acetonitrile (4 mL). Subsequently, the mixture was reacted at 40°C for 2 hours. After the reaction was completed, the reaction solution was separated and purified by preparative HPLC to obtain **compound 59** (50 mg, 37%). Separation method: Instrument: waters 2767 preparative liquid chromatographic instrument; chromatographic column: SunFire@ Prep C18 (19 mm × 150 mm); composition of mobile phases: mobile phase A: acetonitrile; and mobile phase B: water (containing 0.1% trifluoroacetic acid); gradient: 20%-40% acetonitrile gradient.

LC-MS (ESI): m/z = 403.5 [M+H]⁺.

¹H NMR (400 MHz, DMSO-*d*₆) δ 8.62-8.40 (m, 2H), 7.82 (d, 1H), 7.46-7.41 (m, 1H), 7.18 (d, 1H), 6.42 (d, 1H), 5.00-4.81 (m, 2H), 4.63 (d, 2H), 4.26-4.18 (m, 2H), 3.79-3.71 (m, 2H), 3.29- 3.08 (m, 1H), 2.90-2.84 (m, 2H), 2.48 (s, 3H), 2.31-2.27 (m, 6H).

### Example 60:

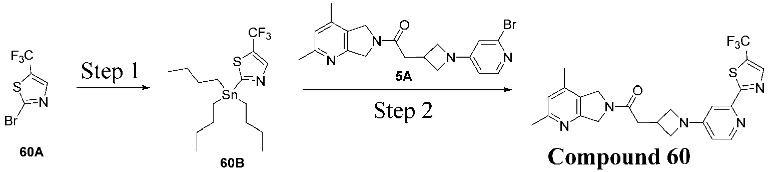

Step 1: With reference to the synthesis method of step 1 in Example 44, compound **60B** (0.5 g, 99.99%) was obtained using **60A** (0.13 g, 0.56 mmol) as a starting material.

Step 2: With reference to the synthesis method of step 2 in Example 44, compound **60** (40 mg, 31.29%) was obtained using **60B** (120 mg, 0.27 mmol) as a starting material.

LC-MS (ESI): m/z = 474.5 [M+H]⁺.

¹H NMR (400 MHz, CDCl₃): δ 8.21-8.10 (m, 2H), 7.17 (s, 1H), 6.94-6.90 (m, 1H), 6.32 (s, 1H), 4.80-4.79 (m, 2H), 4.75-4.74 (m, 2H), 4.32-4.31 (m, 2H), 3.80-3.78 (m, 2H), 3.35-3.30 (m, 1H), 2.84-2.80 (m, 2H), 2.55-2.54 (m, 3H), 2.29-2.26 (m, 3H).

### Example 61:

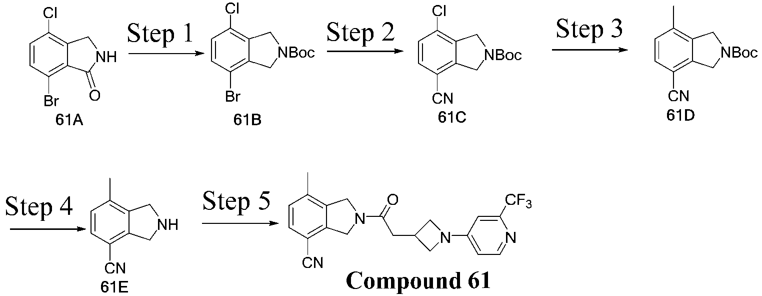

Step 1: Compound **61A** (2.8 g, 11.4 mmol) (synthesized with reference to the method described in WO 2021050964) was dissolved in tetrahydrofuran (40 mL), and in an ice bath, borane tetrahydrofuran (45 mL, 1M THF solution) was slowly added dropwise. After the dropwise addition was completed, the mixture was reacted at 70°C for 6 hours. After the reaction was completed, the mixture was cooled to room temperature and methanol (20 mL) was slowly added dropwise. After the reaction was completely quenched, the system was concentrated under reduced pressure. Dichloromethane (50 mL) and triethylamine (5.0 g, 50 mmol) were added. After the mixture was stirred evenly, di-tert-butyl dicarbonate (4.36 g, 20 mmol) was added. The mixture was reacted for another 1 hour. Saturated sodium bicarbonate (80 mL) was added, and the resulting mixture was extracted with dichloromethane (80 mL× 2). The combined organic phases were washed with saturated aqueous sodium chloride solution (60 mL), dried over anhydrous sodium sulphate, filtered and concentrated. The residue was separated by silica gel column chromatography (PE : EA (v/v) = 20 : 1) to obtain **61B (1.7 g, yield: 45%).**

LC-MS (ESI): m/z = 275.9 [M-^{t}Bu+H]⁺.

¹H NMR (400 MHz, Chloroform-d) δ 7.35 (d, 1H), 7.13 (d, 1H), 4.81-4.57 (m, 4H), 1.53 (s, 9H).

Step 2: Compound **61B** (0.71 g, 2.1 mmol) was dissolved in DMF (20 mL), and then zinc cyanide (0.50 g, 4.3 mmol) and tetrakis(triphenylphosphine)palladium (0.35 g, 0. 3 mmol) were added. Subsequently, under nitrogen atmosphere, the mixture was warmed to 80°C and reacted for 7 hours. After the reaction was completed, the reaction solution was cooled to room temperature. Saturated aqueous sodium bicarbonate solution (80 mL) was added and the resulting mixture was extracted with ethyl acetate (80 mL× 2). The combined organic phases were washed with saturated aqueous sodium chloride solution (60 mL), dried over anhydrous sodium sulphate and filtered. The filtrate was concentrated and the residue was separated by silica gel column chromatography (PE : EA (v/v) = 15 : 1) to obtain **61C (0.32 g, yield: 55%).**

LC-MS (ESI): m/z = 223.1 [M-^{t}Bu+H]⁺.

Step 3: Compound **61C** (0.32 g, 1.1 mmol) was dissolved in 1,4-dioxane (30 mL), and then trimethylboroxine (0.82 g, 50% THF solution), Pd(dppf)Cl₂ (0.15 g, 0.2 mmol) and potassium carbonate (0.30 g, 2.2 mmol) were successively added. Subsequently, under nitrogen atmosphere, the mixture was warmed to 90°C and reacted for 2 hours. After the reaction was completed, the reaction mixture was cooled to room temperature. Saturated aqueous sodium bicarbonate solution (80 mL) was added and the resulting mixture was extracted with ethyl acetate (80 mL× 2). The combined organic phases were washed with saturated aqueous sodium chloride solution (80 mL), dried over anhydrous sodium sulphate, filtered and concentrated. The residue was separated by silica gel column chromatography (PE : EA (v/v) = 10 : 1) to obtain **61D (0.17 g, yield: 60%).**

LC-MS (ESI): m/z = 203.2 [M-^{t}Bu+H]⁺.

Step 4: Compound **61D** (0.17 g, 0.66 mmol) was dissolved in dichloromethane (10 mL), and the mixture was stirred evenly. Then trifluoroacetic acid (2 mL) was added dropwise. After the dropwise addition was completed, the mixture was reacted at room temperature for 1 hour. After the reaction was completed, the mixture was concentrated under reduced pressure to obtain crude compound **61E,** which was directly used in the next reaction.

LC-MS (ESI): m/z = 159.1 [M+H]⁺.

Step 5: With reference to the operation of step 7 in Example 3, **compound 61 (0.13 g, yield: 49%)** was synthesized using compounds **61E** (100 mg, 0.66 mmol) and **4D** (0.17 g, 0.66 mmol) as starting materials.

LC-MS (ESI): m/z = 401.4 [M+H]⁺.

¹H NMR (400 MHz, Methanol-*d*₄) δ 8.14 (d, 1H), 7.58 (d, 1H), 7.30 (d, 1H), 6.69 (t, 1H), 6.50 (d, 1H), 5.08 (s, 1H), 4.91 (d, 2H), 4.77 (s, 1H), 4.30-4.24 (m, 2H), 3.85-3.77 (m, 2H), 3.29-3.18 (m, 1H), 2.96 (d, 2H), 2.38 (s, 3H).

### Example 62:

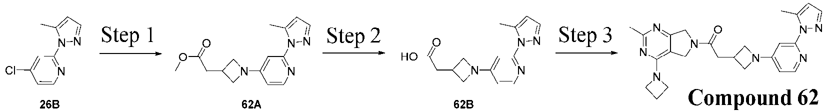

Step 1: Compound **26B** (4.0 g, 20.66 mmol), caesium fluoride (4.01 g, 26.86 mmol) and triethylamine (11.51 mL, 82.64 mmol) were successively dissolved in DMSO (40 mL), and then methyl 2-(azetidin-3-yl)acetate 2,2,2-trifluoroacetate (7.54 g, 30.99 mmol) was added. The mixture was warmed to 100°C and reacted for 16 hours. After the reaction was completed as monitored by TLC, the reaction solution was cooled to room temperature, diluted with water (500 mL), and then extracted with ethyl acetate (100 mL×2). The combined organic phases were dried over anhydrous sodium sulphate, filtered and concentrated. The resulting residue was purified by silica gel column chromatography (PE : EA = 1 : 1) to obtain compound **62A** (1.2 g, yield: 20.30%).

LC-MS(ESI):m/z = 287.1[M+H]⁺.

Step 2: Compound **62A** (1.2 g, 4.19 mmol) was dissolved in mixed solvents of THF : MeOH : H₂O = 3 : 1 : 1 (15 mL), lithium hydroxide (0.1 g, 4.19 mmol) was added, and the mixture was reacted at room temperature for 16 hours. After the reaction was completed as monitored by TLC, the reaction solution was concentrated under reduced pressure to obtain crude compound **62B** (1.3 g).

LC-MS(ESI):m/z = 273.2[M+H]⁺.

Step 3: Compound **62B** (270 mg, 0.97 mmol), 4-(azetidin-1-yl)-2-methyl-6,7-dihydro-5H-pyrrolo[3,4-d]pyrimidine hydrochloride (220 mg, 0.97 mmol, synthesized with reference to the method described in the patent WO 2018066718) and N-methylimidazole (320 mg, 3.88 mmol) were successively dissolved in DMF (5 mL), chloro-N,N,N',N'-tetramethylformamidinium hexafluorophosphate (340 mg, 1.26 mmol) was added, and the mixture was reacted at room temperature for 1 hour. After the reaction was completed as monitored by TLC, the reaction solution was concentrated under reduced pressure, and the residue was purified by silica gel column chromatography (DCM : MeOH = 20 : 1) to obtain compound **62** (100 mg, yield: 23.19%).

¹H NMR (400 MHz, DMSO-d6) δ 8.40-8.38 (m, 1H), 7.95 (d, 1H), 6.71-6.69 (m, 1H), 6.29-6.27 (m, 1H), 6.26-6.24(m, 1H), 4.85-4.33 (m, 4H), 4.20-4.12 (m, 6H), 3.71-3.66 (m, 2H), 3.17-3.06 (m, 1H), 2.86-2.79 (m, 2H), 2.35 (s, 3H), 2.33-2.28 (m, 2H), 2.27 (s, 3H).

LC-MS(ESI):m/z = 445.2[M+H]⁺.

### Example 63:

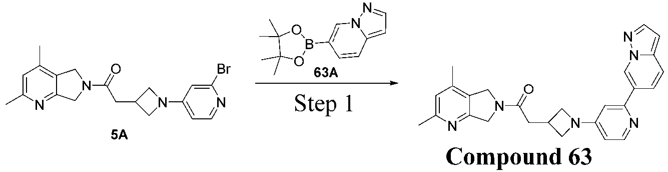

Compound **5A** (100 mg, 0.25 mmol) was dissolved in 1,4-dioxane (8 mL), and **63A** (80 mg, 0.33 mmol), potassium carbonate (86 mg, 0.62 mmol) and water (2 mL) were successively added. Under nitrogen atmosphere, 1,1-bis(diphenylphosphino)ferrocene palladium dichloride (27 mg, 0.04 mmol) was added and then the mixture was warmed to 85°C and stirred for 16 h. After the reaction was completed, the reaction mixture was cooled to room temperature and water (30 mL) was added. The resulting mixture was extracted with ethyl acetate (50 mL×3). The combined organic phases were dried over anhydrous sodium sulphate and concentrated. The resulting crude product was purified by column chromatography (dichloromethane/methanol = 95/5) to obtain a crude product (75 mg), which was further subjected to reverse-phase preparative chromatography (acetonitrile/water = 5/95 - 95/5) to obtain **compound 63** (53 mg, 48.50%).

LC-MS (ESI): m/z=439.7 [M+H]⁺.

¹H NMR (400 MHz, CDCl₃) δ 9.06 (s, 1H), 8.33 - 8.31 (m, 1H), 7.99 - 7.98 (m, 1H), 7.74-7.72 (m, 1H), 7.60-7.58 (m, 1H), 6.92-6.90 (m, 1H), 6.61 (s, 1H), 6.52 (s, 1H), 6.27-6.25 (m, 1H), 4.79 (s, 2H), 4.76-4.74 (m, 2H), 4.32-4.27 (m, 2H), 3.79-3.74 (m, 2H), 3.36-3.30 (m, 1H), 2.86-2.82 (m, 2H), 2.52 (s, 3H), 2.27 (s, 3H).

### Example 64:

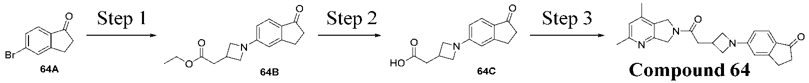

Step 1: **64A** (1.5 g, 7.09 mmol), **1D** (3.65 g, 14.18 mmol), Ruphos-Pd-G3 (0.59 g, 0.71 mmol) and caesium carbonate (6.93 g, 21.27 mmol) were successively added to dioxane (30 mL). Subsequently, under nitrogen atmosphere, the mixture was warmed to 100°C and reacted for 16 h. After the reaction was completed, the mixture was cooled to room temperature and filtered through diatomaceous earth. The filtrate was concentrated under reduced pressure and the residue was separated and purified by silica gel column chromatography (EA/PE = 1/3) to obtain **64B** (0.4 g, 20.4%).

LC-MS (ESI): m/z = 274.1[M+H]⁺.

Step 2: **64B** (0.4 g, 1.46 mmol) and lithium hydroxide monohydrate (0.30 g, 7.20 mmol) were added to mixed solvents of methanol (10 mL) and water (3 mL), and the mixture was reacted at room temperature for 3 h. After the reaction was completed, the reaction mixture was concentrated under reduced pressure to remove most of methanol, water (10 mL) was added, and the mixture was adjusted to pH = 4-5 by dropwise adding dilute hydrochloric acid, and extracted with ethyl acetate. The organic phase was washed with saturated aqueous sodium chloride solution and concentrated under reduced pressure to obtain crude product **64C** (0.35 g, 97.74%).

LC-MS (ESI): m/z = 246.1[M+H]⁺.

Step 3: **Intermediate 1** (0.24 g, 1.08 mmol), **64C** (0.26 g, 1.08 mmol), 2-(7-azobenzotriazole)-N,N,N',N'-tetramethyluronium hexafluorophosphate (0.62 g, 1.63 mmol), and N,N-diisopropylethylamine (0.56 g, 4.33 mmol) were successively added to N,N-dimethylformamide (5 mL), and the mixture was reacted at room temperature for 5 h. After the reaction was completed, water (30 mL) was added and the mixture was extracted with ethyl acetate (20 mL×3). The combined organic phases were washed with saturated aqueous sodium chloride solution, dried over anhydrous sodium sulphate, and filtered. The filtrate was concentrated under reduced pressure and the residue was purified using a C18 reverse-phase column (composition of mobile phases A and B: mobile phase A: acetonitrile; and mobile phase B: water (containing 0.1% aqueous ammonia), (A/B = 55%/45%)) to obtain **compound 64** (100 mg, 24.7%).

¹H NMR (400 MHz, DMSO-d6) δ 7.41 (d, 1H), 6.99 (s, 1H), 6.39-6.36 (m, 2H), 4.79 (d, 2H), 4.57 (d, 2H), 4.16-4.11 (m, 2H), 3.71-3.65 (m, 2H), 3.16-3.06 (m, 1H), 2.96-2.90 (m, 2H), 2.88-2.84 (m, 2H), 2.49-2.45 (m, 2H), 2.42 (s, 3H), 2.22 (d, 3H).

LC-MS (ESI): m/z = 376.2[M+H]⁺.

### Examples 65 and 66:

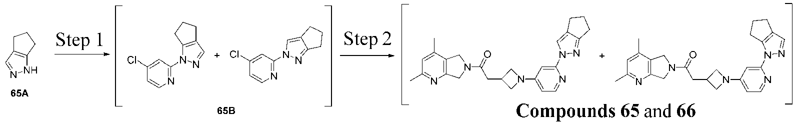

Step 1: Compound 4-chloro-2-fluoropyridine (200 mg, 1.52 mmol) was dissolved in DMSO (5 mL), and potassium carbonate (629 g, 4.56 mmol) and **65A** (197 mg, 1.82 mmol) were successively added. The mixture was stirred evenly and then warmed to 45°C and reacted for 16 hours. After the reaction was completed, the reaction solution was added dropwise to 15 mL of ice water, and then the mixture was extracted with dichloromethane (15 mL×2). The organic phases were combined and concentrated and the residue was separated by silica gel column chromatography (petroleum ether : ethyl acetate (v/v) = 20 : 1) to obtain **65B** (170 mg, 50.9%).

LCMS (ESI): m/z = 220.1 [M+H]⁺.

Step 2: **65B** (170 mg, 0.77 mmol), potassium carbonate (534 mg, 3.87 mmol), caesium fluoride (117 mg, 0.77 mmol) and **2C** (830 mg, 2.31 mmol) were successively dissolved in anhydrous DMF (5 mL), and then the mixture was warmed to 100°C and reacted for 16 hours. After the reaction was completed, the reaction solution was cooled to room temperature and added dropwise to 15 mL of ice water. Then the mixture was extracted with dichloromethane (15 mL×2). The organic phases were combined and concentrated, and the residue was separated by silica gel column chromatography (dichloromethane : methanol (v/v) = 15 : 1) and C18 column chromatography (0.1% aqueous NH₄HCO₃ solution : acetonitrile (v/v) = 3 : 7) to obtain **compound 65** (16 mg, 4.8%) **and compound 66** (5 mg, 1.5%).

**Compound 65:** LC-MS (ESI): m/z = 215.1 [(M+2H)/2]⁺.

¹H NMR (400 MHz, DMSO-*d*₆) δ 8.13 (s, 1H), 7.94-7.92 (m, 1H), 7.00 (s, 1H), 6.70-6.68 (m, 1H), 6.24-6.22 (m, 1H), 4.86 - 4.73 (m, 2H), 4.64 - 4.50 (m, 2H), 4.15 (t, 2H), 3.72-3.66 (m, 2H), 3.18 -3.05 (m, 1H), 2.88-2.83 (m, 2H), 2.69-2.60 (m, 4H), 2.42 (s, 3H), 2.40 - 2.31 (m, 2H), 2.24-2.21 (m, 3H).

**Compound 66:** LC-MS (ESI): m/z = 215.1 [(M+2H)/2]⁺.

¹H NMR (400 MHz, DMSO-*d*₆) δ 7.97-7.95 (m, 1H), 7.38 (s, 1H), 6.99 (s, 1H), 6.74-6.72 (m, 1H), 6.26-6.24 (m, 1H), 4.86 - 4.73 (m, 2H), 4.64 - 4.50 (m, 2H), 4.15 (t, 2H), 3.72-3.66 (m, 2H), 3.18 -3.00 (m, 3H), 2.88-2.83 (m, 2H), 2.58-2.45 (m, 4H), 2.42 (s, 3H), 2.24-2.21 (m, 3H).

### Example 67:

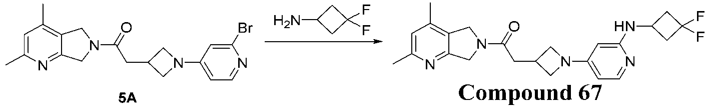

At room temperature, **5A** (100 mg, 0.25 mmol) was dissolved in toluene (5 mL), and then 3,3-difluorocyclobutan-1-amine (32 mg, 0.30 mmol), Pd₂(dba)₃ (46 mg, 0.05 mmol), BINAP (46 mg, 0.075 mmol), and sodium tert-butoxide (72 mg, 0.75 mmol) were successively added. Subsequently, under nitrogen atmosphere, the mixture was warmed to 100°C and reacted for 16 hours. Upon complete depletion of starting materials as monitored by TLC, the reaction was stopped. The reaction solution was cooled to room temperature. To the reaction solution was added EA (15 mL). The organic phase was washed with saturated aqueous sodium chloride solution (20 mL×2), dried over anhydrous sodium sulphate and filtered. The filtrate was concentrated and the residue was separated and purified by silica gel column chromatography to obtain the target **compound 67** (20 mg, 18.71%).

LC-MS (ESI): m/z = 428.1 [M+H]⁺.

¹H NMR (400 MHz, CDCl₃) δ 7.55 (d, 1H), 6.92-6.91 (d, 1H), 5.77-5.75 (d, 1H), 5.02 (s, 1H), 4.78-4.72 (m, 4H), 4.30-4.26 (t, 2H), 3.80-3.74 (m, 3H), 3.33-3.26 (m, 1H), 3.06-295 (m, 2H), 2.83-2.79 (t, 2H), 2.62-2.60 (d, 2H), 2.53-2.52 (d, 3H), 2.27-2.26 (d, 3H).

### Example 68:

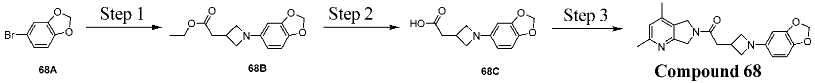

Step 1: Ethyl 2-(azetidin-3-yl)acetate (1 g, 6.98 mmol), **68A** (2.81 g, 13.96 mmol), caesium carbonate (4.55 g, 13.96 mmol), and methanesulphonato(2-dicyclohexylphosphino-2',6'-di-isopropoxy-1,1'-biphenyl)(2'-amino-1,1'-biphenyl-2-yl)palladium (II) (0.58 g, 0.70 mmol) were successively dissolved in 1,4-dioxane (25 mL). Subsequently, under nitrogen atmosphere, the mixture was warmed to 100°C and reacted for 12 hours. After the reaction was completed, the reaction mixture was cooled to room temperature and concentrated under reduced pressure. The residue was separated and purified by column chromatography (petroleum ether : ethyl acetate = 3 : 1) to obtain product **68B** (0.8 g, 43%).

LC-MS (ESI): m/z = 264.1[M+H]⁺.

Step 2: **68B** (0.3 g, 1.14 mmol) was dissolved in mixed solvents of water (2 mL), tetrahydrofuran (2 mL) and methanol (2 mL). Lithium hydroxide (0.082 g, 3.42 mmol) was added and subsequently, the mixture was reacted at room temperature for 2 hours. After the reaction was completed, the reaction solution was concentrated to obtain crude product **68C** (0.2 g).

LC-MS (ESI): m/z = 236.2 [M+H]+.

Step 3: **68C** (0.1 g, 0.43 mmol) and 2,4-dimethyl-5H,6H,7H-pyrrolo[3,4-B]pyridine (0.076 g, 0.52 mmol) were dissolved in acetonitrile (6 mL). Then 1-methylimidazole hydrochloride (0.076 g, 0.65 mmol) and [chloro(dimethylamino)methylene]-dimethylazane hexafluorophosphate (0.16 g, 0.56 mmol) were successively added and the mixture was reacted at room temperature for 1 hour. After the reaction was completed, the mixture was purified by preparative HPLC to obtain **compound 68** (50 mg, 32%). Preparation method: Instrument: waters 2767 preparative liquid chromatographic instrument; chromatographic column: SunFire@ Prep C18 (19 mm×150 mm); composition of mobile phases: mobile phase A: acetonitrile; and mobile phase B: water (containing 0.1% trifluoroacetic acid); gradient: 20%-40% acetonitrile gradient.

LC-MS (ESI): m/z = 366.4 [M+H]⁺.

¹H NMR (400 MHz, DMSO-*d*₆) δ 6.99 (d, 1H), 6.72 (d, 1H), 6.12 (d, 1H), 5.86 (s, 2H), 5.83-5.79 (m, 1H), 4.79 (d, 2H), 4.56 (d, 2H), 3.89 (t, 2H), 3.41 (t, 2H), 3.04-2.93(m, 1H), 2.84-2.74 (m, 2H), 2.42 (s, 3H), 2.22 (d, 3H).

### Example 69:

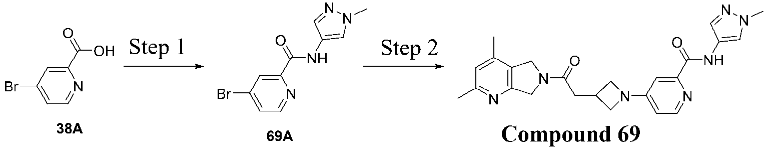

Step 1: With reference to the operation process of step 1 in Example 38, **compound 69A** (0.20 g, yield: 47%) was obtained using compound **38A** (0.3 g, 1.50 mmol) as a starting material.

LC-MS (ESI): m/z = 281.2 [M+H]⁺.

Step 2: With reference to the operation process of step 2 in Example 25, **compound 69** (30 mg, yield: 19%) was obtained using compound **69A** (0.10 g, 0.35 mmol) as a starting material.

LC-MS (ESI): m/z = 446.2 [M+H]⁺.

¹H NMR (400 MHz, CDCl₃): δ 9.89 (s, 1H), 8.16-8.15 (m, 1H), 8.05-8.04 (m, 1H), 7.57-7.56 (m, 1H), 7.23-7.22 (m, 1H), 6.91-6.90 (m, 1H), 6.34-6.32 (m, 1H), 4.79-4.73 (m, 4H), 4.31-4.26 (m, 2H), 3.90 (s, 3H), 3.79-3.74 (m, 2H), 3.33-3.28 (m, 1H), 2.84-2.80 (m, 2H), 2.53-2.52 (m, 3H), 2.27 -2.26 (m, 3H).

### Example 70:

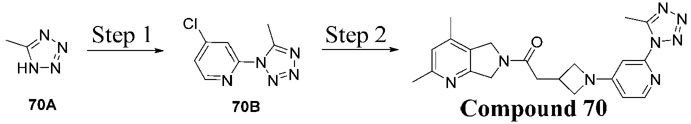

Step 1: At room temperature, **70A** (420 mg, 4.95 mmol) was dissolved in DMSO (10 mL), and 4-chloro-2-fluoropyridine (650 mg, 4.95 mmol) and caesium carbonate (4.84 g, 14.85 mmol) were added. The mixture was reacted at 100°C for 16 hours. Upon complete depletion of starting materials as monitored by TLC, the reaction was stopped. The reaction solution was cooled to room temperature. To the reaction solution was added EA (30 mL), and the organic phase was washed with water (30 mL×3) and saturated aqueous sodium chloride solution (30 mL×2), dried over anhydrous sodium sulphate and filtered. The filtrate was concentrated and the residue was separated and purified by column chromatography (PE : EA = 10 : 1) to obtain compound **70B** (190 mg, 19.62%).

LC-MS (ESI): m/z = 196.1 [M+H]⁺.

Step 2: With reference to the synthesis method of step 2 in **Example 28,** compound **70** (100 mg, 25.49%) was synthesized using **70B** (190 mg, 0.97 mmol) as a starting material.

LC-MS (ESI): m/z = 405.3 [M+H]⁺.

¹H NMR (400 MHz, CDCl₃) δ 8.12-8.10 (d, 1H), 6.95-6.91 (d, 1H), 6.83 (d, 1H), 6.32-6.30 (m, 1H), 4.82-4.80 (d, 2H), 4.76-4.74 (d, 2H), 4.33-4.29 (m, 2H), 3.82-3.78 (m, 2H), 3.38-3.30 (m, 1H), 2.89 (s, 3H), 2.85-2.81 (t, 2H), 2.55-2.54 (d, 3H), 2.29-2.27 (d, 3H).

### Example 71:

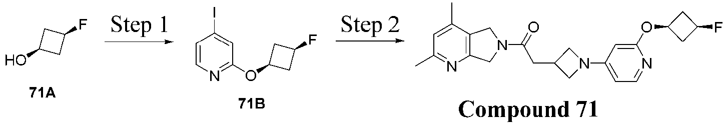

Step 1: With reference to step 1 in Example 25, **compound 71B** (131 mg, yield: 26.93%) was obtained using **71A** (150 mg, 1.66 mmol) and 2-fluoro-4-iodopyridine (407 mg, 1.83 mmol) as starting materials.

LC-MS (ESI): m/z = 294.0 [M+H]⁺.

Step 2: With reference to step 2 in Example 25, **compound 71** (46 mg, yield: 25.47%) was obtained using **71B** (130 mg, 0.44 mmol) as a starting material.

LC-MS (ESI): m/z = 411.5 [M+H]⁺.

¹H NMR (400 MHz, DMSO-d6) δ 7.73-7.69 (m, 1H), 7.01-6.98 (m, 1H), 6.08-6.02 (m, 1H), 5.66-5.61 (m, 1H), 4.98-4.88 (m, 1H), 4.84-4.77 (m, 2H), 4.76-4.65 (m, 2H), 4.64-4.50 (m, 4H), 4.07-4.01 (m, 2H), 3.61-3.55 (m, 2H), 3.13-3.03 (m, 1H), 2.85-2.79 (m, 2H), 2.42 (s, 3H), 2.29- 2.27 (m, 1H), 2.23-2.21 (m, 3H).

### Example 72:

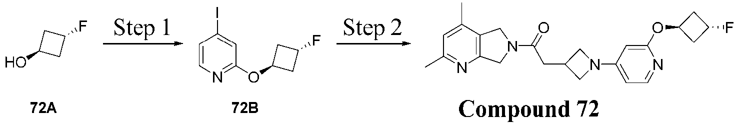

Step 1: With reference to step 1 in Example 25, **compound 72B** (153 mg, yield: 31.45%) was obtained using **72A** (150 mg, 1.66 mmol) and 2-fluoro-4-iodopyridine (407 mg, 1.83 mmol) as starting materials.

LC-MS (ESI): m/z = 294.0 [M+H]⁺.

Step 2: With reference to step 2 in Example 25, **compound 72** (24 mg, yield: 11.46%) was obtained using **compound 72B** (150 mg, 0.51 mmol) as a starting material.

LC-MS (ESI): m/z = 411.5 [M+H]⁺.

¹H NMR (400 MHz, DMSO-*d6*) δ 7.75-7.71 (m, 1H), 7.01-6.99 (m, 1H), 6.06-6.04 (m, 1H), 5.63-5.60 (m, 1H), 5.41-5.33 (m, 1H), 5.33-5.19 (m, 2H), 4.83-4.74 (m, 2H), 4.62-4.47 (m, 4H), 4.07-4.01 (m, 2H), 3.61-3.55 (m, 2H), 3.10-3.04 (m, 1H), 2.86-2.79 (m, 2H), 2.42 (s, 3H), 2.28- 2.27 (m, 1H), 2.24-2.21 (m, 3H).

### Example 73:

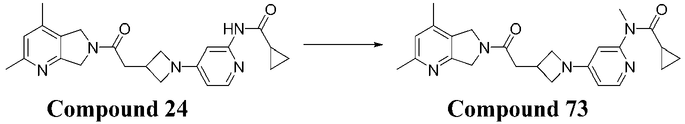

Compound **24** (81 mg, 0.2 mmol) was dissolved in DMF (20 mL), and then sodium hydride (12 mg, 0.3 mmol) was added. The mixture was stirred for half an hour and then iodomethane (34 mg, 0.24 mmol) was added. The resulting mixture was reacted at room temperature for 2 hours. After the reaction was completed, the reaction was quenched with saturated aqueous sodium chloride solution (20 mL), and the reaction solution was extracted with dichloromethane (80 mL × 2). The combined organic phases were dried over anhydrous sodium sulphate and concentrated under reduced pressure. The residue was separated by silica gel column chromatography (dichloromethane : methanol (v/v) = 5 : 1) to obtain compound **73** (40 mg, yield: 48%).

LC-MS (ESI): m/z = 420.4 [M+H]⁺.

¹H NMR (400 MHz, CDCl₃): δ 8.14-8.13 (m, 1H), 6.92-6.90 (m, 1H), 6.21 (s, 1H), 6.19- 6.16 (m, 1H), 4.80-4.75 (m, 2H), 4.74-4.71 (m, 2H), 4.27-4.20 (m, 2H), 3.76-3.69 (m, 2H), 3.34 (s, 3H), 3.32-3.26 (m, 1H), 2.85-2.77 (m, 2H), 2.55-2.50 (m, 3H), 2.29-2.24 (m, 3H), 1.66-1.58 (m, 1H), 1.10-1.02 (m, 2H), 0.73-0.65 (m, 2H).

### Example 74:

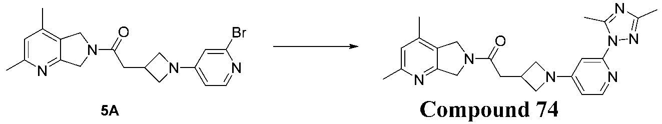

With reference to Example 50, compound **74** (55 mg, yield: 26.43%) was obtained using compound **5A** (200 mg, 0.5 mmol) and 3,5-dimethyl-1,2,4-triazole (97 mg, 1.0 mmol) as starting materials.

LC-MS (ESI): m/z = 418.4[M+H]⁺.

¹H NMR (400 MHz, CDCl₃) δ 8.06 (s, 1H), 6.95 (d, 1H), 6.70 (s, 1H), 6.22 (d, 1H), 4.84-4.74 (m, 4H), 4.31-4.26 (m, 2H), 3.78-3.74 (m, 2H), 3.34-3.26 (m, 1H), 2.82-2.79 (m, 5H), 2.56 (d, 3H), 2.41 (s, 3H), 2.28 (d, 3H).

### Example 75:

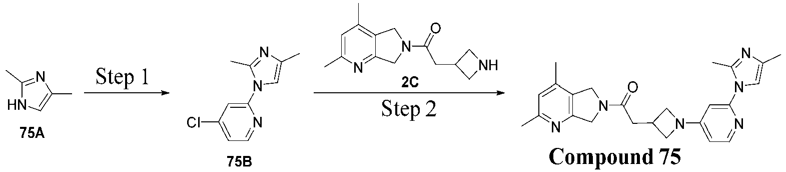

Step 1: With reference to the procedures of step 1 in Example 26, **75B** (210 mg, 21%) was synthesized using **75A** (500 mg, 5.20 mmol) as a starting material.

LC-MS (ESI): m/z = 208.2 [M+H]⁺.

Step 2: With reference to the procedures of step 2 in Example 26, **compound 75** (51 mg, 12%) was synthesized using **75B** (210 mg, 1.01 mmol) as a starting material.

¹H NMR (400 MHz, CDCl₃) δ 8.12-8.10 (m, 1H), 6.94-6.88 (m, 2H), 6.25-6.22 (m, 1H), 6.14-6.12 (m, 1H), 4.79-4.72 (m, 4H), 4.29-4.23 (m, 2H), 3.78-3.72 (m, 2H), 3.38-3.27 (m, 1H), 2.85-2.80 (m, 2H), 2.58-2.49 (m, 6H), 2.30-2.21 (m, 6H).

LC-MS (ESI): m/z = 417.6 [M+H]⁺.

### Example 76:

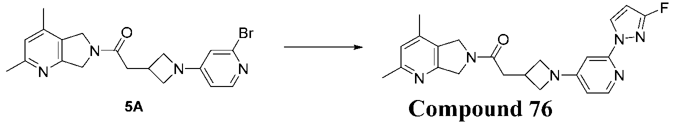

**5A** (400 mg, 1.00 mmol), 3-fluoro-1H-pyrazole (95 mg, 1.10 mmol), caesium carbonate (814 mg, 2.50 mmol), cuprous iodide (57 mg, 0.30 mmol) and L-proline (12 mg, 0.10 mmol) were successively dissolved in DMSO (15 mL), and under nitrogen atmosphere, the mixture was stirred and reacted at 100°C overnight. After the reaction was completed as monitored by TLC, the reaction solution was cooled to room temperature. The reaction was quenched with water (20 mL) and the resulting mixture was extracted with EA (20 mL × 3). The combined organic phases were washed with saturated aqueous sodium chloride solution, dried over anhydrous sodium sulphate and concentrated. The resulting crude product was separated and purified by silica gel column chromatography (dichloromethane : methanol (v/v) = 10 : 1) to obtain the target **compound 76** (135 mg, yield: 33.21%).

LC-MS (ESI): m/z = 407.6 [M+H]⁺.

¹ H NMR (400 MHz, DMSO-d6) δ 8.48-8.46 (m, 1H), 7.99-7.95 (m, 1H), 7.01-6.98 (m, 1H), 6.60-6.57 (m, 1H), 6.33-6.28 (m, 2H), 4.83-4.75 (m, 2H), 4.61-4.53 (m, 2H), 4.17 (t, 2H), 3.74-3.67 (m, 2H), 3.18-3.10 (m, 1H), 2.89-2.83 (m, 2H), 2.44-2.40 (m, 3H), 2.24-2.21 (m, 3H).

### Example 77:

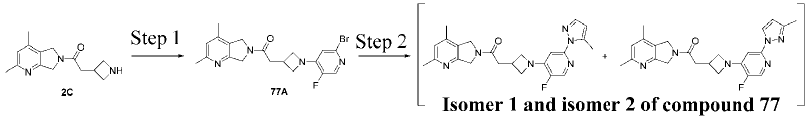

Step 1: **2C** (1.0 g, 4.08 mmol), 2-bromo-5-fluoro-4-iodopyridine (1.23 g, 4.08 mmol) and potassium carbonate (1.69 g, 12.24 mmol) were successively dissolved in dimethyl sulphoxide (20 mL), and then the mixture was warmed to 150°C and reacted for 3 h. After the reaction was completed, the reaction solution was cooled to room temperature, and water (60 mL) was added to the reaction solution. The mixture was extracted with ethyl acetate (25 mL×3). The organic layers were combined, washed with saturated aqueous sodium chloride solution (30 mL), dried over anhydrous sodium sulphate and filtered. The filtrate was concentrated and the residue was separated and purified by silica gel column chromatography (dichloromethane : methanol (v/v) = 30 : 1) to obtain **77A** (683 mg, 40%).

LC-MS (ESI): m/z = 419.3 [M+H]⁺.

Step 2: **77A** (200 mg, 0.48 mmol), 3-methylpyrazole (47 mg, 0.57 mmol), cuprous iodide (19 mg, 0.1 mmol), L-proline (22 mg, 0.19 mmol) and caesium carbonate (469 mg, 1.44 mmol) were successively dissolved in super-dry dimethyl sulphoxide (10 mL), and the mixture was reacted at 150°C under microwave under nitrogen atmosphere for 3 h. After the reaction was completed, the reaction solution was cooled to room temperature, and water (30 mL) was added to the reaction solution. The mixture was extracted with ethyl acetate (15 mL×3). The organic layers were combined, washed with saturated aqueous sodium chloride solution (30 mL), dried over anhydrous sodium sulphate and filtered. The filtrate was concentrated to obtain a crude product. The crude product was purified by preparative high-performance liquid chromatography to obtain **compound 77,** isomer 1 (10 mg, retention time: 2.90 min, yield: 5%) and **compound 77,** isomer 2 (50 mg, retention time: 3.03 min, yield: 24.75%).

HPLC analytical method: 1. Instrument: Shimadzu LC-20AT; 2. chromatographic column: Xtimate C18 4.6*50 mm, 3 µm; 3. Mobile phase A: 0.05% TFA solution, mobile phase B: acetonitrile; 4. Gradient: A 95-5% B 5-95%; 5. Flow rate: 1 mL/min, column temperature: 35°C, wavelength: 210 nm/254 nm, acquisition time: 10 min.

Preparation method: Instrument: waters 2767 preparative liquid chromatographic instrument; chromatographic column: SunFire@ Prep C18 (19 mm × 250 mm). The sample was dissolved in methanol and filtered through a 0.22 µm filter head to prepare a sample solution. Preparative chromatography conditions: composition of mobile phases A and B: mobile phase A: acetonitrile, mobile phase B: water (containing 1% ammonium acetate), gradient elution, mobile phase A: 10%-40%, flow rate: 12 mL/min. Elution time: 25 min.

**Compound 77,** isomer 1: LC-MS (ESI): m/z = 421.4 [M+H]⁺.

¹H NMR (400 MHz, Methanol-*d*₄) δ 7.94 (d, 1H), 7.52 (d, 1H), 7.06 (s, 1H), 6.66 (d, 1H), 6.22 (s, 1H), 4.87 (d, 2H), 4.70 (d, 2H), 4.42-4.40 (m, 2H), 3.96-3.94 (m, 2H), 3.27-3.20 (m, 1H), 2.95-2.93 (m, 2H), 2.55-2.42 (m, 6H), 2.31 (d, 3H).

**Compound 77,** isomer 2: LC-MS (ESI): m/z = 421.4 [M+H]⁺.

¹H NMR (400 MHz, Methanol-*d*₄) δ 8.28 (d, 1H), 7.85 (d, 1H), 7.06 (s, 1H), 6.85 (d, 1H), 6.27 (d, 1H), 4.87 (d, 2H), 4.71 (d, 2H), 4.42-4.40 (m, 2H), 3.97-3.94 (m, 2H), 3.27-3.19 (m, 1H), 2.95-2.92 (m, 2H), 2.49 (s, 3H), 2.32 (d, 6H).

### Example 78:

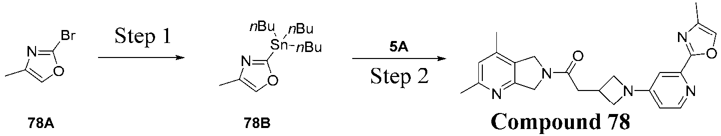

Step 1: With reference to the synthesis method of step 1 in Example 44, compound **78B** (100 mg, 83%) was obtained using **78A** (121 mg, 0.75 mmol) as a starting material.

Step 2: **78B** (100 mg, 0.62 mmol), **5A** (100 mg, 0.25 mmol), Pd(PPh₃)₄ (28 mg, 0.02 mmol) and cuprous iodide (4.7 mg, 0.02 mmol) were successively dissolved in 1,4-dioxane (10 mL), and under nitrogen atmosphere, the mixture was warmed to 100°C and reacted overnight. After the reaction was completed, the reaction solution was cooled to room temperature, water (30 mL) was added to the reaction solution, and the resulting mixture was extracted with ethyl acetate (20 mL×3). The organic phases were combined, washed with saturated aqueous sodium chloride solution (30 mL), dried over anhydrous sodium sulphate, and filtered. The filtrate was concentrated and the resulting residue was separated and purified by silica gel column chromatography (dichloromethane : methanol (v/v) = 25 : 1) to obtain **compound 78** (26 mg, 26%).

¹H NMR (400 MHz, CDCl₃) δ 8.31-8.30 (m, 1H), 7.49 (s, 1H), 7.06 (s, 1H), 6.91-6.90 (m, 1H), 6.29-6.28 (m, 1H), 4.78-4.73 (m, 4H), 4.29 (t, 2H), 3.80-3.74 (m, 2H), 3.35-3.27 (m, 1H), 2.81 (t, 2H), 2.53-2.52 (m, 3H), 2.27-2.26 (m, 6H).

LC-MS (ESI): m/z = 404.4 [M+H]⁺.

### Example 79:

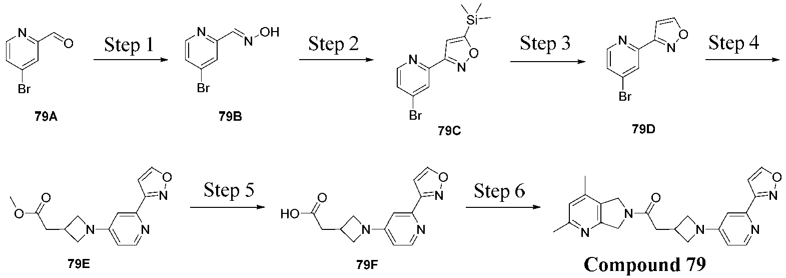

Step 1: Compound **79A** (1.0 g, 5.42 mmol), hydroxylamine hydrochloride (0.56 g, 8.13 mmol), and sodium carbonate (1.44 g, 13.55 mmol) were successively dissolved in mixed solvents of methanol (15 mL) and water (10 mL), and then the mixture was stirred and reacted at room temperature for 1 hour. The reaction solution was concentrated under reduced pressure to partially remove methanol, and water (10 mL) was added to precipitate a solid. The mixture was filtered, and the filter cake was dried to obtain compound **79B** (1.05 g, yield: 99%).

LC-MS (ESI): m/z= 201.1 [M+H]⁺.

Step 2: Compound **79B** (0.60 g, 2.98 mmol) and NBS (0.63 g, 3.54 mmol) were successively dissolved in DMF (12 mL), and under nitrogen atmosphere, the mixture was warmed to 60°C, stirred and reacted for 2 hours. After the reaction was completed, the mixture was cooled to 0°C, and triethylamine (10 mL) was added. Trimethylsilylacetylene (0.44 g, 4.47 mmol) was slowly added dropwise. After the dropwise addition was completed, the mixture was stirred and reacted for another 2 hours. The reaction solution was poured into water, and the resulting mixture was extracted with ethyl acetate three times. The organic phases were combined, washed with saturated aqueous sodium chloride solution, dried over anhydrous sodium sulphate, and concentrated under reduced pressure to obtain a crude product. The crude product was purified using Biotage Isolera One (12 g silica gel column, eluents: 0-10% EA/PE) to obtain compound **79C** (0.21 g, yield: 23.71%).

LC-MS (ESI): m/z = 297.2 [M+H]⁺.

Step 3: **79C** (0.21 g, 0.71 mmol) and potassium carbonate (0.29 g, 2.10 mmol) were successively dissolved in methanol (10 mL), and the mixture was stirred and reacted at room temperature for 1 hour. The reaction solution was filtered and the filtrate was concentrated under reduced pressure to obtain a crude product. The crude product was purified using Biotage Isolera One (12 g silica gel column, eluents: 0-20% EA/PE) to obtain compound **79D** (0.13 g, yield: 81.36%).

LC-MS (ESI): m/z = 226.1 [M+H]⁺.

Step 4: With reference to the synthesis method of step 4 in Example 12, compound **79E** (0.12 g, 75.71%) was obtained using **79D** (0.13 g, 0.58 mmol) as a starting material.

LC-MS (ESI): m/z = 274.2 [M+H]⁺.

Step 5: With reference to the synthesis method of step 5 in Example 12, compound **79F** (0.10 g, 87.66%) was synthesized using **79E** (0.12 g, 3.13 mmol) as a starting material.

LC-MS (ESI): m/z = 260.1 [M+H]⁺.

Step 6: With reference to the synthesis method of step 6 in Example 12, compound **79** (40 mg, 26.34%) was synthesized using **79F** (0.10 g, 0.39 mmol) as a starting material.

LC-MS (ESI): m/z = 390.5 [M+H]⁺.

¹H NMR (400 MHz, CDCl₃): δ 8.47 (s, 1H), 8.28-8.27 (m, 1H), 7.25-7.24 (m, 1H), 7.09-7.08 (m, 1H), 6.91-6.90 (m, 1H), 6.34-6.31 (m, 1H), 4.78-4.73 (m, 4H), 4.33-4.31 (m, 2H), 3.82-3.80 (m, 2H), 3.35-3.32 (m, 1H), 2.85-2.80 (m, 2H), 2.53-2.52 (m, 3H), 2.27-2.26 (m, 3H).

### Example 80:

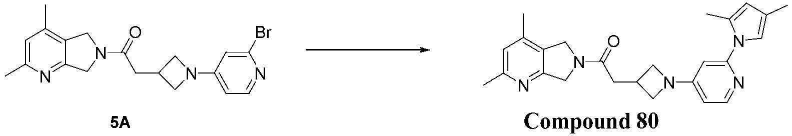

With reference to Example 50, compound **80** (48 mg, yield: 23.10%) was synthesized using compound **5A** (200 mg, 0.5 mmol) and 2,4-dimethylpyrrole (95 mg, 1.0 mmol) as starting materials.

LC-MS (ESI): m/z = 416.3[M+H]⁺.

¹H NMR (400 MHz, CDCl₃) δ 8.09 (d, 1H), 6.91 (d, 1H), 6.79 (d, 1H), 6.16-6.14 (m, 2H), 5.86 (s, 1H), 4.78-4.73 (m, 4H), 4.25-4.21 (m, 2H), 3.74-3.70 (m, 2H), 3.30-3.25 (m, 1H), 2.83-2.79 (t, 2H), 2.52 (d, 3H), 2.35 (d, 3H), 2.27 (d, 3H), 2.08 (d, 3H).

### Example 81:

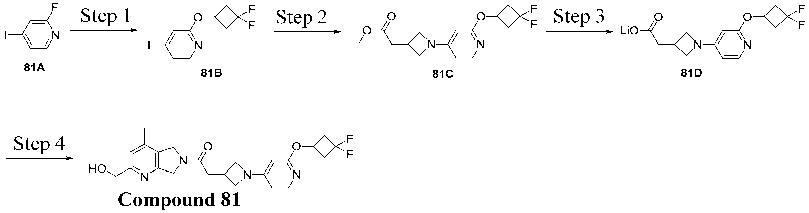

Step 1: Compound 3,3-difluorocyclobutanol (5.26 g, 48.63 mmol) was dissolved in tetrahydrofuran (80 mL), and the mixture was cooled to 0°C. Then NaH (2.07 g, 51.87 mmol) was added. The resulting mixture was stirred for 30 min. Compound **81A** (7.23 g, 32.42 mmol) was added, and then the mixture was naturally warmed to room temperature and reacted for 4 hours. After the reaction was completed as monitored by TLC, the reaction was quenched with saturated aqueous ammonium chloride solution (100 mL), and the mixture was extracted with ethyl acetate (150 mL×2). The organic phases were combined, dried over anhydrous sodium sulphate, and concentrated under reduced pressure. The residue was purified by silica gel column chromatography (petroleum ether : ethyl acetate (v : v) = 3 : 1) to obtain compound **81B** (9.01 g, yield: 89.11%).

LC-MS(ESI):m/z = 312.1[M+H]⁺.

Step 2: **81B** (6.0 g, 19.29 mmol), methyl 3-azetidine acetate (4.98 g, 38.57 mmol), caesium carbonate (18.86 g, 57.87 mmol) and Ruphos Pd G3 (1.68 g, 1.93 mmol) were successively dissolved in toluene (100 mL), and under nitrogen atmosphere, the mixture was warmed to 90°C and reacted for 4 hours. After the reaction was completed as monitored by TLC, the reaction solution was cooled to room temperature. Saturated aqueous ammonium chloride solution (100 mL) was added, and the resulting mixture was extracted with ethyl acetate (150 mL×2). The organic phases were combined, dried over anhydrous sodium sulphate, and concentrated under reduced pressure. The residue was purified by silica gel column chromatography (petroleum ether : ethyl acetate (v : v) = 1 : 1) to obtain compound **81C** (2.4 g, yield: 39.88%).

LC-MS(ESI):m/z = 313.2[M+H]⁺.

Step 3: **81C** (2.30 g, 7.36 mmol) was dissolved in mixed solvents (tetrahydrofuran : methanol : water = 3 : 1 : 1 (30 mL)). Lithium hydroxide (0.26 g, 11.04 mmol) was added, and the resulting mixture was reacted at room temperature for 4 hours. After the reaction was completed as monitored by TLC, the mixture was concentrated under reduced pressure to obtain crude compound **81D** (2.50 g).

LC-MS(ESI):m/z = 305.2[M+H]⁺.

Step 4: Compound (4-methyl-6,7-dihydro-5H-pyrrolo[3,4-b]pyridin-2-yl)methanol (synthesized with reference to the method described in the patent WO 2018002760) (200 mg, 1.22 mmol), compound **81D** (370 mg, 1.22 mmol) and N-methylimidazole (0.4 mL, 4.88 mmol) were successively dissolved in N,N-dimethylformamide (10 mL). Subsequently, chloro-N,N,N',N'-tetramethylformamidinium hexafluorophosphate (410 mg, 1.46 mmol) was added and the resulting mixture was reacted at room temperature for 1 hour. After the reaction was completed as monitored by TLC, the mixture was concentrated under reduced pressure. The residue was purified by silica gel column chromatography (dichloromethane : methanol (v : v) = 20 : 1) to obtain **compound 81** (56 mg, yield: 10.33%).

¹H NMR (400 MHz, DMSO-d6) δ 7.76-7.70 (m, 1H), 7.20 (s, 1H), 6.11-6.03 (m, 1H), 5.67 (s, 1H), 5.44-5.31 (m, 1H), 5.10-4.98 (m, 1H), 4.88-4.45 (m, 6H), 4.10-4.00 (m, 2H), 3.64-3.53 (m, 2H), 3.15-3.00 (m, 3H), 2.88-2.78 (m, 2H), 2.70-2.54 (m, 2H), 2.28 (s, 3H).

LC-MS(ESI):m/z = 445.8[M+H]⁺.

### Example 82:

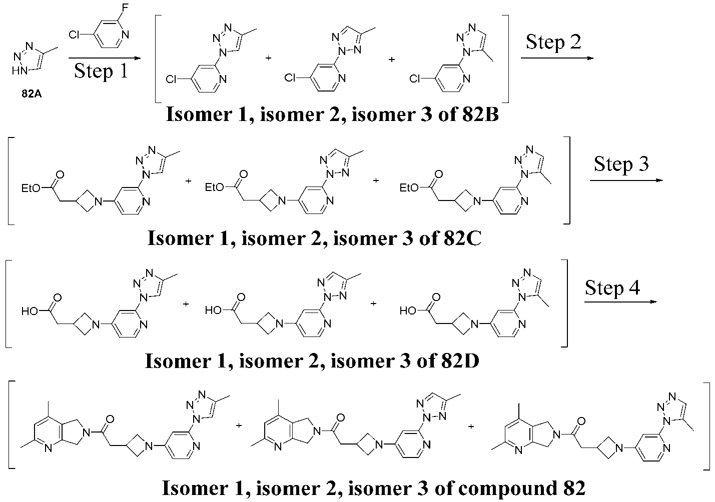

Step 1: With reference to the synthesis method of step 1 in Example **26,** compound **82B** (0.21 g, 66.36%) was synthesized as a mixture of three isomers using **82A** (0.20 g, 2.40 mmol) and 2-fluoro-4-chloropyridine (0.38 g, 2.83 mmol) as starting materials.

LC-MS (ESI): m/z = 195.1 [M+H]⁺.

Step 2: With reference to the synthesis method of step 1 in Example 17, compound **82C** (0.13 g, 69.97%) was synthesized as a mixture of three isomers using **82B** (0.12 g, 0.62 mmol) as a starting material.

LC-MS (ESI): m/z = 302.2 [M+H]⁺.

Step 3: With reference to the synthesis method of step 2 in Example 17, compound **82D** (0.10 g, 99.99%) was synthesized as a mixture of three isomers using **82C** (0.11 g, 0.35 mmol) as a starting material.

LC-MS (ESI): m/z = 274.2 [M+H]⁺.

Step 4: With reference to the synthesis method of step 3 in Example 17, **compound 82** (containing three isomers) was obtained using **82D** (0.12 g, 0.44 mmol) as a starting material. The product was purified by preparative high-performance liquid chromatography to obtain three isomers: **compound 82,** isomer 1 (35 mg, 19.76%), **compound 82,** isomer 2 (25 mg, 14.11%), and **compound 82,** isomer 3 (10 mg, 5.64%), respectively.

Preparation method: Instrument: waters 2767 preparative liquid chromatographic instrument; chromatographic column: SunFire@ Prep C18 (19 mm × 250 mm). The sample was dissolved in DMF and filtered with a 0.45 µm filter to prepare a sample solution. Preparative chromatography conditions: composition of mobile phases A and B: mobile phase A: acetonitrile, mobile phase B: water (containing 1% TFA), gradient elution, mobile phase A: 10% to 70%, flow rate: 12 mL/min. Elution time: 20 min.

HPLC analytical method: Instrument: Shimadzu LC-20AT, Instrument code: CH-Y-J0460; chromatographic column: brand: Welch, chromatographic column model: Xtimate C18 4.6*50 mm, 3 µm. Chromatography conditions: composition of mobile phases A and B: mobile phase A: 0.05% TFA solution; mobile phase B: acetonitrile, gradient elution, mobile phase A: 95%-5%-95%. Elution time: 10 min.

**Compound 82,** isomer 1: retention time: 2.403 min.

LC-MS (ESI): m/z = 404.1 [M+H]⁺.

¹H NMR (400 MHz, CDCl₃): δ 8.18-8.17 (m, 1H), 7.60 (s, 1H), 6.93-6.91 (m, 2H), 6.25-6.23 (m, 1H), 4.79-4.74 (m, 4H), 4.31-4.29 (m, 2H), 3.81-3.78 (m, 2H), 3.35-3.30 (m, 1H), 2.84-2.80 (m, 2H), 2.54 (s, 3H), 2.44-2.43 (m, 3H), 2.28-2.26 (m, 3H).

**Compound 82,** isomer 2: retention time: 2.428 min.

LC-MS (ESI): m/z = 404.1 [M+H]⁺.

¹H NMR (400 MHz, CDCl₃): δ 8.28 (s, 1H), 8.06-8.05 (m, 1H), 7.08-7.07 (m, 1H), 6.97-6.91 (m, 1H), 6.26-6.23 (m, 1H), 4.84-4.74 (m, 4H), 4.34-4.29 (m, 2H), 3.82-3.78 (m, 2H), 3.35-3.29 (m, 1H), 2.85-2.81 (m, 2H), 2.57-2.54 (m, 3H), 2.42 (m, 3H), 2.27-2.26 (m, 3H).

**Compound 82,** isomer 3:
LC-MS (ESI): m/z = 404.2 [M+H]⁺.

HPLC retention time: 2.284 min.

¹H NMR (400 MHz, CDCl₃): δ 8.13-8.10 (m, 1H), 7.52-7.51 (m, 1H), 6.96-6.91 (m, 1H), 6.87 (s, 1H), 6.30-6.28 (m, 1H), 4.83-4.74 (m, 4H), 4.32-4.27 (m, 2H), 3.80-3.75 (m, 2H), 3.35-3.30 (m, 1H), 2.84-2.80 (m, 2H), 2.61 (s, 3H), 2.57-2.54 (m, 3H), 2.30-2.27 (m, 3H).

### Example 83:

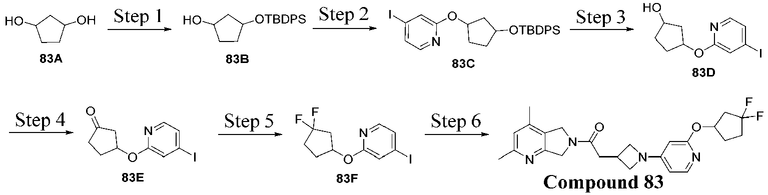

Step 1: At room temperature, **83A** (3.0 g, 29.35 mmol) was dissolved in DMF (10 mL), and imidazole (2.0 g, 29.35 mmol) was added. Tert-butylchlorodiphenylsilane (6.46 g, 23.49 mmol) was added in portions, and then the reaction was continued for 1 hour. After the reaction was completed as monitored by TLC, the reaction was stopped. To the reaction solution was added EA (30 mL), and the organic phase was washed with water (30 mL×3) and saturated aqueous sodium chloride solution (30 mL×2), dried over anhydrous sodium sulphate and filtered. The filtrate was concentrated and the residue was separated and purified by column chromatography (PE : EA = 4 : 1) to obtain compound **83B** (4.0 g, 40.02%).

LC-MS (ESI): m/z = 341.3 [M+H]⁺.

Step 2: With reference to the synthesis route of step 1 in Example 25, compound **83C** (1.5 g, 93.87%) was obtained using compound **83B** (1.0 g, 2.94 mmol) as a starting material.

LC-MS (ESI): m/z = 544.2 [M+H]⁺.

Step 3: At room temperature, **83C** (1.5 g, 2.76 mmol) was dissolved in THF (20 mL), TBAF (0.87 g, 3.31 mmol) was added, and the resulting mixture was warmed to 80°C and reacted for 16 hours. Upon complete depletion of starting materials as monitored by TLC, the reaction was stopped. The reaction mixture was cooled to room temperature, and EA (30 mL) was added. The organic phase was washed with water (30 mL×3) and saturated aqueous sodium chloride solution (30 mL×2), dried over anhydrous sodium sulphate, and filtered. The filtrate was concentrated and the residue was separated and purified by column chromatography (PE : EA = 2 : 1) to obtain compound **83D** (0.72 g, 85.50%).

LC-MS (ESI): m/z = 306.1 [M+H]⁺.

Step 4: At room temperature, **83D** (0.72 g, 2.36 mmol) was dissolved in DCM (10 mL), Dess-Martin periodinane (1.20 g, 2.83 mmol) was added in portions, and then the resulting mixture was reacted at room temperature for 2 hours. Upon complete depletion of starting materials as monitored by TLC, the reaction was stopped. To the reaction solution were added DCM (20 mL), saturated aqueous sodium bicarbonate solution (30 mL) and saturated aqueous sodium thiosulphate solution (30 mL). The resulting mixture was stirred to clarification and subjected to phase separation. The organic phase was washed with saturated aqueous sodium chloride solution (30 mL×2), dried over anhydrous sodium sulphate and filtered. The filtrate was concentrated to obtain crude compound **83E** (600 mg, 83.88%).

LC-MS (ESI): m/z = 304.0 [M+H]⁺.

Step 5: At room temperature, **83E** (600 mg, 1.98 mmol) was dissolved in DCM (10 mL). DAST (0.64 g, 3.96 mmol) was added dropwise to the reaction solution at -78°C under nitrogen atmosphere. After the dropwise addition was completed, the reaction was continued for 2 hours. Upon complete depletion of starting materials as monitored by TLC, the reaction was stopped. The reaction solution was poured into ice water to quench the reaction. DCM (10 mL) was added and the mixture was extracted and subjected to phase separation. The organic phase was washed with saturated aqueous sodium chloride solution (30 mL×2), dried over anhydrous sodium sulphate and filtered. The filtrate was concentrated and the residue was separated and purified by column chromatography (PE : EA = 4 : 1) to obtain **83F** (130 mg, 20.20%).

LC-MS (ESI): m/z = 326.1 [M+H]⁺.

Step 6: With reference to the synthesis route of step 2 in Example 25, compound **83** (10 mg, 6.46%) was obtained using compound **83F** (125 mg, 0.39 mmol) as a starting material.

LC-MS (ESI): m/z = 443.6 [M+H]⁺.

¹H NMR (400 MHz, CDCl₃) δ 7.82-7.80 (d, 1H), 6.91-6.90 (d, 1H), 6.00-5.98 (d, 1H), 5.62 (s, 1H), 4.78-4.72 (m, 4H), 4.22-4.18 (t, 2H), 3.70-3.67 (t, 2H), 3.31-3.21 (m, 1H), 2.81-2.77 (t, 2H), 2.66-2.55 (m, 1H), 2.53-2.52 (d, 3H), 2.41-2.18 (m, 7H), 2.16-1.99 (m, 2H).

### Example 84:

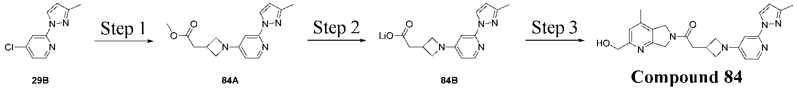

Step 1: **29B** (7 g, 36.15 mmol), methyl 2-(azetidin-3-yl)acetate (18.68 g, 144.60 mmol), caesium fluoride (10.98 g, 72.30 mmol), and triethylamine (14.61 g, 144.60 mmol) were dissolved in dimethyl sulphoxide (60 mL), and then the mixture was warmed to 100°C and reacted for 4 hours. After the reaction was completed, the mixture was cooled to room temperature and diluted with water (200 mL). The resulting mixture was extracted with ethyl acetate three times (100 mL×3). The combined organic phases were dried over anhydrous sodium sulphate, filtered and concentrated under reduced pressure. The resulting residue was purified by silica gel column chromatography (petroleum ether : ethyl acetate (v : v) = 3 : 1) to obtain compound **84A** (1.7 g, yield: 16.41%).

LC-MS (ESI): m/z = 287.3 [M+H]⁺.

Step 2: **84A** (1.65 g, 5.76 mmol) was dissolved in tetrahydrofuran (15 mL), methanol (5 mL) and water (5 mL). Then lithium hydroxide (0.28 g, 11.52 mmol) was added and the resulting mixture was reacted at room temperature for 2 hours. After the reaction was completed, the mixture was concentrated under reduced pressure to obtain crude compound **84B** (1.8 g).

LC-MS (ESI): m/z = 273.1[M+H]⁺.

Step 3: Compound (4-methyl-6,7-dihydro-5H-pyrrolo[3,4-b]pyridin-2-yl)methanol (synthesized with reference to the method described in the patent WO 2018002760) (1.0 g, 6.09 mmol), compound **84B** (1.66 g, 6.09 mmol) and N-methylimidazole (1.9 mL, 34.26 mmol) were successively dissolved in N,N-dimethylformamide (20 mL). Subsequently, chloro-N,N,N',N'-tetramethylformamidinium hexafluorophosphate (2.05 g, 7.31 mmol) was added and the resulting mixture was reacted at room temperature for 1 hour. After the reaction was completed as monitored by TLC, the mixture was concentrated under reduced pressure. The residue was purified by silica gel column chromatography (dichloromethane : methanol (v : v) = 20 : 1) to obtain **compound 84** (1.0 g, yield: 39.25%).

¹H NMR (400 MHz, DMSO-*d6*) δ 8.42-8.38 (m, 1H), 7.99-7.94 (m, 1H), 7.21 (s, 1H), 6.71 (s, 1H), 6.32-6.24 (m, 2H), 5.45-5.38 (m, 1H), 4.88-4.77 (m, 2H), 4.64-4.56 (m, 2H), 4.55-4.50 (m, 2H), 4.21-4.13 (m, 2H), 3.74-3.68 (m, 2H), 3.20-3.10 (m, 1H), 2.90-2.84 (m, 2H), 2.31-2.25 (m, 6H).

LC-MS (ESI): m/z = 419.1[M+H]⁺.

### Example 85:

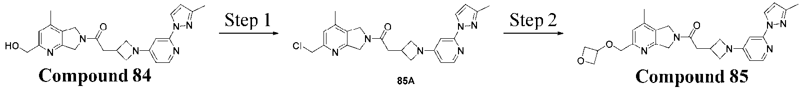

Step 1: **Compound 84** (0.2 g, 0.48 mmol) was dissolved in dichloromethane (6 mL), N,N-dimethylformamide (0.035 g, 0.48 mmol) was added, and then in an ice-water bath, oxalyl chloride (0.12 g, 0.96 mmol) was slowly added. The mixture was reacted at room temperature for 2 hours. After the reaction was completed, saturated aqueous sodium bicarbonate solution was added to quench the reaction. Then the mixture was extracted with dichloromethane three times. The combined organic phases were concentrated to obtain crude product **85A** (0.18 g, 86%).

LC-MS (ESI): m/z = 437.0 [M+H]⁺.

Step 2: Oxetan-3-ol (0.085 g, 1.15 mmol) was dissolved in N,N-dimethylformamide (6 mL), and then sodium hydride (0.017 g, 0.69 mmol) was added. After the mixture was stirred for half an hour, **85A** (0.1 g, 0.23 mmol) was added. Subsequently, the mixture was reacted at room temperature for 2 hours. The reaction solution was diluted with water and then extracted with ethyl acetate three times. The combined organic phases were concentrated and the resulting crude product was separated and purified by preparative HPLC to obtain **compound 85** (12 mg, 11%). HPLC separation method: Instrument: waters 2767 preparative liquid chromatographic instrument; chromatographic column: SunFire@ Prep C18 (19 mm×150 mm); composition of mobile phases: mobile phase A: acetonitrile; and mobile phase B: water (containing 5% ammonium acetate); gradient: 10%-40% acetonitrile gradient.

LC-MS (ESI): m/z = 475.1 [M+H]⁺.

¹H NMR (400 MHz, DMSO-*d*₆) δ 8.42-8.35 (m, 1H), 7.98-7.94 (m, 1H), 7.24-7.17 (m, 1H), 6.73-6.69 (m, 1H), 6.43-6.21 (m, 2H), 4.89-4.79 (m, 2H), 4.73-4.62 (m, 4H), 4.58 (s, 1H), 4.50-4.40 (m, 4H), 4.21-4.13 (m, 2H), 3.74-3.66 (m, 2H), 3.20-3.08 (m, 1H), 2.90-2.83(m, 2H), 2.31-2.24 (m, 6H).

### Example 86:

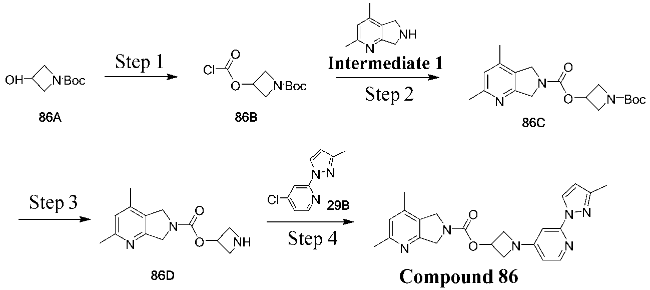

Step 1: **86A** (2.0 g, 11.51 mmol) was dissolved in dichloromethane (20 mL), and in an ice bath, triphosgene (5.12 g, 17.28 mmol) was added. Subsequently, the mixture was stirred at room temperature for 2 h. After the reaction was completed, the reaction mixture was concentrated under reduced pressure to obtain crude compound **86B,** which was directly used in the next reaction.

Step 2: **Intermediate 1** (1.40 g, 4.81 mmol) was dissolved in dichloromethane (30 mL), and triethylamine (4.29 g, 42.44 mmol) and compound **86B** (1.50 g, 4.81 mmol) were successively added. Subsequently, the mixture was stirred at room temperature for 16 h. Upon complete consumption of the starting materials as monitored by LCMS, water (40 mL) was added, and the mixture was stirred for 5 min and extracted with dichloromethane (40 mL). The organic phase was dried over anhydrous sodium sulphate and concentrated under reduced pressure, and the crude product was purified by column chromatography (eluents: petroleum ether : ethyl acetate = 1 : 1) to obtain the target compound **86C** (1.20 g, yield: 29.8%).

LCMS m/z = 292.3 [M - 55]*⁺.*

Step 3: Compound **86C** (0.50 g, 1.44 mmol) was dissolved in dichloromethane (5 mL), and trifluoroacetic acid (5 mL) was added. The mixture was reacted at room temperature for 2 h. After the reaction was completed, the reaction mixture was concentrated under reduced pressure to obtain the crude target compound **86D** (0.5 g), which was directly used in the next reaction.

Step 4: Compound **86D** (500 mg, 2.02 mmol) and **29B** (0.47 g, 2.42 mmol) were successively dissolved in DMF (10 mL), and caesium carbonate (1.97 g, 6.06 mmol) was added. Subsequently, the mixture was warmed to 80°C and stirred for 16 h. Upon complete consumption of the starting materials as monitored by LCMS, the mixture was cooled to room temperature. Water (20 mL) was added, and the mixture was stirred for 5 min and extracted with dichloromethane (20 mL). The organic phase was dried over anhydrous sodium sulphate and concentrated under reduced pressure, and the crude product was purified by column chromatography (eluents: dichloromethane : methanol = 10 : 1) to obtain the crude target compound, which was further purified by preparative HPLC to obtain **compound 86** (100 mg, 12%).

Preparative HPLC separation method: Instrument: waters 2767 preparative liquid chromatographic instrument; chromatography column: SunFire@ Prep C18 (19 mm×250 mm); the sample was dissolved in DMF and filtered with a 0.45 µm filter to prepare a sample solution; preparative chromatography conditions: a. composition of mobile phases A and B: mobile phase A: acetonitrile, and mobile phase B: water (containing 5 mM aqueous ammonia); b. gradient elution, mobile phase A: 40%-80%; c. flow rate: 15 mL/min. d. elution time: 20 min.

LCMS m/z = 405.7 [M+H]⁺.

¹H NMR (400 MHZ, DMSO-d*6*) δ8.41 (d, 1H), 8.01 (d, 1H), 6.99 (s, 1H), 6.78 (d, 1H),6.33-6.35 (m, 1H), 6.30 (d,1H), 5.32-5.39 (m, 1H), 4.71 (s, 1H), 4.63 (d,2H), 4.56 (s, 1H), 4.38 (t, 2H), 4.00-4.03 (m, 2H), 2.41 (s, 3H), 2.28 (s, 3H), 2.22 (d, 3H).

### Example 87:

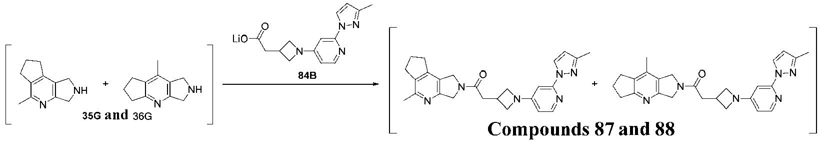

Step 1: A mixture of compound 35G and compound 36G (0.2 g, crude), **84B** (0.23 g, 0.81 mmol), N-methylimidazole (0.23 g, 2.83 mmol) and chloro-N,N,N',N'-tetramethylformamidinium hexafluorophosphate (0.34 g, 1.22 mmol) were successively dissolved in N,N-dimethylformamide (15 mL), and the mixture was reacted at room temperature for 2 hours. After the reaction was completed, the reaction solution was filtered and then directly separated and purified using a reverse-phase preparative column (C18 spherical 20-35 nm 100 A 80 g) (acetonitrile : water (0.1% TFA) = 10%-80%) to obtain **compound 87** (28 mg, 8.07%) and **compound 88** (30 mg, 8.64%).

HPLC analytical method: chromatographic column: Xtimate C18 4.6*50 mm, 3 µm. 2. detection wavelength: 210 nm and 254 nm. 3. flow rate: 1 mL/min; column temperature: 35°C; acquisition time: 10 min; mobile phase: mobile phase A: 0.05% TFA solution, mobile phase B: acetonitrile. **Compound 87,** retention time: 2.576 min; **Compound 88,** retention time: 2.706 min.

**Compound 87:** ¹H NMR (400 MHz, CDCl₃) δ 8.42 (d, 1H), 8.00 (d, 1H), 6.85 (d, 1H), 6.25-6.05 (m, 2H), 4.77-4.71 (m, 4H), 4.31-4.27 (m, 2H), 3.79-3.74 (m, 2H), 3.30 (d, 1H), 2.96-2.72 (m, 6H), 2.48 (s, 3H), 2.36 (s, 3H), 2.20-2.13 (m 2H).

LC-MS (ESI): m/z = 429.3 [M+H]⁺.

**Compound 88:** ¹H NMR (400 MHz, CDCl₃) δ 8.52 (s, 1H), 8.00 (d, 1H), 6.86 (d, 1H), 6.22 (d, 1H), 6.15-6.13 (m, 1H), 4.77-4.72 (m, 4H), 4.34-4.30 (m, 2H), 3.88-3.72 (m, 2H), 3.39-3.23 (m, 1H), 3.08-2.95 (m, 2H), 2.89-2.79 (m, 4H), 2.36 (s, 3H), 2.26-2.11 (m, 5H).

LC-MS (ESI): m/z = 429.3 [M+H]⁺.

### Example 89:

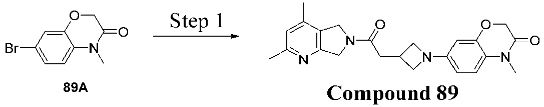

Compound **89A** (250 mg, 1.03 mmol), **2C** (1.11 g, 3.09 mmol), caesium carbonate (1.01 g, 3.10 mmol), X-antphos (30 mg, 0.05 mmol) and Pd₂dba₃ (19 mg, 0.02 mmol) were successively dissolved in anhydrous 1,4-dioxane (5 mL), and then the mixture was warmed to 100°C and reacted for 16 hours. After the reaction was completed, the reaction solution was cooled to room temperature, added dropwise to 15 mL of ice water, and then extracted with ethyl acetate (30 mL×2). The organic phases were combined and concentrated and the residue was separated by silica gel column chromatography (dichloromethane : methanol (v/v) = 15 : 1) and C18 column chromatography (0.1% aqueous NH₄HCO₃ solution : acetonitrile (v/v) = 3 : 7) to obtain **compound 89** (7 mg, 2%).

LC-MS (ESI): m/z =407.7 [M+H]⁺.

1H NMR (400 MHz, DMSO-d*6*) δ 7.03-6.93 (m, 2H), 6.16-6.06 (m, 2H), 4.83-4.74 (m, 2H), 4.61-4.51 (m, 4H), 3.95 (t, 2H), 3.47 (t, 2H), 3.22 (s, 3H), 3.07-2.99 (m, 1H), 2.84-2.78 (m, 2H), 2.42 (s, 3H), 2.25-2.20 (m, 3H).

### Example 90:

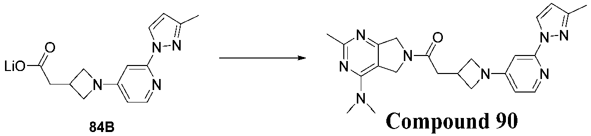

**84B** (0.13 g, 0.48 mmol) and N,N,2-trimethyl-5H,6H,7H-pyrrolo[3,4-d]pyrimidin-4-amine (0.094 g, 0.53 mmol, synthesized with reference to the method described in the patent WO 2018066718) were successively dissolved in acetonitrile (6 mL), and 1-methylimidazole hydrochloride (0.080 g, 0.67 mmol) and [chloro(dimethylamino)methylene]-dimethylazane hexafluorophosphate (0.18 g, 0.62 mmol) were successively added. The mixture was then reacted at 40°C for 2 hours. After the reaction was completed, the mixture was concentrated and the residue was separated and purified by preparative HPLC to obtain **compound 90** (50 mg, 24%). Preparative HPLC separation method: Instrument: waters 2767 preparative liquid chromatographic instrument; chromatographic column: SunFire@ Prep C18 (19 mm × 150 mm); composition of mobile phases: mobile phase A: acetonitrile; and mobile phase B: water (containing 5% trifluoroacetic acid); gradient: 10%-40% acetonitrile gradient.

LC-MS (ESI): m/z = 433.9 [M+H]⁺.

¹H NMR (400 MHz, DMSO-*d*₆) δ 8.59-8.51 (m, 1H), 7.98-7.90 (m, 1H), 6.83 (d, 1H), 6.56 - 6.35 (m, 2H), 5.14 (d, 1H), 4.91 (s, 1H), 4.76 (s, 1H), 4.53 (s, 1H), 4.34-4.21 (m, 2H), 3.87-3.77 (m, 2H), 3.31-3.24(m, 6H), 3.16 (d, 1H), 2.94-2.84 (m, 2H), 2.48 (s, 3H), 2.31 (d, 3H).

### Example 91:

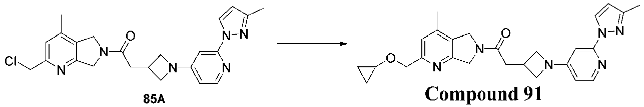

Cyclopropanol (26 mg, 0.44 mmol) was dissolved in DMF (5 mL), and at 0°C, NaH (22 mg, 0.55 mmol) was added. Subsequently, the mixture was warmed to room temperature and reacted for 30 min. Compound **85A** (50 mg, 0.11 mmol) was added and the mixture was reacted at room temperature for 4 hours. After the reaction was completed as monitored by TLC, the mixture was concentrated under reduced pressure. The residue was separated and purified by silica gel column chromatography (DCM : MeOH = 10 : 1) to obtain **compound 91** (4 mg, yield: 7.93%).

¹H NMR (400 MHz, DMSO-d*6*) δ 8.41-8.38 (m, 1H), 7.98-7.93 (m, 1H), 7.17-7.13 (m, 1H), 6.72-6.90 (m, 1H), 6.30-6.24 (m, 2H), 4.88-4.78 (m, 2H), 4.64-4.56 (m, 2H), 4.55-4.51 (m, 2H), 4.20-4.12 (m, 2H), 3.73-3.67 (m, 2H), 3.44-3.40 (m, 1H), 3.17-3.08 (m, 1H), 2.89-2.83 (m, 2H), 2.30-2.24 (m, 6H), 0.59-0.53 (m, 2H), 0.50-0.43 (m, 2H).

LC-MS(ESI):m/z = 459.1[M+H]⁺.

### Example 92:

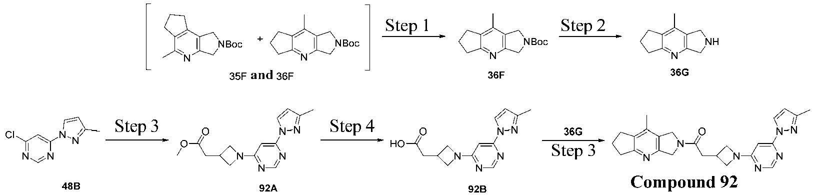

Step 1: A mixture of compounds **35F** and **36F** (16.00 g) was separated by chiral preparative HPLC to obtain compound **35F** (retention time: 0.851 min, 6.11 g, 38.18%) and compound **36F** (retention time: 0.963 min, 6.78 g, 42.37%). HPLC analytical method: chromatographic column: Xtimate C18 4.6*50 mm, 3 µm. 2. detection wavelength: 210 nm and 254 nm. 3. flow rate: 1 mL/min; column temperature: 35°C; acquisition time: 10 min; mobile phase: mobile phase A: 0.05% TFA solution, mobile phase B: acetonitrile. Preparative HPLC method: instrument: SHIMADZU LC-30AD sf; chromatographic column: Chiral AD column; mobile phase: A for CO₂; B for 0.05% DEA in methanol; gradient: 5%-40% B gradient elution; flow rate: 3 mL/min; column temperature: 35°C; wavelength: 220 nm; cycle time: 3.5 min; sample preparation: sample concentration 80 mg/mL, methanol solution; sample injection: 1 mL/injection.

Compound **35F:** ¹H NMR (400 MHz, Chloroform-d) δ 4.88-4.24 (m, 4H), 3.06-2.66 (m, 4H), 2.46 (s, 3H), 2.18-2.11 (m, 2H), 1.52 (d, 9H); LC-MS (ESI): m/z = 275.5 [M+H]⁺.

Compound **36F:** ¹H NMR (400 MHz, Chloroform-d) δ 4.88-4.36 (m, 4H), 3.03-2.84 (m, 4H), 2.18-2.11 (m, 5H), 1.52 (d, 9H); LC-MS (ESI): m/z = 275.5 [M+H]⁺.

Step 2: Compound **36F** (6.78 g, 24.71 mmol) was dissolved in 4 mol/L hydrogen chloride 1,4-dioxane solution (100 mL). Subsequently, the mixture was reacted at 45°C for 4 hours. After the reaction was completed, the mixture was concentrated. To the residue was added water (100 mL). The mixture was adjusted to pH > 8 by dropwise adding saturated aqueous sodium bicarbonate solution and then extracted with dichloromethane (200 mL). The organic phase was washed with saturated aqueous sodium chloride solution, dried over anhydrous sodium sulphate, and concentrated under reduced pressure to obtain compound **36G** (3.6 g, 83.61%).

LC-MS (ESI): m/z = 175.3 [M+H]⁺.

Step 3: Compound **48B** (5.5 g, 28.26 mmol), methyl 2-(azetidin-3-yl)acetate (3.65 g, 28.26 mmol) and potassium carbonate (7.81 g, 56.52 mmol) were successively dissolved in dimethyl sulphoxide (60 mL), and then the mixture was warmed to 100°C and reacted for 2 hours. After the reaction was completed, the mixture was cooled to room temperature. Ethyl acetate (200 mL) and water (200 mL) were added and the mixture was extracted and subjected to phase separation. The organic phase was washed with saturated aqueous sodium chloride solution, dried over anhydrous sodium sulphate, and concentrated under reduced pressure. The resulting residue was separated and purified using a silica gel column (ethyl acetate : petroleum ether (v : v) = 0-50%) to obtain compound **92A** (5.6 g, 68.97%).

LC-MS (ESI): m/z = 288.1 [M+H]⁺.

Step 4: Compound **92A** (5.50 g, 19.14 mmol) was dissolved in tetrahydrofuran solution (40 mL), and lithium hydroxide (0.92 g, 38.47 mmol), methanol (5 mL) and water (5 mL) were successively added. The mixture was reacted at room temperature overnight. After the reaction was completed, the mixture was concentrated under reduced pressure. To the residue was added water (50 mL). The mixture was adjusted to pH 5-6 with 1 mol/L aqueous hydrochloric acid solution, stirred for 1 hour and then filtered. The filter cake was washed with distilled water and dried to obtain the target compound **92B** (4.4 g, 84.12%)

LC-MS (ESI): m/z = 274.0 [M+H]⁺.

Step 5: Compound **36G** (0.30 g, 1.21 mmol), compound **92B** (0.33 g, 1.21 mmol), N-methylimidazole (0.35 g, 4.23 mmol), and chloro-N,N,N',N'-tetramethylformamidinium hexafluorophosphate (0.51 g, 1.81 mmol) were successively dissolved in N,N-dimethylformamide (20 mL), and then the mixture was reacted at room temperature for 2 hours. After the reaction was completed, the mixture was concentrated under reduced pressure, and to the residue were added methanol (10 mL) and dichloromethane (1 mL). The mixture was filtered and the filtrate was separated and purified using a reverse-phase preparative column (C18 spherical 20-35 nm 100 A 80 g) (acetonitrile : water (v : v) = 10%-80%) to obtain **compound 92** (401 mg, 77.16%).

LC-MS (ESI): m/z = 430.6 [M+H]⁺.

¹H NMR (400 MHz, Chloroform-d) δ 8.47-8.28 (m, 2H), 6.66-6.65 (m, 1H), 6.23-6.22 (m, 1H), 4.88-4.62 (m, 4H), 4.44-4.39 (m, 2H), 3.92-3.87 (m, 2H), 3.34-3.26 (m, 1H), 3.04-3.00 (m, 2H), 2.89-2.79 (m, 4H), 2.36 (s, 3H), 2.28-2.08 (m, 5H).

### Example 93:

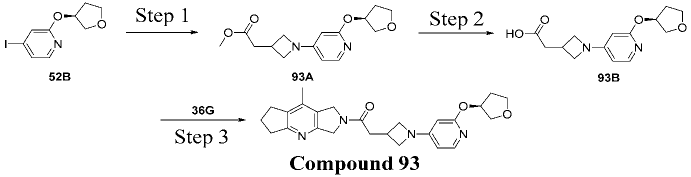

Step 1: **52 B** (350 mg, 1.20 mmol), methyl 2-(azetidin-3-yl)acetate 2,2,2-trifluoroacetate (350 mg, 1.44 mmol), caesium carbonate (1.17 g, 3.59 mmol) and RuPhos Pd G3 catalyst (100 mg, 0.12 mmol) were successively dissolved in toluene (15 mL), and under nitrogen atmosphere, the mixture was warmed to 100°C, stirred and reacted overnight. After the reaction was completed as monitored by TLC, the reaction system was cooled to room temperature and filtered through diatomaceous earth. The filtrate was concentrated under reduced pressure and the residue was separated and purified by silica gel column chromatography (petroleum ether : ethyl acetate (v/v) = 1 : 1) to obtain **compound 93A** (170 mg, yield: 48.46%).

LC-MS (ESI): m/z =293.3[M+H]⁺.

Step 2: **93A** (170 mg, 0.58 mmol) was dissolved in mixed solvents of tetrahydrofuran (4 mL) and water (1 mL). Lithium hydroxide (21 mg, 0.87 mmol) was added and then the mixture was reacted at room temperature for 2 hours. After the reaction was completed, the mixture was concentrated to obtain a lithium salt of **93B** (200 mg, crude).

LC-MS (ESI): m/z =279.1[M+H]⁺.

Step 3: Lithium salt of compound **93B** (0.2 g, crude, 0.58 mmol), **36G** (100 mg, 0.58 mmol), N-methylimidazole (143 mg, 1.74 mmol), and chloro-N,N,N',N'-tetramethylformamidinium hexafluorophosphate (195 mg, 0.70 mmol) were successively dissolved in N,N-dimethylformamide (8 mL), and the mixture was reacted at room temperature for 2 hours. After the reaction was completed, water (30 mL) was added, and the mixture was extracted with ethyl acetate (30 mL×3). The combined organic phases were washed with saturated aqueous sodium chloride solution, dried over anhydrous sodium sulphate, filtered, and concentrated under reduced pressure. The residue was separated and purified by silica gel column chromatography (dichloromethane : methanol (v/v) = 20 : 1) to obtain **compound 93** (50 mg, 19.8%).

LC-MS (ESI): m/z = 435.3 [M+H]⁺.

¹H NMR (400 MHz, DMSO-*d₆*) δ 7.73-7.71 (m, 1H), 6.06-6.04 (m, 1H), 5.62 (s, 1H), 5.44-5.41 (m, 1H), 4.80-4.74 (m, 2H), 4.57-4.52 (m, 2H), 4.06-4.02 (m, 2H), 3.87-3.66 (m, 4H), 3.59-3.56 (m, 2H), 3.10-3.03 (m, 1H), 2.90-2.80(m, 6H), 2.19-2.12 (m, 4H), 2.10-2.02 (m, 2H), 1.96-1.89 (m, 1H).

### Example 94:

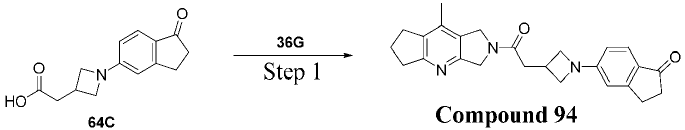

With reference to the operation process of step 2 in Example 93, **compound 94** (140 mg, 34.9%) was obtained using compound **64C** (0.3 g, crude) and **36G** (174 mg, 1.0 mmol) as starting materials.

LC-MS (ESI): m/z = 402.3 [M+H]⁺.

¹H NMR (400 MHz, DMSO-*d*₆) δ 7.41-7.39 (m, 1H), 6.38-6.36 (m, 2H), 4.80-4.75 (m, 2H), 4.57-4.53 (m, 2H), 4.15-4.11 (m, 2H), 3.69-3.67 (m, 2H), 3.12-3.10 (m, 1H), 3.02-2.76 (m, 8H), 2.49-2.46 (m, 2H), 2.16-2.15 (m, 3H), 2.09-1.98 (m, 2H).

### Example 95:

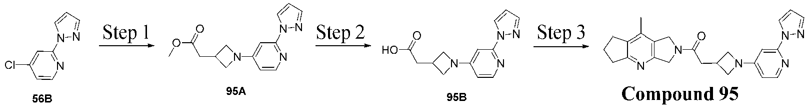

Step 1: **56B** (1.1 g, 6.1 mmol), methyl 2-(azetidin-3-yl)acetate (790 mg, 6.1 mmol), caesium fluoride (1.8 g, 12.2 mmol) and triethylamine (2.5 g, 24.4 mmol) were successively dissolved in dimethyl sulphoxide (20 mL), and then the mixture was warmed to 100°C and reacted for 4 hours. After the reaction was completed, the mixture was cooled to room temperature, diluted with water (100 mL) and extracted with ethyl acetate (100 mL×3). The combined organic phases were dried over anhydrous sodium sulphate, filtered and concentrated under reduced pressure. The resulting residue was purified by silica gel column chromatography (petroleum ether : ethyl acetate (v : v) = 3 : 1) to obtain compound **95A** (1.3 g, yield: 78.3%).

LC-MS (ESI): m/z = 273.6 [M+H]⁺.

Step 2: **95A** (1.3 g, 4.8 mmol) was dissolved in tetrahydrofuran (20 mL) and water (5 mL). Then lithium hydroxide (0.12 g, 5.2 mmol) was added, and the mixture was reacted at room temperature for 2 hours. After the reaction was completed, the mixture was concentrated under reduced pressure to obtain a lithium salt of compound **95B** (1.8 g, crude).

LC-MS (ESI): m/z = 259.1[M+H]⁺.

Step 3: Compound **36G** (175mg, 1.0 mmol), a lithium salt of compound **95B** (258 mg) and N-methylimidazole (328mg, 4 mmol) were successively dissolved in N,N-dimethylformamide (10 mL). Subsequently, chloro-N,N,N',N'-tetramethylformamidinium hexafluorophosphate (336 mg, 1.2 mmol) was added and the mixture was reacted at room temperature for 1 hour. After the reaction was completed as monitored by TLC, the mixture was concentrated under reduced pressure. The residue was purified by silica gel column chromatography (dichloromethane : methanol (v : v) = 10 : 1) to obtain **compound 95** (171 mg, yield: 41.3%).

LC-MS (ESI): m/z =415.3[M+H]⁺.

¹H NMR (400 MHz, CDCl₃) δ 8.60-8.53 (m, 1H), 8.06-7.99 (m, 1H), 7.72-7.66 (m, 1H), 6.98-6.88 (m, 1H), 6.45-6.40 (m, 1H), 6.20-6.13 (m, 1H), 4.79-4.69 (m, 4H), 4.34-4.26 (m, 2H), 3.83- 3.72 (m, 2H), 3.37-3.24 (m, 1H), 3.07-2.98 (m, 2H), 2.92-2.77 (m, 4H), 2.23-2.11 (m, 5H).

### Example 96:

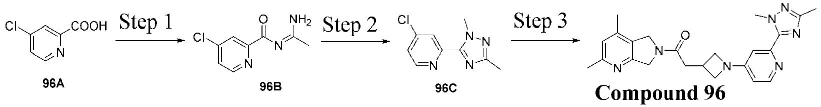

Step 1: At room temperature, **96A** (1.5 g, 9.52 mmol) was dissolved in dry DMF (20 mL). Acetamidine hydrochloride (1.35 g, 14.28 mmol) and DIPEA (2.46 g, 19.04 mmol) were added. After the mixture was stirred evenly, HATU (4.34 g, 11.42 mmol) was added and then the reaction was continued at room temperature for 16 hours. After the reaction was completed, to the reaction solution was added EA (50 mL). The organic phase was washed with water (30 mL×3) and saturated aqueous sodium chloride solution (30 mL×2), dried over anhydrous sodium sulphate, filtered, and concentrated to obtain compound **96B** (1.8 g, 95.68%).

LC-MS (ESI): m/z = 198.3 [M+H]⁺.

Step 2: At room temperature, **96B** (1.8 g, 9.11 mmol) was dissolved in dry DMF (10 mL). Methylhydrazine (500 mg, 10.84 mmol) and glacial acetic acid (2.74 g, 45.64 mmol) were successively added and then the mixture was warmed to 80°C and reacted for 16 hours. After the reaction was completed, the mixture was cooled to room temperature. To the reaction solution was added EA (30 mL), and the organic phase was washed with water (30 mL×3) and saturated aqueous sodium chloride solution (30 mL×2), dried over anhydrous sodium sulphate and filtered. The filtrate was concentrated and the residue was separated and purified by column chromatography (PE : EA = 4 : 1) to obtain compound **96C** (100 mg, 5.26%).

LC-MS (ESI): m/z = 209.1 [M+H]⁺.

Step 3: With reference to the synthesis method of step 2 in **Example 28,** compound **96** (20 mg, 9.98%) was obtained using compound **96C** (100 mg, 0.48 mmol) as a starting material.

LC-MS (ESI): m/z = 418.2 [M+H]⁺.

¹H NMR (400 MHz, CDCl₃) δ 8.27-8.26 (d, 1H), 7.07 (s, 1H), 6.92-6.90 (d, 1H), 6.30-6.28 (d, 1H), 4.78-4.73 (m, 4H), 4.32-4.28 (t, 2H), 4.25 (s, 3H), 3.81-3.76 (m, 2H), 3.35-3.27 (m, 1H), 2.83-2.78 (t, 2H), 2.54-2.53 (d, 3H), 2.42 (s, 3H), 2.27-2.26 (d, 3H).

### Example 97:

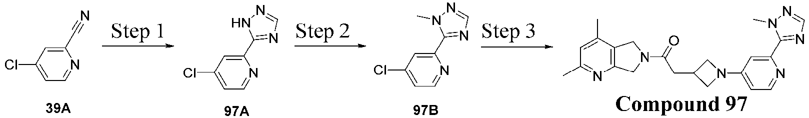

Step 1: **Compound 39A** (1 g, 7.20 mmol) was dissolved in methanol (30 mL). Formamide (0.52 g, 8.66 mmol) and sodium methoxide (0.52 g, 8.66 mmol) were successively added. Subsequently, under nitrogen atmosphere, the mixture was warmed to 70°C, stirred and refluxed overnight. After the reaction was completed as monitored by TLC, the reaction mixture was cooled to room temperature and concentrated under reduced pressure. To the residue was added water (30 mL). The resulting mixture was extracted with ethyl acetate (30 mL×3) and the combined organic phases were washed with saturated aqueous sodium chloride solution, dried over anhydrous sodium sulphate, filtered, and concentrated under reduced pressure. The resulting residue was separated and purified by silica gel column chromatography (petroleum ether : ethyl acetate (v/v) = 1 : 1) to obtain **compound 97A** (213 mg, yield: 16.38%).

LC-MS (ESI): m/z = 181.0 [M+H]⁺.

Step 2: **Compound 97A** (213 mg, 1.18 mmol), potassium carbonate (326 mg, 2.36 mmol) and iodomethane (201 mg, 1.42 mmol) were successively dissolved in DMF (10 mL), and under nitrogen atmosphere, the mixture was stirred at room temperature overnight. After the reaction was completed as monitored by TLC, water (30 mL) was added, and the mixture was extracted with ethyl acetate (30 mL×3). The combined organic phases were washed with saturated aqueous sodium chloride solution, dried over anhydrous sodium sulphate, filtered, and concentrated under reduced pressure. The residue was separated and purified by silica gel column chromatography (petroleum ether : ethyl acetate (v/v) = 1 : 1) to obtain **compound 97B** (90 mg, yield: 39.19%).

LC-MS (ESI): m/z = 195.1 [M+H]⁺.

Step 3: With reference to the operation process of step 1 in Example 10, **compound 97** (10 mg, yield: 6.04%) was obtained using **compound 97B** (90 mg, 0.46 mmol) and **compound 5B** (100 mg, 0.41 mmol) as starting materials.

LC-MS (ESI): m/z = 404.3 [M+H]⁺.

¹H NMR (400 MHz, DMSO-d6) δ 8.27-8.21 (m, 1H), 7.96 (s, 1H), 7.04-7.02 (m, 1H), 7.01-6.99 (m, 1H), 6.47-6.43 (m, 1H), 4.84-4.76 (m, 2H), 4.63-4.53 (m, 2H), 4.23 (s, 3H), 4.19-4.14 (m, 2H), 3.73-3.68 (m, 2H), 3.19-3.10 (m, 1H), 2.89-2.83 (m, 2H), 2.44-2.40 (m, 3H), 2.25-2.20 (m, 3H).

### Example 98:

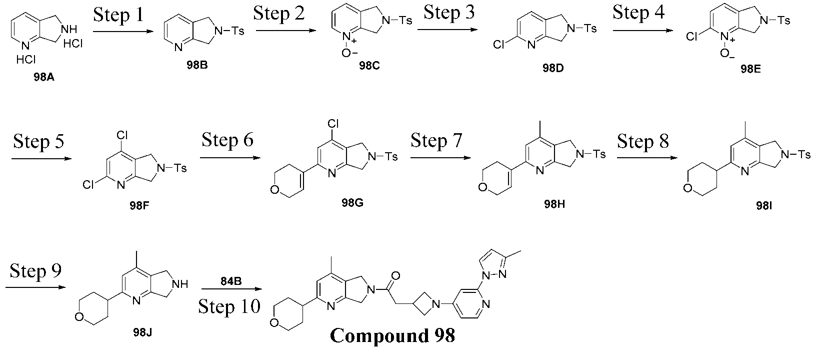

Step 1: Compound **98A** (3.86 g, 20 mmol) was dissolved in dichloromethane (200 mL). Triethylamine (8.1 g, 80 mmol) and p-toluenesulphonyl chloride (4.58 g, 24 mmol) were successively added, and the mixture was stirred at room temperature for 1 hour. After the reaction was completed, water (200 mL) was added to quench the reaction. The mixture was extracted and subjected to phase separation. The aqueous phase was further extracted with dichloromethane (200 mL×2). The combined organic phases were dried over anhydrous sodium sulphate, filtered and concentrated under reduced pressure. The residue was separated and purified by silica gel column chromatography (PE : EA (v/v) = 3 : 1) to obtain the target compound **98B** (5.2 g, yield: 95%).

LC-MS (ESI): m/z =275.2 [M+H]⁺.

Step 2: Compound **98B** (5.2 g, 19 mmol) was dissolved in dichloromethane (100 mL), and m-CPBA (7.7 g, 85% wt, 38 mmol) was added. The mixture was stirred at room temperature for 1 hour. After the reaction was completed, saturated aqueous sodium bicarbonate solution (100 mL) was added to quench the reaction. Subsequently, the mixture was extracted and subjected to phase separation. The organic phase was further washed with saturated aqueous sodium bicarbonate solution (100 mL×2), dried over anhydrous sodium sulphate, filtered and concentrated under reduced pressure. The residue was separated and purified by silica gel column chromatography (DCM : MeOH (v/v) = 10 : 1) to obtain the target compound **98C** (5.1 g, yield: 92%).

LC-MS (ESI): m/z =291.2 [M+H]⁺.

Step 3: Compound **98C** (5.1 g, 17.6 mmol) was dissolved in phosphorus oxychloride (50 mL), and the mixture was warmed to 100°C and reacted for 30 minutes. After the reaction was completed, the mixture was cooled to room temperature and concentrated under reduced pressure to remove phosphorus oxychloride. The residue was quenched with saturated aqueous sodium bicarbonate solution (50 mL), extracted with dichloromethane (50 mL×2), dried over anhydrous sodium sulphate, and concentrated under reduced pressure. The residue was separated and purified by silica gel column chromatography (PE : EA (v/v) = 5 : 1) to obtain the target compound **98D** (2 g, yield: 37%).

LC-MS (ESI): m/z =309.2 [M+H]⁺.

Step 4: With reference to the operation process of step 2, compound **98E** (1.5 g, yield: 70%) was obtained using compound **98D** (2 g, 6.5 mmol) as a starting material.

LC-MS (ESI): m/z = 325.2 [M+H]⁺

Step 5: With reference to the operation process of step 3, compound **98F** (0.93 g, yield: 60%) was obtained using compound **98E** (1.5 g, 4.6 mmol) as a starting material.

LC-MS (ESI): m/z =343.2 [M+H]⁺

Step 6: Compound **98F** (343 mg, 1 mmol) was dissolved in dioxane (50 mL), and 3,6-dihydro-2H-pyran-4-boronic acid pinacol ester (420 mg, 2 mmol), Pd(dppf)Cl₂ (146 mg, 0.2 mmol) and caesium carbonate (978 mg, 3 mmol) were successively added. The mixture was warmed to 100°C and reacted for 3 hours. After the reaction was completed, the mixture was cooled to room temperature and concentrated under reduced pressure. The residue was separated and purified by silica gel column chromatography (PE : EA (v/v) = 10 : 1) to obtain the target compound **98G** (170 mg, yield: 40%).

LC-MS (ESI): m/z = 391.2 [M+H]⁺.

Step 7: Compound **98G** (170 mg, 0.4 mmol) was dissolved in dioxane (30 mL). Trimethylboroxine (151 mg, 1.2 mmol), Pd(dppf)Cl₂ (59 mg, 0.08 mmol) and caesium carbonate (391 mg, 1.2 mmol) were successively added, and the mixture was warmed to 100°C and reacted for 1 hour. After the reaction was completed, the mixture was cooled to room temperature and concentrated under reduced pressure. The residue was separated and purified by silica gel column chromatography (PE : EA (v/v) = 10 : 1) to obtain the target compound **98H** (100 mg, yield: 99%).

LC-MS (ESI): m/z =371.2 [M+H]⁺.

Step 8: Compound **98H** (100 mg, 0.27 mmol) was dissolved in methanol (30 mL), palladium on carbon (50 mg) was added, and the mixture was reacted under hydrogen atmosphere for 1 hour. After the reaction was completed, the mixture was filtered to remove palladium on carbon and concentrated under reduced pressure. Then the residue was separated and purified by silica gel column chromatography (PE : EA (v/v) = 10 : 1) to obtain the target compound **98I** (75 mg, yield: 75%).

LC-MS (ESI): m/z =373.2 [M+H]⁺.

Step 9: Compound **98I** (75 mg, 0.2 mmol) was dissolved in hydrogen bromide acetic acid solution (30 mL), and phenol (56 mg, 0.6 mmol) was added. The mixture was warmed to 100°C and reacted for 18 hours. After the reaction was completed, the reaction solution was concentrated under reduced pressure to remove the solvent. The residue was quenched with saturated aqueous sodium bicarbonate solution (30 mL), extracted with dichloromethane (30 mL×2), dried over anhydrous sodium sulphate, and concentrated under reduced pressure. The residue was separated and purified by silica gel column chromatography (DCM : MeOH (v/v) = 10 : 1) to obtain the target compound **98J** (35 mg, yield: 80%).

LC-MS (ESI): m/z =219.2 [M+H]⁺.

Step 10: Compound **84B** (44 mg, 0.16 mmol) was dissolved in DMF (30 mL), and DIPEA (62 mg, 0.48 mmol) and HATU (73 mg, 0.19 mmol) were successively added. The mixture was activated at room temperature for 20 minutes. Then compound **98J** (35 mg, 0.16 mmol) was added and the mixture was reacted at room temperature for 1 hour. After the reaction was completed, the reaction was quenched with saturated aqueous sodium chloride solution (30 mL). The reaction mixture was extracted with dichloromethane (30 mL×2), dried over anhydrous sodium sulphate, and concentrated under reduced pressure. The residue was separated and purified by silica gel column chromatography (DCM : MeOH (v/v) = 10 : 1) to obtain the target **compound 98** (5 mg, yield: 7%).

LC-MS (ESI): m/z =473.5 [M+H]⁺.

¹H NMR (400 MHz, CDCl₃) δ 8.71 (s, 1H) , 8.01-7.99 (m, 1H), 6.92-6.91 (m, 1H), 6.87-6.86 (m, 1H), 6.26 (s, 1H), 6.17-6.15 (m, 1H), 4.82-4.77 (m, 2H), 4.76-4.72 (m, 2H), 4.41-4.32 (m, 2H), 4.13-4.05 (m, 2H), 3.90-3.80 (m, 2H), 3.57-3.51 (m, 2H), 3.38-3.29 (m, 1H), 2.97-2.89 (m, 1H), 2.86-2.78 (m, 2H), 2.36 (s, 3H), 2.32-2.27 (m, 3H), 1.90-1.82 (m, 4H)

### Example 99:

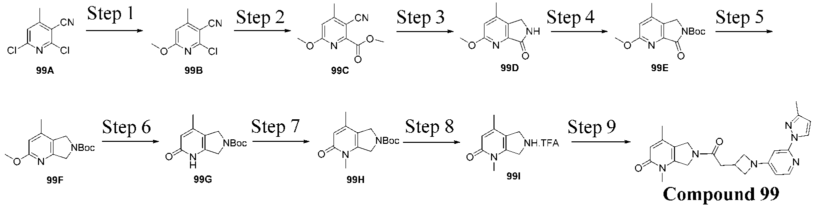

Step 1: **99A** (40 g, 213.88 mmol) was dissolved in methanol (600 mL). Sodium methoxide methanol solution (224.63 mmol) was added dropwise at 0°C. After the dropwise addition was completed, the mixture was reacted at room temperature for 3 h. After the reaction was completed, a large amount of solids were precipitated and filtered. The filtrate was concentrated under reduced pressure to remove most of methanol. Then the residual solution and filter cake were dissolved in dichloromethane (400 mL), and the organic phase was washed with water and concentrated under reduced pressure to obtain crude product **99B** (40 g).

LC-MS (ESI): m/z = 183[M+H]⁺.

Step 2: **99B** (40 g, 219.05 mmol), triethylamine (33.25 g, 328.58 mmol), and 1,1-bis(diphenylphosphino)ferrocene palladium dichloride (8.01 g, 10.95 mmol) were successively dissolved in methanol (350 mL). Subsequently, under CO gas (3 MPa) atmosphere, the mixture was warmed to 100°C and reacted for 16 h. After the reaction was completed, the reaction solution was cooled to room temperature and concentrated under reduced pressure. The residue was separated and purified by silica gel column chromatography (EA/PE = 1/4) to obtain crude product **99C** (30 g).

LC-MS (ESI): m/z = 207.1[M+H]⁺.

Step 3: **99C** (30 g, 145.49 mmol) was dissolved in methanol (500 mL), and raney nickel (8.54 g) was added. Under hydrogen atmosphere, the mixture was reacted at room temperature for 16 h. After the reaction was completed, the mixture was filtered and the filtrate was concentrated under reduced pressure. The residue was separated and purified by silica gel column chromatography (DCM/MeOH = 20/1) to obtain **99D** (7.5 g, 28.93%).

LC-MS (ESI): m/z = 179.1[M+H]⁺.

Step 4: **99D** (7.5 g, 42.09 mmol), di-tert-butyl dicarbonate (13.78 g, 63.14 mmol) and triethylamine (8.52 g, 84.18 mmol) were successively dissolved in tetrahydrofuran (80 mL). 4-Dimethylaminopyridine (1.03 g, 8.43 mmol) was added at 0°C and then the mixture was reacted at room temperature for 16 h. After the reaction was completed, water (50 mL) was added, and the mixture was extracted with ethyl acetate (50 mL×3). The combined organic phases were washed with saturated aqueous sodium chloride solution, dried over anhydrous sodium sulphate and filtered. The filtrate was concentrated under reduced pressure and the residue was separated and purified by silica gel column chromatography (EA/PE = 1/3) to obtain **99E** (11 g, 93.91%).

LC-MS (ESI): m/z = 179.1 [M+H]⁺.

Step 5: **99E** (11 g, 39.53 mmol) was dissolved in THF (100 mL), and borane dimethyl sulphide (12.01 g, 158.09 mmol) was added. The mixture was warmed to 70°C and reacted for 4 h. After the reaction was completed, the mixture was cooled to room temperature, and in an ice bath, methanol was slowly added dropwise to quench the reaction. After the reaction was quenched, the mixture was concentrated under reduced pressure. The residue was separated and purified by silica gel column chromatography (EA/PE = 1/4) to obtain **99F** (5.5 g, 52.64%).

LC-MS (ESI): m/z = 265.1[M+H]⁺.

Step 6: **99F** (5.5 g, 20.81 mmol), sodium iodide (12.48 g, 83.24 mmol) and trimethylchlorosilane (9.05 g, 83.27 mmol) were successively dissolved in acetonitrile (70 mL), and the mixture was warmed to 85°C and reacted for 3 h. Upon completion of the reaction as monitored by LC-MS, the reaction mixture was cooled to room temperature. Triethylamine (10.53 g, 104.03 mmol) and di-tert-butyl dicarbonate (9.08 g, 41.6 mmol) were added and the mixture was reacted at room temperature for 16 h. The reaction mixture was concentrated under reduced pressure to remove most of acetonitrile. Water (60 mL) was added, and the mixture was extracted with ethyl acetate (50 mL×3). The combined organic phases were concentrated under reduced pressure and the residue was separated and purified by silica gel column chromatography (DCM/MeOH = 20/1) to obtain **99G** (5 g, 96.04%).

LC-MS (ESI): m/z = 251.1[M+H]⁺.

Step 7: **99G** (5.00 g, 19.98 mmol) was dissolved in DMF (50 mL), and sodium hydride (1.2 g, 50 mmol) was added in portions at room temperature. After the mixture was stirred for 30 min, iodomethane (14.18 g, 99.9 mmol) was added. The resulting mixture was reacted at room temperature for 16 h. The reaction solution was slowly added to ice water, and the mixture was extracted with ethyl acetate (50 mL×3). The combined organic phases were washed with saturated aqueous sodium chloride solution, dried over anhydrous sodium sulphate and filtered. The filtrate was concentrated under reduced pressure and the residue was separated and purified by silica gel column chromatography (DCM/MeOH = 20/1) to obtain **99H** (1.3 g, 24.62%).

LC-MS (ESI): m/z = 265.1[M+H]⁺.

Step 8: **99H** (0.1 g, 0.38 mmol) was dissolved in dichloromethane (8 mL), trifluoroacetic acid (3 mL) was added, and the mixture was reacted at room temperature for 1 h. The reaction solution was concentrated under reduced pressure to dryness to obtain crude product **99I** (0.2 g), which was directly used in the next reaction without further purification.

LC-MS (ESI): m/z = 165.1[M+H]⁺.

Step 9: **991** (0.2 g), **84B** (0.10 g, 0.38 mmol), N,N-diisopropylethylamine (0.3 g,2.3 mmol), and 2-(7-azobenzotriazole)-N,N,N',N'-tetramethyluronium hexafluorophosphate (0.22 g, 0.57 mmol) were successively dissolved in DMF (5 mL), and the mixture was reacted at room temperature for 16 h. Water (30 mL) was added, and the mixture was extracted with ethyl acetate (20 mL×3). The combined organic phases were washed with saturated aqueous sodium chloride solution and concentrated under reduced pressure. The residue was purified and separated using a C18 reverse-phase column (composition of mobile phases A and B: mobile phase A: acetonitrile; and mobile phase B: water (containing 0.1% aqueous ammonia), (A/B = 45%/55%)) to obtain **compound 99** (0.025 g, 15.72%).

LC-MS (ESI): m/z = 419.2[M+H]⁺.

¹H NMR (400 MHz, DMSO-d6) δ 8.39 (d, 1H), 7.96 (d, 1H), 6.72-6.69 (m, 1H), 6.31-6.22 (m, 2H), 6.17 (s, 1H), 4.90-4.41 (m, 4H), 4.16 (t, 2H), 3.72-3.65(m, 2H), 3.35-3.29 (m, 3H), 3.17-3.09 (m, 1H), 2.86-2.79 (m, 2H), 2.27 (s, 3H), 2.10-2.07 (m, 3H).

### Example 100:

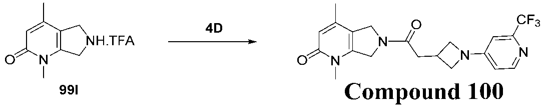

With reference to the operation process of step 9 in Example 99, **compound 100** (0.1 g, 34.18%) was obtained using **99I** (0.2 g, 0.72 mmol) and **4D** (0.19 g, 0.72 mmol) as starting materials.

LC-MS (ESI): m/z = 407.2[M+H]⁺.

¹H NMR (400 MHz, DMSO-d6) δ 8.21 (d, 1H), 6.72-6.69 (m, 1H), 6.56-6.50 (m, 1H), 6.17 (s, 1H), 4.90-4.41 (m, 4H), 4.21-4.13 (m, 2H), 3.73-3.67 (m, 2H), 3.35-3.30 (m, 3H), 3.18-3.05 (m, 1H), 2.84-2.78 (m, 2H), 2.10-2.06 (m, 3H).

### Example 101:

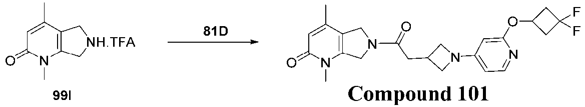

With reference to the operation process of step 9 in Example 99, **compound 101** (0.1 g, 31.25%) was obtained using **99I** (0.2 g, 0.72 mmol) and **81D** (0.19 g, 0.72 mmol) as starting materials.

LC-MS (ESI): m/z = 445.2[M+H]⁺.

¹H NMR (400 MHz, DMSO-d6) δ 7.73 (d, 1H), 6.17 (s, 1H), 6.10-6.06 (m, 1H), 5.68-5.65 (m, 1H), 5.10-4.99 (m, 1H), 4.91-4.40 (m, 4H), 4.04 (t, 2H), 3.61-3.54 (m, 2H), 3.34-3.30 (m, 3H), 3.14-3.01 (m, 3H), 2.82-2.75 (m, 2H), 2.68-2.57 (m, 2H), 2.11-2.06(m, 3H).

### Example 102:

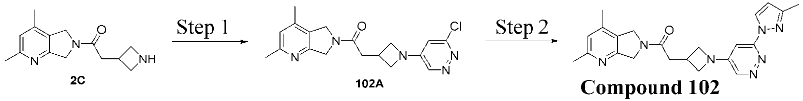

Step 1: 2C (0.7 g, 2.85 mmol), 3,5-dichloropyridazine (0.85 g, 5.71 mmol), and DIPEA (0.92 g, 7.12 mmol) were successively dissolved in isopropanol (20 mL). The mixture was reacted at 80°C overnight. After the reaction was completed, the reaction solution was cooled to room temperature, water (50 mL) was added to the reaction solution, and the resulting mixture was extracted with ethyl acetate (25 mL×3). The organic layers were combined, washed with saturated aqueous sodium chloride solution (30 mL), dried over anhydrous sodium sulphate, and filtered. The filtrate was concentrated and the residue was separated and purified by silica gel column chromatography (dichloromethane : methanol (v/v) = 30 : 1) to obtain **102A** (750 mg, 73%).

LC-MS (ESI): m/z= 358.2 [M+H]⁺.

Step 2: **102A** (0.45 g, 1.26 mmol), 3-methylpyrazole (0.52 g, 6.34 mmol) and sodium hydride (0.2 g, 5.0 mmol, containing 40% mineral oil) were successively dissolved in dry DMF (10 mL). Under nitrogen atmosphere, the mixture was stirred at room temperature for 1 hour, then warmed to 100°C and reacted overnight. After the reaction was completed, the reaction solution was cooled to room temperature, water (30 mL) was added to the reaction solution, and the resulting mixture was extracted with ethyl acetate (20 mL×3). The organic layers were combined, washed with saturated aqueous sodium chloride solution, dried over anhydrous sodium sulphate, and filtered. The filtrate was concentrated and the residue was separated and purified by silica gel column chromatography (dichloromethane : methanol (v/v) = 20 : 1) to obtain **compound 102** (61 mg, 12%).

¹H NMR (400 MHz, CDCl₃) δ 8.61 (d, 1H), 8.25 (t, 1H), 6.93-6.90 (m, 2H), 6.25 (t, 1H), 4.78-4.73 (m, 4H), 4.41-4.36 (m, 2H), 3.89-3.84 (m, 2H), 3.39-3.34 (m, 1H), 2.84-2.80 (m, 2H), 2.53 (s, 3H), 2.37 (s, 3H), 2.27 (d, 3H).

LC-MS (ESI): m/z = 404.4 [M+H]⁺.

### Example 103:

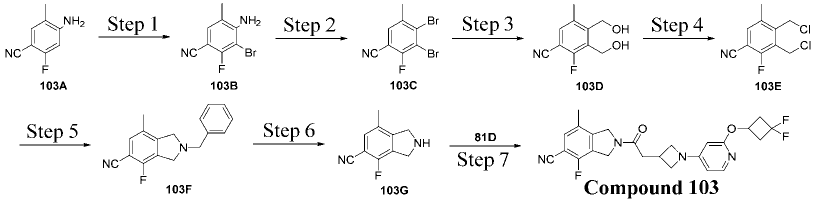

Step 1: Compound **103A** (10.0 g, 66.60 mmol) was dissolved in acetonitrile (100 mL), and N-bromosuccinimide (14.22 g, 79.92 mmol) was added. Subsequently, the mixture was warmed to 80°C and stirred for 16 h. After the reaction was completed, the mixture was cooled to room temperature and concentrated under reduced pressure. To the residue was added water (100 mL), and the resulting mixture was stirred for 5 min and extracted with dichloromethane (100 mL). The organic phase was dried over anhydrous sodium sulphate and concentrated under reduced pressure. The residue was purified by column chromatography (eluents: petroleum ether : ethyl acetate = 3 : 1) to obtain the target compound **103B** (8.0 g, yield: 53%).

LCMS m/z =228.9 [M+H]⁺.

Step 2: Compound **103B** (8.0 g, 34.93 mmol) was dissolved in acetonitrile (100 mL), and copper bromide (11.70 g, 52.32 mmol) and tert-butyl nitrite (10.23 g, 87.33 mmol) were successively added. Subsequently, the mixture was warmed to 80°C and stirred for 16 h. After the reaction was completed, the mixture was cooled to room temperature and concentrated under reduced pressure. To the residue was added water (100 mL), and the resulting mixture was stirred for 5 min and extracted with dichloromethane (100 mL). The organic phase was dried over anhydrous sodium sulphate and concentrated under reduced pressure. The residue was purified by column chromatography (eluents: petroleum ether : ethyl acetate = 5 : 1) to obtain the target compound **103C** (7.5 g, yield: 73%).

LCMS m/z = 291.9 [M+ H]⁺.

Step 3: Compound **103C** (7.5 g, 25.60 mmol) was dissolved in dioxane (80 mL), and (tributylstannyl)methanol (24.66 g, 76.80 mmol) and XPhos-Pd-G2 (2.01 g, 2.56 mmol) were successively added. Subsequently, under nitrogen atmosphere, the mixture was warmed to 90°C and stirred for 12 h. After the reaction was completed, the mixture was cooled to room temperature and concentrated under reduced pressure. The residue was purified by column chromatography (eluents: petroleum ether : ethyl acetate = 1 : 1) to obtain the target compound **103D** (2.0 g, yield: 40%).

¹H NMR (400 MHZ, DMSO-d6) δ7.67 (d, 1H), 5.22 (t, 1H), 5.11 (t, 1H), 4.60-4.62 (m, 4H), 2.38 (s, 3H).

Step 4: Compound **103D** (2.0 g, 10.25 mmol) was dissolved in DCE (100 mL), and thionyl chloride (2.86 g, 22.55 mmol) was added. Subsequently, under nitrogen atmosphere, the mixture was warmed to 80°C and stirred for 2 h. After the reaction was completed, the reaction solution was cooled to room temperature and concentrated under reduced pressure. To the residue was added water (50 mL), and the resulting mixture was stirred for 5 min and extracted with dichloromethane (50 mL). The organic phase was dried over anhydrous sodium sulphate and concentrated under reduced pressure. The residue was purified by column chromatography (eluents: petroleum ether : ethyl acetate = 10 : 1) to obtain the target compound **103E** (2.0 g, yield: 84%).

¹H NMR (400 MHZ, DMSO-d6) δ7.89 (d, 1H), 4.93 (s, 4H), 2.43 (s, 3H).

Step 5: Compound **103E** (2.0 g, 8.62 mmol) was dissolved in acetonitrile (20 mL), and benzylamine (1.85 g, 17.19 mmol) and potassium carbonate (3.57 g, 25.86 mmol) were successively added. Subsequently, under nitrogen atmosphere, the mixture was warmed to 80°C and stirred for 12 h. After the reaction was completed, the reaction solution was cooled to room temperature and concentrated under reduced pressure. To the residue was added water (50 mL), and the resulting mixture was stirred for 5 min and extracted with dichloromethane (50 mL). The organic phase was dried over anhydrous sodium sulphate and concentrated under reduced pressure. The residue was purified by column chromatography (eluents: petroleum ether : ethyl acetate = 5 : 1) to obtain the target compound **103F** (1.5 g, yield: 65%).

LCMS m/z = 267.1 [M+1]⁺.

Step 6: Compound **103F** (1.5 g, 5.63 mmol) was dissolved in chlorobenzene (20 mL), and 4A molecular sieve (1.0 g) was added. The mixture was stirred for 0.5 hours and then 1-chloroethyl chloroformate (1.61 g, 11.26 mmol) was added. Subsequently, under nitrogen atmosphere, the mixture was warmed to 90°C and stirred for 4 h. Methanol (20 mL) was added and the mixture was stirred at 70°C for 2 hours. After the reaction was completed, the reaction mixture was cooled to room temperature and concentrated under reduced pressure. To the residue was added water (50 mL), and the resulting mixture was stirred for 5 min and extracted with dichloromethane/methanol = 10/1 (50 mL). The organic phase was dried over anhydrous sodium sulphate and concentrated under reduced pressure. The residue was purified by column chromatography (eluents: dichloromethane : methanol = 10 : 1) to obtain the target compound **103G** (0.8 g, yield: 80%).

LCMS m/z = 177.2 [M+H]⁺.

Step 7: Compound **103G** (200 mg, 1.14 mmol) and 81D (0.35 g, 1.14 mmol) were dissolved in DMF (10 mL), and triethylamine (0.34 g, 3.39 mmol) and propyl phosphoric anhydride (0.87 g, 1.37 mmol) were successively added. Subsequently, the mixture was stirred at room temperature for 16 h. After the reaction was completed, water (20 mL) was added, and the mixture was stirred for 5 min and extracted with ethyl acetate (20 mL). The organic phase was dried over anhydrous sodium sulphate and concentrated under reduced pressure and the residue was purified by column chromatography (eluents: dichloromethane : methanol = 10 : 1) and further purified by preparative HPLC to obtain compound **103** (80 mg, yield: 15%). Preparative HPLC separation method: Instrument: waters 2767 preparative liquid chromatographic instrument; chromatography column: SunFire@ Prep C18 (19 mm × 250 mm); the sample was dissolved in DMF and filtered with a 0.45 µm filter to prepare a sample solution; preparative chromatography conditions: a. composition of mobile phases A and B: mobile phase A: acetonitrile, and mobile phase B: water (containing 5 mM aqueous ammonia); b. gradient elution, mobile phase A: 30%-70%; c. flow rate: 15 mL/min. d. elution time: 20 min.

LCMS m/z = 457.4 [M+H]⁺.

¹H NMR (400 MHZ, DMSO-d6) δ7.73 (d, 1H), 7.69 (d, 1H), 6.07-6.09 (m, 1H), 5.67 (d, 1H),5.01-5.07 (m, 1H), 4.99 (s,1H), 4.92 (s, 1H), 4.74 (s,1H), 4.69 (s, 1H), 4.05 (t, 2H), 3.58 (t, 2H), 3.02-3.12 (m, 3H), 2.83 (d, 2H), 2.55-2.66 (m, 2H),2.25 (d, 3H).

### Example 104:

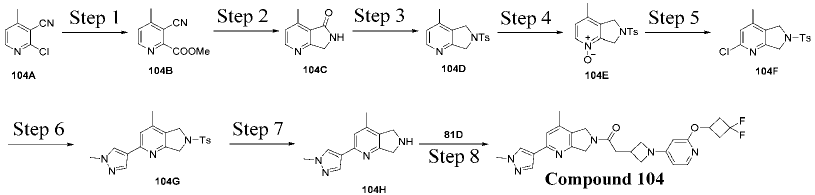

Step 1: With reference to the synthesis method of step 3 in **Example 35,** compound **104B** (1.70 g, 67.01%) was obtained using **104A** (2.2 g, 14.40 mmol) as a starting material.

LC-MS (ESI): m/z = 177.0 [M+H]⁺.

Step 2: With reference to the synthesis method of step 4 in Example 35, compound **104C** (1.30 g, 90.93%) was obtained using **104B** (1.70 g, 9.65 mmol) as a starting material.

LC-MS (ESI): m/z = 149.0 [M+H]⁺.

Step 3: Compound **104C** (1.30 g, 8.77 mmol) was dissolved in tetrahydrofuran (20 mL), and 10 moL/L borane dimethyl sulphide complex (5 mL) was added. Subsequently, the mixture was warmed to 80°C and reacted for 18 hours. The reaction mixture was cooled to room temperature, and concentrated under reduced pressure. To the residue was added 4 mol/L hydrogen chloride methanol solution (30 mL), and the mixture was then warmed to 80°C and reacted for 3 hours. After the reaction was completed, the mixture was cooled to room temperature and concentrated under reduced pressure. Dichloromethane (30 mL) and triethylamine (5 mL) were added. After the mixture was stirred evenly, p-toluenesulphonyl chloride (3.41 g, 17.89 mmoL) was added and then the mixture was reacted at room temperature overnight. After the reaction was completed, the mixture was concentrated under reduced pressure, and the resulting residue was purified using Biotage Isolera One (12 g silica gel column, eluents: 0-30% EA/PE) to obtain compound **104D** (0.56 g, 13.02%).

LC-MS (ESI): m/z = 289.3 [M+H]⁺.

Step 4: In an ice bath, compound **104D** (0.56 g, 1.94 mmol) was dissolved in dichloromethane (20 mL), m-chloroperoxybenzoic acid (0.40 g, 2.31 mmol) was added, and then the mixture was warmed to room temperature, stirred and reacted for 2 hours. Saturated sodium thiosulphate solution (100 mL) was added to the reaction solution to quench the reaction. The mixture was extracted with dichloromethane three times. The combined organic phases were washed with saturated aqueous sodium bicarbonate solution and then washed with saturated aqueous sodium chloride solution, dried over anhydrous sodium sulphate, filtered, and concentrated under reduced pressure. The resulting residue was purified using Biotage Isolera One (12 g silica gel column, eluents: 0-6% MeOH/DCM) to obtain compound **104E** (0.46 g, yield: 77.91%).

LC-MS (ESI): m/z = 305.1 [M+H]⁺.

Step 5: In an ice bath, compound **104E** (0.46 g, 1.94 mmol) was added in portions to phosphorus oxychloride solution (10 mL), and then the mixture was warmed to 100°C, stirred and reacted for 2 hours. After the reaction was completed, the mixture was cooled to room temperature and concentrated under reduced pressure to remove most of phosphorus oxychloride. Then the residue was added dropwise to an ice-cold saturated aqueous sodium bicarbonate solution (50 mL). Subsequently, the mixture was extracted with ethyl acetate three times. The combined organic phases were washed with saturated aqueous sodium chloride solution, dried over anhydrous sodium sulphate, filtered and concentrated under reduced pressure. The resulting residue was purified using Biotage Isolera One (12 g silica gel column, eluents: 0-20% EA/PE) to obtain compound **104F** (0.48 g, yield: 98.47%).

LC-MS (ESI): m/z = 323.0 [M+H]⁺.

Step 6: Compound **104F** (0.13 g, 0.40 mmol), 1-methyl-1H-pyrazole-4-boronic acid pinacol ester (100 mg, 0.48 mmol), Pd(dppf)Cl₂ (29 mg, 0.040 mmol), and potassium carbonate (0.72 g, 0.52 mmol) were successively dissolved in dioxane (10 mL) and water (1 mL), and under nitrogen atmosphere, the mixture was warmed to 90°C, stirred and reacted for 18 hours. After the reaction was completed, the reaction solution was cooled to room temperature, and filtered through diatomaceous earth. Water was added to the filtrate, and the mixture was extracted with ethyl acetate three times. The combined organic phases were washed with saturated aqueous sodium chloride solution, dried over anhydrous sodium sulphate and filtered. The filtrate was concentrated under reduced pressure and the resulting crude product was purified using Biotage Isolera One (12 g silica gel column, eluents: 0-6% MeOH/DCM) to obtain compound **104G** (0.14 g, yield: 95%).

LC-MS (ESI): m/z = 369.1 [M+H]⁺.

Step 7: With reference to the synthesis method of step 9 in Example **98,** compound **104H** (50 mg, 61.41%) was synthesized using **104G** (0.14 g, 0.39 mmol) as a starting material.

LC-MS (ESI): m/z = 215.1 [M+H]⁺.

Step 8: With reference to the synthesis method of step 10 in Example 98, compound **104** (10 mg, 10.11%) was synthesized using **104H** (0.043 g, 0.20 mmol) and **81D** (0.072 g, 0.24 mmol) as starting materials.

LC-MS (ESI): m/z = 495.3 [M+H]⁺.

¹H NMR (400 MHz, CDCl₃): δ 8.10-7.91 (m, 2H), 7.83-7.81 (m, 1H), 7.22-7.20 (m, 1H), 6.04 -6.02 (m, 1H), 5.60 (s, 1H), 5.15 (s, 1H), 4.81-4.80 (m, 2H), 4.77-4.69 (m, 2H), 4.26-4.25 (m, 2H), 3.96-3.95 (m, 3H), 3.74-3.73 (m, 2H), 3.30 (s, 1H), 3.13 (s, 2H), 2.88-2.80 (m, 2H), 2.74-2.71 (m, 2H), 2.32-2.31 (m, 3H).

### Example 105:

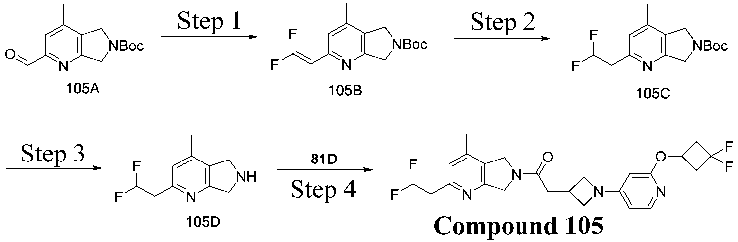

Step 1: Compound **105A** (synthesized with reference to the method described in the patent WO 2018002760) (500 mg, 1.91 mmol) and triphenylphosphine (1.5 g, 5.72 mmol) were successively dissolved in N,N-dimethylformamide (20 mL). The system was purged with nitrogen three times. The mixture was warmed to 100°C, sodium chlorodifluoroacetate (882 mg, 5.72 mmol, dissolved in 5 mL of N,N-dimethylformamide) was slowly added dropwise. After the dropwise addition was completed, the mixture was reacted at 100°C for 2 hours. After the reaction was completed, the reaction solution was cooled to room temperature, poured into saturated aqueous ammonium chloride solution (100 mL), and extracted with ethyl acetate (50 mL×2). The combined organic phases were dried over anhydrous sodium sulphate and filtered. The filtrate was concentrated under reduced pressure and the residue was purified by silica gel column chromatography (petroleum ether : ethyl acetate (v : v) = 5 : 1) to obtain compound **105B** (350 mg, yield: 61.90%).

LC-MS(ESI):m/z = 297.2[M+H]⁺.

Step 2: **105B** (350 mg, 1.18 mmol) was dissolved in ethyl acetate (15 mL), and the system was purged with nitrogen twice. Pd/C catalyst (10% (w/w), 70 mg) was added and the mixture was stirred at room temperature under hydrogen atmosphere overnight. After the reaction was completed, the reaction mixture was filtered and the filtrate was concentrated under reduced pressure. The residue was purified by silica gel column chromatography (petroleum ether : ethyl acetate (v : v) = 5 : 1) to obtain compound **105C** (350 mg, yield: 100%).

LC-MS(ESI):m/z = 299.2[M+H]⁺.

Step 3: Compound **105C** (350 mg, 1.18 mmol) was dissolved in mixed solvents of DCM (6 mL) and TFA (1.5 mL), and the mixture was stirred and reacted at room temperature for 4 h. The reaction system was concentrated to obtain crude trifluoroacetate of compound **105D** (400 mg).

LC-MS(ESI):m/z = 199.1[M+H]⁺.

Step 4: Crude trifluoroacetate of **105D** (400 mg, 1.18 mmol), compound **81D** (351 mg, 1.18 mmol) and N-methylimidazole (387 mg, 4.72 mmol) were successively dissolved in N,N-dimethylformamide (10 mL) and then chloro-N,N,N',N'-tetramethylformamidinium hexafluorophosphate (396 mg, 1.41 mmol) was added. The mixture was reacted at room temperature for 2 hours. After the reaction was completed as monitored by TLC, the mixture was concentrated under reduced pressure. The residue was purified by silica gel column chromatography (dichloromethane : methanol (v : v) = 20 : 1) to obtain **compound 105** (70 mg, yield: 12.41%).

¹H NMR (400 MHz, DMSO-d6) δ7.74-7.72 (m, 1H), 7.14 (s, 1H), 6.52-6.37 (m, 1H), 6.25-6.24 (m, 1H), 5.68-5.67 (m, 1H), 5.08-5.01 (m, 1H), 4.85-4.81 (m, 2H), 4.62-4.58 (m, 2H), 4.07-4.03 (m, 2H), 3.61-3.57 (m, 2H), 3.29-3.27 (m, 2H), 3.13-3.03 (m, 3H), 2.84-2.82 (m, 2H), 2.68-2.61 (m, 2H), 2.27-2.26 (m, 3H).

LC-MS(ESI):m/z = 479.3[M+H]⁺.

### Example 106:

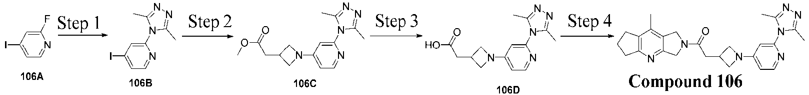

Step 1: With reference to the synthesis method of step 1 in Example 40, compound **106B** was obtained using 3,5-dimethyl-1,2,4-triazole and compound 106A as starting materials.

LC-MS (ESI): m/z = 301.0[M+H]⁺.

Step 2: Compound **106B** (0.7 g, 2.33 mmol), ethyl 2-(azetidin-3-yl)acetate hydrochloride (0.83 g, 2.80 mmol), methanesulphonato(2-dicyclohexylphosphino-2',6'-diisopropoxy-1,1'-biphenyl)(2-amino-1,1'-biphenyl-2-yl)palladium (II) (0.19 g, 0.23 mmol) and caesium carbonate (3.04 g, 9.32 mmol) were successively dissolved in dioxane (10 mL), and under nitrogen atmosphere, the mixture was heated to 100°C and reacted for 16 h. After the reaction was completed, the mixture was cooled to room temperature and filtered. The filtrate was concentrated and the residue was separated and purified by column chromatography (ethyl acetate/petroleum ether = 1/7) to obtain compound **106C** (0.6 g, yield: 85.46%).

LC-MS (ESI): m/z = 302.1[M+H]⁺.

Step 3: With reference to the synthesis method of step 3 in Example 81, compound **106D** was obtained using compound **106C** as a starting material.

LC-MS (ESI): m/z = 288.2[M+H]⁺.

Step 4: With reference to the synthesis method of step 4 in Example 81, compound **106** was obtained using compound **106D** and compound **36G** as starting materials.

¹H NMR (400 MHz, DMSO-*d6*) δ 8.04 (d, 1H),6.63-6.62 (m, 1H), 6.38-6.36 (m, 1H), 4.78 (d, 2H), 4.56 (d, 2H), 4.18-4.14 (m, 2H), 3.702-3.68 (m, 2H), 3.15-3.12 (m, 1H), 2.92 - 2.81 (m, 6H), 2.65 (s, 3H), 2.26 (s, 3H), 2.17-2.16 (m, 3H), 2.10-2.02 (m, 2H).

LC-MS (ESI): m/z = 444.3[M+H]⁺.

### Example 107:

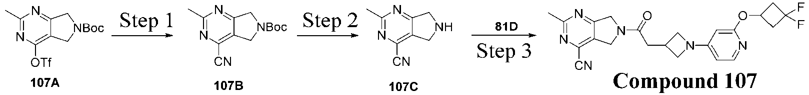

Step 1: **107A** (synthesized according to the patent WO 2018066718) (1.1 g, 2.8 mmol), zinc cyanide (655 mg, 5.6 mmol), and tetrakis(triphenylphosphine)palladium (323 mg, 0.28 mmol) were dissolved in N,N-dimethylformamide (10 mL). After the system was purged with nitrogen, the mixture was warmed to 80°C and reacted overnight. After the reaction was completed, the mixture was concentrated and the resulting residue was purified by silica gel column chromatography (petroleum ether : ethyl acetate (v : v) = 5 : 1) to obtain the target compound **107B** (610 mg, yield: 83.8%).

LC-MS (ESI): m/z = 261.1 [M+H]⁺.

Step 2: **107B** (610 mg, 2.34 mmol) was dissolved in dichloromethane (10 mL), and excess trifluoroacetic acid was added. The mixture was reacted at room temperature overnight. After the reaction was completed, the mixture was concentrated under reduced pressure to obtain the target compound **107C** (537 mg, crude).

LC-MS (ESI): m/z = 161.1[M+H]⁺.

Step 3: Compound **107C** (300 mg, crude), compound **81D** (298 mg, 1 mmol) and N-methylimidazole (328 mg, 4 mmol) were successively dissolved in N,N-dimethylformamide (10 mL). Then chloro-N,N,N',N'-tetramethylformamidinium hexafluorophosphate (336 mg, 1.2 mmol) was added, and the mixture was reacted at room temperature for 1 hour. After the reaction was completed as monitored by TLC, the mixture was concentrated under reduced pressure. The residue was purified by silica gel column chromatography (dichloromethane : methanol (v : v) = 10 : 1) to obtain **compound 107** (71 mg, yield: 16.1%).

LC-MS (ESI): m/z = 441.2[M+H]⁺.

¹H NMR (400 MHz, Chloroform-*d*) δ 7.84-7.76 (m, 1H), 6.06-5.95 (m, 1H), 5.63 (s, 1H), 5.18-5.06 (m, 1H), 5.01-4.92 (m, 2H), 4.88-4.81 (m, 2H), 4.25-4.13 (m, 2H), 3.72-3.60 (m, 2H), 3.31-3.18 (m, 1H), 3.15-3.01 (m, 2H), 2.87-2.59 (m, 7H).

### Example 108:

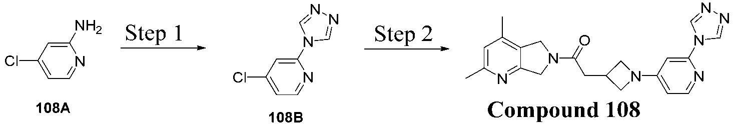

Step 1: **108A** (2.0 g, 15.56 mmol) and diformylhydrazine (2.74 g, 31.12 mmol) were dispersed in DMF (2 mL), and the mixture was reacted at 170°C for 5 hours. After the reaction was completed, the reaction solution was cooled to room temperature, water (30 mL) was added to the reaction solution, and the resulting mixture was extracted with mixed solvents of DCM/*i*-PrOH (20 mL×3). The organic layers were combined, washed with saturated aqueous sodium chloride solution (30 mL), dried over anhydrous sodium sulphate and filtered. The filtrate was concentrated and the residue was separated and purified by silica gel column chromatography (DCM : MeOH (v/v) = 20 : 1) to obtain **108B** (240 mg, 16.6%).

LC-MS (ESI): m/z = 181.1 [M+H]⁺.

Step 2: With reference to the synthesis method of step 2 in Example 29, **compound 108** (35 mg, 20.1%) was obtained using **108B** (100 mg, 0.41 mmol) and compound **2C** (89 mg, 0.49 mmol) as starting materials.

¹H NMR (400 MHz, CDCl₃) δ 8.79 (s, 2H), 8.08-8.07 (m, 1H), 7.06-6.93 (m, 1H), 6.26-6.23 (m, 1H), 6.19-6.18 (m, 1H), 4.95-4.76 (m, 4H), 4.33-4.27 (m, 2H), 3.81-3.77 (m, 2H), 3.36-3.29 (m, 1H), 2.85-2.82 (m, 2H), 2.65-2.56 (m, 3H), 2.36-2.28 (m, 3H).

LC-MS (ESI): m/z = 390.1 [M+H]⁺.

### Example 109:

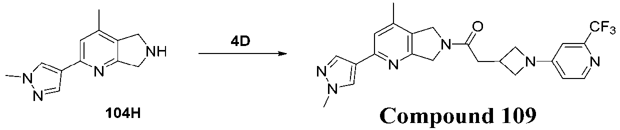

With reference to the synthesis method of step 3 in Example **4,** compound **109** (30 mg, 28.58%) was synthesized using intermediate **104H** (50 mg, 0.23 mmol) and intermediate **4D** (60 mg, 0.23 mmol) as starting materials.

LC-MS (ESI): m/z = 457.5 [M+H]⁺.

¹H NMR (400 MHz, CDCl₃): δ 8.29-8.28 (m, 1H), 7.97-7.92 (m, 2H), 7.26-7.92 (m, 1H), 6.59-6.58 (m, 1H), 6.34-6.32 (m, 1H), 4.88-4.81 (m, 2H), 4.78-4.76 (m, 2H), 4.31-4.26 (m, 2H), 3.96-3.91 (m, 3H), 3.78-3.74 (m, 2H), 3.34-3.31 (m, 1H), 2.84-2.81 (m, 2H), 2.35-2.31 (m, 3H).

### Example 110:

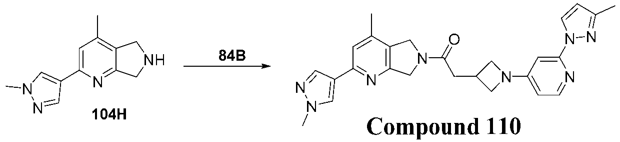

With reference to the synthesis method of step 10 in Example **98,** compound **110** (40 mg, 23.07%) was synthesized using intermediate **104H** (80 mg, 0.37 mmol) and intermediate **84B** (100 mg, 0.37 mmol) as starting materials.

LC-MS (ESI): m/z = 469.4 [M+H]⁺.

¹H NMR (400 MHz, CDCl₃): δ 8.01-8.00 (m, 1H), 7.95-7.92 (m, 2H), 7.26-7.20 (m, 2H), 6.87-6.86 (m, 1H), 6.28 (s, 1H), 6.18-6.16 (m, 1H), 4.83-4.81 (m, 2H), 4.78- 4.76 (m, 2H), 4.42-4.38 (m, 2H), 3.96 (s, 3H), 3.89-3.88 (m, 2H), 3.36-3.31 (m, 1H), 2.86-2.82 (m, 2H), 2.36-2.32 (m, 6H).

### Example 111:

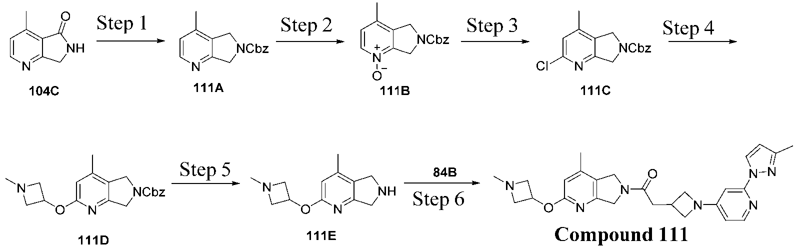

Step 1: With reference to the synthesis method of step 3 in Example 104, compound **111A** (0.75 g, 27.61%) was obtained using **104C** (1.5 g, 10.12 mmol) and benzyl chloroformate (1.9 g, 11.13 mmol) as starting materials.

LC-MS (ESI): m/z = 269.2 [M+H]⁺.

Step 2: With reference to the synthesis method of step 4 in Example 104, compound **111B** (0.78 g, 97.98%) was obtained using **111A** (0.75 g, 2.80 mmol) as a starting material.

LC-MS (ESI): m/z = 285.1 [M+H]⁺.

Step 3: With reference to the synthesis method of step 5 in Example **104,** compound **111C** (0.45 g, 54.25%) was obtained using **111B** (0.78 g, 2.74 mmol) as a starting material.

LC-MS (ESI): m/z = 303.1 [M+H]⁺.

Step 4: Compound **111C** (0.20 g, 0.66 mmol), 1-methylazetidin-3-ol (0.069 g, 0.79 mmol), Pd₂(dba)₃ (0.060 g, 0.066 mmol), BINAP (0.082 g, 0.13 mmol), and caesium carbonate (0.32 g, 0.98 mmol) were successively dissolved in toluene (10 mL). Under nitrogen atmosphere, the mixture was warmed to 90°C, stirred and reacted for 18 hours. After the reaction was completed, the reaction solution was cooled to room temperature and filtered through diatomaceous earth. The filtrate was poured into water and the resulting mixture was extracted with ethyl acetate three times. The combined organic phases were washed with saturated aqueous sodium chloride solution, dried over anhydrous sodium sulphate, and concentrated under reduced pressure to obtain a crude product. The crude product was purified using Biotage Isolera One (12 g silica gel column, eluents: 0-6% MeOH/DCM) to obtain compound **111D** (0.12 g, yield: 51.42%).

LC-MS (ESI): m/z = 354.3 [M+H]⁺.

Step 5: **111D** (0.11 g, 0.31 mmol) was dissolved in methanol (20 mL), 10% palladium on carbon (46 mg) was added, and the mixture was stirred and reacted at room temperature under hydrogen atmosphere for 18 h. The reaction solution was filtered through diatomaceous earth, and the filter cake was washed with ethyl acetate. The filtrate was concentrated to obtain the target compound **111E** (45 mg, 66.20%), which was directly used in the next reaction without further purification.

LC-MS (ESI): m/z = 220.2 [M+H]⁺.

Step 6: With reference to the synthesis method of step 10 in Example **98,** compound **111** (13 mg, 15.25%) was synthesized using **111E** (0.04 g, 0.18 mmol) and **84B** (0.054 g, 0.20 mmol) as starting materials.

LC-MS (ESI): m/z = 495.3 [M+H]⁺.

¹H NMR (400 MHz, CDCl₃): δ 8.39-8.38 (m, 1H), 8.01-7.99 (m, 1H), 6.86-6.85 (m, 1H), 6.50-6.49 (m, 1H), 6.21-6.20 (m, 1H), 6.13-6.11 (m, 1H), 5.29-5.20 (m, 1H), 4.79-4.57 (m, 4H), 4.31-4.26 (m, 2H), 4.06-4.05 (m, 2H), 3.77-3.71 (m, 2H), 3.35-3.26 (m, 3H), 2.89-2.76 (m, 2H), 2.55(s, 3H), 2.37 (s, 3H), 2.22-2.20 (m, 3H).

### Example 112:

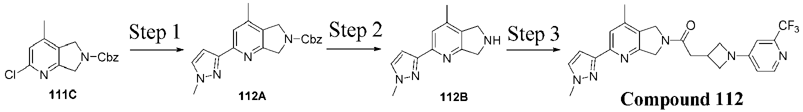

Step 1: **111C** (600 mg, 1.98 mmol), 1-methylpyrazole-3-boronic acid pinacol ester (494 mg, 2.38 mmol), Pd(dppf)Cl₂ (323 mg, 0.40 mmol), and potassium carbonate (684 mg, 4.95 mmol) were successively dissolved in 1,4-dioxane (20 mL) and water (2 mL), and under nitrogen atmosphere, the mixture was warmed to 90°C, stirred and reacted for 18 hours. After the reaction was completed, the reaction solution was cooled to room temperature and filtered through diatomaceous earth. The filtrate was poured into water and the resulting mixture was extracted with ethyl acetate three times. The combined organic phases were washed with saturated aqueous sodium chloride solution, dried over anhydrous sodium sulphate, filtered and concentrated under reduced pressure to obtain a crude product. The crude product was purified using Biotage Isolera One (20 g silica gel column, eluents: 0-75% EA/PE) to obtain **112A** (654 mg, yield: 94.72%).

LC-MS (ESI): m/z = 349.1 [M+H]⁺.

Step 2: **112A** (654 mg, 0.31 mmol) was dissolved in methanol (30 mL), 10% palladium on carbon (100 mg) was added, and the mixture was reacted under hydrogen atmosphere for 18 h. After the reaction was completed, the reaction solution was filtered through diatomaceous earth, and the filter cake was washed with ethyl acetate. The filtrate was concentrated to obtain the target compound **112B** (353 mg), which was directly used in the next reaction without further purification.

Step 3: Intermediate **4D** (90 mg, 0.46 mmol) was dissolved in DMF (10 mL), and TEA (112 mg, 1.11 mmol) and HATU (211 mg, 0.55 mmol) were added. The mixture was stirred at room temperature for 5 minutes. **112B** (80 mg, 0.37 mmol) was added, and the mixture was stirred at room temperature for 1 hour. The reaction solution was poured into water, and the resulting mixture was extracted with ethyl acetate three times. The organic phases were combined, washed with saturated aqueous sodium chloride solution, dried over anhydrous sodium sulphate, and concentrated under reduced pressure to obtain a crude product. The crude product was purified using Biotage Isolera One (12 g silica gel column, eluents: 0-6% MeOH/DCM) to obtain **compound 112** (52 mg, yield: 30.51%).

LC-MS (ESI): m/z = 457.3 [M+H]⁺.

¹H NMR (400 MHz, DMSO-*d6*) δ 8.22-8.18 (m, 1H), 7.75-7.74 (m, 1H), 7.70- 7.67 (m, 1H), 6.77-6.74 (m, 1H), 6.72-6.70 (m, 1H), 6.55-6.52 (m, 1H), 4.90-4.82 (m, 2H), 4.66-4.59 (m, 2H), 4.22-4.15 (m, 2H), 3.90 (s, 3H), 3.75-3.69 (m, 2H), 3.19- 3.10 (m, 1H), 2.90-2.83 (m, 2H), 2.33-2.30 (m, 3H).

### Example 113:

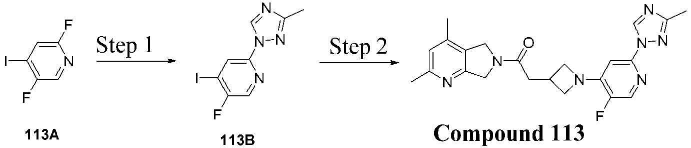

Step 1: With reference to step 1 in Example 26, compound **113B** (0.21 g, 16.6%) was synthesized using **113A** (1.0 g, 4.15 mmol) and 3-methyl-1H-1,2,4-thiazole (0.33 g, 3.94 mmol) as starting materials.

LC-MS (ESI): m/z = 305.0 [M+H]⁺.

Step 2: Compound **2C** (97 mg, 0.40 mmol), compound **113B** (0.10 g, 0.33 mmol), XantPhos (76 mg, 0.13 mmol), Pd₂(dba)₃ (60 mg, 0.066 mmol), and caesium carbonate (0.22 g, 0.66 mmol) were successively dissolved in 1,4-dioxane (10 mL), and under nitrogen atmosphere, the mixture was warmed to 100°C and reacted for 16 hours. After the reaction was completed, the mixture was cooled to room temperature, diluted with ethyl acetate, and filtered through diatomaceous earth. The filtrate was concentrated under reduced pressure to obtain a crude product. The crude product was purified using Biotage Isolera One (12 g silica gel column, eluents: 0-6% MeOH/DCM) to obtain compound **113** (32 mg, yield: 23.1%).

LC-MS (ESI): m/z = 422.3 [M+H]⁺.

¹H NMR (400 MHz, CDCl₃): δ 8.90 (s, 1H), 7.87 (d, 1H), 6.97 - 6.92 (m, 1H), 6.79 - 6.77 (m, 1H), 4.84 - 4.74 (m, 4H), 4.49 - 4.44 (m, 2H), 3.98 - 3.94 (m, 2H), 3.36 - 3.29 (m, 1H), 2.84 - 2.80 (m, 2H), 2.57 - 2.55 (m, 3H), 2.47 (s, 3H), 2.30 - 2.27 (m, 3H).

### Example 114:

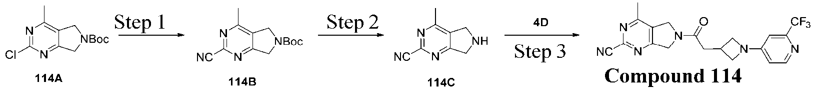

Step 1: Compound **114A** (0.2 g, 0.74 mmol, synthesized with reference to the method described in the patent WO 2020139916) was dissolved in DMF (20 mL), and zinc cyanide (0.17 g, 1.48 mmol) and tetrakis(triphenylphosphine)palladium (0.092 g, 0.08 mmol) were added. Subsequently, under nitrogen atmosphere, the mixture was warmed to 140°C and reacted under microwave for 0.5 hours. After the reaction was completed, the mixture was cooled to room temperature. Saturated aqueous sodium bicarbonate solution (50 mL) was added and the resulting mixture was extracted with ethyl acetate (50 mL× 2). The combined organic phases were washed with saturated aqueous sodium chloride solution (60 mL), dried over anhydrous sodium sulphate and filtered and the filtrate was concentrated. The residue was separated by silica gel column chromatography (PE : EA (v/v) = 15 : 1) to obtain **114B** (0.1 g, 51.81%).

LC-MS (ESI): m/z = 261.2 [M+H]⁺.

Step 2: Compound **114B** (0.1 g, 0.38 mmol) was dissolved in dichloromethane (5 mL), trifluoroacetic acid (2 mL) was added, and the resulting mixture was stirred and reacted at room temperature for 2 hours. After the reaction was completed, saturated aqueous sodium bicarbonate solution (15 mL) was added, and the resulting mixture was extracted with ethyl acetate (15 mL× 2). The combined organic phases were washed with saturated aqueous sodium chloride solution (20 mL), dried over anhydrous sodium sulphate and filtered. The filtrate was concentrated to obtain crude compound **114C** (62 mg), which may be directly used in the next reaction without further purification.

LC-MS (ESI): m/z = 161.1 [M+H]⁺.

Step 3: With reference to the synthesis method of step 7 in Example **4,** compound **114** (35 mg, 27.87%) was synthesized using **114C** (0.05 g, 0.31 mmol) and intermediate **4D** (97 mg, 0.37 mmol) as starting materials.

LC-MS (ESI): m/z = 403.2 [M+H]⁺.

¹H NMR (400 MHz, CDCl₃): δ 8.28-8.24 (m, 1H), 6.58-6.57 (m, 1H), 6.35-6.33 (m, 1H), 4.91-4.85 (m, 4H), 4.34-4.28 (m, 2H), 3.81-3.78 (m, 2H), 3.35-3.31 (m, 1H), 2.95-2.83 (m, 2H), 2.58-2.57 (m, 3H).

### Example 115:

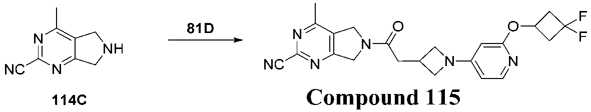

With reference to the synthesis method of step 4 in Example **81,** compound **115** (20 mg, 14.55 %) was synthesized using **114C** (0.05 g, 0.31 mmol) and intermediate **81D** (0.11 g, 0.37 mmol) as starting materials.

LC-MS (ESI): m/z = 441.2 [M+H]⁺.

¹H NMR (400 MHz, CDCl₃): δ 7.74-7.72 (m, 1H), 5.94-5.92 (m, 1H), 5.56-5.55 (m, 1H), 5.07-5.03 (m, 1H), 4.82- 4.78 (m, 4H), 4.14-4.10 (m, 2H), 3.61-3.57 (m, 2H), 3.21-3.16 (m, 1H), 3.07-2.97 (m, 2H), 2.76-2.72 (m, 2H), 2.68-2.56 (m, 2H), 2.51-2.50 (m, 3H).

### Example 116:

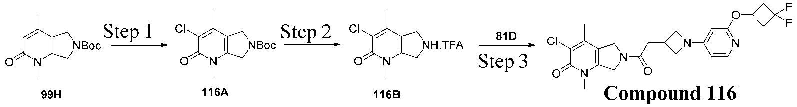

Step 1: **99H** (0.4 g, 1.51 mmol) was dissolved in DMF (5 mL), N-chlorosuccinimide (0.24 g, 1.81 mmol) was added, and the mixture was reacted at room temperature for 16 h. Water (20 mL) was added. The mixture was extracted with ethyl acetate (20 mL×3), the combined organic phases were washed with saturated aqueous sodium chloride solution, dried over anhydrous sodium sulphate and filtered, and the filtrate was concentrated under reduced pressure. The residue was separated and purified by silica gel column chromatography (EA/PE = 4/1) to obtain **116A** (0.2 g, 44.23%).

LC-MS (ESI): m/z = 299.1[M+H]⁺.

Step 2: **116A** (0.1 g, 0.38 mmol) was dissolved in dichloromethane (8 mL), and trifluoroacetic acid (3 mL) was added. The mixture was reacted at room temperature for 1 h. The reaction solution was concentrated under reduced pressure to obtain crude product **116B** (0.2 g), which was directly used in the next reaction.

LC-MS (ESI): m/z = 199.1[M+H]⁺.

Step 3: Crude product **116B** (0.2 g), **81D** (0.11 g, 0.36 mmol), N,N-diisopropylethylamine (0.21 g, 1.65 mmol), and 2-(7-azobenzotriazole)-N,N,N',N'-tetramethyluronium hexafluorophosphate (0.19 g, 0.49 mmol) were successively dissolved in DMF (5 mL), and the mixture was reacted at room temperature for 16 h. Water (30 mL) was added. The mixture was extracted with ethyl acetate (20 mL×3). The combined organic phases were washed with saturated aqueous sodium chloride solution and concentrated under reduced pressure. The resulting crude compound was separated using a C18 reverse-phase column (composition of mobile phases A and B: mobile phase A: acetonitrile; and mobile phase B: water (containing 0.1% aqueous ammonia), (A/B = 45%/55%)) to obtain **compound 116** (0.025 g, 15.71%).

LC-MS (ESI): m/z = 479.2[M+H]⁺.

¹H NMR (400 MHz, DMSO-d6) δ 7.73 (d, 1H), 6.11-6.06 (m, 1H), 5.67 (s, 1H), 5.12-4.99 (m, 1H), 4.93-4.43 (m, 4H), 4.07-4.02 (m, 2H), 3.62-3.55 (m, 2H), 3.45-3.39 (m, 3H), 3.14-3.02 (m, 3H), 2.84-2.75 (m, 2H), 2.67-2.54 (m, 2H), 2.24-2.17 (m, 3H).

### Example 117:

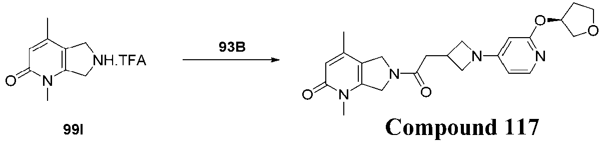

**99I** (0.11 g, 0.38 mmol), **93B** (0.11 g, 0.38 mmol), N,N-diisopropylethylamine (0.25 g, 1.9 mmol), and 2-(7-azobenzotriazole)-N,N,N',N'-tetramethyluronium hexafluorophosphate (0.22 g, 0.57 mmol) were successively dissolved in DMF (5 mL), and the mixture was reacted at room temperature for 16 h. Water (30 mL) was added. The mixture was extracted with ethyl acetate (30 mL×3). The combined organic phases were washed with saturated aqueous sodium chloride solution and concentrated under reduced pressure. The resulting crude compound was purified using a C18 reverse-phase column (composition of mobile phases A and B: mobile phase A: acetonitrile; and mobile phase B: water (containing 0.1% aqueous ammonia), (A/B = 45%/55%) to obtain **compound 117** (0.05 g, 29.79%).

LC-MS (ESI): m/z = 425.2[M+H]⁺.

¹H NMR (400 MHz, DMSO-d6) δ 7.73 (d, 1H), 6.17 (s, 1H), 6.07-6.02 (m, 1H), 5.63 (s, 1H), 5.46-5.40 (m, 1H), 4.92-4.37 (m, 4H), 4.09-3.99 (m, 2H), 3.90-3.65 (m, 4H), 3.60-3.51 (m, 2H), 3.31-3.25 (m, 3H), 3.12-2.99 (m, 1H), 2.83-2.72 (m, 2H), 2.22-2.11 (m, 1H), 2.11-2.04 (m, 3H), 1.97-1.88 (m, 1H).

### Example 118:

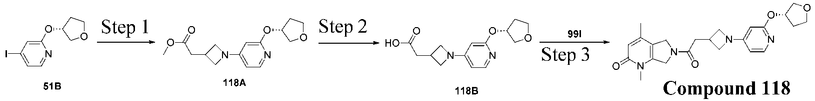

Step 1: **51B** (350 mg, 1.20 mmol), methyl 2-(azetidin-3-yl)acetate 2,2,2-trifluoroacetate (350 mg, 1.44 mmol), caesium carbonate (1.17 g, 3.59 mmol) and RuPhos Pd G3 catalyst (100 mg, 0.12 mmol) were successively dissolved in dioxane (15 mL), and under nitrogen atmosphere, the mixture was warmed to 100°C, stirred and reacted overnight. After the reaction was completed as monitored by TLC, the reaction system was cooled to room temperature and filtered through diatomaceous earth. The filtrate was concentrated under reduced pressure and the residue was separated and purified by silica gel column chromatography (petroleum ether : ethyl acetate (v/v) = 1 : 1) to obtain compound **118A** (170 mg, yield: 48.46%).

LC-MS (ESI): m/z = 293.3[M+H]⁺.

Step 2: **118A** (170 mg, 0.58 mmol) was dissolved in mixed solvents of tetrahydrofuran (4 mL) and water (1 mL), lithium hydroxide monohydrate (120 mg, 2.9 mmol) was added, and then the mixture was reacted at room temperature for 2 hours. After the reaction was completed, the reaction mixture was concentrated to obtain a lithium salt of **118B** (280 mg, crude).

LC-MS (ESI): m/z = 279.1[M+H]⁺.

Step 3: Lithium salt of compound **118B** (0.2 g, 0.58 mmol), **99I** (0.16 g, 0.58 mmol), N,N-diisopropylethylamine (0.37 g, 2.9 mmol), and 2-(7-azobenzotriazole)-N,N,N',N'-tetramethyluronium hexafluorophosphate (330 mg, 0.87 mmol) were successively dissolved in N,N-dimethylformamide (8 mL), and the mixture was reacted at room temperature for 2 hours. After the reaction was completed, water (30 mL) was added, and the mixture was extracted with ethyl acetate (30 mL×3). The combined organic phases were washed with saturated aqueous sodium chloride solution, dried over anhydrous sodium sulphate, filtered and concentrated under reduced pressure. The residue was purified and separated using a C18 reverse-phase column (composition of mobile phases A and B: mobile phase A: acetonitrile; and mobile phase B: water (containing 0.1% aqueous ammonia), (A/B = 45%/55%)) to obtain **compound 118** (0.05 g, 20.49%).

LC-MS (ESI): m/z = 425.2[M+H]⁺.

¹H NMR (400 MHz, DMSO-d6) δ 7.73 (d, 1H), 6.17 (s, 1H), 6.07-6.02 (m, 1H), 5.63 (s, 1H), 5.46-5.40 (m, 1H), 4.90 - 4.38 (m, 4H), 4.08-3.99 (m, 2H), 3.89-3.77 (m, 2H), 3.75-3.66(m, 2H), 3.60-3.52 (m, 2H), 3.31-3.25 (m, 3H), 3.12-3.01 (m, 1H), 2.82-2.74 (m, 2H), 2.21-2.10 (m, 1H), 2.11-2.05 (m, 3H), 1.96-1.87 (m, 1H).

### Example 119:

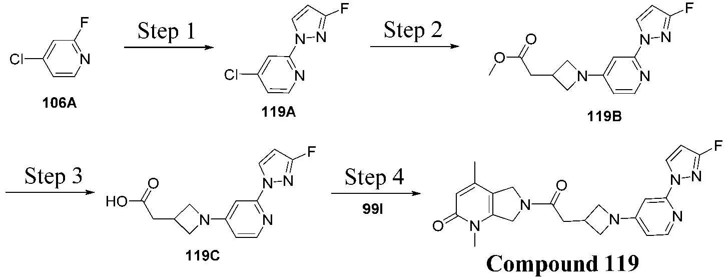

Step 1: 4-chloro-2-fluoropyridine (1 g, 7.63 mmol) and 3-fluoro-1H-pyrazole (0.66 g, 7.63 mmol) were dissolved in dimethyl sulphoxide (10 mL), and potassium carbonate (3.16 g, 22.89 mmol) was added. The mixture was warmed to 45°C and stirred overnight. After the reaction was completed, the mixture was cooled to room temperature, diluted with ethyl acetate and washed with water. The organic phase was dried over anhydrous sodium sulphate and concentrated. The resulting crude product was separated and purified by column chromatography (petroleum ether : ethyl acetate = 5 : 1) to obtain compound **119A** (1.35 g, 90%).

LC-MS (ESI): m/z = 198.1[M+H]⁺.

Steps 2 and 3: With reference to the operation processes of steps 2 and 3 in Example 59, compound **119C** (1.8 g) was obtained using compound **119A** (1.35 g, 6.83 mmol) as a starting material.

Step 4: With reference to the operation process of step 9 in Example 99, **compound 119** (0.1 g, 33%) was obtained using compound **119C** (0.2 g, 0.72 mmol) and compound **99I** (0.1 g, 0.72 mmol) as starting materials.

LC-MS (ESI): m/z = 423.2[M+H]⁺.

¹H NMR (400 MHz, CDCl₃) δ 8.39 (s, 1H), 7.98 (d, 1H), 6.72 (s, 1H), 6.35 (s, 1H), 6.14 (d, 1H), 5.99 (d, 1H), 4.72 (d, 2H), 4.59 (d, 2H), 4.27 (t, 2H), 3.79-3.65 (m, 2H), 3.44 (s, 3H), 3.28 (s, 1H), 2.79 (d, 2H), 2.12 (s, 3H).

### Example 120:

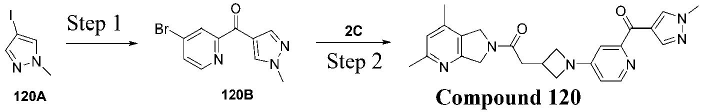

Step 1: Compound **120A** (1.00 g, 4.81 mmol) was dissolved in anhydrous THF (20 mL), and at 0°C, isopropylmagnesium bromide (5.77 mL, 5.77 mmol, 1M THF solution) was added. The mixture was reacted at room temperature for 2 hours. Upon complete depletion of starting materials as monitored by TLC, 4-bromo-N-methoxy-N-methylpicolinamide (1.53 g, 5.77 mmol, synthesized according to the patent WO 2022095904) was added and then the mixture was reacted at room temperature for two hours. Upon complete depletion of starting materials as monitored by TLC, 50 mL of saturated aqueous ammonium chloride solution was added to quench the reaction. The mixture was extracted with 100 mL of ethyl acetate twice. The organic phases were combined and concentrated. The residue was subjected to silica gel column chromatography (petroleum ether : ethyl acetate (v/v) = 3 : 1) to obtain compound **120B** (320 mg, 15%).

¹H NMR (400 MHz, DMSO-d6) δ 8.77 (s, 1H), 8.68-8.63 (m, 1H), 8.23 (d, , 1H), 8.20-8.15 (m, 1H), 7.99-7.93 (m, 1H), 3.94 (s, 3H).

Step 2: Compound **120B** (300 mg, 1.13 mmol), caesium carbonate (1.10 g, 3.38mmol), **2C** (554 mg, 2.26 mmol) and Ruphos Pd G3 (95 mg, 0.11 mmol) were successively dissolved in anhydrous 1,4-dioxane (5 mL), and the mixture was reacted at 90°C for 16 hours. After the reaction was completed, the reaction solution was added dropwise to 15 mL of ice water, and the mixture was extracted with 15 mL of dichloromethane twice. The organic phases were combined and concentrated. The residue was successively separated by silica gel column chromatography (dichloromethane : methanol (v/v) = 15 : 1) and C18 column chromatography (0.1% aqueous NH₄HCO₃ solution : acetonitrile (v/v) = 18% - 48%) to obtain **compound 120** (42 mg, 9%).

LC-MS (ESI): m/z = 431.3 [M+H]⁺.

¹H NMR (400 MHz, DMSO-d6) δ 8.74 (s, 1H), 8.28 (d, 1H), 8.18 (s, 1H), 7.10 - 6.90 (m, 2H), 6.58-6.52 (m, 1H), 4.91-4.71 (m, 2H), 4.60-4.54 (m, 2H), 4.17 (t, 2H), 3.92 (s, 3H), 3.70 (t, 2H), 3.14 (s, 1H), 2.86 (d, 2H), 2.42 (s, 3H), 2.25-2.21 (m, 3H).

### Example 121:

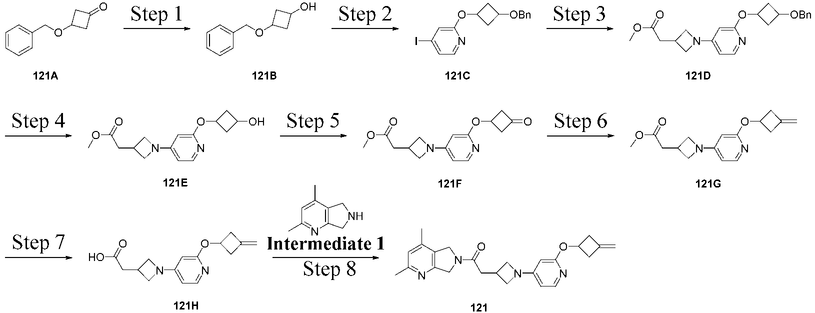

Step 1: 3-(Benzyloxy)-1-cyclobutanone (20.00 g, 114 mmol) was dissolved in methanol (200 mL), and in an ice bath, sodium borohydride (8.59 g, 227 mmol) was added in portions. Subsequently, the mixture was reacted at room temperature for 5 hours. After the reaction was completed, the mixture was concentrated to remove most of methanol, and to the residue were added saturated aqueous ammonium chloride solution (200 mL) and ethyl acetate (200 mL). The resulting mixture was extracted and subjected to phase separation. The organic phase was dried over anhydrous sodium sulphate and then concentrated to obtain the target compound **121B** (20.00 g, 98.87%).

LC-MS (ESI): m/z = 201.1 [M+Na]⁺.

Step 2: Compound **121B** (20.98 g, 118 mmol) was dissolved in tetrahydrofuran (250 mL). Under nitrogen atmosphere, sodium hydride (5.38 g, 135 mmol) was added in portions in an ice bath, and after the mixture was stirred for 1 hour, 2-fluoro-4-iodopyridine (25.00 g, 112 mmol) was added in portions and then the mixture was reacted at room temperature for 2 hours. After the reaction was completed, saturated aqueous sodium chloride solution (300 mL) and ethyl acetate (300 mL) were added, and the mixture was extracted and subjected to phase separation. The organic phase was dried over anhydrous sodium sulphate and concentrated and the residue was separated and purified by silica gel column chromatography (petroleum ether : tetrahydrofuran (v : v) = 0-10%) to obtain compound **121C** (38.00 g, 88.91%).

LC-MS (ESI): m/z = 382.0 [M+H]⁺.

Step 3: Compound **121C** (38.00 g, 100 mmol), caesium carbonate (97.43 g, 299 mmol) and methyl 2-(azetidin-3-yl)acetate trifluoroacetate (25.45 g, 105 mmol) were successively dissolved in 1,4-dioxane (380 mL). Under nitrogen atmosphere, methanesulphonato(2-dicyclohexylphosphino-2',6'-diisopropoxy-1,1'-biphenyl)(2-amino-1,1'-biphenyl-2-yl)palladium (II) (1.67 g, 1.99 mmol) was added and then the mixture was warmed to 110°C and reacted for 5 hours. After the reaction was completed, the reaction solution was cooled to room temperature and filtered through diatomaceous earth. The filter cake was washed with ethyl acetate (500 mL) and the filtrate was washed with saturated aqueous sodium chloride solution three times, dried over anhydrous sodium sulphate and concentrated under reduced pressure. The residue was separated and purified by silica gel column chromatography (tetrahydrofuran : petroleum ether (v : v) = 0-50%) to obtain compound **121D** (36.00 g, 94.43%).

LC-MS (ESI): m/z = 383.2 [M+H]⁺.

Step 4: Compound **121D** (34.00 g, 89 mmol) and 10% Pd/C (6.8 g, 6 mmol) were successively dissolved in methanol (340 mL), and under hydrogen atmosphere, the mixture was warmed to 60°C and reacted overnight. After the reaction was completed, the mixture was filtered, the filter cake was washed with methanol (50 mL), and the filtrate was concentrated under reduced pressure to obtain compound **121E** (25.00 g, 96.20%).

LC-MS (ESI): m/z = 293.1 [M+H]⁺.

Step 5: Compound **121E** (25.00 g, 86 mmol) was dissolved in dichloromethane (250 mL), Dess-Martin periodinane (54.41 g, 128 mmol) was added in portions, and subsequently the mixture was reacted at room temperature overnight. After the reaction was completed, the mixture was concentrated, and to the residue was added ethyl acetate (250 mL). The resulting mixture was stirred for 1 hour and filtered. The filter cake was washed with ethyl acetate and the filtrate was washed with saturated aqueous sodium bicarbonate solution and then washed with saturated aqueous sodium chloride solution, dried over anhydrous sodium sulphate, and concentrated under reduced pressure. The residue was separated and purified by silica gel column chromatography (methanol : dichloromethane (v : v) = 0-10%) to obtain compound **121F** (13.00 g, 52.36%).

LC-MS (ESI): m/z = 293.1 [M+H]⁺.

Step 6: Compound **121F** (700 mg, 2.41 mmol) was dissolved in tetrahydrofuran (20 mL). Under nitrogen atmosphere, chlorobis(5-cyclopentadienyldimethylaluminium-methyltitanium) toluene solution (0.5 mol/L, 7.23 mL) was added and then the mixture was reacted at room temperature for 12 hours. After the reaction was completed, 20% aqueous sodium hydroxide solution (2 mL) was added, and the mixture was filtered. To the filtrate were added ethyl acetate and saturated aqueous sodium chloride solution. The resulting mixture was extracted and subjected to phase separation. The organic phase was dried over anhydrous sodium sulphate and concentrated and the residue was separated and purified by silica gel column chromatography (n-hexane : tetrahydrofuran (v : v) = 0-100%) to obtain the title compound **121G** (60 mg, 8.63%).

LC-MS (ESI): m/z = 289.0 [M+H]⁺.

Step 7: Compound **121G** (60 mg, 0.21 mmol) was dissolved in tetrahydrofuran (3 mL), and lithium hydroxide (7.5 mg, 0.32 mmol), methanol (0.2 mL) and water (0.5 mL) were successively added. Subsequently, the mixture was warmed to 45°C and reacted for 4 hours. After the reaction was completed, the mixture was concentrated under reduced pressure to obtain compound **121H** (70 mg, crude).

LC-MS (ESI): m/z = 275.2 [M+H]⁺.

Step 8: **Intermediate 1** (35 mg, 0.19 mmol), compound **121H** (57 mg, 0.21 mmol), N,N-diisopropylethylamine (39 mg, 0.47 mmol), and 2-(7-azobenzotriazole)-N,N,N',N'-tetramethyluronium hexafluorophosphate (64 mg, 0.23 mmol) were successively dissolved in N,N-dimethylformamide (5 mL), and the mixture was reacted at room temperature for 2 hours. After the reaction was completed, the mixture was filtered, and the filtrate was separated and purified using a reverse-phase preparative column (C18 spherical 20-35 nm 100A 80 g) (acetonitrile: water (0.1% TFA) = 10%-80%) to obtain **compound 121** (34 mg, 44.43%).

¹H NMR (400 MHz, Chloroform-d) δ 7.27-7.25 (m, 3H), 6.91-6.90 (m, 1H), 5.58-5.56 (m, 1H), 4.94-4.92 (m, 2H), 4.77-4.72 (m, 4H), 4.21-4.16 (m, 2H), 3.69-3.65 (m, 2H), 3.33-3.09 (m, 3H), 2.95-2.82 (m, 2H), 2.80-2.76 (m, 2H), 2.53-2.52 (m, 3H), 2.26 (s, 3H).

LC-MS (ESI): m/z = 405.1 [M+H]⁺.

### Example 122:

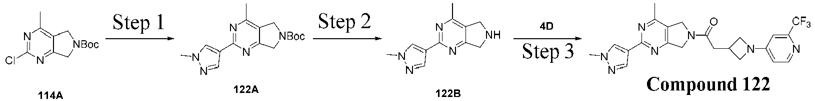

Step 1: With reference to the synthesis method of step 6 in Example 104, compound **122A** (0.2 g, 85.53%) was obtained using compound **114A** (0.2 g, 0.74 mmol) and 1-methyl-1H-pyrazole-4-boronic acid pinacol ester (0.185 g, 0.88 mmol) as starting materials.

LC-MS (ESI): m/z = 316.1 [M+H]⁺.

Step 2: With reference to the synthesis method of step 2 in Example 114, compound **122B** (0.13 g, 95.23%) was obtained using **122A** (0.2 g, 0.63 mmol) as a starting material.

LC-MS (ESI): m/z =216.1 [M+H]⁺.

Step 3: With reference to the synthesis method of step 7 in Example 4, **compound 122** (35 mg, 27.45%) was obtained using **122B** (0.06 g, 0.28 mmol) and intermediate **4D** (86 mg, 0.33 mmol) as starting materials.

LC-MS (ESI): m/z = 458.1 [M+H]⁺.

¹H NMR (400 MHz, CDCl₃): δ 8.29-8.27 (m, 1H), 8.18-8.16 (m, 1H), 8.12-8.10 (m, 1H), 6.59 -6.58 (m, 1H), 6.37-6.35 (m, 1H), 4.80-4.77 (m, 4H), 4.35-4.30 (m, 2H), 3.97 (s, 3H), 3.84-3.80 (m, 2H), 3.35-3.31 (m, 1H), 2.87-2.82 (m, 2H), 2.49-2.48 (m, 3H).

### Example 123:

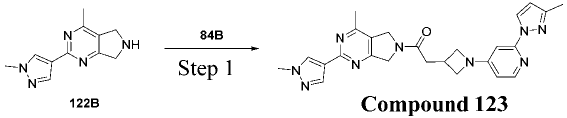

With reference to the synthesis method of step 3 in Example **84, compound 123** (6 mg, 9.17%) was obtained using **122B** (0.03 g, 0.14 mmol) and **84B** (0.05 g, 0.18 mmol) as starting materials.

LC-MS (ESI): m/z= 470.1 [M+H]⁺.

¹H NMR (400 MHz, CDCl₃) δ 8.11-8.09 (m, 1H), 8.03-8.01 (m, 1H), 7.94-7.92 (m, 1H), 6.82- 6.80 (m, 1H), 6.27-6.26 (m, 1H), 6.13-6.12 (m, 1H), 4.74-4.70 (m, 4H), 4.43-4.41 (m, 2H), 3.91-3.89 (m, 5H), 3.34-3.33 (m, 1H), 2.79-2.77 (m, 3H), 2.42-2.41 (m, 3H), 2.28 (s, 3H).

### Example 124:

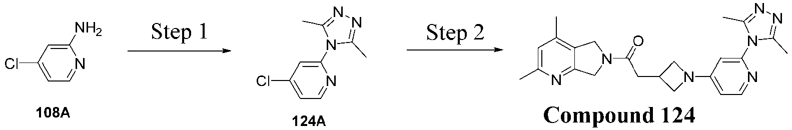

Step 1: **108A** (2.0 g, 15.56 mmol) and 1,1-dimethoxy-N,N-dimethylethylamine (18.05 g, 140.04 mmol) were added to a reaction flask, and the mixture was warmed to 130°C, reacted for 2 hours and then cooled to 0°C. Concentrated hydrochloric acid was added dropwise to adjust the mixture to pH 7-8. To the reaction flask were successively added acethydrazide (6.92 g, 93.36 mmol) and N,N-dimethylacetamide (15 mL). The mixture was stirred for 30 minutes, then warmed to 140°C and reacted for 3 hours. After the reaction was completed, the reaction solution was cooled to room temperature, and to the reaction solution was added 60 mL of water. The mixture was extracted with mixed solvents of DCM/i-PrOH (20 mL×3). The organic layers were combined, washed with saturated aqueous sodium chloride solution (30 mL), dried over anhydrous sodium sulphate and filtered. The filtrate was concentrated and the residue was separated and purified by silica gel column chromatography (DCM : MeOH (v/v)= 20 : 1) to obtain **124A** (710 mg, 21.8%).

LC-MS (ESI): m/z = 209.0 [M+H]⁺.

Step 2: With reference to the synthesis method of step 2 in Example 29, **compound 124** (141 mg, 27.6%) was obtained using **124A** (310 mg, 1.46 mmol) and compound **2C** (300 mg, 1.22 mmol) as starting materials.

¹H NMR (400 MHz, CDCl₃) δ 8.18 (d, 1H), 6.93 (d, 1H), 6.34-6.32 (m, 1H), 6.09-6.08 (m, 1H), 4.79-4.73 (m, 4H), 4.31-4.26 (m, 2H), 3.80 -3.76 (m, 2H), 3.37-3.32 (m, 1H), 2.85-2.81 (m, 2H), 2.54 (s, 3H), 2.53 (s,6H), 2.37-2.26 (m, 3H).

LC-MS (ESI): m/z = 418.7 [M+H]⁺.

### Example 125:

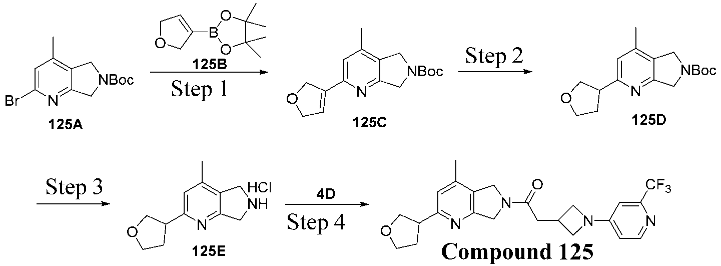

Step 1: With reference to the procedures of step 6 in Example 98, **125C** (434 mg, 83%) was synthesized using **125A** (540 mg, 1.73 mmol) and **125B** (338 mg, 1.73 mmol) as starting materials.

LC-MS (ESI): m/z = 303.2 [M+H]⁺.

Step 2: With reference to the procedures of step 8 in Example 98, **125D** (390 mg, 89%) was synthesized using **125C** (434 mg, 1.44 mmol) as a starting material.

LC-MS (ESI): m/z = 305.5 [M+H]⁺.

Step 3: **125D** (390 mg, 1.28 mmol) was dissolved in dichloromethane (6 mL), and hydrogen chloride 1,4-dioxane solution (4 M, 2 mL) was added. Subsequently, the mixture was reacted at room temperature for 2 h. After the completion of the reaction, the mixture was concentrated under reduced pressure to remove the solvent and then dried to obtain crude product **125E** (280 mg, 91%), which was directly used in the next reaction without further purification.

LC-MS (ESI): m/z = 205.2 [M+H]⁺.

Step 4: With reference to the procedures of step 7 in Example 4, **compound 125** (39 mg, 21%) was synthesized using **125E** (100 mg, 0.42 mmol) and **4D** (108 mg, 0.42 mmol) as starting materials.

LC-MS (ESI): m/z = 447.7 [M+H]⁺.

¹H NMR (400 MHz, CDCl₃) δ 8.30-8.28 (m, 1H), 7.03-6.97 (m, 1H), 6.59-6.57 (m, 1H), 6.36-6.33 (m, 1H), 4.85-4.74 (m, 2H), 4.75 (s, 2H), 4.33-4.26 (m, 2H), 4.17-4.05 (m, 2H), 3.96-3.86 (m, 2H), 3.80-3.76 (m, 2H), 3.67-3.56 (m, 1H), 3.37-3.30 (m, 1H), 2.85-2.80 (m, 2H), 2.44-2.35 (m, 1H), 2.32-2.28 (m, 3H), 2.21-2.14 (m, 1H).

### Example 126:

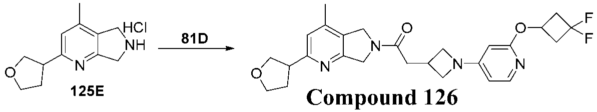

With reference to the procedures of step 4 in Example 81, **compound 126** (44 mg, 18%) was synthesized using **125E** (120 mg, 0.50 mmol) and **81D** (149 mg, 0.50 mmol) as starting materials.

LC-MS (ESI): m/z = 485.8 [M+H]⁺.

¹H NMR (400 MHz, CDCl₃) δ 7.80-7.78 (m, 1H), 6.96 (s, 1H), 6.01-5.98 (m, 1H), 5.64-5.62 (m, 1H), 5.16-5.06 (m, 1H), 4.80-4.70 (m, 4H), 4.22-4.11 (m, 3H), 4.11-4.04 (m, 1H), 3.96-3.85 (m, 2H), 3.68-3.63 (m, 2H), 3.60-3.50 m, 1H), 3.31-3.20 (m, 1H), 3.13-3.02 (m, 2H), 2.82-2.77 (m, 2H), 2.76-2.61 (m, 2H), 2.41-2.31 (m, 1H), 2.29-2.27 (m, 3H), 2.23-2.13 (m, 1H).

### Example 127:

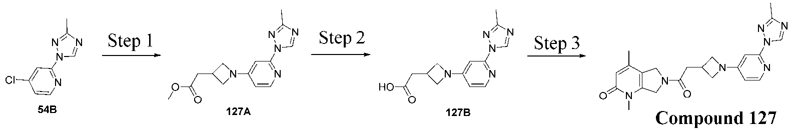

Step 1: **54B** (1.1 g, 5.65 mmol), methyl 2-(azetidin-3-yl)acetate 2,2,2-trifluoroacetate (2.06 g, 8.48 mmol), caesium carbonate (5.52g, 16.95 mmol) and RuPhos Pd G3 catalyst (470 mg, 0.57 mmol) were successively dissolved in dioxane (30 mL), and under nitrogen atmosphere, the mixture was warmed to 100°C, stirred and reacted overnight. After the reaction was completed as monitored by TLC, the reaction system was cooled to room temperature and filtered through diatomaceous earth. The filtrate was concentrated under reduced pressure and the residue was separated and purified by silica gel column chromatography (petroleum ether : ethyl acetate (v/v) = 1 : 1) to obtain compound **127A** (1 g, yield: 61.58%).

LC-MS (ESI): m/z = 288.1[M+H]⁺.

Step 2: **127A** (0.16 g, 0.55 mmol) was dissolved in mixed solvents of tetrahydrofuran (4 mL) and water (1 mL). Lithium hydroxide monohydrate (120 mg, 2.75 mmol) was added and the mixture was reacted at room temperature for 2 hours. After the reaction was completed, the reaction solution was concentrated to obtain a lithium salt of **127B** (280 mg, crude).

LC-MS (ESI): m/z = 274.1[M+H]⁺.

Step 3: **99I** (0.13 g, 0.45 mmol), **127B** (280 mg, crude), N,N-diisopropylethylamine (0.29 g, 2.25 mmol), and 2-(7-azobenzotriazole)-N,N,N',N'-tetramethyluronium hexafluorophosphate (0.26 g, 0.68 mmol) were successively dissolved in DMF (5 mL), and the mixture was reacted at room temperature for 16 h. Water (30 mL) was added, and the mixture was extracted with ethyl acetate (20 mL×3). The combined organic phases were washed with saturated aqueous sodium chloride solution and concentrated under reduced pressure. The residue was purified and separated using a C18 reverse-phase column (composition of mobile phases A and B: mobile phase A: acetonitrile; and mobile phase B: water (containing 0.1% aqueous ammonia), (A/B = 45%/55%)) to obtain **compound 127** (0.05 g, yield: 25.51%).

LC-MS (ESI): m/z = 420.2[M+H]⁺.

¹H NMR (400 MHz, DMSO-d6) δ 9.08 (s, 1H), 8.02-7.99 (m, 1H), 6.66-6.62 (m, 1H), 6.38-6.34 (m, 1H), 6.17 (s, 1H), 4.92-4.40 (m, 4H), 4.22-4.14 (m, 2H), 3.76-3.67 (m, 2H), 3.34-3.30 (m, 3H), 3.20-3.07 (m, 1H), 2.85-2.79 (m, 2H), 2.36 (s, 3H), 2.12-2.06 (m, 3H).

### Example 128:

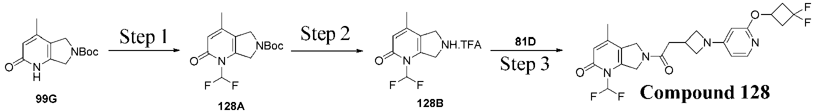

Step 1: **99G** (0.6 g, 2.40 mmol) was dissolved in tetrahydrofuran (30 mL), sodium tert-butoxide (0.92 g, 9.6 mmol) was added, and the mixture was stirred at room temperature for 30 min. Then trimethyl(bromodifluoromethyl)silane (0.97 g, 4.8 mmol) was added dropwise. After the dropwise addition was completed, the mixture was reacted at room temperature for another 2 h. Water (30 mL) was added. The mixture was extracted with ethyl acetate (20 mL × 3), the combined organic phases were washed with saturated aqueous sodium chloride solution, dried over anhydrous sodium sulphate, and filtered, and the filtrate was concentrated under reduced pressure. Then the residue was separated and purified by silica gel column chromatography (EA/PE = 1/2) to obtain **128A** (0.1 g, 13.89%).

LC-MS (ESI): m/z = 301.2[M+H]⁺.

Step 2: **128A** (0.1 g, 0.33 mmol) was dissolved in dichloromethane (8 mL), and trifluoroacetic acid (3 mL) was added. The mixture was reacted at room temperature for 1 h. The reaction solution was concentrated under reduced pressure to obtain crude product **128B** (0.2 g), which was directly used in the next reaction without further purification.

LC-MS (ESI): m/z = 201.1[M+H]⁺.

Step 3: Crude product **128B** (0.2 g), **81D** (0.12 g, 0.40 mmol), N,N-diisopropylethylamine (0.21 g, 1.65 mmol), and 2-(7-azobenzotriazole)-N,N,N',N'-tetramethyluronium hexafluorophosphate (0.19 g, 0.49 mmol) were successively dissolved in DMF (5 mL), and the mixture was reacted at room temperature for 16 h. Water (30 mL) was added, and the mixture was extracted with ethyl acetate (20 mL×3). The combined organic phases were washed with saturated aqueous sodium chloride solution and concentrated under reduced pressure. The residue was purified and separated using a C18 reverse-phase column (composition of mobile phases A and B: mobile phase A: acetonitrile; and mobile phase B: water (containing 0.1% aqueous ammonia), (A/B = 45%/55%)) to obtain **compound 128** (0.06 g, 39.24%).

LC-MS (ESI): m/z = 481.2[M+H]⁺.

¹H NMR (400 MHz, DMSO-d6) δ 8.12-7.79 (m, 1H), 7.75-7.71 (m, 1H), 6.36-6.29 (m, 1H), 6.11-6.05 (m, 1H), 5.69-5.63 (m, 1H), 5.10-4.99 m, 1H), 4.96-4.36 (m, 4H), 4.09-3.99 (m, 2H), 3.61-3.52 (m, 2H), 3.11-2.98 (m, 3H), 2.85-2.76 (m, 2H), 2.69-2.56 (m, 2H), 2.17-2.11 (m, 3H).

### Example 129:

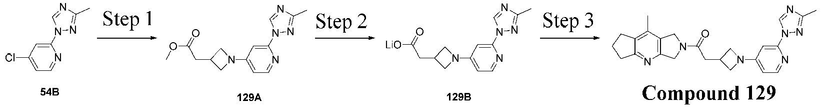

Step 1: Compound **54B** (2.8 g, 14.38 mmol), methyl 2-(azetidin-3-yl)acetate (2.79 g, 21.57 mmol) and caesium fluoride (2.84 g, 18.69 mmol) were dissolved in DMSO (30 mL), triethylamine (6 mL, 43.14 mmol) was added, and then the mixture was warmed to 100°C and reacted for 16 hours. After the reaction was completed as monitored by TLC, the reaction solution was cooled to room temperature, then diluted with water (150 ml), and extracted with ethyl acetate (100 mL×3). The combined organic phases were dried over anhydrous sodium sulphate and concentrated under reduced pressure. The residue was purified by silica gel column chromatography (PE : EA = 1 : 2) to obtain compound **129A** (3.0 g, yield: 72.64%).

LC-MS(ESI):m/z = 288.2[M+H]⁺.

Step 2: Compound **129A** (3.0 g, 10.44 mmol) was dissolved in mixed solvents of THF (30 mL), MeOH (10 mL) and H₂O (10 mL). Lithium hydroxide (0.37 g, 15.66 mmol) was added and the resulting mixture was reacted at room temperature for 4 hours. After the reaction was completed as monitored by TLC, the reaction mixture was concentrated under reduced pressure to obtain crude compound **129B** (3.3 g).

LC-MS(ESI):m/z = 274.1[M+H]⁺.

Step 3: Compound **129B** (280 mg, 1.0 mmol), **36G** (175 mg, 1.0 mmol) and N-methylimidazole (0.33 mL, 4.0 mmol) were successively dissolved in DMF (5 mL), TCHF (340 mg, 1.2 mmol) was added, and the mixture was reacted at room temperature for 1 hour. After the reaction was completed as monitored by TLC, the reaction mixture was diluted with water (20 mL). A white solid was precipitated and filtered. The filter cake was collected, dried and further purified by silica gel column chromatography (DCM : MeOH = 10 : 1) to obtain **compound 129** (60 mg, yield: 13.97%).

¹H NMR (400 MHz, DMSO-d6) δ 9.08 (s, 1H), 8.03-7.98 (m, 1H), 6.66-6.20 (m, 1H), 6.38- 6.34 (m, 1H), 4.84-4.74 (m, 2H), 4.60-4.51 (m, 2H), 4.22-4.15 (m, 2H), 3.76-3.69 (m, 2H), 3.17- 3.08 (m, 1H), 2.92-2.80 (m, 6H), 2.36 (s, 3H), 2.19-2.14 (m, 3H), 2.11-2.02 (m, 2H).

LC-MS(ESI):m/z = 430.3[M+H]⁺.

### Example 130:

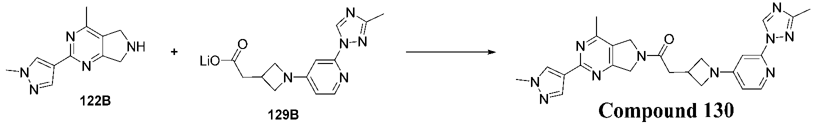

With reference to the synthesis method of step 3 in Example 84, **compound 130** (6 mg, 5.92%) was obtained using **122B** (0.1 g, 0.46 mmol) and intermediate **129B** (0.13 g, 0.46 mmol) as starting materials.

LC-MS (ESI): m/z = 471.2 [M+H]⁺.

¹H NMR (400 MHz, CDCl₃): δ 9.22 (s, 1H), 8.18-8.15 (m, 1H), 8.11-8.09 (m, 1H), 8.03-8.02 (m, 1H), 6.78-6.77 (m, 1H), 6.23-6.21 (m, 1H), 4.80-4.77 (m, 4H), 4.38-4.34 (m, 2H), 3.96 (s, 3H), 3.85-3.83 (m, 2H), 3.36-3.33 (m, 1H), 2.86-2.82 (m, 2H), 2.49-2.48 (m, 6H).

### Example 131:

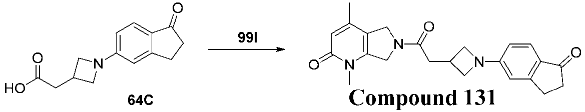

With reference to the synthesis method of step 9 in Example 99, **compound 131** (45 mg, 28.1%) was obtained using **64C** (100 mg, 0.41 mmol) and **99I** (107 mg, 0.41 mmol) as starting materials.

LC-MS (ESI): m/z =392.2 [M+H]⁺.

¹H NMR (400 MHz, DMSO-*d*₆) δ 7.46-7.33 (m, 1H), 6.42-6.29 (m, 2H), 6.17 (s, 1H), 4.88 (s, 1H), 4.65 (s, 2H), 4.39 (s, 1H), 4.21-4.05 (m, 2H), 3.73-3.62 (m, 2H), 3.33-3.31 (m, 3H), 3.17-3.01 (m, 1H), 2.99-2.87 (m, 2H), 2.87-2.75 (m, 2H), 2.49-2.46 (m, 2H), 2.12-2.05 (m, 3H).

### Example 132:

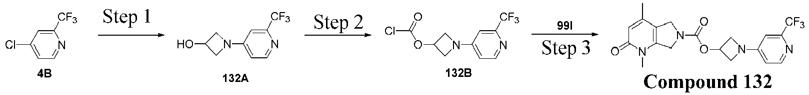

Step 1: At room temperature, compound **4B** (1.0 g, 5.51 mmol) was dissolved in dimethyl sulphoxide (10 mL), and azetidin-3-ol (0.48 g, 6.61 mmol), N,N-diisopropylethylamine (1.42 g, 11.02 mmol) and caesium fluoride (1.68 g, 11.02 mmol) were successively added. Subsequently, the mixture was warmed to 110°C and reacted for 3 h. After the reaction was completed, the reaction mixture was cooled to room temperature, water (20 mL) was added and the resulting mixture was extracted with ethyl acetate (20 mL×3). The combined organic phases were washed with saturated aqueous sodium chloride solution (20 mL), dried over anhydrous sodium sulphate and filtered. The filtrate was concentrated and the residue was separated and purified by silica gel column chromatography (dichloromethane : methanol (v/v) = 19 : 1) to obtain compound **132A** (1.0 g, 83%).

LC-MS (ESI): m/z = 219.2 [M+H]⁺.

Step 2: At room temperature, compound **132A** (0.2 g, 0.92 mmol) was dissolved in tetrahydrofuran (5.0 mL), and triphosgene (0.41 g, 1.38 mmol) and N,N-diisopropylethylamine (0.36 g, 2.76 mmol) were added in an ice bath. Subsequently, the mixture was stirred at room temperature for 2 h. After the reaction was completed, the reaction mixture was filtered and the filtrate was concentrated under reduced pressure to obtain crude compound **132C** (0.23 g), which was directly used in the next reaction.

LC-MS (ESI): m/z = 281.0 [M+H]⁺.

Step 3: At room temperature, compound **132B** (0.23 g) was dissolved in dichloromethane (3.0 mL), and then **99I** (0.12 g, 0.74 mmol) and triethylamine (0.23 g, 2.22 mmol) were added. Subsequently, the mixture was reacted at room temperature for 16 h. After the reaction was completed, the reaction mixture was filtered and the filtrate was concentrated under reduced pressure. The residue was purified and separated using a C18 reverse-phase column (composition of mobile phases A and B: mobile phase A: acetonitrile; and mobile phase B: water (containing 0.1% aqueous ammonia), (A/B = 45%/55%)) to obtain **compound 132** (0.06 g, 20%).

LC-MS (ESI): m/z = 409.2 [M+H]⁺.

¹H NMR (400 MHz, CDCl₃) δ 8.32 (d, 1H), 6.63 (d, 1H), 6.39 (dd, 1H), 6.36-6.31 (m, 1H), 5.49 -5.42 (m, 1H), 4.71-4.65 (m, 2H), 4.57-4.53 (m, 2H), 4.45-4.39 (m, 2H), 4.09-4.02 (m, 2H), 3.45- 3.39 (m, 3H), 2.14-2.09 (m, 3H).

### Example 133:

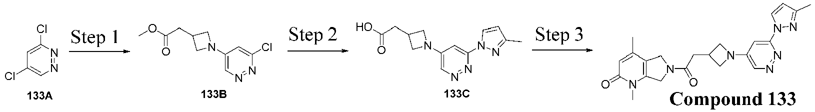

Step 1: Methyl 2-(azetidin-3-yl)acetate 2,2,2-trifluoroacetate (3.9 g, 16.11 mmol), **133A** (3 g, 20.14 mmol), and DIPEA (7.81 g, 60.42 mmol) were successively dissolved in isopropanol (50 mL), and the mixture was reacted at 80°C overnight. After the reaction was completed, the reaction solution was cooled to room temperature, water (100 mL) was added to the reaction solution, and the resulting mixture was extracted with ethyl acetate (50 mL×3). The organic layers were combined, washed with saturated sodium chloride aqueous solution (50 mL), dried over anhydrous sodium sulphate and filtered and the filtrate was concentrated. The residue was separated and purified by silica gel column chromatography (petroleum ether : ethyl acetate (v/v) = 1 : 1) to obtain **133B** (2.5 g, 51%).

LC-MS (ESI): m/z = 242.1[M+H]⁺.

Step 2: **133B** (0.8 g, 3.31 mmol), 3-methylpyrazole (1.09 g, 13.24 mmol) and sodium hydride (0.53 g, 13.24 mmol, containing 40% mineral oil) were successively dissolved in dry DMF (10 mL). Under nitrogen atmosphere, the mixture was stirred at room temperature for 1 hour, then warmed to 100°C and reacted overnight. After the reaction was completed, the reaction solution was cooled to room temperature, water (30 mL) was added to the reaction solution, and the resulting mixture was extracted with ethyl acetate (20 mL×3). The organic layers were combined, washed with saturated aqueous sodium chloride solution, dried over anhydrous sodium sulphate, and filtered. The filtrate was concentrated and the residue was separated and purified by silica gel column chromatography (dichloromethane : methanol (v/v) = 7 : 1) to obtain **133C** (300 mg, 33%).

LC-MS (ESI): m/z = 274.1[M+H]⁺.

Step 3: **99I** (0.1 g, 0.36 mmol), **133C** (0.12 g, 0.43 mmol), N,N-diisopropylethylamine (0.19 g, 1.44 mmol), and 2-(7-azobenzotriazole)-N,N,N',N'-tetramethyluronium hexafluorophosphate (0.21 g, 0.54 mmol) were successively dissolved in DMF (5 mL), and the mixture was reacted at room temperature for 16 h. Water (30 mL) was added, and the mixture was extracted with ethyl acetate (20 mL×3). The combined organic phases were washed with saturated aqueous sodium chloride solution and concentrated under reduced pressure. The residue was purified and separated using a C18 reverse-phase column (composition of mobile phases A and B: mobile phase A: acetonitrile; and mobile phase B: water (containing 0.1% TFA), (A/B = 45%/55%)) to obtain **compound 133** (15 mg, 10 %).

LC-MS (ESI): m/z = 420.2[M+H]⁺.

¹H NMR (400 MHz, DMSO-d6) δ 8.58-8.56 (m, 1H), 8.41-8.38 (m, 1H), 6.80-6.76 (m, 1H), 6.38 (d, 1H), 6.17 (s, 1H), 4.92-4.41 (m, 4H), 4.34-4.23 (m, 2H), 3.85-3.77 (m, 2H), 3.35-3.30 (m, 3H), 3.21-3.12 (m, 1H), 2.87-2.81 (m, 2H), 2.29 (s, 3H), 2.11-2.06 (m, 3H).

### Example 134:

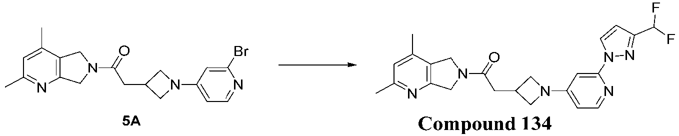

With reference to the synthesis method of step 1 in Example 76, **compound 134** (80 mg, 36.61%) was obtained using **5A** (0.2 g, 0.5 mmol) and 3-(difluoromethyl)-1H-pyrazole (0.07 g, 0.6 mmol) as starting materials.

LC-MS (ESI): m/z = 439.3 [M+H]⁺.

¹H NMR (400 MHz, CDCl₃) δ 8.63 (s, 1H), 8.03 (s, 1H), 6.97-7.89 (m, 2H), 6.76-6.75 (m, 1H), 6.64-6.62 (m, 1H), 6.21 (s, 1H), 4.83-4.80 (m, 2H), 4.76-4.74 (m, 2H), 4.34-4.30 (m, 2H), 3.83-3.78 (m, 2H), 3.35-3.30 (m, 1H), 2.84-2.80 (m, 2H), 2.57-2.55 (m, 3H), 2.30-2.27 (m, 3H).

### Example 135:

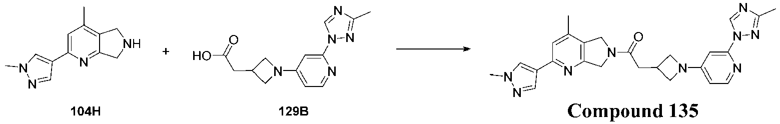

With reference to the synthesis method of step 3 in Example 84, **compound 135** (30 mg, 16.69%) was obtained using **104H** (0.1 g, 0.46 mmol) and intermediate **129B** (0.13 g, 0.46 mmol) as starting materials.

LC-MS (ESI): m/z= 470.1 [M+H]⁺.

¹H NMR (400 MHz, CDCl₃): δ 9.06 (s, 1H), 8.03-8.01 (m, 1H), 7.95-7.92 (m, 1H), 7.31-7.21 (m, 2H), 6.76-6.75 (m, 1H), 6.22-6.19 (m, 1H), 4.94-4.77 (m, 4H), 4.35-4.30 (m, 2H), 3.97-3.96 (m, 3H), 3.82-3.78 (m, 2H), 3.35-3.30 (m, 1H), 2.85-2.83 (m, 2H), 2.49 (s, 3H), 2.38-2.32 (m, 3H).

### Example 136:

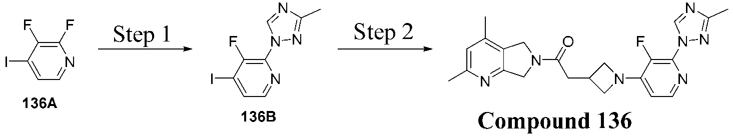

Step 1: With reference to step 1 in Example 26, compound **136B** (0.71 g, crude, directly used in the next step) was synthesized using **136A** (1.0 g, 4.15 mmol) and 3-methyl-1H-1,2,4-thiazole (0.33 g, 3.94 mmol) as starting materials.

LC-MS (ESI): m/z =305.0 [M+H]⁺.

Step 2: With reference to the synthesis method of step 2 in Example 113, **compound 136** (54 mg, 19.48%) was obtained using compound **2C** (200 mg, 0.66 mmol) and compound **136B** (0.16 g, crude) as starting materials.

LC-MS (ESI): m/z = 422.3 [M+H]⁺.

¹H NMR (400 MHz, CDCl₃) δ 8.68-8.67 (m, 1H), 7.93 (d, 1H), 6.92 (d, 1H), 6.29-6.26 (m, 1H), 4.81-4.73 (m, 4H), 4.49-4.44 (m, 2H), 3.98-3.93 (m, 2H), 3.34 - 3.27 (m, 1H), 2.84-2.80 (m, 2H), 2.55-2.51 (m, 6H), 2.26 (d, 3H).

### Example 137:

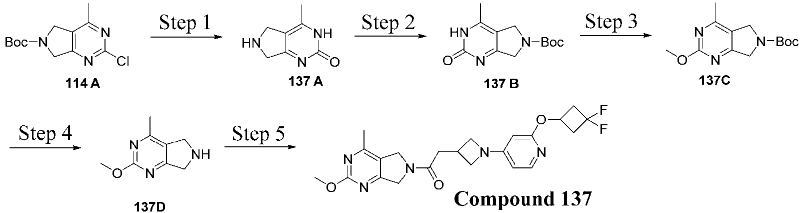

Step 1: Compound **114A** (1 g, 3.71 mmol) was dissolved in 3 N hydrochloric acid (15 mL), and then the mixture was warmed to 100°C and stirred for 1 hour. After the reaction was completed, the mixture was cooled to room temperature and concentrated to obtain crude compound **137A,** which was directly used in the next step.

LC-MS (ESI): m/z = 152.1[M+H]⁺.

Step 2: Compound **137A** (obtained in step 1) was dissolved in dichloromethane (10 mL), diisopropylethylamine (1.44 g, 11.13 mmol) and di-tert-butyl dicarbonate (1.21 g, 5.57 mmol) were successively added, and then the mixture was reacted at room temperature for 3 h. After the reaction was completed, the mixture was concentrated and the residue was separated and purified by silica gel column chromatography (DCM/MeOH = 10/1) to obtain compound **137B** (0.85 mg, two-step yield: 91%).

LC-MS (ESI): m/z = 252.1[M+H]⁺.

Step 3: Compound **137B** (0.5 g, 1.98 mmol) was dissolved in acetonitrile (10 mL), and potassium carbonate (0.82 g, 5.94 mmol) was added. Iodomethane (0.56 g, 3.96 mmol) was added dropwise. After the dropwise addition was completed, the mixture was reacted at room temperature for 3 hours. After the reaction was completed, the mixture was filtered and the filtrate was concentrated. The resulting residue was separated and purified by silica gel column chromatography (EA/PE = 1/4) to obtain compound **137C** (20 mg, 3.8%).

LC-MS (ESI): m/z = 266.2[M+H]⁺.

Step 4: Compound **137C** (20 mg, 0.08 mmol) was dissolved in dichloromethane (3 mL), trifluoroacetic acid (1 mL) was added, and then the mixture was reacted at room temperature for 1 h. After the reaction was completed, the mixture was concentrated to obtain crude compound **137D,** which was directly used in the next reaction.

LC-MS (ESI): m/z = 166.1[M+H]⁺.

Step 3: Crude compound **137D,** compound **81D** (22 mg, 0.08 mmol), N,N-diisopropylethylamine (31 mg, 0.24 mmol), and 2-(7-azobenzotriazole)-N,N,N',N'-tetramethyluronium hexafluorophosphate (61 mg, 0.16 mmol) were successively dissolved in DMF (5 mL), and the mixture was reacted at room temperature for 16 h. Water (30 mL) was added, and the mixture was extracted with ethyl acetate (20 mL). The combined organic phases were washed with saturated aqueous sodium chloride solution and concentrated under reduced pressure. The resulting residue was purified and separated using a C18 reverse-phase column (composition of mobile phases A and B: mobile phase A: acetonitrile; and mobile phase B: water (containing 0.1% aqueous ammonia), (A/B = 45%/55%)) to obtain **compound 137** (18 mg, 55%).

LC-MS (ESI): m/z = 446.2[M+H]⁺.

¹H NMR (400 MHz, CDCl₃) δ = 7.81-7.80 (m, 1H), 6.02-6.00 (m, 1H), 5.62-5.61 (m, 1H), 5.17 -5.08 (m, 1H), 4.73 (s, 1H), 4.70 (s, 3H), 4.21 (t, 2H), 4.02-4.01 (m, 3H), 3.68 (t, 2H), 3.31- 3.19 (m, 1H), 3.15-3.05 (m, 2H), 2.82-2.75 (m, 2H), 2.73-2.63 (m, 2H), 2.43-2.41 (m, 3H).

### Example 138:

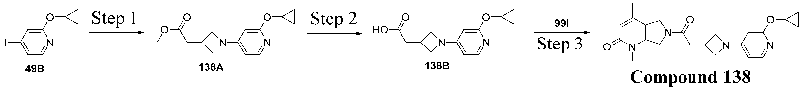

Step 1: With reference to the synthesis method of step 1 in Example 64, compound **138A** (800 mg, 61.5%) was obtained using compound **49B** (1.3 g, 4.98 mmol) and methyl 2-(azetidin-3-yl) acetate 2,2,2-trifluoroacetate (1.1 g, 4.98 mmol) as starting materials.

LCMS (ESI): m/z =263.3 [M+H]⁺.

Step 2: With reference to the synthesis method of step 2 in Example 64, compound **138B** (700 mg, 92.5%) was obtained using compound **138A** (800 mg, 3.05 mmol) as a starting material.

LCMS (ESI): m/z =249.2 [M+H]⁺.

Step 3: With reference to the synthesis method of step 9 in Example 99, **compound 138** (35 mg, 22.2%) was obtained using **138B** (100 mg, 0.40 mmol) and **99I** (107 mg, 0.41 mmol) as starting materials.

LC-MS (ESI): m/z =395.2 [M+H]⁺.

¹H NMR (400 MHz, DMSO-*d*₆) δ 7.77-7.73 (m, 1H), 6.17 (s, 1H), 6.08-6.04 (m, 1H), 5.69-5.65 (m, 1H), 4.88 (s, 1H), 4.65 (s, 2H), 4.42 (s, 1H), 4.17-4.07 (m, 1H), 4.07-4.00 (m, 2H), 3.60-3.52 (m, 2H), 3.33-3.30 (m, 3H), 3.15-2.99 (m, 1H), 2.84-2.74 (m, 2H), 2.11-2.06 (m, 3H), 0.72-0.66 (m, 2H), 0.61-0.56 (m, 2H).

### Example 139:

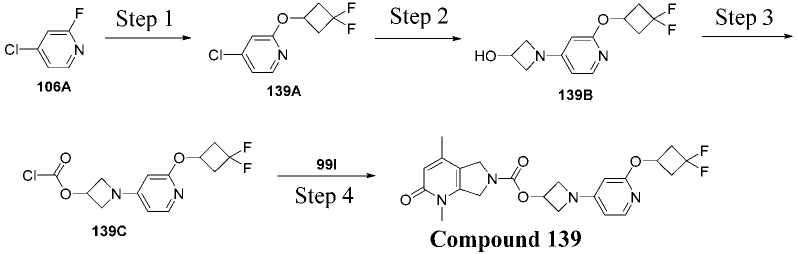

Step 1: With reference to the synthesis method of step 1 in Example 25, compound **139A** (2.01 g, 60.2%) was obtained using 3,3-difluorocyclobutanol (1.64 g, 15.2 mmol) and 2-fluoro-4-chloropyridine (2.00 g, 15.2 mmol) as starting materials.

LC-MS(ESI):m/z = 220.1[M+H]⁺.

Step 2: **139A** (2.01 g, 9.15 mmol), azetidin-3-ol (1.00 g, 13.7 mmol), N, N-diisopropylethylamine (3.54 g, 27.4 mmol) and caesium fluoride (1.39 g, 9.15 mmol) were successively dissolved in N, N-dimethylformamide (100 mL), and then the mixture was warmed to 110°C and reacted for 16 hours. After the reaction was completed as monitored by TLC, the mixture was cooled to room temperature. Saturated aqueous ammonium chloride solution (300 mL) was added, and the mixture was extracted with ethyl acetate (100 mL×5). The combined organic phases were dried over anhydrous sodium sulphate and concentrated under reduced pressure. The residue was purified by silica gel column chromatography (petroleum ether : ethyl acetate (v : v) = 1 : 1) to obtain compound **139B** (1.81 g, 77.2%).

LC-MS(ESI):m/z = 257.1[M+H]⁺.

Step 3: **139B** (800 mg, 3.12 mmol) was dissolved in dried tetrahydrofuran (40 mL), and N, N-diisopropylethylamine (1.21 g, 9.37 mmol) and triphosgene (1.39 g, 4.69 mmol) were added in an ice bath. Then the mixture was reacted at room temperature. After the reaction was completed as monitored by TLC, the mixture was filtered and the filtrate was concentrated to obtain crude compound **139C** (1.80 g), which was directly used in the next reaction.

LC-MS(ESI):m/z = 319.1[M+H]⁺.

Step 4: **99I** (200 mg, 0.72 mmol), crude compound **139C** (800 mg) and N, N-diisopropylethylamine (464 mg, 2.60 mmol) were successively dissolved in dichloromethane (30 mL), and the mixture was reacted at room temperature overnight. After the reaction was completed as monitored by TLC, the mixture was concentrated under reduced pressure and the resulting residue was separated and purified by preparative HPLC to obtain **compound 139** (75 mg, 23.4%).

Preparative HPLC separation and purification method: 1. instrument: waters 2767 preparative liquid chromatographic instrument; chromatographic column: SunFire@ Prep C18 (19 mm×250 mm); 2. The sample was filtered with a 0.45 µm filter to prepare a sample solution. 3. Preparative chromatography conditions: a. composition of mobile phases A and B: mobile phase A: acetonitrile; and mobile phase B: water (containing 0.5% ammonium acetate); b. gradient elution, mobile phase A: 5% - 50%; c. flow rate: 12 mL/min; d. elution time: 30 min.

¹H NMR (400 MHz, Methanol-*d*₄) *δ* 7.74 (d, 1H), 6.33 (s, 1H), 6.14 (dd, 1H), 5.75 (d, 1H), 5.37 (tq, 1H), 5.05-4.92 (m, 1H), 4.85-4.80 (overlapped, m, 1H), 4.78-4.70 (m, 1H), 4.64 (t, 1H), 4.56 (t, 1H), 4.33 (ddt, 2H), 4.02-3.93 (m, 2H), 3.46 (d, 3H), 3.15-2.98 (m, 2H), 2.72-2.54 (m, 2H), 2.18 (dd, 3H).

LC-MS(ESI):m/z = 447.2[M+H]⁺.

### Example 140:

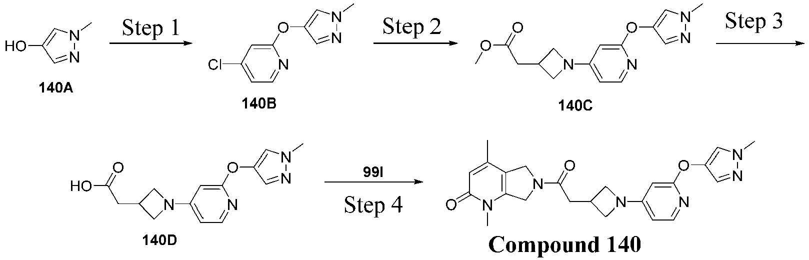

Step 1: With reference to the synthesis method of step 1 in Example 29, compound **140B** (800 mg, 37.5%) was obtained using compound **140A** (1.0 g, 10.2 mmol) as a starting material.

LCMS (ESI): m/z = 210.1 [M+H]⁺.

Step 2: With reference to the synthesis method of step 2 in Example 29, compound **140C** (500 mg, 43.2%) was obtained using compound **140B** (800 mg, 3.83 mmol) and methyl 2-(azetidin-3-yl)acetate 2,2,2-trifluoroacetate (1.1 g, 4.98 mmol) as starting materials.

LCMS (ESI): m/z =303.2 [M+H]⁺.

Step 3: With reference to the synthesis method of step 2 in Example 64, compound **140D** (400 mg, 22.2%) was obtained using **140C** (500 mg, 1.66 mmol) as a starting material.

LC-MS (ESI): m/z =289.2 [M+H]⁺.

Step 4: With reference to the synthesis method of step 9 in Example 99, **compound 140** (40 mg, 26.3%) was obtained using **140D** (100 mg, 0.35 mmol) and **99I** (91 mg, 0.41 mmol) as starting materials.

LC-MS (ESI): m/z =435.2 [M+H]⁺.

¹H NMR (400 MHz, DMSO-*d*₆) δ 7.74-7.70 (m, 2H), 7.32 (s, 1H), 6.18-6.10 (m, 2H), 5.84-5.76 (m, 1H), 4.88 (s, 1H), 4.65 (s, 2H), 4.43 (s, 1H), 4.10-4.03 (m, 2H), 3.79 (s, 3H), 3.64-3.55 (m, 2H), 3.33-3.30 (m, 3H), 3.13-3.03 (m, 1H), 2.84-2.74 (m, 2H), 2.11-2.04 (m, 3H).

### Example 141:

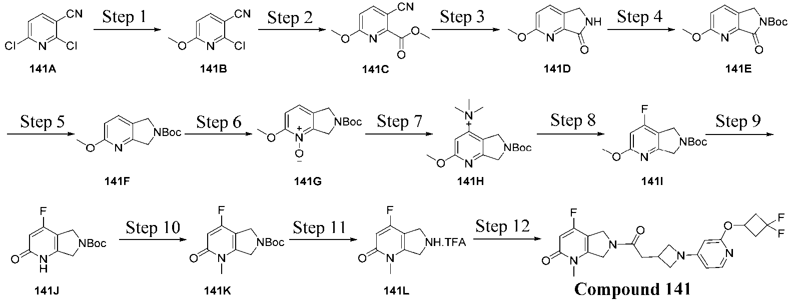

Step 1: With reference to the operation method of step 1 in Example 99, crude compound **141B** (47 g) was obtained using compound **141A** (50 g, 289 mmol) as a starting material.

LC-MS (ESI): m/z = 169[M+H]⁺.

Step 2: With reference to the operation process of step 2 in Example 99, crude compound **141C** (40 g) was obtained using compound **141B** (45 g, 266.9 mmol) as a starting material.

LC-MS (ESI): m/z = 193.1[M+H]⁺.

Step 3: With reference to the operation process of step 3 in Example 99, compound **141D** (11 g) was obtained using compound **141C** (40 g) as a starting material.

LC-MS (ESI): m/z = 165.1[M+H]⁺.

Step 4: With reference to the operation method of step 4 in Example 99, compound **141E** (15 g, 84.7%) was obtained using compound **141D** (11 g, 67 mmol) as a starting material.

LC-MS (ESI): m/z = 165.1[M+H-100]⁺.

Step 5: With reference to the operation process of step 5 in Example 99, compound **141F** (9 g, 67.88%) was obtained using compound **141E** (14 g, 53 mmol) as a starting material.

LC-MS (ESI): m/z = 251.1[M+H]⁺.

Step 6: **141F** (9 g, 36 mmol) was dissolved in dichloromethane (100 mL), m-chloroperoxybenzoic acid (23.27 g, 107.88 mmol, purity: 80%) was added, and then the mixture was reacted at 30°C for 16 h. Aqueous sodium thiosulphate solution was added to the reaction solution. The resulting mixture was extracted and subjected to phase separation. The organic phase was concentrated under reduced pressure and then the residue was separated and purified by silica gel column chromatography (DCM : MeOH = 12 : 1) to obtain **141G** (5 g, 52.22%).

LC-MS (ESI): m/z = 267.1[M+H]⁺.

Step 7: **141G** (4 g, 15.02 mmol) was dissolved in dichloromethane (70 mL). A solution of trimethylamine (5.33 g, 90.12 mmol) in methanol was added at 0°C, and then trifluoromethanesulphonic anhydride (21.19 g, 75.1 mmol) was added dropwise. After the dropwise addition was completed, the mixture was warmed to room temperature and reacted for 30 min. The mixture was adjusted to pH = 7-8 by dropwise adding aqueous sodium bicarbonate solution, then extracted and subjected to phase separation. The organic phase was concentrated under reduced pressure to obtain crude product **141H** (4.4 g).

LC-MS (ESI): m/z = 308.2[M+H]⁺.

Step 8: Crude product **141H** (4.4 g, 14.27 mmol) and potassium fluoride (4.15 g, 71.35 mmol) were dissolved in N,N-dimethylformamide (40 mL), and then the mixture was warmed to 100°C and reacted for 3 h. After the reaction was completed, the mixture was cooled to room temperature. Water (150 mL) was added and the mixture was extracted with ethyl acetate (30 mL×3). The combined organic phases were washed with saturated aqueous sodium chloride solution, and the organic phase was concentrated under reduced pressure. Then the residue was separated and purified by silica gel column chromatography (eluents: EA/PE = 1/4) to obtain compound **141I** (0.23 g, 6.01%).

LC-MS (ESI): m/z = 269.1[M+H]⁺.

Step 9: With reference to the operation process of step 6 in Example 99, compound **141J** (0.17 g, 79.28%) was obtained using compound **141I** (0.23 g, 0.86 mmol) as a starting material.

LC-MS (ESI): m/z = 251.1[M+H]⁺.

Step 10: **141J** (0.17 g, 0.67 mmol) and potassium carbonate (0.28 g, 2.01 mmol) were successively dissolved in N,N-dimethylformamide (5 mL), and iodomethane (0.48 g, 3.35 mmol) was added. Then the mixture was reacted at room temperature for 16 h. Water (30 mL) was added and the resulting mixture was extracted with ethyl acetate (15 mL×3). The combined organic phases were washed with saturated aqueous sodium chloride solution and concentrated under reduced pressure. Then the residue was separated and purified by silica gel column chromatography (DCM : MeOH = 20 : 1) to obtain **141K** (0.11 g, 61.32%).

LC-MS (ESI): m/z = 269.1[M+H]⁺.

Step 11: **141K** (0.05 g, 0.19 mmol) was dissolved in dichloromethane (5 mL), trifluoroacetic acid (2 mL) was added, and the mixture was reacted at room temperature for 1 h and concentrated under reduced pressure to obtain crude product **141L** (0.06 g).

LC-MS (ESI): m/z = 169.1[M+H]⁺.

Step 12: **141L** (0.054 g, 0.19 mmol), **81D** (0.062 g, 0.21 mmol), N,N-diisopropylethylamine (0.1 g, 0.76 mmol), and 2-(7-azobenzotriazole)-N,N,N',N'-tetramethyluronium hexafluorophosphate (0.11 g, 0.29 mmol) were successively dissolved in DMF (5 mL), and then the mixture was reacted at room temperature for 16 h. Water (30 mL) was added and the resulting mixture was extracted with ethyl acetate (15 mL×3). The combined organic phases were washed with saturated aqueous sodium chloride solution and concentrated under reduced pressure. Then the residue was separated and purified using a C18 reverse-phase column (composition of mobile phases A and B: mobile phase A: acetonitrile; and mobile phase B: water (containing 0.1% TFA), (A/B = 45%/55%)), and the prepared solution was concentrated to remove most of acetonitrile. The residue was adjusted to pH 8 by dropwise adding saturated aqueous sodium bicarbonate solution, and extracted with ethyl acetate. The organic phase was concentrated to obtain compound **141** (0.045 g, 52.44%).

LC-MS (ESI): m/z = 449.2[M+H]⁺.

¹H NMR (400 MHz, DMSO-*d*₆) δ 7.75-7.71 (m, 1H), 6.19 (d, 1H), 6.10-6.06 (m, 1H), 5.69-5.65 (m, 1H), 5.10-5.01 (m, 1H), 4.97-4.46 (m, 4H), 4.08-4.00 (m, 2H), 3.60-3.53 (m, 2H), 3.37-3.32 (m, 3H), 3.14-3.01 (m, 3H), 2.83-2.76 (m, 2H), 2.68-2.54 (m, 2H).

### Example 142:

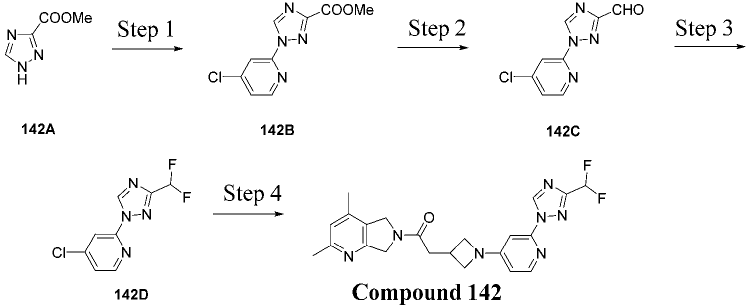

Step 1: **142A** (1.27 g, 10.0 mmol) was dissolved in DMF (5 mL), and 4-chloro-2-fluoropyridine (1.31 g, 10.0 mmol) and potassium carbonate (2.76 g, 20.0 mmol) were successively added. Subsequently, under nitrogen atmosphere, the resulting mixture was warmed to 80°C and reacted overnight. After the reaction was completed as monitored by TLC, the reaction was stopped. After the reaction mixture was cooled to room temperature, EA (30 mL) was added, and the organic phase was washed with saturated aqueous sodium chloride solution (30 mL×3), dried over anhydrous sodium sulphate, filtered and concentrated under reduced pressure. The residue was separated and purified by silica gel column chromatography (PE : EA = 10 : 1) to obtain compound **142B** (900 mg, 37.74%).

LC-MS (ESI): m/z = 239.0 [M+H]⁺.

Step 2: **142B** (450 mg, 1.89 mmol) was dissolved in THF (5 mL) at room temperature. Under nitrogen atmosphere, diisobutylaluminium hydride (323 mg, 2.27 mmol) was slowly added dropwise at -78°C, and after the dropwise addition was completed, the mixture was warmed to -20°C and the reaction was continued for 2 hours. Upon complete depletion of starting materials as monitored by TLC, the reaction was stopped. Saturated ammonium chloride solution was added to the reaction solution to quench the reaction. The reaction mixture was warmed to room temperature. EA (15 mL) was added, and the organic phase was washed with water (20 mL×3) and saturated aqueous sodium chloride solution (30 mL×2), dried over anhydrous sodium sulphate, filtered and concentrated under reduced pressure. The residue was separated and purified by silica gel column chromatography (PE : EA = 4 : 1) to obtain compound **142C** (220 mg, 55.93%).

LC-MS (ESI): m/z = 209.1 [M+H]⁺.

Step 3: **142C** (220 mg, 1.05 mmol) was dissolved in DCM (5 mL) at room temperature. Under nitrogen atmosphere, DAST (203 mg, 1.26 mmol) was added dropwise to the reaction solution at -78°C, and after the dropwise addition was completed, the mixture was warmed to -20°C and the reaction was continued for 2 hours. Upon complete depletion of starting materials as monitored by TLC, the reaction was stopped. The reaction solution was poured into ice water to quench the reaction. DCM (10 mL) was added and the mixture was extracted and subjected to phase separation. The organic phase was washed with saturated aqueous sodium chloride solution (30 mL×2), dried over anhydrous sodium sulphate and filtered. The filtrate was concentrated and the residue was separated and purified by column chromatography (PE : EA = 4 : 1) to obtain **142D** (65 mg, 26.73%).

LC-MS (ESI): m/z= 231.1 [M+H]⁺.

Step 4: With reference to the synthesis method of step 2 in **Example 28,** compound **142** (40 mg, 32.29%) was synthesized using **142D** (65 mg, 0.28 mmol) as a starting material.

LC-MS (ESI): m/z = 440.1 [M+H]⁺.

¹H NMR (400 MHz, CDCl₃) δ 9.16 (s, 1H), 8.04-8.03 (d, 1H), 6.97-6.92 (d, 1H), 6.81-6.78 (d, 2H), 6.25-6.24 (d, 1H), 4.83-4.74 (m, 4H), 4.34-4.29 (m, 2H), 3.82-3.78 (m, 2H), 3.37-3.29 (m, 1H), 2.84-2.81 (t, 2H), 2.57-2.55 (d, 3H), 2.30-2.27 (d, 3H).

### Example 143:

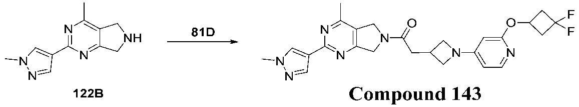

With reference to the synthesis method of step 4 in Example 81, **compound 143** (20 mg, 15.35%) was obtained using **122B** (0.07 g, 0.32 mmol) and intermediate **81D** (0.08 g, 0.26 mmol) as starting materials.

LC-MS (ESI): m/z= 496.3 [M+H]⁺.

¹H NMR (400 MHz, CDCl₃): δ 8.11-8.09 (m, 1H), 8.03-8.01 (m, 1H), 7.81-7.80 (m, 1H), 6.02-6.01 (m, 1H), 5.49-5.45 (m, 1H), 5.17-5.10 (m, 1H), 4.75-4.69 (m, 4H), 4.32-4.26 (m, 2H), 3.90-3.75 (m, 5H), 3.34-3.27 (m, 1H), 3.14-3.08 (m, 3H), 2.80-2.67 (m, 3H), 2.42 (s, 3H).

### Example 144:

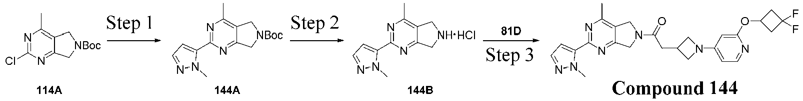

Step 1: With reference to the synthesis method of step 6 in Example 104, compound **144A** (600 mg, 90%) was obtained using **114A** (567 mg, 2.10 mmol) and 1-methyl-1H-pyrazole-5-boronic acid pinacol ester (660 mg, 3.15 mmol) as starting materials.

LC-MS (ESI): m/z = 316.2 [M+H]⁺.

Step 2: Compound **144A** (600 mg, 1.90 mmol) was dissolved in dichloromethane (10 mL), HCl dioxane solution (20 mL, 4 N) was slowly added, and the mixture was stirred at room temperature for 1 hour. After the reaction was completed, the mixture was concentrated to obtain crude compound **144B** (488 mg).

LC-MS (ESI): m/z = 216.1 [M+H]⁺.

Step 3: Compound **144B** (160 mg, crude), compound **81D** (220 mg, 0.74 mmol), triethylamine (300 mg, 2.96 mmol) and N-methylimidazole (91 mg, 1.11 mmol) were successively dissolved in N,N-dimethylformamide (10 mL), chloro-N,N,N',N'-tetramethylformamidinium hexafluorophosphate (420 mg, 1.48 mmol) was then added, and the mixture was reacted at room temperature for 1 hour. After the reaction was completed as monitored by TLC, the mixture was concentrated under reduced pressure. The residue was separated and purified by silica gel column chromatography (dichloromethane : methanol (v/v) = 25 : 1) to obtain compound **144** (240 mg, 65%).

¹H NMR (400 MHz, CDCl₃) δ 7.81-7.79 (m, 1H), 7.52-7.51 (m, 1H), 7.08-7.05 (m, 1H), 6.01-5.99 (m, 1H), 5.64-5.63 (m, 1H), 5.17-5.07 (m, 1H), 4.82-4.81 (m, 4H), 4.34-4.33 (m, 3H), 4.21-4.17 (m, 2H), 3.68-3.65 (m, 2H), 3.30-3.23 (m, 1H), 3.13- 3.03 (m, 2H), 2.84-2.80 (m, 2H), 2.75-2.65 (m, 2H), 2.55-2.53 (m, 3H).

LC-MS (ESI): m/z = 496.3 [M+H]⁺.

### Example 145:

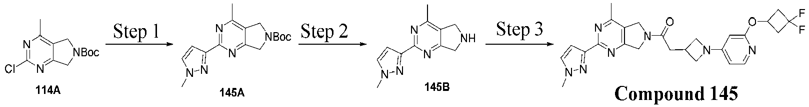

Step 1: With reference to the synthesis method of step 6 in Example 104, compound **145A** (0.21 g, 89.8%) was obtained using compound **114A** (0.2 g, 0.74 mmol) and 1-methylpyrazole-3-boronic acid pinacol ester (0.18 g, 0.89 mmol) as starting materials.

LC-MS (ESI): m/z = 316.2 [M+H]⁺.

Step 2: With reference to the synthesis method of step 2 in Example 114, crude compound **145B** (0.17 g, directly used in the next step) was obtained using **145A** (0.2 g, 0.63 mmol) as a starting material.

LC-MS (ESI): m/z =216.2 [M+H]⁺.

### Step 3:

With reference to the synthesis method of step 7 in Example 4, **compound 145** (42 mg, 18.79%) was obtained using **145B** (0.13 g, 0.46 mmol) and intermediate **81D** (0.16 g, 0.55 mmol) as starting materials.

LC-MS (ESI): m/z = 496.2 [M+H]⁺.

¹H NMR (400 MHz, CDCl₃): δ 7.80-7.79 (m, 1H), 7.44-7.43 (m, 1H), 7.04-7.02 (m, 1H), 6.01-5.99 (m, 1H), 5.63-5.62 (m, 1H), 5.15-5.09 (m, 1H), 4.84-4.79 (m, 4H), 4.22-4.18 (m, 2H), 4.03 (s, 3H), 3.71-3.66 (m, 2H), 3.29-3.23 (m, 1H), 3.14-3.03 (m, 2H), 2.83-2.63 (m, 4H), 2.56 (d, 3H).

### Example 146:

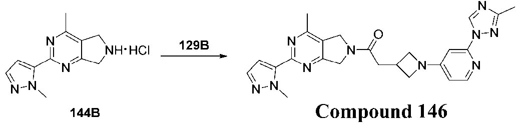

Step 1: With reference to the synthesis method of step 3 in Example 144, compound **146** (82 mg, 24%) was obtained using compound **144B** (160 mg, crude) and compound **129B** (202 mg, 0.73 mmol) as starting materials.

¹H NMR (400 MHz, CDCl₃) δ 9.02 (s, 1H), 8.03-8.02 (m, 1H), 7.52-7.51 (m, 1H), 7.08-7.05 (m, 1H), 6.76-6.75 (m, 1H), 6.21-6.19 (m, 1H), 4.83-4.82 (m, 4H), 4.34-4.30 (m, 5H), 3.80-3.77 (m, 2H), 3.37-3.28 (m, 1H), 2.88-2.83 (m, 2H), 2.55-2.54 (m, 3H), 2.49 (s, 3H).

LC-MS (ESI): m/z = 471.3 [M+H]⁺.

### Example 147:

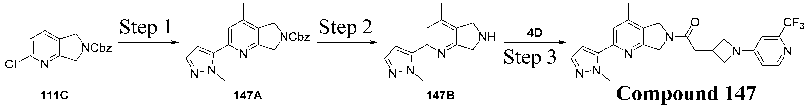

Step 1: With reference to the synthesis method of step 1 in Example 112, compound **147A** (160 mg, 77%) was obtained using compound **111C** (180 mg, 0.60 mmol) and 1-methyl-1H-pyrazole-5-boronic acid pinacol ester (187 mg, 0.90 mmol) as starting materials.

LC-MS (ESI): m/z = 349.2 [M+H]⁺.

Step 2: With reference to the synthesis method of step 2 in Example 112, compound **147B** (80 mg, crude) was obtained using compound **147A** (160 mg, 0.46 mmol) as a starting material.

LC-MS (ESI): m/z = 215.1 [M+H]⁺.

Step 3: With reference to the synthesis method of step 3 in Example 144, compound **147** (47 mg, 55%) was obtained using compound **147B** (40 mg, crude) and compound **4D** (78 mg, 0.30 mmol) as starting materials.

¹H NMR (400 MHz, CDCl₃) δ 8.29-8.28 (m, 1H), 7.51-7.50 (m, 1H), 7.34-7.32 (m, 1H), 6.59-6.56 (m, 2H), 6.36-6.34 (m, 1H), 4.87-4.81 (m, 4H), 4.34-4.29 (m, 2H), 4.19-4.17 (m, 3H), 3.82-3.78 (m, 2H), 3.39-3.33 (m, 1H), 2.88-2.85 (m, 2H), 2.36- 2.35 (m, 3H).

LC-MS (ESI): m/z = 457.1 [M+H]⁺.

### Example 148:

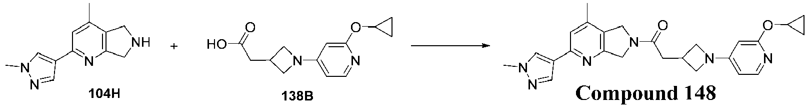

With reference to the synthesis method of step 8 in Example 104, **compound 148** (60 mg, 43.82%) was obtained using **138B** (0.092 g, 0.37 mmol) and intermediate **104H** (0.066 g, 0.31 mmol) as starting materials.

LC-MS (ESI): m/z= 445.1 [M+H]⁺.

¹H NMR (400 MHz, CDCl₃): δ 7.92-7.89 (m, 3H), 7.20-7.19 (m, 1H), 6.03-6.01 (m, 1H), 5.72-5.71 (m, 1H), 4.80-4.75 (m, 4H), 4.26-4.22 (m, 2H), 4.13-4.11 (m, 1H), 3.95 (s, 3H), 3.73-3.71 (m, 2H), 3.31-3.28 (m, 1H), 2.84-2.80 (m, 2H), 2.32-2.31 (m, 3H), 0.90-0.79 (m, 4H).

### Example 149:

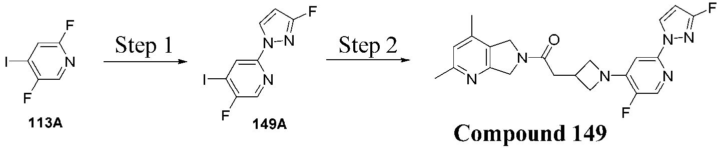

Step 1: Compound **113A** (150 mg, 0.62 mmol), 3-fluoro-1H-pyrazole (59 mg, 0.68 mmol), and potassium carbonate (171 mg, 1.24 mmol) were successively dissolved in dimethyl sulphoxide (10 mL), and under nitrogen atmosphere, the mixture was warmed to 70°C, stirred and reacted for 18 hours. After the reaction mixture was cooled to room temperature, the mixture was filtered and the filtrate was poured into water. The resulting mixture was extracted with ethyl acetate three times. The combined organic phases were washed with saturated aqueous sodium chloride solution, dried over anhydrous sodium sulphate and concentrated under reduced pressure to obtain a crude product. The crude product was purified by Biotage Isolera One (12 g silica gel column, eluents: 0-20% EA/PE) to obtain compound **149A** (134 mg, yield: 70.11%).

LC-MS (ESI): m/z= 308.0 [M+H]⁺.

Step 2: With reference to the operation process of step 1 in Example 10, **compound 149** (25 mg, 18.06%) was obtained using **149A** (121 mg, 0.40 mmol) and 2C (80 mg, 0.33 mmol) as starting materials.

LC-MS (ESI): m/z = 425.2 [M+H]⁺.

¹H NMR (400 MHz, CDCl₃) δ 8.32-8.25 (m, 1H), 7.88-7.80 (m, 1H), 6.99-6.90 (m, 1H), 6.79-6.71 (m, 1H), 6.01-5.94 (m, 1H), 4.90-4.78 (m, 2H), 4.78-4.72 (m, 2H), 4.48-4.40 (m, 2H), 3.97-3.89 (m, 2H), 3.35-3.23 (m, 1H), 2.85-2.78 (m, 2H), 2.60- 2.53 (m, 3H), 2.33-2.25 (m, 3H).

### Example 150:

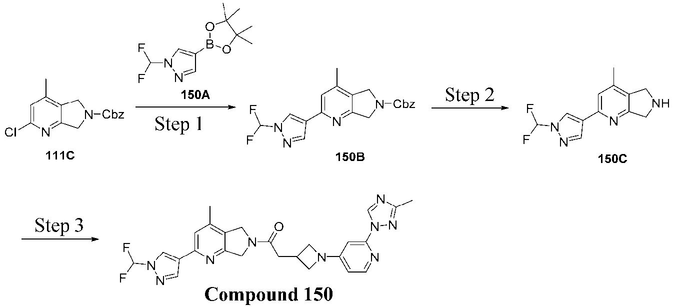

Step 1: With reference to the operation process of step 1 in Example 112, compound **150B** (324 mg, yield: 85.07%) was obtained using **111C** (300 mg, 0.99 mmol) and **150A** (290 mg, 1.19 mmol) as starting materials.

LC-MS (ESI): m/z = 385.2 [M+H]⁺.

Step 2: With reference to the operation process of step 2 in Example 112, compound **150C** (200 mg, directly used in the next reaction without purification) was obtained using **150B** (324 mg, 0.84 mmol) as a starting material.

LC-MS (ESI): m/z = 251.1 [M+H]⁺.

Step 3: With reference to the operation process of step 3 in Example 112, compound **150** (23 mg, yield: 17.52%) was obtained using **150C** (65 mg) and **129B** (78 mg, 0.29 mmol) as starting materials.

LC-MS (ESI): m/z = 506.3 [M+H]⁺.

¹H NMR (400 MHz, CDCl₃) δ 9.08 (s, 1H), 8.40-8.32 (m, 1H), 8.15-8.07 (m, 1H), 8.02 (s, 1H), 7.38-7.27 (m, 1H), 7.23-7.05 (m, 1H), 6.77 (s, 1H), 6.27-6.13 (m, 1H), 4.87-4.75 (m, 4H), 4.37-4.29 (m, 2H), 3.85-3.78 (m, 2H), 3.34 (s, 1H), 2.88-2.79 (m, 2H), 2.49 (s, 3H), 2.35 (s, 3H).

### Example 151:

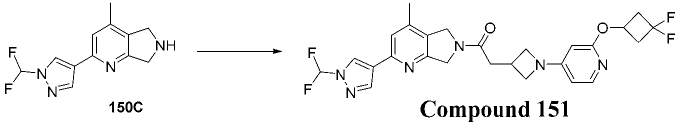

With reference to the operation process of step 3 in Example **112, compound 151** (25 mg, yield: 18.14%) was obtained using **150C** (65 mg) and **81D** (93 mg, 0.31 mmol) as starting materials.

LC-MS (ESI): m/z = 531.3 [M+H]⁺.

¹H NMR (400 MHz, CDCl₃) δ 8.41-8.28 (m, 1H), 8.15-8.08 (m, 1H), 7.82-7.78 (m, 1H), 7.39-7.27 (m, 1H), 7.25-7.06 (m, 1H), 6.03-5.94 (m, 1H), 5.66-5.61 (m, 1H), 5.17-5.07 (m, 1H), 4.82 (s, 2H), 4.81-4.75 (m, 2H), 4.24-4.16 (m, 2H), 3.73-3.63 (m, 2H), 3.32-3.22 (m, 1H), 3.15-3.02 (m, 2H), 2.86-2.78 (m, 2H), 2.77-2.62 (m, 2H), 2.37-2.31 (m, 3H).

### Example 152:

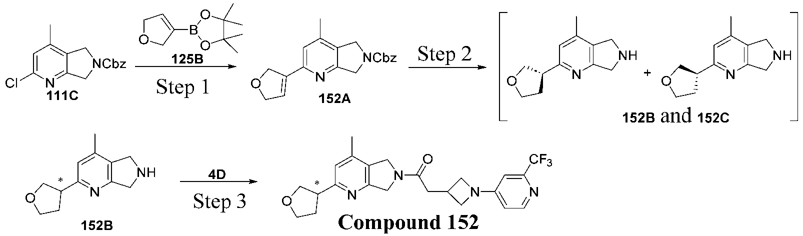

Step 1: With reference to the procedures of step 6 in Example 98, **152A** (420 mg, 94%) was synthesized using **111C** (400 mg, 1.32 mmol) and **125B** (286 mg, 1.47 mmol) as starting materials.

LC-MS (ESI): m/z = 337.1 [M+H]⁺.

Step 2: With reference to the procedures of step 5 in Example 111, a crude product was synthesized using **152A** (420 mg, 1.25 mmol) as a starting material. The crude product was subjected to preparative SFC to obtain **152B** (86 mg, 34%) and **152C** (92 mg, 36%).

SFC separation method: instrument: Waters 150 Prep-SFC, column: Chiral OJ column, mobile phases: A for CO₂; B for 0.1% NH₃•H₂O in MeOH, gradient: 20% Phase B, flow rate: 80 mL/min, back pressure: 100 bar, column temperature: 25°C, wavelength: 220 nm. Retention time: **152B:** tR = 0.831 min; **152C:** tR = 1.016 min.

**152B:** LC-MS (ESI): m/z = 205.1 [M+H]⁺.

**152C:** LC-MS (ESI): m/z = 205.1 [M+H]⁺.

Step 3: With reference to the procedures of step 7 in Example 4, **compound 152** (41 mg, 45%) was synthesized using **152B** (42 mg, 0.21 mmol) and **4D** (54 mg, 0.21 mmol) as starting materials.

LC-MS (ESI): m/z = 447.0 [M+H]⁺.

¹H NMR (400 MHz, CDCl₃) δ 8.30-8.28 (m, 1H), 7.02-6.96 (m, 1H), 6.59-6.57 (m, 1H), 6.35-6.32 (m, 1H), 4.84-4.74 (m, 4H), 4.32-4.26 (m, 2H), 4.17-4.05 (m, 2H), 3.96-3.86 (m, 2H), 3.80-3.74 (m, 2H), 3.65-3.56 (m, 1H), 3.36-3.29 (m, 1H), 2.85-2.80 (m, 2H), 2.43-2.34 (m, 1H), 2.32-2.28 (m, 3H), 2.21-2.14 (m, 1H).

### Example 153:

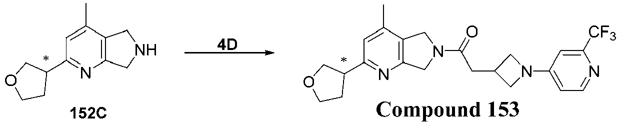

With reference to the procedures of step 7 in Example 4, **compound 153** (46 mg, 45%) was synthesized using **152C** (47 mg, 0.23 mmol) and **4D** (60 mg, 0.23 mmol) as starting materials.

LC-MS (ESI): m/z = 447.3 [M+H]⁺.

¹H NMR (400 MHz, CDCl₃) δ 8.30-8.27 (m, 1H), 7.01-6.96 (m, 1H), 6.59-6.57 (m, 1H), 6.35-6.32 (m, 1H), 4.83-4.74 (m, 4H), 4.32-4.26 (m, 2H), 4.16-4.05 (m, 2H), 3.96-3.86 (m, 2H), 3.79-3.74 (m, 2H), 3.64-3.55 (m, 1H), 3.36-3.29 (m, 1H), 2.84-2.79 (m, 2H), 2.43-2.32 (m, 1H), 2.31-2.27 (m, 3H), 2.23-2.12 (m, 1H).

### Example 154:

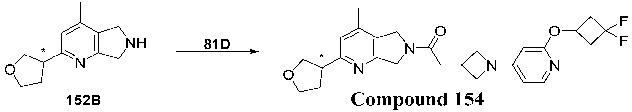

With reference to the procedures of step 4 in Example 81, **compound 154** (43 mg, 41%) was synthesized using **152B** (44 mg, 0.22 mmol) and **81D** (64 mg, 0.22 mmol) as starting materials.

LC-MS (ESI): m/z = 485.1 [M+H]⁺.

¹H NMR (400 MHz, CDCl₃) δ 7.80-7.78 (m, 1H), 6.96 (s, 1H), 6.01-5.98 (m, 1H), 5.64-5.62 (m, 1H), 5.15-5.07 (m, 1H), 4.79-4.72 (m, 4H), 4.21-4.12 (m, 3H), 4.11-4.04 (m, 1H), 3.96-3.85 (m, 2H), 3.68-3.63 (m, 2H), 3.60-3.50 (m, 1H), 3.30-3.21 (m, 1H), 3.13-3.02 (m, 2H), 2.82-2.77 (m, 2H), 2.74-2.62 (m, 2H), 2.41-2.31 (m, 1H), 2.29-2.27 (m, 3H), 2.23-2.13 (m, 1H).

### Example 155:

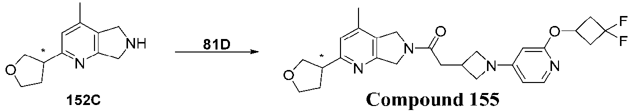

With reference to the procedures of step 4 in Example 81, **compound 155** (40 mg, 37%) was synthesized using **152C** (45 mg, 0.22 mmol) and **81D** (66 mg, 0.22 mmol) as starting materials.

LC-MS (ESI): m/z = 485.1 [M+H]⁺.

¹H NMR (400 MHz, CDCl₃) δ 7.82-7.80 (m, 1H), 6.96 (s, 1H), 6.03-6.00 (m, 1H), 5.62-5.60 (m, 1H), 5.17-5.09 (m, 1H), 4.78-4.73 (m, 4H), 4.25-4.21 (m, 2H), 4.17-4.12 (m, 1H), 4.10-4.04 (m, 1H), 3.96-3.85 (m, 2H), 3.76-3.68 (m, 2H), 3.58-3.50 (m, 1H), 3.32-3.24 (m, 1H), 3.17-3.05 (m, 2H), 2.83-2.77 (m, 2H), 2.75-2.65 (m, 2H), 2.41-2.31 (m, 1H), 2.29-2.27 (m, 3H), 2.23-2.12 (m, 1H).

### Example 156:

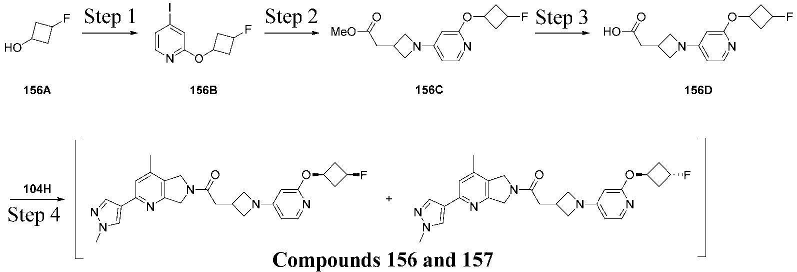

Step 1: With reference to the synthesis method of step 1 in Example 25, compound **156B** (534 mg, 73%) was obtained using **156A** (450 mg, 5.00 mmol) and 2-fluoro-4-iodopyridine (555 mg, 2.50 mmol) as starting materials.

LC-MS (ESI): m/z = 294.0 [M+H]⁺.

Step 2: With reference to the synthesis method of step 2 in Example 59, compound **156C** (422 mg, 78%) was obtained using **156B** (534 mg, 1.82 mmol) and methyl 2-(azetidin-3-yl)acetate (235 mg, 1.82 mmol) as starting materials.

LC-MS (ESI): m/z = 295.2 [M+H]⁺

Step 3: With reference to the synthesis method of step 3 in Example 59, compound **156D** (500 mg, crude) was obtained using **156C** (534 mg, 1.82 mmol) as a starting material.

LC-MS (ESI): m/z = 281.1 [M+H]⁺

Step 4: With reference to the synthesis method of step 3 in Example 144, a mixture was obtained using **156D** (120 mg, crude) and **104H** (60 mg, 0.28 mmol) as starting materials. The mixture was purified by preparative high-performance liquid chromatography to obtain two compounds, i.e., compound **156** (35 mg, 26%) and compound **157** (27 mg, 20%), respectively.

Preparation method: Instrument: waters 2767 preparative liquid chromatographic instrument; chromatographic column: SunFire@ Prep C18 (19 mm × 250 mm). The sample was dissolved in DMF and filtered with a 0.45 µm filter to prepare a sample solution. Preparative chromatography conditions: composition of mobile phases A and B: mobile phase A: acetonitrile, and mobile phase B: water (containing 5 mM ammonium acetate); gradient elution, mobile phase A: 10%-80%; flow rate: 15 mL/min. Elution time: 25 min.

HPLC analytical method: Instrument: Shimadzu LC-20AT, instrument code: CH-Y-J0460; chromatographic column: brand: Welch, chromatographic column model: Xtimate C18, 4.6*50 mm, 3 µm. Chromatography conditions: composition of mobile phases A and B: mobile phase A: 0.1% aqueous ammonia solution, mobile phase B: acetonitrile, gradient elution, mobile phase A: 95%-5%-95%. Elution time: 10 min. Retention time: Compound **156:** t_{R} = 2.272 min; Compound **157:** t_{R} = 2.288 min.

Compound **156:** ¹H NMR (400 MHz, CDCl₃) δ 7.94-7.83 (m, 3H), 7.21-7.20 (m, 1H), 6.01-6.00 (m, 1H), 5.54 (s, 1H), 5.40-5.20 (m, 2H), 4.80-4.75 (m, 4H), 4.25 (s, 2H), 3.96-3.95 (m, 3H), 3.74 (s, 2H), 3.30 (s, 1H), 2.84-2.69 (m, 4H), 2.60-2.52 (m, 2H), 2.32-2.31 (m, 3H).

LC-MS (ESI): m/z = 477.3 [M+H]⁺.

Compound 157: ¹H NMR (400 MHz, CDCl₃) δ 7.94-7.89 (m, 2H), 7.84-7.82 (m, 1H), 7.21-7.20 (m, 1H), 6.01-5.99 (m, 1H), 5.56 (s, 1H), 5.40-5.20 (m, 2H), 4.80- 4.75 (m, 4H), 4.24-4.20 (m, 2H), 3.96-3.95 (m, 3H), 3.71 (s, 2H), 3.29 (s, 1H), 2.83- 2.66 (m, 4H), 2.58-2.54 (m, 2H), 2.32-2.31 (m, 3H).

LC-MS (ESI): m/z = 477.3 [M+H]⁺.

### Example 158:

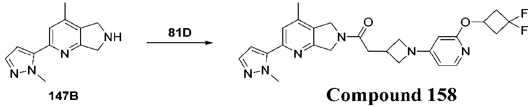

With reference to the synthesis method of step 3 in Example **144,** compound **158** (45 mg, 30%) was obtained using compound **147B** (40 mg, crude) and compound **81D** (90 mg, 0.30 mmol) as starting materials.

¹H NMR (400 MHz, CDCl₃) δ 7.84-7.83 (m, 1H), 7.52-7.50 (m, 1H), 7.34-7.33 (m, 1H), 6.57-6.56 (m, 1H), 6.05-6.04 (m, 1H), 5.60 (s, 1H), 5.17 (s, 1H), 4.86-4.81 (m, 4H), 4.28 (s, 2H), 4.19-4.17 (m, 3H), 3.77 (s, 2H), 3.33 (s, 1H), 3.15 (s, 2H), 2.85 -2.73 (m, 4H), 2.36-2.35 (m, 3H).

LC-MS (ESI): m/z = 495.4 [M+H]⁺.

### Example 159:

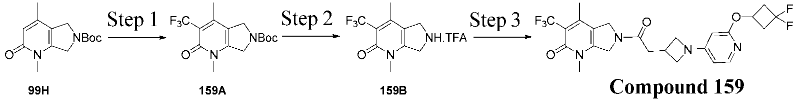

Step 1: **99H** (1 g, 3.78 mmol), (trifluoromethyl)trimethylsilane (1.61 g, 11.34 mmol), potassium fluoride (0.44 g, 7.56 mmol),
[bis(trifluoroacetoxy)iodo]benzene (2.44 g, 5.67 mmol), and copper acetate (0.34 g, 1.89 mmol) were successively dissolved in acetonitrile (20 mL), and under nitrogen atmosphere, the mixture was reacted at room temperature for 16 h. Water (20 mL) was added and the resulting mixture was extracted with ethyl acetate (20 mL×3). The combined organic phases were washed with saturated aqueous sodium chloride solution and concentrated under reduced pressure. Then the residue was separated and purified by silica gel column chromatography (EA/PE = 1/1) to obtain compound **159A** (0.14 g, 11%).

LC-MS (ESI): m/z =333.1[M+H]⁺.

Step 2: **159A** (0.08 g, 0.24 mmol) was dissolved in dichloromethane (6 mL), trifluoroacetic acid (2 mL) was added, and the mixture was reacted at room temperature for 2 h. The reaction solution was concentrated under reduced pressure to obtain crude compound **159B** (0.1 g).

LC-MS (ESI): m/z = 233.1[M+H]⁺.

Step 3: Crude product **159B** (0.1 g, 0.24 mmol), **81D** (0.072 g, 0.24 mmol), N,N-diisopropylethylamine (0.16 g, 1.2 mmol), and 2-(7-azobenzotriazole)-N,N,N',N'-tetramethyluronium hexafluorophosphate (0.14 g, 0.36 mmol) were successively dissolved in DMF (5 mL), and then the mixture was reacted at room temperature for 16 h. Water (20 mL) was added and the resulting mixture was extracted with ethyl acetate (20 mL×3). The combined organic phases were washed with saturated aqueous sodium chloride solution and concentrated under reduced pressure. The residue was purified using a C18 reverse-phase column (composition of mobile phases A and B: mobile phase A: acetonitrile; and mobile phase B: water (containing 0.1% TFA), (A/B = 45%/55%)), and the prepared solution was concentrated to remove most of acetonitrile. The residue was adjusted to pH 8 by dropwise adding saturated aqueous sodium bicarbonate solution, and extracted with ethyl acetate. The organic phase was concentrated to obtain **compound 159** (0.06 g, 48.5%)

LC-MS (ESI): m/z = 513.2[M+H]⁺.

¹H NMR (400 MHz, CDCl₃) δ 7.84-7.77 (m, 1H), 6.04-5.98 (m, 1H), 5.61 (s, 1H), 5.20-5.08 (m, 1H), 4.84-4.74 (m, 2H), 4.71-4.61 (m, 2H), 4.21 (t, 2H), 3.75-3.61 (m, 2H), 3.47 (s, 3H), 3.32-3.19 (m, 1H), 3.17-3.03 (m, 2H), 2.84-2.61 (m, 4H), 2.36-2.27 (m, 3H).

### Example 160:

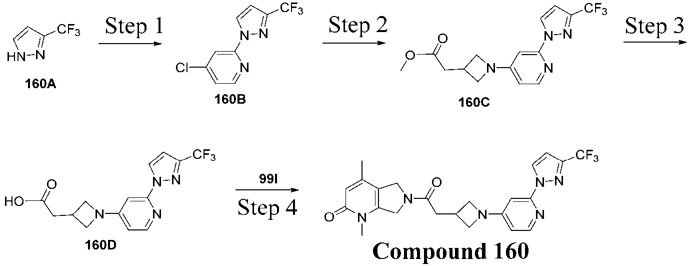

Step 1: With reference to the synthesis method of step 1 in Example 26, compound **160B** (0.21 g, 58.33%) was obtained using **160A** (0.20 g, 1.47 mmol) and 2-fluoro-4-chloropyridine (0.19 g, 1.47 mmol) as starting materials.

LC-MS (ESI): m/z= 248.2 [M+H]⁺.

Step 2: With reference to the synthesis method of step 1 in Example 17, compound **160C** (0.13 g, 78.78%) was obtained using **160B** (0.12 g, 0.48 mmol) as a starting material.

LC-MS (ESI): m/z= 341.2 [M+H]⁺.

Step 3: With reference to the synthesis method of step 2 in Example 17, compound **160D** (0.12 g, 99.99%) was synthesized using **160C** (0.13 g, 0.38 mmol) as a starting material.

LC-MS (ESI): m/z= 327.2 [M+H]⁺.

Step 4: With reference to the synthesis method of step 3 in Example **17,** compound **160** (0.11 g, 63.5%) was obtained using **160D** (0.12 g, 0.36 mmol) and **99I** (0.2 g, 0.72 mmol) as starting materials.

LC-MS (ESI): m/z= 473.2 [M+H]⁺.

¹H NMR (400 MHz, DMSO-*d*₆) δ 8.76-8.68 ( m, 1H), 8.04-8.01(m, 1H), 7.03 -6.94 (m, 1H), 6.76 (s, 1H), 6.44-6.38 (m, 1H), 6.17 (s, 1H), 4.92-4.85 (m, 1H), 4.70 -4.66 (m, 2H), 4.48-4.39 (m, 1H), 4.24-4.15 (m, 2H), 3.77-3.69 (m, 2H), 3.28-3.27 (m, 3H), 3.18-3.07 (m, 1H), 2.86-2.77 (m, 2H), 2.11-2.05 (m, 3H).

### Example 161:

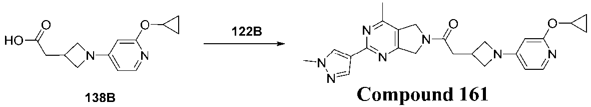

With reference to the synthesis method of step 3 in Example **122,** compound **161** (120 mg, 48.31%) was synthesized using **138B** (0.17 g, 0.67 mmol) and compound **122B** (0.12 g, 0.56 mmol) as starting materials.

LC-MS (ESI): m/z= 446.3 [M+H]⁺.

¹H NMR (400 MHz, DMSO-*d*₆): δ 8.36-8.34 (m, 1H), 8.00-7.99 (m, 1H), 7.80- 7.79 (m, 1H), 6.22-6.20 (m, 1H), 5.92 (s, 1H), 4.87-4.85 (m, 2H), 4.64-4.60 (m, 2H), 4.23-4.15 (m, 3H), 3.90 (s, 3H), 3.77-3.73(m, 2H), 3.15-3.11 (m, 1H), 2.88-2.86 (m, 2H), 2.44-2.43 (m, 3H), 0.80-0.79 (m, 2H), 0.70-0.69 (m, 2H).

### Example 162:

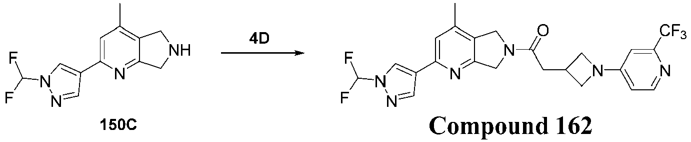

With reference to the operation process of step 3 in Example 112, compound **162** (25 mg, yield: 12.7%) was obtained using **150C** (100 mg, 0.40 mmol) and **4D** (125 mg, 0.48 mmol) as starting materials.

LC-MS (ESI): m/z = 493.3 [M+H]⁺.

¹H NMR (400 MHz, CDCl₃) δ 8.40-8.35 (m, 1H), 8.32-8.25 (m, 1H), 8.12 (s, 1H), 7.38-7.27 (m, 1H), 7.23-7.06 (m, 1H), 6.61-6.56 (m, 1H), 6.38-6.32 (m, 1H), 4.87-4.76 (m, 4H), 4.36-4.28 (m, 2H), 3.86-3.77 (m, 2H), 3.35 (s, 1H), 2.91- 2.80 (m, 2H), 2.37-2.33 (m, 3H).

### Example 163:

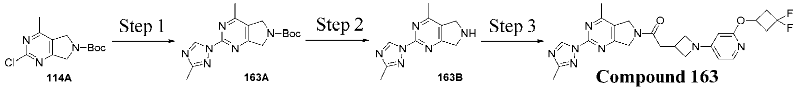

Step 1: **114A** (400 mg, 1.48 mmol) was dissolved in DMF (5 mL), 3-methyl-1H-1,2,4-triazole (0.15 g, 1.78 mmol) and potassium carbonate (0.61 g, 4.44 mmol) were successively added, and then the mixture was warmed to 100°C and reacted overnight. After the reaction was completed as monitored by TLC, the reaction was stopped. After the reaction solution was cooled to room temperature, EA (30 mL) was added, and the organic phase was washed with saturated aqueous sodium chloride solution (30 mL×3), dried over anhydrous sodium sulphate, filtered and concentrated under reduced pressure. The residue was separated and purified by silica gel column chromatography (PE : EA = 10 : 1) to obtain compound **163A** (200 mg, 42.63%).

LC-MS (ESI): m/z = 317.2 [M+H]⁺.

Step 2: **163A** (200 mg, 0.63 mmol) was dissolved in DCM (10 mL) at room temperature, and hydrogen chloride dioxane solution (230 mg, 6.3 mmol, 4 mol/L) was added dropwise. After the dropwise addition was completed, the reaction was continued for 2 hours. Upon complete depletion of starting materials as monitored by TLC, the reaction was stopped. The reaction solution was concentrated under reduced pressure to obtain crude compound **163B** (132 mg, 96.56%).

LC-MS (ESI): m/z = 217.1 [M+H]⁺.

Step 3: With reference to the synthesis method of step 4 in **Example 81,** compound **163** (30 mg, 29.69%) was obtained using **163B** (44 mg, 0.2 mmol) and **81D** (66 mg, 0.22 mmol) as starting materials.

LC-MS (ESI): m/z= 497.3 [M+H]⁺.

¹H NMR (400 MHz, CDCl₃) δ 9.14-9.13 (d, 1H), 7.82-7.82 (d, 1H), 6.02-6.01 (d, 1H), 5.63-5.62 (d, 1H), 5.17-5.08 (m, 1H), 4.88-4.82 (t, 4H), 4.24-4.20 (t, 2H), 3.73-3.68 (m, 2H), 3.32-3.24 (m, 1H), 3.15-3.05 (m, 2H), 2.85-2.79 (m, 2H), 2.77-2.65 (m, 2H), 2.59-2.58 (d, 3H), 2.56 (s, 3H).

### Example 164:

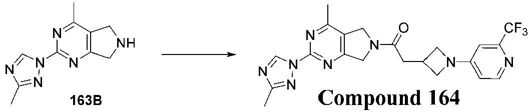

With reference to the synthesis method of step 7 in Example 4, compound **164** (30 mg, 32.16%) was obtained using **163B** (44 mg, 0.2 mmol) and **4D** (62 mg, 0.24 mmol) as starting materials.

LC-MS (ESI): m/z= 429.3 [M+H]⁺.

¹H NMR (400 MHz, CDCl₃) δ 9.14-9.13 (d, 1H), 8.29-8.26 (t, 1H), 6.59 (s, 1H), 6.36-6.35 (d, 1H), 4.87-4.82 (d, 4H), 4.34-4.29 (m, 2H), 3.83-3.79 (t, 2H), 3.39-3.30 (m, 1H), 2.91-2.82 (m, 2H), 2.60-2.59 (d, 3H), 2.55 (s, 3H).

### Example 165:

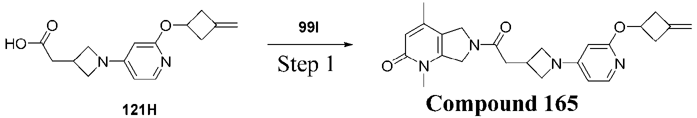

**991** (50 mg, 0.30 mmol), compound **121H** (89.49 mg, 0.30 mmol), 1-methylpyrazole (61.57 mg, 0.75 mmol), and chloro-N,N,N',N'-tetramethylformamidinium hexafluorophosphate (101.01 mg, 0.36 mmol) were successively dissolved in N,N-dimethylformamide (5 mL), and then the mixture was stirred and reacted at room temperature for 2 hours. After the reaction was completed, the mixture was filtered and the filtrate was separated and purified using a reverse-phase preparative column (C18 spherical 20-35 nm 100A 40 g) (acetonitrile : water = 10%-80%) to obtain compound **165** (36 mg, 28.12%);

¹H NMR (400 MHz, Chloroform-d) δ 7.31-7.29 (m, 1H), 6.37-6.35 (m, 1H), 5.65-6.53 (m, 1H), 5.50-5.49 (m, 1H), 5.26-5.18 (m, 1H), 4.95-4.94 (m, 2H), 4.74-4.70 (m, 2H), 4.64-4.54 (m, 2H), 4.23-4.19 (m, 2H), 3.70-3.67 (m, 2H), 3.45-3.44(m, 3H), 3.31-3.12 (m, 3H), 2.95-2.86 (m, 2H), 2.77-2.75 (m, 2H), 2.13-2.12 (m, 3H).

LC-MS (ESI): m/z = 421.3 [M+H]⁺;

### Example 166:

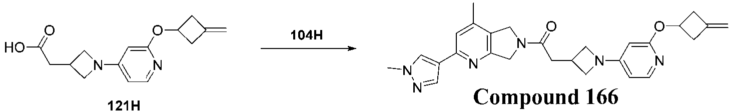

**104H** (50 mg, 0.23 mmol), compound **121H** (69.40 mg, 0.25 mmol), 1-methylpyrazole (47.21 mg, 0.58 mmol), and chloro-N,N,N',N'-tetramethylformamidinium hexafluorophosphate (77.44 mg, 0.28 mmol) were successively dissolved in N,N-dimethylformamide (5 mL), and then the mixture was stirred and reacted at room temperature for 2 hours. After the reaction was completed, the mixture was filtered and the filtrate was separated and purified using a reverse-phase preparative column (C18 spherical 20-35 nm 100 A 40 g) (acetonitrile : water = 10%-80%) to obtain compound **166** (13 mg, 11.84%);

¹H NMR (400 MHz, Chloroform-d) δ 8.26-7.81 (m, 2H), 7.30-7.28 (m, 2H), 5.67 -5.56 (m, 1H), 5.42-5.41 (m, 1H), 5.29-5.21 (m, 1H), 4.99-4.69 (m, 6H), 4.25-4.21 (m, 2H), 3.96-3.95 (m, 3H), 3.72-3.69 (m, 2H), 3.30-3.12 (m, 3H), 2.92-2.87 (m, 2H), 2.81-2.78 (m, 2H), 2.35-2.32 (m, 3H).

LC-MS (ESI): m/z = 471.1 [M+H]⁺.

### Example 167:

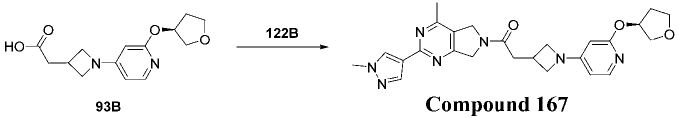

With reference to the synthesis method of step 3 in Example 122, compound **167** (23 mg, 15.09%) was obtained using **93B** (0.078 g, 0.28 mmol) and intermediate **122B** (0.06 g, 0.28 mmol) as starting materials.

LC-MS (ESI): m/z= 476.3 [M+H]⁺.

¹H NMR (400 MHz, CDCl₃): δ 8.18-8.16 (m, 1H), 8.10-8.08 (m, 1H), 7.84-7.82 (m, 1H), 6.01-6.00 (m, 1H), 5.65-5.64 (m, 1H), 5.59 (s, 1H), 4.79-4.76 (m, 4H), 4.24- 4.20 (m, 2H), 4.03-3.85 (m, 7H), 3.70 (s, 2H), 3.28 (s, 1H), 2.83-2.79 (m, 2H), 2.48- 2.47 (m, 3H), 2.28-2.25 (m, 1H), 2.13-2.10 (m, 1H).

### Example 168:

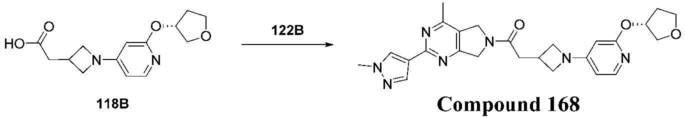

With reference to the synthesis method of step 3 in Example **122,** compound **168** (50 mg, 37.72%) was obtained using **118B** (0.078 g, 0.28 mmol) and intermediate **122B** (0.060 g, 0.28 mmol) as starting materials.

LC-MS (ESI): m/z= 476.2 [M+H]⁺.

¹H NMR (400 MHz, CDCl₃): δ 8.18-8.16 (m, 1H), 8.10-8.08 (m, 1H), 7.83-7.82 (m, 1H), 6.01-5.99 (m, 1H), 5.65-5.64 (m, 1H), 5.59 (s, 1H), 4.79-4.76 (m, 4H), 4.24- 4.20 (m, 2H), 4.03-3.85 (m, 7H), 3.69 (s, 2H), 3.27 (s, 1H), 2.83-2.78 (m, 2H), 2.48- 2.47 (m, 3H), 2.27-2.24 (m, 1H), 2.13-2.11 (m, 1H).

### Example 169:

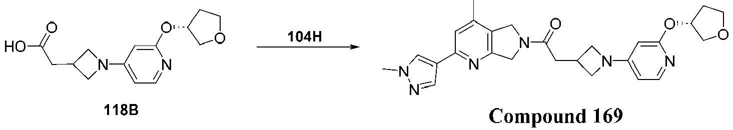

With reference to the synthesis method of step 3 in Example **122,** compound **169** (30 mg, 22.58%) was obtained using **118B** (0.078 g, 0.28 mmol) and intermediate **104H** (0.06 g, 0.28 mmol) as starting materials.

LC-MS (ESI): m/z= 475.3 [M+H]⁺.

¹H NMR (400 MHz, CDCl₃): δ 7.94-7.89 (m, 2H), 7.83-7.81 (m, 1H), 7.20-7.19 (m, 1H), 6.01-5.99 (m, 1H), 5.65-5.64 (m, 1H), 5.58 (s, 1H), 4.80-4.75 (m, 4H), 4.23- 4.19 (m, 2H), 4.03-3.85 (m, 7H), 3.69 (s, 2H), 3.27 (s, 1H), 2.83-2.79 (m, 2H), 2.32- 2.31 (m, 3H), 2.28-2.21 (m, 1H), 2.13-2.10 (m, 1H).

### Example 170:

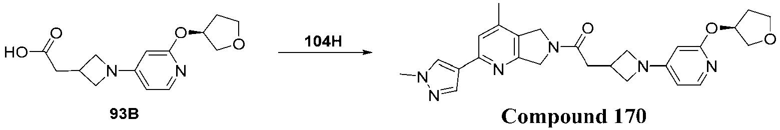

With reference to the synthesis method of step 3 in Example **122,** compound **170** (15 mg, 11.29%) was obtained using **93B** (0.078 g, 0.28 mmol) and intermediate **104H** (0.06 g, 0.28 mmol) as starting materials.

LC-MS (ESI): m/z= 475.3 [M+H]⁺.

¹H NMR (400 MHz, CDCl₃): δ 7.94-7.89 (m, 2H), 7.84-7.82 (m, 1H), 7.21-7.20 (m, 1H), 6.02-6.00 (m, 1H), 5.64-5.63 (m, 1H), 5.60 (s, 1H), 4.80-4.69 (m, 4H), 4.23- 4.21 (m, 2H), 4.03-3.86 (m, 7H), 3.72 (s, 2H), 3.29 (s, 1H), 2.83-2.79 (m, 2H), 2.32- 2.31 (m, 3H), 2.28-2.21 (m, 1H), 2.14-2.10 (m, 1H).

### Example 171:

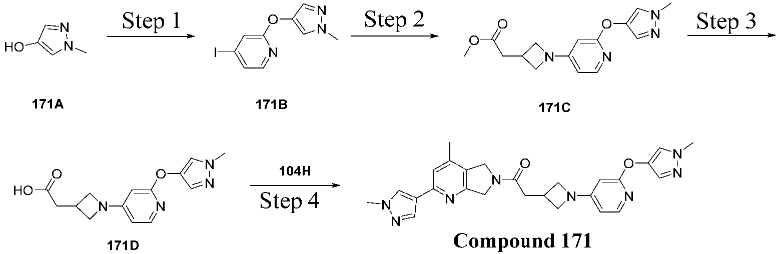

Step 1: With reference to the synthesis method of step 1 in Example **25,** compound **171B** (0.40 g, 76.67%) was obtained using **171A** (0.17 g, 1.73 mmol) and 2-fluoro-4-iodopyridine (0.46 g, 2.1 mmol) as starting materials.

LC-MS (ESI): m/z= 302.1 [M+H]⁺.

Step 2: With reference to the synthesis method of step 2 in Example **59,** compound **171C** (0.26 g, 83.53%) was obtained using **171B** (0.31 g, 1.03 mmol) and methyl 2-(azetidin-3-yl)acetate (0.16 g, 1.24 mmol) as starting materials.

LC-MS (ESI): m/z= 303.3 [M+H]⁺.

Step 3: With reference to the synthesis method of step 3 in Example **59,** compound **171D** (0.25 g, 99.99%) was obtained using **171C** (0.26 g, 0.86 mmol) as a starting material.

LC-MS (ESI): m/z= 289.1 [M+H]⁺.

Step 4: With reference to the synthesis method of step 3 in Example **122,** compound **171** (35 mg, 25.91%) was obtained using **171D** (0.080 g, 0.28 mmol) and intermediate **104H** (0.060 g, 0.28 mmol) as starting materials.

LC-MS (ESI): m/z= 485.1 [M+H]⁺.

¹H NMR (400 MHz, CDCl₃): δ 7.94-7.91 (m, 2H), 7.88-7.86 (m, 1H), 7.47 (s, 1H), 7.40 (s, 1H), 7.21-7.20 (m, 1H), 6.07-6.05 (m, 1H), 5.74-5.73 (m, 1H), 4.80-4.75 (m, 4H), 4.26-4.22 (m, 2H), 3.96-3.95 (m, 3H), 3.88 (s, 3H), 3.75-3.72 (m, 2H), 3.31- 3.28 (m, 1H), 2.84-2.79 (m, 2H), 2.32-2.31 (m, 3H).

### Example 172:

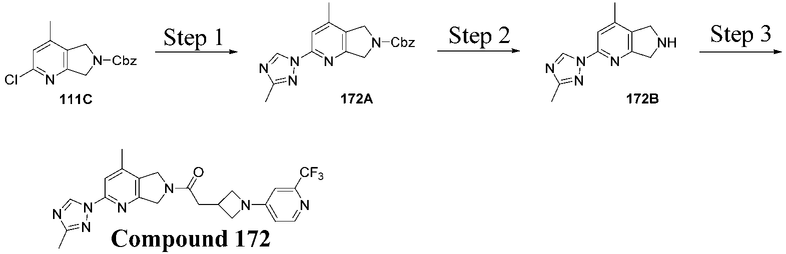

Step 1: **111C** (300 mg, 0.99 mmol) was dissolved in DMF (5 mL), 3-methyl-1H-1,2,4-triazole (165 mg, 1.98 mmol) and caesium carbonate (0.97 g, 2.97 mmol) were successively added, and then the mixture was warmed to 130°C and reacted for 48 hours. After the reaction was completed, the reaction mixture was cooled to room temperature. EA (20 mL) was added, and the organic phase was washed with saturated aqueous sodium chloride solution (30 mL×3), dried over anhydrous sodium sulphate, filtered and concentrated under reduced pressure. The residue was separated and purified by silica gel column chromatography to obtain compound **172A** (50 mg, 14.44%).

LC-MS (ESI): m/z = 350.2 [M+H]⁺.

Step 2: **172A** (50 mg, 0.14 mmol) was dissolved in methanol (10 mL) at room temperature, and 10% palladium on carbon (74 mg, 0.07 mmol) was added. The reaction was continued for 16 hours under hydrogen atmosphere. Upon complete depletion of starting materials as monitored by TLC, the reaction was stopped. The reaction mixture was filtered and the filtrate was concentrated under reduced pressure to obtain compound **172B** (28 mg, 90.90%), which was directly used in the next reaction without further purification.

LC-MS (ESI): m/z = 216.1 [M+H]⁺.

Step 3: With reference to the synthesis method of step 7 in Example 4, compound **172** (10 mg, 16.81%) was obtained using **172B** (28 mg, 0.13 mmol) and **4D** (41 mg, 0.16 mmol) as starting materials.

LC-MS (ESI): m/z= 458.0 [M+H]⁺.

¹H NMR (400 MHz, DMSO-*d6*) δ 9.17-9.14 (d, 1H), 8.22-8.20 (d, 1H), 7.63-7.62 (d, 1H), 6.71 (s, 1H), 6.54-6.53 (m, 1H), 4.90-4.89 (d, 2H), 4.67-4.65 (d, 2H), 4.21-4.17 (t, 2H), 3.73-3.70 (m, 2H), 3.18-3.10 (m, 1H), 2.89-2.87 (d, 2H), 2.39-2.38 (d, 6H).

### Example 173:

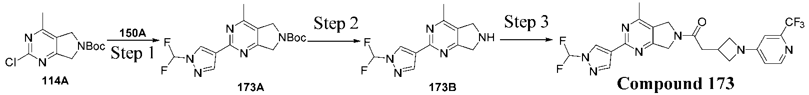

Step 1: With reference to the operation method of step 1 in Example **150, compound 173A** (325 mg, yield: 78.92%) was obtained using **114A** (250 mg, 0.93 mmol) and **150A** (272 mg, 1.12 mmol) as starting materials.

LC-MS (ESI): m/z = 352.2 [M+H]⁺.

Step 2: Compound **173A** (257 mg, 0.73 mmoL) was dissolved in DCM (2 mL), hydrogen chloride (10 mL, 4 M in 1,4-dioxane) was added, and then the mixture was reacted at room temperature for 1 hour. After the reaction was completed, the mixture was concentrated to obtain crude compound **173B** (259 mg), which was directly used in the next step without further purification.

Step 3: With reference to the operation process of step 3 in Example **112, compound 173** (101 mg, two-step yield: 28%) was obtained using crude product **173B** (259 mg) and **4D** (150 mg, 0.58 mmol) as starting materials.

LC-MS (ESI): m/z = 494.0 [M+H]⁺.

¹H NMR (400 MHz, DMSO-d6) δ 8.86-8.82 (m, 1H), 8.36-8.33 (m, 1H), 8.23- 8.20 (m, 1H), 8.03-7.69 (m, 1H), 6.74-6.70 (m, 1H), 6.56-6.51 (m, 1H), 4.94-4.88 (m, 2H), 4.70-4.63 (m, 2H), 4.22-4.15 (m, 2H), 3.75-3.68 (m, 2H), 3.19-3.08 (m, 1H), 2.87 (d, J = 7.7 Hz, 2H), 2.49-2.46 (m, 3H).

### Example 174:

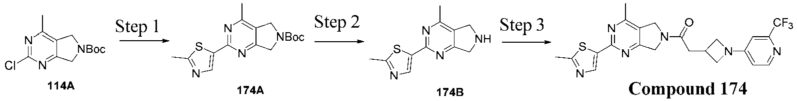

Step 1: With reference to the operation process of step 1 in Example **150, compound 174A** (143 mg, yield: 77.35%) was obtained using **114A** (150 mg, 0.56 mmol) and 2-methylthiazole-5-boronic acid pinacol ester (151 mg, 0.67 mmol) as starting materials.

LC-MS (ESI): m/z = 333.1 [M+H]⁺.

Step 2: Compound **174A** (143 mg, 0.43 mmol) was dissolved in DCM (2 mL), hydrogen chloride (10 mL, 4 M in 1,4-dioxane) was added, and then the mixture was reacted at room temperature for 1 hour. After the reaction was completed, the mixture was concentrated to obtain crude compound **174B** (127 mg), which was directly used in the next step without further purification.

### Step 3:

With reference to the operation process of step 3 in Example **112, compound 174** (21 mg, two-step yield: 10.30%) was obtained using crude product **174B** (127 mg) and **4D** (150 mg, 0.58 mmol) as starting materials.

LC-MS (ESI): m/z = 475.0 [M+H]⁺.

¹H NMR (400 MHz, DMSO-d6) δ 8.36-8.33 (m, 1H), 8.23-8.19 (m, 1H), 6.74- 6.70 (m, 1H), 6.57-6.51 (m, 1H), 4.94-4.87 (m, 2H), 4.70-4.62 (m, 2H), 4.21-4.15 (m, 2H), 3.74-3.69 (m, 2H), 3.18-3.09 (m, 1H), 2.87 (dd, J = 7.7, 3.6 Hz, 2H), 2.70 (s, 3H), 2.48-2.44 (m, 3H).

### Example 175:

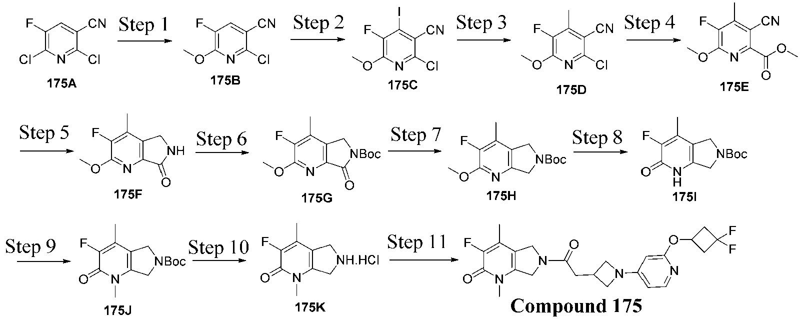

Step 1: **175A** (50 g, 261.79 mmol) was dissolved in methanol (500 mL), and a solution of sodium methoxide (42.85 g, 261.79 mmol, 33%) in methanol was added dropwise at 20°C. After the dropwise addition was completed, the mixture was stirred at room temperature for 16 h. A solid was precipitated in the reaction process, water (1000 mL) was added, and the mixture was filtered. The solid was washed with water (300 mL) and dried to obtain **175B** (40 g, 81.90%).

¹H NMR (400 MHz, CDCl₃) δ 7.58 (d, 1H), 4.11 (s, 3H).

Step 2: **175B** (25 g, 134.00 mmol) and iodine (68.02 g, 268 mmol) were dissolved in tetrahydrofuran (250 mL). Under nitrogen atmosphere, lithium diisopropylamide (43.06 g, 402 mmol) was slowly added dropwise at -78°C and the mixture was stirred for 1 h. Then the mixture was slowly warmed to room temperature and stirred for 2 h. Aqueous sodium thiosulphate solution (300 mL) was added. The mixture was extracted with ethyl acetate (200 mL×3). The combined organic phases were washed with saturated aqueous sodium chloride solution and concentrated under reduced pressure. The residue was separated by silica gel column chromatography to obtain **175C** (35 g, 83.59%).

¹H NMR (400 MHz, CDCl3) 4.10 (s, 3H).

Step 3: **175C** (34 g, 108.81 mmol), methylboronic acid (32.57 g, 544.05 mmol),
1,1'-bis(diphenylphosphino)ferrocene palladium dichloride (II) (7.96 g, 10.88 mmol), and potassium phosphate (46.19 g, 217.62 mmol) were successively added to mixed solvents of 1,4-dioxane (350 mL) and water (30 mL), and under nitrogen atmosphere, the mixture was warmed to 80°C and reacted for 16 h. After the reaction was completed, the mixture was cooled to room temperature and filtered. The filtrate was concentrated under reduced pressure to remove most of 1,4-dioxane and then water (200 mL) was added. The mixture was extracted with ethyl acetate (150 mL×3). The organic phases were combined, washed with saturated aqueous sodium chloride solution and concentrated under reduced pressure. The residue was separated and purified by silica gel column chromatography to obtain **175D** (14 g, 64.14%).

¹H NMR (400 MHz, CDCl₃) δ 4.08 (s, 3H), 2.47 (d, 3H).

Step 4: **175D** (13 g, 64.81 mmol), 1,1'-bis(diphenylphosphino)ferrocene palladium dichloride (II) (4.74 g, 6.48 mmol) and triethylamine (9.84 g, 97.22 mmol) were added to methanol (150 mL), and the mixture was warmed to 100°C and reacted under CO gas (3 MPa) atmosphere for 16 h. After the reaction was completed, the mixture was cooled to room temperature and concentrated under reduced pressure. Water (150 mL) was added, and the resulting mixture was extracted with dichloromethane (100 mL×3). The organic phases were combined and concentrated under reduced pressure. The residue was separated and purified by silica gel column chromatography to obtain **175E** (7 g, 48.18%).

LC-MS (ESI): m/z = 225.1 [M+H-100]⁺.

Step 5: **175E** (7 g, 31.22 mmol) and raney nickel (1.83 g, 31.22 mmol) were added to methanol (140 mL), and the mixture was reacted at 40°C under H₂ atmosphere for 16 h. DCM (50 mL) was added and the mixture was filtered. The filter cake was washed with methanol and the filtrate was concentrated under reduced pressure to obtain crude product **175F** (6 g, 97.95%).

LC-MS (ESI): m/z = 197.1 [M+H]⁺.

Step 6: **175F** (6 g, 30.58 mmol), di-tert-butyl dicarbonate (10.01 g, 45.87 mmol), and triethylamine (4.64 g, 45.87 mmol) were dissolved in tetrahydrofuran (60 mL), 4-dimethylaminopyridine (DMAP) (0.37 g, 3.06 mmol) was added, and then the mixture was reacted at room temperature for 2 h. Water (50 mL) was added and the mixture was extracted with ethyl acetate (50 mL×3). The combined organic phases were washed with dilute hydrochloric acid (pH = 2-3) and saturated aqueous sodium chloride solution and concentrated under reduced pressure to obtain crude product **175G** (8 g, 88.28%).

LC-MS (ESI): m/z = 297.1 [M+H]⁺.

Step 7: **175G** (6 g, 20.25 mmol) was dissolved in tetrahydrofuran (60 mL). Borane dimethyl sulphide complex (4.62 g, 60.75 mmol) was added and then the mixture was reacted at 70°C for 3 h. After the reaction was completed, the resulting mixture was cooled to room temperature, and methanol (20 mL) was added dropwise in an ice bath to quench the reaction. After the dropwise addition was completed, the mixture was stirred for another 2 h. The reaction solution was concentrated under reduced pressure. Water (60 mL) was added, and the resulting mixture was extracted with ethyl acetate (50 mL×3). The combined organic phases were washed with saturated aqueous sodium chloride solution and concentrated under reduced pressure. The residue was separated and purified by silica gel column chromatography to obtain **175H** (4 g, 69.97%)

LC-MS (ESI): m/z = 283.1[M+H]⁺.

Step 8: **175H** (3 g, 10.63 mmol) and sodium iodide (7.17 g, 47.84 mmol) were successively dissolved in acetonitrile (60 mL), and trimethylchlorosilane (4.62 g, 42.52 mmol) was added. Subsequently, the mixture was warmed to 85°C and reacted for 3 h. The mixture was cooled to room temperature. Water (40 mL), sodium bicarbonate (3.57g, 42.52 mmol) and di-tert-butyl dicarbonate (3.48 g, 15.95 mmol) were successively added, and the mixture was reacted at room temperature for 16 h. The mixture was concentrated under reduced pressure to remove most of acetonitrile and filtered. The filter cake was washed with water and ethyl acetate (20 mL) and dried to obtain **175I** (1.4 g, 49.11%).

¹H NMR (400 MHz, DMSO-d6) δ 4.40-4.31 (m, 4H), 2.09-2.05 (m, 3H), 1.44 (d, 9H).

LC-MS (ESI): m/z = 269.1 [M+H]⁺.

Step 9: **175I** (0.4 g, 1.49 mmol), potassium carbonate (0.41 g, 2.98 mmol), and iodomethane (1.06 g, 7.45 mmol) were successively dissolved in N,N-dimethylformamide (10 mL), and then the mixture was reacted at room temperature for 3 h. Water (50 mL) was added, and the resulting mixture was extracted with ethyl acetate (20 mL×3). The combined organic phases were washed with saturated aqueous sodium chloride solution and concentrated under reduced pressure. The residue was separated and purified by silica gel column chromatography to obtain **175J** (0.3 g, 71.27%).

LC-MS (ESI): m/z = 283.1[M+H]⁺.

Step 10: **175J** (0.1 g, 0.35 mmol) was dissolved in 4 mol/L hydrogen chloride dioxane solution (3 mL, 4 M), and the mixture was reacted at room temperature for 2 h. The reaction solution was concentrated under reduced pressure to obtain **175K** (0.08 g).

LC-MS (ESI): m/z = 183.1[M+H]⁺.

Step 11: **175K** (0.08 g, 0.37 mmol), **81D** (0.11 g, 0.37 mmol), N,N-diisopropylethylamine (0.24 g, 1.85 mmol), and 2-(7-azobenzotriazole)-N,N,N',N'-tetramethyluronium hexafluorophosphate (0.21 g, 0.55 mmol) were successively dissolved in N,N-dimethylformamide (5 mL), and the mixture was reacted at room temperature for 16 h. Water (30 mL) was added, and the resulting mixture was extracted with ethyl acetate (20 mL×3). The combined organic phases were washed with saturated aqueous sodium chloride solution and concentrated under reduced pressure. The residue was purified by HPLC to obtain compound **175** (0.06 g, 35.46%).

LC-MS (ESI): m/z = 463.2[M+H]⁺.

¹H NMR (400 MHz, CDCl₃) δ 7.80 (d, 1H), 6.01-5.97 (m, 1H), 5.63 (d, 1H), 5.17-5.07 (m, 1H), 4.75-4.57 (m, 4H), 4.22-4.14 (m, 2H), 3.67-3.61(m, 2H), 3.50 (s, 3H), 3.31-3.18 (m, 1H), 3.15-3.02 (m, 2H), 2.80-2.73 (m, 2H), 2.72-2.61 (m, 2H), 2.16-2.10 (m, 3H).

### Example 176:

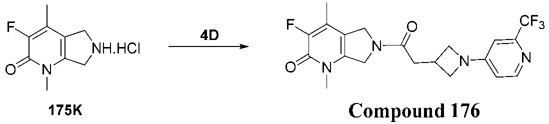

With reference to the operation process of step 11 in Example 175, compound **176** (0.05 g, 32.2%) was obtained using **175K** (0.08 g, 0.37 mmol) and **4D** (0.1 g, 0.37 mmol) as starting materials.

LC-MS (ESI): m/z = 425.2[M+H]⁺.

¹H NMR (400 MHz, CDCl₃) δ 8.29-8.23(m, 1H), 6.59-6.56 (m, 1H), 6.36-6.31(m, 1H), 4.78-4.57 (m, 4H), 4.33-4.25 (m, 2H), 3.83-3.72 (m, 2H), 3.52-3.48 (m, 3H), 3.38-3.27 (m, 1H), 2.85-2.78 (m, 2H), 2.17-2.11 (m, 3H).

### Example 177:

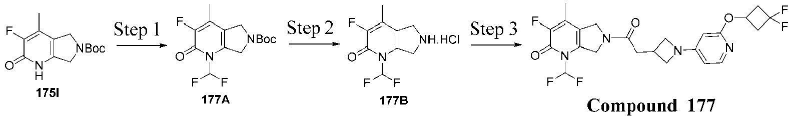

Step 1: **175I** (1 g, 3.73 mmol) was dissolved in tetrahydrofuran (20 mL), sodium tert-butoxide (1.79 g, 18.65 mmol) was added, and the mixture was stirred at room temperature for 20 min. Trimethyl(bromodifluoromethyl)silane (2.27 g, 11.19 mmol) was added dropwise. After the dropwise addition was completed, the mixture was reacted at room temperature for 16 h. Water (20 mL) was added. The mixture was extracted with ethyl acetate (20 mL × 3), the combined organic phases were washed with saturated aqueous sodium chloride solution, dried over anhydrous sodium sulphate and filtered, and the filtrate was concentrated under reduced pressure. Then the residue was separated and purified by silica gel column chromatography to obtain 177A (0.45 g, 37.93%).

¹H NMR (400 MHz, CDCl₃) δ 7.99-7.65 (m, 1H), 4.80-4.67 (m, 2H), 4.47-4.37 (m, 2H), 2.14 (d, 3H), 1.52 (s, 9H).

Step 2: **177A** (0.45 g, 1.41 mmol) was dissolved in hydrogen chloride dioxane (5 mL, 4 M), and the mixture was reacted at room temperature for 2 h. The reaction solution was concentrated under reduced pressure to obtain **177B** (0.37 g).

LC-MS (ESI): m/z = 219.1[M+H]⁺.

Step 3: **177B** (0.07 g, 0.32 mmol), **81D** (0.095 g, 0.32 mmol), N,N-diisopropylethylamine (0.21 g, 1.6 mmol), and 2-(7-azobenzotriazole)-N,N,N',N'-tetramethyluronium hexafluorophosphate (0.18 g, 0.48 mmol) were dissolved in N,N-dimethylformamide (5 mL), and the mixture was reacted at room temperature for 16 h. Water (20 mL) was added and the mixture was extracted with ethyl acetate (20 mL×3). The combined organic phases were washed with saturated aqueous sodium chloride solution and concentrated under reduced pressure to obtain a crude product. The crude product was further separated and purified by preparative HPLC to obtain compound **177** (0.05 g, 31.27%).

LC-MS (ESI): m/z = 499.2[M+H]⁺.

¹H NMR (400 MHz, CDCl₃) δ 8.00-7.66 (m, 2H), 6.04-5.95 (m, 1H), 5.62 (s, 1H), 5.19-5.08 (m, 1H), 4.87 (s, 2H), 4.60-4.52 (m, 2H), 4.24-4.15 (m, 2H), 3.73-3.59 (m, 2H), 3.29-3.20 (m, 1H), 3.15-3.02 (m, 2H), 2.81-2.59 (m, 4H), 2.19-2.14 (m, 3H).

### Example 178:

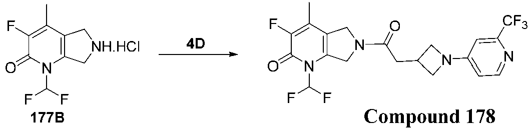

With reference to the operation process of step 3 in Example 177, compound **178** (0.05 g, 33.85%) was obtained using **177B** (0.07 g, 0.32 mmol) and **4D** (0.083 g,0.32 mmol) as starting materials.

LC-MS (ESI): m/z = 461.1[M+H]⁺.

¹H NMR (400 MHz, CDCl₃) δ 8.30-8.23 (m, 1H), 8.00-7.67 (m, 1H), 6.59-6.56 (m, 1H), 6.37-6.31 (m, 1H), 4.90-4.85 (m, 2H), 4.62-4.52 (m, 2H), 4.33-4.24 (m, 2H), 3.81-3.72 (m, 2H), 3.38-3.25 (m, 1H), 2.84-2.76 (m, 2H), 2.20-2.14 (m, 3H).

### Example 179:

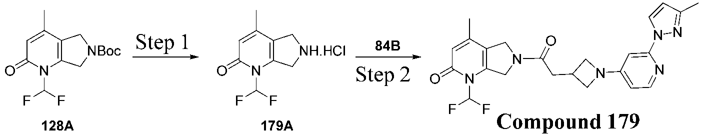

Step 1: **128A** (2 g, 6.66 mmol) was dissolved in hydrogen chloride dioxane (30 mL, 4 M), and the mixture was reacted at room temperature for 6 h. The reaction solution was concentrated under reduced pressure to obtain **179A** (1.6 g).

LC-MS (ESI): m/z = 201.2[M+H]⁺.

Step 2: **179A** (0.04 g, 0.17 mmol), **84B** (0.046 g, 0.17 mmol), N,N-diisopropylethylamine (0.11 g, 0.85 mmol), and 2-(7-azobenzotriazole)-N,N,N',N'-tetramethyluronium hexafluorophosphate (0.097 g, 0.26 mmol) were successively dissolved in N,N-dimethylformamide (5 mL), and the mixture was reacted at room temperature for 16 h. Water (30 mL) was added and the mixture was extracted with ethyl acetate (20 mL×3). The combined organic phases were washed with saturated aqueous sodium chloride solution and concentrated under reduced pressure to obtain a crude product. The crude product was further separated and purified by preparative HPLC to obtain compound **179** (0.032 g, 41.66%).

LC-MS (ESI): m/z =455.2[M+H]⁺.

¹H NMR (400 MHz, CDCl₃) δ 8.47 (s, 1H), 8.00 (d, 1H), 7.98-7.64 (m, 1H), 6.85 (d, 1H), 6.35-6.30 (m, 1H), 6.22 (d, 1H), 6.16-6.10 (m, 1H), 4.91-4.85 (m, 2H), 4.58-4.49 (m, 2H), 4.34-4.25 (m, 2H), 3.83-3.71 (m, 2H), 3.35-3.23 (m, 1H), 2.81-2.75 (m, 2H), 2.37 (s, 3H), 2.19-2.14 (m, 3H).

### Example 180:

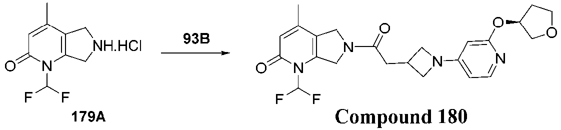

With reference to the operation process of step 2 in Example 179, compound **180** (0.025 g, 32.12%) was obtained using **179A** (0.04 g, 0.17 mmol) and **93B** (0.047 g,0.17 mmol) as starting materials.

LC-MS (ESI): m/z =461.2[M+H]⁺.

¹H NMR (400 MHz, CDCl₃) δ 7.99-7.63 (m, 2H), 6.34-6.30 (m, 1H), 6.03-5.97 (m, 1H), 5.65-5.55 (m, 2H), 4.92-4.84 (m, 2H), 4.57-4.49(m, 2H), 4.20 (t, 2H), 4.04- 3.82 (m, 4H), 3.74-3.62(m, 2H), 3.32-3.20 (m, 1H), 2.76 (d, 2H), 2.35-2.21(m, 1H), 2.18-2.14 (m, 3H), 2.14-2.07 (m, 1H).

### Example 181:

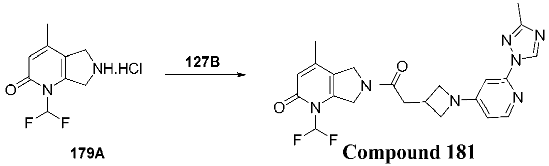

With reference to the operation process of step 2 in Example **179,** compound **181** (0.015 g, 19.48%) was obtained using **179A** (0.04 g, 0.17 mmol) and **127B** (0.046 g, 0.17 mmol) as starting materials.

LC-MS (ESI): m/z =456.2[M+H]⁺.

¹H NMR (400 MHz, CDCl₃) δ 9.57 (s, 1H), 8.04-7.98 (m, 1H), 7.97-7.63(m, 1H), 6.80-6.75 (m, 1H), 6.35-3.31 (m, 1H), 6.26-6.21 (m, 1H), 4.92-4.86 (m, 2H), 4.59-4.50(m, 2H), 4.46-4.38 (m, 2H), 3.99-3.88(m, 2H), 3.42-3.33 (m, 1H), 2.87-2.79 (m, 2H), 2.49 (s, 3H), 2.19-2.14 (m, 3H).

### Example 182:

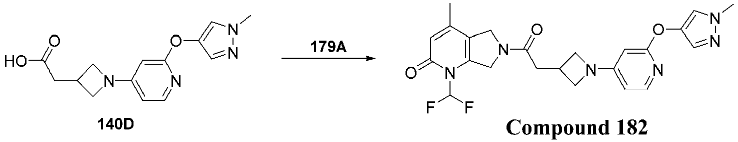

With reference to the synthesis method of step 9 in Example 99, **compound 182** (27 mg, 25.3%) was obtained using **140D** (52 mg, 0.21 mmol) and **179A** (50 mg, 0.21 mmol) as starting materials.

LC-MS (ESI): m/z =471.1 [M+H]⁺.

¹H NMR (400 MHz, DMSO-*d*₆) δ 8.13-7.79 (m, 1H), 7.78-7.70 (m, 2H), 7.32 (s, 1H), 6.39-6.29 (m, 1H), 6.26-6.07 (m, 1H), 5.84-5.76 (m, 1H), 4.93 (s, 1H), 4.69-4.59 (m, 2H), 4.39 (s, 1H), 4.10-3.99 (m, 2H), 3.79 (s, 3H), 3.65-3.52 (m, 2H), 3.12-2.98 (m, 1H), 2.87-2.76 (m, 2H), 2.19-2.06 (m, 3H).

### Example 183:

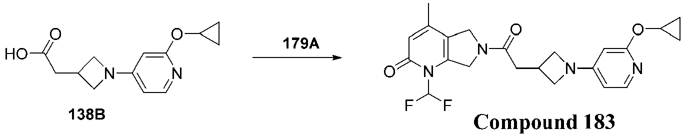

With reference to the synthesis method of step 9 in Example 99, compound **183** (28 mg, 31.1%) was obtained using **138D** (100 mg, 0.21 mmol) and **179A** (50 mg, 0.21 mmol) as starting materials.

LC-MS (ESI): m/z =431.0 [M+H]⁺.

¹H NMR (400 MHz, DMSO-*d*₆) δ 8.15-7.80 (m, 1H), 7.79-7.71 (m, 1H), 6.36- 6.28 (m, 1H), 6.12-5.93 (m, 1H), 5.77-5.61 (m, 1H), 4.92 (s, 1H), 4.70-4.55 (m, 2H), 4.39 (s, 1H), 4.13-4.08 (m, 1H), 4.07-3.98 (m, 2H), 3.60-3.53 (m, 2H), 3.12-2.99 (m, 1H), 2.88-2.77 (m, 2H), 2.17-2.11 (m, 3H), 0.72-0.64 (m, 2H), 0.61-0.55 (m, 2H).

### Example 184:

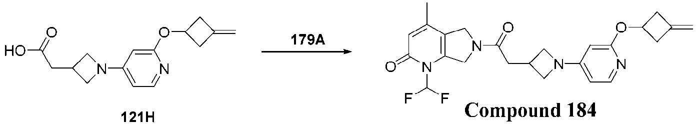

**179A** (66 mg, 0.28 mmol), compound **121H** (84.49 mg, 0.31 mmol), 1-methylpyrazole (57.47 mg, 0.70 mmol), and chloro-N,N,N',N'-tetramethylformamidinium hexafluorophosphate (94.27 mg, 0.34 mmol) were successively dissolved in N,N-dimethylformamide (5 mL), and the mixture was reacted at room temperature for 2 hours. After the reaction was completed, the mixture was filtered and the filtrate was separated and purified using a reverse-phase preparative column to obtain compound **184** (52 mg, 40.84%);

1H NMR (400 MHz, DMSO-*d*₆) δ 8.15-7.76 (m, 1H), 7.64-7.62 (m, 1H), 6.34-6.32 (m, 1H), 5.78-5.75 (m, 1H), 5.08-5.07 (m, 1H), 5.03-4.86 (m, 4H), 4.68-4.59 (m, 2H), 4.39-4.38 (m, 1H), 4.12-4.05 (m, 3H), 3.65-3.61 (m, 2H), 3.07-2.97 (m, 5H), 2.83-2.78 (m, 2H), 2.15-2.14 (m, 3H).

LC-MS (ESI): m/z = 457.3[M+H]⁺.

### Examples 185 and 186:

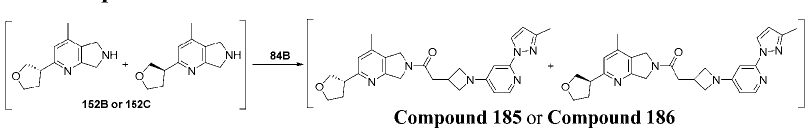

With reference to the procedures of step 7 in Example 4, **compound 185** (36 mg, 43%) was obtained using **152B** (37 mg, 0.18 mmol) and **84B** (50 mg, 0.18 mmol) as starting materials.

LC-MS (ESI): m/z = 459.3 [M+H]⁺.

¹H NMR (400 MHz, CDCl₃) δ 8.53 (s, 1H), 8.01-7.99 (m, 1H), 6.96 (s, 1H), 6.86 (s, 1H), 6.24-6.21 (m, 1H), 6.16-6.12 (m, 1H), 4.82-4.70 (m, 4H), 4.35-4.30 (m, 2H), 4.17-4.12 (m, 1H), 4.11-4.04 (m, 1H), 3.96-3.84 (m, 2H), 3.83-3.76 (m, 2H), 3.60-3.50 (m, 1H), 3.36-3.26 (m, 1H), 2.84-2.79 (m, 2H), 2.41-2.31 (m, 4H), 2.30-2.27 (m, 3H), 2.23-2.15 (m, 1H).

With reference to the procedures of step 7 in Example 4, **compound 186** (33 mg, 37%) was obtained using **152C** (40 mg, 0.20 mmol) and **84B** (54 mg, 0.20 mmol) as starting materials.

LC-MS (ESI): m/z = 459.3 [M+H]⁺.

¹H NMR (400 MHz, CDCl₃) δ 8.40 (s, 1H), 8.01-7.99 (m, 1H), 6.96 (s, 1H), 6.85 (s, 1H), 6.22-6.20 (m, 1H), 6.14-6.11 (m, 1H), 4.82-4.71 (m, 4H), 4.34-4.25 (m, 2H), 4.17-4.12 (m, 1H), 4.11-4.04 (m, 1H), 3.96-3.84 (m, 2H), 3.83-3.76 (m, 2H), 3.60-3.50 (m, 1H), 3.35-3.24 (m, 1H), 2.84-2.78 (m, 2H), 2.41-2.31 (m, 4H), 2.39-2.27 (m, 3H), 2.24-2.13 (m, 1H).

### Example 187:

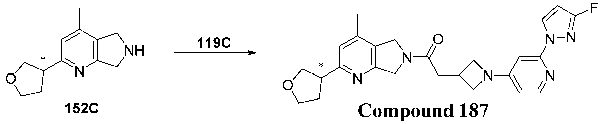

With reference to the procedures of step 7 in Example 4, **compound 187** (41 mg, 49%) was obtained using **152C** (37 mg, 0.22 mmol) and **119C** (50 mg, 0.22 mmol) as starting materials.

LC-MS (ESI): m/z = 463.2 [M+H]⁺.

¹H NMR (400 MHz, CDCl₃) δ 8.40 (s, 1H), 8.00-7.97 (m, 1H), 6.96 (s, 1H), 6.73 (s, 1H), 6.16-6.13 (m, 1H), 6.01-5.98 (m, 1H), 4.83-4.71 (m, 4H), 4.31-4.25 (m, 2H), 4.17-4.12 (m, 1H), 4.11-4.04 (m, 1H), 3.96-3.85 (m, 2H), 3.78-3.73 (m, 2H), 3.60-3.50 (m, 1H), 3.34-3.25 (m, 1H), 2.84-2.79 (m, 2H), 2.41-2.31 (m, 1H), 2.30-2.27 (m, 3H), 2.23-2.12 (m, 1H).

### Example 188:

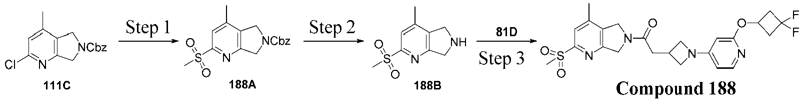

Step 1: Compound **111C** (0.18 g, 0.6 mmol), sodium methanesulphonate (0.25 g, 1.8 mmol), copper trifluoromethanesulphonate-toluene complex (0.12 g, 0.24 mmol), and N,N'-dimethylethylenediamine (0.03 g, 0.36 mmol) were successively dissolved in DMSO (12 mL). Subsequently, under nitrogen atmosphere, the mixture was warmed to 120°C and reacted for 24 hours. After the reaction was completed, the reaction solution was cooled to room temperature and poured into water. Then the mixture was extracted with ethyl acetate. The organic phase was washed with saturated aqueous sodium chloride solution and concentrated under reduced pressure. The resulting residue was purified by column chromatography to obtain compound **188A** (0.12 g, 58.27%).

LC-MS (ESI): m/z= 347.0 [M+H]⁺.

Step 2: With reference to the synthesis method of step 5 in Example **111,** compound **188B** (0.056 g, 76.15%) was obtained using **188A** (0.12 g, 0.35 mmol) as a starting material.

LC-MS (ESI): m/z= 213.0 [M+H]⁺.

Step 3: With reference to the synthesis method of step 4 in Example **81,** compound **188** (40 mg, 30.78%) was obtained using **188B** (0.056 g, 0.26 mmol) and intermediate **81D** (0.078 g, 0.26 mmol) as starting materials.

LC-MS (ESI): m/z= 493.0 [M+H]⁺.

¹H NMR (400 MHz, CDCl₃): δ 7.88-7.87 (m, 1H), 7.81-7.80 (m, 1H), 6.01-5.99 (m, 1H), 5.64-5.63 (m, 1H), 5.12-5.11 (m, 1H), 4.89-4.83 (m, 4H), 4.20-4.18 (m, 2H), 3.70-3.65 (m, 2H), 3.28-3.24 (m, 1H), 3.23-3.20 (m, 3H), 3.14-3.03 (m, 2H), 2.83-2.79 (m, 2H), 2.75-2.62 (m, 2H), 2.44-2.43 (m, 3H).

### Example 189:

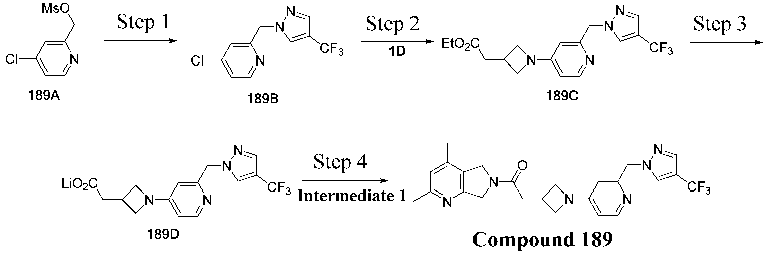

Step 1: 4-(Trifluoromethyl)-1H-pyrazole (1.23 g, 9.02 mmol) was dissolved in DMF (20 mL). Under nitrogen atmosphere, the mixture was cooled to 0°C, and NaH (722 mg, 18.04 mmol, 60%) was added in portions. After the addition, the mixture was stirred at 0°C for 30 min. Compound **189A** (2.0 g, 9.02 mmol, synthesized with reference to the method described in the patent WO 2011114148) was added and then the resulting mixture was warmed to room temperature and reacted for 18 h. After the reaction was completed, water was added to quench the reaction. The reaction mixture was extracted with ethyl acetate twice. The combined organic phases were washed with water and then washed with saturated aqueous sodium chloride solution, dried over anhydrous sodium sulphate, filtered and concentrated to obtain a crude product. The crude product was purified by column chromatography to obtain the target compound **189B** (1.1 g, yield: 46.61%).

LC-MS (ESI): m/z= 262.1 [M+H]⁺.

Step 2: **189B** (1.1 g, 4.21 mmol), methyl 2-(azetidin-3-yl)acetate trifluoroacetate (1.52 g, 6.32 mmol), caesium fluoride (1.28 g, 8.42 mmol), and triethylamine (1.28 g, 12.63 mmol) were successively dissolved in dimethyl sulphoxide (20 mL). Subsequently, the mixture was warmed to 100°C and reacted for 18 hours. After the reaction was completed, the mixture was cooled to room temperature, diluted with water (80 mL), and extracted with ethyl acetate three times (80 mL×3). The combined organic phases were dried over anhydrous sodium sulphate, filtered and concentrated under reduced pressure. The residue was purified by silica gel column chromatography to obtain compound **189C** (0.26 g, yield: 13.55%).

LC-MS (ESI): m/z = 369.2 [M+H]⁺.

Step 3: **189C** (0.26 g, 0.71 mmol) was dissolved in tetrahydrofuran (10 mL), methanol (2 mL) and water (2 mL), and then lithium hydroxide monohydrate (0.06 g, 1.42 mmol) was added. The mixture was reacted at room temperature for 2 hours. After the reaction was completed, the mixture was adjusted to about pH = 6 with 1 N HCl, and extracted with ethyl acetate (50 mL×3). The combined organic phases were washed with water and concentrated under reduced pressure to obtain crude compound **189D** (0.14 g).

LC-MS (ESI): m/z = 341.1 [M+H]⁺.

Step 4: **Intermediate** 1 (91 mg, 0.41 mmol), compound **189D** (0.14 g, crude, 0.41 mmol) and N-methylimidazole (0.169 g, 2.06 mmol) were successively dissolved in N,N-dimethylformamide (10 mL), and then chloro-N,N,N',N'-tetramethylformamidinium hexafluorophosphate (0.116 g, 0.41 mmol) was added. The mixture was reacted at room temperature for 1 hour. After the reaction was completed as monitored by TLC, the mixture was concentrated under reduced pressure. The residue was purified by silica gel column chromatography (dichloromethane : methanol (v : v) = 20 : 1) to obtain **compound 189** (0.037 g, yield: 19.17%).

LC-MS (ESI): m/z = 471.5 [M+H]⁺.

¹H NMR (400 MHz, DMSO-*d*₆) δ 8.44 (s, 1H), 8.05-8.03 (m, 1H), 7.88 (s, 1H), 6.99 (s, 1H), 6.27-6.25 (m, 1H), 6.18-6.19 (m, 1H), 5.29 (s, 2H), 4.81-4.72 (m, 2H), 4.58-4.54 (m, 2H), 4.08-4.04 (m, 2H), 3.61-3.57 (m, 2H), 3.12-3.05 (m, 1H), 2.83-2.82 (m, 2H), 2.42 (s, 3H), 2.23-2.22 (m, 3H).

### Example 190:

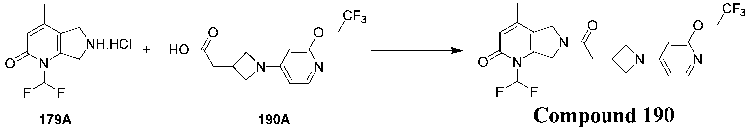

Compound **190A** (60.0 mg, 0.21 mmol, synthesized with reference to the method described in the patent WO 2024088408) and compound **179A** (50.0 mg, 0.21 mmol) were dissolved in N,N-dimethylformamide (3 mL), and N,N-diisopropylethylamine (108.0 mg, 0.84 mmol) and N,N,N',N'-tetramethyl-O-(7-azabenzotriazol-1-yl)uronium hexafluorophosphate (96.0 mg, 0.25 mmol) were successively added. After the addition was completed, the mixture was reacted at room temperature for 15 hours. After the reaction was completed, the mixture was diluted with water (20 mL), and then extracted with ethyl acetate (20 mL×3). The organic phases were combined and concentrated and the residue was purified by preparative HPLC to obtain compound 190 (40.0 mg, 40%).

LC-MS (ESI):m/z = 473.2 [M+H]⁺.

¹H NMR (400 MHz, CD₃OD) δ 7.89 (t, 1H), 7.73-7.72 (m, 1H), 6.34 (s, 1H), 6.13 -6.11 (m, 1H), 5.76-5.75 (m, 1H), 5.00-4.82 (m, 2H), 4.74-4.70 (m, 2H), 4.67-4.51 (m, 2H), 4.18-4.13 (m, 2H), 3.71-3.67 (m, 2H), 3.22-3.15 (m, 1H), 2.89-2.86 (m, 2H), 2.21 (s, 3H).

### Example 191:

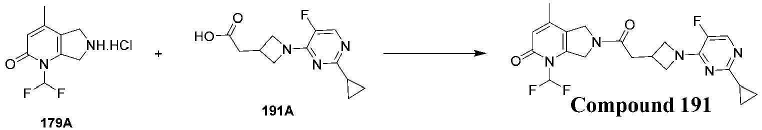

Compound **191A** (80.0 mg, 0.32 mmol, synthesized with reference to the method described in the patent WO 2024088408) and compound **179A** (75 mg, 0.32 mmol) were dissolved in N,N-dimethylformamide (3 mL), and N,N-diisopropylethylamine (165.0 mg, 1.28 mmol) and N,N,N',N'-tetramethyl-O-(7-azabenzotriazol-1-yl)uronium hexafluorophosphate (146.0 mg, 0.38 mmol) were successively added. After the addition was completed, the mixture was reacted at room temperature for 15 hours. After the reaction was completed, the reaction mixture was diluted with water (20 mL) and then extracted with ethyl acetate (20 mL×3). The organic phases were combined and concentrated. The residue was purified by preparative HPLC to obtain compound **191** (20.0 mg, 14.5%).

LC-MS (ESI):m/z = 434.2 [M+H]⁺.

¹H NMR (400 MHz, CD₃OD) δ 7.89 (t, 1H), 7.81-7.79 (m, 1H), 6.34 (s, 1H), 5.00 -4.82 (m, 2H), 4.68-4.51 (m, 2H), 4.45-4.40 (m, 2H), 3.99-3.96 (m, 2H), 3.21-3.18 (m, 1H), 2.89-2.87 (m, 2H), 2.21 (s, 3H), 1.98-1.92 (m, 1H), 0.96-0.87 (m, 4H).

### Example 192:

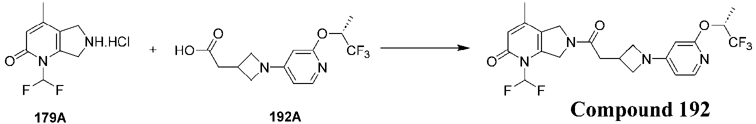

Compound **192A** (60.0 mg, 0.20 mmol, synthesized with reference to the method described in the patent WO 2024088408) and compound **179A** (47.0 mg, 2.73 mmol) were dissolved in N,N-dimethylformamide (3 mL), and N,N-diisopropylethylamine (103.0 mg, 0.80 mmol) and N,N,N',N'-tetramethyl-O-(7-azabenzotriazol-1-yl)uronium hexafluorophosphate (91.0 mg, 0.24 mmol) were successively added. After the addition was completed, the mixture was reacted at room temperature for 15 hours. After the reaction was completed, the mixture was diluted with water (20 mL), and then extracted with ethyl acetate (20 mL×3). The organic phases were combined and concentrated and the residue was purified by preparative HPLC to obtain compound 192 (45.0 mg, 46%).

LC-MS (ESI):m/z = 487.2 [M+H]⁺.

### Example 193:

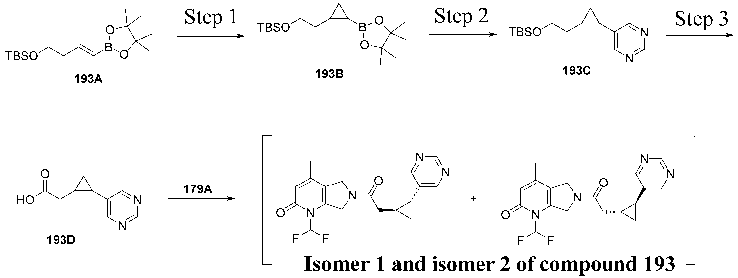

Step 1: **193A** (2.0 g, 6.40 mmol) was dissolved in dichloromethane (30 mL), and diethylzinc hexane solution (32.02 mmol, 32 mL, 1M) was added dropwise at 0°C. After the dropwise addition was completed, the mixture was stirred at 0°C for 0.5 hours. Diiodomethane (8.58 g, 32.02 mmol) and trifluoroacetic acid (3.65 g, 32.02 mmol) were added and the resulting mixture was stirred at 0°C for 1 hour, then warmed to room temperature and stirred for 2 hours. Saturated aqueous ammonium chloride solution was added dropwise to quench the reaction. The reaction mixture was extracted with dichloromethane and the organic phase was concentrated. The residue was separated and purified by silica gel chromatography to obtain compound **193B** (2.0 g, 95.7%).

LC-MS (ESI): m/z= 327.2 [M+H]⁺.

Step 2: Under nitrogen atmosphere, palladium acetate (II) (14 mg, 0.074 mmol) and di(1-adamantyl)-n-butylphosphine (cataCXium A; 39 mg, 0.12 mmol) were dissolved in 2-methyl-2-butanol (6 mL) at room temperature and the mixture was stirred for 1 h. Then water (5 mL), caesium carbonate (1.05 g, 3.68 mmol), and 5-bromopyrimidine (171 mg, 1.22 mmol) were successively added. After the mixture were stirred evenly, a solution of **193B** (0.4 g, 1.22 mmol) in 2-methylbutan-2-ol (5 mL) was added dropwise. After the dropwise addition was completed, the mixture was warmed to 75°C and reacted for 16 hours. After the reaction was completed, the reaction mixture was cooled to room temperature and filtered. The filter cake was washed with methanol, and the filtrate was concentrated. The residue was diluted with ethyl acetate (25 mL) and the organic phase was washed with water (15 mL). The aqueous phase was extracted with ethyl acetate (20 mL×2). The combined organic phases were washed with saturated aqueous sodium chloride solution (10 mL), dried over anhydrous sodium sulphate and filtered, and the filtrate was concentrated under reduced pressure. Then the residue was separated and purified by silica gel column chromatography to obtain compound **193C** (0.34 g, 76%)

LC-MS (ESI): m/z= 279.2[M+H]⁺.

Step 3: Ruthenium trichloride (8 mg, 0.04 mmol) and compound **193C** (0.34 g, 1.22 mmol) were successively dissolved in acetonitrile (6 mL). Sodium periodate (863 mg, 4.03 mmol) and water (3 mL) were added and then the mixture was reacted at room temperature for 4 hours. The reaction was quenched with 1 M hydrochloric acid (3 mL), and the reaction mixture was concentrated. To the residue was added acetonitrile (20 mL). The mixture was stirred for 5 minutes and filtered. The filtrate was concentrated to obtain compound **193D** (206 mg, 95%).

LC-MS (ESI): m/z= 179.2[M+H]⁺.

Step 4: With reference to the synthesis method of step 3 in Example **17,** compound **193** (0.11 g, 63.5%) was obtained using **193D** (0.12 g, 0.36 mmol) and **179A** (0.2 g, 0.72 mmol) as starting materials. The compound was further subjected to chiral preparative HPLC to obtain compound **193,** isomer 1 (25 mg, 41%) and compound **193,** isomer 2 (22 mg, 38%). Chiral HPLC analytical method: 1. instrument: SHIMADZU LC-30ADsf; 2. chromatographic column: Chiral Whelk column; 3. mobile phase system: A for CO₂; B for 0.05% DEA in ethanol; 4. gradient: B for 40% 5. flow rate: 3 mL/min. Compound **193,** isomer 1 (retention time: 3.038 min) and compound **193,** isomer 2 (retention time: 3.051min). Chiral preparative HPLC separation conditions: 1. instrument: Waters 150 Prep-SFC; 2. chromatographic column: Chiral AD column; 3. mobile phase: A for CO₂; B for 0.1% NH3•H2O in ethanol and acetonitrile; 4. gradient: B for 55%; 5. flow rate: 100 mL/min.

Compound **193,** isomer 1: LC-MS (ESI): m/z = 361.2[M+H]⁺.

¹H NMR (400 MHz, CD₃OD) δ 8.96-8.90 (m, 1H), 8.65-8.57 (m, 2H), 8.05-7.71 (m, 1H), 6.36-6.32 (m, 1H), 4.87-4.83 (m, 2H), 4.77-4.55 (m, 2H), 2.78-2.67 (m, 1H), 2.58-2.48 (m , 1H), 2.48 (s , 3H), 1.91-1.83 (m,1H), 1.60-1.49 (m,1H), 1.24- 1.06 (m, 2H).

Compound **193, isomer 2:** LC-MS (ESI): m/z = 361.2[M+H]⁺.

¹H NMR (400 MHz, CD₃OD) δ 8.96-8.90 (m, 1H), 8.65-8.57 (m, 2H), 8.05-7.71 (m, 1H), 6.36-6.32 (m, 1H), 4.87-4.83 (m, 2H), 4.77-4.55 (m, 2H), 2.78-2.67 (m, 1H), 2.58-2.48 (m, 1H), 2.48 (s , 3H), 1.91-1.83 (m,1H), 1.60-1.49 (m,1H),1.24-1.06 (m, 2H).

### Example 194:

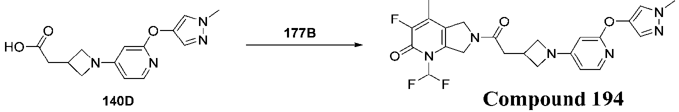

With reference to the synthesis method of step 9 in Example 99, **compound 194** (25 mg, 26.9%) was obtained using **140D** (55 mg, 0.19 mmol) and **177B** (50 mg, 0.19 mmol) as starting materials.

LC-MS (ESI): m/z =489.1 [M+H]⁺.

¹H NMR (400 MHz, DMSO-*d*₆) δ 8.24-7.83 (m, 1H), 7.79-7.64 (m, 2H), 7.32 (s, 1H), 6.21-6.05 (m, 1H), 5.92-5.78 (m, 1H), 4.93 (s, 1H), 4.66 (s, 2H), 4.43 (s, 1H), 4.14 -3.95 (m, 2H), 3.79 (s, 3H), 3.69-3.57 (m, 2H), 3.14-3.01 (m, 1H), 2.89-2.73 (m, 2H), 2.20-2.08 (m, 3H).

### Example 195:

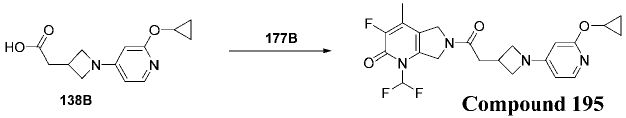

With reference to the synthesis method of step 9 in Example 99, **compound 195** (14 mg, 16.5%) was obtained using **138D** (47 mg, 0.19 mmol) and **177B** (50 mg, 0.19 mmol) as starting materials.

LC-MS (ESI): m/z =449.2 [M+H]⁺.

¹H NMR (400 MHz, DMSO-*d*₆) δ 8.19-7.85 (m, 1H), 7.78-7.72 (m, 1H), 6.10- 6.03 (m, 1H), 5.72-5.63 (m, 1H), 4.92 (s, 1H), 4.66 (s, 2H), 4.43 (s, 1H), 4.15-4.07 (m, 1H), 4.07-3.99 (m, 2H), 3.61-3.49 (m, 2H), 3.10-2.98 (m, 1H), 2.87-2.76 (m, 2H), 2.16 -2.09 (m, 3H), 0.73-0.65 (m, 2H), 0.62-0.54 (m, 2H).

### Example 196:

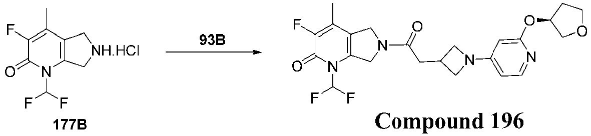

With reference to the operation process of step 3 in Example 177, compound **196** (0.035 g, 31%) was obtained using **177B** (0.06 g, 0.24 mmol) and **93B** (0.073 g, 0.26 mmol) as starting materials.

LC-MS (ESI): m/z = 479.2[M+H]⁺.

¹H NMR (400 MHz, CDCl₃) δ 8.00-7.66 (m, 2H), 6.00-5.95 (m, 1H), 5.66-5.63 (m, 1H), 5.59-5.53 (m, 1H), 4.90-4.85 (m, 2H), 4.59-4.53(m, 2H), 4.21-4.13 (m, 2H), 4.04-3.95 (m, 2H), 3.92-3.82 (m, 2H), 3.68-3.58 (m, 2H), 3.29-3.17 (m, 1H), 2.80-2.73 (m, 2H), 2.29-2.18 (m, 1H), 2.18-2.14 (m, 3H), 2.13-2.06(m, 1H).

### Example 197:

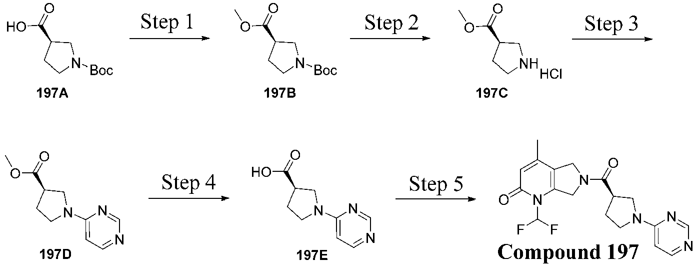

Step 1: Compound **197A** (3.0 g, 13.9 mmol) was dissolved in N,N-dimethylformamide (30 mL) at room temperature, potassium carbonate (5.75 g, 41.7 mmol) and iodomethane (2.96 g, 20.8 mmol) were successively added, and then the mixture was reacted at room temperature for 3 h. After the reaction was completed, water (30 mL) was added and the resulting mixture was extracted with ethyl acetate (30 mL×3). The combined organic phases were washed with saturated aqueous sodium chloride solution (20 mL), dried over anhydrous sodium sulphate and filtered. The filtrate was concentrated and the residue was separated and purified by silica gel column chromatography to obtain compound **197B** (3.1 g, 97%).

LC-MS (ESI): m/z = 174.1 [M+H-*^{t}*Bu]⁺.

Step 2: **197B** (3.1 g, 13.5 mmol) was dissolved in DCM (15 mL) at room temperature, and hydrogen chloride dioxane solution (15.5 mL, 62.1 mmol, 4 mol/L) was added dropwise. After the dropwise addition was completed, the reaction was continued for 3 h. After the reaction was completed, the reaction mixture was concentrated under reduced pressure to obtain crude hydrochloride of compound **197C** (1.7 g, 97%).

LC-MS (ESI): m/z = 130.1 [M+H]⁺.

Step 3: Hydrochloride of compound **197C** (500 mg, 3.87 mmol) was dissolved in N,N-dimethylacetamide (10 mL) at room temperature, and 4-chloropyrimidine (886 mg, 7.74 mmol) and N,N-diisopropylethylamine (2.50 g, 19.4 mmol) were added. Subsequently, the mixture was warmed to 95°C and reacted for 16 h. After the reaction was completed, the reaction solution was cooled to room temperature, water (15 mL) was added and the resulting mixture was extracted with ethyl acetate (15 mL×3). The combined organic phases were washed with saturated aqueous sodium chloride solution (15 mL), dried over anhydrous sodium sulphate and filtered. The filtrate was concentrated and the residue was separated and purified by silica gel column chromatography to obtain compound **197D** (400 mg, 50%).

LC-MS (ESI): m/z = 208.1 [M+H]⁺.

Step 4: With reference to the synthesis method of step 2 in Example 17, compound **197E** (350 mg, 94%) was obtained using **197D** (400 mg, 19.3 mmol) as a starting material.

LC-MS (ESI): m/z= 194.1 [M+H]⁺.

Step 5: With reference to the operation process of step 2 in Example 179, compound **197** (30 mg, 31%) was obtained using **197E** (50 mg, 0.26 mmol) and **179A** (61 mg,0.26 mmol) as starting materials.

LC-MS (ESI): m/z = 376.1 [M+H]⁺.

¹H NMR (400 MHz, CD₃OD) δ 8.43 (s, 1H), 8.10 (d, 1H), 8.06-7.75 (m, 1H), 6.57 -6.53 (m, 1H), 6.36 (s, 1H), 5.21-5.07 (m, 1H), 4.88-4.82 (m, 2H), 4.57-4.52 (m, 1H), 3.93-3.50 (m, 5H), 2.46-2.36 (m, 1H), 2.34-2.25 (m, 1H), 2.24-2.21 (m, 3H).

### Example 198:

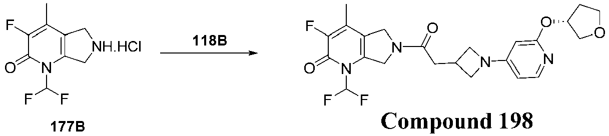

With reference to the operation process of step 3 in Example 177, compound **198** (0.04 g, 35%) was obtained using **177B** (0.06 g, 0.24 mmol) and **118B** (0.073 g, 0.26 mmol) as starting materials.

LC-MS (ESI): m/z = 479.2[M+H]⁺.

¹H NMR (400 MHz, CDCl₃) δ 8.01-7.65 (m, 2H), 5.99-5.95(m, 1H), 5.64 (d, 1H), 5.57-5.51 (m, 1H), 4.90-4.83 (m, 2H), 4.60-4.52 (m, 2H), 4.16 (t, 2H), 4.03-3.94 (m, 2H), 3.93-3.83 (m, 2H), 3.67-3.58 (m, 2H), 3.29-3.16 (m, 1H), 2.78-2.72 (m, 2H), 2.28-2.19 (m, 1H), 2.18-2.14 (m, 3H), 2.14-2.07 (m, 1H).

### Example 199:

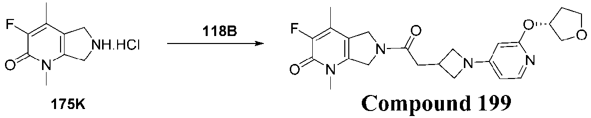

With reference to the operation process of step 3 in Example 177, compound **199** (0.045 g, 44.5%) was obtained using **175K** (0.05 g, 0.23 mmol) and **118B** (0.07 g, 0.25 mmol) as starting materials.

LC-MS (ESI): m/z = 443.2[M+H]⁺.

¹H NMR (400 MHz, CDCl₃) δ 7.81 (d, 1H), 6.00-5.96 (m, 1H), 5.64 (d, 1H), 5.59-5.53 (m, 1H), 4.76-4.68 (m, 2H), 4.65-4.57 (m, 2H), 4.17 (t, 2H), 4.03-3.95 (m, 2H), 3.93-3.84 (m, 2H), 3.67-3.59 (m, 2H), 3.50 (s, 3H), 3.30-3.18 (m, 1H), 2.80-2.73 (m, 2H), 2.31-2.18 (m, 1H), 2.16-2.06 (m, 4H).

### Example 200:

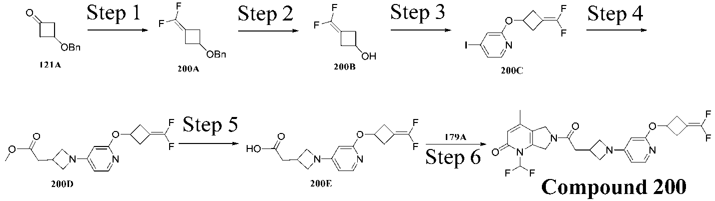

Step 1: 3-Benzyloxy-1-cyclobutanone (8.00 g, 45.45 mmol) and difluoromethyl(2-pyridyl)sulphone (7.89 g, 40.9 mmol) were dissolved in DMF (100 mL). Under nitrogen atmosphere, the mixture was cooled to -60°C, and a solution of potassium tert-butoxide (7.65 g, 68.18 mmol) in DMF (100 mL) was added dropwise. After the dropwise addition was completed, the reaction mixture was warmed to 0°C and stirred for 1 hour. Concentrated hydrochloric acid (20 mL) was added to quench the reaction. The mixture was stirred at room temperature for 1 hour. The reaction solution was diluted with water (50 mL) and extracted with ethyl acetate (200 mL×3). The combined organic phases were washed with saturated brine, dried over anhydrous sodium sulphate and filtered and the filtrate was concentrated under reduced pressure. The residue was purified by silica gel column chromatography to obtain compound **200B** (3.01 g, 32%).

LC-MS (ESI):m/z = 211.2 [M+H]⁺.

Step 2: Compound **200B** (3.01 g, 14.29 mmol) was dissolved in dichloromethane (30 mL), and the mixture was cooled to -70°C. Boron tribromide (5.36 g, 21.44 mmol) was added dropwise, and after the dropwise addition was completed, the mixture was stirred for another 0.5 hours. Saturated aqueous sodium bicarbonate solution was added dropwise to quench the reaction. The mixture was extracted with dichloromethane (20 mL×3). The combined organic phases were washed with saturated aqueous sodium chloride solution, dried over anhydrous sodium sulphate and filtered to obtain a solution of compound **200C** in dichloromethane, which was directly used in the next reaction.

LC-MS (ESI):m/z = 121.2 [M+H]⁺.

Step 3: With reference to the synthesis method of step 1 in Example 25, compound **200D** (0.519 g, 37.8%) was obtained using **200C** (0.509 g, 4.24 mmol) and 2-fluoro-4-iodopyridine (1.35 g, 5.15 mmol) as starting materials.

LC-MS (ESI):m/z = 324.2 [M+H]⁺.

Step 4: With reference to the synthesis method of step 2 in Example 59, compound **200E** (0.518 g, 99.6%) was obtained using **200D** (0.519 g,1.60 mmol) and ethyl 2-(azetidin-3-yl) acetate (0.822 g, 3.2 mmol) as starting materials.

LC-MS (ESI):m/z = 325.2 [M+H]⁺.

Step 5: With reference to the synthesis method of step 3 in Example 59, compound **200F** (0.495 g, 99.9%) was obtained using **200E** (0.518 g, 1.60 mmol) as a starting material.

LC-MS (ESI):m/z = 311.2 [M+H]⁺.

Step 6: With reference to the synthesis method of step 3 in Example 17, compound 200 (35 mg, 25.91%) was obtained using **200F** (0.080 g, 0.025 mmol) and **179A** (0.073 g, 0.039 mmol) as starting materials.

LC-MS (ESI):m/z = 493.2 [M+H]⁺.

¹H NMR (400 MHz, CDCl₃) δ 8.03-7.96 (m, 1H), 7.86-7.78 (m, 3H), 7.70-7.48 (m, 1H), 6.34-6.29 (m, 2H), 6.04-5.98 (m, 2H), 5.62-5.53 (m, 2H), 5.30-5.21(m, 2H), 4.91-4.83 (m, 2H), 4.53-4.38 (m, 2H),4.30-4.14(m, 1H) ,3.31-3.13 (m, 3H), 2.18-2.13 (m, 3H).

### Example 201:

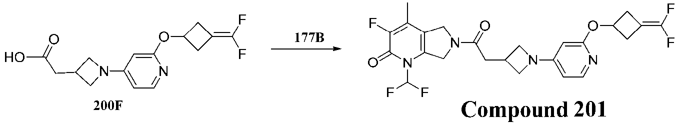

With reference to the synthesis method of step 3 in Example 17, compound **201** (31 mg, 30.48%) was obtained using **200F** (0.080 g, 0.025 mmol) and **177B** (0.063 g, 0.040 mmol) as starting materials.

LC-MS (ESI):m/z = 511.2 [M+H]⁺.

¹H NMR (400 MHz, CDCl₃) δ 8.05-7.93 (m, 1H), 7.86-7.78 (m, 1H), 7.70-7.48 (m, 1H), 7.46-6.96 (m, 1H),6.34-6.29 (m, 2H), 6.04-5.98 (m, 2H), 5.62-5.53 (m, 2H), 5.30-5.21 (m, 2H), 4.91-4.83 (m, 2H), 4.53-4.38 (m, 2H),4.30-4.14(m, 1H), 3.31-3.13 (m, 3H), 2.18-2.13 (m, 3H).

### Example 202:

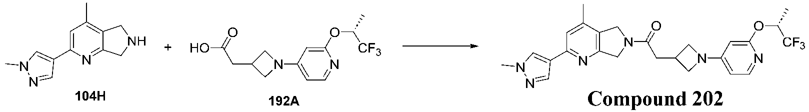

With reference to the procedures of step 7 in Example 4, **compound 202** (66 mg, 28%) was synthesized using **104H** (100 mg, 0.47 mmol) and **192A** (142 mg, 0.47 mmol) as starting materials.

LC-MS (ESI): m/z = 501.3 [M+H]⁺.

¹H NMR (400 MHz, CDCl₃) δ 7.97-7.88 (m, 2H), 7.89-7.77 (m, 1H), 7.21-7.19 (m, 1H), 6.04-5.97 (m, 1H), 5.80-5.69 (m, 2H), 4.84-4.72 (m, 4H), 4.23-4.16 (m, 2H), 3.70-3.64 (m, 2H), 3.32-3.21 (m, 1H), 2.83-2.77 (m, 2H), 2.32-2.30 (m, 3H), 1.48-1.44 (m, 3H).

### Example 203:

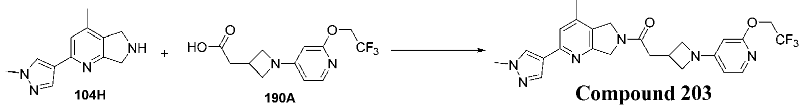

With reference to the procedures of step 7 in Example 4, **compound 203** (58 mg, 26%) was synthesized using **104H** (100 mg, 0.47 mmol) and **190A** (136 mg, 0.47 mmol) as starting materials.

LC-MS (ESI): m/z = 487.2 [M+H]⁺.

¹H NMR (400 MHz, CDCl₃) δ 7.99-7.90 (m, 2H), 7.80-7.78 (m, 1H), 7.24-7.19 (m, 1H), 6.05-6.03 (m, 1H), 5.75 (s, 1H), 4.90-4.69 (m, 6H), 4.25-4.17 (m, 2H), 3.97-3.94 (m, 3H), 3.71-3.66 (m, 2H), 3.31-3.23 (m, 1H), 2.83-2.77 (m, 2H), 2.36-2.30 (m, 3H).

### Example 204:

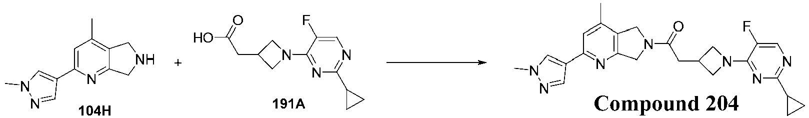

With reference to the procedures of step 7 in Example 4, **compound 204** (64 mg, 31%) was synthesized using **104H** (100 mg, 0.47 mmol) and **191A** (117 mg, 0.47 mmol) as starting materials.

LC-MS (ESI): m/z = 448.3 [M+H]⁺.

¹H NMR (400 MHz, CDCl₃) δ 7.95-7.88 (m, 2H), 7.86-7.83 (m, 1H), 7.21-7.18 (m, 1H), 4.83-4.72 (m, 4H), 4.51-4.45 (m, 2H), 4.00-3.93 (m, 5H), 3.33-3.22 (m, 1H), 2.83-2.77 (m, 2H), 2.31 (s, 3H), 2.07-1.98 (m, 1H), 1.01-0.96 (m, 2H), 0.93-0.86 (m, 2H).

### Example 205:

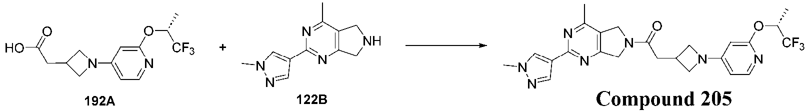

With reference to the synthesis method of step 3 in Example **122,** compound **205** (100 mg, 50%) was obtained using compound **192A** (122 mg, 0.40 mmol) and intermediate **122B** (86 mg, 0.40 mmol) as starting materials.

LC-MS (ESI): m/z = 502.3 [M+H]⁺.

¹H NMR (400 MHz, CDCl₃) δ 8.18-8.16 (m, 1H), 8.10-8.08 (m, 1H), 7.80-7.78 (m, 1H), 6.03-6.01 (m, 1H), 5.79-5.71 (m, 2H), 4.78-4.76 (m, 4H), 4.22-4.17 (m, 2H), 3.96 (s, 3H), 3.68-3.65 (m, 2H), 3.29-3.24 (m, 1H), 2.82-2.78 (m, 2H), 2.48-2.47 (m, 3H), 1.47-1.45 (m, 3H).

### Example 206:

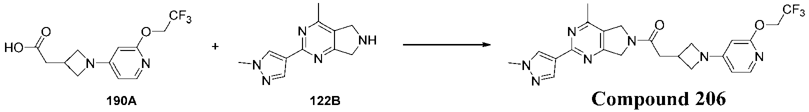

With reference to the synthesis method of step 3 in Example **122,** compound **206** (80 mg, 23.55%) was obtained using **190A** (0.20 g, 0.70 mmol) and intermediate **122B** (0.15 g, 0.70 mmol) as starting materials.

LC-MS (ESI): m/z= 488.2 [M+H]⁺.

¹H NMR (400 MHz, CDCl₃): δ 8.18-8.16 (m, 1H), 8.10-8.08 (m, 1H), 7.80-7.79 (m, 1H), 6.06-6.04 (m, 1H), 5.75 (s, 1H), 4.79-4.70 (m, 6H), 4.24-4.20 (m, 2H), 3.96 (s, 3H), 3.71-3.67(m, 2H), 3.29-3.26 (m, 1H), 2.83-2.78 (m, 2H), 2.48-2.47 (m, 3H).

### Example 207:

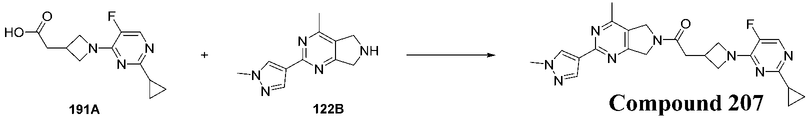

With reference to the synthesis method of step 3 in Example **122,** compound **207** (80 mg, 38.39%) was obtained using **191A** (0.12 g, 0.46 mmol) and intermediate **122B** (0.10 g, 0.46 mmol) as starting materials.

LC-MS (ESI): m/z= 449.2 [M+H]⁺.

¹H NMR (400 MHz, CDCl₃): δ 8.17-8.15 (m, 1H), 8.09-8.07 (m, 1H), 7.96-7.93 (m, 1H), 4.79-4.76 (m, 4H), 4.60-4.56 (m, 2H), 4.11-4.08 (m, 2H), 3.96 (s, 3H), 3.35-3.33 (m, 1H), 2.85-2.80 (m, 2H), 2.48-2.47 (m, 3H), 2.22-2.19 (m, 1H), 1.10-1.06 (m, 4H).

### Example 208:

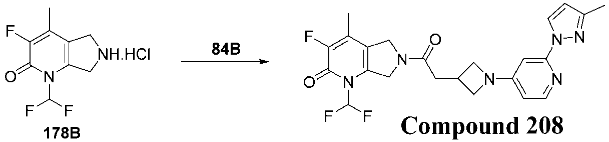

With reference to the operation process of step 3 in Example 177, compound **208** (0.04 g, 36%) was obtained using **177B** (0.06 g, 0.24 mmol) and **84B** (0.072 g, 0.26 mmol) as starting materials.

LC-MS (ESI): m/z = 473.2[M+H]⁺.

¹H NMR (400 MHz, CDCl₃)δ 8.43 (d, 1H), 8.05-7.63 (m, 2H), 6.85 (d, 1H), 6.21 (d, 1H), 6.12 (dd, 1H), 4.91-4.84 (m, 2H), 4.59-4.52 (m, 2H), 4.29 (t, 2H), 3.78-3.72 (m, 2H), 3.35-3.21 (m, 1H), 2.78-2.76 (m, 2H), 2.36 (s, 3H), 2.18-2.16 (m, 3H).

### Example 209:

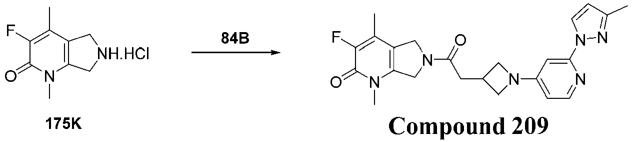

With reference to the operation process of step 3 in Example 177, compound **209** (0.03 g, 30%) was obtained using **175K** (0.05 g, 0.23 mmol) and **84B** (0.07 g, 0.25 mmol) as starting materials.

LC-MS (ESI): m/z = 437.2[M+H]⁺.

¹H NMR (400 MHz, CDCl₃) δ 8.41 (d, 1H), 8.01 (d, 1H), 6.85 (d, 1H), 6.21 (d, 1H), 6.13 (dd, 1H), 4.77-4.68 (m, 2H), 4.65-4.60 (m, 2H), 4.28 (t, 2H), 3.76-3.72 (m, 2H), 3.51 (s, 3H), 3.34-3.21 (m, 1H), 2.80-2.76 (m, 2H), 2.37 (s, 3H), 2.16-2.14(m, 3H).

### Example 210:

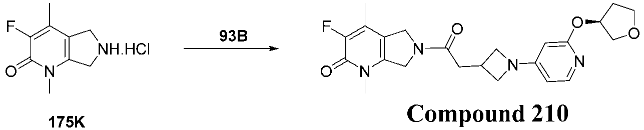

With reference to the operation process of step 3 in Example 177, compound **210** (0.04 g, 39.5%) was obtained using **175K** (0.05 g, 0.23 mmol) and **93B** (0.07 g, 0.25 mmol) as starting materials.

LC-MS (ESI): m/z = 443.2[M+H]⁺.

¹H NMR (400 MHz, CDCl₃) δ 7.80 (d, 1H), 5.97 (dd, 1H), 5.64 (d, 1H), 5.57-5.53 (m, 1H), 4.78-4.67 (m, 2H), 4.64-4.59 (m, 2H), 4.16 (t, 2H), 4.01-3.95 (m, 2H), 3.92-3.84 (m, 2H), 3.64-3.60 (m, 2H), 3.50 (s, 3H), 3.29-3.16 (m, 1H), 2.78-2.75(m, 2H), 2.27-2.18 (m, 1H), 2.16-2.06 (m, 4H).

### Example 211:

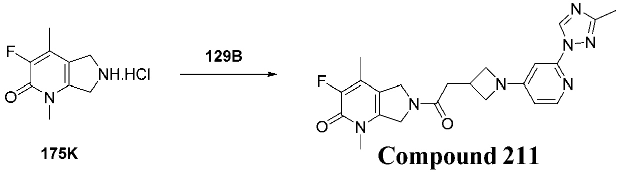

With reference to the operation process of step 3 in Example 177, compound **211** (55 mg, 47%) was obtained using compound **175K** (60 mg, 0.27 mmol) and compound **129B** (75 mg, 0.27 mmol) as starting materials.

LC-MS (ESI): m/z = 438.2[M+H]⁺.

1H NMR (400 MHz, CDCl3) δ = 8.99 (s, 1H), 8.02 (d, 1H), 6.74 (d, 1H), 6.20-6.18 (m, 1H), 4.74-4.70 (m, 2H), 4.67-4.57 (m, 2H), 4.29 (t, 2H), 3.77-3.73 (m, 2H), 3.50 (s, 3H), 3.35-3.23 (m, 1H), 2.81-2.78 (m, 2H), 2.48 (s, 3H), 2.15-2.13 (m, 3H).

### Example 212:

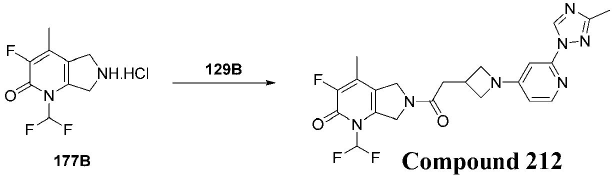

With reference to the operation process of step 3 in Example 177, compound **212** (52 mg, 46%) was obtained using compound **177B** (60 mg, 0.24 mmol) and compound **129B** (67 mg, 0.24 mmol) as starting materials.

LC-MS (ESI): m/z = 474.2[M+H]⁺.

¹H NMR (400 MHz, CDCl₃) δ = 9.01 (s, 1H), 8.02 (d, 1H), 7.84-7.68 (m, 1H), 6.74 (d, 1H), 6.20-6.18 (m, 1H), 4.88 (s, 2H), 4.58 (s, 1H), 4.55 (s, 1H), 4.29 (t, 2H), 3.78-3.73 (m, 2H), 3.32- 3.27 (m, 1H), 2.80-2.77 (m, 2H), 2.48 (s, 3H), 2.18-2.16 (m, 3H).

### Example 213:

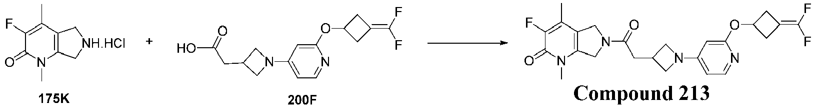

With reference to the synthesis method of step 3 in Example **17,** compound **213** (34 mg, 22.3%) was obtained using **200F** (0.1 g, 0.033 mmol) and **175K** (0.084 g, 0.039 mmol) as starting materials.

LC-MS (ESI):m/z = 475.2 [M+H]⁺.

¹H NMR (400 MHz, DMSO-d₆) δ 7.76-7.66 (m, 1H), 6.10-6.02 (m, 1H), 5.65-5.61 (m, 1H), 5.24-5.13 (m, 1H), 4.94-4.83 (m,1H), 4.73-4.60 (m, 2H), 4.49-4.42 (m, 1H), 4.09-3.97 (m, 2H), 3.60-3.52 (m, 2H), 3.41-3.39 (m, 2H), 3.39-3.37 (m, 1H), 3.28-3.26 (m, 3H), 3.15-3.07 (m, 2H), 2.75-2.65 (m,2H), 2.11-2.06 (m, 3H).

### Example 214:

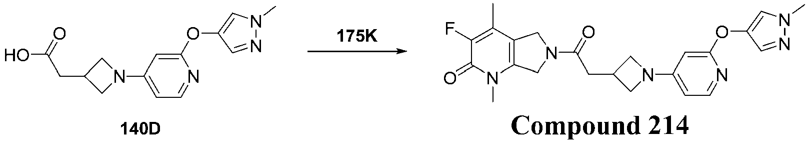

With reference to the synthesis method of step 9 in Example 99, **compound 214** (30 mg, 35.2%) was obtained using **140D** (55 mg, 0.19 mmol) and **175K** (41 mg, 0.19 mmol) as starting materials.

### Example 215:

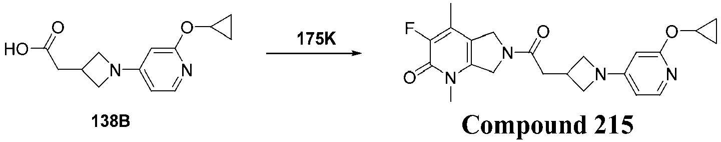

With reference to the synthesis method of step 9 in Example 99, **compound 215** (28 mg, 35.8%) was obtained using **138D** (47 mg, 0.19 mmol) and **175K** (41 mg, 0.19 mmol) as starting materials.

LC-MS (ESI): m/z =413.1 [M+H]⁺.

¹H NMR (400 MHz, DMSO-d₆) δ 7.87-7.58 (m, 1H), 6.18-5.96 (m, 1H), 5.68 (s, 1H), 4.88 (s, 1H), 4.70-4.59 (m, 2H), 4.46 (s, 1H), 4.15-4.08 (m, 1H), 4.07-4.00 (m, 2H), 3.60-3.50 (m, 2H), 3.45 -3.34 (m, 3H), 3.14-2.93 (m, 1H), 2.91-2.71 (m, 2H), 2.19-1.98 (m, 3H), 0.72-0.64 (m, 2H), 0.63- 0.52 (m, 2H).

LC-MS (ESI): m/z =453.3 [M+H]⁺.

¹H NMR (400 MHz, DMSO-*d*₆) δ 7.80-7.70 (m, 2H), 7.33 (s, 1H), 6.23-6.07 (m, 1H), 6.01-5.69 (m, 1H), 4.88 (s, 1H), 4.70-4.64 (m, 2H), 4.47 (s, 1H), 4.12-4.03 (m, 2H), 3.79 (s, 3H), 3.64-3.56 (m, 2H), 3.46- 3.38 (m, 3H), 3.14-3.01 (m, 1H), 2.85-2.78 (m, 2H), 2.13-2.06 (m, 3H).

### Example 216:

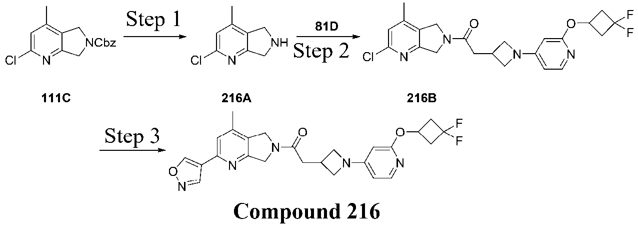

Step 1: Compound **111C** (90 mg, 0.30 mmol) was dissolved in hydrogen bromide acetic acid solution (10 mL), and the mixture was stirred at room temperature for 2 hours. After the reaction was completed, the solvent was pumped off to obtain crude product **216A** (100 mg).

LC-MS (ESI): m/z = 169.1 [M+H]⁺.

Step 2: With reference to the synthesis method of step 3 in Example **144,** compound **216B** (100 mg, 74%) was obtained using **216A** (100 mg) and compound **81D** (89.5 mg, 0.30 mmol) as starting materials.

LC-MS (ESI): m/z = 449.2 [M+H]⁺.

Step 3: Compound **216B** (100 mg, 0.22 mmol), 4-isoxazole boronic acid pinacol ester (85.8 mg, 0.44 mmol), potassium fluoride (38.3 mg, 0.66 mmol) and chloro(2-dicyclohexylphosphino-2',4',6'-triisopropyl-1,1'-biphenyl)[2-(2'-amino-1,1'-biphenyl)]palladium (17.3 mg, 0.022 mmol) were dissolved in dimethyl sulphoxide (10 mL) and water (2 mL), and the mixture was warmed to 100°C and stirred for 3 hours. After the reaction was completed, the mixture was cooled to room temperature, diluted with water (10 mL), and extracted with ethyl acetate (25 mL×3). The organic layers were combined, washed with saturated aqueous sodium chloride solution (30 mL), dried over anhydrous sodium sulphate and filtered. Then the filtrate was concentrated and the residue was separated and purified by silica gel column chromatography to obtain compound **216** (41 mg, 39%).

LC-MS (ESI): m/z = 482.1 [M+H]⁺.

¹H NMR (400 MHz, CDCl₃) δ 8.30 (s, 1H), 8.14 (s, 1H), 7.81-7.80 (m, 1H), 7.22-7.19 (m, 1H), 6.02-6.00 (m, 1H), 5.62 (s, 1H), 5.17-5.10 (m, 1H), 4.86-4.77 (m, 4H), 4.24-4.20 (m, 2H), 3.70-3.67 (m, 2H), 3.30-3.24 (m, 1H), 3.15-3.05 (m, 2H), 2.83-2.64 (m, 4H), 2.40-2.39 (m, 3H).

### Example 217:

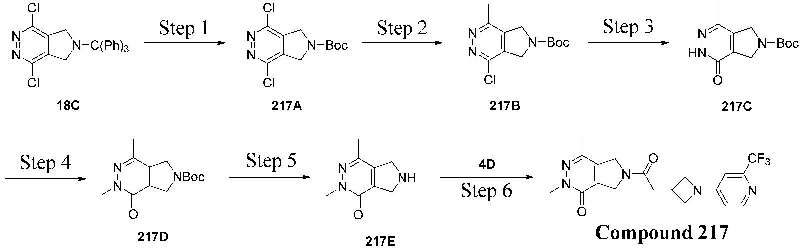

Step 1: Compound **18C** (7.80 g, 18.04 mmol) was dissolved in 4 mol/L hydrogen chloride methanol solution (100 mL), and the mixture was reacted at room temperature for 2 hours. After the reaction was completed, the mixture was concentrated and to the residue was added methanol (100 mL). The resulting mixture was stirred for 5 minutes and then concentrated. To the residue was added methanol (100 mL). Triethylamine was added dropwise to adjust the mixture to pH > 7. Then di-tert-butyl dicarbonate (5.91 g, 27.06 mmol) was added in portions, and the mixture was reacted at room temperature overnight. After the reaction was completed, the mixture was concentrated and to the residue were added dichloromethane (200 mL) and water (200 mL). The resulting mixture was extracted and subjected to phase separation. The organic phase was dried over anhydrous sodium sulphate and concentrated under reduced pressure and the residue was separated and purified by silica gel column chromatography to obtain compound **217A** (4.40 g, 84.05%).

LC-MS(ESI):m/z = 291.1 [M+H]⁺.

Step 2: Compound **217A** (4.40 g, 15.16 mmol) was dissolved in tetrahydrofuran (100 mL), bis(triphenylphosphine)palladium dichloride (1.06 g, 1.52 mmol) was added, and methylzinc chloride tetrahydrofuran solution (11.2 mL, 1.5 mol/L) was added dropwise under nitrogen atmosphere. After the dropwise addition was completed, the mixture was warmed to 80°C and reacted for 4 hours. After the reaction was completed, the mixture was cooled to room temperature, and saturated aqueous ammonium chloride solution (5 mL) was added to quench the reaction. The reaction solution was filtered through diatomaceous earth, and the filter cake was washed with tetrahydrofuran. The filtrate was concentrated under reduced pressure, and to the residue were added dichloromethane and saturated aqueous sodium bicarbonate solution. The mixture was extracted and subjected to phase separation and the organic phase was dried over anhydrous sodium sulphate and concentrated under reduced pressure. The residue was separated and purified using a reverse-phase silica gel column to obtain compound **217B** (1.05 g, 25.67%).

LC-MS(ESI):m/z = 270.3[M+H]⁺.

Step 3: Compound **217B** (0.50 g, 1.85 mmol), sodium hydroxide (0.22 g, 5.55 mmol), and sodium acetate (0.46 g, 5.55 mmol) were successively dissolved in acetic acid (20 mL), and then the mixture was warmed to 110°C and reacted for 8 hours. After the reaction was completed, the mixture was concentrated and the residue was separated and purified using a silica gel column to obtain compound **217C** (139 mg, 29.84%).

LC-MS(ESI):m/z = 252.3[M+H]⁺.

Step 4: Compound **217C** (136 mg, 0.54 mmol), iodomethane (0.15 g, 1.08 mmol), and potassium carbonate (0.15 g, 1.08 mmol) were successively dissolved in N,N-dimethylformamide (20 mL), and then the mixture was reacted at room temperature overnight. After the reaction was completed, ethyl acetate and saturated aqueous sodium chloride solution were added, and the mixture was extracted and subjected to phase separation. The organic phase was dried over anhydrous sodium sulphate and concentrated to obtain compound **217D** (130 mg, 90.53%).

LC-MS(ESI):m/z = 266.2[M+H]⁺.

Step 5: Compound **217D** (130 mg, 0.49 mmol) was dissolved in 4.0 mol/L hydrogen chloride 1,4-dioxane solution, and the mixture was stirred at room temperature for 2 hours. After the reaction was completed, the mixture was concentrated under reduced pressure to obtain compound **217E** (80.94 mg, crude).

LC-MS(ESI):m/z = 166.2[M+H]⁺.

Step 6: Compound **217E** (67 mg, 0.41 mmol), compound **4D** (110 mg, 0.41 mmol), and N-methylimidazole (50 mg, 61 mmol) were successively dissolved in N,N-dimethylformamide (3 mL). After the mixture was stirred evenly, chloro-N,N,N',N'-tetramethylformamidinium hexafluorophosphate (0.14 g, 0.49 mmol) was added and the reaction was continued at room temperature for 2 h. After the reaction was completed, the mixture was filtered and the filtrate was separated and purified using a reverse-phase preparative column (C18 spherical 20-35 nm 100A 80 g) (acetonitrile : water (0.1% ammonium acetate) = 5%-80%) to obtain **compound 217** (70 mg, 42.36%).

LC-MS (ESI): m/z = 408.0 [M+H]⁺.

¹H NMR (400 MHz, DMSO-*d*₆) δ 8.23-8.17 (m, 1H), 6.72-6.68 (m, 1H), 6.55-6.50 (m, 1H), 4.86-4.80 (m, 1H), 4.75-4.69 (m, 1H), 4.63-4.57 (m, 1H), 4.49-4.43 (m, 1H), 4.21-4.10 (m, 2H), 3.73-3.66 (m, 2H), 3.64 (s, 3H), 3.17-3.02 (m, 1H), 2.90 -2.76 (m, 2H), 2.27-2.20 (m, 3H).

### Example 218:

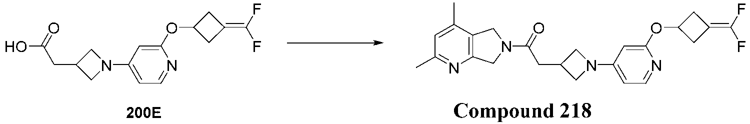

Compound **200E** (100 mg, 0.32 mmol), compound 2,4-dimethyl-6,7-dihydro-5H-pyrrolo[3,4-B]pyridine dihydrochloride (60 mg, 0.32 mmol) and N-methylimidazole (40 mg, 0.48 mmol) were successively dissolved in N,N-dimethylformamide (3 mL). After the mixture was stirred evenly, chloro-N,N,N',N'-tetramethylformamidinium hexafluorophosphate (110 mg, 0.38 mmol) was added and the reaction was continued at room temperature for 2 h. After the reaction was completed, the mixture was filtered and the filtrate was separated and purified using a reverse-phase preparative column (C18 spherical 20-35 nm 100A 80 g) (acetonitrile: water (0.1% ammonium acetate) = 5%-80%) to obtain **compound 218** (8 mg, 5.59%).

LC-MS (ESI): m/z = 441.3 [M+H]⁺.

¹H NMR (400 MHz, DMSO-d6) δ 7.59-7.51 (m, 1H), 7.03-6.97 (m, 1H), 5.70-5.61 (m, 1H), 5.20-5.06 (m, 1H), 4.96-4.91 (m, 1H), 4.84-4.73 (m, 2H), 4.61-4.48 (m, 2H), 4.10-4.00 (m, 2H), 3.66-3.54 (m, 2H), 3.11-2.96 (m, 5H), 2.87-2.75 (m, 2H), 2.45-2.39 (m, 3H), 2.26-2.19 (m, 3H).

### Example 219:

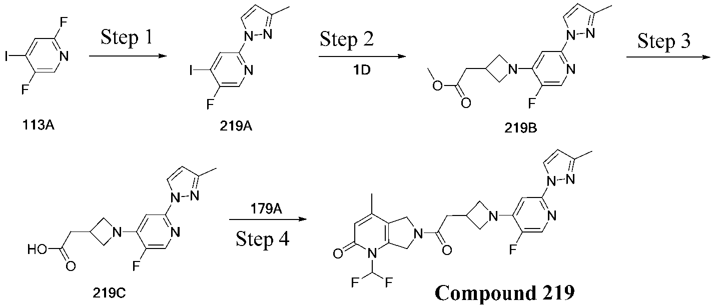

Compound **113A** (5.00 g, 20.75 mmol) was dissolved in dimethyl sulphoxide (30 mL) at room temperature, potassium carbonate (8.60 g, 41.7 mmol) and 3-methylpyrazole (1.87 g, 22.83 mmol) were successively added, and then the mixture was reacted at 70°C for 4 h. After the reaction was completed, water (150 mL) was added and the resulting mixture was extracted with ethyl acetate (150 mL×3). The combined organic phases were washed with saturated aqueous sodium chloride solution (50 mL), dried over anhydrous sodium sulphate and filtered. The filtrate was concentrated and the residue was separated and purified by silica gel column chromatography to obtain compound **219A** (2.2 g, 35%).

LC-MS (ESI): m/z = 340.2 [M+H]⁺.

Step 2: **219A** (400.00 mg, 1.32 mmol), methyl 2-(azetidin-3-yl)acetate trifluoroacetate (353.10 mg, 1.45 mmol), and N,N-diisopropylethylamine (511.79 mg, 3.96 mmol) were successively dissolved in dimethyl sulphoxide (5 mL), and then the mixture was warmed to 130°C and reacted for 16 hours. After the reaction was completed, the mixture was cooled to room temperature, diluted with water (40 mL) and extracted with ethyl acetate (40 mL×3). The combined organic phases were dried over anhydrous sodium sulphate and filtered and the filtrate was concentrated under reduced pressure. The residue was purified by silica gel column chromatography to obtain compound **219B** (187.0 mg, 47%)

LC-MS (ESI): m/z = 305.1 [M+H]⁺.

Step 3: **219B** (89.00 mg, 0.29 mmol) was dissolved in tetrahydrofuran (2 mL), methanol (2 mL) and water (2 mL), and then lithium hydroxide monohydrate (27.78 mg, 1.16 mmol) was added. The mixture was reacted at room temperature for 2 hours. After the reaction was completed, the mixture was adjusted to about pH = 6 with 1 N HCl, and extracted with ethyl acetate (50 mL×3). The combined organic phases were washed with water and concentrated under reduced pressure to obtain crude compound **219C** (67.0 mg, 85%).

LC-MS (ESI): m/z = 391.0 [M+H]⁺.

Step 4: With reference to the operation process of step 2 in Example 179, compound **219** (14.0 mg, 13%) was obtained using **219C** (67.00 mg, 0.23 mmol) and **179A** (54.43 mg,0.23 mmol) as starting materials.

LC-MS (ESI): m/z = 473.3 [M+H]⁺.

¹H NMR (400 MHz, CDCl₃) δ 8.31 (d, 1H), 7.86 (d, 1H), 7.99-7.64 (m, 1H), 6.88 (d, 1H), 6.32 (d, 1H), 6.20 (d, 1H), 4.88 (s, 2H), 4.54 (dd, 2H), 4.44 (t, 2H), 3.91 (dd, 2H), 3.33-3.18 (m, 1H), 2.78 (d, 2H), 2.35 (s, 3H), 2.16 (s, 3H).

### Example 220:

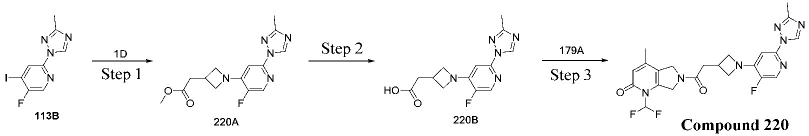

Step 1: **113B** (400.00 mg, 1.32 mmol), methyl 2-(azetidin-3-yl)acetate trifluoroacetate (321.00 mg, 1.32 mmol), and N,N-diisopropylethylamine (511.79 mg, 3.96 mmol) were successively dissolved in dimethyl sulphoxide (6 mL), and then the mixture was warmed to 130°C and reacted for 16 hours. After the reaction was completed, the mixture was cooled to room temperature, diluted with water (40 mL) and extracted with ethyl acetate (40 mL×3). The combined organic phases were dried over anhydrous sodium sulphate, filtered and concentrated under reduced pressure and the residue was purified by silica gel column chromatography to obtain compound **220A** (280.0 mg, 70%).

LC-MS (ESI): m/z = 306.4 [M+H]⁺.

Step 2: **220A** (280.00 mg, 0.92 mmol) was dissolved in tetrahydrofuran (2 mL), methanol (2 mL) and water (2 mL), and then lithium hydroxide monohydrate (88.14 mg, 3.68 mmol) was added. The mixture was reacted at room temperature for 2 hours. After the reaction was completed, the mixture was adjusted to about pH = 6 with 1 N HCl, and extracted with ethyl acetate (50 mL×3). The combined organic phases were washed with water and concentrated under reduced pressure to obtain crude compound **220B** (170.0 mg, 64%).

LC-MS (ESI): m/z = 392.1 [M+H]⁺.

Step 3: With reference to the operation process of step 2 in Example 179, compound **220** (9.0 mg, 11%) was obtained using **220B** (50.00 mg, 0.17 mmol) and **179A** (54.42 mg,0.17 mmol) as starting materials.

LC-MS (ESI): m/z = 474.2 [M+H]⁺.

¹H NMR (400 MHz, CDCl₃) δ 8.90 (s, 1H), 7.88 (d, 1H), 8.00-7.63 (m, 1H), 6.78 (d, 1H), 6.33 (d, 1H), 4.89 (s, 2H), 4.54 (d, 2H), 4.46 (t, 2H), 3.93 (dd, 2H), 3.29 (dt, 1H), 2.79 (d, 2H), 2.47 (s, 3H), 2.17 (d, 3H).

### Example 221:

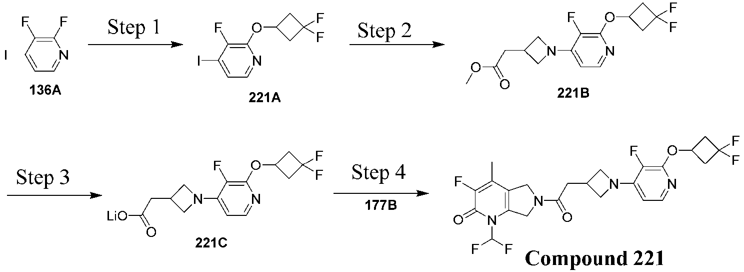

Steps 1-3: With reference to the operation processes of steps 1-3 of Example 81, compound **221C** was obtained using compound **136A** (1 g, 4.15 mmol) as a starting material.

Step 2: With reference to the operation process of step 3 in Example 177, compound **221** (45 mg, 44%) was obtained using compound **177B** (50 mg, 0.2 mmol) and compound **221C** (65 mg, 0.2 mmol) as starting materials.

LC-MS (ESI): m/z = 517.2[M+H]⁺.

¹H NMR (400 MHz, CDCl₃) δ = 7.99-7.67 (m, 1H), 7.55 (d, 1H), 6.01 (t, 1H), 5.20-5.08 (m, 1H), 4.88 (s, 2H), 4.57 (d, 2H), 4.37-4.32 (m, 2H), 3.84-3.80 (m, 2H), 3.25-2.18 (m, 1H), 3.15-3.05 (m, 2H), 2.82-2.67 (m, 4H), 2.17 (s, 3H).

### Example 222:

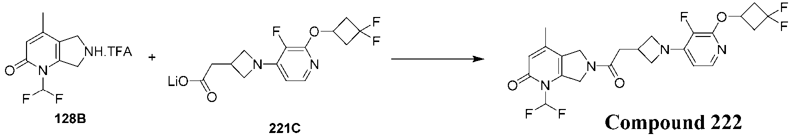

With reference to the operation process of step 3 in Example 177, compound **222** (40 mg, 46%) was obtained using compound **128B** (50 mg, 0.17 mmol) and compound **221C** (56 mg, 0.17 mmol) as starting materials.

LC-MS (ESI): m/z = 499.2[M+H]⁺.

¹H NMR (400 MHz, CDCl₃) δ = 7.97-7.65 (m, 1H), 7.55 (d, 1H), 6.32 (d, 1H), 6.01 (t, 1H), 5.20-5.08 (m, 1H), 4.88 (s, 2H), 4.53 (d, 2H), 4.37-4.32 (m, 2H), 3.87-3.77 (m, 2H), 3.28-3.16 (m, 1H), 3.15-3.05 (m, 2H), 2.84-2.68 (m, 4H), 2.16 (s, 3H).

### Example 223:

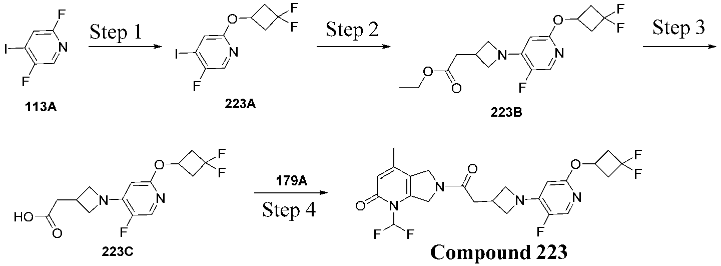

Step 1: Compound 3,3-difluorocyclobutanol (2.69 g, 24.9 mmol) was dissolved in tetrahydrofuran (30 mL), and NaH (1.24 g, 31.13 mmol, 60%) was added at 0°C. After the mixture was stirred for 30 min, compound **113A** (3 g, 12.45 mmol) was added, and the mixture was reacted at 0°C for 2 hours. After the reaction was completed as monitored by TLC, saturated aqueous ammonium chloride solution was added to quench the reaction. The mixture was extracted with ethyl acetate. The combined organic phases were dried over anhydrous sodium sulphate, and concentrated under reduced pressure. The residue was purified by silica gel column chromatography to obtain compound **223A** (2 g, 48.82%).

LC-MS (ESI): m/z = 330.0[M+H]⁺.

Step 2: **223A** (2 g, 6.08 mmol), **1D** (2.35 g, 9.12 mmol), caesium carbonate (6.93 g, 21.28 mmol) and Ruphos Pd G3 (0.51 g, 0.61 mmol) were successively dissolved in 1,4-dioxane (60 mL), and under nitrogen atmosphere, the mixture was warmed to 100°C and reacted for 16 hours. After the reaction was completed as monitored by TLC, the mixture was cooled to room temperature. Saturated aqueous ammonium chloride solution was added, and the mixture was extracted with ethyl acetate. The combined organic phases were dried over anhydrous sodium sulphate and concentrated under reduced pressure. The residue was purified by silica gel column chromatography to obtain compound **223B** (1.5 g, 71.67%).

LC-MS (ESI): m/z = 345.1[M+H]⁺.

Step 3: **223B** (1.5 g, 4.36 mmol) was dissolved in mixed solvents (methanol : water = 1 : 1 (20 mL)), lithium hydroxide (0.52 g, 21.8 mmol) was added, and the mixture was reacted at room temperature for 4 hours. After the reaction was completed as monitored by TLC, the reaction mixture was concentrated under reduced pressure to remove most of methanol and the residue was adjusted to pH = 3-4 with dilute hydrochloric acid. A solid was precipitated and the resulting mixture was filtered. The filter cake was washed with water and dried to obtain **223C** (1 g, 72.58%).

LC-MS (ESI): m/z = 317.1[M+H]⁺.

Step 4: With reference to the operation process of step 3 in Example 177, compound **223** (0.06 g, 57%) was obtained using **179A** (0.05 g, 0.21mmol) and **223C** (0.073g, 0.23mmol) as starting materials.

LC-MS (ESI): m/z = 499.2[M+H]⁺.

¹H NMR (400 MHz, CDCl₃) δ 7.99-7.62 (m, 2H), 6.33-6.32(m, 1H), 5.63 (d, 1H), 5.12-5.04(m, 1H), 4.88 (s, 2H), 4.55-4.52(m, 2H), 4.36 (t, 2H), 3.88-3.81 (m, 2H), 3.29-3.21 (m, 1H), 3.14-3.04 (m, 2H), 2.79-2.77 (m, 2H), 2.73 - 2.58 (m, 2H), 2.17-2.15 (m, 3H).

### Example 224:

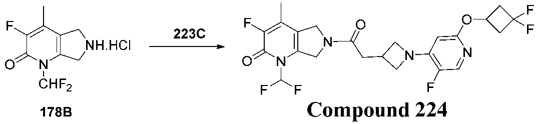

With reference to the operation process of step 3 in Example 177, compound **224** (0.05 g, 49.3%) was obtained using **177B** (0.05 g, 0.20 mmol) and **223C** (0.07 g, 0.22 mmol) as starting materials.

LC-MS (ESI): m/z = 517.2[M+H]⁺.

¹H NMR (400 MHz, CDCl₃) δ 7.99-7.67 (m, 1H), 7.64 (d, 1H), 5.64 (d, 1H), 5.10-5.03 (m, 1H), 4.89-4.86 (m, 2H), 4.58-4.55 (m, 2H), 4.34 (t, 2H), 3.84-3.79 (m, 2H), 3.30 - 3.17 (m, 1H), 3.13-3.03 (m, 2H), 2.79-2.76 (m, 2H), 2.72-2.60 (m, 2H), 2.17-2.16 (m, 3H).

### Bioassay methods:

DZ-01 of the present invention described below was prepared with reference to the method described in Example 11 of the patent WO 2018002760, and has the following structure:

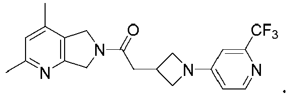

### 1. Determination of the PAM agonistic activity of compound on M4 receptor

1). Cell plating: M4-CHO cells were digested, collected, and resuspended. After cell counting, the cell suspension was seeded into a 384-well cell plate at a density of: 1.2 × 10⁴ cells/25 µL/well. The cell plate was then cultured in a 37°C, 5% CO₂ incubator for about 16-20 h.
2). Day 2: Assay Buffer was prepared according to the instructions of the FLIPR Calcium 6 Assay Kit. Component A (20×) was thawed to room temperature and diluted with the Assay Buffer to prepare a loading buffer (1×), which was stored at room temperature until use.
3). The culture medium was removed from the cell plate. 40 µL of the loading buffer (1×) was quickly added to each well. The cell plate was then centrifuged and incubated at 37°C in the dark for 120 min.
4). Working solutions of the positive control compound and the test compounds were prepared. An acetylcholine (Ach) agonist compound solution was prepared (final concentration: 3 nM). The PAM compound and the Ach agonist solution were mixed at a 1 : 1 ratio, and the mixture was transferred into a 384-well compound source plate at 20 µL per well.
5). The cell plate, the compound source plate, and the pipette tips were placed into the corresponding positions within the FLIPR instrument. 10 µL of the diluted compound from step 4 was added into each experimental well using the FLIPR Tetra. Data was collected at the wavelength of 515 nm-575 nm.
6). The signal values were plotted against the compound concentrations. Nonlinear regression analysis was performed using GraphPad Prism software for curve fitting and EC₅₀ calculation.

The experimental results are shown in Table 1, where A represents EC₅₀ < 100 nm; B represents 100 nM ≤ EC₅₀ < 500 nM, and C represents 500 nM ≤ EC₅₀.

**Table 1. Agonistic activity of compounds on M4 receptor**

| Compound | EC₅₀ | Compound | EC₅₀ |
|---|---|---|---|
| **Compound 1** | A | **compound 5** | A |
| **Compound 6** | A | **Compound 7** | A |
| **Compound 12,** isomer 1 | A | **Compound 12,** isomer 2 | A |
| **Compound 14** | A | **Compound 16** | A |
| **Compound 21** | A | **Compound 22** | A |
| **Compound 23** | A | **Compound 24** | A |
| **Compound 25** | A | **Compound 26** | A |
| **Compound 27** | A | **Compound 29** | A |
| **Compound 30** | A | **Compound 36** | A |
| **Compound 37** | A | **Compound 38** | A |
| **Compound 39** | A | **Compound 40** | A |
| **Compound 43** | A | **Compound 44** | A |
| **Compound 46** | A | **Compound 47** | A |
| **Compound 48** | A | **Compound 49** | A |
| **Compound 50** | A | **Compound 51** | A |
| **Compound 52** | A | **Compound 53** | A |
| **Compound 54** | A | **Compound 55** | A |
| **Compound 56** | A | **Compound 58** | A |
| **Compound 62** | A | **Compound 63** | A |
| **Compound 65** | A | **Compound 67** | A |
| **Compound 75** | A | **Compound 76** | A |
| **Compound 77,** isomer 2 | A | **Compound 78** | A |
| **Compound 81** | A | **Compound 83** | A |
| **Compound 84** | A | **Compound 85** | A |
| **Compound 87** | A | **Compound 90** | A |
| **Compound 91** | A | **Compound 92** | A |
| **Compound 93** | A | **Compound 94** | A |
| **Compound 95** | A | **Compound 96** | A |
| **Compound 97** | A | **Compound 98** | A |
| **Compound 99** | A | **Compound 100** | A |
| **Compound 101** | A | **Compound 102** | A |
| **Compound 103** | A | **Compound 104** | A |
| **Compound 107** | A | **Compound 108** | A |
| **Compound 109** | A | **Compound 111** | A |
| **Compound 112** | A | **Compound 115** | A |
| **Compound 116** | A | **Compound 117** | A |
| **Compound 118** | A | **Compound 119** | A |
| **Compound 120** | A | **Compound 121** | A |
| **Compound 122** | A | **Compound 123** | A |
| **Compound 125** | A | **Compound 126** | A |
| **Compound 127** | A | **Compound 128** | A |
| **Compound 130** | A | **Compound 131** | A |
| **Compound 133** | A | **Compound 134** | A |
| **Compound 137** | A | **Compound 138** | A |
| **Compound 139** | A | **Compound 141** | A |
| **Compound 143** | A | **Compound 144** | A |
| **Compound 146** | A | **Compound 148** | A |
| **Compound 149** | A | **Compound 150** | A |
| **Compound 152** | A | **Compound 153** | A |
| **Compound 154** | A | **Compound 155** | A |
| **Compound 156** | A | **Compound 157** | A |
| **Compound 158** | A | **Compound 158** | A |
| **Compound 160** | A | **Compound 162** | A |
| **Compound 163** | A | **Compound 164** | A |
| **Compound 165** | A | **Compound 166** | A |
| **Compound 168** | A | **Compound 169** | A |
| **Compound 170** | A | **Compound 171** | A |
| **Compound 172** | A | **Compound 173** | A |
| **Compound 174** | A | **Compound 175** | A |
| **Compound 176** | A | **Compound 177** | A |
| **Compound 178** | A | **Compound 179** | A |
| **Compound 180** | A | **Compound 182** | A |
| **Compound 184** | A | **Compound 185** | A |
| **Compound 186** | A | **Compound 187** | A |
| **Compound 188** | A | **Compound 189** | A |
| **Compound 195** | A | **Compound 198** | A |
| **Compound 199** | A | **Compound 200** | A |
| **Compound 201** | A | **Compound 204** | A |
| **Compound 207** | A | **Compound 208** | A |
| **Compound 209** | A | **Compound 210** | A |
| **Compound 211** | A | **Compound 213** | A |
| **Compound 214** | A | **Compound 215** | A |
| **Compound 216** | A | | |

| | | | |
|---|---|---|---|
| Conclusion: the compounds of the present invention, such as the Example compounds, show high agonist activities on the M4 receptor. | | | |

### 2. Pharmacokinetic test in mice

**2.1 Experimental animals:** male C57 mice, 20-25 g, divided into an intravenous group and an intragastric group.

**2.2 Experimental design:** on the day of the experiment, the C57 mice were randomly grouped according to their body weights. The animals were fasted with water available for 12-14 h one day before the administration, and were fed 4 h after the administration.

**Table 2. Administration information**

| Group | Number | Administration information | | | | | |
|---|---|---|---|---|---|---|---|
| | Male | Test compound | Administr ation dosage (mg/kg) | Administration concentration (mg/mL) | Administration volume (mL/kg) | Collected sample | Mode of administration |
| G1 | 3 | Compound 104 | 2.5 | 0.5 | 5 | Plasma | Intravenous administration |
| G2 | 12 | | 10 | 1 | 10 | Plasma | Intragastric administration |
| G3 | 3 | Compound 122 | 2.5 | 0.5 | 5 | Plasma | Intravenous administration |
| G4 | 12 | | 10 | 1 | 10 | Plasma | Intragastric administration |
| G5 | 3 | Compound 128 | 2.5 | 0.5 | 5 | Plasma | Intravenous administration |
| G6 | 12 | | 10 | 1 | 10 | Plasma | Intragastric administration |
| G7 | 3 | DZ-01 | 2.5 | 0.5 | 5 | Plasma | Intravenous administration |
| G8 | 12 | | 10 | 1 | 10 | Plasma | Intragastric administration |

| | | | | | | | |
|---|---|---|---|---|---|---|---|
| Note: Vehicle for intravenous administration: 5% DMA + 5% Solutol + 90% Saline; vehicle for intragastric administration: 5% DMSO + 95% (0.5% MC); | | | | | | | |

After intragastric administration, whole blood was collected at 0 min, 5 min, 15 min, 30 min, 1 h, 2 h, 4 h, and 7 h, and brain tissue was collected at 5 min,15 min, and 2 h. The whole blood was centrifuged to separate the plasma; the brain tissue was rinsed with cold physiological saline to remove the residual blood on the surface, drained out and then homogenized. Before analysis and detection, all samples were stored at -80°C, and the samples were quantitatively analysed by LC-MS/MS.

**Table 3. Pharmacokinetic parameters of test compounds in mice**

| Test compound | Cmax (ng/mL) | AUC₀₋ₜ (hr*ng/mL) | CL (mL/min/kg) | T_{1/2} (h) | F (%) |
|---|---|---|---|---|---|
| Compound 104 | 4536 | 16855 | 11.1 | 2.20 | >98 |
| Compound 122 | 6401 | 26253 | 9.24 | 2.94 | >98 |
| Compound 128 | 6033 | 25655 | 6.91 | 2.25 | >98 |
| DZ-01 | 3795 | 3611 | 75.1 | 1.23 | >98 |

**Table 4. Pharmacokinetic parameters of test compounds in brain of mice**

| Test compound | Administration mode | Plasma AUC₀₋ₜ (hr*ng/mL) | Brain tissue AUC₀₋ₜ(hr*ng/g) | Brain/plasma ratio |
|---|---|---|---|---|
| Compound 104 | i.g. (10 mg/kg) | 2520 | 4776 | 0.528 |
| Compound 128 | i.g. (10 mg/kg) | 9675 | 5618 | 0.581 |

| | | | | |
|---|---|---|---|---|
| Conclusion: the compounds of the present invention, such as Example compounds 104, 122 and 128, have good pharmacokinetic characteristics in mice. | | | | |

### 3. Pharmacokinetic test in rats

**3.1 Experimental animals:** male SD rats, about 220 g, 6-8 weeks old, divided into an intravenous group and an intragastric group.

**3.2 Experimental design:** on the day of the experiment, the SD rats were randomly grouped according to their body weights. The animals were fasted with water available for 12-14 h one day before the administration, and were fed 4 h after the administration.

**Table 5. Administration information**

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| 3-1 | 3 | Compound 29 | 2.5 | 0.5 | 5 | Plasma | Intravenous administration |
| 3-2 | 12 | | 10 | 1 | 10 | Plasma | Intragastric administration |
| 3-3 | 3 | Compound 128 | 2.5 | 0.5 | 5 | Plasma | Intravenous administration |
| 3-4 | 12 | | 10 | 1 | 10 | Plasma | Intragastric administration |

| | | | | | | | |
|---|---|---|---|---|---|---|---|
| Note: Vehicle for intravenous administration: 5% DMA + 5% Solutol + 90% Saline; vehicle for intragastric administration: 5% DMSO + 95% (0.5% MC); | | | | | | | |

After intragastric administration, whole blood was collected at 0 min, 5 min, 15 min, 30 min, 1 h, 2 h, 4 h, 6 h, 8 h, and 24 h, and brain tissue was collected at 5 min, 15 min, 2 h, and 24 h. The whole blood was centrifuged to separate the plasma; the brain tissue was rinsed with cold physiological saline to remove the residual blood on the surface, drained out and then homogenized. Before analysis and detection, all samples were stored at -80°C, and the samples were quantitatively analysed by LC-MS/MS.

**Table 6. Pharmacokinetic parameters of test compound in plasma of rats**

| Test compound | Cmax (ng/mL) | AUC₀₋ₜ (hr*ng/mL) | CL (mL/min/kg) | T_{1/2} (h) | F (%) |
|---|---|---|---|---|---|
| Compound 29 | 7957 | 12239 | 18.9 | 0.646 | >98 |
| Compound 128 | 5144 | 34023 | 6.33 | 4.68 | >98 |

**Table 7. Pharmacokinetic parameters of test compounds in brain of rats**

| Test compound | Administration mode | Plasma AUC₀₋ₜ (hr*ng/mL) | Brain tissue AUC₀₋ₜ(hr*ng/g) | Brain/plasma ratio |
|---|---|---|---|---|
| Compound 128 | i.g. (10 mg/kg) | 46106 | 22917 | 0.497 |

| | | | | |
|---|---|---|---|---|
| Conclusion: the compounds of the present invention, such as Example compounds 29 and 128, have good pharmacokinetic characteristics in rats. | | | | |

### 4. Pharmacokinetic test in beagle dogs

**Experimental animals:** male beagle dogs, about 8-11 kg, divided into an intravenous group and an intragastric group.

**Experimental method:** on the day of the experiment, the beagle dogs were randomly grouped according to their body weights. The animals were fasted with water available for 12-14 h one day before the administration, and were fed 4 h after the administration. The animals were administered according to Table 6.

**Table 8. Administration information**

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Example compounds | 1 | 1 | 1 | Plasma | Intravenous administration |
| G2 | 3 | Example compounds | 5 | 1 | 5 | Plasma | Intragastric administration |

| | | | | | | | |
|---|---|---|---|---|---|---|---|
| Note: Vehicle for intravenous administration: 5% DMA+5% Solutol+90% Saline; vehicle for intragastric administration: 5% DMSO+95% (0.5% MC) | | | | | | | |

Before and after the administration, 1 ml of blood was taken from the jugular veins or limb veins, and placed in an EDTAK2 centrifuge tube. Centrifugation was performed at 5000 rpm at 4°C for 10 min, and plasma was collected. The blood collection time points for both the intravenous administration group and the intragastric administration group were: 0, 5 min, 15 min, 30 min, 1 h, 2 h, 4 h, 6 h, 8 h, 10 h, 12 h, 24 h, 48 h, and 72 h. Before analysis and detection, all samples were stored at -80°C. The samples were analysed quantitatively by LC-MS/MS.

**Conclusion:** the compounds of the present invention, such as Example compounds, have good pharmacokinetic characteristics in beagle dogs.

### 5. Pharmacokinetic test in monkeys

**Experimental animals:** male cynomolgus monkeys, 3-5 kg, 3-6 years old, divided into an intravenous group and an intragastric group.

**Experimental method:** on the day of the experiment, the monkeys were randomly grouped according to their body weights. The cynomolgus monkeys were fasted with water available for 14-18 h one day before the administration, and were fed 4 h after the administration.

**Table 9. Administration information**

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Compound 128 | 1 | 0.5 | 2 | Plasma | Intravenous administration |
| G2 | 3 | | 5 | 1 | 5 | Plasma | Intragastric administration |

| | | | | | | | |
|---|---|---|---|---|---|---|---|
| Note: Vehicle for intravenous administration: 5% DMA+5% Solutol+90% Saline; vehicle for intragastric administration: 5% DMSO+95% (0.5% MC) | | | | | | | |

Before and after the administration, 1.0 mL of blood was taken from the limb veins and placed in an EDTAK2 centrifuge tube. Centrifugation was carried out at 5000 rpm at 4°C for 10 min, and the plasma was collected. The blood collection time points for the intravenous group and intragastric administration group were: 0 min, 5 min, 15 min, 30 min, 1 h, 2 h, 4 h, 6 h, 8 h, 10 h, 12 h, and 24 h. Before analysis and detection, all samples were stored at -80°C, and the samples were quantitatively analysed by LC-MS/MS.

**Table 10. Pharmacokinetic parameters of test compound in plasma of monkeys**

| Test compound | Cmax (ng/mL) | AUC₀₋ₜ (hr*ng/mL) | CL (mL/min/kg) | T_{1/2} (h) | F (%) |
|---|---|---|---|---|---|
| Compound 128 | 495 | 5936 | 10.1 | 7.19 | 71.4 |

| | | | | | |
|---|---|---|---|---|---|
| Conclusion: the compounds of the present invention, such as Example compound 128, have good pharmacokinetic characteristics in monkeys. | | | | | |

### 6. Test for hERG potassium ion channel

**Experimental platform:** electrophysiological manual patch-clamp system
**Cell line:** Chinese hamster ovary (CHO) cell lines stably expressing hERG potassium ion channel
**Experimental method:** in CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current (I _{hERG}) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula: $Inhibition % = \left[1-\left(I/Io\right)\right] \times 100 %$ where Inhibition% represents the percentage of inhibition of hERG potassium current by the compound, and *I* and *I*o represent the amplitude of hERG potassium current after and before the administration, respectively.

Compound IC₅₀ was calculated using GraphPad Prism 5 software by fitting according to the following equation: $Y = Bottom + \left(Top-Bottom\right) / \left(1+{10}^{∧}\left(\left(LogIC50-X\right)*HillSlope\right)\right)$

Among the equation, X represents the Log value of the tested concentration of the test sample, Y represents the inhibition percentage at the corresponding concentration, and Bottom and Top represent the minimum and maximum inhibition percentage, respectively.

Conclusion: the compounds of the present invention, such as the Example compounds, have no inhibition on hERG.

### 7. Liver microsomal stability test

In this experiment, liver microsomes of five species, including human, dog, rat, and mouse, were used as *in vitro* models to evaluate the metabolic stability of the test compound.

At 37°C, 1 µM test compound was co-incubated with microsomal protein and coenzyme NADPH. At given time points of the reaction (5 min, 10 min, 20 min, 30 min and 60 min), the reaction was terminated by adding ice-cold acetonitrile containing an internal standard. The LC-MS/MS method was used to measure the concentration of the test compound in the sample. T_{1/2} was calculated using the natural logarithm (ln) of the residual rate of the drug in the incubation system and the incubation time. In addition, the intrinsic clearance in liver microsomes CL_{int(mic)} and the intrinsic clearance in liver CL_{int(Liver)} were further calculated.

Conclusion: the compounds of the present invention, such as the Example compounds, have good stability in liver microsomes.

### 8. CYP450 enzyme inhibition test

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the reaction was completed, the sample was treated and liquid chromatography-tandem mass spectrometry (LC-MS/MS) was used to quantitatively detect metabolites produced by specific substrates, changes in CYP enzyme activity were determined, and IC₅₀ value was calculated to evaluate the inhibitory potential of the test compound on each CYP enzyme subtype.

Conclusion: the compounds of the present invention, such as the Example compounds, do not inhibit the CYP enzyme.

### 9. Test of Caco2 permeability

In the experiment, a monolayer of Caco-2 cells was used, and incubated in triplicate in a 96-well Transwell plate. A transport buffer solution (HBSS, 10 mM HEPES, pH 7.4 ± 0.05) containing the compound of the present invention (2 µM) or the control compound digoxin (10 µM), nadolol (2 µM) or metoprolol (2 µM) was added to the administration end hole on the apical side or basal side. A DMSO-containing transport buffer solution was added to the corresponding receiving end hole. After incubation for 2 hours at 37°C ± 1°C, the cell plate was removed, and an appropriate amount of samples was taken from both the apical side and basal side and transferred to a new 96-well plate. Subsequently, acetonitrile containing an internal standard was added to precipitate the protein. The samples were analysed using LC MS/MS, and the concentrations of the compound of the present invention and the control compound were determined. Concentration data were used to calculate apparent permeability coefficients for transport from the apical side to the basal side, and from the basal side to the apical side of the cell monolayer, and thus to calculate the efflux ratio. The integrity of the cell monolayer after 2 hours of incubation was evaluated by leakage of Lucifer Yellow.

Conclusion: the compounds of the present invention, such as the Example compounds, exhibit good permeability.

## Claims

1. A compound as represented by formula (I), or a stereoisomer, a deuterated substance, a solvate, a co-crystal or a pharmaceutically acceptable salt thereof, **characterised in that** 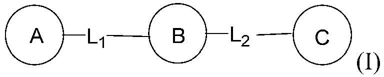
A is selected from 3- to 20-membered heterocycloalkyl or 5- to 20-membered heteroaryl; the heterocycloalkyl and heteroaryl contain 1-5 heteroatoms selected from N, O or S; and the heterocycloalkyl and heteroaryl are optionally substituted with 0-5 R^{A};
B is selected from a bond, C₃₋₁₀ cycloalkyl, C₆₋₁₂ aryl, 3- to 12-membered heterocycloalkyl or 5- to 12-membered heteroaryl; the heterocycloalkyl and heteroaryl contain 1-5 heteroatoms selected from N, O or S; and the cycloalkyl, aryl, heterocyclyl and heteroaryl are optionally substituted with 0-5 R^{B};
C is selected from C₃₋₁₂ cycloalkyl, C₆₋₁₀ aryl, 3- to 14-membered heterocycloalkyl or 5- to 14-membered heteroaryl; the heterocycloalkyl and heteroaryl contain 1-5 heteroatoms selected from N, O or S; and the cycloalkyl, heterocycloalkyl, aryl and heteroaryl are optionally substituted with 0-5 R^{C};
L₁ and L₂ are each independently W₁-R^{L}-W₂, wherein L₁ is connected to A on the left side, and L₂ is connected to B on the left side;
R^{L} is selected from a bond, C₁₋₄ alkylene, C₂₋₄ alkenylene, C₂₋₄ alkynylene, C₃₋₆ cycloalkyl, 5- to 12-membered heteroaryl, or 4- to 10-membered heterocycloalkyl containing 1-3 heteroatoms selected from N, O or S, and the alkylene, alkenylene, alkynylene, cycloalkyl, heteroaryl and heterocycloalkyl are optionally further substituted with 1-4 R^{L1}; each R^{L1} is independently selected from halogen, =O, C₁₋₄ alkyl, C₂₋₄ alkenyl, C₁₋₄ alkoxy, 3- to 6-membered cycloalkyl or 5- to 6-membered heteroaryl, and the alkyl, alkoxy, cycloalkyl and heteroaryl are optionally further substituted with 1-4 substituents selected from halogen, CN, OH, C₁₋₄ alkoxy and NH₂;
W₁ and W₂ are each independently selected from a bond, C₁₋₄ alkylene, -O-, - S-, -S(=O)-, -S(=O)₂-, -NR^{W1}-, -CONR^{W1}-, -NR^{W1}CO-, -C(=O)O-, -OC(=O)-, - C(=S)-, -C(=O)- or -(C₁₋₄ alkylene)-NR^{W1}CO-, and the alkylene is optionally further substituted with 1-4 groups selected from halogen, =O, C₁₋₄ alkyl, CN, OH and NH₂;
R^{W1} is selected from H, C₁₋₄ alkyl, or halogen;
alternatively, L₁ is 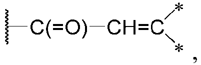 wherein the " * " end is connected to ring B;
each R^{A} is independently selected from halogen, =O, CN, OH, COOH, C₁₋₄ alkyl, C₁₋₄ alkoxy, C₂₋₆ alkenyl, C₂₋₆ alkynyl, 5- to 12-membered heteroaryl, - NRₐCO-C₃₋₆ cycloalkyl, 4- to 12-membered heterocycloalkyl, -O-C₃₋₆ cycloalkyl, - C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4-to 12-membered heterocycloalkyl), -C₁₋₄ alkyl-OC(=O)-NRₐₐ-C₃₋₆ cycloalkyl, - NRₐₐR_{bb}, -SO₂-C₁₋₄ alkyl, -C₃₋₆ cycloalkyl, -NRₐₐ-C₃₋₆ cycloalkyl, 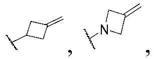 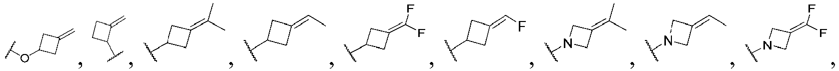 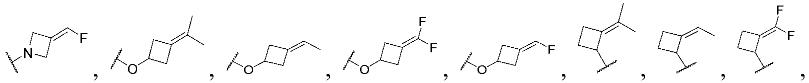 or 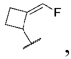 and the alkyl, alkenyl, alkynyl, heteroaryl, cycloalkyl and heterocycloalkyl are optionally further substituted with 1-5 groups selected from halogen, OH, NH₂, CN, C₁₋₄ alkoxy, -O-halogenated C₁₋₄ alkyl, C₁₋₄ alkyl, -C₁₋₄ alkylhydroxy, -C(=O) C₁₋₄ alkyl, or halogenated C₁₋₄ alkyl;
alternatively, two R^{A}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
each R^{B} is independently selected from halogen, CN, =O, OH, -SF₅, C₁₋₄ alkyl, C₁₋₄ alkoxy, C₂₋₆ alkenyl, or C₂₋₆ alkynyl, and the alkyl, alkoxy, alkenyl and alkynyl are optionally further substituted with a group selected from halogen or C₁₋₂ alkyl;
each R^{C} is independently selected from D, halogen, CN, =O, OH, C₁₋₄ alkyl, C₁₋₄ alkoxy, halogenated C₁₋₄ alkyl, halogenated C₁₋₄ alkoxy, C₃₋₅ cycloalkyl, C₆₋₁₀ cycloalkyl, C₂₋₄ alkenyl, C₂₋₄ alkynyl, -SCF₃, -SF₅, 5- to 6-membered heteroaryl, 8-to 10-membered bicyclic heteroaryl, 4- to 12-membered heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-C₅₋₁₁ spirocycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocyclyl), -C(=O)NRₐ-(4- to 6-membered heterocyclyl), 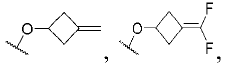 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl, heterocycloalkyl, spirocycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl;
each n is independently selected from 0 or 1;
Rₐ is selected from H, halogen, 3- to 6-membered cycloalkyl, C₁₋₄ alkyl, or halogenated 3- to 6-membered cycloalkyl;
Rₐₐ and R_{bb} are each independently selected from H, halogen, =O, C₁₋₄ alkyl, C₁₋₄ alkylhydroxy, halogenated C₁₋₄ alkyl, C₁₋₄ alkoxy or halogenated C₁₋₄ alkoxy;
alternatively, Rₐₐ and R_{bb}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, two R^{C}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, R^{B} and R^{C}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl.

2. The compound, or the stereoisomer, the deuterated substance, the solvate, the co-crystal or the pharmaceutically acceptable salt thereof according to claim 1, **characterised in that**
A is selected from 5- to 6-membered monocyclic heteroaryl, 6- to 10-membered bicyclic heterocycloalkyl, 9- to 10-membered bicyclic heteroaryl, 10- to 14-membered tricyclic heterocycloalkyl, or 10- to 14-membered tricyclic heteroaryl, and the heterocycloalkyl and heteroaryl are optionally substituted with 1-5 R^{A};
each R^{A} is independently selected from halogen, =O, CN, OH, COOH, C₁₋₄ alkyl, C₁₋₄ alkoxy, 5- to 6-membered heteroaryl, -NRaCO-C₃₋₆ cycloalkyl, 4- to 12-membered heterocycloalkyl, -O-C₃₋₆ cycloalkyl, -C₁₋₄ alkyl-(5- to 6-membered heteroaryl), -O-(4- to 12-membered heterocycloalkyl), -C₁₋₄ alkyl-(4- to 12-membered heterocycloalkyl), -C₁₋₄ alkyl-O-(4- to 12-membered heterocycloalkyl), -C₁₋₄ alkyl-O-C₃₋₆ cycloalkyl, -C₁₋₄ alkyl-OC(=O)-(4- to 12-membered heterocycloalkyl), -C₁₋₄ alkyl-OC(=O)-NRₐₐ-C₃₋₆ cycloalkyl, -NRₐₐR_{bb}, -SO₂-C₁₋₄ alkyl, -C₃₋₆ cycloalkyl, -NRₐₐ-C₃₋₆ cycloalkyl, 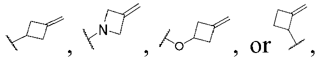 and the alkyl, heteroaryl, cycloalkyl and heterocycloalkyl are optionally further substituted with 1-5 groups selected from halogen, OH, NH₂, CN, C₁₋₄ alkyl, C₁₋₂ alkoxy, -C₁₋₂ alkylhydroxy, -C(=O) C₁₋₂ alkyl, or halogenated C₁₋₂ alkyl;
alternatively, two R^{A}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
B is selected from a bond, 4- to 7-membered monocyclic heterocycloalkyl, C₃₋₈ cycloalkyl, 5- to 6-membered heteroaryl, 5- to 6-membered heteroaryl fused 5- to 6-membered heterocycloalkyl, 5- to 12-membered spiro heterocycloalkyl, 3- to 6-membered cycloalkyl fused 5- to 6-membered heterocycloalkyl, or benzo 5- to 6-membered heterocycloalkyl, and the heterocycloalkyl, heteroaryl, cycloalkyl and aryl are optionally substituted with 1-5 R^{B};
each R^{B} is independently selected from halogen, =O, or C₁₋₂ alkyl, and the alkyl is optionally further substituted with halogen;
C is selected from 5- to 6-membered monocyclic heteroaryl, phenyl, phenyl fused 4- to 6-membered carbocyclyl, phenyl fused 4- to 6-membered heterocycloalkyl, 5- to 6-membered heterocycloalkyl fused 5- to 6-membered heteroaryl, 5- to 6-membered heteroaryl fused 5- to 6-membered carbocyclyl, 8- to 12-membered tricyclic cycloalkyl, or 8- to 14-membered tricyclic heterocyclyl; the heteroaryl, heterocycloalkyl and heterocyclyl contain 1-4 heteroatoms selected from N, O or S; and the phenyl, heterocycloalkyl and carbocyclyl are optionally substituted with 1-5 R^{C};
each R^{C} is independently selected from D, halogen, CN, =O, C₁₋₂ alkyl, C₁₋₂ alkoxy, halogenated C₁₋₂ alkyl, halogenated C₁₋₂ alkoxy, C₃₋₅ cycloalkyl, C₆₋₁₀ cycloalkyl, 5- to 6-membered heteroaryl, 8- to 10-membered bicyclic heteroaryl, 4-to 12-membered heterocycloalkyl, -N=S(=O)RₐₐR_{bb}, -P(=O)RₐₐR_{bb}, -NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, - C(=O)NRₐ-C₃₋₆ cycloalkyl, -O-C₃₋₆ cycloalkyl, -O-(4- to 12-membered heterocycloalkyl), -O-C₅₋₁₁ spirocycloalkyl, -O-(5- to 6-membered heteroaryl), - NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), - NRₐC(=O)-(5- to 6-membered heteroaryl), -C(=O)NRₐ-(4- to 6-membered heteroaryl), -C(=O)NRₐ-(4- to 6-membered heterocycloalkyl), 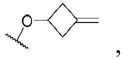 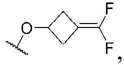 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl, heterocycloalkyl, spirocycloalkyl and heterocyclyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O) C₁₋₄ alkyl, or -S(=O)₂C₁₋₄ alkyl;
Rₐ is selected from H, halogen, 3- to 6-membered cycloalkyl, C₁₋₂ alkyl, or halogenated 3- to 6-membered cycloalkyl;
Rₐₐ and R_{bb} are each independently selected from H, halogen, =O, C₁₋₄ alkyl, C₁₋₄ alkylhydroxy, halogenated C₁₋₄ alkyl, C₁₋₄ alkoxy or halogenated C₁₋₄ alkoxy;
alternatively, Rₐₐ and R_{bb}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
alternatively, R^{B} and R^{C}, together with the atoms to which they are connected, form 5- to 6-membered heterocyclyl, and the heterocyclyl is optionally further substituted with 1-4 groups selected from halogen or C₁₋₂ alkyl;
Li is selected from a bond, C₁₋₂ alkylene, -C(=O)-C₁₋₂ alkylene-, -N(C₁₋₂ alkylene)-C(=O)-C₁₋₂ alkylene-, -NH-C(=O)-C₁₋₂ alkylene-, -S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-C₂₋₄ alkenylene, - C(=O)-NH-, -C(=O)-N(CH₃)-, -C(=O)-O-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, 3- to 6-membered cycloalkyl, 5- to 6-membered heteroaryl, or C₁₋₂ alkyl-C₁₋₂ alkoxy;
alternatively, L₁ is 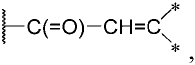
wherein the " * " end is connected to ring B;
L₂ is selected from a bond, C₁₋₂ alkylene, C₂₋₄ alkenylene, C₂₋₄ alkynylene, - C(=O)-NH-, -NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
provided that: when L₂ is selected from -O- or -NH-, L₁ is not -C(=O)-, or B1 is not a bond.

3. The compound, or the stereoisomer, the deuterated substance, the solvate, the co-crystal or the pharmaceutically acceptable salt thereof according to claim 2, **characterised by** having a structure of formula (I-1a), (I-2), (I-3), (I-4), (I-5) or (I-1b): 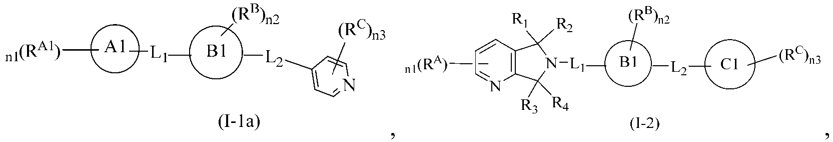 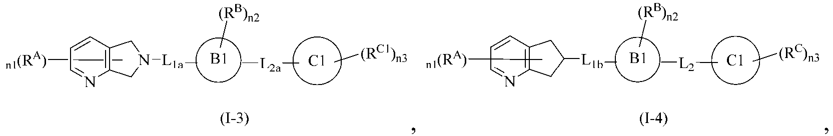 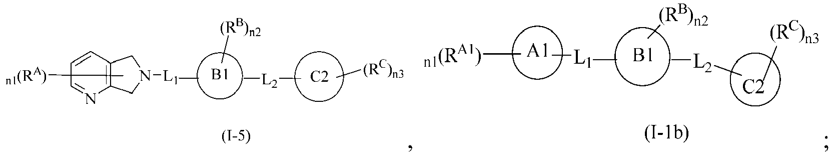
A1 is selected from the following groups: 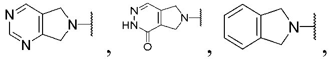 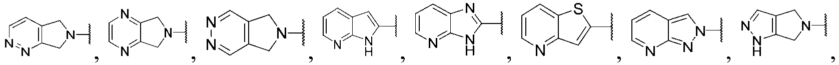 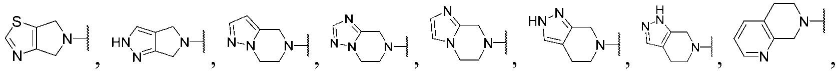 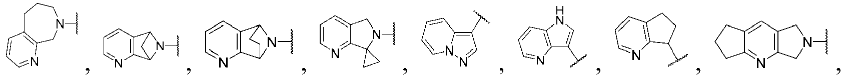 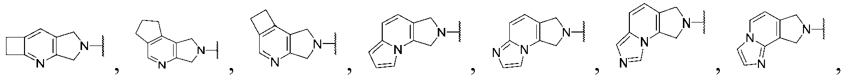 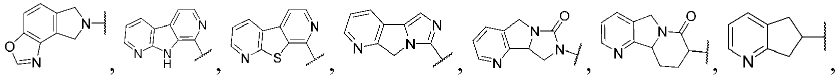 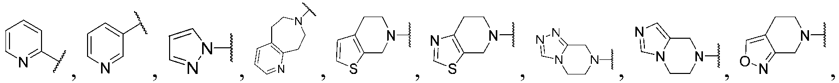 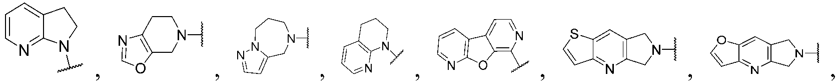 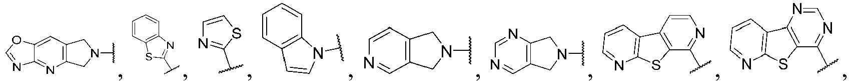 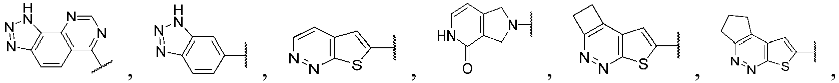 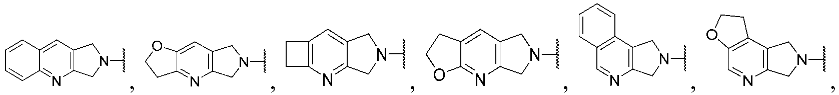 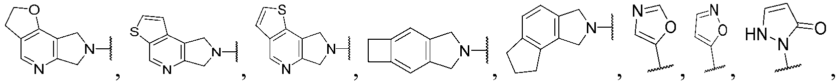 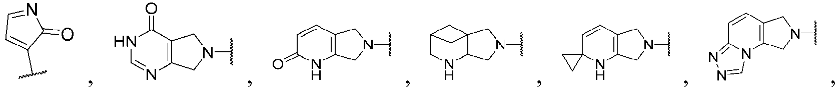 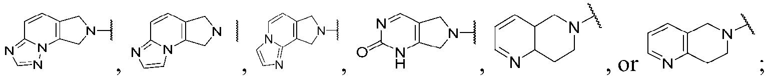
B1 is selected from the following groups: a bond, 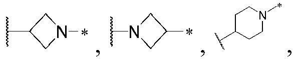 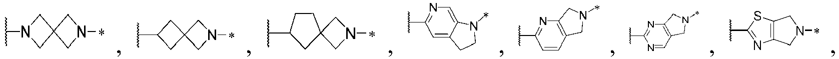 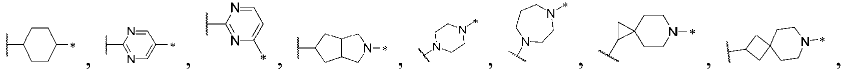 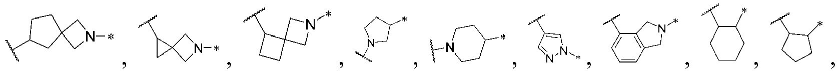 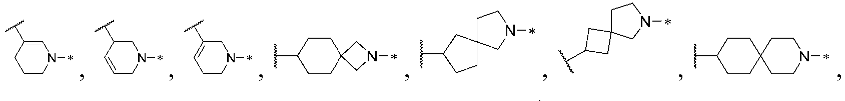 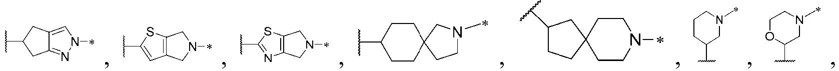 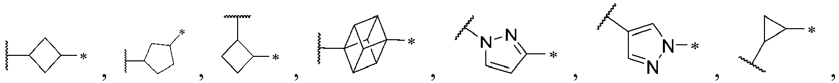 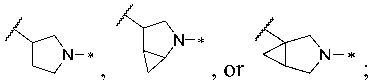
wherein the 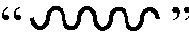 end is connected to L₁ or L₁ₐ or L_{1b}, and the "*" end is **connected** to L₂ or L₂ₐ;
C1 is the following group: 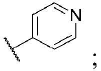
C2 is selected from the following groups: 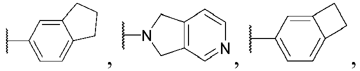 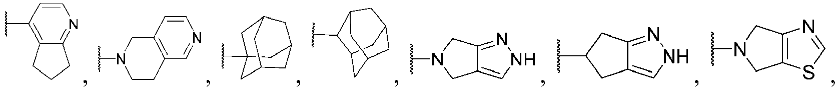 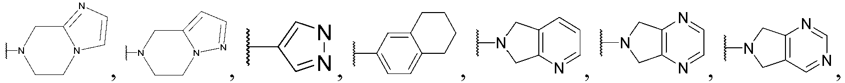 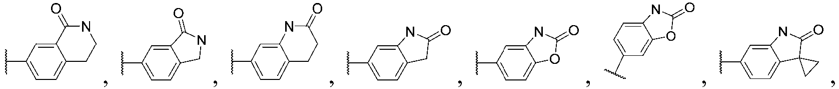 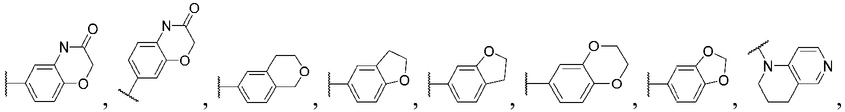 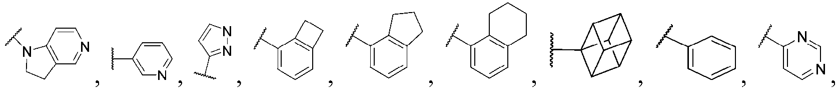 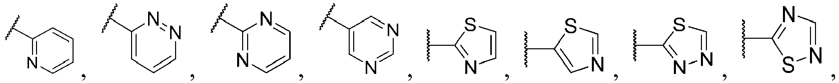 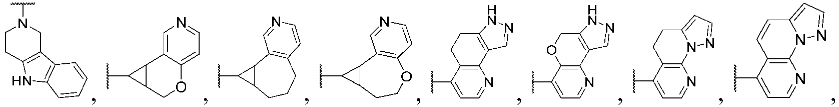 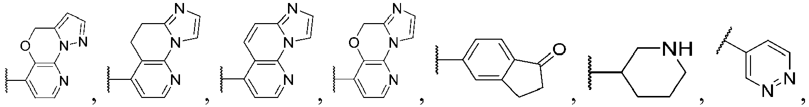 or 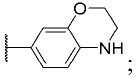
provided that: when C2 is 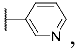 L₂ is not a bond; or when C2 is 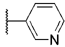 and L₂ is a bond, n3 is not 0 and R^{C} is not F;
R₁, R₂, R₃ and R₄ are each independently selected from H or halogen; provided that R₁, R₂, R₃ and R₄ are not all H;
alternatively, R₃ and R₄ together form =O;
formula (I-3) satisfies one or more of the following conditions:
(1). L₁ₐ is selected from: a bond, C₁₋₂ alkylene, -C(=O)-CH(CH₃)-, -C(=O)-C(RₐR_{b})-, -N(C₁₋₂ alkylene)-C(=O)-C₁₋₂ alkylene-, -NH-C(=O)-C₁₋₂ alkylene-, - S(=O)₂-C₁₋₂ alkylene-, -C(=S)-C₁₋₂ alkylene-, -4- to 6-membered heterocycloalkyl containing 1-3 heteroatoms selected from N, O or S-C₁₋₂ alkylene-, -C(=O)-C₃₋₆ cycloalkyl-, -C₁₋₂ alkylene-C(=O)-, -C(=O)-C(=O)-, C₂₋₄ alkenylene, -C(=O)-C₂₋₄ alkenylene, -C(=O)-NH-, -C(=O)-N(CH₃)-, -C(=O)-O-, 5- to 6-membered heteroaryl-C₁₋₂ alkylene-, 4- to 6-membered monocyclic heterocycloalkyl, C₃₋₆ cycloalkyl, 5- to 6-membered heteroaryl, -C(=O)-, 4- to 6-membered monocyclic heterocycloalkyl-C₁₋₂ alkylene-, 4- to 6-membered monocyclic heterocycloalkyl-C(=O)-C₁₋₂ alkylene-, -C₁₋₂ alkylene-4- to 6-membered monocyclic heterocycloalkyl-, or -C₁₋₂ alkylene-NH-C(=O)-C₁₋₂ alkylene-, and the alkylene, heterocycloalkyl, cycloalkyl, alkenylene, heteroaryl and heterocycloalkyl are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
L₁ₐ is connected to ring B1 on the right side;
Rₐ and R_{b} are each independently selected from H, halogen, 3- to 6-membered cycloalkyl, or C₁₋₂ alkyl; provided that Rₐ and R_{b} are not both H;
L₂ₐ is selected from a bond, C₂₋₄ alkenylene, C₂₋₄ alkynylene, -C(=O)-NH-, - NH-, -O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl; provided that when L₁ₐ is -C(=O)-, and L₂ₐ is selected from -NH- or -O-, B1 is not a bond;
(2). when L₁ₐ is selected from: -C(=O)-CH₂- or -C(=O)-CH₂CH₂-, and L₂ₐ is a bond, at least one R^{C1} is selected from =O, 5- to 6-membered heteroaryl, 8- to 10-membered bicyclic heteroaryl, 4- to 12-membered heterocycloalkyl, - N=S(=O)RaaRbb, -P(=O)RaaRbb, -NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, - O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), -NRₐ-(5- to 6-membered heteroaryl), -NRₐS(=O)₂-C₃₋₆ cycloalkyl, cubane, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocyclyl), -C(=O)NRₐ-(4- to 6-membered heteroaryl), or 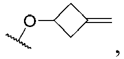 and the alkyl, cycloalkyl, heteroaryl, heterocycloalkyl and cubane are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, - NH-C(=O) C₁₋₄ alkyl, or -S(=O)₂C₁₋₄ alkyl;
(3). L₁ₐ is selected from: -C(=O)-CH₂- or -C(=O)-CH₂CH₂-, and L₂ₐ is selected from C₁₋₂ alkylene, C₂₋₄ alkenylene, C₂₋₄ alkynylene, -C(=O)-NH-, -NH-, - O-, -N(C₁₋₂ alkylene)-, or -C(=O)-, and the alkylene, alkenylene and alkynylene are optionally further substituted with 1-3 R^{L1}; each R^{L1} is independently selected from halogen, =O, C₁₋₂ alkyl, halogenated C₁₋₂ alkyl, C₂₋₄ alkenyl, C₁₋₂ alkoxy, halogenated C₁₋₂ alkoxy, or 3- to 6-membered cycloalkyl;
(4). when L₁ₐ is: -C(=O)-CH₂-, and L₂ₐ is a bond, at least one R^{A} is not C₁₋₂ alkyl, and the compound is not 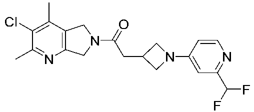 and 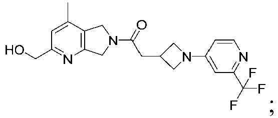
L_{1b} is selected from: a bond, 4- to 6-membered monocyclic heterocycloalkyl, or -C(=O)-NH-, and the heterocycloalkyl is optionally further substituted with 1-4 groups selected from halogen, =O, or C₁₋₂ alkyl;
alternatively, 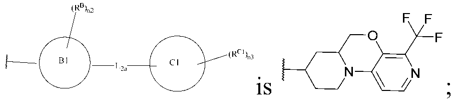
each R^{A} is independently selected from halogen, =O, C₁₋₂ alkyl, -CF₃, 5- to 6-membered heteroaryl, -NRaCO-C₃₋₆ cycloalkyl, 4- to 12-membered heterocycloalkyl, -C₁₋₂ alkyl-(5- to 6-membered heteroaryl), -O-(4- to 12-membered heterocycloalkyl), -C₁₋₂ alkyl-(4- to 12-membered heterocycloalkyl), - C₁₋₂ alkyl-O-(4- to 12-membered heterocycloalkyl), -C₁₋₂ alkyl-O-C₃₋₆ cycloalkyl, - C₁₋₂ alkyl-OC(=O)-(4- to 12-membered heterocycloalkyl), -C₁₋₂ alkyl-OC(=O)-NRₐₐ-C₃₋₆ cycloalkyl, -NRₐₐR_{bb}, -SO₂-C₁₋₂ alkyl, -C₃₋₆ cycloalkyl, -NRₐₐ-C₃₋₆ cycloalkyl, 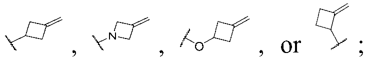 the alkyl is optionally further substituted with 1-4 groups selected from OH, NH₂, CN, C₁₋₂ alkyl, or C₁₋₂ alkoxy; and the heteroaryl, cycloalkyl and heterocycloalkyl are optionally further substituted with 1-4 groups selected from halogen, OH, NH₂, CN, C₁₋₂ alkyl, -C₁₋₂ alkylhydroxy, halogenated C₁₋₂ alkyl, -C(=O)C₁₋₂ alkyl, or C₁₋₂ alkoxy;
each R^{A1} is independently selected from halogen, =O, CN, OH, COOH, C₁₋₄ alkyl, C₁₋₄ alkoxy, 5- to 6-membered heteroaryl, -SO₂-C₁₋₄ alkyl, -NRaCO-C₃₋₆ cycloalkyl, 4- to 12-membered heterocycloalkyl, -O-C₃₋₆ cycloalkyl, -C₁₋₂ alkyl-(5-to 6-membered heteroaryl), or -NRₐₐR_{bb}, and the alkyl, heteroaryl, cycloalkyl and heterocycloalkyl are optionally further substituted with 1-5 groups selected from halogen, OH, NH₂, CN, C₁₋₄ alkyl, halogenated C₁₋₄ alkyl, or C₁₋₄ alkoxy;
alternatively, two R^{A}, together with the atoms to which they are connected, form C₃₋₆ carbocyclyl or 4- to 6-membered heterocyclyl, and the carbocyclyl and heterocyclyl are optionally further substituted with 1-4 groups selected from halogen, OH, NH₂, CN, or C₁₋₂ alkyl; each R^{B} is independently selected from halogen, =O, or C₁₋₂ alkyl, and the alkyl is optionally further substituted with halogen;
each R^{C} is independently selected from D, halogen, C₁₋₂ alkyl, C₁₋₂ alkoxy, halogenated C₁₋₂ alkyl, halogenated C₁₋₃ alkoxy, C₃₋₄ cycloalkyl, 5- to 6-membered heteroaryl, CN, =O, -NH-S(=O)₂-C₁₋₂ alkyl, -O-(5- to 6-membered heteroaryl), -O-C₃₋₆ cycloalkyl, -O-(4- to 12-membered heterocycloalkyl), 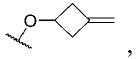 or 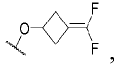 and the alkyl, heteroaryl and cycloalkyl are optionally further substituted with 1-4 groups selected from halogen, C₁₋₂ alkyl, or halogenated C₁₋₂ alkyl;
each R^{C1} is independently selected from halogen, C₁₋₂ alkyl, halogenated C₁₋₂ alkyl, halogenated C₁₋₃ alkoxy, C₃₋₅ cycloalkyl, C₆₋₁₀ cycloalkyl, 5- to 6-membered heteroaryl, 8- to 10-membered bicyclic heteroaryl, 6- to 9-membered spiro heterocycloalkyl, 7- to 10-membered fused heterocycloalkyl, 6- to 8-membered bridged heterocycloalkyl, 4- to 6-membered monocyclic heterocycloalkyl, - N=S(=O)RₐₐR_{bb}, -P(=O)RₐₐR_{bb}, -NRₐS(=O)₂C₁₋₄ alkyl, -NRₐP(=O)(C₁₋₄ alkyl)₂, - NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4- to 12-membered heterocycloalkyl), -O-(5- to 6-membered heteroaryl), -NRₐ-C₃₋₆ cycloalkyl, -NRₐ-(4- to 12-membered heterocycloalkyl), - NRₐ-(5- to 6-membered heteroaryl), -NRₐS(=O)₂-C₃₋₆ cycloalkyl, -C₁₋₄ alkyl-(5- to 6-membered heteroaryl), -C₁₋₄ alkyl-(C₆₋₁₀ aryl), -C₁₋₄ alkyl-(4- to 6-membered heterocycloalkyl), -NRₐC(=O)-O-Rₐ, -(C₁₋₄ alkyl)ₙ-O-(C₁₋₄ alkyl)ₙ-(4- to 6-membered heterocyclyl), -NRₐC(=O)-(4- to 6-membered heterocycloalkyl), - NRₐC(=O)-(5- to 6-membered heteroaryl), -C(=O)NRₐ-(5- to 6-membered heteroaryl), 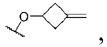 -C(=O)-C₃₋₆ cycloalkyl, -C(=O)-(4- to 12-membered heterocycloalkyl), or -C(=O)-(5- to 6-membered heteroaryl), and the alkyl, cycloalkyl, heteroaryl and heterocycloalkyl are optionally further substituted with 1-4 groups selected from halogen, OH, NH₂, CN, C₁₋₄ alkyl, halogenated C₁₋₄ alkyl, deuterated C₁₋₄ alkyl, =O, -C(=O) C₁₋₄ alkyl, -NH-C(=O)C₁₋₄ alkyl, or -S(=O)₂C₁₋₄ alkyl;
Rₐₐ and R_{bb} are each independently selected from H, halogen, =O, C₁₋₄ alkyl, C₁₋₄ alkylhydroxy, halogenated C₁₋₄ alkyl, C₁₋₄ alkoxy or halogenated C₁₋₄ alkoxy;
alternatively, Rₐₐ and R_{bb}, together with the atoms to which they are connected, form C₃₋₆ carbocyclyl or 4- to 10-membered heterocyclyl, and the carbocyclyl and heterocyclyl are optionally further substituted with 1-4 groups selected from halogen, OH, NH₂, CN, or C₁₋₄ alkyl;
n1, n2 and n3 are each independently selected from 0, 1, 2, 3, 4 or 5.

4. The compound, or the stereoisomer, the deuterated substance, the solvate, the co-crystal or the pharmaceutically acceptable salt thereof according to claim 3, **characterised in that**, in the compound of formula (I-1a), (1-2), (I-3), (I-4), (1-5) or (1-1b),
each R^{A} is independently selected from F, Cl, =O, CN, methyl, ethyl, - NHCO-C₃₋₆ cycloalkyl, 4- to 6-membered monocyclic heterocycloalkyl, 5-membered heteroaryl, 6-membered heteroaryl, -C₁₋₂ alkyl-(5-membered heteroaryl), -C₁₋₂ alkyl-(6-membered heteroaryl), -O-(4- to 6-membered monocyclic heterocycloalkyl), -C₁₋₂ alkyl-(4- to 6-membered monocyclic heterocycloalkyl), - C₁₋₂ alkyl-O-(4- to 6-membered monocyclic heterocycloalkyl), -C₁₋₂ alkyl-O-C₃₋₆ cycloalkyl, -C₁₋₂ alkyl-OC(=O)-(4- to 6-membered monocyclic heterocycloalkyl), - C₁₋₂ alkyl-OC(=O)-NH-C₃₋₆ cycloalkyl, -NHRₐₐ, -SO₂CH₃, cyclopropyl, cyclobutyl, -NH-cyclopropyl, -NH-cyclobutyl, oxolanyl, 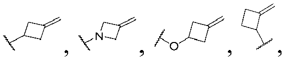 -CF₃, or 7- to 10-membered bicyclic heterocycloalkyl; the methyl and ethyl are optionally further substituted with 1-4 groups selected from OH, NH₂, CN, methyl, ethyl, or methoxy; and the heteroaryl, cycloalkyl and heterocycloalkyl are optionally further substituted with 1-4 groups selected from F, Cl, OH, NH₂, CN, methyl, ethyl, - CHF₂, -CH₂F, -CF₃, -C(=O) CH₃, -O-CH₃, or -O-CH₂CH₃;
alternatively, two R^{A}, together with the atoms to which they are connected, form 5- to 6-membered heterocyclyl, and the heterocyclyl is optionally further substituted with 1-4 groups selected from F, Cl, OH, NH₂, CN, methyl, or ethyl;
each R^{A1} is independently selected from F, Cl, =O, CN, OH, COOH, - SO₂CH₃, methyl, ethyl, -O-C₃₋₆ cycloalkyl, -NHRₐₐ, 5-membered heteroaryl, or 4-to 6-membered heterocycloalkyl, and the methyl, ethyl, cycloalkyl and 4- to 6-membered heterocycloalkyl are optionally further substituted with 1-5 groups selected from F, Cl, OH, NH₂, CN, methyl, ethyl, -CHF₂, -CH₂F, -CF₃, or methoxy;
each R^{B} is independently selected from F, Cl, =O, methyl, or ethyl, and the methyl and ethyl are optionally further substituted with a group selected from F or Cl;
each R^{C} is independently selected from D, F, Cl, methyl, ethyl, -CHF₂, -CH₂F, -CF₃, -CH₂CF₃, -CH₂CHF₂, -CH₂CH₂F, -OCHF₂, -OCH₂F, -OCF₃, -OCH₂CF₃, - OCH(CH₃)CF₃, cyclopropyl, cyclobutyl, 5-membered heteroaryl, 6-membered heteroaryl, -CN, =O, -NH-S(=O)₂-C₁₋₂ alkyl, -O-(5-membered heteroaryl), -O-C₃₋₆ cycloalkyl, -O-(4- to 6-membered monocyclic heterocycloalkyl), 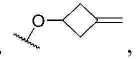 or 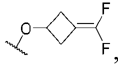 and the methyl, ethyl, cyclopropyl, cyclobutyl, heteroaryl, cycloalkyl and heterocycloalkyl are optionally further substituted with 1-4 groups selected from F, Cl, methyl, ethyl, -CHF₂, -CH₂F, or -CF₃;
each R^{C1} is independently selected from F, Cl, methyl, ethyl, -CHF₂, -CH₂F, - CF₃, -CH₂CF₃, -CH₂CHF₂, -CH₂CH₂F, -OCHF₂, -OCH₂F, -OCF₃, -OCH₂CF₃, - OCH(CH₃)CF₃, cyclopropyl, cyclobutyl, cyclopentyl, cubane, 5-membered heteroaryl, 6-membered heteroaryl, 8-membered fused heteroaryl, 9-membered fused heteroaryl, 10-membered fused heteroaryl, 7- to 8-membered spiro heterocycloalkyl, 8- to 9-membered fused heterocycloalkyl, 7-membered bridged heterocycloalkyl, 4- to 6-membered monocyclic heterocycloalkyl, -N=S(=O)RₐₐR_{bb}, -P(=O)RₐₐR_{bb}, -NRₐS(=O)₂C₁₋₄ alkyl, -NRₐP(=O)(C₁₋₄ alkyl)₂, -NRₐS(=O)₂NRₐₐR_{bb}, -NRₐC(=O)-C₃₋₆ cycloalkyl, -C(=O)NRₐ-C₃₋₆ cycloalkyl, -O-C₃₋₆ cycloalkyl, -O-(4-to 6-membered heterocycloalkyl), -O-(5- to 6-membered heteroaryl), -NH-C₃₋₆ cycloalkyl, -NH-(4- to 6-membered heterocycloalkyl), -NH-(5- to 6-membered heteroaryl), -NH-S(=O)₂-C₃₋₆ cycloalkyl, -C₁₋₂ alkyl-(5-membered heteroaryl), -C₁₋₂ alkyl-(6-membered heteroaryl), -C₁₋₂ alkyl-phenyl, -C₁₋₂ alkyl-(4- to 6-membered heterocycloalkyl), -NRₐC(=O)-O-Rₐ, -(C₁₋₂ alkyl)ₙ-O-(C₁₋₂ alkyl)ₙ-(4- to 6-membered heterocyclyl), -NRₐC(=O)-(4- to 6-membered heterocyclyl), - C(=O)NRₐ-(4- to 6-membered heterocyclyl), 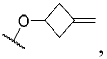 -C(=O)-C₃₋₆ cycloalkyl, - C(=O)-(4- to 12-membered heterocycloalkyl), or -C(=O)-(5- to 6-membered heteroaryl), and the cyclopropyl, cyclobutyl, cyclopentyl, alkyl, cycloalkyl, heterocyclyl, heteroaryl, heterocycloalkyl and cubane are optionally further substituted with 1-4 groups selected from F, Cl, OH, NH₂, CN, methyl, ethyl, deuterated methyl, -CHF₂, -CH₂F, -CF₃, -CH₂CF₃, -CH₂CHF₂, -CH₂CH₂F, =O, - C(=O) C₁₋₄ alkyl, -NH-C(=O) C₁₋₄ alkyl, or -S(=O)₂C₁₋₂ alkyl;
Rₐ is selected from H, F, Cl, methyl, ethyl, cyclopropyl, cyclobutyl, cyclopentyl, or difluorocyclobutyl;
Rₐₐ and R_{bb} are each independently selected from H, F, Cl, =O, methyl, ethyl, -CH₂-CH₂-OH, -CH₂-OH, -CHF₂, -CH₂F, -CF₃, -CH₂CF₃, -CH₂CHF₂, -CH₂CH₂F, -OCH₃, -OCH₂CH₃, -OCHF₂, -OCH₂F, -OCF₃, -OCH₂CF₃, -OCH₂CHF₂, or - OCH₂CH₂F;
alternatively, Rₐₐ and R_{bb}, together with the atoms to which they are connected, form 4- to 6-membered heterocyclyl, and the heterocyclyl is optionally further substituted with 1-4 groups selected from F, Cl, OH, NH₂, CN, methyl, or ethyl; n1 is selected from 0, 1, 2 or 3;
n2 is selected from 0 or 1;
n3 is selected from 0, 1, 2, 3 or 4;
L₁ is selected from a bond, vinylene, -CH₂-, -CH₂-CH₂-, 4- to 6-membered monocyclic heterocycloalkyl, (4- to 6-membered monocyclic heterocycloalkyl)-C(=O)-CH₂-, -(4- to 6-membered monocyclic heterocycloalkyl)-CH₂-, -CH₂-(4- to 6-membered monocyclic heterocycloalkyl)-, -CH(CH₃)NHC(=O)-CH₂-, - CH₂NHC(=O)-CH₂-, -S(=O)₂-CH₂-, -C(=S)-CH₂-, -C(=O)-C₃₋₆ cycloalkyl, - C(=O)-CH(CH₃)-, -C(=O)-CH(C₃₋₆ cycloalkyl)-, -C(=O)-CF₂-, -C(=O)-C(CH₃)₂-, - CH₂-C(=O)-, -C(=O)-C(=O)-, C₃₋₆ cycloalkyl, -(5- to 6-membered heteroaryl)-CH₂-, 5- to 6-membered heteroaryl, -C(=O)-, -C(=O)-CH₂-, -N(CH₃)-CH₂-, -CF₂-CH₂-, -C(=O)-C₂₋₄ alkenylene, -C(=O)-O-, -C(=O)-NH-, or -C(=O)-N(CH₃)-, and the -CH₂-, alkenylene, cycloalkyl, heterocycloalkyl and heteroaryl are optionally further substituted with 1-3 R^{L1}; each R^{L1} is independently selected from F, Cl, Br, =O, methyl, ethyl, -CF₃, -CHF₂, -CH₂F, vinyl, propenyl, methoxy, ethoxy, -OCF₃, -OCHF₂, -OCH₂F, cyclopropyl, cyclobutyl, methyl-methoxy, or 5-membered heteroaryl;
alternatively, Li is 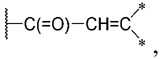 wherein the " * " end is connected to ring B;
L₂ is selected from a bond, vinylene, ethynylene, -CH₂-, -CH₂-CH₂-, -O-, - NH-, -C(=O)-, or -N(CH₃)-.

5. The compound, or the stereoisomer, the deuterated substance, the solvate, the co-crystal or the pharmaceutically acceptable salt thereof according to claim 1, **characterised by** having a structure of formula (II): 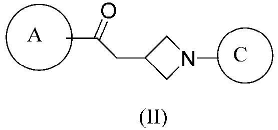
ring A is selected from the following structures optionally further substituted with 1, 2 or 3 R^{A}: 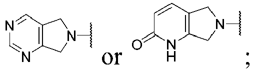 or is 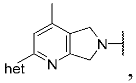 wherein het is 5-membered heteroaryl, and the heteroaryl is optionally substituted with 1 and 2 groups selected from halogen, C₁₋₂ alkyl, halogenated C₁₋₂ alkyl, or deuterated C₁₋₂ alkyl;
each R^{A} is independently selected from halogen, CN, C₁₋₄ alkyl, C₁₋₄ alkoxy, halogenated C₁₋₄ alkyl, -O-C₃₋₆ cycloalkyl, 4- to 6-membered heterocycloalkyl, 5-to 6-membered heteroaryl, or -SO₂-C₁₋₂ alkyl, and the heteroaryl, cycloalkyl and heterocycloalkyl are optionally further substituted with 1-2 groups selected from halogen, OH, NH₂, CN, C₁₋₂ alkyl, C₁₋₂ alkoxy, -C(=O)C₁₋₂ alkyl, or halogenated C₁₋₂ alkyl;
ring C is selected from 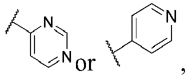 and the ring C is optionally further substituted with 1, 2 or 3 R^{C};
each R^{C} is independently selected from halogen, halogenated C₁₋₃ alkyl, halogenated C₁₋₄ alkoxy, C₃₋₅ cycloalkyl, 5- to 6-membered heteroaryl, -O-C₃₋₆ cycloalkyl, -O-(4- to 12-membered heterocycloalkyl), -O-(5- to 6-membered heteroaryl), -O-C₅₋₈ spirocycloalkyl, 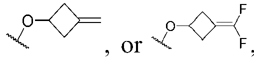 and the heteroaryl, cycloalkyl and spirocycloalkyl are optionally further substituted with 1-3 groups selected from halogen, C₁₋₂ alkyl, halogenated C₁₋₂ alkyl, or deuterated C₁₋₂ alkyl.

6. The compound, or the stereoisomer, the deuterated substance, the solvate, the co-crystal or the pharmaceutically acceptable salt thereof according to claim 5, **characterised in that**
ring A is selected from 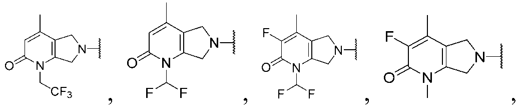 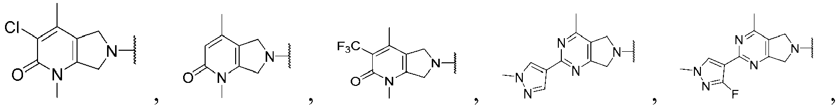 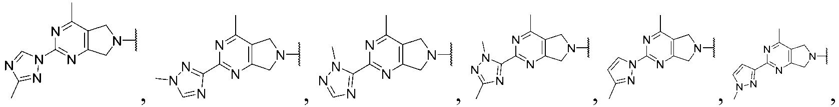 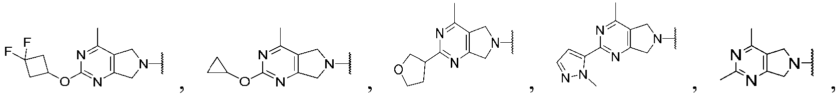 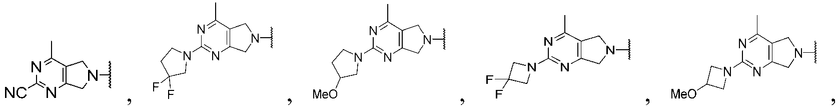 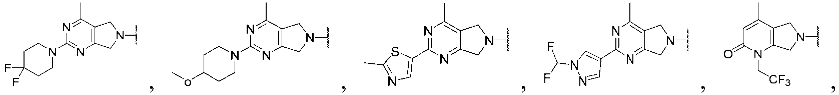 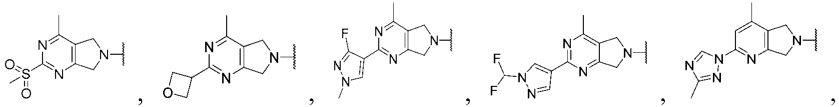 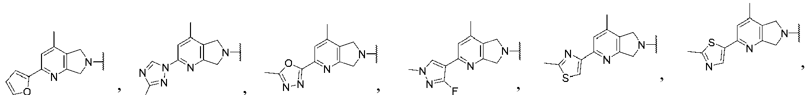 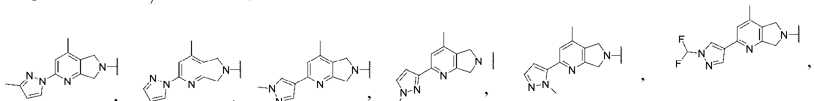 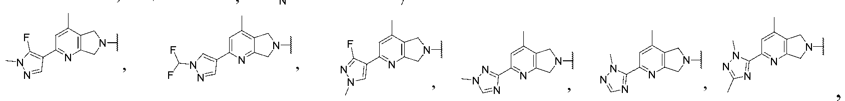 or 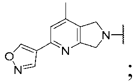
each R^{C} is independently selected from 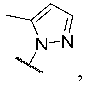 F, Cl, CF₃, CHF₂, CH₂F, - OCHF₂, -OCH₂F, -OCF₃, -OCH₂CF₃, -OCH(CH₃)CF₃, cyclopropyl, cyclobutyl,

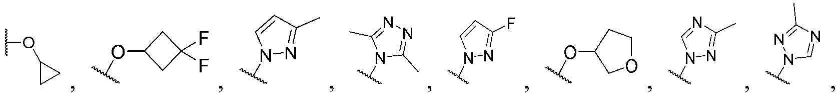

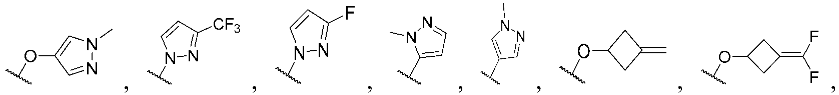

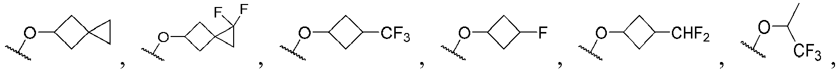

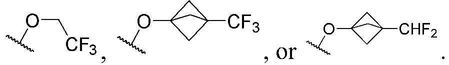

7. The compound, or the stereoisomer, the deuterated substance, the solvate, the co-crystal or the pharmaceutically acceptable salt thereof according to claim 1, **characterised in that** the compound is of a structure selected from one of the structures in Table A.

8. A pharmaceutical composition or pharmaceutical preparation, **characterised by** comprising the compound, or the stereoisomer, the deuterated substance, the solvate, the co-crystal or the pharmaceutically acceptable salt thereof according to any one of claims 1-7, and a pharmaceutically acceptable carrier and/or excipient.

9. The pharmaceutical composition or pharmaceutical preparation according to claim 8, **characterised by** comprising 1-1500 mg of the compound, or the stereoisomer, the deuterated substance, the solvate, the co-crystal or the pharmaceutically acceptable salt thereof according to any one of claims 1-7, and a carrier and/or excipient.

10. Use of the compound, or the stereoisomer, the deuterated substance, the solvate, the co-crystal or the pharmaceutically acceptable salt thereof according to any one of claims 1-7, or the composition according to claim 8 or 9 in the preparation of a medicament for treating/preventing a CHRM4-mediated disease.

11. The use according to claim 10, **characterised in that** the CHRM4-mediated disease is selected from Alzheimer's disease, schizophrenia or psychosis, pain, addiction, sleep disorder, cognitive impairment, Parkinson's disease, Parkinson's disease-levodopa-induced dyskinesia, Huntington's disease, dyskinesia, xerostomia, pulmonary arterial hypertension, chronic obstructive pulmonary disease, asthma, urinary incontinence, glaucoma, trisomy 21 syndrome, cerebral amyloid angiopathy, dementia, hereditary cerebral haemorrhage with amyloidosis-Dutch type, prion disease, amyotrophic lateral sclerosis, progressive supranuclear palsy, head trauma, stroke, pancreatitis, inclusion body myositis, other peripheral amyloidosis, diabetes, autism and atherosclerosis; preferably Alzheimer's disease, schizophrenia, pain, addiction and sleep disorder.

12. A method for treating a disease in a mammal or human, **characterised by** comprising administering to a subject a therapeutically effective amount of the compound, or the stereoisomer, the deuterated substance, the solvate or the pharmaceutically acceptable salt thereof according to any one of claims 1-7, wherein the therapeutically effective amount is preferably 1-1500 mg, and the disease is selected from Alzheimer's disease, schizophrenia or psychosis, pain, addiction, sleep disorder, cognitive impairment, Parkinson's disease, Parkinson's disease-levodopa-induced dyskinesia, Huntington's disease, dyskinesia, xerostomia, pulmonary arterial hypertension, chronic obstructive pulmonary disease, asthma, urinary incontinence, glaucoma, trisomy 21 syndrome, cerebral amyloid angiopathy, dementia, hereditary cerebral haemorrhage with amyloidosis-Dutch type, prion disease, amyotrophic lateral sclerosis, progressive supranuclear palsy, head trauma, stroke, pancreatitis, inclusion body myositis, other peripheral amyloidosis, diabetes, autism and atherosclerosis; preferably Alzheimer's disease, schizophrenia, pain, addiction and sleep disorder, preferably Alzheimer's disease, schizophrenia, pain, addiction and sleep disorder, preferably Alzheimer's disease, schizophrenia, pain, addiction and sleep disorder.
